# EUROPEAN PATENT APPLICATION

(11) **EP 4 653 436 A1**
(43) Date of publication of application: **26.11.2025**
(21) Application number: 24744409.4
(22) Date of filing: 19.01.2024
(51) Int. Cl.: C07D 471/04, C07D 495/04, C07D 487/04, C07D 519/00, A61K 31/4188, A61K 31/407, A61P 35/00, A61P 37/00, A61P 31/00, A61P 29/00

(54) **HETEROCYCLIC COMPOUNDS, PREPARATION METHOD THEREFOR AND USE THEREOF**

(30) Priority: 19.01.2023 CN 202310059581; 18.08.2023 CN 202311044089
(71) Applicant: GUANGZHOU YUFAN NANTU BIOTECHNOLOGIES CO., LTD, Guangzhou, Guangdong 511453 (CN)
(72) Inventor: LIN, Xingyu, Zhuhai, Guangdong 519031 (CN); AN, Na, Zhuhai, Guangdong 519031 (CN); LU, Tingting, Zhuhai, Guangdong 519031 (CN); LIU, Yahui, Zhuhai, Guangdong 519031 (CN)
(74) Representative: Michalski Hüttermann & Partner Patentanwälte mbB
(86) International application number: PCT/CN2024/073346
(87) International publication number: WO 2024/153240

(57) **Abstract**

A heterocyclic compound and a preparation method therefor and an use thereof, especially an use in Cbl-b inhibition, are provided. The compound has good Cbl-b inhibitory activity and is expected to be used for the prevention and treatment of diseases related to Cbl-b activity.

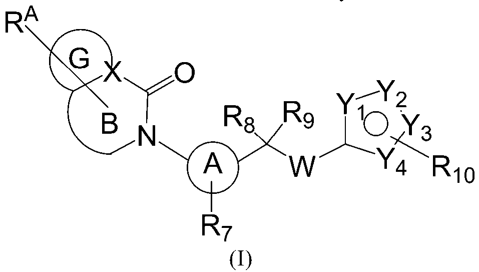

## Description

### TECHNICAL FIELD

The present disclosure relates to the field of chemical pharmaceuticals, and specifically to a heterocyclic compound (especially a pyridone-fused five-membered heterocyclic compound) and a preparation method therefor and an use thereof, especially its use in Cbl-b inhibition.

### BACKGROUND

Ubiquitin is a small protein composed of 76 amino acids with a highly conserved sequence, found in eukaryotic cells. The main function of ubiquitin is to tag target proteins, which are then recognized and degraded by the proteasome. This process is known as the ubiquitin-proteasome system (UPS). Among the enzymes involved, ubiquitin-protein ligase (E3) directly binds to the protein and determines the specificity of degradation.

The degradation of proteins via the lysosome or proteasome following protein ubiquitination is essential for maintaining normal cellular homeostasis. Dysfunction in this process is closely associated with the development of many diseases, such as tumors and autoimmune diseases.

The Casitas B-lineage lymphoma (Cbl) family of proteins are E3 ubiquitin ligases with a RING (Really Interesting New Gene) finger domain, and includes Cbl, Cbl-b, and Cbl-c. Among them, Cbl-b has been identified as a key regulator of adaptive immune responses. Cbl-b is essential for establishing the activation threshold of T cells and for regulating peripheral T cell tolerance through multiple mechanisms. Recent studies indicate that Cbl-b also modulates innate immune responses and plays a critical role in host defense against pathogens and in antitumor immunity (see, for example, Tang R, Langdon W.Y., Zhang J. Regulation of immune responses by E3 ubiquitin ligase Cbl-b[J], Cellular Immunology, 2018). These findings suggest that targeting Cbl-b may represent a promising therapeutic strategy, such as through the use of Cbl-b inhibitors, for the treatment of immune-related human diseases, including autoimmune disorders, infections, and cancers. However, to date, there have been few reports on Cbl-b inhibitors.

### SUMMARY

To overcome the shortcomings of the prior art, the present disclosure provides a heterocyclic compound (especially a pyridone-fused five-membered heterocyclic compound) and a preparation method therefor and an use thereof, especially its use in Cbl-b inhibition.

In a first aspect, the present disclosure provides a compound, having the following structure:

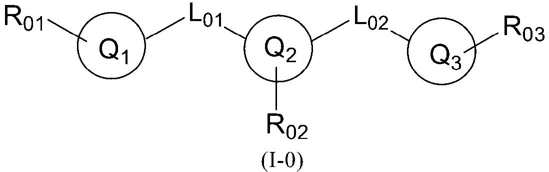

Specifically,
Q1-ring is an aliphatic ring, an aromatic ring, or a heterocyclic ring;
Q2-ring is an aliphatic ring, an aromatic ring, or a heterocyclic ring;
Q3-ring is an aliphatic ring, an aromatic ring, or a heterocyclic ring;
R₀₁, R₀₂, and R₀₃ are each one or more independent substituents on the Q₁-ring, Q₂-ring, and Q₃-ring, they are each selected from: H, D, =O, halogen, cyano, nitro, azido, -OR₀₄, -C(O)R₀₄, -C(O)OR₀₄, -NR₀₅C(O)OR₀₄, -OC(O)R₀₄, -NR₀₅SO₂R₀₄, -SO₂NR₀₄R₀₅, -NR₀₅C(O)R₀₄, -C(O)NR₀₄R₀₅, -NR₀₄R₀₅, -SR₀₄, -S(O)R₀₄, -S(O)₂R₀₄, -SO₃H, C₁-C₁₀ alkyl, C₂-C₁₀ alkenyl, C₂-C₁₀ alkynyl, -(CH₂)ₜ₁(C₆-C₁₀ aryl), -SO₂(CH₂)ₜ₁(C₆-C₁₀ aryl), -S(CH₂)ₜ₁(C₆-C₁₀ aryl), -O(CH₂)ₜ₁(C₆-C₁₀ aryl), -(CH₂)ₜ₁(4-10 membered heterocyclyl), -SO₂(CH₂)ₜ₁(4-10 membered heterocyclyl), -S(CH₂)ₜ₁(4-10 membered heterocyclyl), -O(CH₂)ₜ₁(4-10 membered heterocyclyl), -(CH₂)ₜ₁(C₃-C₁₀ cycloalkyl), -SO₂(CH₂)ₜ₁(C₃-C₁₀ cycloalkyl), -S(CH₂)ₜ₁(C₃-C₁₀ cycloalkyl), and -O(CH₂)ₜ₁(C₃-C₁₀ cycloalkyl), and t1 is an integer selected from 0 to 10 (such as 0, 1, 2, 3, 4, 5, 6, 7, 8, 9, and 10); among them 0-6 methylene units in the C₁-C₁₀ alkyl are optionally substituted with groups selected from: -Cy-, -O-, -S-, -S-S-, -Si-, -C(O)-, -C(S)-, -C(O)O-, -OC(O)-, -OC(O)O-, -C(O)N(R₀₄)-, -N(R₀₄)C(O)-, -N(R₀₄C(O)O-, -N(R₀₄)C(O)N(R₀₅)-, -N(R₀₄)-, -S(O)₂-, -S(O)₂N(R₀₄)-, -N(R₀₄)S(O)₂-, -S(O)-, -S(O)N(R₀₄)-, -N(R₀₄)S(O)-, -OP(O)(OR₀₄)O-, -P(O)(OR₀₄)O-, -P(O)-, -OP(O)N(R₀₄)-, -P(O)N(R₀₄)-, -P(O)(N(R₀₄R₀₅))-, -OP(O)(OR₀₄)₂N(R₀₅)-, -P(O)(OR₀₄)₂N(R₀₅)-, -N(R₀₄)P(O)(OR₀₅)O-, -N(R₀₄)P(O)-, 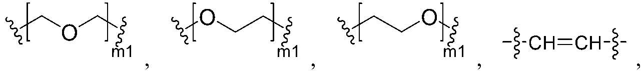 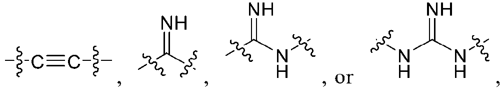 and m1 is an integer selected from 1 to 10 (such as 1, 2, 3, 4, 5, 6, 7, 8, 9, and 10); among them H on the C₁-C₂₀ alkyl, C₂-C₁₀ alkenyl, C₂-C₁₀ alkynyl, C₃-C₁₀ cycloalkyl, C₆-C₁₀ aryl, or 4-10 membered heterocyclyl is optionally substituted with one or more R₀₆ groups;
each R₀₄ and R₀₅ are independently selected from a group consisting of H, D, C₁-C₁₀ alkyl, C₂-C₁₀ alkenyl, C₂-C₁₀ alkynyl, -(CH₂)ₜ₁(C₆-C₁₀ aryl), -(CH₂)ₜ₁(4-10 membered heterocyclyl), and -(CH₂)ₜ₁(C₃-C₁₀ cycloalkyl), and t1 is an integer selected from 1 to 10 (such as 1, 2, 3, 4, 5, 6, 7, 8, 9, and 10); among them H on the C₁-C₁₀ alkyl, C₂-C₁₀ alkenyl, C₂-C₁₀ alkynyl, C₃-C₁₀ cycloalkyl, C₆-C₁₀ aryl, or 4-10 membered heterocyclyl is optionally substituted by one or more R₀₆ groups;
each R₀₄ and R₀₅ are independently selected from: H, D, C₁-C₁₀ alkyl, C₂-C₁₀ alkenyl, C₂-C₁₀ alkynyl, -(CH₂)ₜ₁(C₆-C₁₀ aryl), -(CH₂)ₜ₁(4-10 membered heterocyclyl), and -(CH₂)ₜ₁(C₃-C₁₀ cycloalkyl), and t1 is an integer selected from 1 to 10 (such as 1, 2, 3, 4, 5, 6, 7, 8, 9, and 10); among them, H on the C₁-C₁₀ alkyl, C₂-C₁₀ alkenyl, C₂-C₁₀ alkynyl, C₃-C₁₀ cycloalkyl, C₆-C₁₀ aryl, 4-10 membered heterocyclyl is optionally substituted with one or more R₀₆ groups;
each R₀₆ is selected from: D, halogen, cyano, nitro, azido, C₁-C₁₀ alkyl, C₂-C₁₀ alkenyl, C₂-C₁₀ alkynyl, C₁-C₁₀ haloalkyl, -OR₀₇, -C(O)R₀₇, -C(O)OR₀₇, -NR₀₈C(O)OR₀₇, -OC(O)R₀₇, -NR₀₈SO₂R₀₇, -SO₂NR₀₇R₀₈, -NR₀₇C(O)R₀₈, -C(O)NR₀₇R₀₈, -NR₀₇R₀₈, -SR₀₇, -S(O)R₀₇, -S(O)₂R₀₇, -SO₃H, -(CH₂)ₜ₂(C₆-C₁₀ aryl), -SO₂(CH₂)ₜ₂(C₆-C₁₀ aryl), -S(CH₂)ₜ₂(C₆-C₁₀ aryl), -O(CH₂)ₜ₂(C₆-C₁₀ aryl), -(CH₂)ₜ₂(4-10 membered heterocyclyl), -SO₂(CH₂)ₜ₂(4-10 membered heterocyclyl), -S(CH₂)ₜ₂(4-10 membered heterocyclyl), -O(CH₂)ₜ₂(4-10 membered heterocyclyl), -(CH₂)ₜ₂(C₃-C₁₀ cycloalkyl), -SO₂(CH₂)ₜ₂(C₃-C₁₀ cycloalkyl), -S(CH₂)ₜ₂(C₃-C₁₀ cycloalkyl), and -O(CH₂)ₜ₂(C₃-C₁₀ cycloalkyl), and t2 is an integer selected from 1 to 10 (such as 0, 1, 2, 3, 4, 5, 6, 7, 8, 9, and 10); each R₀₇ and R₀₈ are independently selected from: H, C₁-C₁₀ alkyl, C₂-C₁₀ alkenyl, C₂-C₁₀ alkynyl, -(CH₂)ₜ(C₆-C₁₀ aryl), -(CH₂)ₜ(4-10 membered heterocyclyl), and -(CH₂)ₜ(C₃-C₁₀ cycloalkyl);
each R₀₇ and R₀₈ are independently selected from: H, D, C₁-C₁₀ alkyl, C₂-C₁₀ alkenyl, C₂-C₁₀ alkynyl, -(CH₂)ₜ₃(C₆-C₁₀ aryl), -(CH₂)ₜ₃(4-10 membered heterocyclyl), and -(CH₂)ₜ₃(C₃-C₁₀ cycloalkyl), and t3 is an integer selected from 1 to 10 (such as 0, 1, 2, 3, 4, 5, 6, 7, 8, 9, and 10); among them H on the C₁-C₁₀ alkyl, C₂-C₁₀ alkenyl, C₂-C₁₀ alkynyl, C₃-C₁₀ cycloalkyl, C₆-C₁₀ aryl, and 4-10 membered heterocyclyl is optionally substituted with groups selected from: D, halogen, cyano, nitro, azido, C₁-C₁₀ alkyl, -N(C₀-C₁₀ alkyl)(C₀-C₁₀ alkyl), -O(C₀-C₁₀ alkyl), -C(O)(C₀-C₁₀ alkyl), -C(O)O(C₀-C₁₀ alkyl), -OC(O)(C₀-C₁₀ alkyl), -C(O)N(C₀-C₁₀ alkyl)(C₀-C₁₀ alkyl), -N(C₀-C₁₀ alkyl)C(O)(C₀-C₁₀ alkyl), -SO₂N(C₀-C₁₀ alkyl)(C₀-C₁₀ alkyl), -N(C₀-C₁₀ alkyl)SO₂(C₀-C₁₀ alkyl), -OCH₂F, -OCHF₂, -OCF₃, C₃-C₁₀ cycloalkyl, C₆-C₁₀ aryl, and 4-10 membered heterocyclyl;
L₀₁ and L₀₂ are linking groups each independently selected from: a single bond and C₁-C₁₀ alkylidene; among them, 0-6 methylene units in the C₁-C₁₀ alkyl are optionally substituted with groups selected from: -Cy-, -O-, -S-, -S-S-, -Si-, -C(O)-, -C(S)-, -C(O)O-, -OC(O)-, -OC(O)O-, -C(O)N(R_{L1})-, -N(R_{L1})C(O)-, -N(R_{L1})C(O)O-, -N(R_{L1})C(O)N(R_{L2})-, -N(R_{L1})-, -S(O)₂-, -S(O)₂N(R_{L1})-, -N(R_{L1})S(O)₂-, -S(O)-, -S(O)N(R_{L1})-, -N(R_{L1})S(O)-, , -S(O)-, -S(O)N(R_{L1})-, -N(R_{L1})S(O)-, -OP(O)(OR_{L1})O-, -P(O)(OR_{L1})O-, -P(O)-, -OP(O)N(R_{L1})-, -P(O)N(R_{L1})-, -P(O)(N(R_{L1}R_{L2}))-, -OP(O)(OR_{L1})₂N(R_{L1})-, -P(O)(OR_{L1})₂N(R_{L2})-, -N(R_{L1})P(O)(OR_{L2})O-, -N(R_{L1})P(O)-, 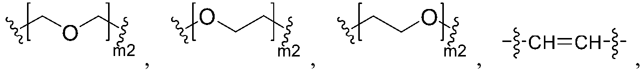 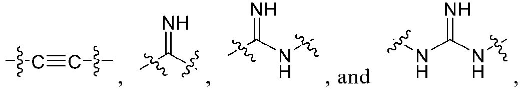 and m2 is an integer selected from 1 to 10 (such as 1, 2, 3, 4, 5, 6, 7, 8, 9, and 10); and H on the C₁-C₁₀ alkyl group is optionally substituted with one or more R_{L3} groups;
each R_{L1} and R_{L2} are independently selected from: H, D, C₁-C₁₀ alkyl, C₂-C₁₀ alkenyl, C₂-C₁₀ alkynyl, -(CH₂)ₜ₄(C₆-C₁₀ aryl), -(CH₂)ₜ₄(4-10 membered heterocyclyl), and -(CH₂)ₜ₄(C₃-C₁₀ cycloalkyl), and t4 is an integer selected from 1 to 10 (such as 0, 1, 2, 3, 4, 5, 6, 7, 8, 9, and 10); among them H on the C₁-C₁₀ alkyl, C₂-C₁₀ alkenyl, C₂-C₁₀ alkynyl, C₃-C₁₀ cycloalkyl, C₆-C₁₀ aryl, and 4-10 membered heterocyclyl is optionally substituted with groups selected from D, halogen, cyano, nitro, azido, C₁-C₁₀ alkyl, -N(C₀-C₁₀ alkyl)(C₀-C₁₀ alkyl), -O(C₀-C₁₀ alkyl), -C(O)(C₀-C₁₀ alkyl), -C(O)O(C₀-C₁₀ alkyl), -OC(O)(C₀-C₁₀ alkyl), -C(O)N(C₀-C₁₀ alkyl)(C₀-C₁₀ alkyl), -N(C₀-C₁₀ alkyl)C(O)(C₀-C₁₀ alkyl), -SO₂N(C₀-C₁₀ alkyl)(C₀-C₁₀ alkyl), -N(C₀-C₁₀ alkyl)SO₂(C₀-C₁₀ alkyl), -OCH₂F, -OCHF₂, -OCF₃, C₃-C₁₀ cycloalkyl, C₆-C₁₀ aryl, or 4-10 membered heterocyclyl;
R_{L3} is selected from: D, halogen, cyano, nitro, azido, C₁-C₁₀ alkyl, C₂-C₁₀ alkenyl, C₂-C₁₀ alkynyl, -(CH₂)ₜ₅(C₆-C₁₀ aryl), -(CH₂)ₜ₅(4-10 membered heterocyclyl), and -(CH₂)ₜ₅(C₃-C₁₀ cycloalkyl), and t5 is an integer selected from 1 to 10 (such as 0, 1, 2, 3, 4, 5, 6, 7, 8, 9, and 10); among them H on the C₁-C₁₀ alkyl, C₂-C₁₀ alkenyl, C₂-C₁₀ alkynyl, C₃-C₁₀ cycloalkyl, C₆-C₁₀ aryl, 4-10 membered heterocyclyl is optionally substituted by groups selected from: D, halogen, cyano, nitro, azido, C₁-C₁₀ alkyl, -N(C₀-C₁₀ alkyl)(C₀-C₁₀ alkyl), -O(C₀-C₁₀ alkyl), -C(O)(C₀-C₁₀ alkyl), -C(O)O(C₀-C₁₀ alkyl), -OC(O)(C₀-C₁₀ alkyl), -C(O)N(C₀-C₁₀ alkyl)(C₀-C₁₀ alkyl), -N(C₀-C₁₀ alkyl)C(O)(C₀-C₁₀ alkyl), -SO₂N(C₀-C₁₀ alkyl)(C₀-C₁₀ alkyl), -N(C₀-C₁₀ alkyl)SO₂(C₀-C₁₀ alkyl), -OCH₂F, -OCHF₂, -OCF₃, C₃-C₁₀ cycloalkyl, C₆-C₁₀ aryl, or 4-10 membered heterocyclyl; and
each -Cy- is independently an optionally substituted divalent ring selected from arylene, cycloalkylene, or heterocyclylene.

Specifically, each -Cy- is independently an optionally substituted divalent ring selected from: phenylene, bicyclic arylene, tricyclic arylene, monocyclic cycloalkylene, bicyclic cycloalkylene, tricyclic cycloalkylene, monocyclic heteroarylene, bicyclic heteroarylene, tricyclic heteroarylene, monocyclic heterocycloalkylene, bicyclic heterocycloalkylene, and tricyclic heterocycloalkylene.

In some embodiments of the present disclosure, each -Cy- is independently an optionally substituted divalent ring selected from: monocyclic cycloalkylene, bicyclic cycloalkylene, monocyclic saturated heterocycloalkylene, and bicyclic saturated heterocycloalkylene.

Specifically, each -Cy- is optionally substituted by groups selected from: halogen, cyano, nitro, azido, C₁-C₁₀ alkyl, -N(C₀-C₁₀ alkyl)(C₀-C₁₀ alkyl), -O(C₀-C₁₀ alkyl), -CON(C₀-C₁₀ alkyl)(C₀-C₁₀ alkyl), -N(C₀-C₁₀ alkyl)CO(C₀-C₁₀ alkyl), -SO₂N(C₀-C₁₀ alkyl)(C₀-C₁₀ alkyl), -N(C₀-C₁₀ alkyl)SO₂(C₀-C₁₀ alkyl), -OCH₂F, -OCHF₂, -OCF₃, C₃-C₁₀ cycloalkyl, C₆-C₁₀ aryl, or 4-10 membered heterocyclyl.

In some embodiments of the present disclosure, each -Cy- is independently selected from the following:

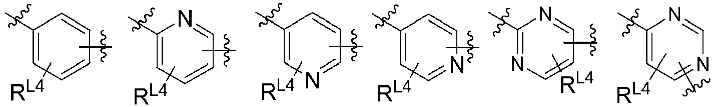

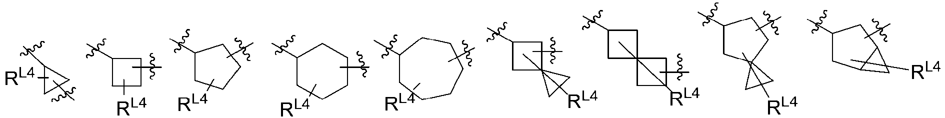

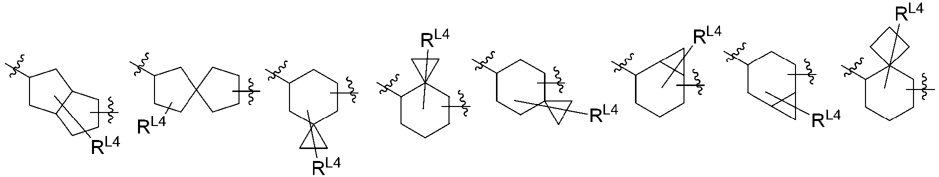

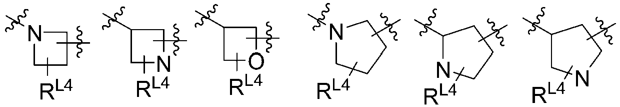

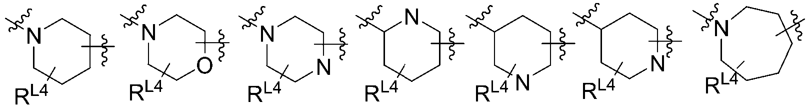

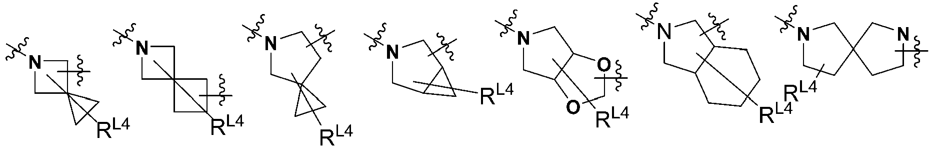

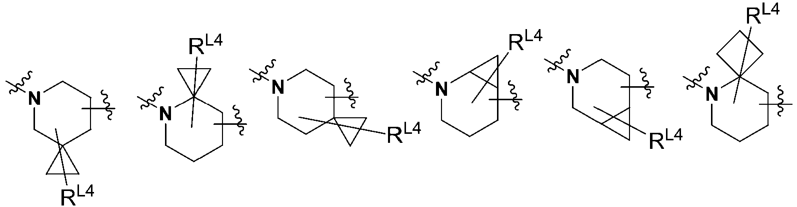

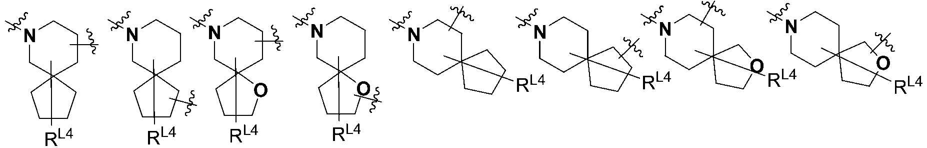

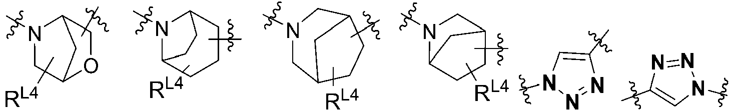

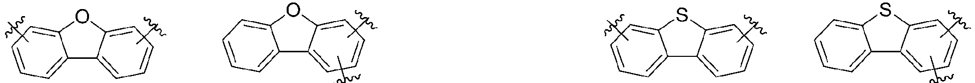

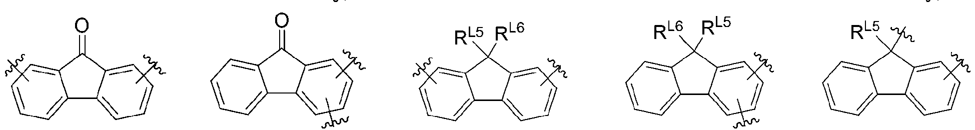

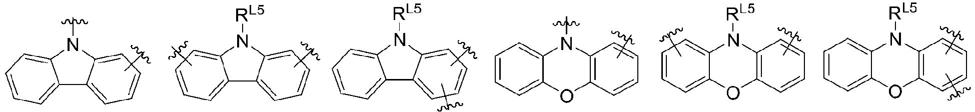

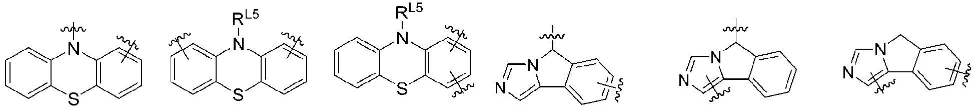

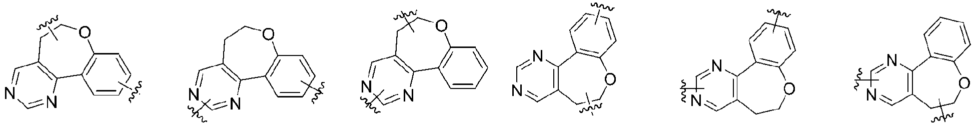

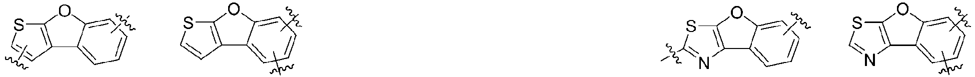

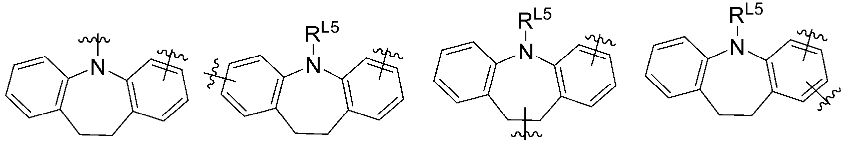

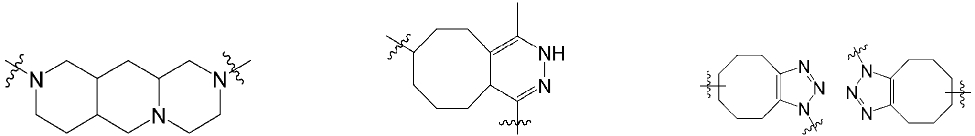

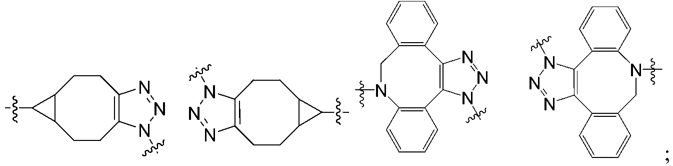

Specifically, R^{L4} is selected from a group consisting of H, D, halogen, cyano, nitro, azido, C₁-C₁₀ alkyl, -N(C₀-C₁₀ alkyl)(C₀-C₁₀ alkyl), -O(C₀-C₁₀ alkyl), -C(O)(C₀-C₁₀ alkyl), -C(O)O(C₀-C₁₀ alkyl), -OC(O)(C₀-C₁₀ alkyl), -C(O)N(C₀-C₁₀ alkyl)(C₀-C₁₀ alkyl), -N(C₀-C₁₀ alkyl)C(O)(C₀-C₁₀ alkyl), -SO₂N(C₀-C₁₀ alkyl)(C₀-C₁₀ alkyl), -N(C₀-C₁₀ alkyl)SO₂(C₀-C₁₀ alkyl), -OCH₂F, -OCHF₂, -OCF₃, C₃-C₁₀ cycloalkyl, C₆-C₁₀ aryl, and 4-10 membered heterocyclyl; and
R^{L5} and R^{L6} are independently selected from a group consisting of H, D, C₁-C₁₀ alkyl; or, R^{L5} and R^{L6}, together with the atom to which they are attached, form a cycloalkylene or a heterocyclylene.

In some embodiments of the present disclosure, Q₁-ring is a monocyclic heterocycle including a lactam structure; For example, Q1-ring may be

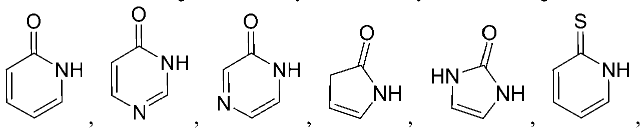

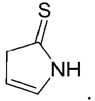

In some embodiments of the present disclosure, Q₁-ring is a bicyclic heterocycle including a lactam structure; in some preferred embodiments of the present disclosure, Q₁-ring is 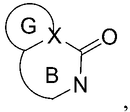 among which X is CH or N, B-ring is 5-7 membered heterocyclic ring, and G-ring is 4-8 membered heterocyclic ring, benzene ring or 4-8 membered heterocyclic ring; for example, Q₁-ring may be

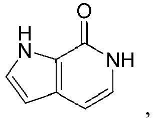

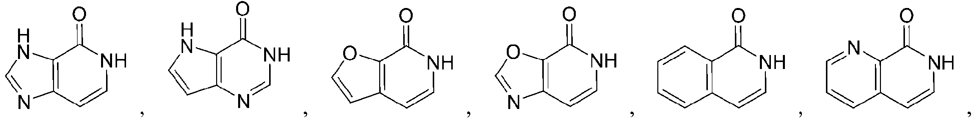

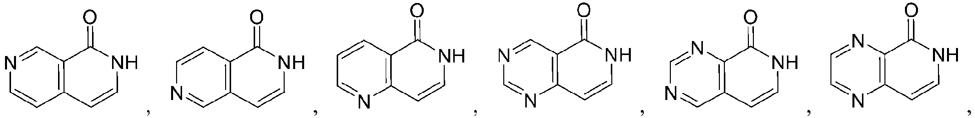

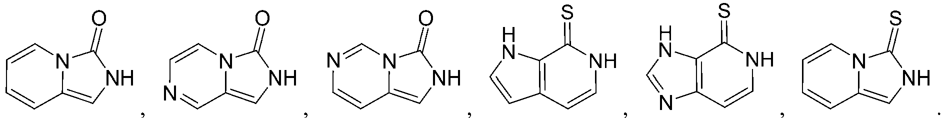

In some embodiments of the present disclosure, 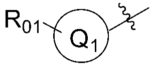 moiety is 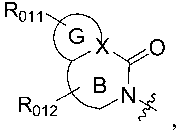 among which R₀₁₁ and R₀₁₂ have the same definition as R₀₁.

In some embodiments of the present disclosure, 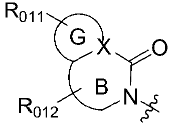 is 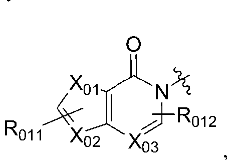 among which X₀₁ is selected from: CH₂, NH, O, C(O), 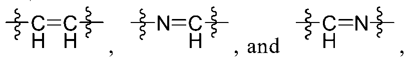 and X₀₂ and X₀₃ are independently selected from CH and N.

In some embodiments of the present disclosure, X₀₁ is NH.

In some embodiments of the present disclosure, X₀₁ is O.

In some embodiments of the present disclosure, X₀₂ is CH.

In some embodiments of the present disclosure, X₀₃ is CH.

In some embodiments of the present disclosure, R₀₁₁ includes the following group: 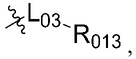 among which L₀₃ is selected from: a single bond, -O-, -S-, -C(O)-, -C(R_{L1}R_{L2})-, and -OC(R_{L1}R_{L2})-, R₀₁₃ is -NR₀₁₄R₀₁₅ or substituted or unsubstituted nitrogen-containing heterocyclyl (especially saturated nitrogen-containing heterocyclyl).

Specifically, R_{L1} and R_{L2} are independently selected from: H, D, halogen, C₁-C₆ alkyl, and C₁-C₆ haloalkyl.

In some embodiments of the present disclosure, L₀₃ is -C(R_{L1}R_{L2})-, especially -CH₂- or -CD₂-.

Specifically, R₀₁₄ and R₀₁₅ are independently selected from: H, D, and C₁-C₁₀ alkyl; among them, 0-6 methylene units in the C₁-C₁₀ alkyl group are optionally substituted with groups selected from: -O-, -S-, -S-S-, -Si-, -C(O)-, -C(S)-, -C(O)O-, -OC(O)-, -OC(O)O-, -C(O)N(C₀-C₁₀ alkyl)-, -N(C₀-C₁₀ alkyl)C(O)-, -N(C₀-C₁₀ alkyl)C(O)O-, -N(C₀-C₁₀ alkyl)C(O)N(C₀-C₁₀ alkyl)-, -N(C₀-C₁₀ alkyl)-, -S(O)₂-, -S(O)₂N(C₀-C₁₀ alkyl)-, -N(C₀-C₁₀ alkyl)S(O)₂-, C₃-C₁₀ cycloalkylene (such as 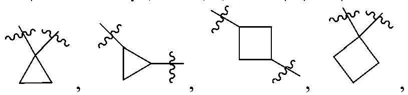 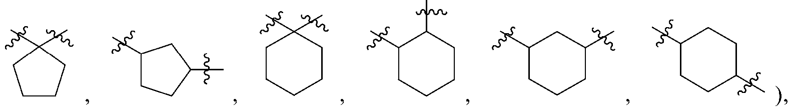 C₆-C₁₀ arylene (e.g., phenylene), and 4-10 membered heterocyclylene; and H on the C₁-C₁₀ alkyl is optionally substituted with groups selected from: D, halogen, cyano, nitro, azido, -N(C₀-C₁₀ alkyl)(C₀-C₁₀ alkyl), -O(C₀-C₁₀ alkyl), -C(O)(C₀-C₁₀ alkyl), -C(O)O(C₀-C₁₀ alkyl), -OC(O)(C₀-C₁₀ alkyl), -C(O)N(C₀-C₁₀ alkyl)(C₀-C₁₀ alkyl), -N(C₀-C₁₀ alkyl)C(O)(C₀-C₁₀ alkyl), -SO₂N(C₀-C₁₀ alkyl)(C₀-C₁₀ alkyl), -N(C₀-C₁₀ alkyl)SO₂(C₀-C₁₀ alkyl), -OCH₂F, -OCHF₂, -OCF₃, C₃-C₁₀ cycloalkyl, C₆-C₁₀ aryl, or 4-10 membered heterocyclyl; H on the C₃-C₁₀ cycloalkylene, C₆-C₁₀ arylene, 4-10 membered heterocyclylene is optionally substituted with groups selected from: D, halogen, cyano, nitro, azido, -N(C₀-C₁₀ alkyl)(C₀-C₁₀ alkyl), -O(C₀-C₁₀ alkyl), -C(O)(C₀-C₁₀ alkyl), -C(O)O(C₀-C₁₀ alkyl), -OC(O)(C₀-C₁₀ alkyl), -C(O)N(C₀-C₁₀ alkyl)(C₀-C₁₀ alkyl), -N(C₀-C₁₀ alkyl)C(O)(C₀-C₁₀ alkyl), -SO₂N(C₀-C₁₀ alkyl)(C₀-C₁₀ alkyl), -N(C₀-C₁₀ alkyl)SO₂(C₀-C₁₀ alkyl), -OCH₂F, -OCHF₂, -OCF₃, C₃-C₁₀ cycloalkyl, C₆-C₁₀ aryl, or 4-10 membered heterocyclyl.

Specifically, H on the nitrogen-containing heterocyclyl is optionally substituted with groups selected from: D, halogen, cyano, nitro, azido, C₁-C₁₀ alkyl, -N(C₀-C₁₀ alkyl)(C₀-C₁₀ alkyl), -O(C₀-C₁₀ alkyl), -C(O)(C₀-C₁₀ alkyl), -C(O)O(C₀-C₁₀ alkyl), -OC(O)(C₀-C₁₀ alkyl), -C(O)N(C₀-C₁₀ alkyl)(C₀-C₁₀ alkyl), -N(C₀-C₁₀ alkyl)C(O)(C₀-C₁₀ alkyl), -SO₂N(C₀-C₁₀ alkyl)(C₀-C₁₀ alkyl), -N(C₀-C₁₀ alkyl)SO₂(C₀-C₁₀ alkyl), -OCH₂F, -OCHF₂, -OCF₃, C₃-C₁₀ cycloalkyl, C₆-C₁₀ aryl, or 4-10 membered heterocyclyl; H on the C₁-C₁₀ alkyl, C₃-C₁₀ cycloalkyl, C₆-C₁₀ aryl, 4-10 membered heterocyclyl is optionally substituted with groups selected from: D, halogen, cyano, nitro, azido, -N(C₀-C₁₀ alkyl)(C₀-C₁₀ alkyl), -O(C₀-C₁₀ alkyl), -C(O)(C₀-C₁₀ alkyl), -C(O)O(C₀-C₁₀ alkyl), -OC(O)(C₀-C₁₀ alkyl), -C(O)N(C₀-C₁₀ alkyl)(C₀-C₁₀ alkyl), -N(C₀-C₁₀ alkyl)C(O)(C₀-C₁₀ alkyl), -SO₂N(C₀-C₁₀ alkyl)(C₀-C₁₀ alkyl), -N(C₀-C₁₀ alkyl)SO₂(C₀-C₁₀ alkyl), -OCH₂F, -OCHF₂, -OCF₃, C₃-C₁₀ cycloalkyl, C₆-C₁₀ aryl, or 4-10 membered heterocyclyl.

Specifically, R₀₁₂ is selected from: H, D, halogen, cyano, nitro, azido, C₁-C₁₀ alkyl, -N(C₀-C₁₀ alkyl)(C₀-C₁₀ alkyl), -O(C₀-C₁₀ alkyl), -C(O)(C₀-C₁₀ alkyl), -C(O)O(C₀-C₁₀ alkyl), -OC(O)(C₀-C₁₀ alkyl), -C(O)N(C₀-C₁₀ alkyl)(C₀-C₁₀ alkyl), -N(C₀-C₁₀ alkyl)C(O)(C₀-C₁₀ alkyl), -SO₂N(C₀-C₁₀ alkyl)(C₀-C₁₀ alkyl), -N(C₀-C₁₀ alkyl)SO₂(C₀-C₁₀ alkyl), -OCH₂F, -OCHF₂, -OCF₃, C₃-C₁₀ cycloalkyl, C₆-C₁₀ aryl, and 4-10 membered heterocyclyl; among them H on the C₁-C₁₀ alkyl, C₃-C₁₀ cycloalkyl, C₆-C₁₀ aryl, 4-10 membered heterocyclyl is optionally substituted with groups selected from:D, halogen, cyano, nitro, azido, -N(C₀-C₁₀ alkyl)(C₀-C₁₀ alkyl), -O(C₀-C₁₀ alkyl), -C(O)(C₀-C₁₀ alkyl), -C(O)O(C₀-C₁₀ alkyl), -OC(O)(C₀-C₁₀ alkyl), -C(O)N(C₀-C₁₀ alkyl)(C₀-C₁₀ alkyl), -N(C₀-C₁₀ alkyl)C(O)(C₀-C₁₀ alkyl), -SO₂N(C₀-C₁₀ alkyl)(C₀-C₁₀ alkyl), -N(C₀-C₁₀ alkyl)SO₂(C₀-C₁₀ alkyl), -OCH₂F, -OCHF₂, -OCF₃, C₃-C₁₀ cycloalkyl, C₆-C₁₀ aryl, or 4-10 membered heterocyclyl.

Specifically, in the definition of R₀₁₃, the nitrogen-containing heterocycle is a saturated 4-10 membered nitrogen-containing heterocycle, such as

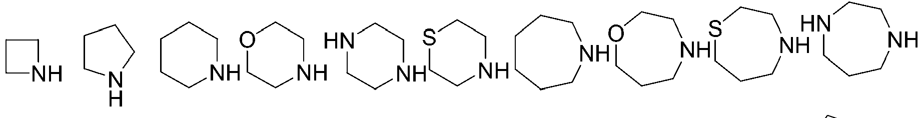

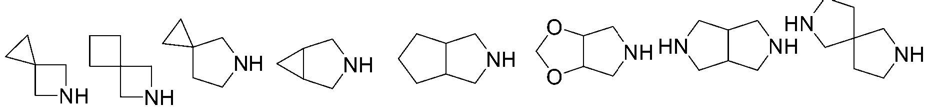

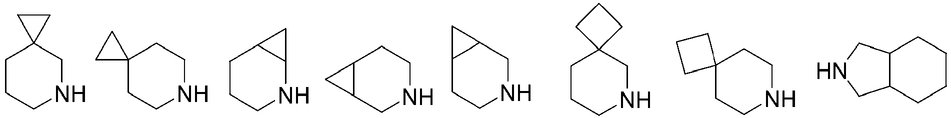

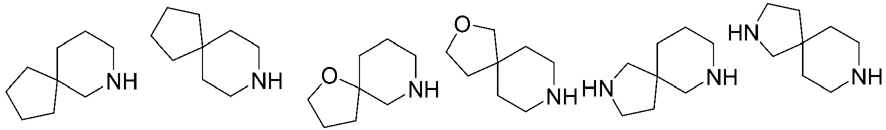

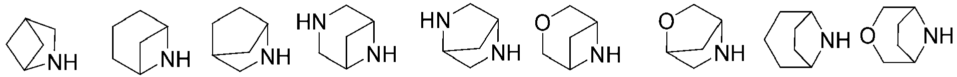

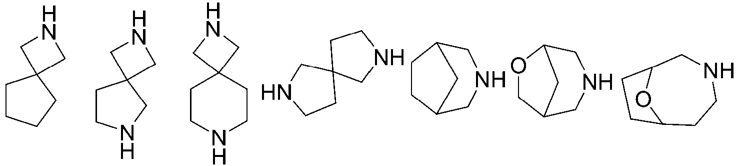

In some embodiments of the present disclosure, R₀₁₃ is selected from:

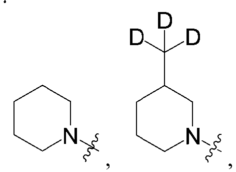

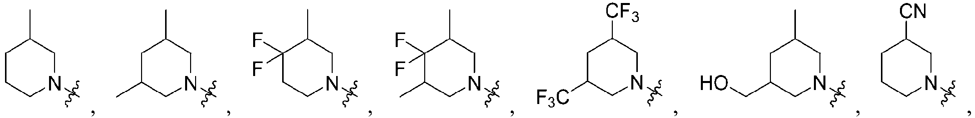

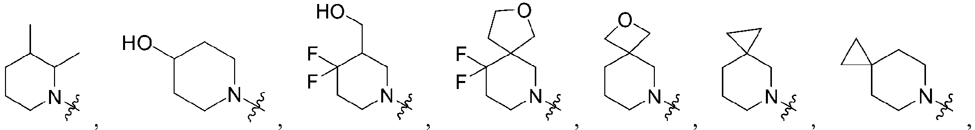

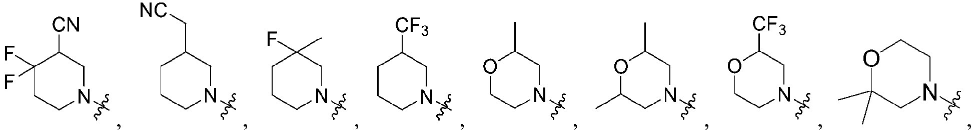

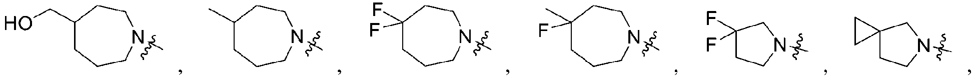

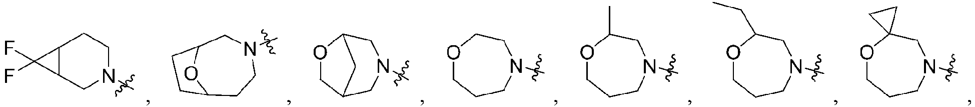

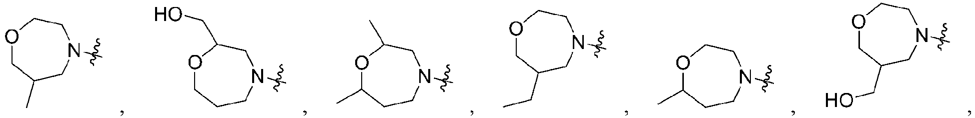

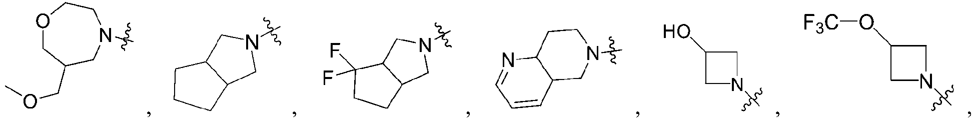

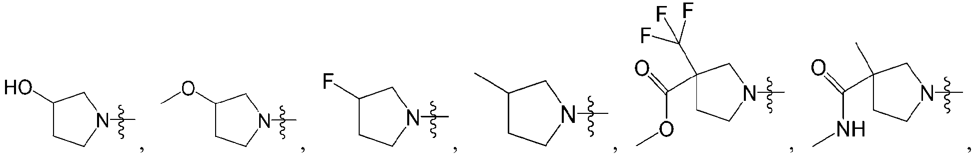

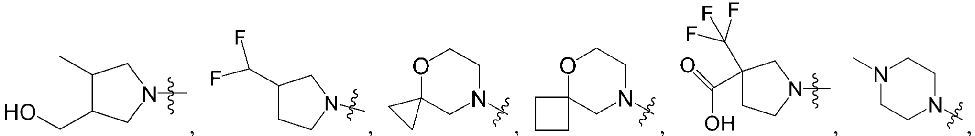

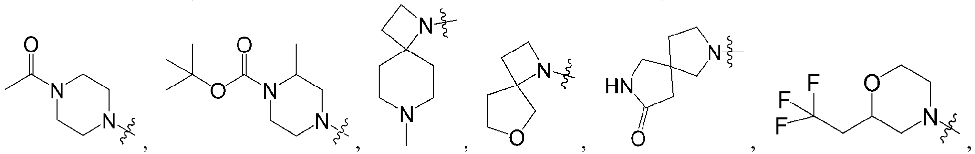

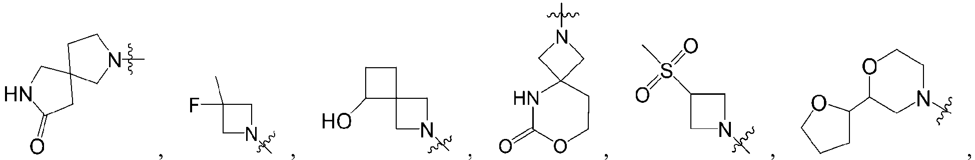

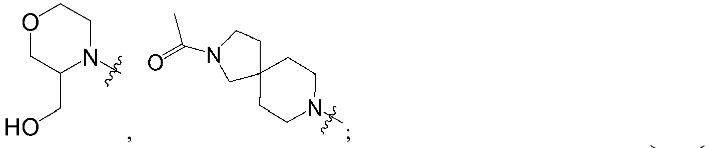

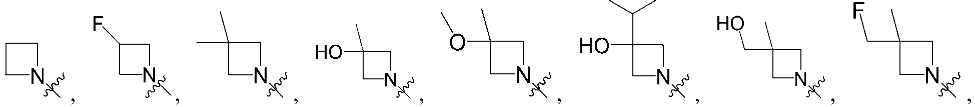

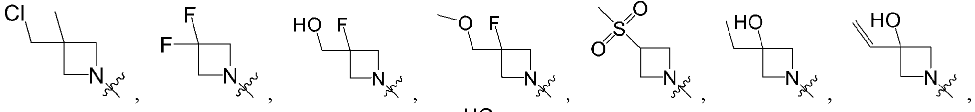

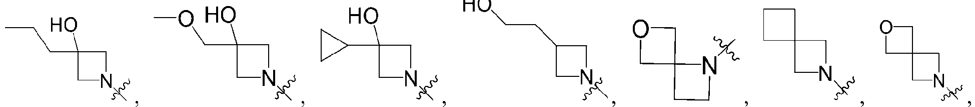

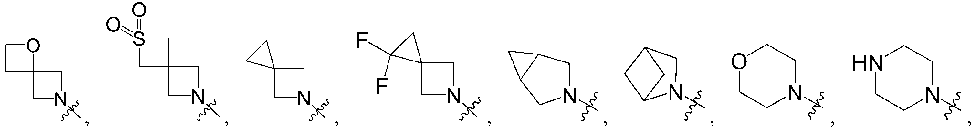

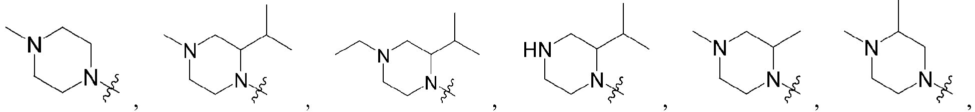

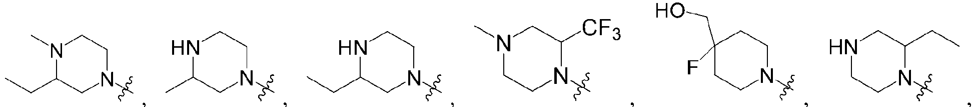

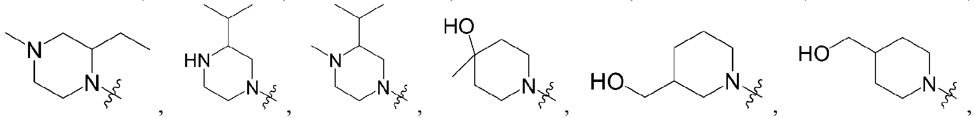

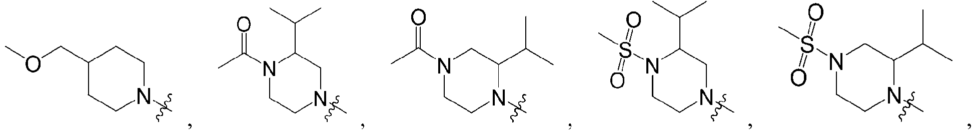

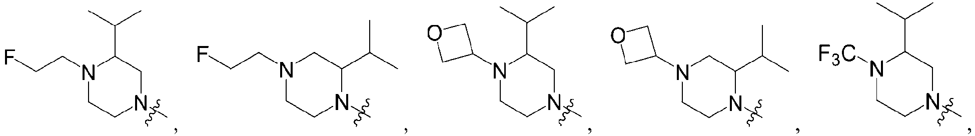

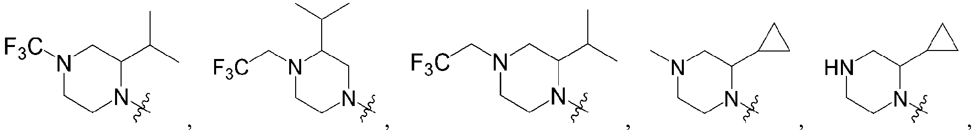

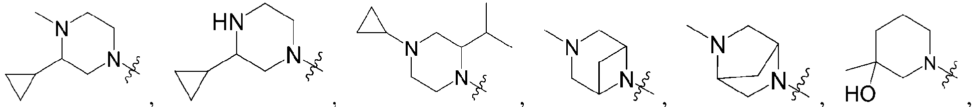

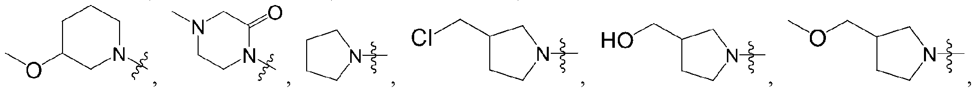

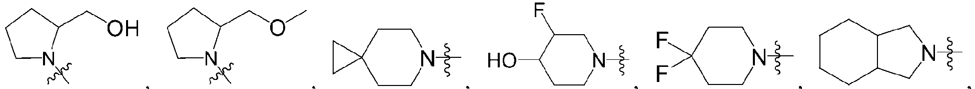

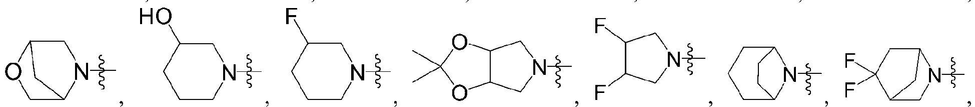

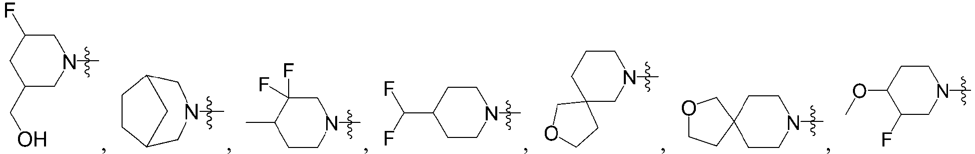

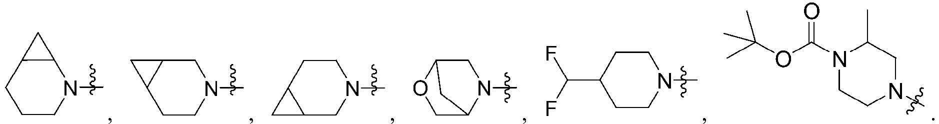

In some embodiments of the present disclosure, R₀₁₁ is selected from:

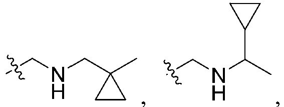

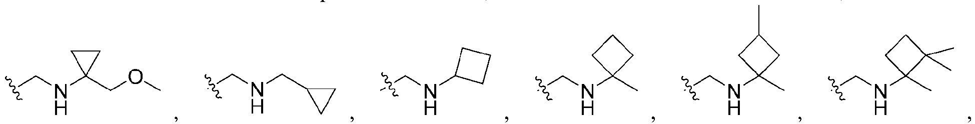

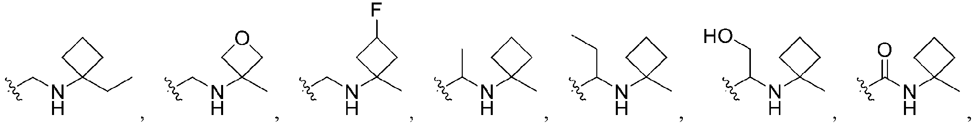

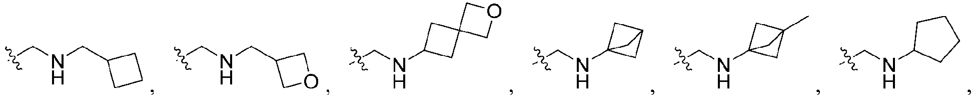

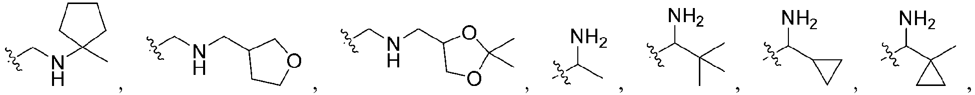

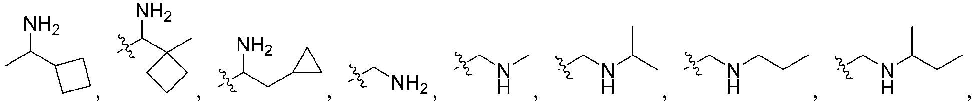

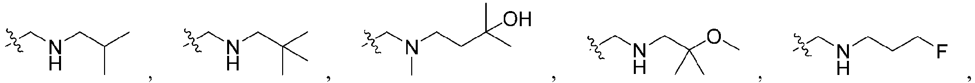

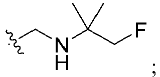

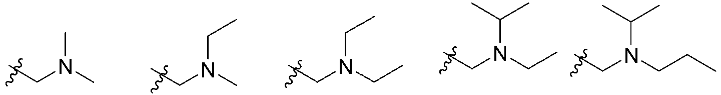

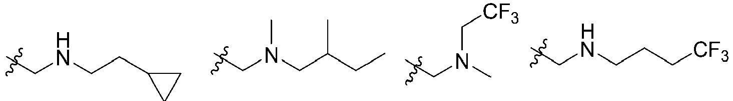

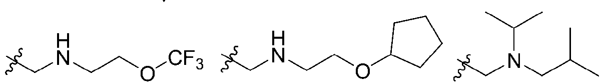

In some embodiments of the present disclosure, 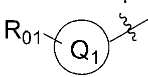 moiety has the following structure 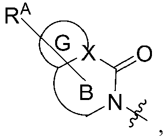 as described below.

In some embodiments of the present disclosure, Q₂-ring is an aromatic ring or a heterocyclic aromatic ring; in some preferred embodiments of the present disclosure, Q₂-ring is a benzene ring or a bioisostere thereof related to the benzene ring (such as

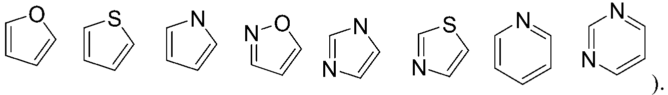

In some embodiments of the present disclosure, 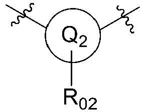 moiety is selected from the following structure:

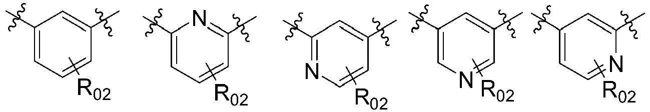

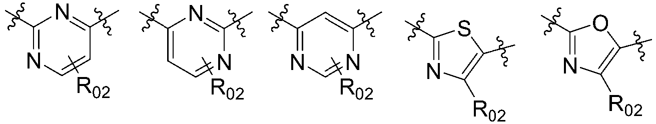

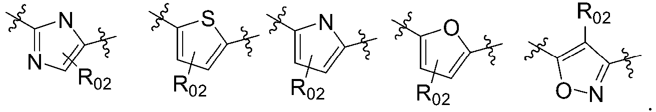

Specifically, R₀₂ is selected from: H, D, halogen, cyano, nitro, azido, C₁-C₁₀ alkyl, -N(C₀-C₁₀ alkyl)(C₀-C₁₀ alkyl), -O(C₀-C₁₀ alkyl), -C(O)(C₀-C₁₀ alkyl), -C(O)O(C₀-C₁₀ alkyl), -OC(O)(C₀-C₁₀ alkyl), -C(O)N(C₀-C₁₀ alkyl)(C₀-C₁₀ alkyl), -N(C₀-C₁₀ alkyl)C(O)(C₀-C₁₀ alkyl), -SO₂N(C₀-C₁₀ alkyl)(C₀-C₁₀ alkyl), -N(C₀-C₁₀ alkyl)SO₂(C₀-C₁₀ alkyl), -OCH₂F, -OCHF₂, -OCF₃, C₃-C₁₀ cycloalkyl, C₆-C₁₀ aryl, and 4-10 membered heterocyclyl; among them H on the C₁-C₁₀ alkyl, C₃-C₁₀ cycloalkyl, C₆-C₁₀ aryl, and 4-10 membered heterocyclyl is optionally substituted with groups selected from: D, halogen, cyano, nitro, azido, -N(C₀-C₁₀ alkyl)(C₀-C₁₀ alkyl), -O(C₀-C₁₀ alkyl), -C(O)(C₀-C₁₀ alkyl), -C(O)O(C₀-C₁₀ alkyl), -OC(O)(C₀-C₁₀ alkyl), -C(O)N(C₀-C₁₀ alkyl)(C₀-C₁₀ alkyl), -N(C₀-C₁₀ alkyl)C(O)(C₀-C₁₀ alkyl), -SO₂N(C₀-C₁₀ alkyl)(C₀-C₁₀ alkyl), -N(C₀-C₁₀ alkyl)SO₂(C₀-C₁₀ alkyl), -OCH₂F, -OCHF₂, -OCF₃, C₃-C₁₀ cycloalkyl, C₆-C₁₀ aryl, or 4-10 membered heterocyclyl.

More specifically, R₀₂ is selected from: H, D, halogen, cyano, C₁-C₆ alkyl, and C₁-C₆ haloalkyl.

In some embodiments of the present disclosure, 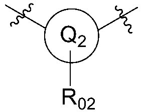 moiety has the following structure: 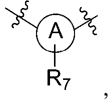 as described below.

In some embodiments of the present disclosure, Q₃-ring is an aromatic ring or a heterocyclic aromatic ring; in some preferred embodiments of the present disclosure, Q₃-ring is a 5 membered heteroaromatic ring, such as 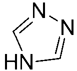 or a bioisostere thereof.

In some embodiments of the present disclosure, 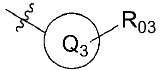 moiety is 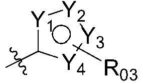 and Y₁, Y₂, Y₃ and Y₄ are independently selected from: C, N, O, and S, and any two of Y₁,Y₂, Y₃ and Y₄ are two O atoms, two S atoms, or an O atom and an S atom are not directly bonded.

Specifically, R₀₃ is selected from: H, D, C₁-C₆ alkyl, C₁-C₆ haloalkyl, and C₃-C₆ cycloalkyl.

More specifically, 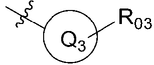 moiety has the following structure:

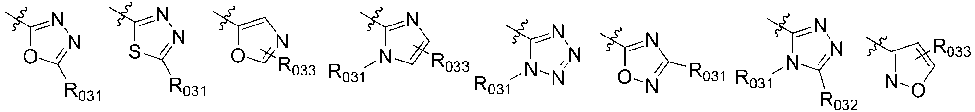

Among them, R₀₃₃ is one or more independent substituents on the ring and R₀₃₁ to R₀₃₂ are each defined as described above for R₀₃.

More specifically, R₀₃₁, R₀₃₂ and R₀₃₃ may be independently selected from: H, D, C₁-C₆ alkyl, C₁-C₆ haloalkyl, and C₃-C₆ cycloalkyl; further specifically, R₀₃₁, R₀₃₂, and R₀₃₃ may be independently selected from: H, D, -CH₃, -CHF₂, -CH₂F, -CF₃,

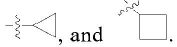

In some embodiments of the present disclosure, 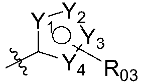 moiety is 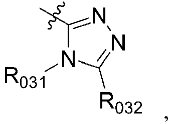 especially

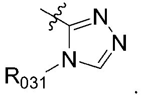

More specifically, R₀₃₁ may be selected from: -CH₃, -CHF₂, -CH₂F, -CF₃, -CH₃. 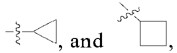 especially

In some embodiments of the present disclosure, 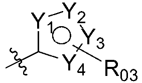 moiety is 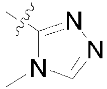 or

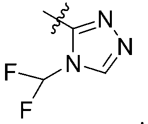

In some embodiments of the present disclosure, 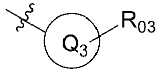 moiety has the following structure: 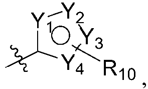 as described below.

In some preferred embodiments of the present disclosure, L₀₁ is a single bond.

In some embodiments of the present disclosure, L₀₁ is selected from: -C(O)O-, -OC(O)-, -C(O)N(R_{L1})-, -N(R_{L1})C(O)-, -N(R_{L1})-, -S(O)₂-, -S(O)₂N(R_{L1})-, and -N(R_{L1})S(O)₂-; among them R_{L1} may be selected from: H, D, and C₁-C₆ alkyl, especially H. In an embodiment of the present disclosure, L₀₁ is -NHC(O)- or -C(O)NH-.

In some embodiments of the present disclosure, the compound has the following structure:

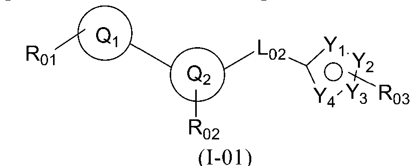

Specifically, Y₁, Y₂, Y₃ and Y₄ are independently selected from: C, N, O, and S , and any two of Y₁,Y₂, Y₃ and Y₄ are two O atoms, two S atoms, or an O atom and an S atom are not directly bonded.

In some embodiments of the present disclosure, L₀₂ includes cycloalkylene or heterocycloalkylene, such as 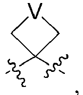 among which V is selected from: a single bond, O, S, NH, 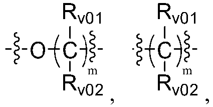 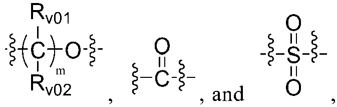 m is 1, 2, or 3, and Rᵥ₀₁ and Rᵥ₀₂ are independently selected from: H, D, halogen, cyano, nitro, C₁-C₁₀ alkyl, C₂-C₁₀ alkenyl, C₂-C₁₀ alkynyl, -(C₀-C₆ alkylidene)-(C₃-C₁₀ cycloalkyl), -(C₀-C₆ alkylidene)-(C₆-C₁₀ aryl), -(C₀-C₆ alkylidene)-(4-10 membered heterocyclyl), -O(C₀-C₁₀ alkyl), -S(C₀-C₁₀ alkyl), -C(O)(C₀-C₁₀ alkyl), -C(O)N(C₀-C₁₀ alkyl)(C₀-C₁₀ alkyl), -N(C₀-C₁₀ alkyl)(C₀-C₁₀ alkyl), -C(O)O(C₀-C₁₀ alkyl), -S(O)₂(C₀-C₁₀ alkyl), -S(O)₂-(C₀-C₆ alkylidene)-(C₃-C₁₀ cycloalkyl), -S(O)₂-(C₀-C₆ alkylidene)-(4-10 membered heterocyclyl), -S(O)₂N(C₀-C₁₀ alkyl)(C₀-C₁₀ alkyl), -S(O)₂N(C₀-C₁₀ alkyl)((C₀-C₆ alkylidene)-(C₃-C₁₀ cycloalkyl)), -S(O)₂N(C₀-C₁₀ alkyl)((C₀-C₆ alkylidene)-(4-10 membered heterocyclyl)), C₁-C₁₀ haloalkyl, C₁-C₁₀ cyanoalkyl, C₁-C₁₀ hydroxyalkyl, C₁-C₁₀ aminoalkyl, C₁-C₁₀ carboxyalkyl, C₁-C₁₀ alkoxyalkyl, and C₁-C₁₀ alkylaminoalkyl, or , Rᵥ₀₁ and Rᵥ₀₂, together with the carbon atom to which they are attached, form a cycloalkyl or a heterocyclyl, among which the cycloalkyl or the heterocyclyl is optionally substituted with one or more independent substituents selected from: oxo(=O), (=alkylidene), halogen, cyano, nitro, C₁-C₁₀ alkyl, C₂-C₁₀ alkenyl, C₂-C₁₀ alkynyl, -(C₀-C₆ alkylidene)-(C₃-C₁₀ cycloalkyl), -(C₀-C₆ alkylidene)-(C₆-C₁₀ aryl), -(C₀-C₆ alkylidene)-(4-10 membered heterocyclyl), -O(C₀-C₁₀ alkyl), -S(C₀-C₁₀ alkyl), -C(O)(C₀-C₁₀ alkyl), -C(O)N(C₀-C₁₀ alkyl)(C₀-C₁₀ alkyl), -N(C₀-C₁₀ alkyl)(C₀-C₁₀ alkyl), -C(O)O(C₀-C₁₀ alkyl), -S(O)₂(C₀-C₁₀ alkyl), -S(O)₂-(C₀-C₆ alkylidene)-(C₃-C₁₀ cycloalkyl), -S(O)₂-(C₀-C₆ alkylidene)-(4-10 membered heterocyclyl), -S(O)₂N(C₀-C₁₀ alkyl)(C₀-C₁₀ alkyl), -S(O)₂N(C₀-C₁₀ alkyl)((C₀-C₆ alkylidene)-(C₃-C₁₀ cycloalkyl)), -S(O)₂N(C₀-C₁₀ alkyl)((C₀-C₆ alkylidene)-(4-10 membered heterocyclyl)), C₁-C₁₀ haloalkyl, C₁-C₁₀ cyanoalkyl, C₁-C₁₀ hydroxyalkyl, C₁-C₁₀ aminoalkyl, C₁-C₁₀ carboxyalkyl, C₁-C₁₀ alkoxyalkyl, and C₁-C₁₀ alkylaminoalkyl;

Specifically, H on the alkylidene, alkyl, alkenyl, alkynyl, cycloalkyl, aryl, and heterocyclyl is optionally substituted by a substituent.

In some embodiments of the present disclosure, L₀₂ has the following structure: 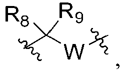 as described below.

In some embodiments of the present disclosure, the compound has the following structure:

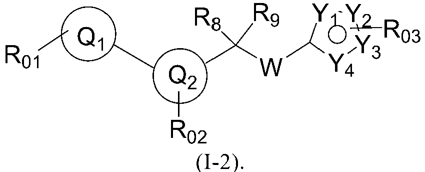

In some embodiments of the present disclosure, the compound has the following structure:

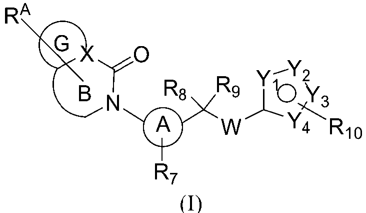

Specifically,
A-ring is an aromatic ring or a heterocyclic aromatic ring;
B-ring is 5-7 membered heterocyclic ring;
G-ring is 5-7 membered heterocyclic ring;
X is CH or N;
R^{A} is one or more independent substituents on the ring 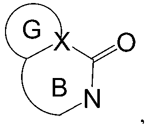 each independently selected from: H, D, 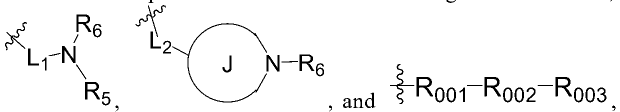 among which L₁ is selected from a single bond, C(O), and C(R₃R₄), L₂ is selected from: a single bond, O, S, C(R₃R₄), N(R₅), and 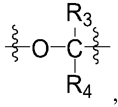 R₀₀₁ is selected from: a single bond and C₁-C₁₀ alkylidene; R₀₀₂ is selected from: a single bond, C₁-C₁₀ alkylidene, O, S, N(R₂), S(O)₂, S(O)₂N(R₂), S(O), S(O)N(R₂), C(O), C(O)O, C(O)N(R₂), OC(O), OC(O)N(R₂), N(R₂)C(O)O, N(R₂)C(O), and N(R₂)S(O)₂, and R₀₀₃ is selected from: H, D, halogen, cyano, nitro, C₁-C₁₀ alkyl, C₁-C₁₀ haloalkyl, C₂-C₁₀ alkenyl, C₂-C₁₀ alkynyl, -(C₀-C₆ alkylidene)-(C₃-C₁₀ cycloalkyl), -(C₀-C₆ alkylidene)-(C₆-C₁₀ aryl), and -(C₀-C₆ alkylidene)-(4-10 membered heterocyclyl); among them H on the alkylene, alkyl, alkenyl, alkynyl, cycloalkyl, aryl, and heterocyclyl is each optionally substituted with one or more of independent R groups;
R₂ is selected from: H, D, C₁-C₁₀ alkyl, and -(C₀-C₆ alkylidene)-(C₃-C₁₀ cycloalkyl);
R₃ and R₄ are independently selected from: H, D, halogen, cyano, nitro, C₁-C₁₀ alkyl, C₁-C₁₀ haloalkyl, C₂-C₁₀ alkenyl, C₂-C₁₀ alkynyl, -(C₀-C₆ alkylidene)-(C₃-C₁₀ cycloalkyl), -(C₀-C₆ alkylidene)-(C₆-C₁₀ aryl), and -(C₀-C₆ alkylidene)-(4-10 membered heterocyclyl); among them H on the alkylidene, alkyl, alkenyl, alkynyl, cycloalkyl, aryl, and heterocyclyl is optionally substituted with one or more of independent R groups;
or, R₃ and R₄, together with the carbon atom to which they are attached, form a cycloalkyl or a heterocyclyl, among which the cycloalkyl or the heterocyclyl is optionally substituted with one or more of independent R groups;
J-ring is 3-10 membered nitrogen-containing heterocyclic ring (J-ring is bonded to L₂ via a carbon atom), and the J-ring is optionally substituted by groups selected from: oxo(=O), halogen, cyano, nitro, C₁-C₁₀ alkyl, C₂-C₁₀ alkenyl, C₂-C₁₀ alkynyl, -(C₀-C₆ alkylidene)-(C₃-C₁₀ cycloalkyl), -(C₀-C₆ alkylidene)-(C₆-C₁₀ aryl), -(C₀-C₆ alkylidene)-(4-10 membered heterocyclyl), -O(C₀-C₁₀ alkyl), -S(C₀-C₁₀ alkyl), -C(O)(C₀-C₁₀ alkyl), -C(O)N(C₀-C₁₀ alkyl)(C₀-C₁₀ alkyl), -N(C₀-C₁₀ alkyl)(C₀-C₁₀ alkyl), -C(O)O(C₀-C₁₀ alkyl), -S(O)₂(C₀-C₁₀ alkyl), -S(O)₂-(C₀-C₆ alkylidene)-(C₃-C₁₀ cycloalkyl), -S(O)₂-(C₀-C₆ alkylidene)-(4-10 membered heterocyclyl), -S(O)₂N(C₀-C₁₀ alkyl)(C₀-C₁₀ alkyl), -S(O)₂N(C₀-C₁₀ alkyl)((C₀-C₆ alkylidene)-(C₃-C₁₀ cycloalkyl)), -S(O)₂N(C₀-C₁₀ alkyl)((C₀-C₆ alkylidene)-(4-10 membered heterocyclyl)), C₁-C₁₀ haloalkyl, C₁-C₁₀ cyanoalkyl, C₁-C₁₀ hydroxyalkyl, C₁-C₁₀ aminoalkyl, C₁-C₁₀ carboxyalkyl, C₁-C₁₀ alkoxyalkyl, or C₁-C₁₀ alkylaminoalkyl; among them H on the alkylene, alkyl, alkenyl, alkynyl, cycloalkyl, aryl, and heterocyclyl is optionally substituted with one or more of independent R groups;
R₅ and R₆ are independently selected from: H, D, C₁₋₁₀ alkyl, -(C₀-C₆ alkylidene)-(C₃-C₁₀ cycloalkyl), -(C₀-C₆ alkylidene)-(C₆-C₁₀ aryl), and -(C₀-C₆ alkylidene)-(4-10 membered heterocyclyl); among them the alkylidene, alkyl, cycloalkyl, aryl, and heterocyclyl is each optionally substituted with one or more independent substituents selected from: halogen, cyano, nitro, C₁-C₁₀ alkyl, C₂-C₁₀ alkenyl, C₂-C₁₀ alkynyl, -(C₀-C₆ alkylidene)-(C₃-C₁₀ cycloalkyl), -(C₀-C₆ alkylidene)-(C₆-C₁₀ aryl), -(C₀-C₆ alkylidene)-(4-10 membered heterocyclyl), -O(C₀-C₁₀ alkyl), -S(C₀-C₁₀ alkyl), -C(O)(C₀-C₁₀ alkyl), -C(O)N(C₀-C₁₀ alkyl)(C₀-C₁₀ alkyl), -N(C₀-C₁₀ alkyl)(C₀-C₁₀ alkyl), -C(O)O(C₀-C₁₀ alkyl), -S(O)₂(C₀-C₁₀ alkyl), -S(O)₂-(C₀-C₆ alkylidene)-(C₃-C₁₀ cycloalkyl), -S(O)₂-(C₀-C₆ alkylidene)-(4-10 membered heterocyclyl), -S(O)₂N(C₀-C₁₀ alkyl)(C₀-C₁₀ alkyl), -S(O)₂N(C₀-C₁₀ alkyl)((C₀-C₆ alkylidene)-(C₃-C₁₀ cycloalkyl)), -S(O)₂N(C₀-C₁₀ alkyl)((C₀-C₆ alkylidene)-(4-10 membered heterocyclyl)), C₁-C₁₀ haloalkyl, C₁-C₁₀ cyanoalkyl, C₁-C₁₀ hydroxyalkyl, C₁-C₁₀ aminoalkyl, C₁-C₁₀ carboxyalkyl, C₁-C₁₀ alkoxyalkyl, and C₁-C₁₀ alkylaminoalkyl; among them H on the alkylene, alkyl, alkenyl, alkynyl, cycloalkyl, and heterocyclyl is each optionally substituted with one or more of independent R groups;
or, R5 and R6, together with the nitrogen atom to which they are attached, form a heterocyclyl, and the heterocyclyl is optionally substituted with one or more independent substituents selected from: oxo(=O), halogen, cyano, nitro, C₁-C₁₀ alkyl, C₂-C₁₀ alkenyl, C₂-C₁₀ alkynyl, -(C₀-C₆ alkylidene)-(C₃-C₁₀ cycloalkyl), -(C₀-C₆ alkylidene)-(C₆-C₁₀ aryl), -(C₀-C₆ alkylidene)-(4-10 membered heterocyclyl), -O(C₀-C₁₀ alkyl), -S(C₀-C₁₀ alkyl), -C(O)(C₀-C₁₀ alkyl), -C(O)N(C₀-C₁₀ alkyl)(C₀-C₁₀ alkyl), -N(C₀-C₁₀ alkyl)(C₀-C₁₀ alkyl), -C(O)O(C₀-C₁₀ alkyl), -S(O)₂(C₀-C₁₀ alkyl), -S(O)₂-(C₀-C₆ alkylidene)-(C₃-C₁₀ cycloalkyl), -S(O)₂-(C₀-C₆ alkylidene)-(4-10 membered heterocyclyl), -S(O)₂N(C₀-C₁₀ alkyl)(C₀-C₁₀ alkyl), -S(O)₂N(C₀-C₁₀ alkyl)((C₀-C₆ alkylidene)-(C₃-C₁₀ cycloalkyl)), -S(O)₂N(C₀-C₁₀ alkyl)((C₀-C₆ alkylidene)-(4-10 membered heterocyclyl)), C₁-C₁₀ haloalkyl, C₁-C₁₀ cyanoalkyl, C₁-C₁₀ hydroxyalkyl, C₁-C₁₀ aminoalkyl, C₁-C₁₀ carboxyalkyl, C₁-C₁₀ alkoxyalkyl, and C₁-C₁₀ alkylaminoalkyl; among them H on the alkylene, alkyl, alkenyl, alkynyl, cycloalkyl, aryl, and heterocyclyl is optionally substituted with one or more of independent R groups;
R₇ is one or more independent substituents on the A ring, each independently selected from: H, D, halogen, cyano, nitro, and 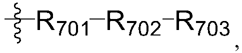 among which, R₇₀₁ is selected from: a single bond and C₁₋₁₀ alkylidene; R₇₀₂ is selected from: a single bond, C₁₋₁₀ alkylidene, O, S, N(R₇₀₄), S(O)₂, S(O)₂N(R₇₀₄), S(O), S(O)N(R₇₀₄), C(O), C(O)O, C(O)N(R₇₀₄), OC(O), OC(O)N(R₇₀₄), N(R₇₀₄)C(O)O, N(R₇₀₄)C(O), and N(R₇₀₄)S(O)₂, R₇₀₃ is selected from: H, D, halogen, cyano, nitro, C₁₋₁₀ alkyl, C₁₋₁₀ haloalkyl, C₂₋₁₀ alkenyl, C₂₋₁₀ alkynyl, -(C₀-C₆ alkylidene)-(C₃-C₁₀ cycloalkyl), -(C₀-C₆ alkylidene)-(C₆-C₁₀ aryl), and -(C₀-C₆ alkylidene)-(4-10 membered heterocyclyl), and R₇₀₄ is selected from: H, D, and C₁₋₁₀ alkyl; among them H on the alkylene, alkyl, alkenyl, alkynyl, cycloalkyl, aryl, and heterocyclyl is each optionally substituted with one or more of independent R groups;
R₈ and R₉ are independently selected from: H, D, halogen, cyano, nitro, C₁-C₁₀ alkyl, C₂-C₁₀ alkenyl, C₂-C₁₀ alkynyl, -(C₀-C₆ alkylidene)-(C₃-C₁₀ cycloalkyl), -(C₀-C₆ alkylidene)-(C₆-C₁₀ aryl), -(C₀-C₆ alkylidene)-(4-10 membered heterocyclyl), -O(C₀-C₁₀ alkyl), -S(C₀-C₁₀ alkyl), -C(O)(C₀-C₁₀ alkyl), -C(O)N(C₀-C₁₀ alkyl)(C₀-C₁₀ alkyl), -N(C₀-C₁₀ alkyl)(C₀-C₁₀ alkyl), -C(O)O(C₀-C₁₀ alkyl), -S(O)₂(C₀-C₁₀ alkyl), -S(O)₂-(C₀-C₆ alkylidene)-(C₃-C₁₀ cycloalkyl), -S(O)₂-(C₀-C₆ alkylidene)-(4-10 membered heterocyclyl), -S(O)₂N(C₀-C₁₀ alkyl)(C₀-C₁₀ alkyl), -S(O)₂N(C₀-C₁₀ alkyl)((C₀-C₆ alkylidene)-(C₃-C₁₀ cycloalkyl)), -S(O)₂N(C₀-C₁₀ alkyl)((C₀-C₆ alkylidene)-(4-10 membered heterocyclyl)), C₁-C₁₀ haloalkyl, C₁-C₁₀ cyanoalkyl, C₁-C₁₀ hydroxyalkyl, C₁-C₁₀ aminoalkyl, C₁-C₁₀ carboxyalkyl, C₁-C₁₀ alkoxyalkyl, and C₁-C₁₀ alkylaminoalkyl; among them H on the alkylene, alkyl, alkenyl, alkynyl, cycloalkyl, aryl, and heterocyclyl is each optionally substituted with one or more of independent R groups;
or, R₈ and R₉ together with the carbon atom to which they are attached, form a cycloalkyl or a heterocyclyl, and the cycloalkyl or the heterocyclyl is optionally substituted with one or more independent substituents selected from: oxo(=O), (=alkylidene), halogen, cyano, nitro, C₁-C₁₀ alkyl, C₂-C₁₀ alkenyl, C₂-C₁₀ alkynyl, -(C₀-C₆ alkylidene)-(C₃-C₁₀ cycloalkyl), -(C₀-C₆ alkylidene)-(C₆-C₁₀ aryl), -(C₀-C₆ alkylidene)-(4-10 membered heterocyclyl), -O(C₀-C₁₀ alkyl), -S(C₀-C₁₀ alkyl), -C(O)(C₀-C₁₀ alkyl), -C(O)N(C₀-C₁₀ alkyl)(C₀-C₁₀ alkyl), -N(C₀-C₁₀ alkyl)(C₀-C₁₀ alkyl), -C(O)O(C₀-C₁₀ alkyl), -S(O)₂(C₀-C₁₀ alkyl), -S(O)₂-(C₀-C₆ alkylidene)-(C₃-C₁₀ cycloalkyl), -S(O)₂-(C₀-C₆ alkylidene)-(4-10 membered heterocyclyl), -S(O)₂N(C₀-C₁₀ alkyl)(C₀-C₁₀ alkyl), -S(O)₂N(C₀-C₁₀ alkyl)((C₀-C₆ alkylidene)-(C₃-C₁₀ cycloalkyl)), -S(O)₂N(C₀-C₁₀ alkyl)((C₀-C₆ alkylidene)-(4-10 membered heterocyclyl)), C₁-C₁₀ haloalkyl, C₁-C₁₀ cyanoalkyl, C₁-C₁₀ hydroxyalkyl, C₁-C₁₀ aminoalkyl, C₁-C₁₀ carboxyalkyl, C₁-C₁₀ alkoxyalkyl, and C₁-C₁₀ alkylaminoalkyl; among them H on the alkylene, alkyl, alkenyl, alkynyl, cycloalkyl, aryl, and heterocyclyl is each optionally substituted with one or more of independent R groups;
W is a single bond, 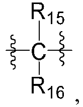 or S, among which R₁₅ and R₁₆ are independently selected from: H, D, halogen, cyano, nitro, C₁-C₁₀ alkyl, C₂-C₁₀ alkenyl, C₂-C₁₀ alkynyl, -(C₀-C₆ alkylidene)-(C₃-C₁₀ cycloalkyl), -(C₀-C₆ alkylidene)-(C₆-C₁₀ aryl), -(C₀-C₆ alkylidene)-(4-10 membered heterocyclyl), -O(C₀-C₁₀ alkyl), -S(C₀-C₁₀ alkyl), -C(O)(C₀-C₁₀ alkyl), -C(O)N(C₀-C₁₀ alkyl)(C₀-C₁₀ alkyl), -N(C₀-C₁₀ alkyl)(C₀-C₁₀ alkyl), -C(O)O(C₀-C₁₀ alkyl), -S(O)₂(C₀-C₁₀ alkyl), -S(O)₂-(C₀-C₆ alkylidene)-(C₃-C₁₀ cycloalkyl), -S(O)₂-(C₀-C₆ alkylidene)-(4-10 membered heterocyclyl), -S(O)₂N(C₀-C₁₀ alkyl)(C₀-C₁₀ alkyl), -S(O)₂N(C₀-C₁₀ alkyl)((C₀-C₆ alkylidene)-(C₃-C₁₀ cycloalkyl)), -S(O)₂N(C₀-C₁₀ alkyl)((C₀-C₆ alkylidene)-(4-10 membered heterocyclyl)), C₁-C₁₀ haloalkyl, C₁-C₁₀ cyanoalkyl, C₁-C₁₀ hydroxyalkyl, C₁-C₁₀ aminoalkyl, C₁-C₁₀ carboxyalkyl, C₁-C₁₀ alkoxyalkyl, and C₁-C₁₀ alkylaminoalkyl; among them H on the alkylene, alkyl, alkenyl, alkynyl, cycloalkyl, aryl, and heterocyclyl is each optionally substituted with one or more of independent R groups;
or, R₁₅ and R₁₆, together with the carbon atom to which they are attached, form a cycloalkyl or a heterocyclyl, among which H on the cycloalkyl or the heterocyclyl is optionally substituted with one or more independent substituents selected from: oxo(=O), (= alkylidene), halogen, cyano, nitro, C₁-C₁₀ alkyl, C₂-C₁₀ alkenyl, C₂-C₁₀ alkynyl, -(C₀-C₆ alkylidene)-(C₃-C₁₀ cycloalkyl), -(C₀-C₆ alkylidene)-(C₆-C₁₀ aryl), -(C₀-C₆ alkylidene)-(4-10 membered heterocyclyl), -O(C₀-C₁₀ alkyl), -S(C₀-C₁₀ alkyl), -C(O)(C₀-C₁₀ alkyl), -C(O)N(C₀-C₁₀ alkyl)(C₀-C₁₀ alkyl), -N(C₀-C₁₀ alkyl)(C₀-C₁₀ alkyl), -C(O)O(C₀-C₁₀ alkyl), -S(O)₂(C₀-C₁₀ alkyl), -S(O)₂-(C₀-C₆ alkylidene)-(C₃-C₁₀ cycloalkyl), -S(O)₂-(C₀-C₆ alkylidene)-(4-10 membered heterocyclyl), -S(O)₂N(C₀-C₁₀ alkyl)(C₀-C₁₀ alkyl), -S(O)₂N(C₀-C₁₀ alkyl)((C₀-C₆ alkylidene)-(C₃-C₁₀ cycloalkyl)), -S(O)₂N(C₀-C₁₀ alkyl)((C₀-C₆ alkylidene)-(4-10 membered heterocyclyl)), C₁-C₁₀ haloalkyl, C₁-C₁₀ cyanoalkyl, C₁-C₁₀ hydroxyalkyl, C₁-C₁₀ aminoalkyl, C₁-C₁₀ carboxyalkyl, C₁-C₁₀ alkoxyalkyl, and C₁-C₁₀ alkylaminoalkyl; among them H on the alkylene, alkyl, alkenyl, alkynyl, cycloalkyl, aryl, and heterocyclyl is each optionally substituted with one or more of independent R groups;
or, R₁₅ and R₉, together with the intervening carbon atom, form a cycloalkyl, an aryl, or a heterocyclyl, among which H on the cycloalkyl, the aryl, or the heterocyclyl is optionally substituted with one or more independent substituents selected from: oxo(=O), (= alkylidene), halogen, cyano, nitro, C₁-C₁₀ alkyl, C₂-C₁₀ alkenyl, C₂-C₁₀ alkynyl, -(C₀-C₆ alkylidene)-(C₃-C₁₀ cycloalkyl), -(C₀-C₆ alkylidene)-(C₆-C₁₀ aryl), -(C₀-C₆ alkylidene)-(4-10 membered heterocyclyl), -O(C₀-C₁₀ alkyl), -S(C₀-C₁₀ alkyl), -C(O)(C₀-C₁₀ alkyl), -C(O)N(C₀-C₁₀ alkyl)(C₀-C₁₀ alkyl), -N(C₀-C₁₀ alkyl)(C₀-C₁₀ alkyl), -C(O)O(C₀-C₁₀ alkyl), -S(O)₂(C₀-C₁₀ alkyl), -S(O)₂-(C₀-C₆ alkylidene)-(C₃-C₁₀ cycloalkyl), -S(O)₂-(C₀-C₆ alkylidene)-(4-10 membered heterocyclyl), -S(O)₂N(C₀-C₁₀ alkyl)(C₀-C₁₀ alkyl), -S(O)₂N(C₀-C₁₀ alkyl)((C₀-C₆ alkylidene)-(C₃-C₁₀ cycloalkyl)), -S(O)₂N(C₀-C₁₀ alkyl)((C₀-C₆ alkylidene)-(4-10 membered heterocyclyl)), C₁-C₁₀ haloalkyl, C₁-C₁₀ cyanoalkyl, C₁-C₁₀ hydroxyalkyl, C₁-C₁₀ aminoalkyl, C₁-C₁₀ carboxyalkyl, C₁-C₁₀ alkoxyalkyl, and C₁-C₁₀ alkylaminoalkyl; among them H on the alkylene, alkyl, alkenyl, alkynyl, cycloalkyl, aryl, and heterocyclyl is each optionally substituted with one or more of independent R groups;
Y₁, Y₂, Y₃ and Y₄ are independently selected from: C, N, O, and S, and any two of Y₁, Y₂, Y₃ and Y₄ are two O atoms, two S atoms, or an O atom and an S atom are not directly bonded;
R₁₀ is one or more independent substituents on the ring, each R₁₀ independently selected from: H, D, oxo(=O), halogen, cyano, nitro, C₁-C₁₀ alkyl, C₂-C₁₀ alkenyl, C₂-C₁₀ alkynyl, -(C₀-C₆ alkylidene)-(C₃-C₁₀ cycloalkyl), -(C₀-C₆ alkylidene)-(C₆-C₁₀ aryl), -(C₀-C₆ alkylidene)-(4-10 membered heterocyclyl), -O(C₀-C₁₀ alkyl), -S(C₀-C₁₀ alkyl), -C(O)(C₀-C₁₀ alkyl), -C(O)N(C₀-C₁₀ alkyl)(C₀-C₁₀ alkyl), -N(C₀-C₁₀ alkyl)(C₀-C₁₀ alkyl), -C(O)O(C₀-C₁₀ alkyl), -S(O)₂(C₀-C₁₀ alkyl), -S(O)₂-(C₀-C₆ alkylidene)-(C₃-C₁₀ cycloalkyl), -S(O)₂-(C₀-C₆ alkylidene)-(4-10 membered heterocyclyl), -S(O)₂N(C₀-C₁₀ alkyl)(C₀-C₁₀ alkyl), -S(O)₂N(C₀-C₁₀ alkyl)((C₀-C₆ alkylidene)-(C₃-C₁₀ cycloalkyl)), -S(O)₂N(C₀-C₁₀ alkyl)((C₀-C₆ alkylidene)-(4-10 membered heterocyclyl)), C₁-C₁₀ haloalkyl, C₁-C₁₀ cyanoalkyl, C₁-C₁₀ hydroxyalkyl, C₁-C₁₀ aminoalkyl, C₁-C₁₀ carboxyalkyl, C₁-C₁₀ alkoxyalkyl, and C₁-C₁₀ alkylaminoalkyl; among them H on the alkylene, alkyl, alkenyl, alkynyl, cycloalkyl, aryl, and heterocyclyl is each optionally substituted with one or more of independent R groups;
each R group is independently selected from: D, halogen, cyano, nitro, and 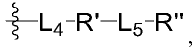 among which L₄ and L₅ are independently selected from: a single bond, O, S, N(R"'), S(O)₂, S(O)₂N(R‴), S(O), S(O)N(R‴), C(O), C(O)O, C(O)N(R‴), OC(O), OC(O)N(R‴), N(R‴)C(O)O, N(R‴)C(O), and N(R‴)S(O)₂;
each R' group is independently selected from: a single bond, C₁-C₁₀ alkylidene, C₂-C₁₀ alkenylene, phenylene, C₃-C₁₀ cycloalkylene, and 4-10 membered heterocyclylene;
each R" group is independently selected from: H, D, -CD₃, halogen, cyano, nitro, C₁-C₁₀ alkyl, C₁-C₁₀ haloalkyl, C₂-C₁₀ alkenyl, C₂-C₁₀ alkynyl, C₃-C₁₀ cycloalkyl, and 4-10 membered heterocyclyl; and
each R‴ group is independently selected from: H, D, C₁-C₁₀ alkyl, C₁-C₁₀ haloalkyl, C₂-C₁₀ alkenyl, C₂-C₁₀ alkynyl, C₃-C₁₀ cycloalkyl, and 4-10 membered heterocyclyl.

In some embodiments of the present disclosure, A-ring is a benzene ring or 5-6 membered heteroaromatic ring, such as, 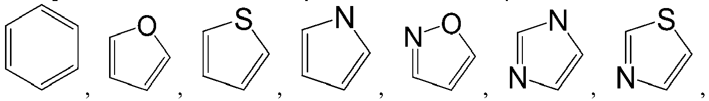 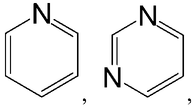 especially benzene ring.

In some embodiments of the present disclosure, 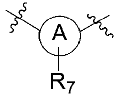 moiety is selected from: 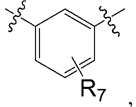 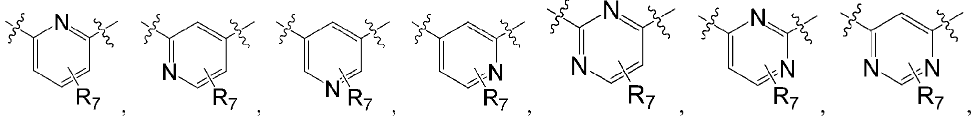 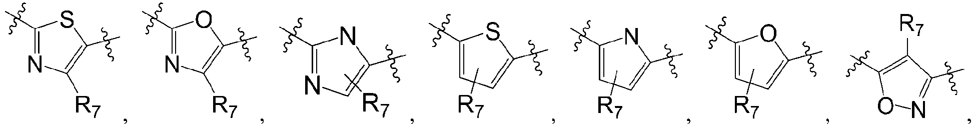 especially

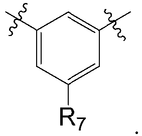

In some embodiments of the present disclosure, B-ring is 5 or 6 membered heterocyclic ring, such as 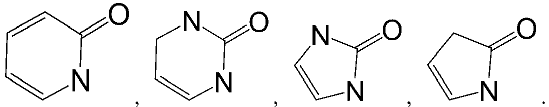 In some embodiments of the present disclosure, G-ring is 5 or 6 membered heterocyclic ring, such as

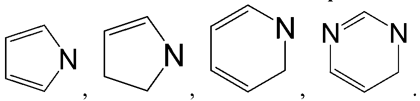

In some embodiments of the present disclosure, the ring 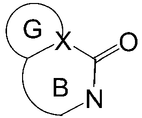 is

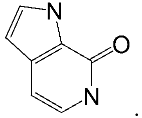

In some embodiments of the present disclosure, the ring 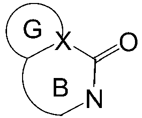 is

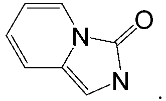

In some embodiments of the present disclosure, the ring 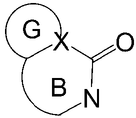 is

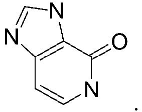

In some embodiments of the present disclosure, at least one R^{A} is 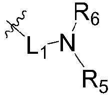 or

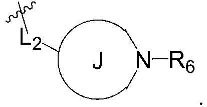

In some embodiments of the present disclosure, the compound has the following structure:

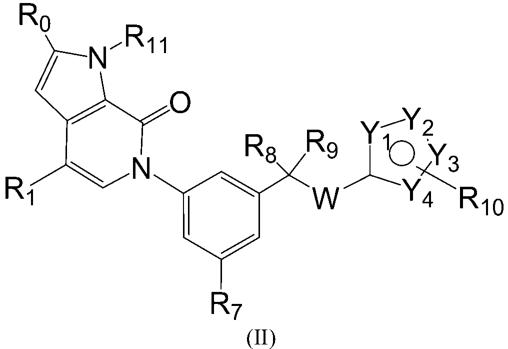

Specifically,

R₀ and R₁ are independently selected from: H, D, 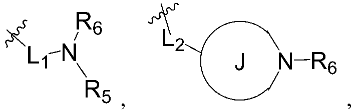 and 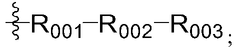 and

R₁₁ is selected from: H, D, C₁-C₁₀ alkyl, C₁-C₁₀ haloalkyl, C₂-C₁₀ alkenyl, C₂-C₁₀ alkynyl, -(C₀-C₆ alkylidene)-(C₃-C₁₀ cycloalkyl), -(C₀-C₆ alkylidene)-(C₆-C₁₀ aryl), and -(C₀-C₆ alkylidene)-(4-10 membered heterocyclyl); among them the alkylidene, alkyl, alkenyl, alkynyl, cycloalkyl, aryl, and heterocyclyl are each optionally substituted with one or more of independent R groups.

In some embodiments of the present disclosure, the compound has the following structure:

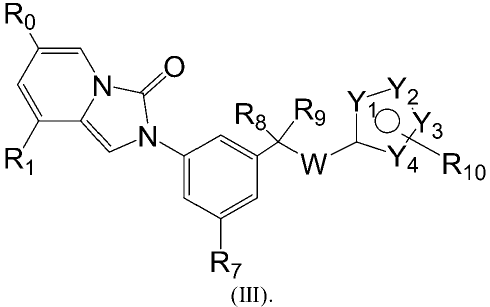

In some embodiments of the present disclosure, the compound has the following structure:

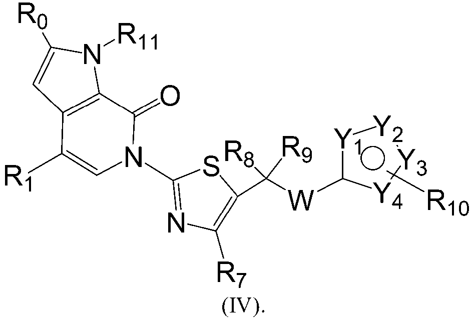

In some embodiments of the present disclosure, the compound has the following structure:

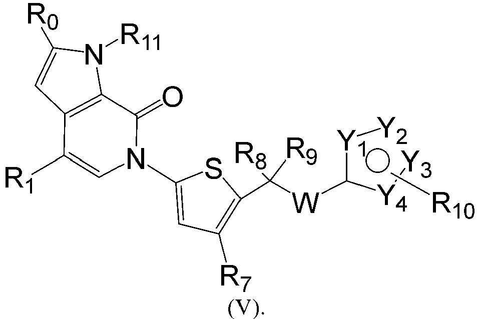

In some embodiments of the present disclosure, the compound has the following structure:

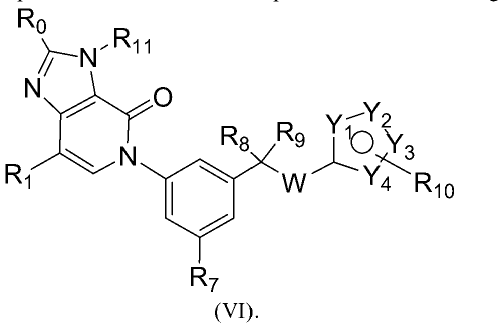

Specifically, L₄ and L₅ can be independently selected from: a single bond, O, S, N(H), S(O)₂, S(O)₂N(H), C(O), C(O)O, C(O)N(H), OC(O), N(H)C(O), and N(H)S(O)₂.

Specifically, R' group may be independently selected from: a single bond and C₁-C₃ alkylidene.

Specifically, R" group may be independently selected from: H, D, -CD₃, halogen, cyano, nitro, C₁-C₆ alkyl, C₁-C₆ haloalkyl, and C₃-C₆ cycloalkyl.

Specifically, R‴ group can be independently selected from H, D, and C₁-C₃ alkyl.

Specifically, each R group may be independently selected from: D, -CD₃, halogen, cyano, nitro, C₁-C₆ alkyl, -(C₀-C₃ alkylidene)-(C₃-C₆ cycloalkyl), -(C₀-C₃ alkylidene)-(phenyl), -(C₀-C₃ alkylidene)-(4-6 membered heterocyclyl), -O(C₀-C₆ alkyl), -S(C₀-C₆ alkyl), -C(O)(C₀-C₆ alkyl), -C(O)N(C₀-C₆ alkyl)(C₀-C₆ alkyl), -N(C₀-C₆ alkyl)(C₀-C₆ alkyl), -C(O)O(C₀-C₆ alkyl), -S(O)₂(C₀-C₆ alkyl), -S(O)₂-(C₀-C₃ alkylidene)-(C₃-C₆ cycloalkyl), -S(O)₂-(C₀-C₃ alkylidene)-(4-6 membered heterocyclyl), -S(O)₂N(C₀-C₆ alkyl)(C₀-C₆ alkyl), -S(O)₂N(C₀-C₆ alkyl)((C₀-C₃ alkylidene)-(C₃-C₆ cycloalkyl)), -S(O)₂N(C₀-C₆ alkyl)((C₀-C₃ alkylidene)-(4-6 membered heterocyclyl)), C₁-C₆ haloalkyl, C₁-C₆ cyanoalkyl, C₁-C₆ hydroxyalkyl, C₁-C₆ carboxyalkyl, C₁-C₆ alkoxyalkyl, and C₁-C₆ alkylaminoalkyl; among them the alkylidene, alkyl, cycloalkyl, and heterocyclyl is optionally substituted with one or more independent substituents selected from: D, -CD₃, halogen (e.g., -F), cyano, -OH, C₁₋₆ alkoxyl (such as 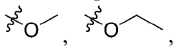 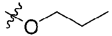 ), -NH₂, C₁₋₆ alkylamino (such as 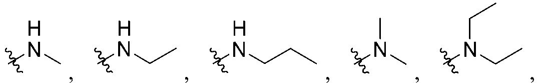 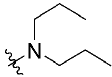 ), C₃₋₆ cycloalkyl (such as 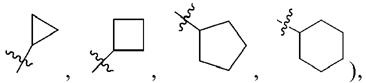 saturated 4-6 membered heterocycloalkyl (such as 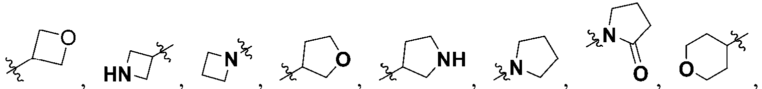 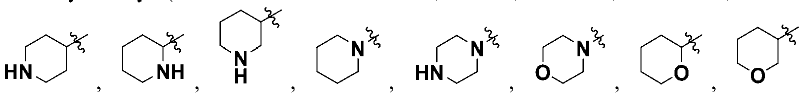 ), sulfonyl (such as -S(O)₂(C₀-C₆ alkyl), -S(O)₂-(C₀-C₆ alkylidene)-(C₃-C₆ cycloalkyl), -S(O)₂-(C₀-C₆ alkylidene)-(4-6 membered heterocyclyl), -S(O)₂N(C₀-C₆ alkyl)(C₀-C₆ alkyl), -S(O)₂N(C₀-C₆ alkyl)((C₀-C₆ alkylidene)-(C₃-C₆ cycloalkyl)), and -S(O)₂N(C₀-C₆ alkyl)((C₀-C₆ alkylidene)-(4-6 membered heterocyclyl)), such as 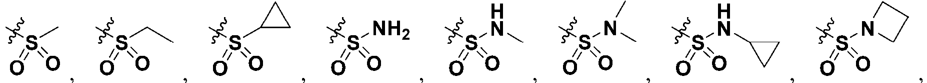 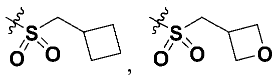 ), acyl (such as -C(O)(C₀-C₆ alkyl), and -C(O)N(C₀-C₆ alkyl)(C₀-C₆ alkyl), such as 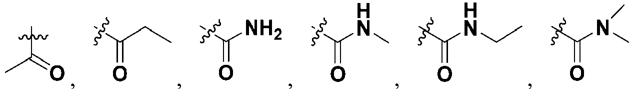 ), C₁-C₆ haloalkoxyl (such as -OCF₃), and -C(O)O(C₀-C₆ alkyl) (such as -COOH and -COOCH₃).

In an example of the present disclosure, R₀ is selected from: H, D, C₁-C₆ alkyl, C₁-C₆ haloalkyl, C₃-C₆ cycloalkyl, and C₁-C₆ hydroxyalkyl (such as 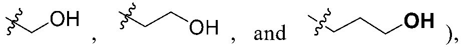 C₁-C₆ alkoxyalkyl (such as 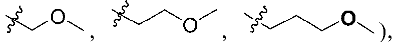 C₁-C₆ amidoalkyl (such as -CH₂-CONH₂ and -CH₂CH₂-CONH₂).

In an example of the present disclosure, R₀ is 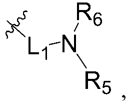 especially 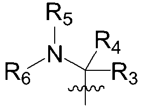 or

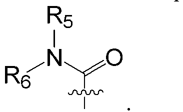

Specifically, in the 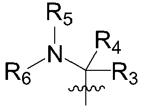 structure, R₃ and R₄ can be independently selected from: H, D, halogen, and C₁-C₆ alkyl (such as -CH₃, 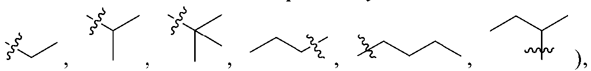 C₁-C₆ haloalkyl (such as -CHF₂, -CH₂F, -CF₃, -CH₂-CF₃, and -CH₂Cl), C₁-C₆ cyanoalkyl (such as 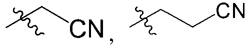 ), C₁-C₆ hydroxyalkyl (such as 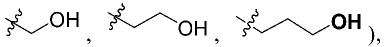 C₁-C₆ alkoxyalkyl (such as 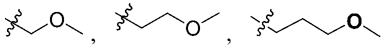 ), C₁₋₆ aminoalkyl (such as 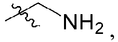 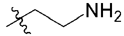 ), C₁-C₆ alkylaminoalkyl (such as 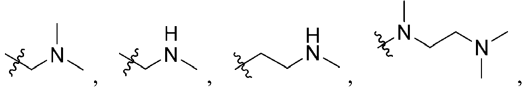 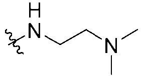 ), C₃-C₆ cycloalkyl (such as 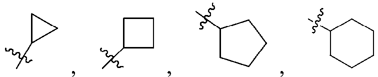 ), and C₄-C₁₀ cycloalkylalkyl (such as

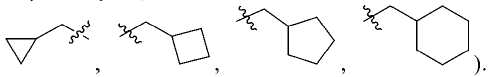

In some embodiments of the present disclosure, R₃ is H; R₄ may be selected from: H, D, C₁-C₆ alkyl, C₁-C₆ haloalkyl, C₁-C₆ hydroxyalkyl, C₃-C₆ cycloalkyl, and C₄-C₁₀ cycloalkylalkyl.

In an embodiment of the present disclosure, R₃ is H, and R₄ is H.

In an embodiment of the present disclosure, R₃ is D, and R₄ is D.

In an embodiment of the present disclosure, R₃ is H, and R₄ is -CH₃.

In an embodiment of the present disclosure, R₃ is H, and R₄ is cyclopropyl.

In an embodiment of the present disclosure, R₃ is H, and R₄ is cyclobutyl.

In an embodiment of the present disclosure, R₃ is H, and R₄ is

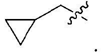

In an embodiment of the present disclosure, R₃ is H, and R₄ is

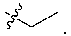

In an embodiment of the present disclosure, R₃ is H, and R₄ is

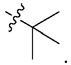

In an embodiment of the present disclosure, R₃ is H, and R₄ is

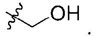

In an example of the present disclosure, R₅ is selected from: H, D, C₁-C₆ alkyl, C₁-C₆ haloalkyl, C₁-C₆ cyanoalkyl, C₁-C₆ hydroxyalkyl, and C₁-C₆ aminoalkyl; more specifically, R₅ may be selected from: H, D, -CH₃, -CHF₂, -CH₂F, -CH₂Cl, -CF₃, -CH₂OH, -CH₂NH₂, and -CH₂CN.

In some embodiments of the present disclosure, R₅ is H.

In an example of the present disclosure, R₆ is selected from: H, D, C₁-C₆ alkyl (such as -CH₃, 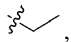 , 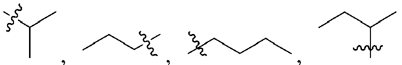 ), C₃-C₆ cycloalkyl (such as 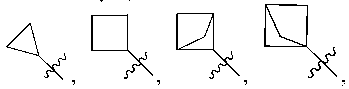 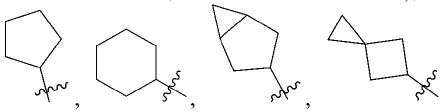 ), C₄-C₁₀ cycloalkylalkyl (such as 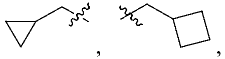 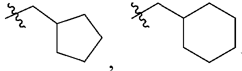 ), saturated 4-10 membered heterocyclyl (such as 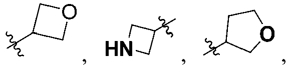 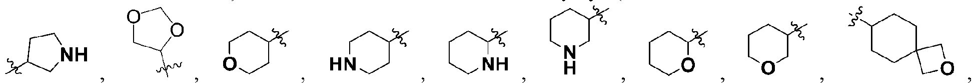 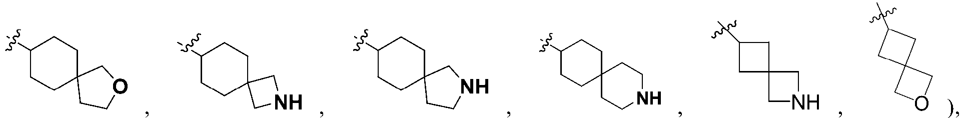 and heterocyclylalkyl, among which the alkyl, cycloalkyl, cycloalkylalkyl, heterocyclyl, and heterocyclylalkyl is optionally substituted with one or more independent substituents selected from: C₁-C₆ alkyl, C₂-C₆ alkenyl, C₃-C₆ cycloalkyl, 4-6 membered heterocyclyl, halogen, hydroxyl, C₁-C₆ hydroxyalkyl, C₁-C₆ alkoxyl, C₁-C₆ alkoxyalkyl, cyano, C₁-C₆ cyanoalkyl, carboxyl, C₁-C₆ carboxyalkyl, C₁-C₆ haloalkyl, and C₂-C₆ sulfonyl.

More specifically, R₆ is selected from: H, D, C₁-C₆ alkyl, C₁-C₆ haloalkyl, C₁-C₆ hydroxyalkyl, C₁-C₆ alkoxyalkyl, C₃-C₆ cycloalkyl, C₄-C₁₀ cycloalkylalkyl, and saturated 4-10 membered heterocyclyl, among which the cycloalkyl, cycloalkylalkyl, and heterocyclyl is optionally substituted with one or more substituents selected from: halogen and C₁-C₃ alkyl.

In some embodiments of the present disclosure, R₆ is selected from: H, D, C₁-C₆ alkyl, C₁-C₆ haloalkyl, C₁-C₆ hydroxyalkyl, C₁-C₆ alkoxyalkyl,

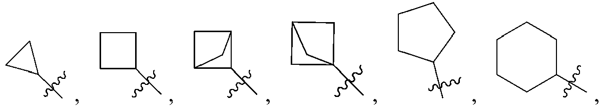

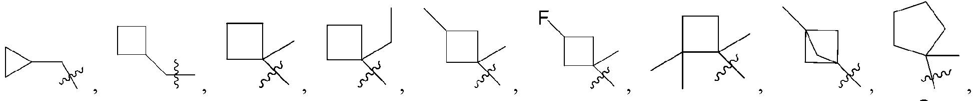

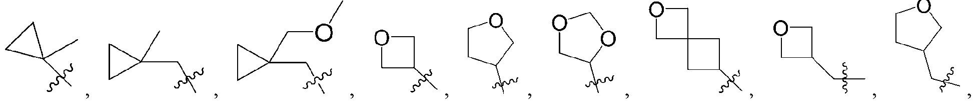

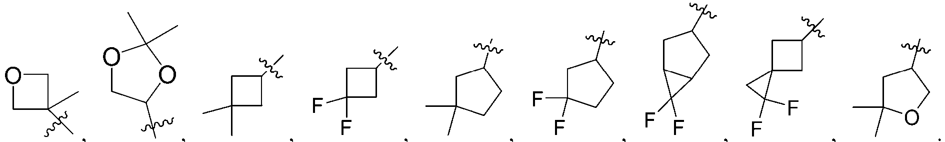

In some embodiments of the present disclosure, R₀ is selected from:

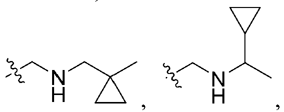

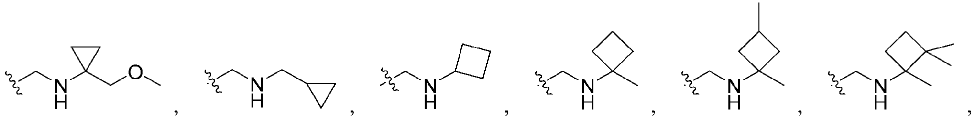

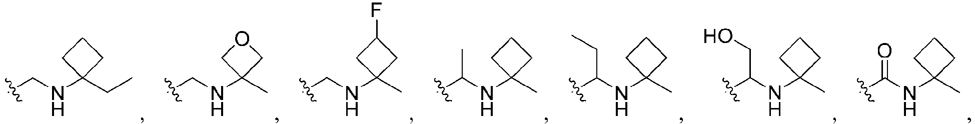

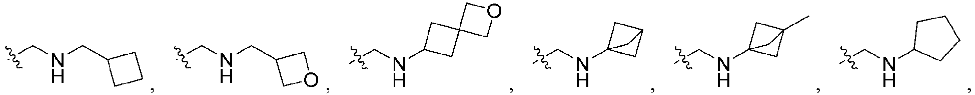

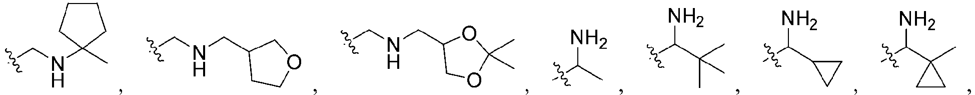

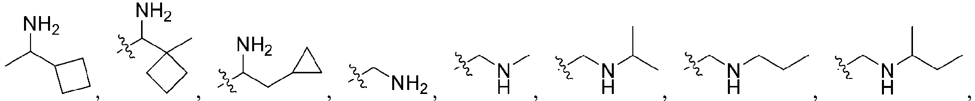

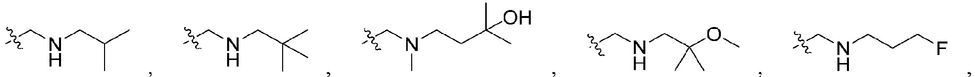

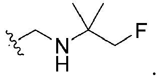

In some embodiments of the present disclosure, R₀ is selected from:

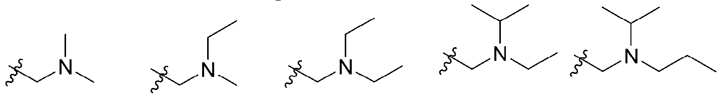

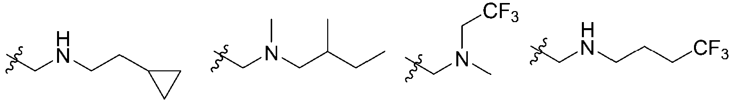

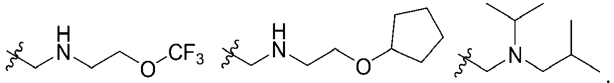

In an example of the present disclosure, R₅ and R₆, together with the nitrogen atom to which they are attached, form heterocyclyl 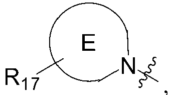 that is, 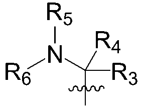 may be 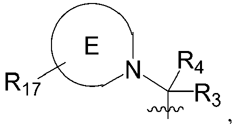 among which E-ring is 4-14 membered heterocyclic ring (which may be a monocyclic or polycyclic system, including fused, spiro, or bridged structures; each ring may be a saturated or unsaturated heterocyclic ring, optionally containing one or more additional heteroatoms, such as a 3-10 membered monocyclic ring, fused bicyclic ring or bridged heterocycloalkyl, or a 5-10 membered monocyclic ring or bicyclic heteroaryl, among which each heterocycloalkyl or heteroaryl optionally further includes one or two additional heteroatoms selected from nitrogen and oxygen); and
R₁₇ is one or more independent substituents on the E-ring, each R₁₇ independently selected from: H, D, (=O), halogen, cyano, nitro, C₁-C₁₀ alkyl, C₁-C₁₀ deuterated alkyl, C₂-C₁₀ alkenyl, C₂-C₁₀ alkynyl, -(C₀-C₆ alkylidene)-(C₃-C₁₀ cycloalkyl), -(C₀-C₆ alkylidene)-(C₆-C₁₀ aryl), -(C₀-C₆ alkylidene)-(4-10 membered heterocyclyl), -O(C₀-C₁₀ alkyl), -S(C₀-C₁₀ alkyl), -C(O)(C₀-C₁₀ alkyl), -C(O)N(C₀-C₁₀ alkyl)(C₀-C₁₀ alkyl), -N(C₀-C₁₀ alkyl)(C₀-C₁₀ alkyl), -C(O)O(C₀-C₁₀ alkyl), -S(O)₂(C₀-C₁₀ alkyl), -S(O)₂-(C₀-C₆ alkylidene)-(C₃-C₁₀ cycloalkyl), -S(O)₂-(C₀-C₆ alkylidene)-(4-10 membered heterocyclyl), -S(O)₂N(C₀-C₁₀ alkyl)(C₀-C₁₀ alkyl), -S(O)₂N(C₀-C₁₀ alkyl)((C₀-C₆ alkylidene)-(C₃-C₁₀ cycloalkyl)), -S(O)₂N(C₀-C₁₀ alkyl)((C₀-C₆ alkylidene)-(4-10 membered heterocyclyl)), C₁-C₁₀ haloalkyl, C₁-C₁₀ cyanoalkyl, C₁-C₁₀ hydroxyalkyl, C₁-C₁₀ aminoalkyl, C₁-C₁₀ carboxyalkyl, C₁-C₁₀ alkoxyalkyl, and C₁-C₁₀ alkylaminoalkyl; among them the alkylidene, alkyl, alkenyl, alkynyl, cycloalkyl, aryl, and heterocyclyl are each optionally substituted with one or more independent substituents selected from: D, halogen, cyano, nitro, C₁₋₁₀ alkyl, -(C₀-C₆ alkylidene)-(C₃-C₁₀ cycloalkyl), -(C₀-C₆ alkylidene)-(C₆-C₁₀ aryl), -(C₀-C₆ alkylidene)-(4-10 membered heterocyclyl), -O(C₀-C₁₀ alkyl), -S(C₀-C₁₀ alkyl), -C(O)(C₀-C₁₀ alkyl), -C(O)N(C₀-C₁₀ alkyl)(C₀-C₁₀ alkyl), -N(C₀-C₁₀ alkyl)(C₀-C₁₀ alkyl), -C(O)O(C₀-C₁₀ alkyl), -S(O)₂(C₀-C₁₀ alkyl), -S(O)₂-(C₀-C₆ alkylidene)-(C₃-C₁₀ cycloalkyl), -S(O)₂-(C₀-C₆ alkylidene)-(4-10 membered heterocyclyl), -S(O)₂N(C₀-C₁₀ alkyl)(C₀-C₁₀ alkyl), -S(O)₂N(C₀-C₁₀ alkyl)((C₀-C₆ alkylidene)-(C₃-C₁₀ cycloalkyl)), and -S(O)₂N(C₀-C₁₀ alkyl)((C₀-C₆ alkylidene)-(4-10 membered heterocyclyl)); among them the heterocyclyl is optionally substituted with one or more independent substituents selected from: halogen, cyano, nitro, hydroxyl, amino, C₁-C₁₀ alkyl, substituted or unsubstituted phenyl, or 4-6 membered heterocyclyl.

In some embodiments, E-ring is a saturated heterocyclic ring, such as: 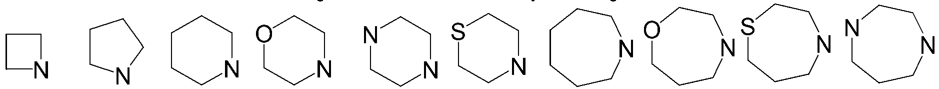 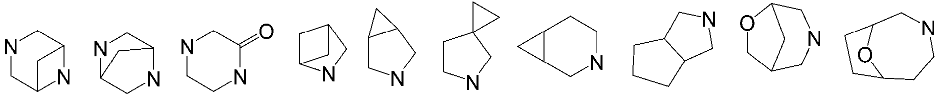 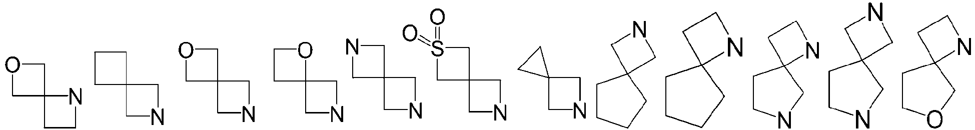 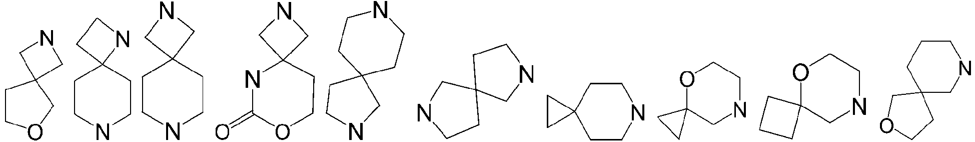 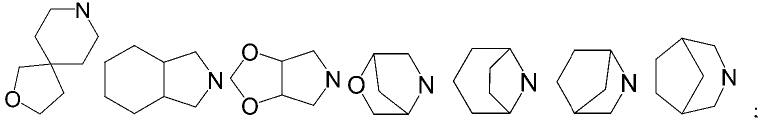 especially

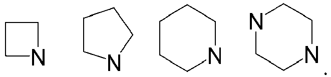

In some embodiments, E-ring is a partially unsaturated heterocyclic ring, such as

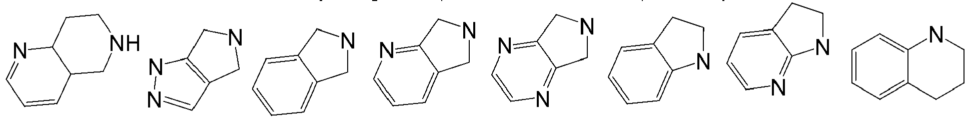

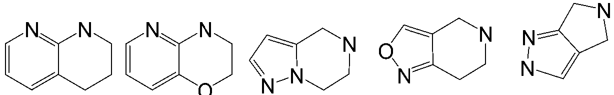

More specifically, each R₁₇ can be independently selected from: H, D, (=O), halogen (such as -F), C₁-C₆ alkyl (such as -CH₃, 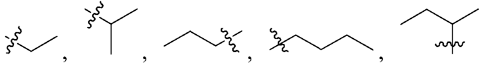 ), C₁-C₆ deuterated alkyl (such as 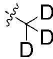 ), C₂-C₆ alkenyl (such as 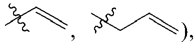 C₁-C₆ haloalkyl (such as -CHF₂, -CH₂F, -CH₂Cl, -CF₃, -CH₂CF₃, and -CH₂CH₂F), cyano, C₁-C₆ cyanoalkyl (such as 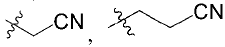 ), -OH, C₁-C₆ alkoxyl (such as 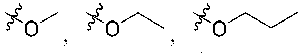 ), C₁-C₆ deuterated alkoxyl (such as 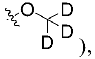 C₁-C₆ hydroxyalkyl (such as 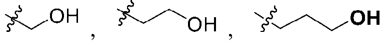 ), C₁-C₆ alkoxyalkyl (such as 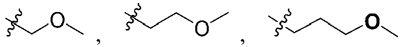 ), -NH₂, C₁-C₆ alkylamino (such as 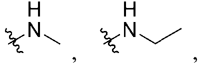 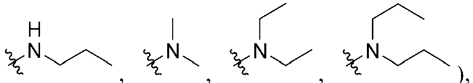 C₁-C₆ alkylaminoalkyl (such as 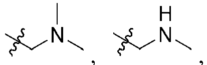 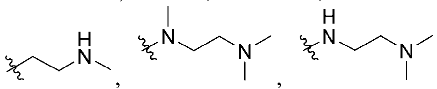 ), -(C₀-C₃ alkylidene)-(C₃-C₆ cycloalkyl) (such as 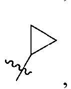 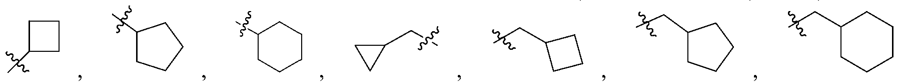 ), -(C₀-C₆ alkylidene)-(saturated 4-10 membered heterocyclyl) (such as 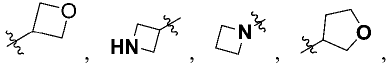 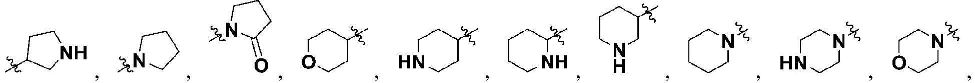 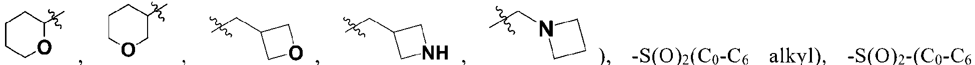 alkylidene)-(C₃-C₆ cycloalkyl), -S(O)₂-(C₀-C₆ alkylidene)-(4-6 membered heterocyclyl), -S(O)₂N(C₀-C₆ alkyl)(C₀-C₆ alkyl), -S(O)₂N(C₀-C₆ alkyl)((C₀-C₆ alkylidene)-(C₃-C₆ cycloalkyl)), -S(O)₂N(C₀-C₆ alkyl)((C₀-C₆ alkylidene)-(4-6 membered heterocyclyl)) (such as 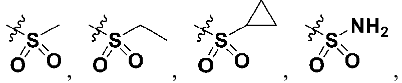 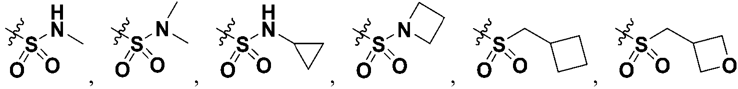 ), -C(O)(C₀-C₆ alkyl) (such as 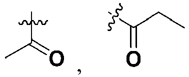 ), -C(O)O(C₀-C₆ alkyl) (such as 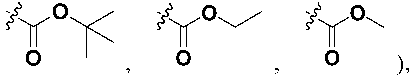 -C(O)N(C₀-C₆ alkyl)(C₀-C₆ alkyl) (such as 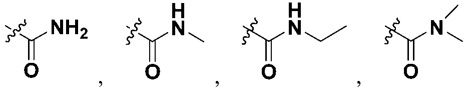 ), C₁-C₆ haloalkoxyl (e.g., -OCF₃), -C(O)O(C₀-C₆ alkyl) (such as -COOH and -COOCH₃).

In some embodiments of the present disclosure, 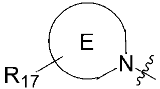 is 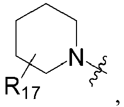 specifically 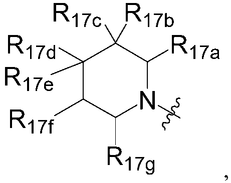 among which R₁₇ₐ to R_{17g} each has the same definition as described above for R₁₇, or two from R₁₇ₐ to R_{17g}, together with the intervening carbon atom, form a cycloalkyl or a heterocyclyl.

Specifically, R₁₇ₐ and R_{17g} are independently selected from: H, D, halogen, C₁-C₆ alkyl, C₁-C₆ haloalkyl, C₁-C₆ hydroxyalkyl, cyano, C₁-C₆ cyanoalkyl, and C₁-C₆ alkoxyalkyl.

In some embodiments of the present disclosure, R₁₇ₐ is H, D, -CH₃, -CF₃, or -CH₂OH, especially H.

In some embodiments of the present disclosure, R_{17g} is H, D, -CH₃, -CF₃, or -CH₂OH, especially H.

Specifically, R_{17b} is selected from: H, D, C₁-C₆ alkyl, C₁-C₆ deuterated alkyl, C₁-C₆ haloalkyl, C₁-C₆ hydroxyalkyl, cyano, C₁-C₆ cyanoalkyl, and C₁-C₆ alkoxyalkyl, and R_{17c} is selected from: H, D, halogen, C₁-C₆ alkyl, and C₁-C₆ deuterated alkyl; or, R_{15b} and R_{15c}, together with the intervening carbon atom, form a 3-6 membered cycloalkyl or heterocycloalkyl.

In some embodiments of the present disclosure, R_{17b} is selected from: H, D, -CD₃, -CN, -CH₃, -CF₃, -CH₂-CN, -CH₂-OH, and -CH₂OCH₃.

In some embodiments of the present disclosure, R_{17c} is selected from: H, D, -CD₃, F, and -CH₃.

More specifically, R_{17b} and R_{17c}, together with the carbon atom to which they are attached, form a 4-5 membered heterocycloalkyl or a 3-4 membered cycloalkyl.

In some embodiments of the present disclosure, R_{17b} and R_{17c}, together with the carbon atom to which they are attached, form

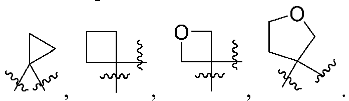

Specifically, R_{17d} and R₁₇ₑ are independently selected from: H, D, halogen, C₁-C₆ alkyl; or, R_{15d} and R₁₅ₑ, together with the carbon atom to which they are attached, form a 3-4 membered cycloalkyl.

More specifically, R_{17d} and R₁₇ₑ are independently selected from: H, D, halogen, and C₁-C₃ alkyl.

In some embodiments of the present disclosure, R_{17d} is selected from: H, D, F, and -CH₃.

In some embodiments of the present disclosure, R₁₇ₑ is selected from: H, D, F, and -CH₃.

In some embodiments of the present disclosure, R_{17d} and R₁₇ₑ, together with the carbon atom to which they are attached, form

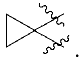

Specifically, R_{17f} is selected from: H, D, C₁-C₆ alkyl, C₁-C₆ haloalkyl, C₁-C₆ hydroxyalkyl, cyano, and C₁-C₆ cyanoalkyl.

In some embodiments of the present disclosure, R_{17f} is selected from: -CN, -CH₃, -CF₃, -CH₂-CN, -CH₂-OH, and -CH₂OCH₃.

In some embodiments of the present disclosure, 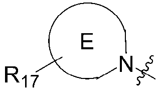 is 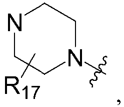 specifically 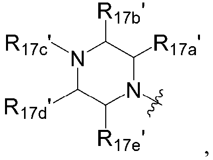 among which R₁₇ₐ' to R₁₇ₑ' each has the same definition as described above for R₁₇, or two of R₁₇ₐ' to R₁₇ₑ', together with the annular atom to which they are attached, form a cycloalkyl or a heterocyclyl.

In some embodiments of the present disclosure, R₁₇ₐ' is selected from: H, D, C₁-C₆ alkyl such as -CH₃, 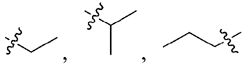 ), and C₃-C₄ cycloalkyl (such as

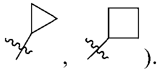

In some embodiments of the present disclosure, R_{17b}' is selected from: H, D, and C₁-C₆ alkyl (such as methyl, ethyl, and isopropyl).

In some embodiments of the present disclosure, R_{17c}' is selected from: H, D, halogen, C₁-C₆ alkyl (such as -CH₃, 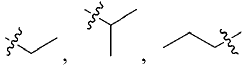 ), C₁-C₆ haloalkyl (such as -CHF₂, -CH₂F, -CH₂Cl, -CF₃, -CH₂CF₃, and -CH₂CH₂F), and -C(O)O(C₀-C₆ alkyl) (such as

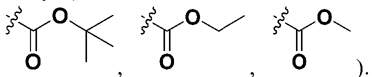

In some embodiments of the present disclosure, R_{17d}' is selected from: H, D, C₁-C₆ alkyl (such as -CH₃, 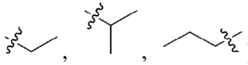 ), and C₃-C₄ cycloalkyl (such as

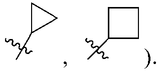

In some embodiments of the present disclosure, R₁₇ₑ' is selected from: H, D, C₁-C₆ alkyl, especially H.

In some embodiments of the present disclosure, 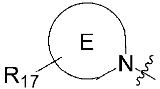 is selected from the following structure:

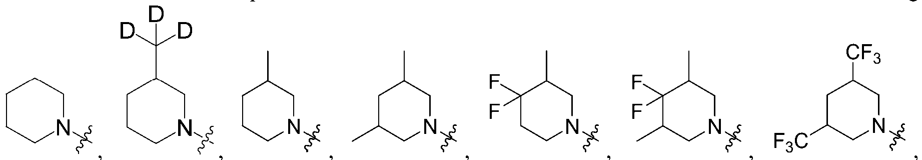

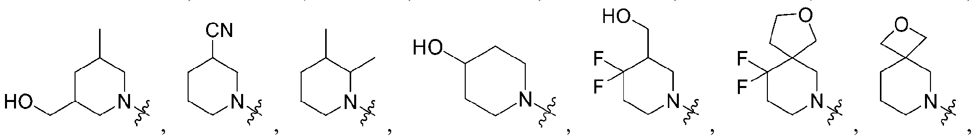

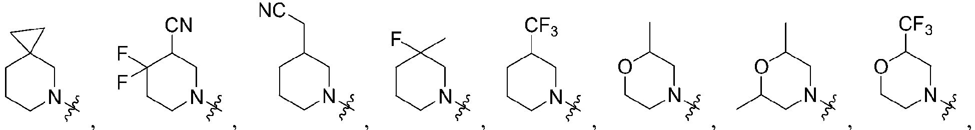

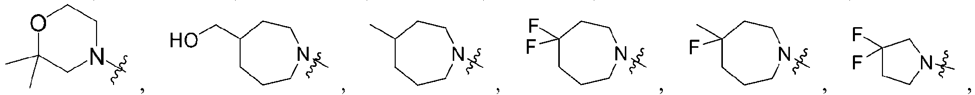

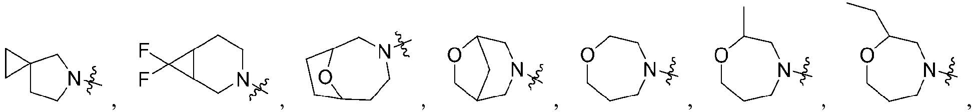

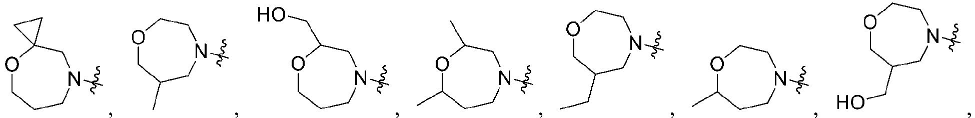

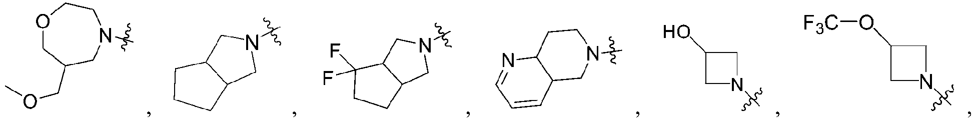

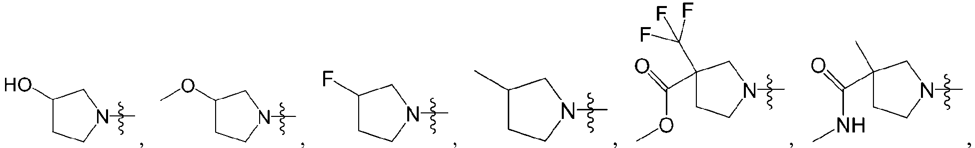

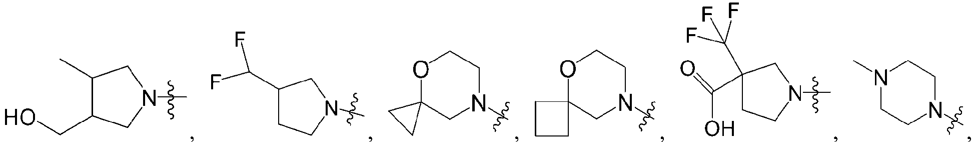

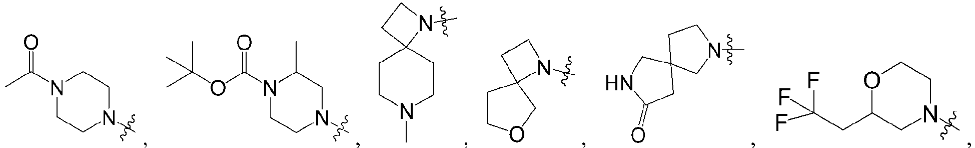

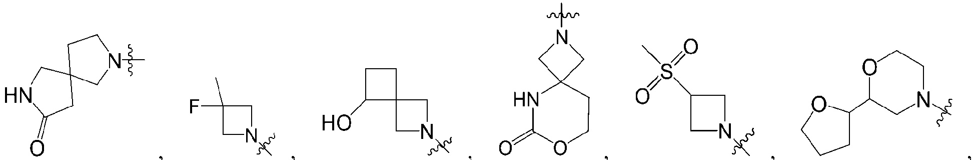

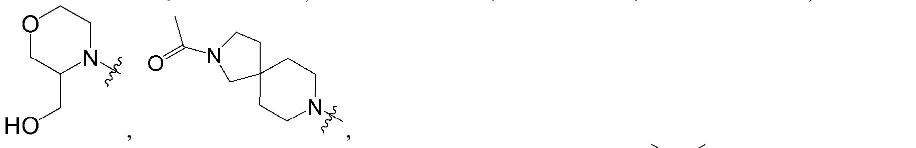

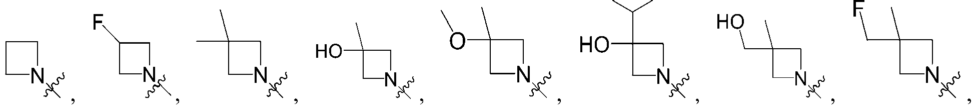

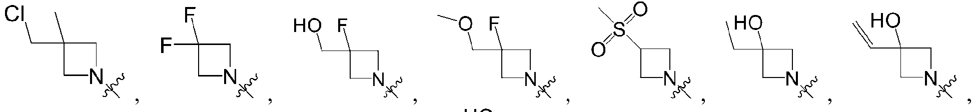

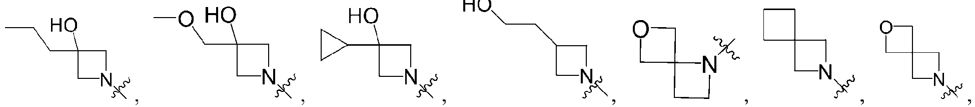

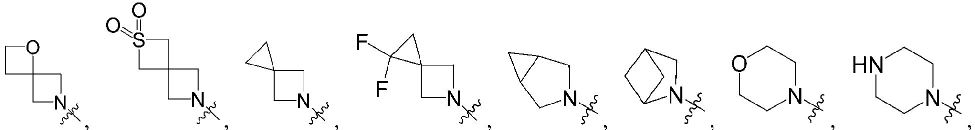

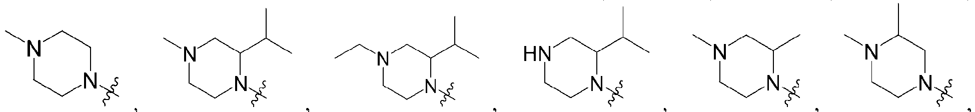

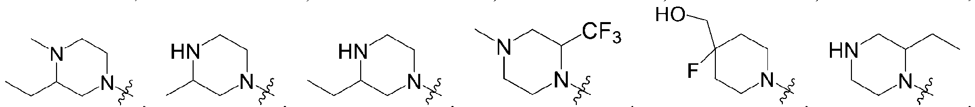

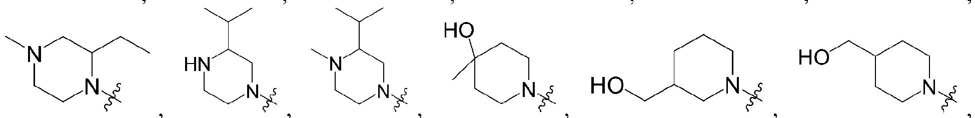

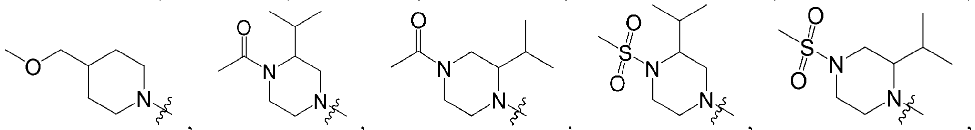

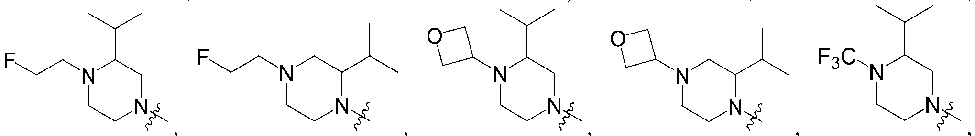

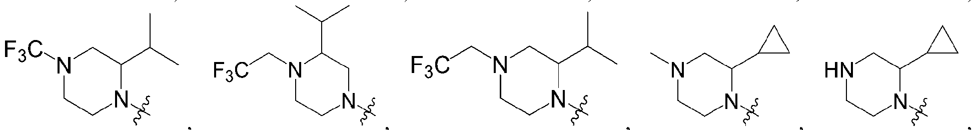

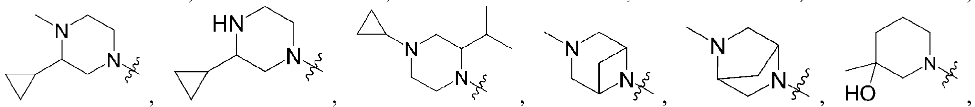

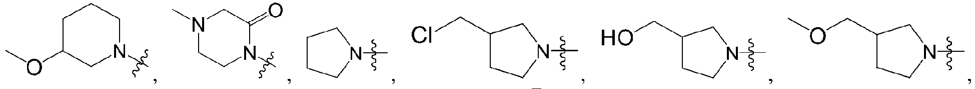

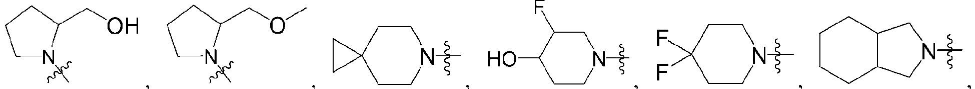

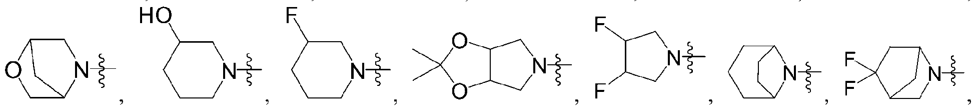

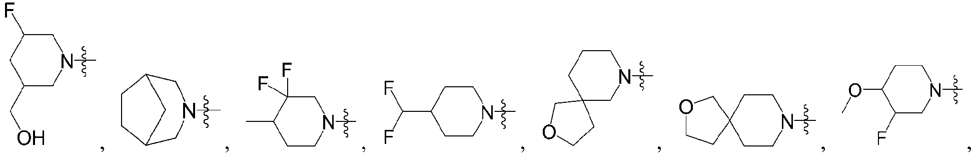

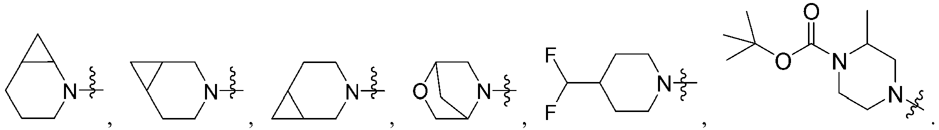

In some embodiments of the present disclosure, 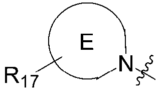 is selected from the following structure:

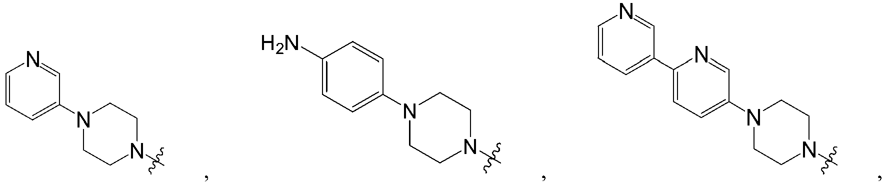

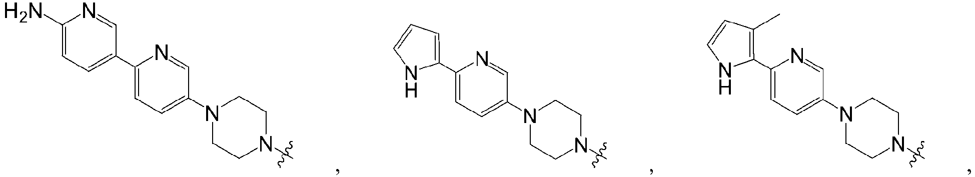

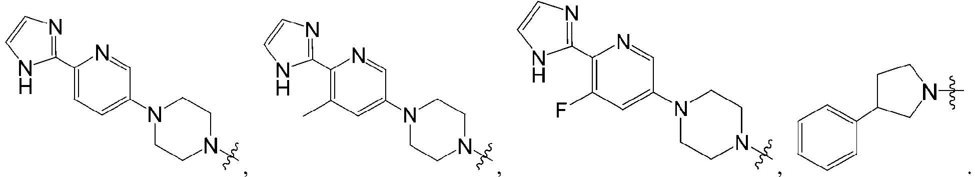

In some embodiments of the present disclosure, 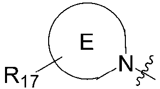 is selected from the following structure:

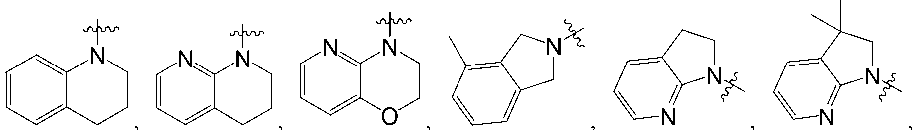

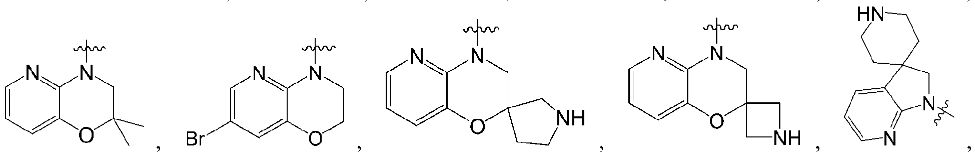

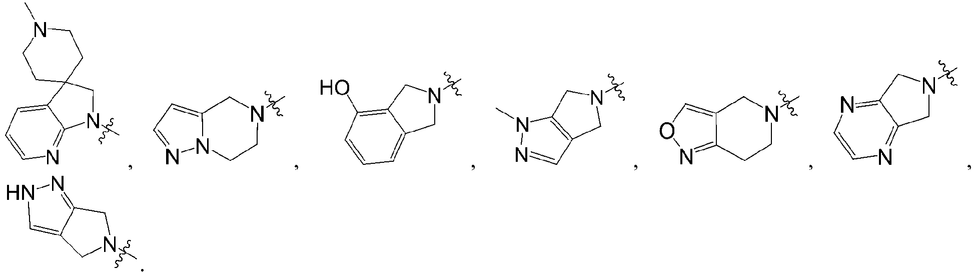

In some embodiments of the present disclosure, 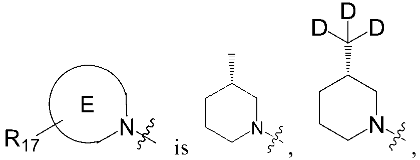 and

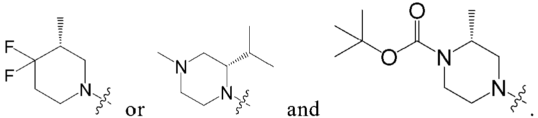

In an example of the present disclosure, R₀ is

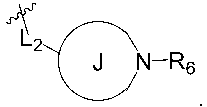

Specifically, in the structure of 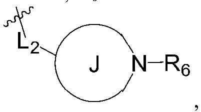 R₃ and R₄ can be independently selected from: H, D, halogen, C₁-C₆ alkyl (such as -CH₃, 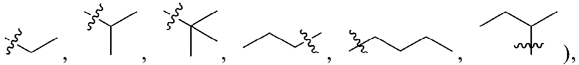 C₁-C₆ haloalkyl (such as -CHF₂, -CH₂F, -CF₃, -CH₂-CF₃, and -CH₂Cl), C₁-C₆ cyanoalkyl (such as 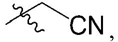 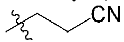 ), C₁-C₆ hydroxyalkyl (such as 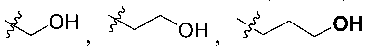 ), C₁-C₆ alkoxyalkyl (such as 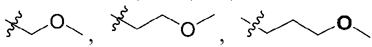 ), C₁-C₆ aminoalkyl (such as 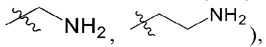 C₁-C₆ alkylaminoalkyl (such as 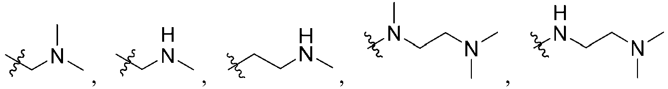 ), C₃-C₆ cycloalkyl (such as 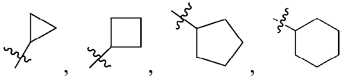 ), and C₄-C₁₀₀ cycloalkylalkyl (such as

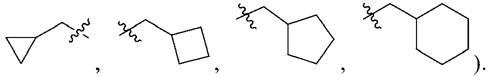

In some embodiments of the present disclosure, R₃ is H; R₄ may be selected from: H, D, C₁-C₆ alkyl, C₁-C₆ haloalkyl, C₁-C₆ hydroxyalkyl, C₃-C₆ cycloalkyl, and C₄-C₁₀ cycloalkylalkyl.

In an embodiment of the present disclosure, R₃ is H, and R₄ is H.

In an embodiment of the present disclosure, R₃ is D, and R₄ is D.

In an embodiment of the present disclosure, R₃ is H, and R₄ is -CH₃.

In an embodiment of the present disclosure, R₃ is H, and R₄ is cyclopropyl.

In an embodiment of the present disclosure, R₃ is H, and R₄ is cyclobutyl.

In an embodiment of the present disclosure, R₃ is H, and R₄ is

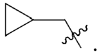

In an embodiment of the present disclosure, R₃ is H, and R₄ is

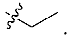

In an embodiment of the present disclosure, R₃ is H, and R₄ is

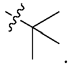

In an embodiment of the present disclosure, R₃ is H, and R₄ is

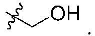

Specifically, in the structure of 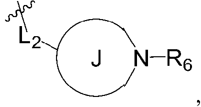 R₅ may be selected from H, D, and C₁-C₆ alkyl.

In some embodiments of the present disclosure, R₅ is H.

In some embodiments of the present disclosure, L₂ is a single bond.

In some embodiments of the present disclosure, L₂ is O.

In some embodiments of the present disclosure, L₂ is C(R₃R₄), especially CHR₃, such as CH₂.

In some embodiments of the present disclosure, L₂ is 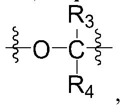 especially 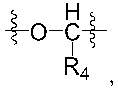 such as

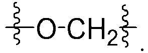

In some embodiments of the present disclosure, L₂ is N(R₅), such as NH.

In some embodiments of the present disclosure, 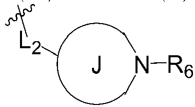 is 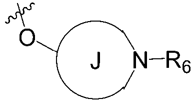 or

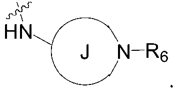

Specifically, J-ring is a 3-10 membered nitrogen-containing heterocyclic ring, which may be selected from: monocyclic ring, spirocyclic ring, bridged bicyclic ring, and fused bicyclic ring.

In some embodiments of the present disclosure, J-ring is a 5 or 6 membered heteroaromatic ring, such as

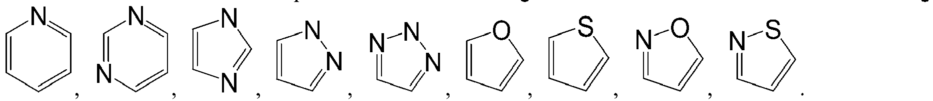

In some embodiments of the present disclosure, J-ring is a saturated 4-8 membered heterocyclic ring, such as

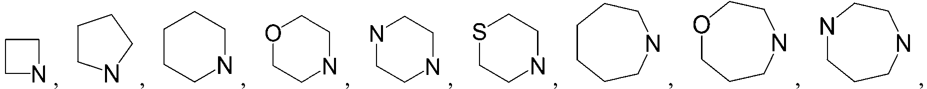

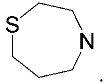

Specifically, R₆ may be selected from: H, D, C₁-C₆ alkyl, and C₃-C₆ cycloalkyl (such as cyclopropyl and cyclobutyl); in some embodiments of the present disclosure, R₆ is H.

In some embodiments of the present disclosure, 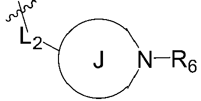 is selected from the following structure:

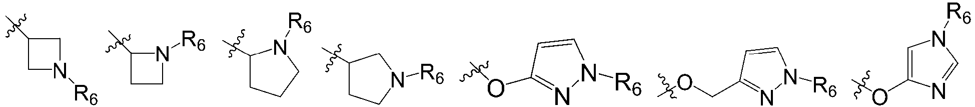

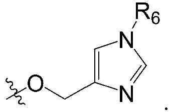

Specifically, R₁ is 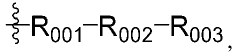 among which, R₀₀₁ is selected from: a single bond and C₁-C₆ alkylidene, R₀₀₂ is selected from: a single bond, C₁-C₆ alkylidene, O, S, N(R₂), S(O)₂, S(O)₂N(R₂), S(O), S(O)N(R₂), C(O), C(O)O, C(O)N(R₂), OC(O), OC(O)N(R₂), N(R₂)C(O)O, N(R₂)C(O), and N(R₂)S(O)₂, and R₀₀₃ is selected from: H, D, halogen, cyano, nitro, hydroxyl, C₁-C₆ alkyl, C₁-C₆ haloalkyl, C₂-C₆ alkenyl, C₂-C₆ alkynyl, C₃-C₆ cycloalkyl, C₃-C₆ halocycloalkyl, C₄-C₁₀ cycloalkylalkyl, saturated 4-10 membered heterocyclyl, and heterocyclylalkyl; R₁ is optionally substituted with one or more independent substituents selected from: halogen, hydroxyl, C₁-C₆ alkoxyl, amino, C₁-C₆ alkylamino, cyano, and carboxyl.

In some embodiments of the present disclosure, R₀₀₂ is selected from: a single bond, O, S(O)₂, S(O)₂N(R₂), C(O), C(O)N(R₂), and N(R₂)S(O)₂.

In some embodiments of the present disclosure, R₂ is selected from: H, D, C₁-C₃ alkyl (such as methyl and ethyl), and C₃-C₆ cycloalkyl (such as

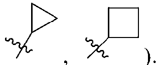

In some embodiments of the present disclosure, R₀₀₃ is selected from: H, D, halogen, cyano, nitro, hydroxyl, C₁-C₃ alkyl, C₁-C₃ haloalkyl, C₂-C₃ alkenyl, C₃-C₆ cycloalkyl, and saturated 4-6 membered heterocyclyl.

More specifically, R₁ may be selected from: H, D, halogen (e.g., Br), hydroxyl, C₁-C₆ alkyl (such as -CH₃, 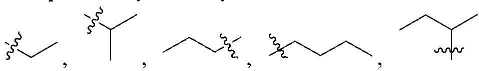 ), C₁-C₆ haloalkyl (such as -CHF₂, -CH₂F, -CF₃, -CH₂-CF₃, and -CH₂Cl), C₂-C₆ alkenyl (such as 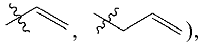 C₁-C₆ alkoxyl (such as 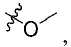 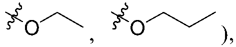 C₁-C₆ haloalkoxyl (such as -OCHF₂, -OCH₂F, and -OCF₃), C₁-C₆ alkoxyalkyl (such as 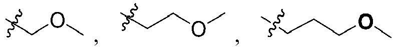 ), C₁-C₆ haloalkoxyalkyl (such as 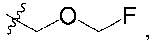 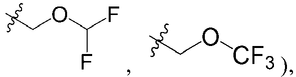 C₃-C₆ cycloalkyl (such as 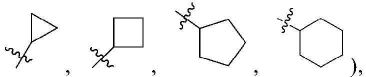 C₃-C₆ halocycloalkyl (such as 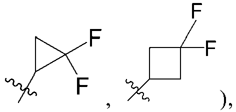 C₄-C₁₀ cycloalkylalkyl (such as 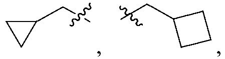 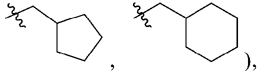 saturated 4-6 membered heterocyclyl (such as 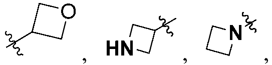 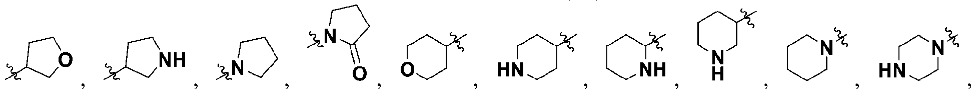 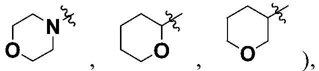 -S(O)₂(C₀-C₆ alkyl), -S(O)₂-(C₀-C₆ alkylidene)-(C₃-C₆ cycloalkyl), -S(O)₂-(C₀-C₆ alkylidene)-(4-6 membered heterocyclyl), -S(O)₂N(C₀-C₆ alkyl)(C₀-C₆ alkyl), -S(O)₂N(C₀-C₆ alkyl)((C₀-C₆ alkylidene)-(C₃-C₆ cycloalkyl)), -S(O)₂N(C₀-C₆ alkyl)((C₀-C₆ alkylidene)-(4-6 membered heterocyclyl)) (such as

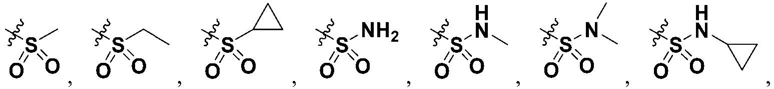

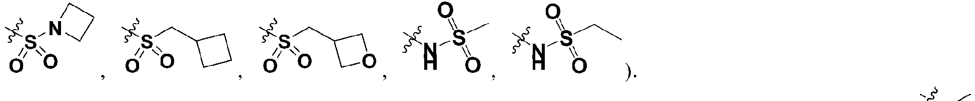

In some embodiments of the present disclosure, R₁ is selected from: H, D, F, Br, -CH₃, 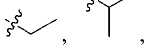 -CHF₂, -CH₂F, -CF₃, -CH₂-CF₃, 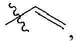 -OH, 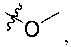 -OCHF₂, -OCH₂F, -OCF₃,

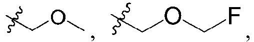

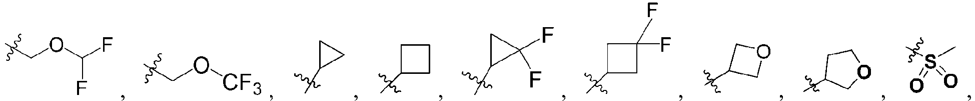

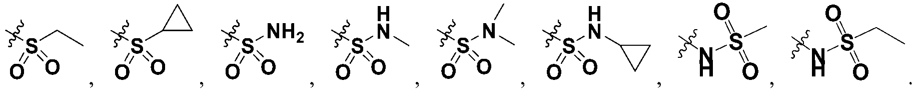

Specifically, R₇ may be selected from: H, D, halogen, cyano, C₁-C₆ alkyl, C₁-C₆ haloalkyl, -(C₀-C₆ alkylidene)-(C₃-C₆ cycloalkyl), -(C₀-C₆ alkylidene)-(4-6 membered heterocyclyl), -C(O)(C₁-C₆ alkyl), -C(O)N(C₀-C₆ alkyl)(C₀-C₆ alkyl), -N(C₀-C₆ alkyl)(C₀-C₆ alkyl), -N(C₀-C₆ alkyl)((C₀-C₆ alkylidene)-(C₃₋₆ cycloalkyl)), -N(C₀₋₆ alkyl)((C₀₋₆ alkylidene)-(4-6 membered heterocyclyl)), -O-(C₀₋₆ alkylidene)-(C₃-C₆ cycloalkyl), -O-(C₀-C₆ alkylidene)-(4-6 membered heterocyclyl), -S(O)₂(C₀-C₆ alkyl), -S(O)₂-(C₀-C₆ alkylidene)-(C₃-C₆ cycloalkyl), -S(O)₂-(C₀-C₆ alkylidene)-(4-6 membered heterocyclyl), -S(O)₂N(C₀-C₆ alkyl)(C₀-C₆ alkyl), -S(O)₂N(C₀-C₆ alkyl)((C₀-C₆ alkylidene)-(C₃-C₆ cycloalkyl)), and -S(O)₂N(C₀-C₆ alkyl)((C₀-C₆ alkylidene)-(4-6 membered heterocyclyl)). More specifically, among them the 4-6 membered heterocyclyl is saturated 4-6 membered heterocyclyl, such as

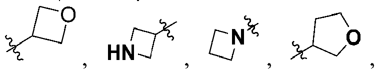

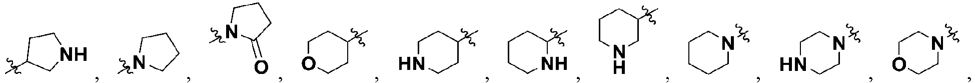

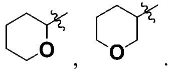

In some embodiments of the present disclosure, R₇ is selected from: -H, D, -Cl, -CN, -COOH, -CONH₂,

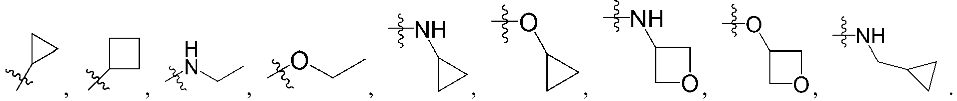

In an embodiment of the present disclosure, R₇ is H.

In an example of the present disclosure, R₈ and R₉ may be independently selected from: H, D, halogen, C₁-C₆ alkyl, C₁-C₆ haloalkyl, and 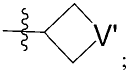 V' is selected from: a single bond, O, 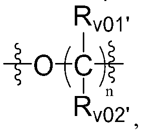 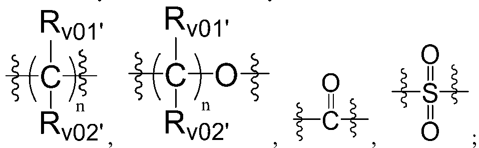 n is 1, 2 or 3, Rᵥ₀₁' and Rᵥ₀₂' are independently selected from: H, D, halogen, cyano, nitro, hydroxyl, amino, C₁-C₆ alkyl, C₁-C₆ deuterated alkyl, C₁-C₆ alkoxyl, C₁-C₆ deuterated alkoxyl, C₃-C₆ cycloalkyl, 3-8 membered heterocycloalkyl (such as 3, 4, 5, 6, 7, and 8 membered O and/or N-containing heterocycloalkyl), or, Rᵥ₀₁' and Rᵥ₀₂', together with the carbon atom to which they are attached, form a cycloalkyl or a heterocyclyl; among them the alkyl is optionally substituted with one or more independent substituents selected from: halogen, cyano, nitro, hydroxyl, C₁-C₆ alkoxyl (such as 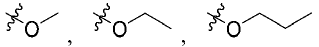 ), amino, C₁-C₆ alkylamino (such as 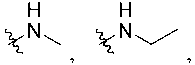 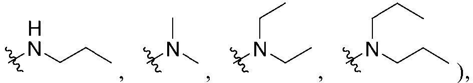 -S(O)₂(C₀-C₆ alkyl), -S(O)₂-(C₀-C₆ alkylidene)-(C₃-C₆ cycloalkyl), -S(O)₂-(C₀-C₆ alkylidene)-(4-6 membered heterocyclyl), -S(O)₂N(C₀-C₆ alkyl)(C₀-C₆ alkyl), -S(O)₂N(C₀-C₆ alkyl)((C₀-C₆ alkylidene)-(C₃-C₆ cycloalkyl)), -S(O)₂N(C₀-C₆ alkyl)((C₀-C₆ alkylidene)-(4-6 membered heterocyclyl)) (such as 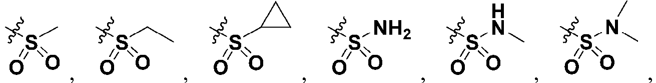 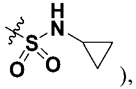 -C(O)(C₀-C₆ alkyl), and -C(O)N(C₀-C₆ alkyl)(C₀-C₆ alkyl) (such as 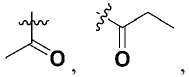 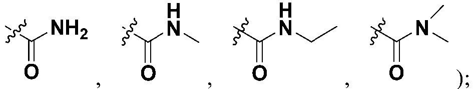 more specifically, R₈ and R₉ may be independently selected from: H, D, F, -CH₃, -CHF₂, -CH₂F, -CF₃,

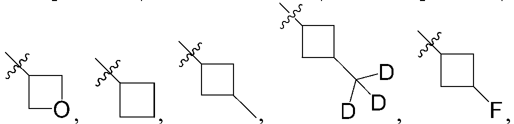

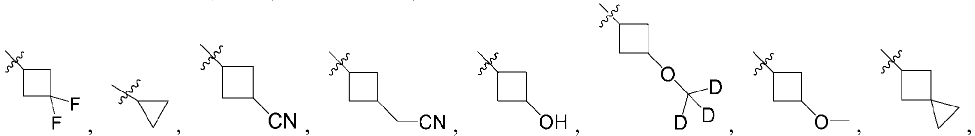

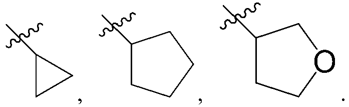

In some embodiments of the present disclosure, R₈ is H, and R₉ is selected from: halogen, C₁-C₆ alkyl, and C₁-C₆ haloalkyl.

In some other embodiments of the present disclosure, R₈ and R₉ are independently selected from: halogen, C₁-C₆ alkyl, and C₁-C₆ haloalkyl.

In some embodiments of the present disclosure, R₈ and R₉ are both methyl.

In some other embodiments of the present disclosure, R₈ and R₉ are both H.

In some other embodiments of the present disclosure, R₈ and R₉ are both halogen (e.g., F).

In some other embodiments of the present disclosure, R₈ is methyl and R₉ is halogen (e.g., F).

In some other embodiments of the present disclosure, R₈ is H, and R₉ is

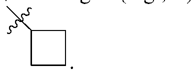

In an example of the present disclosure, R₈ and R₉, together with the intervening carbon atom, form 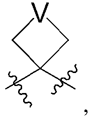 and W is a single bond, 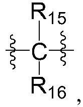 or S, among which V is selected from: a single bond, O, 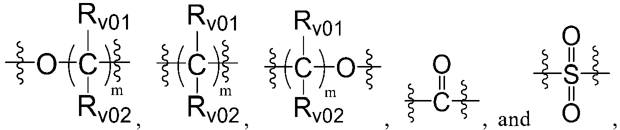 and m is 1, 2 or 3; Rᵥ₀₁ and Rᵥ₀₂ are independently selected from: H, D, halogen, cyano, nitro, hydroxyl, amino, C₁-C₆ alkyl, C₁-C₆ deuterated alkyl, C₁-C₆ alkoxyl, and C₁-C₆ deuterated alkoxyl, or, Rᵥ₀₁ and Rᵥ₀₂, together with the intervening carbon atom, form a cycloalkyl or a heterocyclyl; among them the alkyl is optionally substituted with one or more independent substituents selected from: halogen, cyano, nitro, hydroxyl, C₁-C₆ alkoxyl (such as 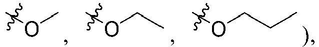 amino, C₁-C₆ alkylamino (such as 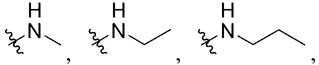 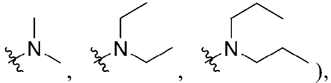 -S(O)₂(C₀-C₆ alkyl), -S(O)₂-(C₀-C₆ alkylidene)-(C₃-C₆ cycloalkyl), -S(O)₂-(C₀-C₆ alkylidene)-(4-6 membered heterocyclyl), -S(O)₂N(C₀-C₆ alkyl)(C₀-C₆ alkyl), -S(O)₂N(C₀-C₆ alkyl)((C₀-C₆ alkylidene)-(C₃-C₆ cycloalkyl)), -S(O)₂N(C₀-C₆ alkyl)((C₀-C₆ alkylidene)-(4-6 membered heterocyclyl)) (such as 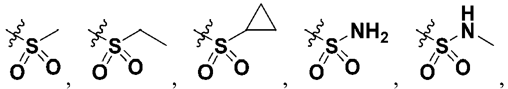 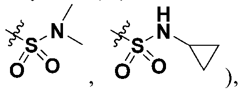 -C(O)(C₀-C₆ alkyl), and -C(O)N(C₀-C₆ alkyl)(C₀-C₆ alkyl) (such as 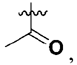 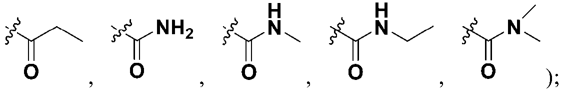 or, Rᵥ₀₁ and Rᵥ₀₂ from C₁-C₆ alkylidene (e.g.,

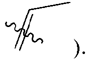

Specifically, Rᵥ₀₁ and Rᵥ₀₂ may be independently selected from: H, D, halogen, cyano, nitro, hydroxyl, amino, C₁-C₆ alkyl (such as -CH₃, 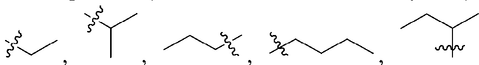 ), C₁-C₆ deuterated alkyl (e.g., 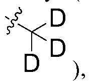 C₁-C₆ haloalkyl (such as -CHF₂, -CH₂F, -CF₃, -CH₂-CF₃, and -CH₂Cl), C₁-C₆ cyanoalkyl (such as 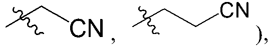 C₁-C₆ hydroxyalkyl (such as 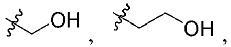 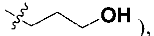 C₁-C₆ alkoxyalkyl (such as 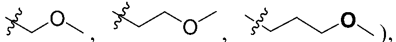 C₁-C₆ deuterated alkoxyl (e.g., 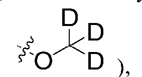 C₁-C₆ aminoalkyl (such as 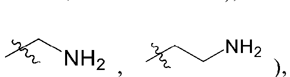 and C₁-C₆ alkylaminoalkyl (such as

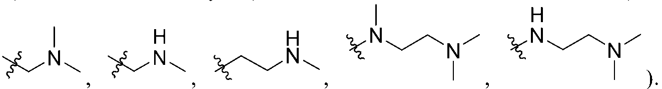

In some embodiments of the present disclosure, Rᵥ₀₁ is H, Rᵥ₀₂ is not H, and 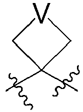 may be 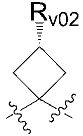 or

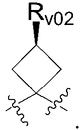

In an embodiment of the present disclosure, Rᵥ₀₁ and Rᵥ₀₂ are both H.

In an embodiment of the present disclosure, Rᵥ₀₁ is H, and Rᵥ₀₂ is -CD₃.

In an embodiment of the present disclosure, Rᵥ₀₁ and Rᵥ₀₂ are both halogen (e.g., F).

In an embodiment of the present disclosure, Rᵥ₀₁ is H, and Rᵥ₀₂ is halogen (e.g., F).

In an embodiment of the present disclosure, Rᵥ₀₁ is H, and Rᵥ₀₂ is methyl.

In some other embodiments of the present disclosure, Rᵥ₀₁ is cyano or C₁₋₆ cyanoalkyl, and Rᵥ₀₂ is selected from: H, D, C₁-C₆ alkyl, and C₁-C₆ haloalkyl; in an embodiment of the present disclosure, Rᵥ₀₁ is -CN or -CH₂-CN, and Rᵥ₀₂ is H.

In an embodiment of the present disclosure, Rᵥ₀₁ is H, and Rᵥ₀₂ is hydroxyl.

In an embodiment of the present disclosure, Rᵥ₀₁ is H, and Rᵥ₀₂ is C₁-C₃ alkoxyl (e.g., methoxyl).

In an embodiment of the present disclosure, Rᵥ₀₁ is H, and Rᵥ₀₂ is C₁₋C₃ deuterated alkoxyl (e.g., deuterated methoxyl).

In some embodiments of the present disclosure, Rᵥ₀₁ and Rᵥ₀₂, together with the intervening carbon atom, form a C₃-C₆ cycloalkyl.

In an embodiment of the present disclosure, Rᵥ₀₁ and Rᵥ₀₂ form

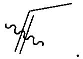

In some embodiments of the present disclosure, 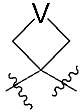 is 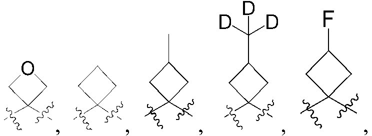 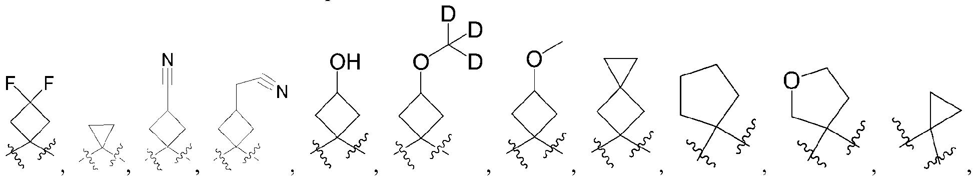 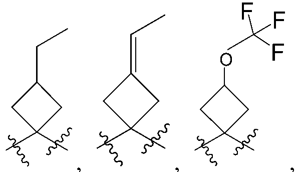 especially

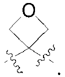

In an example of the present disclosure, R₁₅ and R₉, together with the intervening carbon atom, form 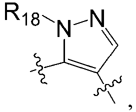 among which R₁₈ is selected from: H, D, C₁-C₆ alkyl, C₁-C₆ haloalkyl, and C₃-C₆ cycloalkyl.

Specifically, R₁₈ may be selected from: C₁-C₆ alkyl (such as -CH₃, 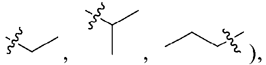 C₁-C₆ haloalkyl (such as -CHF₂, -CH₂F, -CH₂Cl, and -CF₃), and C₃-C₆ cycloalkyl (such as

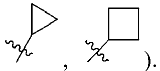

In some embodiments of the present disclosure, R₁₈ is selected from: CH₃, -CF₃, and

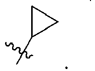

In an example of the present disclosure, R₁₅ and R₉, together with the intervening carbon atom, form 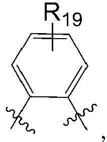 among which R₁₉ is selected from: H, D, halogen, -CN, -NO₂, -OH, C₁-C₆ alkyl, C₁-C₆ haloalkyl, and C₃-C₆ cycloalkyl.

In some embodiments of the present disclosure, R₁₉ is H.

Specifically, R₁₅ and R₁₆ may be independently selected from: H, D, halogen(e.g., F), -O(C₀-C₆ alkyl), -S(C₀-C₆ alkyl), -N(C₀-C₆ alkyl)(C₀-C₆ alkyl), C₁-C₆ alkyl, C₁-C₆ haloalkyl, C₁-C₆ hydroxyalkyl, and C₁-C₆ aminoalkyl; more specifically, R₁₅ and R₁₆ may be independently selected from: H, D, F, -OH, -CH₃, -CHF₂, -CH₂F, and -CF₃; in some embodiments of the present disclosure, R₁₅ and R₁₆ are both H; in some other embodiments of the present disclosure, R₁₅ is H, and R₁₆ is F; in some other embodiments of the present disclosure, R₁₅ is H, and R₁₆ is -OH.

In some embodiments of the present disclosure, W is a single bond, -CH₂-, 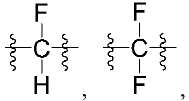 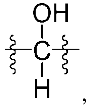 or S.

Specifically, R₁₀ is selected from: H, D, C₁-C₆ alkyl, C₁-C₆ haloalkyl, and C₃-C₆ cycloalkyl.

Specifically, 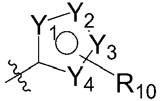 can be selected from:

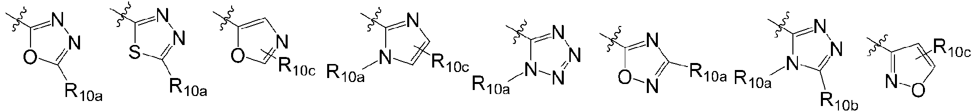

Specifically, R_{10c} is one or more independent substituents on the ring, and R₁₀ₐ to R_{10c} are each defined as described above for R₁₀.

More specifically, R₁₀ₐ, R_{10b} and R_{10c} may be independently selected from: H, D, C₁-C₆ alkyl, C₁-C₆ haloalkyl, and C₃-C₆ cycloalkyl; further specifically, R₁₀ₐ, R_{10b} and R_{10c} may be independently selected from: H, D, -CH₃, -CHF₂, -CH₂F, -CF₃,

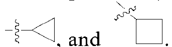

In an example of the present disclosure, 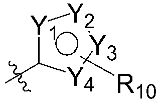 is

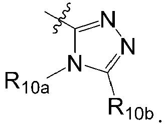

More specifically,

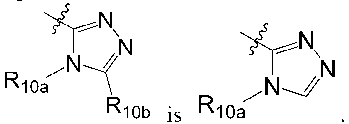

More specifically, R₁₀ₐ may be selected from: -CH₃, -CHF₂, -CH₂F, -CF₃, -CH₃. 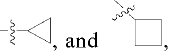 especially

In some embodiments of the present disclosure,

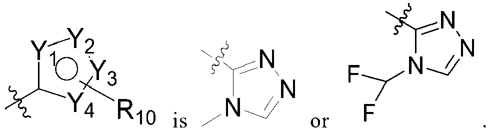

In some embodiments of the present disclosure, the compound has the following structure:

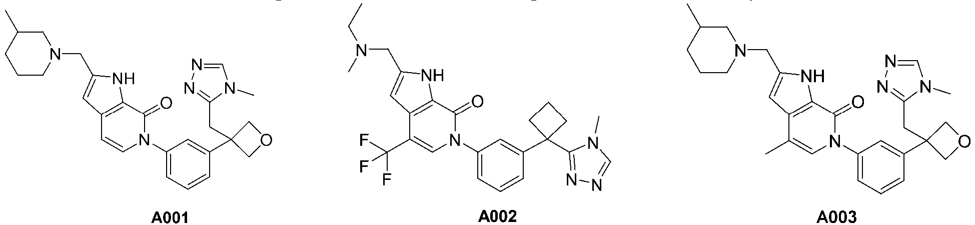

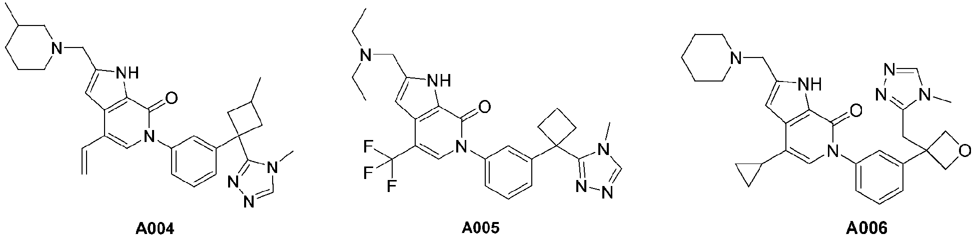

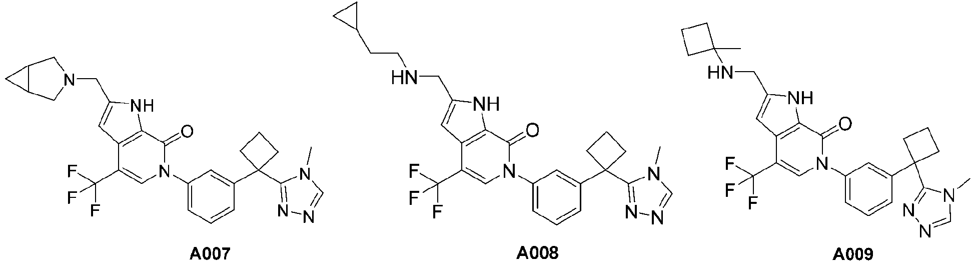

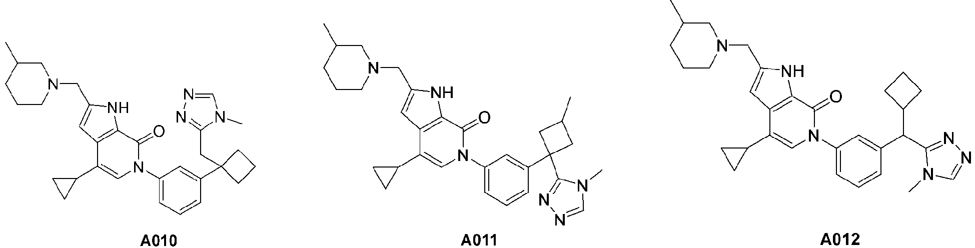

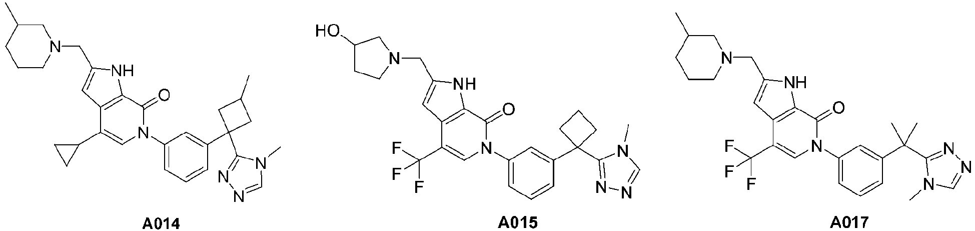

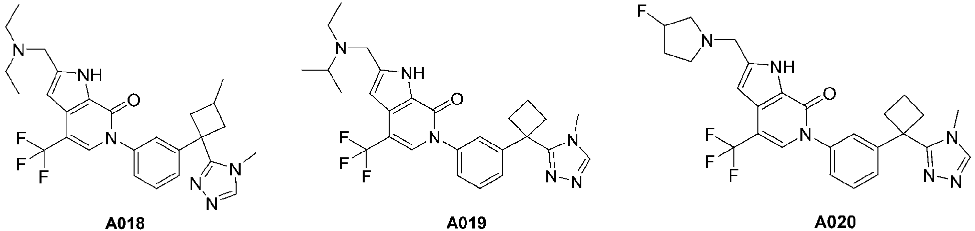

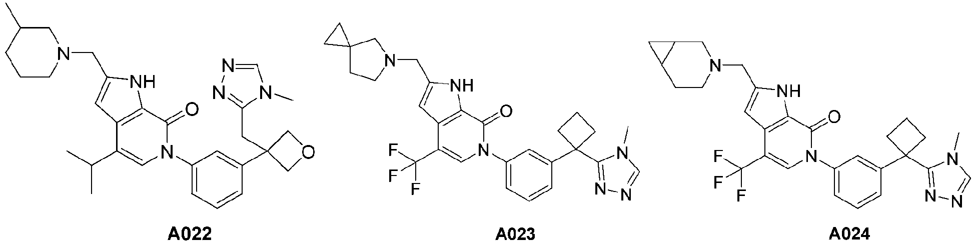

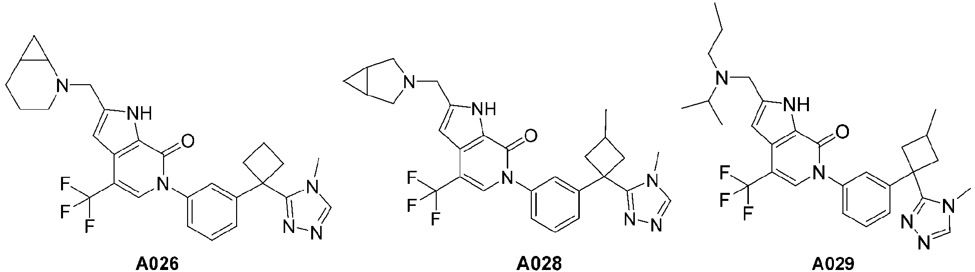

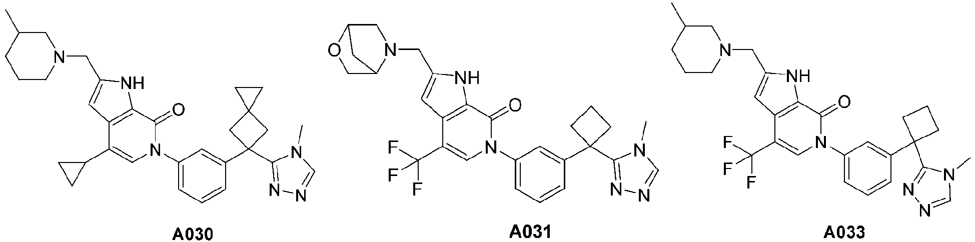

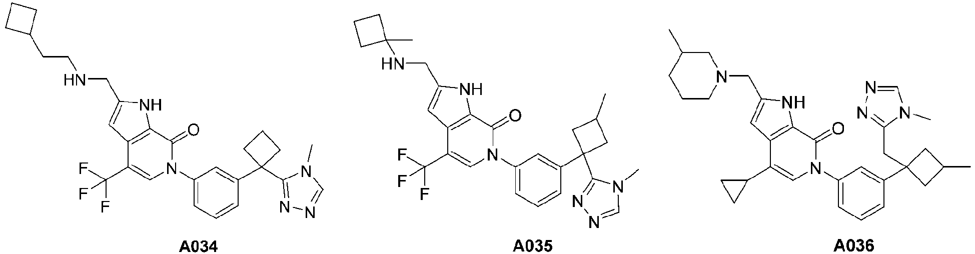

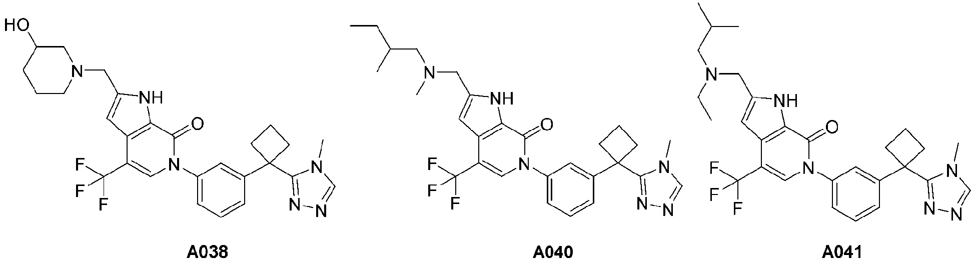

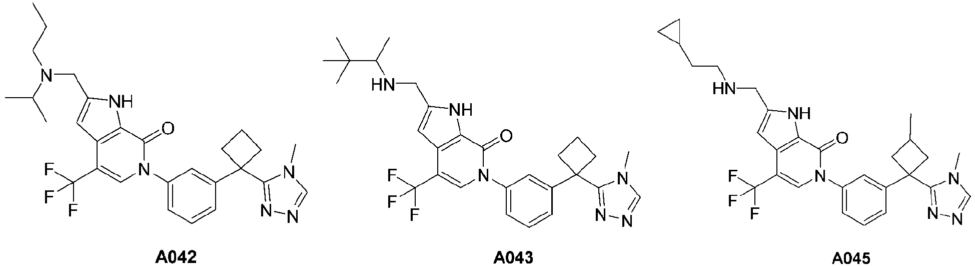

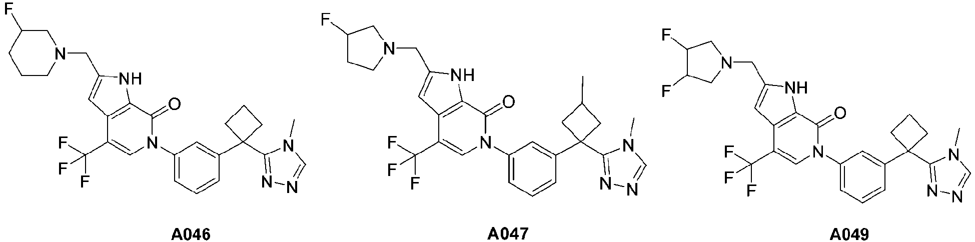

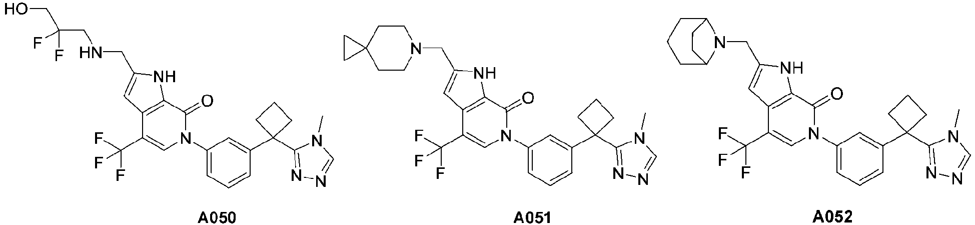

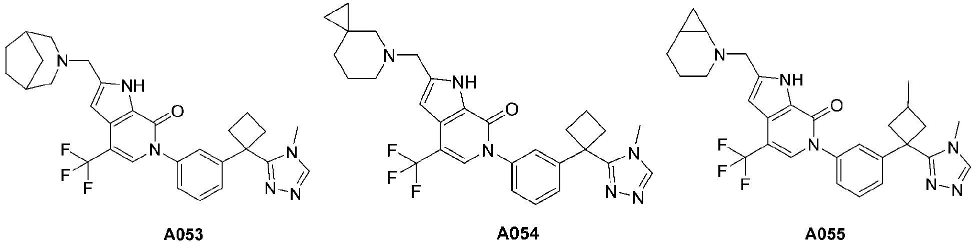

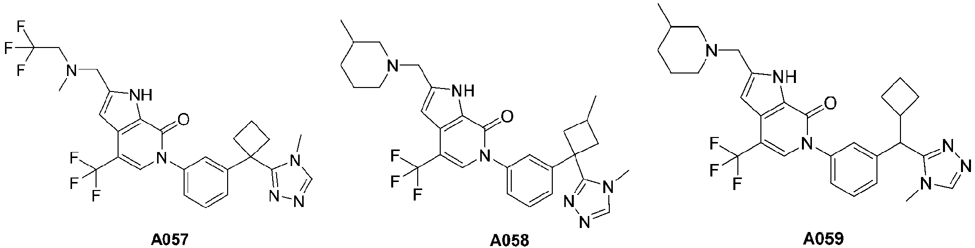

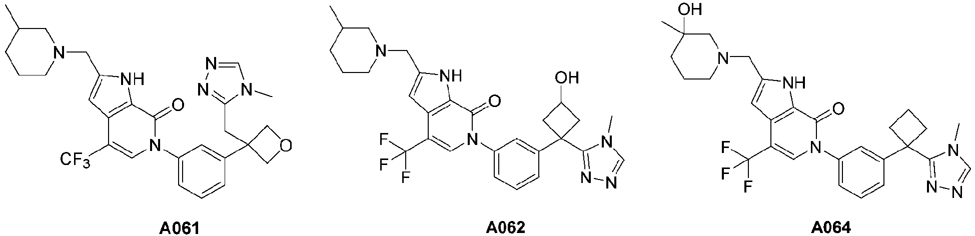

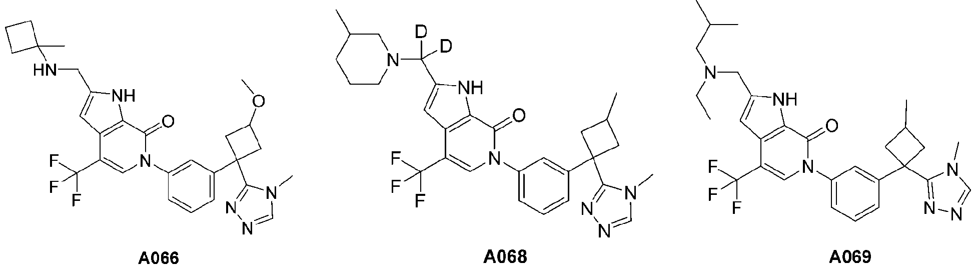

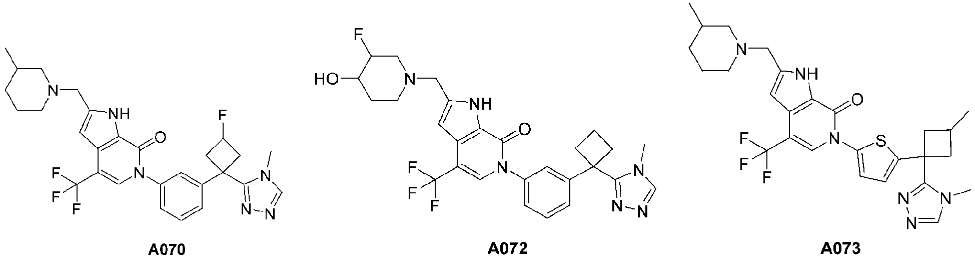

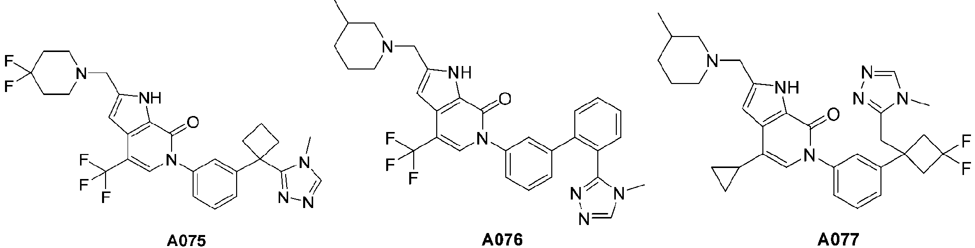

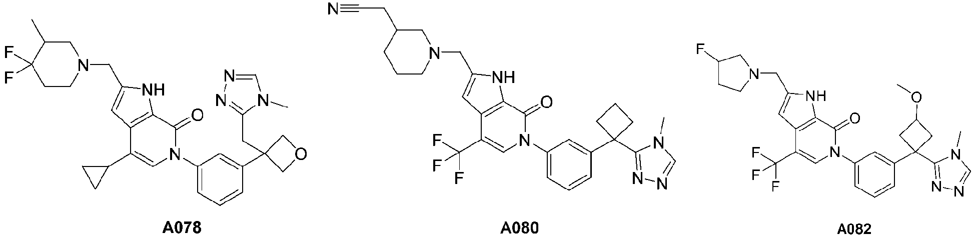

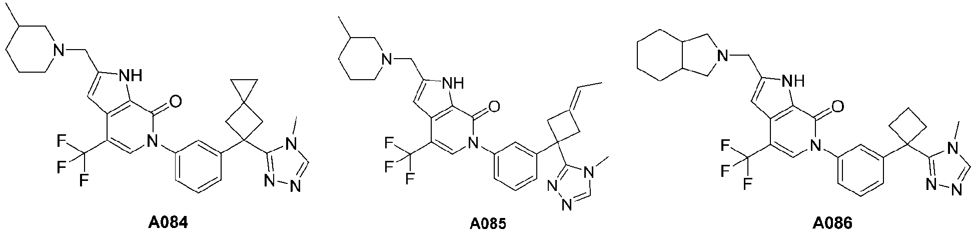

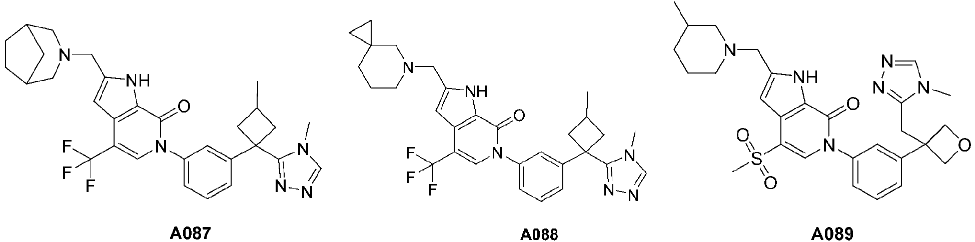

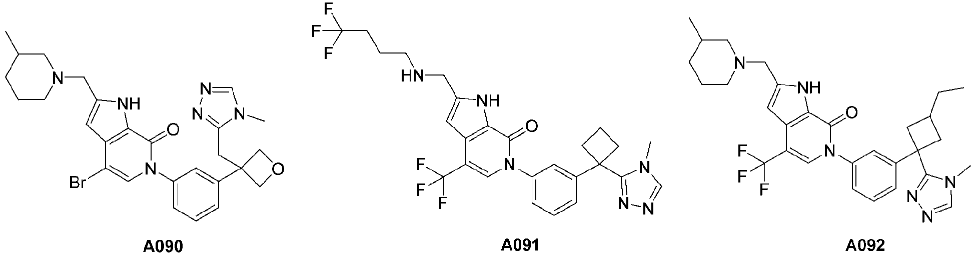

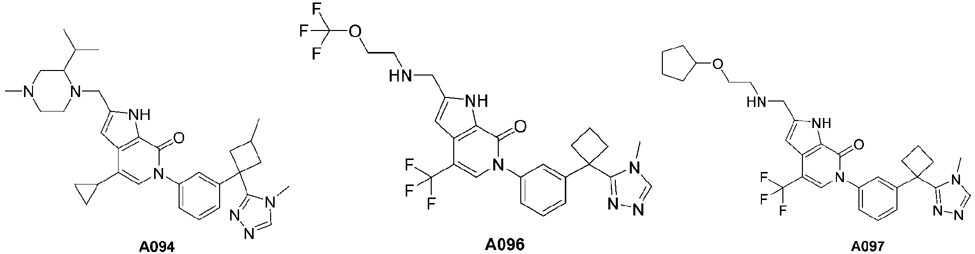

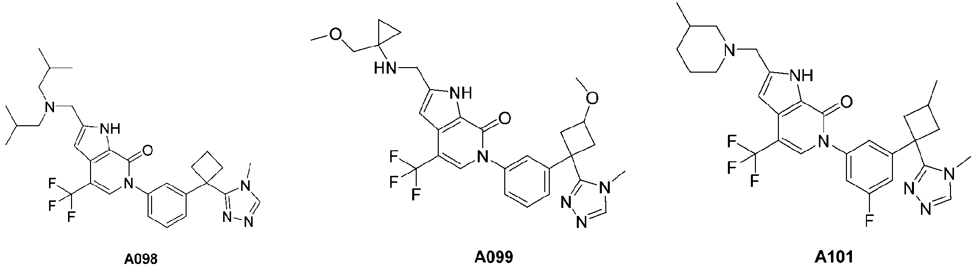

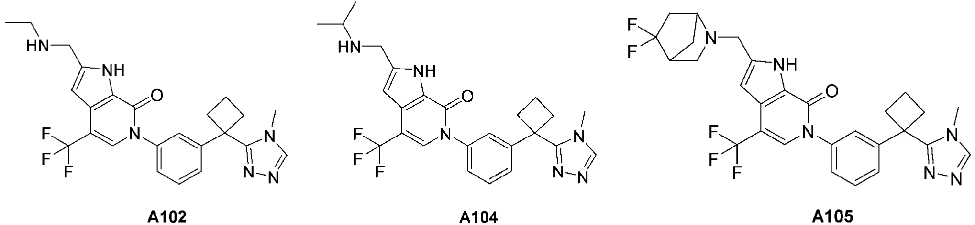

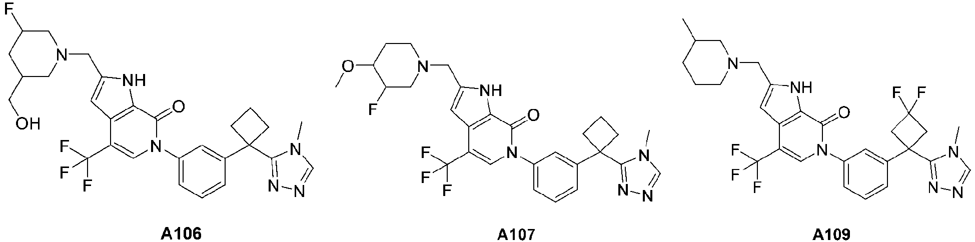

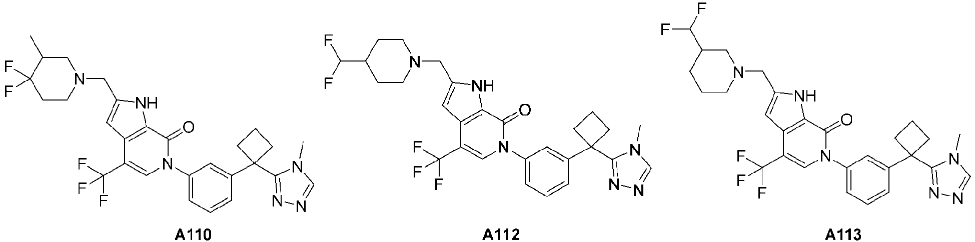

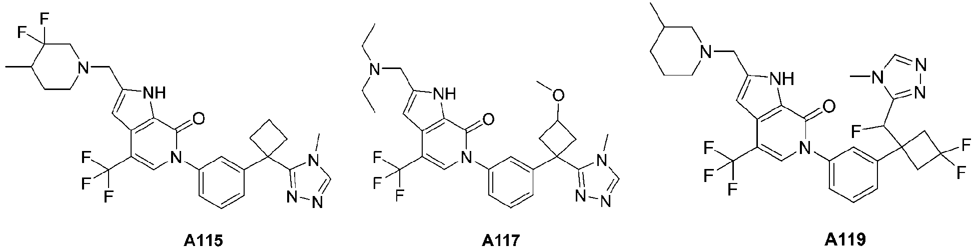

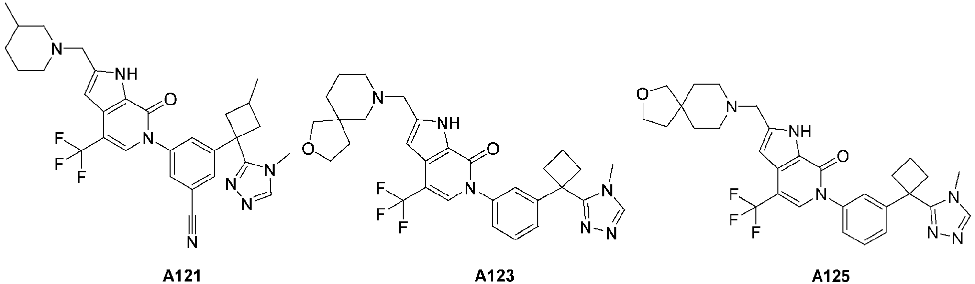

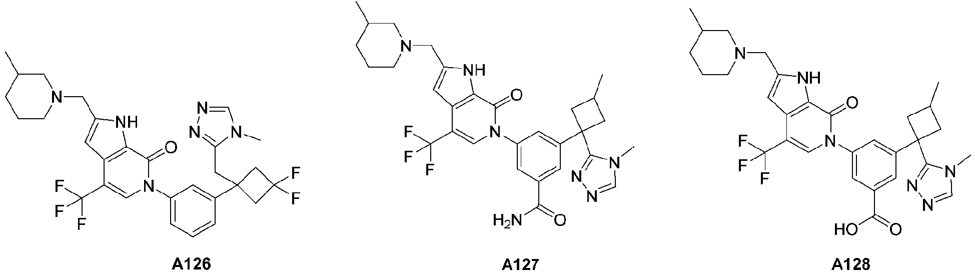

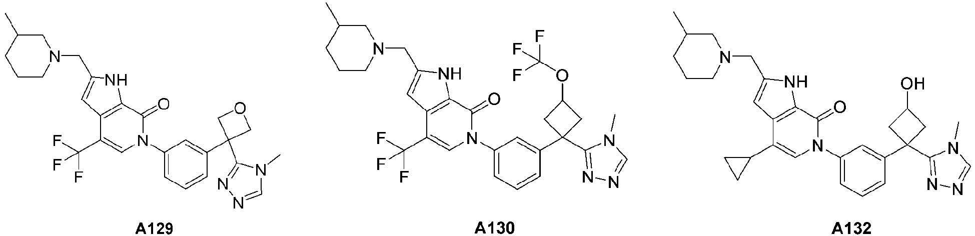

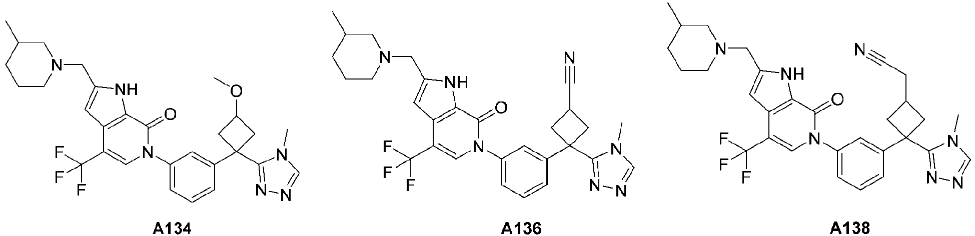

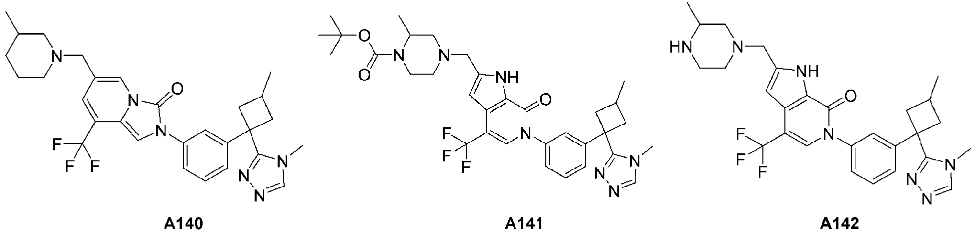

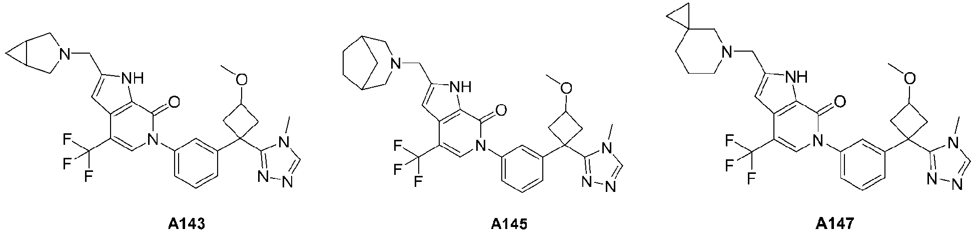

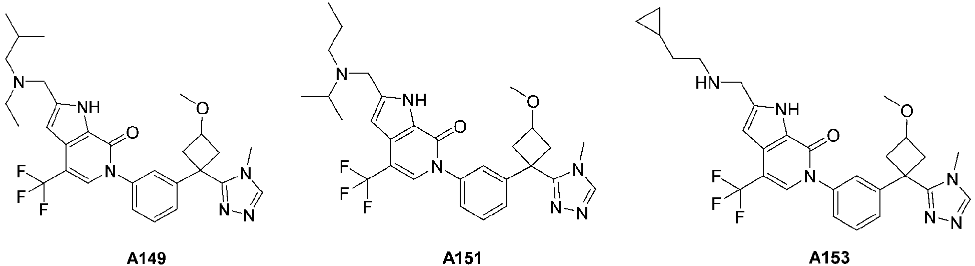

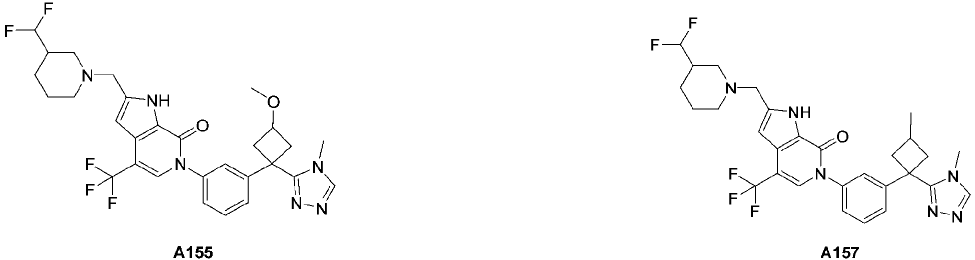

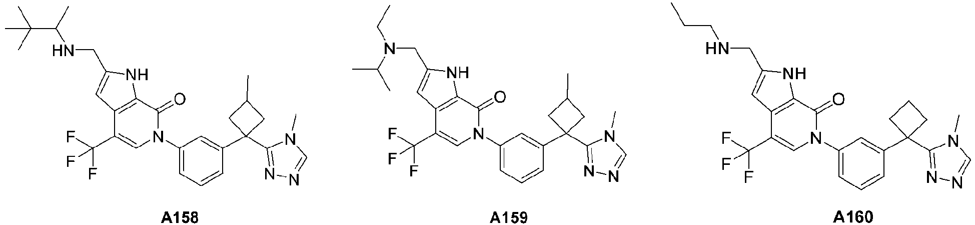

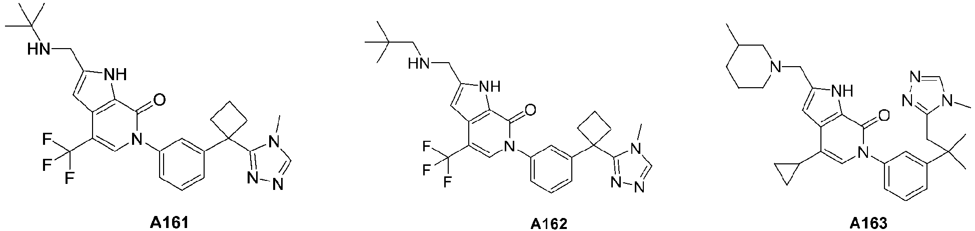

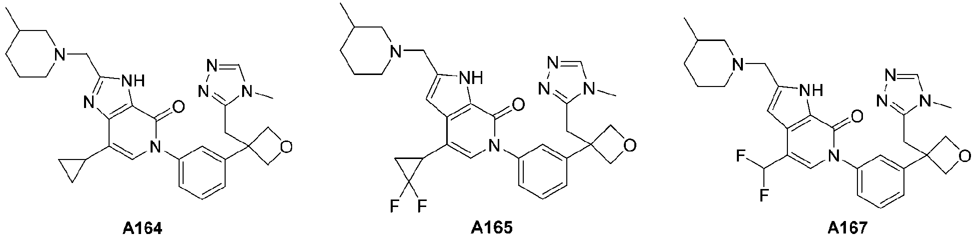

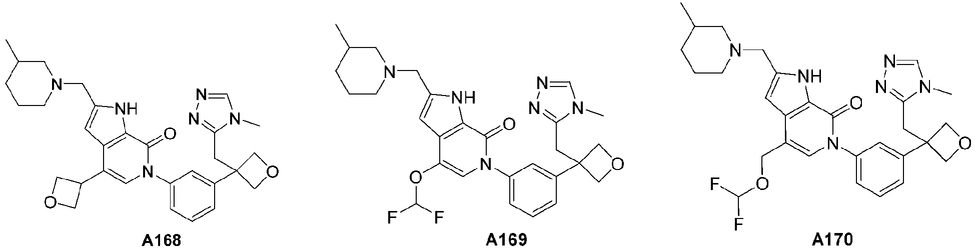

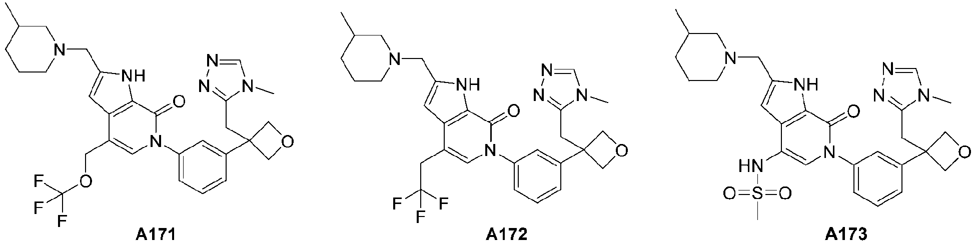

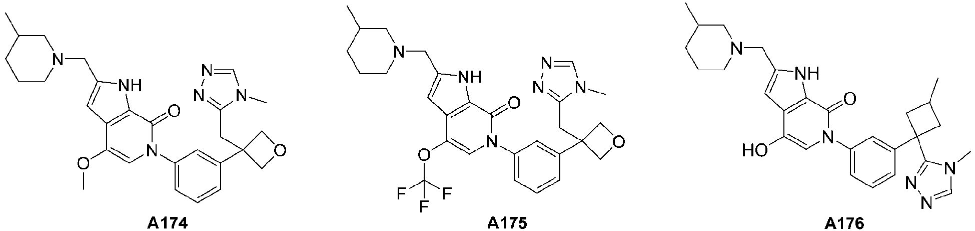

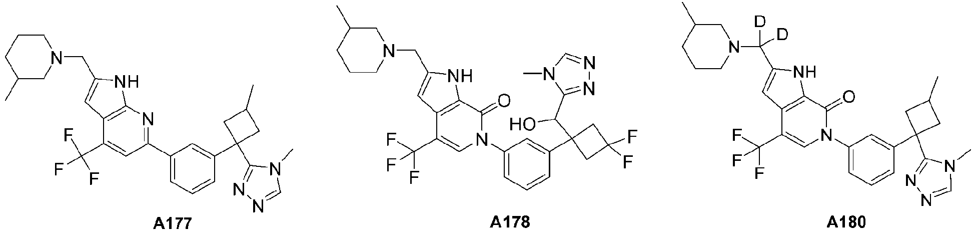

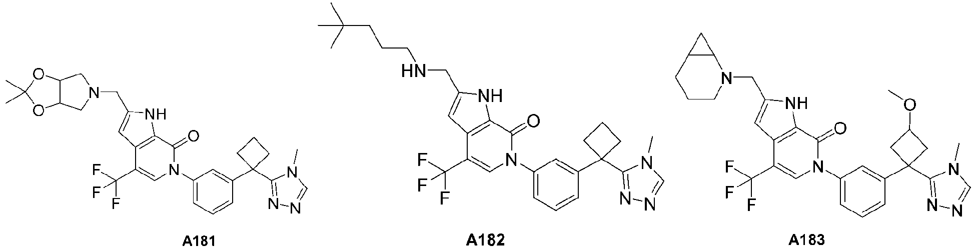

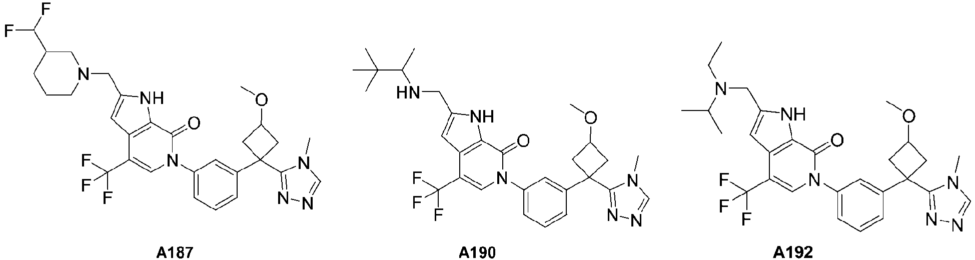

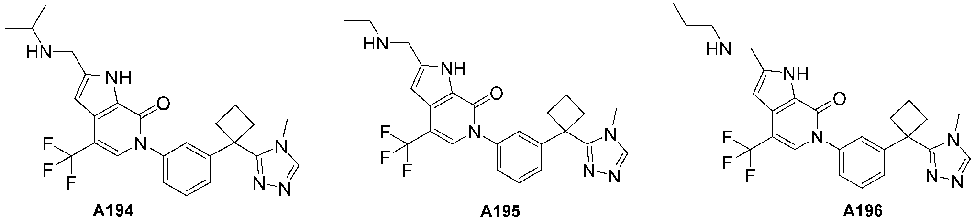

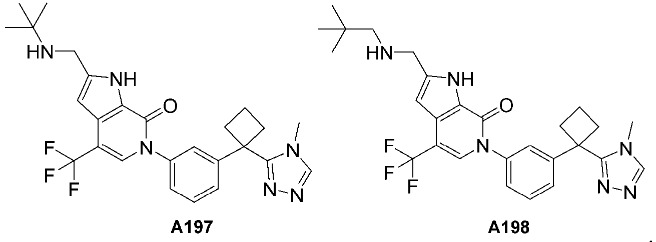

In a second aspect, the present disclosure provides the pharmaceutically acceptable salt, stereoisomer, ester, prodrug, solvate, or deuterated compound as described in the first aspect.

In some embodiments of the present disclosure, the stereoisomer has the following structure:

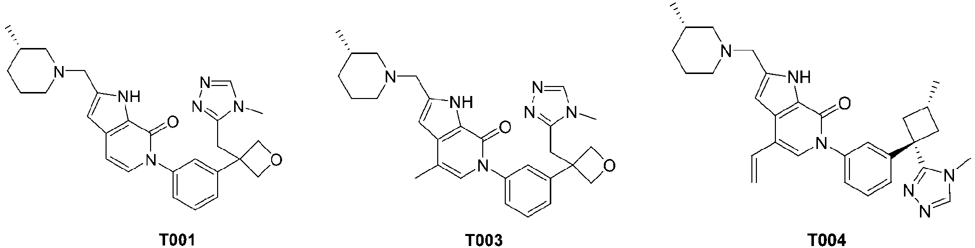

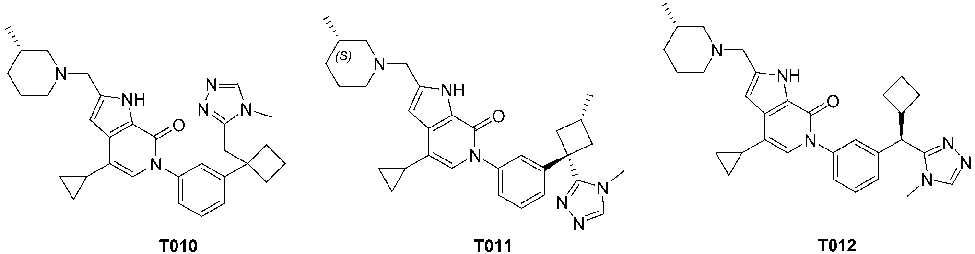

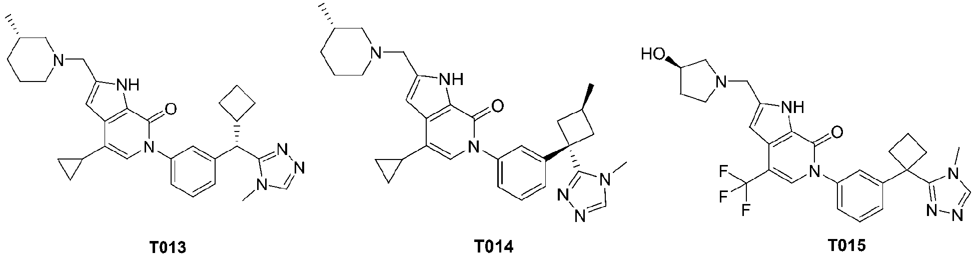

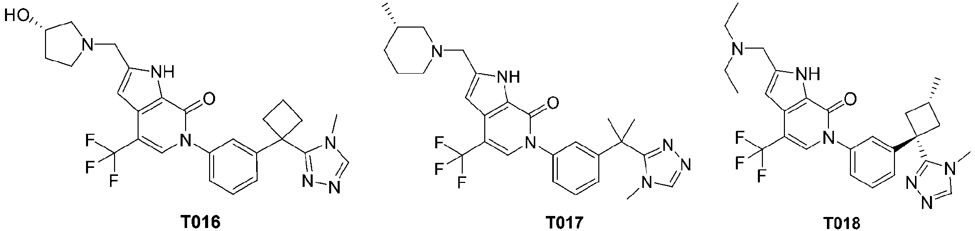

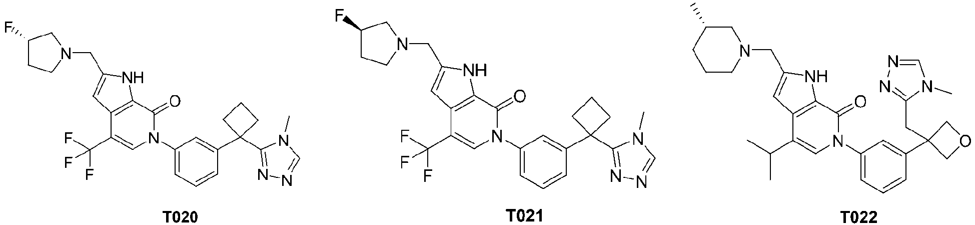

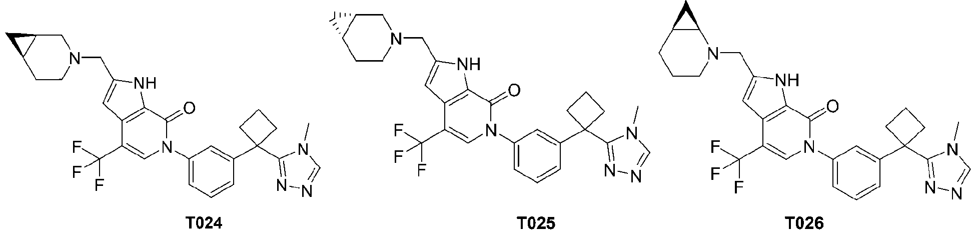

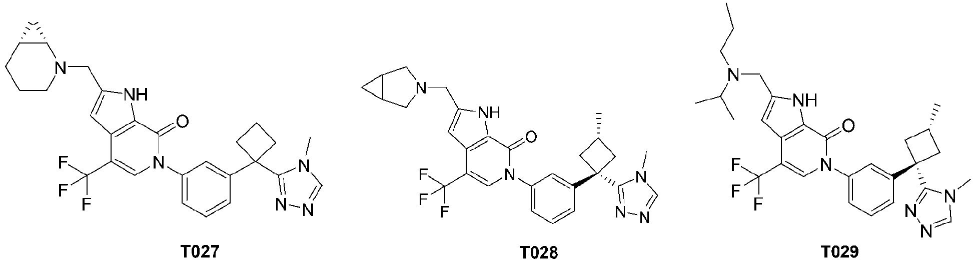

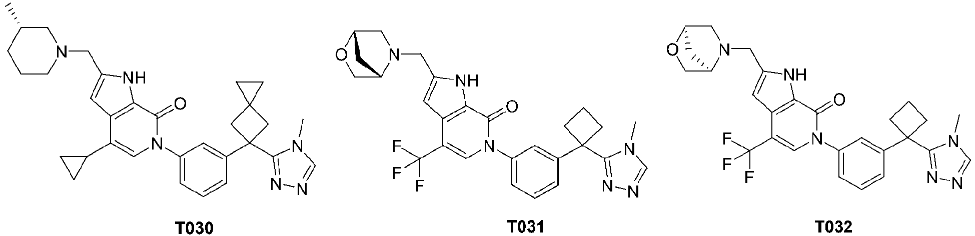

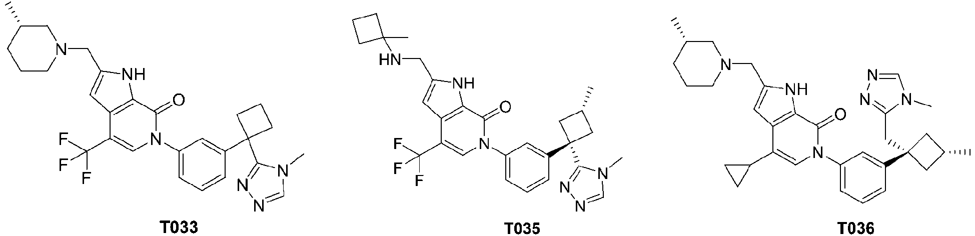

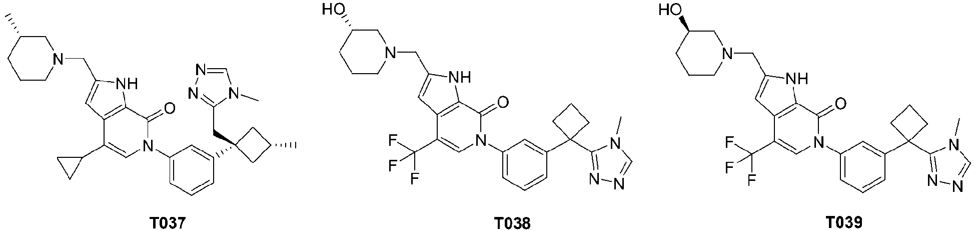

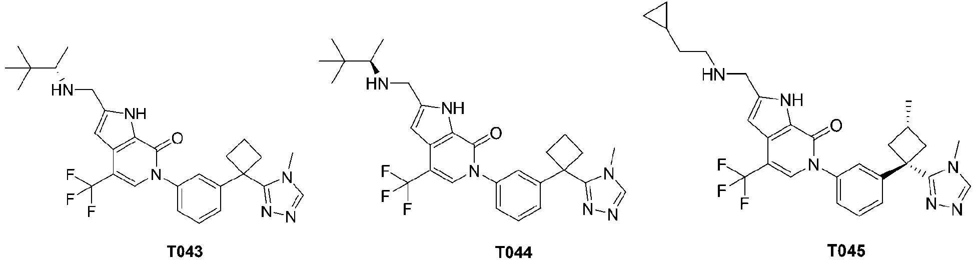

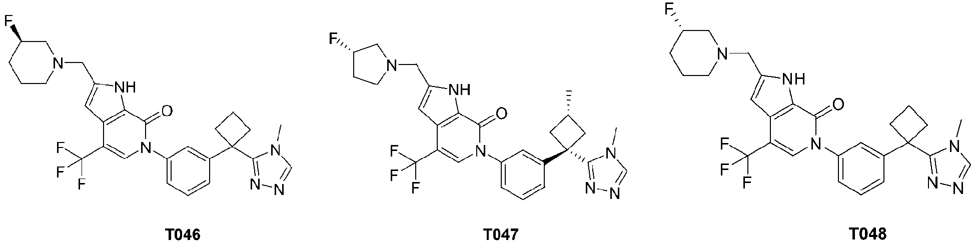

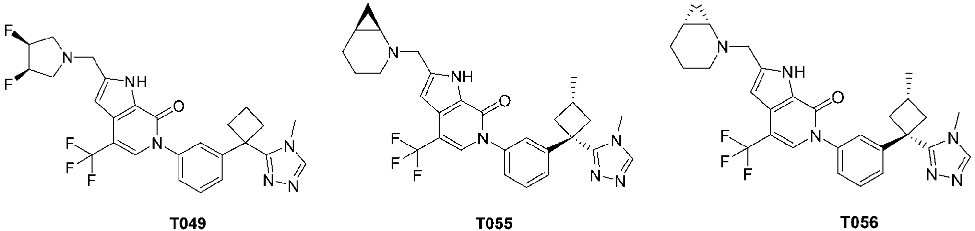

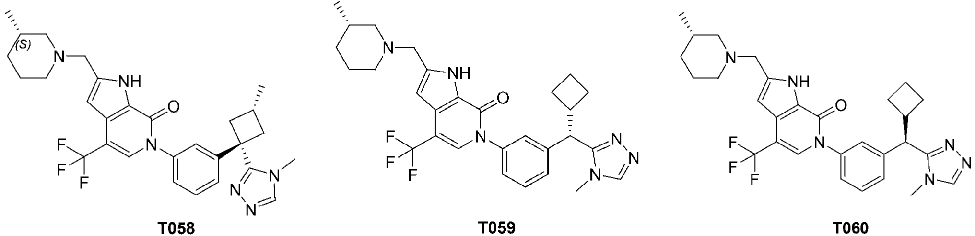

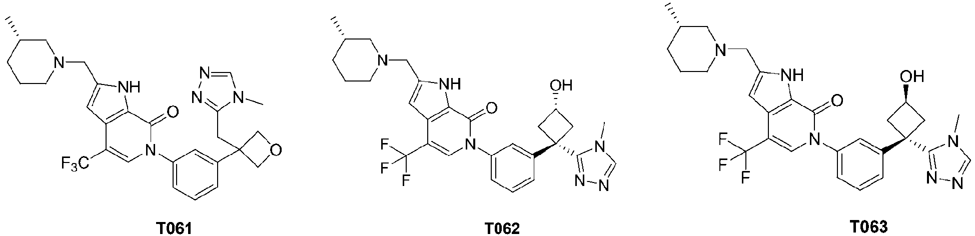

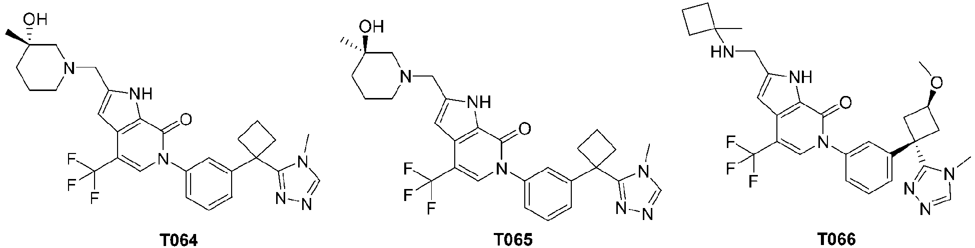

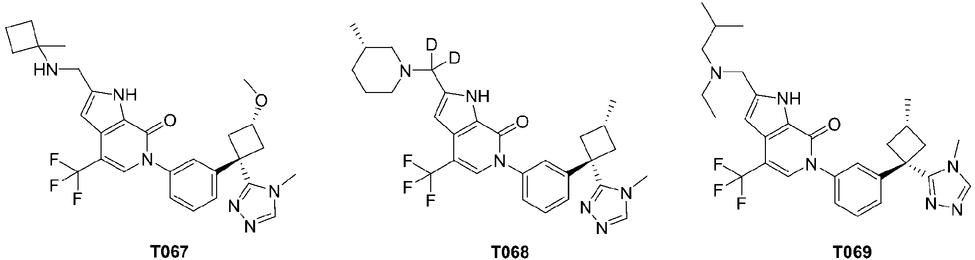

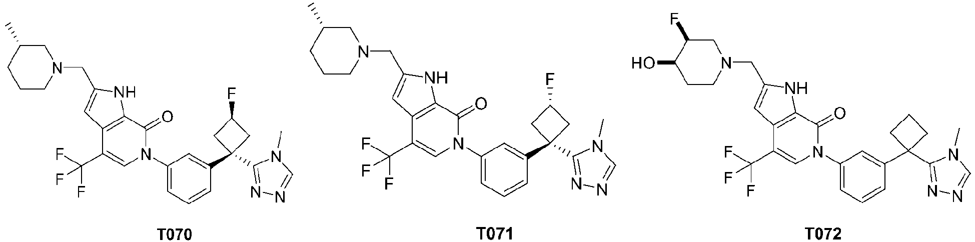

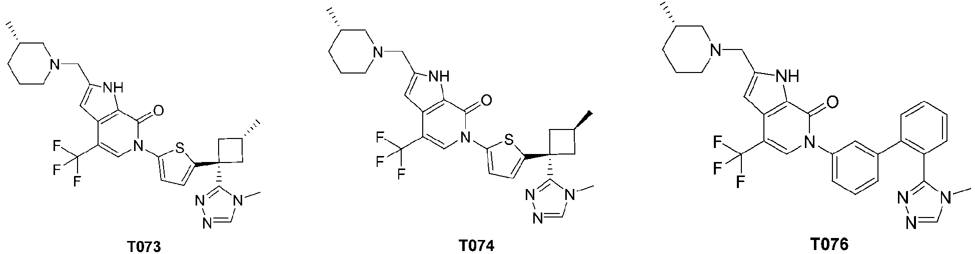

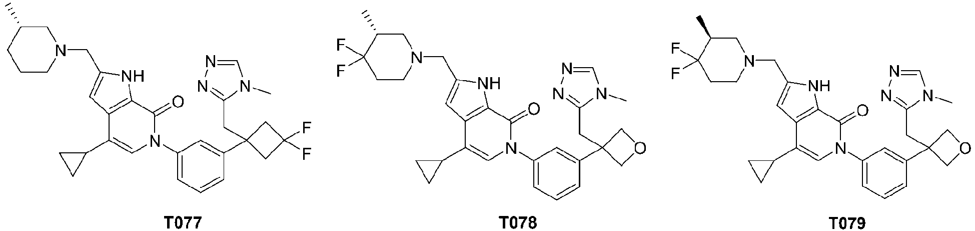

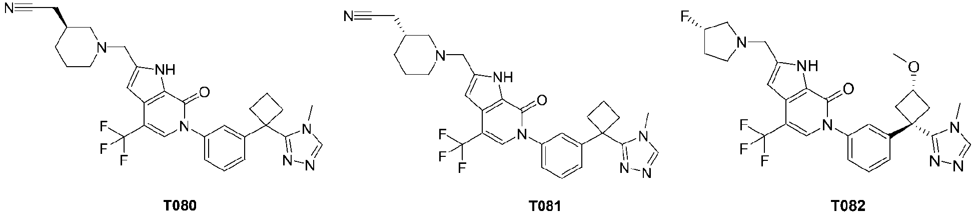

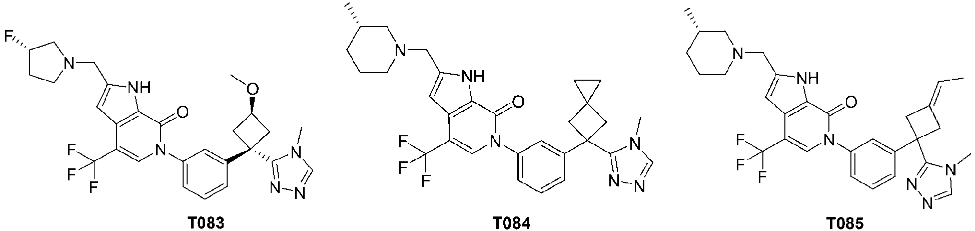

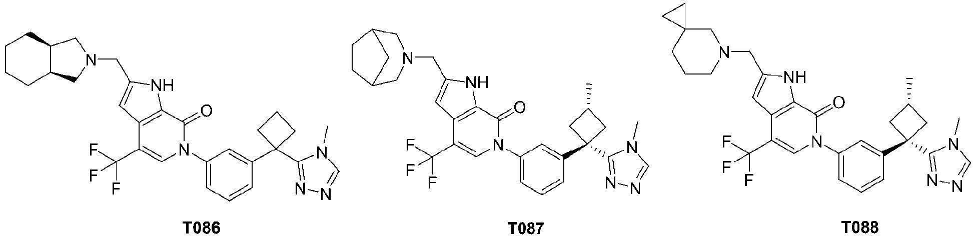

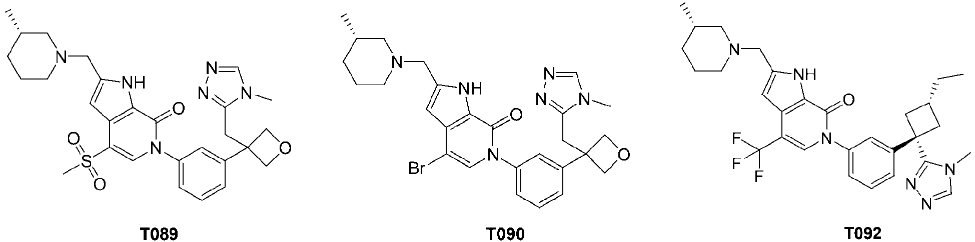

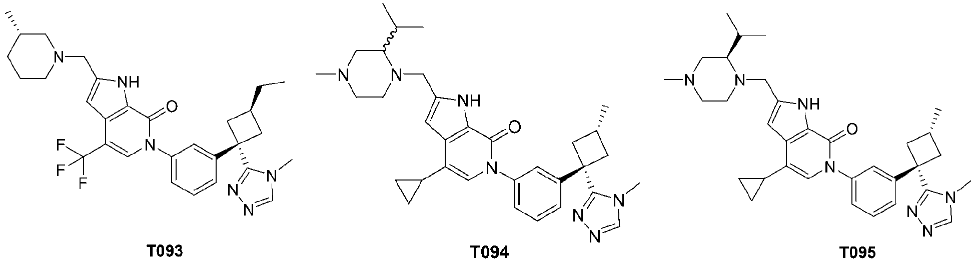

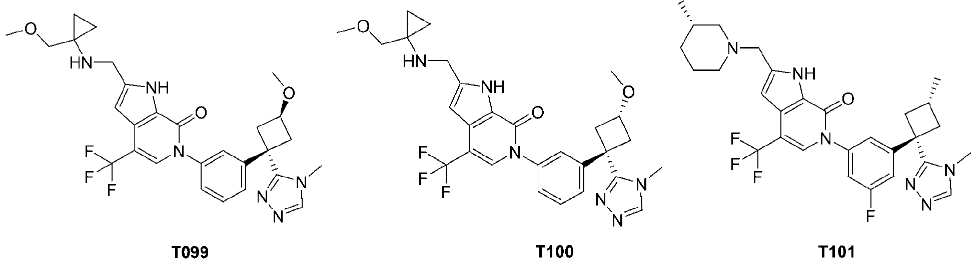

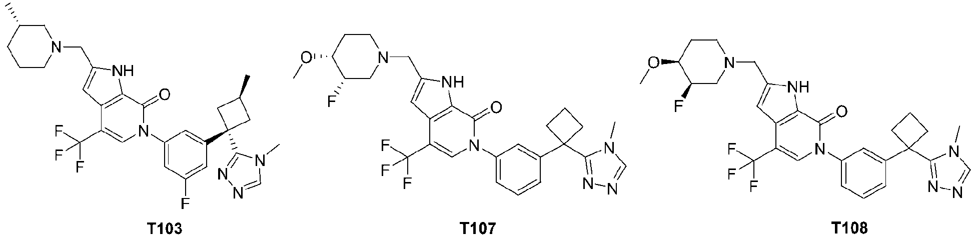

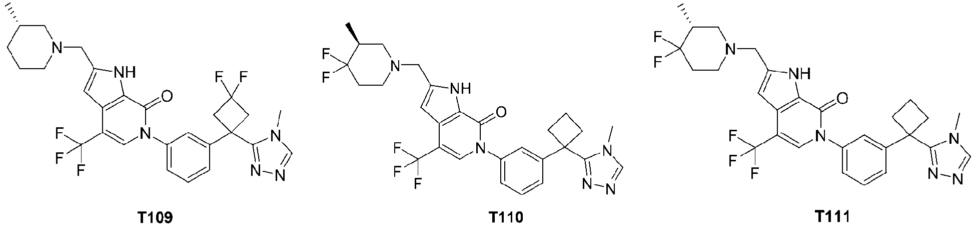

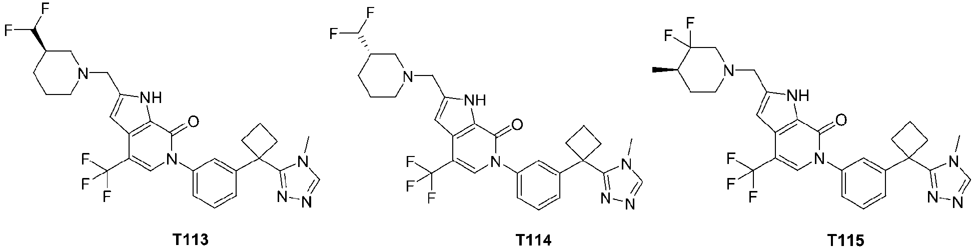

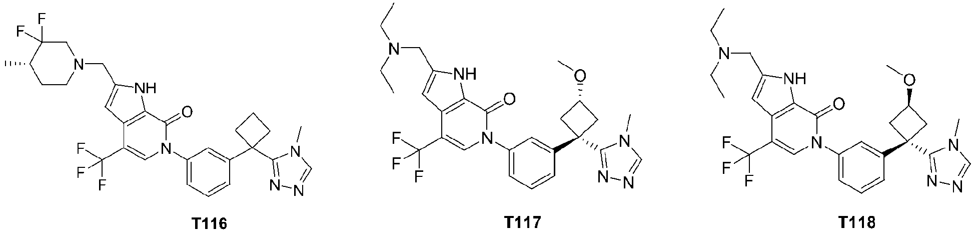

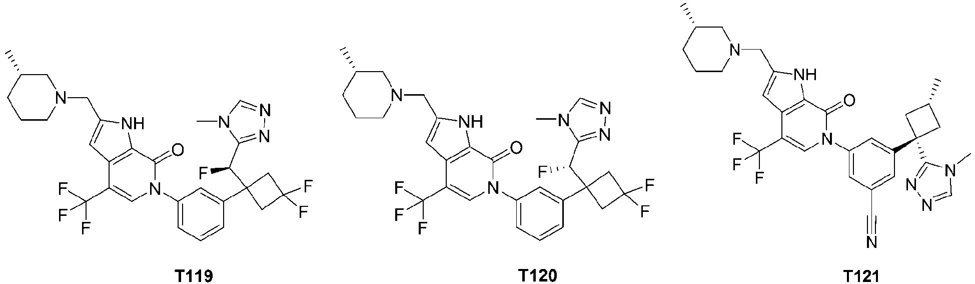

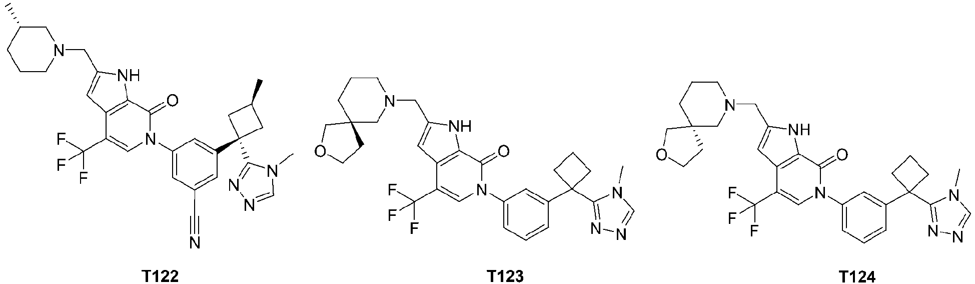

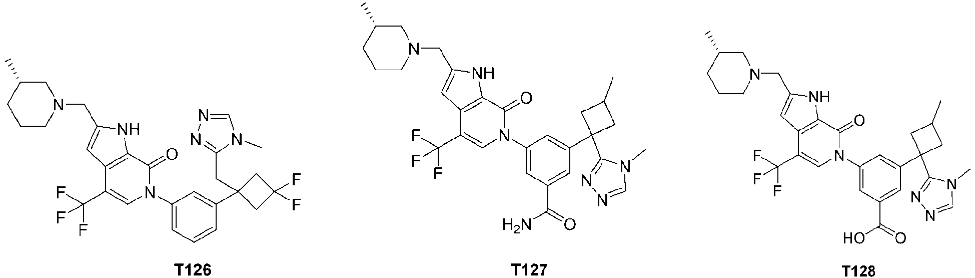

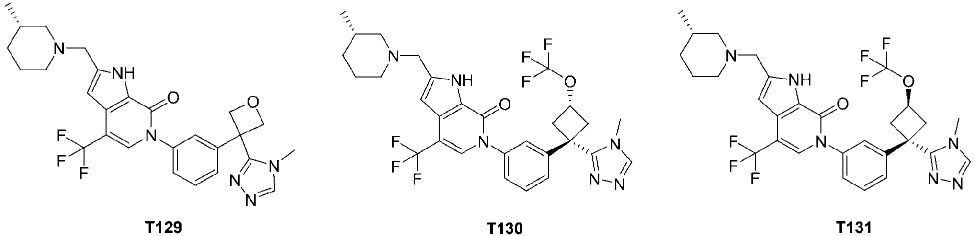

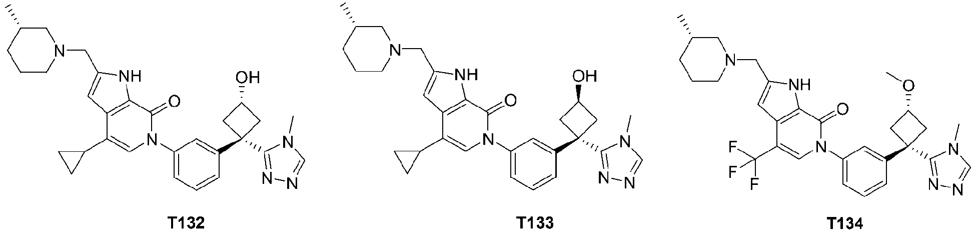

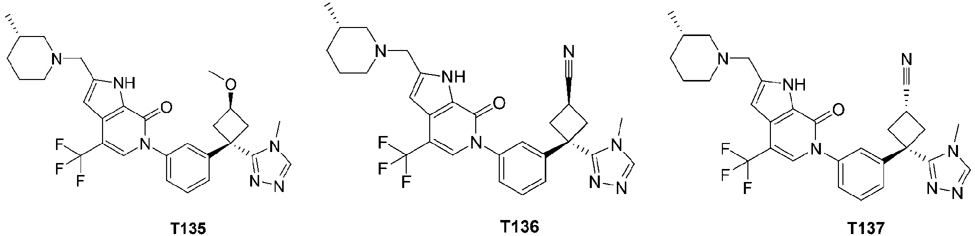

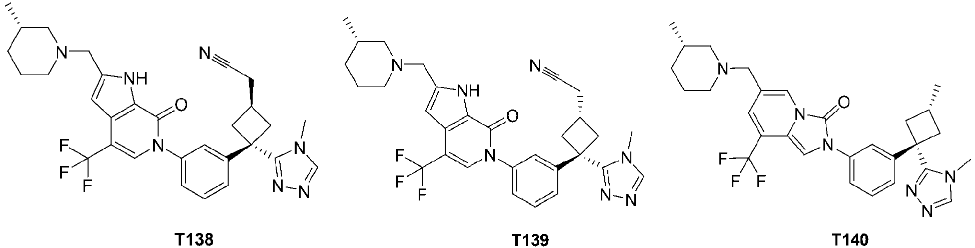

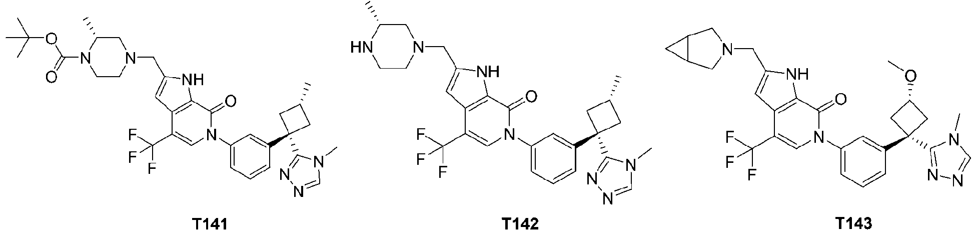

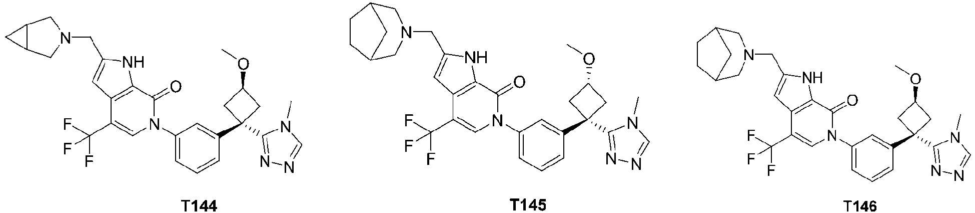

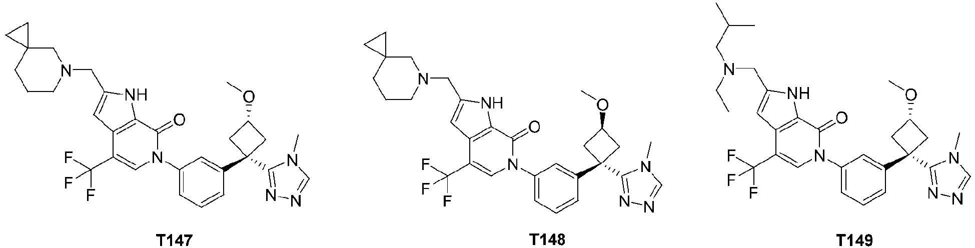

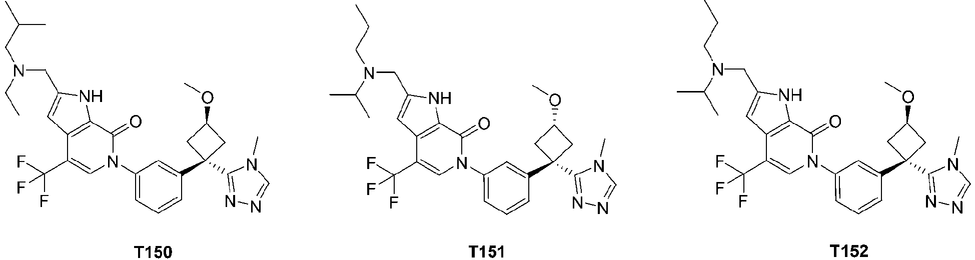

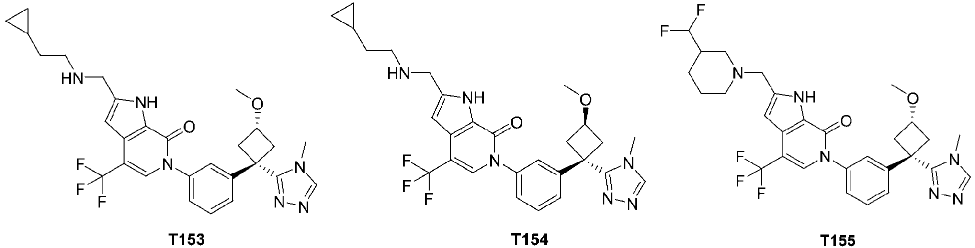

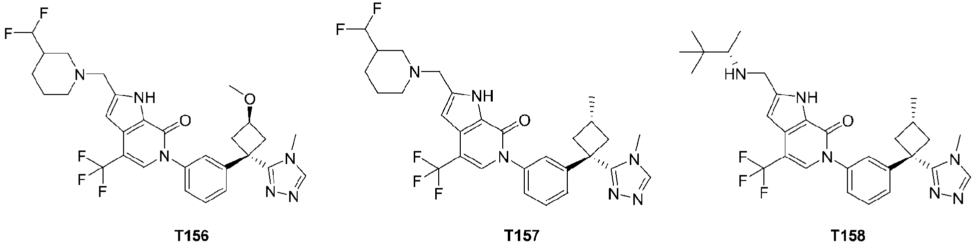

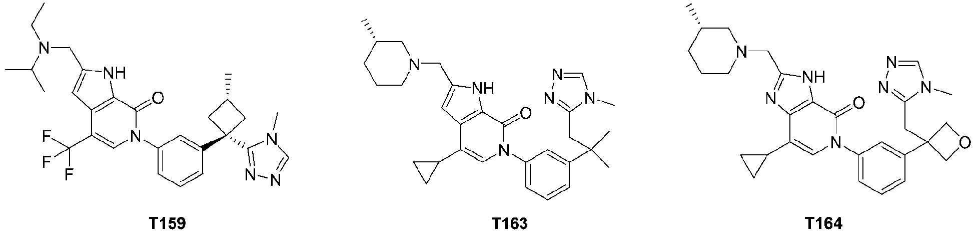

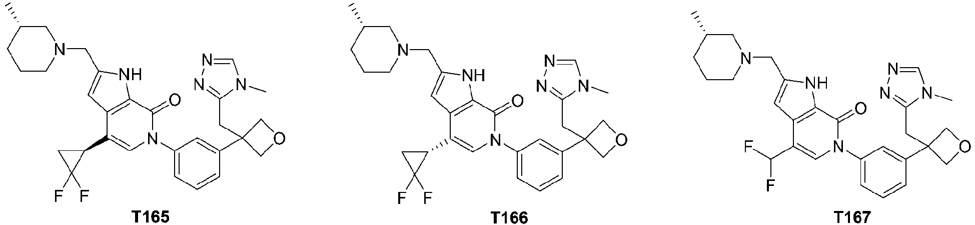

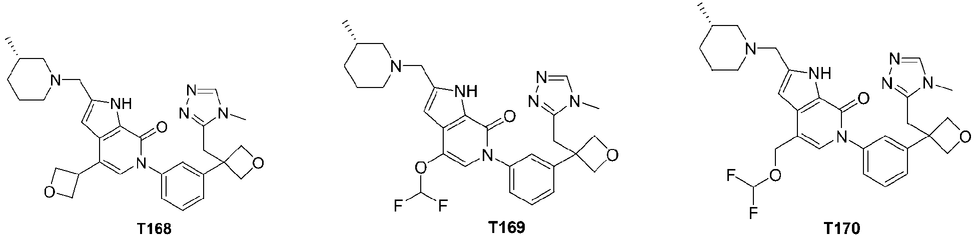

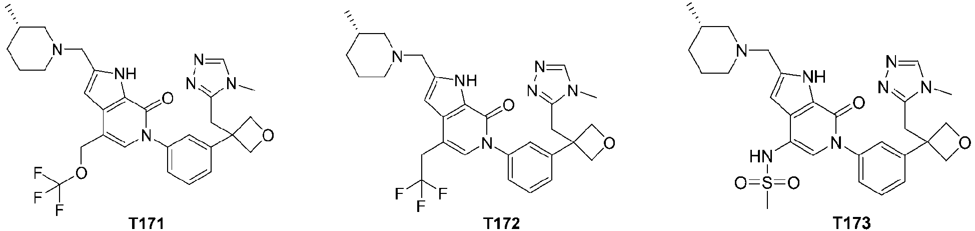

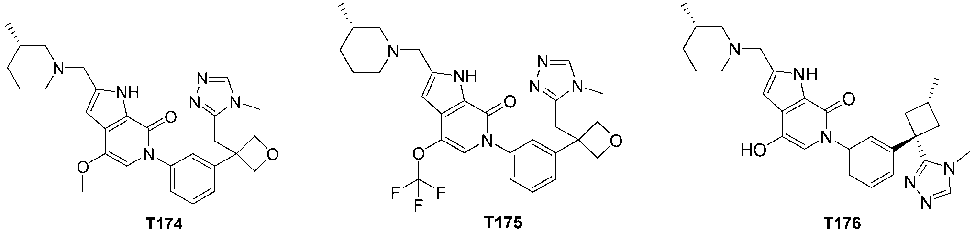

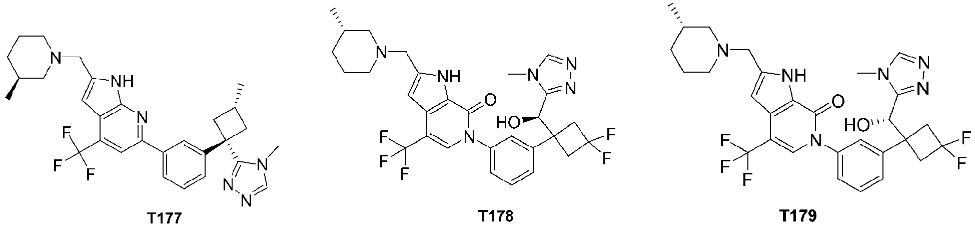

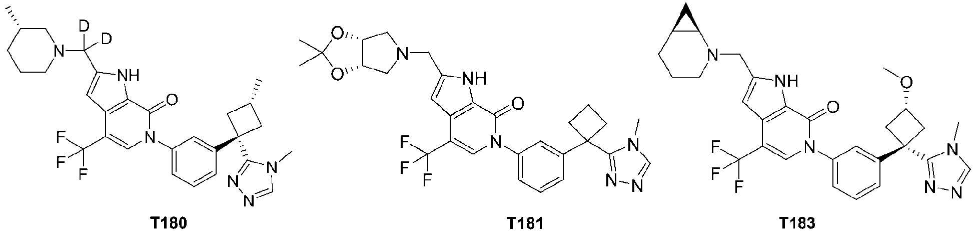

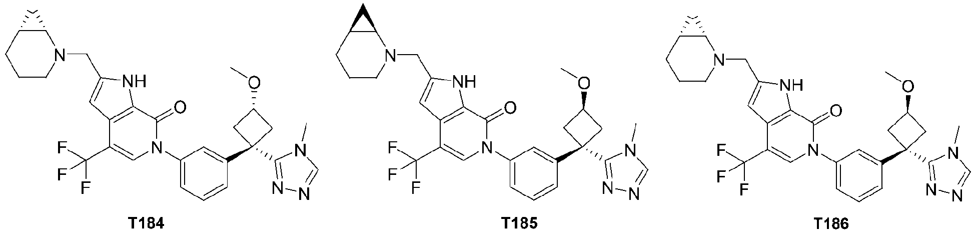

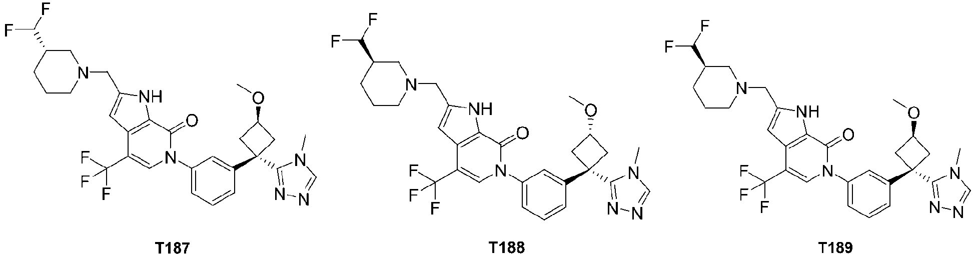

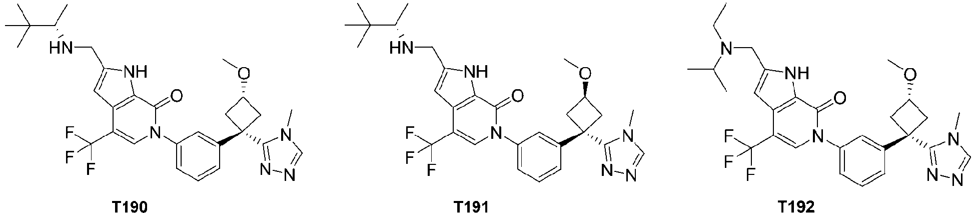

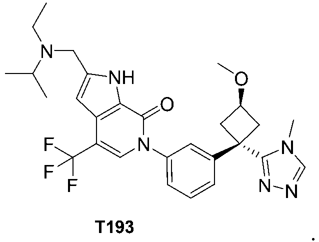

In a third aspect, the present disclosure provides a pharmaceutical composition, including the pharmaceutically acceptable salt, stereoisomer, ester, prodrug, solvate, or deuterated compound thereof as described in the first aspect, and one or more pharmaceutically acceptable excipients.

Specifically, the pharmaceutically acceptable excipients may be selected from one or more of: disintegrants, binders, lubricants, suspending agents, stabilizers, fillers, absorption enhancers, surfactants, flavoring agents, antioxidants, preservatives and the like.

Specifically, in the pharmaceutical composition, the compound or the pharmaceutically acceptable salt, stereoisomer, ester, prodrug, solvate, or deuterated compound thereof (a first active ingredient) as described in the first aspect may used alone, or used in combination with other type of active ingredients (a second active ingredient).

In some embodiments of the present disclosure, the other type of active ingredient is a serotonin receptor antagonist, which, when co-administered with the compound or the pharmaceutically acceptable salt, stereoisomer, ester, prodrug, solvate, or deuterated compound thereof as described in the first aspect, produces a synergistic effect.

In some embodiments of the present disclosure, the serotonin receptor antagonist is a serotonin inhibitor, such as ondansetron, granisetron, palonosetron, or dolasetron.

In some embodiments, the serotonin receptor antagonist is ondansetron.

An 8 mg dose of ondansetron may be administered at least 30 minutes or one hour prior to administration of the first active ingredient.

In some embodiments, a 16 mg dose of ondansetron may be administered prior to administration of the first active ingredient.

In some embodiments, a 24 mg dose of ondansetron may be administered prior to administration of the first active ingredient.

In some embodiments, the serotonin receptor antagonist is granisetron.

A 1 mg dose of granisetron may be administered one hour prior to administration of the first active ingredient.

In some embodiments, a 2 mg dose of granisetron may be administered prior to administration of the first active ingredient.

In some embodiments, the serotonin receptor antagonist is dolasetron.

A 100 mg dose of dolasetron may be administered at least one hour prior to administration of the first active ingredient.

In some embodiments, a 200 mg dose of dolasetron may be administered prior to administration of the first active ingredient.

In some embodiments, the serotonin receptor antagonist is palonosetron.

A 0.25 mg dose of palonosetron may be administered at least 30 minutes prior to administration of the first active ingredient.

In some embodiments, a 0.5 mg dose of palonosetron may be administered at least 30 minutes prior to administration of the first active ingredient.

In some embodiments, a 0.75 mg dose of palonosetron may be administered at least 30 minutes prior to administration of the first active ingredient.

In some embodiments of the present disclosure, the other type of active ingredient is an oncolytic virus.

Specifically, for combination use, the compound or the pharmaceutically acceptable salt, stereoisomer, ester, prodrug, solvate, or deuterated derivative thereof as described in the first aspect, and the second active ingredient may be administered in the same dosage form or in separate dosage forms. The two may be administered simultaneously, separately, or sequentially.

Specifically, the pharmaceutical composition may be administered via any suitable route, such as enteral or parenteral administration (such as intravenous, intramuscular, subcutaneous, intraperitoneal, intranasal, intradermal, infusion, intracerebral, or rectal routes.).

Specifically, the pharmaceutical composition may be in any suitable dosage form. For example, enteral dosage forms include, but are not limited to, tablets, pellets, powders, granules, capsules, lozenges, syrups, liquids, emulsions, suspensions, and the like. Parenteral dosage forms include, for example, injectable formulations such as injections (e.g., for subcutaneous, intravenous, intramuscular, or intraperitoneal administration); respiratory formulations such as sprays, aerosols, and dry powders; transdermal formulations such as topical solutions, lotions, ointments, plasters, pastes, patches, and the like; mucosal formulations such as eye drops, ophthalmic ointments, nasal drops, mouthwashes, and sublingual tablets; and cavity administration forms such as suppositories, aerosols, effervescent tablets, drops, and pellets, which may be used for vaginal, urethral, nasal, or aural delivery.

Specifically, the various dosage forms of the pharmaceutical composition may be prepared by conventional methods known in the pharmaceutical field. For example, the active ingredient may be mixed with one or more pharmaceutically acceptable excipients and then formulated into the desired dosage form.

Specifically, in the pharmaceutical composition, the compound, or the pharmaceutically acceptable salt, stereoisomer, ester, prodrug, or solvate thereof as described in the first aspect, may constitute from 0.1% to 99.5% by weight, for example, 0.5%, 1%, 5%, 10%, 20%, 30%, 40%, 50%, 60%, 70%, 80%, 90%, 95%, or 99%.

Specifically, in the pharmaceutical composition, the amount of the compound, or the pharmaceutically acceptable salt, stereoisomer, ester, prodrug, or solvate thereof as described in the first aspect is a therapeutically effective amount, which may range or be adjusted between 0.1 mg and 1000 mg, 0.1 mg and 900 mg, 0.1 mg and 800 mg, 0.1 mg and 750 mg, 0.1 mg and 700 mg, 0.1 mg and 600 mg, 0.1 mg and 500 mg, 0.1 mg and 400 mg, 0.1 mg and 300 mg, 0.1 mg and 250 mg, 0.1 mg and 200 mg, 0.1 mg and 100 mg, 0.1 mg and 50 mg, 0.1 mg and 25 mg, 0.1 mg and 20 mg, 0.1 mg and 15 mg, 0.1 mg and 10 mg, 0.1 mg and 7.5 mg, 0.1 mg and 5 mg, 0.1 mg and 2.5 mg, 0.25 mg and 20 mg, 0.25 mg and 15 mg, 0.25 mg and 12 mg, 0.25 mg and 10 mg, 0.25 mg and 7.5 mg, 0.25 mg and 5 mg, 0.25 mg and 2.5 mg, 0.5 mg and 20 mg, 0.5 mg and 15 mg, 0.5 mg and 12 mg, 0.5 mg and 10 mg, 0.5 mg and 7.5 mg, 0.5 mg and 5 mg, 0.5 mg and 2.5 mg, 1 mg and 20 mg, 1 mg and 15 mg, 1 mg and 12 mg, 1 mg and 10 mg, 1 mg and 7.5 mg, 1 mg and 5 mg, or 1 mg and 2.5 mg, depending on the specific application and potency of the active component.

If the pharmaceutical composition further includes a second active ingredient, and the amount of the second active ingredient is a therapeutically effective amount, the amount may range or be adjusted between 0.1 mg and 1000 mg, 0.1 mg and 900 mg, 0.1 mg and 800 mg, 0.1 mg and 750 mg, 0.1 mg and 700 mg, 0.1 mg and 600 mg, 0.1 mg and 500 mg, 0.1 mg and 400 mg, 0.1 mg and 300 mg, 0.1 mg and 250 mg, 0.1 mg and 200 mg, 0.1 mg and 100 mg, 0.1 mg and 50 mg, 0.1 mg and 25 mg, 0.1 mg and 20 mg, 0.1 mg and 15 mg, 0.1 mg and 10 mg, 0.1 mg and 7.5 mg, 0.1 mg and 5 mg, 0.1 mg and 2.5 mg, 0.25 mg and 20 mg, 0.25 mg and 15 mg, 0.25 mg and 12 mg, 0.25 mg and 10 mg, 0.25 mg and 7.5 mg, 0.25 mg and 5 mg, 0.25 mg and 2.5 mg, 0.5 mg and 20 mg, 0.5 mg and 15 mg, 0.5 mg and 12 mg, 0.5 mg and 10 mg, 0.5 mg and 7.5 mg, 0.5 mg and 5 mg, 0.5 mg and 2.5 mg, 1 mg and 20 mg, 1 mg and 15 mg, 1 mg and 12 mg, 1 mg and 10 mg, 1 mg and 7.5 mg, 1 mg and 5 mg, or 1 mg and 2.5 mg.

In a fourth aspect, the present disclosure provides a Cbl-b inhibitor, including the pharmaceutically acceptable salt, stereoisomer, ester, prodrug, solvate, or deuterated compound thereof as described in the first aspect.

Specifically, the Cbl-b inhibitor exhibits inhibitory effects on Cbl-b, including but not limited to inhibiting Cbl-b protein activity..

In a fifth aspect, the present disclosure provides a use of the compound or the pharmaceutically acceptable salt, stereoisomer, ester, prodrug, solvate, or deuterated compound thereof as described in the first aspect, in the preparation of a drug for preventing and/or treating a disease related to Cbl-b activity.

Specifically, the diseases related to Cbl-b activity are those that may benefit from prevention and/or treatment by inhibiting Cbl-b, such as autoimmune diseases, inflammatory diseases, tumors, diseases caused by pathogen infections, or diseases associated with pathogen infections.

Specifically, the autoimmune disease includes, but is not limited to, organ-specific autoimmune disease and systemic autoimmune disease, such as Achalasia, Addison's disease, adult Still's disease, agammaglobulinemia, alopecia areata, amyloidosis, ankylosing spondylitis, anti-glomerular basement membrane nephritis, antiphospholipid syndrome, autoimmune angioedema, autoimmune autonomic dysfunction, autoimmune encephalomyelitis, autoimmune hepatitis, autoimmune inner ear disease, autoimmune myocarditis, autoimmune oophoritis, autoimmune orchitis, autoimmune pancreatitis, autoimmune retinopathy, autoimmune urticaria, acute motor sensory axonal neuropathy, Balo's disease (also known as concentric sclerosis), Behcet's disease, benign mucous membrane pemphigoid (also known as cicatricial pemphigoid), bullous pemphigoid, Castleman disease, celiac disease, Chagas disease, chronic inflammatory demyelinating polyneuropathy, chronic recurrent multifocal osteomyelitis, Churg-Strauss syndrome (also known as allergic granulomatosis and angiitis or eosinophilic granulomatosis with polyangiitis), Cogan syndrome, cold agglutinin disease, congenital heart block, coxsackievirus myocarditis, CREST syndrome, Crohn's disease, dermatitis herpetiformis, dermatomyositis, Devic's disease (also known as neuromyelitis optica), discoid lupus erythematosus, Dressler syndrome, endometriosis, eosinophilic esophagitis, eosinophilic fasciitis, erythema nodosum, essential mixed cryoglobulinemia, Evans syndrome, fibromyalgia, fibrosing alveolitis, giant cell arteritis, giant cell myocarditis, glomerulonephritis, Goodpasture syndrome, granulomatosis with polyangiitis, Graves' disease, Guillain-Barre syndrome, Hashimoto's thyroiditis, hemolytic anemia, Henoch-Schonlein purpura (also known as allergic purpura), herpes gestationis or pemphigoid gestationis, hidradenitis suppurativa, hypogammaglobulinaemia, IgA nephropathy, IgG4-related sclerosing diseases (also known as IgG4-related systemic disease and IgG4-associated disease), hyper-IgG4 disease, and immune thrombocytopenic purpura, inclusion body myositis, interstitial cystitis, juvenile idiopathic arthritis, juvenile dermatomyositis, Kawasaki disease, Lambert-Eaton myasthenic syndrome, leukocytoclastic vasculitis (also known as hypersensitivity vasculitis), lichen planus, lichen sclerosus et atrophicus, ligneous conjunctivitis, linear IgA disease, chronic Lyme disease, Meniere's disease, microscopic polyangiitis, mixed connective tissue disease, Mooren's ulcer, Mucha-Habermann disease (also known as Pityriasis lichenoides et varioliformis acuta), multifocal motor neuropathy, multiple sclerosis, myasthenia gravis, myositis, narcolepsy, neonatal lupus, neutropenia, ocular cicatricial pemphigoid, optic neuritis, palindromic rheumatism, pediatric autoimmune neuropsychiatric disorders associated with streptococcal infections (PANDAS), paraneoplastic cerebellar degeneration, paroxysmal nocturnal hemoglobinuria, Parry-Romberg syndrome, pars planitis (also known as peripheral uveitis), Parsonage-Turner syndrome (also known as brachial neuritis), pemphigus, peripheral neuropathy, perivenous encephalomyelitis, pernicious anemia, POEMS syndrome, polyarteritis nodosa, autoimmune polyendocrine syndrome type I, II, and III, polymyalgia rheumatica, polymyositis, post-myocardial infarction syndrome, post-pericardotomy syndrome, primary biliary cholangitis, primary sclerosing cholangitis, progesterone dermatitis, psoriasis, psoriatic arthritis, pure red cell aplasia, pyoderma gangrenosum, Raynaud's phenomenon, reactive arthritis, reflex sympathetic dystrophy (also known as complex regional pain syndrome), relapsing polychondritis, restless legs syndrome, retroperitoneal fibrosis, rheumatic fever, rheumatoid arthritis, sarcoidosis, Schmidt syndrome (also known as polyglandular autoimmune syndrome type 2), scleritis, scleroderma, Sjogren's syndrome, autoimmune orchitis and spermatogenic autoimmunity, stif person syndrome, subacute bacterial endocarditis, Susac syndrome, sympathetic ophthalmia, systemic lupus erythematosus, Takayasu arteritis, temporal arteritis, thyroid eye disease, Tolosa-Hunt syndrome, type 1 diabetes mellitus (also known as autoimmune diabetes or insulin-dependent diabetes mellitus), ulcerative colitis, undifferentiated connective tissue disease, uveitis, vasculitis, vitiligo, and Koyanagi Harada disease; especially systemic lupus erythematosus, type 1 diabetes mellitus, rheumatoid arthritis, multiple sclerosis, ankylosing spondylitis, psoriasis, ulcerative colitis, and Crohn's disease.

Specifically, the inflammatory disease includes, but is not limited to, one or more of a group consisting of gout, chronic obstructive pulmonary disease, interstitial lung disease, inflammatory bowel disease, sepsis, asthma, and allergy.

Specifically, the tumor includes, but is not limited to blastoma, medulloblastoma, retinoblastoma, sarcoma, liposarcoma, synovial sarcoma, neuroendocrine tumor, carcinoid tumor, gastrinoma, islet cell carcinoma, mesothelioma, schwannoma, acoustic neuroma, meningioma, adenocarcinoma, melanoma, squamous cell carcinoma, epithelial squamous cell carcinoma, lung cancer Lung cancer (including small cell lung cancer and non-small cell lung cancer), lung adenocarcinoma, squamous-cell lung cancer, peritoneal cancer, hepatocellular carcinoma, gastric cancer, intestinal cancer, pancreatic cancer, glioblastoma, cervical cancer, ovarian cancer, liver cancer, bladder cancer, liver cancer, breast cancer (especially metastatic breast cancer), colon cancer, rectal cancer, colorectal cancer, uterine cancer, salivary gland cancer, kidney cancer, prostate cancer, vulvar cancer, thyroid cancer, liver cancer, anal cancer, penile cancer, Merkel cell carcinoma, esophageal cancer, biliary tumors, head and neck cancer, and hematological malignancies.

More specifically, the tumor is a hematologic malignancy, such as leukemia, lymphoma, or multiple myeloma (MM).

Specifically, the leukemia may include chronic lymphocytic leukemia, chronic myeloid leukemia, acute lymphoblastic leukemia (ALL), acute myeloid leukemia (AML), and acute monoblastic leukemia, especially acute myeloid leukemia. Specifically, the leukemia may be relapsed, refractory, or resistant.

Specifically, the lymphoma may be a B-cell lymphoma (e.g., diffuse large B-cell lymphoma (DLBCL), follicular lymphoma, mucosa-associated lymphoid tissue (MALT) lymphoma, small lymphocytic lymphoma/chronic lymphocytic leukemia, mantle cell lymphoma (MCL)), T-cell or NK-cell lymphoma, especially diffuse large B-cell lymphoma (DLBCL).

In an example of the present disclosure, the tumor is a solid tumor, including but not limited to neuroblastoma, renal cell carcinoma, colon cancer, colorectal cancer, breast cancer, epithelial squamous cell carcinoma, melanoma, stomach cancer, esophageal cancer, gastroesophageal junction (GEJ) cancer, brain cancer, lung cancer (e.g., non-small cell lung cancer, NSCLC), pancreatic cancer, cervical cancer, ovarian cancer, liver cancer, bladder cancer, prostate cancer, testicular cancer, thyroid cancer, uterine cancer, adrenal cancer, head and neck cancer, or urothelial carcinoma; especially, ovarian cancer, stomach cancer, gastroesophageal junction (GEJ) cancer, head and neck squamous cell carcinoma, metastatic or unresectable melanoma, non-small cell lung cancer, metastatic castration-resistant prostate cancer (mCRPC), malignant pleural mesothelioma (MPM), breast cancer, metastatic urothelial carcinoma, cervical cancer, and metastatic colorectal cancer.

Specifically, the pathogens can be microorganisms, parasites (such as protozoa and helminths), or other agents. Specifically, the microorganisms can be selected from one or more of a group consisting of viruses, chlamydiae, rickettsiae, mycoplasmas, bacteria, spirochetes, fungi, and the like.

In some embodiments of the present disclosure, the athogens may be viruses, including but not limited to Adenoviridae (e.g., adenovirus), Herpesviridae (e.g., HSV1 (oral herpes), HSV-2 (external genital herpes), VZV (varicella), EBV (Epstein-Barr virus), CMV (cytomegalovirus)), Poxviridae (e.g., smallpox virus and cowpox virus), Papillomaviridae (e.g., papillomavirus (HPV)), Parvoviridae (e.g., B19 virus), Hepadnaviridae (e.g., hepatitis B virus), Polyomaviridae (e.g., polyomavirus), Reoviridae (e.g., reovirus and rotavirus), Picornaviridae (e.g., enterovirus and foot-and-mouth disease virus), Caliciviridae (e.g., Norwalk virus and hepatitis E virus), Togaviridae (e.g., rubella virus), Arenaviridae (e.g., lymphocytic choriomeningitis virus), Retroviridae (e.g., HIV-1, HIV-2, and HTLV-1), Flaviviridae (e.g., dengue virus, Zika virus, Japanese encephalitis virus, chikungunya virus, yellow fever virus, hepatitis C virus, and West Nile virus), Orthomyxoviridae (e.g., influenza viruses including influenza A, B, and C viruses), Paramyxoviridae (e.g., human parainfluenza virus types 1 - 4, Sendai virus, mumps virus, measles virus, respiratory syncytial virus, and Newcastle disease virus), Bunyaviridae (e.g., California encephalitis virus and hantaviruses), Rhabdoviridae (e.g., rabies virus), Filoviridae (e.g., Ebola virus and Marburg virus), Coronaviridae (e.g., HCoV-229E, HCoV-OC43, HCoV-NL63, HCoV-HKU1, SARS-CoV, MERS-CoV, and SARS-CoV-2), Astroviridae (e.g., astroviruses), and Bornaviridae (e.g., Borna virus).

Specifically, the pathogen-induced or pathogen-associated diseases include, but are not limited to, influenza, SARS, COVID-19, viral hepatitis (e.g., hepatitis A, B, C, and D), AIDS, rabies, dengue fever, Ebola virus disease, and the like.

In a sixth aspect, the present disclosure provides a use of the compound or the pharmaceutically acceptable salt, stereoisomer, ester, prodrug, solvate, or deuterated compound thereof as described in the first aspect, preventing and/or treating a disease related to Cbl-b activity.

Specifically, the disease is as defined in the fifth aspect of the present disclosure.

In a seventh aspect, the present disclosure provides a method for preventing and/or treating diseases related to Cbl-b activity, which includes administering to a subject in need thereof the compound, or the pharmaceutically acceptable salt, stereoisomer, ester, prodrug, solvate, or deuterated compound thereof as described in the first aspect, or the pharmaceutical composition as described in the third aspect, or the Cbl-b inhibitor as described in the fourth aspect.

Specifically, the subject may be a mammal, especially a human.

Specifically, the disease is as defined in the fifth aspect of the present disclosure..

Specifically, in the method, the compound, or the pharmaceutically acceptable salt, stereoisomer, ester, prodrug, solvate, or deuterated form thereof as described in the first aspect, may be used alone or in combination with other types of pharmaceutical preparations and/or treatment methods.

Specifically, the other types of pharmaceutical preparations and/or treatment methods include, but are not limited to: immune checkpoint inhibitors, antineoplastic agents, glucocorticoids, nonsteroidal anti-inflammatory drugs, antitumor vaccines, Toll-like receptor (TLR) agonists and inhibitors, adoptive cell immunotherapy, or radiotherapy.

Specifically, the immune checkpoint inhibitor is an antagonist of at least one inhibitory checkpoint molecule, the inhibitory checkpoint molecules including, but not limited to: PD-1 (CD279), PD-L1 (CD274), CTLA-4 (CD125), LAG3 (CD223), PVR (CD155), PVRL2 (CD112), PVRL3 (CD113), TIGIT, TIM3 (CD366), and VISTA. More specifically, the immune checkpoint inhibitor is an antagonist of at least one inhibitory checkpoint molecule selected from PD-1 (CD279), PD-L1 (CD274), and CTLA-4 (CD152).

In an example of the present disclosure, the at least one inhibitory checkpoint molecule includes PD-1; specifically the immune checkpoint inhibitor is optionally selected from a group consisting of pembrolizumab, nivolumab, cemiplimab, and their biosimilars.

In an embodiment of the present disclosure, the at least one inhibitory checkpoint molecule includes PD-L1; optionally, the immune checkpoint inhibitor is selected from a group consisting of atezolizumab, avelumab, durvalumab, and their biosimilars.

In an embodiment of the present disclosure, the at least one inhibitory checkpoint molecule includes CTLA-4; specifically, the immune checkpoint inhibitor is optionally selected from ipilimumab, tremelimumab, and their biosimilars.

Specifically, the anti-tumor agents include but are not limited to: cytotoxic antibiotics, plant alkaloids, antimetabolites, alkylating agents, platinum compounds, and protein kinase inhibitors.

Specifically, the cytotoxic antibiotics include but are not limited to: ixabepilone, mitomycin, plicamycin, bleomycin, pixantrone, amrubicin, valrubicin, pirarubicin, mitoxantrone, idarubicin, zorubicin, aclarubicin, epirubicin, daunorubicin, doxorubicin, and actinomycin.

Specifically, the plant alkaloids include but are not limited to: trabectedin, cabazitaxel, polyaniline paclitaxel, docetaxel, paclitaxel, demecolcine, teniposide, etoposide, vinflunine, vinflonine, vinorelbine, vindesine, vincristine, and vinblastine.

Specifically, the anti-metabolites include but are not limited to: fluorouridine, trifluridine, tegafur, fluorouracil, decitabine, azacitidine, capecitabine, gemcitabine, carmofur, cytarabine, nelarabine, clofarabine, fludarabine, cladribine, thioguanine, mercaptopurine, pralatrexate, pemetrexed, raltitrexed, and methotrexate.

Specifically, the alkylating agents include but are not limited to: dacarbazine, temozolomide, pipobroman, mitobronitol, ethoglucid, uracil mustard, ranimustine, nimustine, fotemustine, streptozotocin, semustine, lomustine, carmustine, carboquone, triaziquone, thiotepa, mannomustine, altretamine, busulfan, bendamustine, prednimustine, trofosfamide, ifosfamide, mechlorethamine, melphalan, chlorambucil, and cyclophosphamide.

Specifically, the platinum compounds include but are not limited to: cisplatin, carboplatin, oxaliplatin, saplatin, and polyplatin.

Specifically, the protein kinase inhibitors include but are not limited to: BTK inhibitors, PI3K inhibitors, SYK inhibitors, and JAK inhibitors.

Specifically, the glucocorticoids include but are not limited to: hydrocortisone, dexamethasone, betamethasone, and prednisone.

Specifically, the non-steroidal anti-inflammatory drugs include but are not limited to: aspirin, ibuprofen, diclofenac, and rofecoxib.

Specifically, the TLR agonists include but are not limited to: TLR3 agonist Poly-ICLC, TLR4 agonist MPLA, TLR7 agonist GS-9620, TLR8 agonist ssRNA40, TLR7 agonist TLR7-agonist-1, TLR8 agonist Motolimod, and TLR9 agonist CPG7079 or 1018ISS.

Specifically, the TLR inhibitors include but are not limited to: TLR1/2 inhibitor CU CPT 22, TLR4 inhibitor atractylenolide, TLR2 inhibitor C29, TLR8 inhibitor CU-CPT-9a, and TLR7/8/9 inhibitor CPG-52364.

In an eighth aspect, the present disclosure provides a method for preventing and/or treating immune-related diseases (such as autoimmune diseases, inflammatory diseases, tumors), which includes the step of administering the compound, or the pharmaceutically acceptable salt, stereoisomer, ester, prodrug, solvate, or deuterated compound thereof as described in the first aspect, or the pharmaceutical composition as described in the third aspect, or the Cbl-b inhibitor as described in the fourth aspect to a subject in need thereof.

In an embodiment of the present disclosure, the method is a method for preventing and/or autoimmune diseases.

In another embodiment of the present disclosure, the method is a method for preventing and/or inflammatory diseases.

In another embodiment of the present disclosure, the method is a method for preventing and/or tumors.

Specifically, the subject can be a mammal, especially a human.

Specifically, the disease is as defined in the fifth aspect of the present disclosure.

In a ninth aspect, the present disclosure provides a method for regulating immune cell activity, which includes the step of contacting immune cells with an effective amount of the compound, or the pharmaceutically acceptable salt, stereoisomer, ester, prodrug, solvate, or deuterated compound thereof as described in the first aspect, or the pharmaceutical composition as described in the third aspect, or the Cbl-b inhibitor as described in the fourth aspect to regulate immune cell activity.

Specifically, the immune cells include T cells, B cells, or NK cells.

Specifically, the immune cells are separated from a blood sample of a mammalian subject.

Specifically, the immune cells are tumor infiltrating lymphocytes (TILs) isolated from tumors of mammalian subjects with tumors.

Specifically, the immune cells are human immune cells.

In a tenth aspect, the present disclosure provides a method for regulating an immune response, which includes the step of administering the compound, or the pharmaceutically acceptable salt, stereoisomer, ester, prodrug, solvate, or deuterated compound thereof as described in the first aspect, or the pharmaceutical composition as described in the third aspect, or the Cbl-b inhibitor as described in the fourth aspect, to a subject in need thereof.

In an eleventh aspect, the present disclosure provides a method for preparing modified immune cells, which includes culturing a cell population containing immune cells in the presence of an effective amount of the compound, or the pharmaceutically acceptable salt, stereoisomer, ester, prodrug, solvate, or deuterated compound thereof as described in the first aspect, or the pharmaceutical composition as described in the third aspect, or the Cbl-b inhibitor as described in the fourth aspect, to regulate the activity of the immune cells and thus to produce modified immune cells.

In a twelfth aspect, the present disclosure provides a modified immune cell, which is prepared by the method as described in the eleventh aspect.

In a thirteenth aspect, the present disclosure provides a method for treating or preventing nausea or vomiting or both in a patient receiving Cbl treatment, which includes administering an effective amount of a serotonin receptor antagonist to a subject.

Specifically, the Cbl treatment includes administering an effective amount of the compound, or the pharmaceutically acceptable salt, stereoisomer, ester, prodrug, solvate, or deuterated compound thereof as described in the first aspect of the present disclosure, or the Cbl-b inhibitor as described in the fourth aspect.

Specifically, the serotonin receptor antagonist is as described in the third aspect of the present disclosure.

In a fourteenth aspect, the present disclosure provides a use of the compound, or the pharmaceutically acceptable salt, stereoisomer, ester, prodrug, solvate, or deuterated compound thereof as described in the first aspect, in the preparation of a protein degradation targeted chimera (PROTAC).

In a fifteenth aspect, the present disclosure provides a PROTAC compound, which includes an E3 ubiquitin ligase ligand structure part (E3L), a target protein ligand structure part (PL), and, optionally, a linking bond or linking group for connecting E3L and PL, in which E3L comes from the compound, or the pharmaceutically acceptable salt, stereoisomer, ester, prodrug, solvate, or deuterated compound as described in the first aspect.

The present disclosure prepares a series of compounds, which have better Cbl-b inhibitory activity and are expected to be used for the prevention and treatment of diseases related to Cbl-b activity.

### DETAILED DESCRIPTION OF THE EMBODIMENTS

Unless otherwise defined, all scientific and technical terms used in the present disclosure have the same meanings as are generally understood by those skilled in the art to which the present disclosure relates.

In the present disclosure, the term "aliphatic group" refers to a linear or branched hydrocarbon chain that is fully saturated or contains one or more unsaturated units (such as "alkyl", "alkenyl", and "alkynyl"), or a cyclic hydrocarbyl that is fully saturated or contains one or more unsaturated units (also referred to herein as " alicyclic ring", and "cycloalkyl"), which is connected to other parts of the molecule by a single bond. Suitable aliphatic groups include, but are not limited to, linear or branched, substituted or unsubstituted alkyl, alkenyl, alkynyl, and mixtures thereof, such as (cycloalkyl)alkyl, (cycloalkenyl)alkyl, and (cycloalkyl)alkenyl.

The term "carbon ring" is composed entirely of carbon atoms and can be divided into alicyclic rings and aromatic rings.

The term "alkyl" refers to a linear or branched hydrocarbon radical that contains no unsaturated bonds and is connected to other parts of the molecule via a single bond. Typical alkyl groups contain 1 to 10 (e.g., 1, 2, 3, 4, 5, 6, 7, 8, 9, and 10) carbon atoms, especially 1 to 6 carbon atoms, such as methyl, ethyl, n-propyl, isopropyl, n-butyl, isobutyl, tert-butyl, n-pentyl, isopentyl, neopentyl, tert-pentyl, n-hexyl, and isohexyl. In the present disclosure, C₀ alkyl refers to -H. If the alkyl is substituted with a cycloalkyl, it corresponds to "cycloalkylalkyl", such as cyclopropylmethyl, cyclopropylethyl, cyclobutylmethyl, cyclopentylmethyl, and cyclohexylmethyl. In the present disclosure, the cycloalkylalkyl can be -(alkylidene)-(cycloalkyl), for example, the C₄₋₁₀ cycloalkylalkyl can be -(C₁₋₄ alkylidene)-(C₃₋₆ cycloalkyl). If the alkyl is substituted with an aryl, it corresponds to "aralkyl" such as benzyl, diphenylmethyl, or phenethyl. In the present disclosure, the aralkyl can be -(alkylidene)-(aryl), for example, C₆₋₁₀ aralkyl can be -(C₁₋₄ alkylene)-(phenyl). If the alkyl is substituted with heterocyclyl, it corresponds to "heterocyclylalkyl". In the present disclosure, the heterocyclylalkyl can be -(alkylidene)-(heterocyclyl), such as -(C₁₋₄ alkylidene)-(4-10 membered heterocyclyl).

The term "alkylidene" refers to hydrocarbyl (divalent alkyl) formed by the loss of two hydrogen atoms in an alkane molecule. Typical alkylidene herein has 1 to 10 (e.g., 1, 2, 3, 4, 5, 6, 7, 8, 9, and 10) carbon atoms, preferably containing 1 to 6 carbon atoms, and examples of alkylidene include methylene (-CH₂-), ethylene (-CH₂CH₂-), propylene (-CH₂CH₂CH₂-, -CH(CH₃)CH₂- or -CH₂-CH(CH₃)-) In the present disclosure, C₀ alkylidene refers to a single bond.

The term "cycloalkyl" refers to an alicyclic hydrocarbon that may be a monocyclic, bicyclic, tricyclic, or tetracyclic ring system. The ring system may be a fused, spirocyclic, or bridged ring system. The cycloalkyl may contain 3-18 carbon atoms, preferably 3-10 (e.g., 3, 4, 5, 6, 7, 8, 9, and 10) carbon atoms, especially monocyclic groups containing 3-6 carbon atoms, such as cyclopropyl, cyclobutyl, cyclopentyl, cyclohexyl, or adamantyl.

The term "cycloalkylene" refers to a divalent group formed by the loss of two hydrogen atoms in an alicyclic hydrocarbon. Typical cycloalkylene herein has 3 to 10 (e.g., 3, 4, 5, 6, 7, 8, 9, and 10) carbon atoms, preferably containing 3 to 6 carbon atoms, and examples of cycloalkylene are

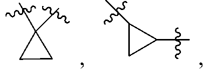

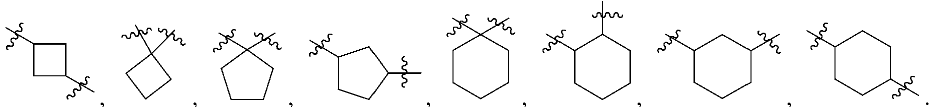

The term "alkoxyl" refers to a substituent formed by replacing the hydrogen in the hydroxyl with an alkyl, such as an alkoxyl containing 1-10 carbon atoms, such as methoxyl, ethoxyl, propoxyl, and butoxyl.

The term "alkylamino" refers to a substituent formed by replacing one or both hydrogens in the amino (-NH₂) with an alkyl, such as an alkylamino containing 1-10 carbon atoms, for example,

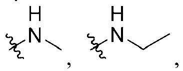

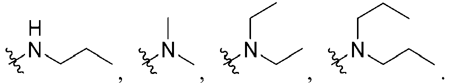

The term "halogen" refers to fluorine, chlorine, bromine, or iodine.

The term "haloalkyl" refers to a group formed by replacing one or more hydrogens in an alkyl with halogen atoms (such as fluorine, chlorine, bromine, or iodine), such as -CHF₂, -CH₂F, -CH₂Cl, -CF₃, -CH₂-CF₃, -CH₂CH₂-CF₃, and -CH₂CH₂CH₂-CF₃, especially methyl and ethyl substituted with one, two or three halogen atoms (F, Cl, Br, and I).

The term "aryl" refers to a monocyclic or polycyclic radical, including a polycyclic radical containing a monoaryl group and/or a condensed aryl group, such as containing 1-3 monocyclic or condensed rings and 6-18 (e.g., 6, 8, 10, 12, 14, 16, and 18) carbon ring atoms, such as phenyl, naphthyl, biphenyl, and indenyl.

The term "heterocyclyl" refers to a 3-18 membered non-aromatic ring group containing 2 to 17 carbon atoms and 1 to 10 heteroatoms. The heterocyclyl can be a single-ring, double-ring, triple-ring, or quadruple-ring system, including fused, spirocyclic, or bridged ring systems. The heterocyclyl can be partially saturated (heteroaryl) or fully saturated (heterocycloalkyl). Suitable heteroaryl in the compounds of the present disclosure contains 1, 2, or 3 heteroatoms selected from N, O, or S atoms. The heteroaryl includes, for example, coumarin (including 8-coumarin), quinolyl (including 8-quinolyl), isoquinolyl, pyridyl, pyrazinyl, pyrazolyl, pyrimidyl, furanyl, pyrrolyl, thienyl, thiazolyl, isothiazolyl, triazolyl, tetrazolyl, isoxazolyl, oxazolyl, imidazolyl, indolyl, isoindolyl, indazolyl, indazinyl, phthalazinyl, pteridinyl, purinyl, oxadiazolyl, thiadiazolyl, furazyl, pyridazinyl, triazinyl, cinnolyl, benzimidazolyl, benzofuranyl, benzofuranyl, benzothienyl, benzothiazolyl, benzoxazolyl, quinazolinyl, quinoxalinyl, naphthyridyl, and furanopyridyl. Suitable heterocycloalkyl in the compounds of the present disclosure contains 1, 2, or 3 heteroatoms selected from N, O, or S atoms. The heterocycloalkyl includes, for example, pyrrolidine, tetrahydrofuranyl, dihydrofuran, tetrahydrothienyl, tetrahydrothianyl, piperidinyl, morpholino, thiomorpholinyl, oxathialkyl, piperazinyl, azetidinyl, oxetidinyl, thietanyl, homopiperidinyl, oxacyclopropanyl, thiocyclopropanyl, azeptinyl, oxazetidinyl, diaziheptinyl, triaziheptinyl, 1,2,3,6-tetrahydropyridyl, 2-pyrrolinyl, 3-pyrrolinyl, dihydroindolyl, 2H-pyranyl, 4H-pyranyl, dioxohexanyl, 1,3-dioxolane, pyrazolinyl, dithialkyl, dithiolanyl, dihydropyranyl, dihydrothienyl, pyrazolidinyl, imidazolinyl, imidazolidinyl, 3-azabicyclo[3.1.0]hexyl, 3-azabicyclo[4.1.0]heptyl, and 3H-indolyl and quinazolinyl.

In the present disclosure, "D" refers to deuterium; "substituted with deuterium" means replacing one or more hydrogen atoms with a corresponding number of deuterium atoms.

It should be recognized that there are some variations in the abundance of natural isotopes in the synthesized compounds depending on the source of the chemical materials used in the synthesis. Therefore, the compounds of the present disclosure will inherently contain a small amount of deuterated isotopologues. Despite this variation, the concentration of stable hydrogen and carbon isotopes in this natural abundance is still very low and insignificant compared to the degree of stable isotopic substitution of the compounds of the present disclosure, see, for example, Wada, E et al., Seikagaku, 1994, 66: 15; Gannes, LZ et al., Comp Biochem Physiol Mol Integr Physiol, 1998, 119: 725.

In the compounds of the present disclosure, any atom not specified as deuterium is present in its natural isotopic abundance. Unless otherwise stated, when a position is specifically designated as "H" or "hydrogen", the position should be understood to have hydrogen in its natural abundance isotopic composition. Similarly, unless otherwise stated, when a position is specifically designated as "D" or "Deuterium", the position should be understood to have deuterium with an abundance at least 3000-fold higher than the natural abundance of deuterium (which is 0.015%) (i.e., at least 45% deuterium incorporation).

The term "isotopic enrichment ratio" herein refers to the ratio between the isotopic abundance of a particular isotope and its natural abundance.

In other embodiments, the compound of the present disclosure has an isotopic enrichment ratio for each specified deuterium atom of at least 3500 (52.5% deuterium incorporation at each specified deuterium atom), at least 4000 (60% deuterium incorporation), at least 4500 (67.5% deuterium incorporation), at least 5000 (75% deuterium incorporation), at least 5500 (82.5% deuterium incorporation), at least 6000 (90% deuterium incorporation), at least 6333.3 (95% deuterium incorporation), at least 6466.7 (97% deuterium incorporation), at least 6600 (99% deuterium incorporation), or at least 6633.3 (99.5% deuterium incorporation).

The term " isotopologue" refers to a substance in which the chemical structure differs from the specific compound of the present disclosure only in its isotopic composition.

The term "pharmaceutically acceptable salts" includes acid addition salts and base addition salts.

The term "acid addition salt" includes, but is not limited to, salts from inorganic acids (such as hydrochloric acid, nitric acid, phosphoric acid, sulfuric acid, hydrobromic acid, hydroiodic acid, and phosphonic acid), and salts from organic acids (such as aliphatic monocarboxylic acids and dicarboxylic acids, phenyl-substituted alkanoic acids, hydroxyalkanoic acids, alkanedioic acids, aromatic acids, and aliphatic and aromatic sulfonic acids). Therefore, these salts include but are not limited to sulfate, pyrosulfate, hydrogen sulfate, sulfite, bisulfite, nitrate, phosphate, monohydrogen phosphate, dihydrogen phosphate, metaphosphate, pyrophosphate, hydrochloride, hydrobromide, iodate, acetate, propionate, caprylate, isobutyrate, oxalate, malonate, succinate, suberate, sebacate, fumarate, maleate, mandelate, benzoate, chlorobenzoate, methylbenzoate, dinitrobenzoate, phthalate, benzenesulfonate, toluenesulfonate, phenylacetate, citrate, lactate, tartrate, and methanesulfonate, and also contains amino acid salts such as arginine, gluconate, and galacturonate. The acid addition salt can be prepared by contacting the free base form with a sufficient amount of the desired acid in a conventional manner to form a salt. The free base form can be regenerated by contacting the salt form with the base, and the free base is separated in a conventional manner.

The term "alkali addition salt" refers to a salt formed with a metal or amine, such as hydroxides of alkali metals and alkaline earth metals, or formed with an organic amine. Examples of metals used as cations include, but are not limited to, sodium, potassium, magnesium, and calcium. Examples of suitable amines include, but are not limited to, N,N'-dibenzylethylenediamine, chloroprocaine, choline, diethanolamine, ethylenediamine (ethane-1,2-diamine), N-methylglucosamine, and procaine. The alkali addition salt can be prepared by contacting the free acid form with a sufficient amount of the desired base in a conventional manner to form a salt. The free acid form can be regenerated by contacting the salt form with the acid, and the free acid is separated in a conventional manner.

The term "stereoisomer" includes the existence of enantiomers, diastereomers, and geometric isomers. Some compounds of the present disclosure have cyclic hydrocarbyl that can be substituted on more than one carbon atom, in which case all their geometric forms, including cis and trans, and mixtures thereof, are within the scope of the present disclosure.

The term "solvate" refers to the physical combination of the compound of the present disclosure with one or more solvent molecules. The physical bonding includes various degrees of ionic and covalent bonding, including hydrogen bonding. In some cases, solvates can be separated, for example, when one or more solvent molecules are incorporated into the lattice of a crystalline solid. The solvate includes a solution phase and a separable solvate. Representative solvates include ethanolates and methanolates.

The term "prodrug" refers to a form of the compound of formula I that is suitable for administration to a patient without excessive toxicity, irritation, and allergic reactions and is effective for its application purpose, including acetal, ester, and zwitterionic forms. The prodrug is transformed in the body, such as by hydrolysis in the blood, to obtain the parent compound.

The terms "patient" or "subject" and the like are used interchangeably herein and refer to any animal or its cells treated according to the methods described herein, whether in vitro or in situ. Specifically, the animals include mammals, such as rats, mice, guinea pigs, rabbits, dogs, monkeys, or humans, especially humans.

The term "treatment" refers to the prevention, cure, reversal, alleviation, reduction, minimization, suppression, cessation, and/or stopping of one or more clinical symptoms of a disease after its onset.

The term "prevention" refers to treatment administered prior to the onset of a disease in order to avoid, minimize, or make the occurrence or progression of the disease more difficult.

The term "diseases associated with Cbl-b activity" primarily refers to diseases related to abnormal Cbl-b activity, especially those for which inhibition of Cbl-b may be beneficial for prevention and/or treatment, such as autoimmune diseases, inflammatory diseases, and tumors.

The term "tumor" refers to an abnormal mass of tissue, the growth of which exceeds and is uncoordinated with that of normal tissues. Tumors can be "benign" or "malignant", depending on the following characteristics: degree of cell differentiation (including morphology and function), growth rate, local invasion and metastasis. "Benign tumors" are usually well-differentiated and are characterized by slower growth compared to malignant tumors, remaining confined to their site of origin. In addition, benign tumors do not have the ability to infiltrate, invade, or metastasize to distant sites. In some cases, certain "benign" tumors may later develop into malignant tumors, which may be caused by additional genetic alterations in subpopulations of the neoplastic cells. Such tumors are referred to as "precancerous tumors." "Malignant tumors" are usually poorly differentiated (anaplastic) and have characteristic rapid growth, accompanied by progressive infiltration, invasion, and destruction of surrounding tissues. In addition, malignant tumors often have the ability to metastasize to distant sites.

The term "solid tumor" refers to a palpable or visible mass that can be detected through clinical examinations such as X-ray imaging, CT scans, ultrasound, or physical palpation. In some embodiments of the present disclosure, the solid tumor is selected from advanced or metastatic malignant solid tumors. The term "advanced or metastatic malignant solid tumor" refers to a malignant solid tumor confirmed by histology or cytology that is advanced, unresectable, and/or metastatic, recurrent, or refractory, and for which standard therapies are ineffective or no proven effective treatment is available. According to the present disclosure, malignant solid tumors include but are not limited to cancer, sarcoma, melanoma, and lymphoma.

The term "cancer" refers to a malignant tumor (Stedman's Medical Dictionary, 25th ed.; Hensyl ed.; Williams & Wilkins: Philadelphia, 1990).

The term "autoimmune disease" refers to a disease caused by the body's immune response to its own antigens, resulting in damage to its own tissues. The American Autoimmune Related Diseases Association has a relatively comprehensive list of autoimmune diseases.

The term "inflammation" is the body's defensive response to stimuli, manifested as redness, swelling, heat, pain, and dysfunction; it can be infectious inflammation caused by infection or non-infectious inflammation not caused by infection, such as inflammation caused by immune responses (such as various types of hypersensitivity reactions and inflammation associated with autoimmune diseases). The term "inflammatory disease" refers to a disease with inflammation.

The term "CAR-T immunotherapy" refers to chimeric antigen receptor T cell immunotherapy, which is one of the more effective treatments for malignant tumors. Its principle involves using the patient's own immune cells to target and eliminate cancer cells It belongs to a cell therapy.

The terms "Cbl-b inhibitor" and "Cbl-b antagonist" have the same meaning and refer to molecules that reduce, inhibit, or otherwise decrease one or more biological activities of Cbl-b. The inhibitory effect of using Cbl-b inhibitors does not necessarily indicate complete elimination of Cbl-b activity. Compared with the control, Cbl-b activity can be reduced by a significant amount, for example, Cbl-b activity is reduced by at least about 5%, 10%, 15%, 20%, 25%, 30%, 35%, 40%, 45%, 50%, 55%, 60%, 65%, 70%, 75%, 80%, 85%, 90%, 95%, or 100%.

The disclosures of various publications, patents and published patent specifications cited herein are incorporated herein by reference in their entirety.

The technical solution of the present disclosure will be clearly and completely described below with reference to the embodiments. Obviously, the embodiments described are some rather than all of the embodiments of the present disclosure. Based on the embodiments described herein, all other embodiments obtained by those of ordinary skill in the art without creative work are within the scope of protection of the present disclosure.

### Examples of Synthesis

### Example 1: Synthesis of compound T001

### 1. General steps for preparation of (S)-4-bromo-2-((3-methylpiperidin-1-yl)methyl)-1-tosyl-1,6-dihydro-7H-pyrrolo[2,3-c]pyridin-7-one:

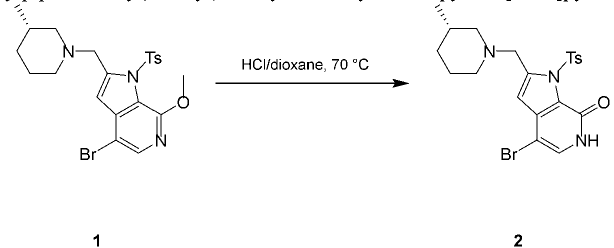

4-bromo-7-methoxy-2-[[(3S)-3-methyl-1-piperidyl]methyl]-1-(p-tolylsulfonyl)pyrrolo[2,3-c]pyridine (500 mg, 1.02 mmol) was added to a round-bottom flask, followed by HCl/ dioxane (4 M, 5.00 mL). The mixture was stirred at 70°C for 1 hr. LCMS showed the reaction was completed. The reaction mixture was diluted with EtOAc (50.0 mL) poured into saturated aqueous NaHCO₃ (30.0 mL) slowly, adjust pH = 8 (saturated aqueous NaHCO₃). The organic phase was washed with brine (50.0 mL), dried over Na₂SO₄. The organic phase was concentrated in vacuum. The residue was purified by column chromatography (SiO₂, DCM/ MeOH = 1/ 0 to 95/ 5). TLC (Plate 1, DCM/ MeOH = 10/ 1, UV 254 nm, R_{f} (product) = 0.6). Compound (S)-4-bromo-2-((3-methylpiperidin-1-yl)methyl)-1-tosyl-1,6-dihydro-7H-pyrrolo[2,3-c]pyridin-7-one (295 mg, 616 µmol, 60.7% yield) was obtained as a yellow solid. It was confirmed by H NMR.
¹H NMR: (DMSO-*d*₆, 400 MHz)
*δ* 11.41 (br s, 1H), 8.42 (d, *J* = 8.4 Hz, 2H), 7.39 (d, *J* = 8.4 Hz, 2H), 7.36 (br d, *J* = 4.4 Hz, 1H), 6.54 (s, 1H), 3.86 (s, 2H), 2.77 (br d, *J* = 8.4 Hz, 2H), 2.39 (s, 3H), 1.90-1.98 (m, 1H), 1.65-1.76 (m, 2H), 1.56-1.63 (m, 2H), 1.42-1.51 (m, 1H), 0.85-0.93 (m, 1H), 0.82 (d, *J =* 6.4 Hz, 3H)

### 2. General steps for preparation of (S)-4-bromo-6-(3-(3-((4-methyl-4H-1,2,4-triazol-3-yl)methyl)oxetan-3-yl)phenyl)-2-((3-methylpiperidin-1-yl)methyl)-1-tosyl-1,6-dihydro-7H-pyrrolo[2,3-c]pyridin-7-one and (S)-6-(3-(3-((4-methyl-4H-1,2,4-triazol-3-yl)methyl)oxetan-3-yl)phenyl)-2-((3-methylpiperidin-1-yl)methyl)-1-tosyl-1,6-dih ydro-7H-pyrrolo[2,3-c]pyridin-7-one:

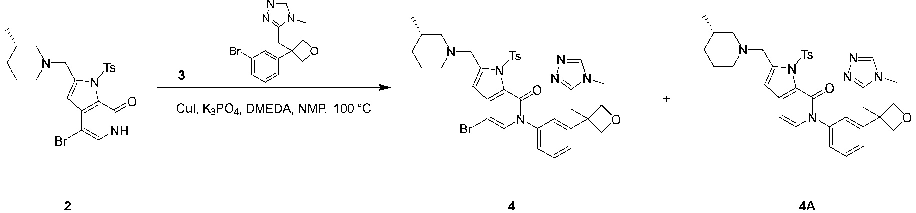

To a mixture of (S)-4-bromo-2-((3-methylpiperidin-1-yl)methyl)-1-tosyl-1,6-dihydro-7H-pyrrolo[2,3-c]pyridin-7-one (100 mg, 209 µmol), 3-[[3-(3-bromophenyl)oxetan-3-yl]methyl]-4-methyl-1,2,4-triazole (64.4 mg, 209 µmol), K₃PO₄ (133 mg, 627 µmol) and CuI (39.8 mg, 209 µmol) in NMP (1.50 mL) was added DMEDA (36.8 mg, 418 µmol, 45.0 µL). The suspension was degassed under vacuum and purged with N₂ several times. The mixture was stirred under N₂ at 100 °C for 2 hrs. LCMS showed the reaction was completed. The reaction mixture was diluted with EtOAc (30.0 mL), washed with brine (30.0 mL * 3), dried over Na₂SO₄. The organic phase was concentrated in vacuum. The residue was purified by column chromatography (SiO₂, DCM/ MeOH = 1/ 0 to 91/ 9). TLC (Plate 1, DCM/ MeOH = 10/ 1, UV 254 nm, R_{f} (product) = 0.4). Compound (S)-4-bromo-6-(3-(3-((4-methyl-4H-1,2,4-triazol-3-yl)methyl)oxetan-3-yl)phenyl)-2-((3-methylpiperidin-1-yl)meth yl)-1-tosyl-1,6-dihydro-7H-pyrrolo[2,3-c]pyridin-7-one (22.0 mg, 31.1 µmol, 14.9% yield) was obtained as a white solid, compound (S)-6-(3-(3-((4-methyl-4H-1,2,4-triazol-3-yl)methyl)oxetan-3-yl)phenyl)-2-((3-methylpiperidin-1-yl)methyl)-1-tos yl-1,6-dihydro-7H-pyrrolo[2,3-c]pyridin-7-one (120 mg, 191 µmol, 91.5% yield) was obtained as a white solid.

### 3. General steps for preparation of (S)-6-(3-(3-((4-methyl-4H-1,2,4-triazol-3-yl)methyl)oxetan-3-yl)pheny l)-2-((3-methylpiperidin-1-yl)methyl)-1,6-dihydro-7H-pyrrolo[2,3-c]pyridin-7-one (compound T001):

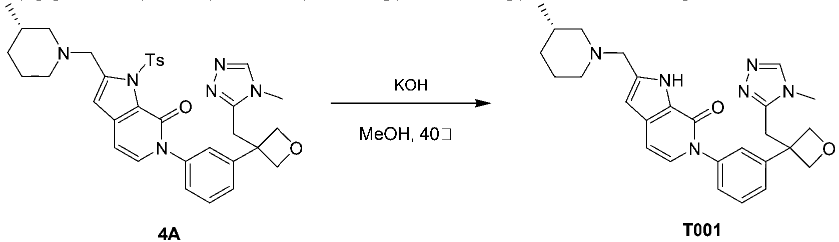

To a solution of (S)-6-(3-(3-((4-methyl-4H-1,2,4-triazol-3-yl)methyl)oxetan-3-yl)phenyl)-2-((3-methylpiperidin-1-yl)methyl)-1-tos yl-1,6-dihydro-7H-pyrrolo[2,3-c]pyridin-7-one (120 mg, 191 µmol) in MeOH (5.00 mL) was added KOH (214 mg, 3.83 mmol). The resulting mixture was stirred at 40 °C for 1 hr. LCMS showed desired compound was detected. The reaction mixture was diluted with aqueous NaHCO₃ (10.0 mL). The residue was purified by Prep-HPLC (column: Xtimate C 18 150 * 40 mm * 10 um; mobile phase: [water (NH₄HCO₃)-ACN]; gradient: 14%-54% B over 36 min). Compound (S)-6-(3-(3-((4-methyl-4H-1,2,4-triazol-3-yl)methyl)oxetan-3-yl)phenyl)-2-((3-methylpiperidin-1-yl)methyl)-1,6-di hydro-7H-pyrrolo[2,3-c]pyridin-7-one (6.20 mg, 13.0 µmol, 6.83% yield, 99.63% purity) was obtained as a green solid. Confirmed by H NMRand LCMS.
¹H NMR: (DMSO-*d*₆, 400 MHz)
*δ* 11.93 (br s, 1H), 7.96-8.80 (m, 1H), 7.35-7.42 (m, 1H), 7.29 (br d, *J* = 7.6 Hz, 1H), 6.98-7.09 (m, 2H), 6.91 (br d, *J* = 5.6 Hz, 1H), 6.54 (br d, *J* = 6.4 Hz, 1H), 6.22 (s, 1H), 4.91 (br d, *J* = 19.6 Hz, 4H), 3.56 (br d, *J* = 14.4 Hz, 4H), 2.96 (br s, 3H), 2.71-2.81 (m, 2H), 2.51-2.52 (m, 1H), 1.88 (br t, *J* = 10.0 Hz, 1H), 1.54-1.69 (m, 4H), 1.45 (br d, *J* = 12.0 Hz, 1H), 0.80 (br d, *J* = 5.6 Hz, 3H)
LCMS: m/z = 473.2 (M+H)⁺, Rt = 1.413 min

### Example 2: Synthesis of compound T002

### 1. General steps for preparation of 2-((ethyl(methyl)amino)methyl)-6-(3-(1-(4-methyl-4H-1,2,4-triazol-3-yl)cyclobutyl)phenyl)-4-(trifluoromethyl)-1, 6-dihydro-7H-pyrrolo[2,3-c]pyridin-7-one (compound T002):

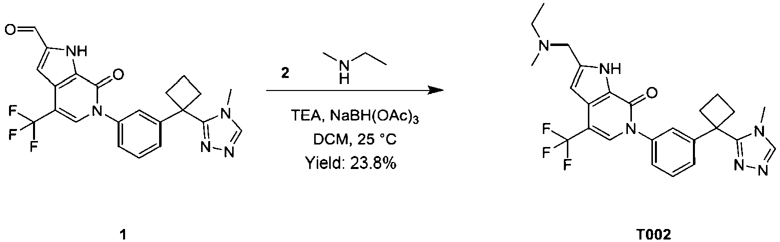

To a solution of 6-[3-[1-(4-methyl-1,2,4-triazol-3-yl)cyclobutyl]phenyl]-7-oxo-4-(trifluoromethyl)-1H-pyrrolo[2,3-c]pyridine-2-car baldehyde (50.0 mg, 113 µmol) in dichloromethane (2.00 mL) was added dropwise TEA (11.4 mg, 113 µmol, 15.7 µL) adjust pH = 7, N-methylethanamine (13.3 mg, 226 µmol, 19.4 µL) was added to the mixture, stirred at 25 °C for 0.5 hr, then NaBH(OAc)₃ (60.0 mg, 283 µmol) was added to the mixture, stirred at 25 °C for 1 hr. LCMS showed desired compound was detected. The reaction mixture was diluted with aqueous NaHCO₃ (8.00 mL), extracted with dichloromethane (12.0 mL * 3), the combined organic layers were washed with H₂O (8.00 mL), dried over Na₂SO₄, filtered and concentrated under reduced pressure to give a residue. The residue was purified by Prep-HPLC (column: Welch Xtimate C18 40 * 200 mm 7 um; mobile phase: [water (FA)-ACN]; gradient:0%-36.0% B over 25 mins). Compound 2-((ethyl(methyl)amino)methyl)-6-(3-(1-(4-methyl-4H-1,2,4-triazol-3-yl)cyclobutyl)phenyl)-4-(trifluoromethyl)-1, 6-dihydro-7H-pyrrolo[2,3-c]pyridin-7-one (13.1 mg, 27.0 µmol, 23.8% yield, 99.9% purity) was obtained as a colorless oil. Confirmed by H NMR and LCMS .
LCMS: m/z = 485.2 (M+H)⁺, Rt = 0.552 min
¹H NMR: (DMSO- *d*₆, 400 MHz)
*δ* 12.86 (s, 1H), 9.68 (s, 1H), 7.83 (d, *J=* 1.2 Hz, 1H), 7.54-7.62 (m, 2H), 7.42-7.48 (m, 2H), 6.78 (s, 1H), 4.47 (d, *J =* 3.2 Hz, 1H), 4.42 (d, *J* = 4.8 Hz, 1H), 3.47 (s, 3H), 3.14-3.23 (m, 1H), 2.96-3.06 (m, 3H), 2.76-2.84 (m, 2H), 2.66 (d, *J* = 4.4 Hz, 3H), 1.96-2.10 (m, 2H), 1.29 (t, *J =* 7.2 Hz, 3H)
LCMS: m/z = 485.2 (M+H)⁺, Rt = 1.290 min

### Example 3: Synthesis of compound T003

### 1. General steps for preparation of 4-bromo-7-methoxy-1-(p-tolylsulfonyl)pyrrolo[2,3-c]pyridine

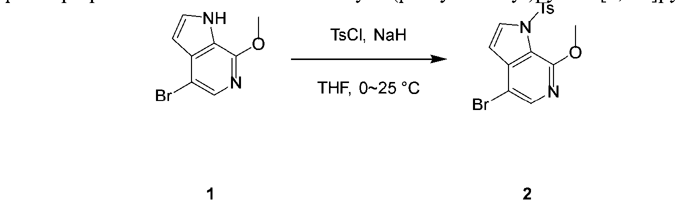

To a solution of 4-bromo-7-methoxy-1H-pyrrolo[2,3-c]pyridine (3.00 g, 13.2 mmol) in THF (30.0 mL) was added NaH (1.06 g, 26.4 mmol, 60% purity) at 0 °C under N₂. The mixture was stirred under N₂ at 0 °C for 0.5 hr. Then 4-methylbenzenesulfonyl chloride (3.78 g, 19.8 mmol) was added to the mixture at 0 °C under N₂. The mixture was stirred under N₂ at 25 °C under N₂ for 2 hrs. TLC (PE/ EtOAc = 5/ 1, product 1 R_{f} = 0.50) indicated new spot formed. The reaction mixture was poured into saturated aqueous NH₄Cl (50.0 mL) slowly. The mixture was extracted with EtOAc (50.0 mL), washed with saturated aqueous NH₄Cl (50.0 mL), brine (50.0 mL), dried over Na₂SO₄. The organic phase was concentrated in vacuum. The crude product was purified by column chromatography (SiO₂, PE/ EtOAc = 1/ 0 to 10/ 1). To afford 4-bromo-7-methoxy-1-(p-tolylsulfonyl)pyrrolo[2,3-c]pyridine (3.00 g, 7.05 mmol, 53.4% yield, 89.6% purity) as a yellow solid which was confirmed by H NMR and LCMS.
¹H NMR: (400 MHz, CDCl₃)
*δ* 7.98 (d, *J =* 3.6 Hz, 1H), 7.90 (s, 1H), 7.77 (d, *J =* 8.4 Hz, 2H), 7.29 (d, *J =* 8.4 Hz, 2H), 6.69 (d, *J =* 3.6 Hz, 1H), 3.89 (s, 3H), 2.41 (s, 3H)
LCMS: m/z = 382.9 (M+H)⁺, Rt = 1.730 min

### 2. General steps for preparation of 4-bromo-7-methoxy-1-(p-tolylsulfonyl)pyrrolo[2,3-c]pyridine-2-carbal dehyde

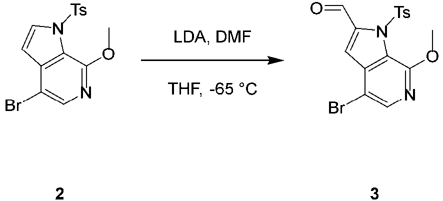

To a solution of 4-bromo-7-methoxy-1-(p-tolylsulfonyl)pyrrolo[2,3-c]pyridine (3.00 g, 7.87 mmol) in THF (30.0 mL) was added LDA (2 M in THF, 5.90 mL) at -65 °C under N₂. The mixture was stirred under N₂ at -65 °C for 0.5 hr. Then a solution of DMF (1.15 g, 15.7 mmol, 1.21 mL) in THF (10.0 mL) was added to the mixture at -65 °C under N₂. The mixture was stirred under N₂ at -65 °C under N₂ for 2 hrs. TLC (PE/ EtOAc = 5/ 1, product 1 R_{f} = 0.40) indicated new spot formed. LCMS showed desired mass was detected. The reaction mixture was poured into saturated aqueous NH₄Cl (50.0 mL) slowly. The mixture was extracted with EtOAc (50.0 mL), washed with saturated aqueous NH₄Cl (50.0 mL), brine (50.0 mL), dried over Na₂SO₄. The organic phase was concentrated in vacuum. The crude product was purified by column chromatography (SiO₂, PE/ EtOAc = 1/ 0 to 6/ 1). To afford 4-bromo-7-methoxy-1-(p-tolylsulfonyl)pyrrolo[2,3-c]pyridine-2-carbaldehyde (2.00 g, 4.89 mmol, 62.1% yield) as a yellow solid which was confirmed by H NMR.
¹H NMR: (400 MHz, CDCl₃)
*δ* 10.44 (s, 1H), 7.98 (s, 1H), 7.88 (d, *J =* 8.4 Hz, 2H), 7.38 (s, 1H), 7.34 (d, *J =* 8.4 Hz, 2H), 3.92 (s, 3H), 2.44 (s, 3H)

### 3. General steps for preparation of 4-bromo-7-methoxy-2-[[(3S)-3-methyl-1-piperidyl]methyl]-1-(p-tolylsu lfonyl)pyrrolo[2,3-c]pyridine

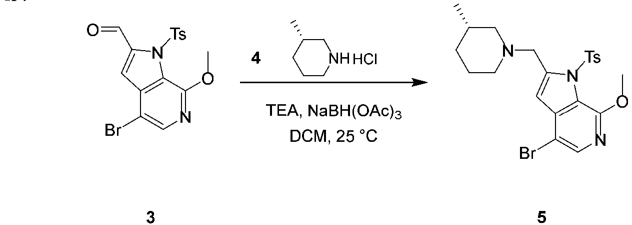

To a solution of (3S)-3-methylpiperidine (497 mg, 3.66 mmol, HCl) in DCM (20.0 mL) was added TEA (1.24 g, 12.2 mmol, 1.70 mL) adjust pH = 8. The mixture was added 4-bromo-7-methoxy-1-(p-tolylsulfonyl)pyrrolo[2,3-c]pyridine-2-carbaldehyde (1.00 g, 2.44 mmol) and stirred under N₂ at 25 °C for 0.5 hr. The NaBH(OAc)₃ (1.29 g, 6.11 mmol) was added to the mixture. The mixture was stirred under N₂ at 25 °C for 2 hrs. TLC (DCM/ MeOH = 10/ 1, product 1 R_{f} = 0.50) indicated new spot formed. The reaction mixture was diluted with DCM (20.0 mL), washed with brine (20.0 mL*2), dried over Na₂SO₄. The organic phase was concentrated in vacuum. The crude product was purified by column chromatography (SiO₂, DCM/ MeOH = 1/ 0 to 15/ 1). To afford 4-bromo-7-methoxy-2-[[(3S)-3-methyl-1-piperidyl]methyl]-1-(p-tolylsulfonyl)pyrrolo[2,3-c]'pyridine (1.10 g, 2.04 mmol, 83.6% yield, 91.5% purity) as a yellow solid which was confirmed by H NMR and LCMS .
¹H NMR: (400 MHz, CDCl₃)
*δ* 8.39 (d, *J* = 8.4 Hz, 2H), 7.90 (s, 1H), 7.30 (d, *J* = 8.0 Hz, 2H), 6.57 (s, 1H), 3.95 (s, 2H), 3.83 (s, 3H), 2.87-2.97 (m, 2H), 2.43 (s, 3H), 1.94-2.02 (m, 1H), 1.70-1.81 (m, 2H), 1.60-1.70 (m, 3H), 0.91-0.98 (m, 1H), 0.86 (br d, *J =* 5.6 Hz, 3H)
LCMS: m/z = 494.0 (M+H)⁺, Rt = 1.050 min

### 4. General steps for preparation of 7-methoxy-4-methyl-2-[[(3S)-3-methyl-1-piperidyl]methyl]-1-(p-tolyls ulfonyl)pyrrolo[2,3-c]pyridine

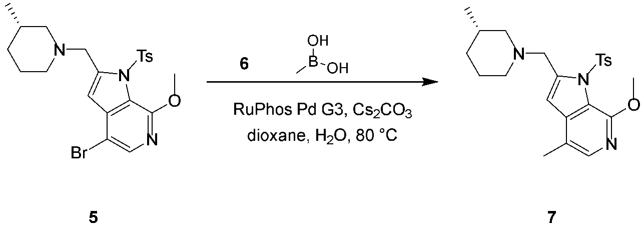

To a solution of 4-bromo-7-methoxy-2-[[(3S)-3-methyl-1-piperidyl]methyl]-1-(p-tolylsulfonyl)pyrrolo[2,3-c]pyridine (300 mg, 609 µmol) and methylboronic acid (365 mg, 6.09 mmol) in dioxane (12.0 mL) / H₂O (3.00 mL) was added Cs₂CO₃ (397 mg, 1.22 mmol) and RuPhos Pd G3 (51.0 mg, 60.9 µmol). The suspension was degassed under vacuum and purged with N₂ several times. The mixture was stirred under N₂ at 80 °C for 2 hrs. TLC (DCM/ MeOH = 10/ 1, product 1 R_{f} = 0.40) indicated new spot formed. The reaction mixture was diluted with EtOAc (20.0 mL). The mixture was filtered and the filter cake was washed with EtOAc (10 mL*3). The filter was washed with brine (50.0 mL*2), dried over Na₂SO₄. The organic phase was concentrated in vacuum. The crude product was purified by column chromatography (SiO₂, DCM/ MeOH = 1/ 0 to 15/ 1). To afford 7-methoxy-4-methyl-2-[[(3S)-3-methyl-1-piperidyl]methyl]-1-(p-tolylsulfonyl)pyrrolo[2,3-c]pyridine (250 mg, 566 µmol, 92.9% yield, 96.8% purity) as a yellow solid which was confirmed by H NMR and LCMS.
¹H NMR: (400 MHz, CDCl₃)
*δ* 8.37 (br d, *J =* 8.4 Hz, 2H), 7.62 (d, *J =* 0.8 Hz, 1H), 7.28 (d, *J =* 8.4 Hz, 2H), 6.51 (s, 1H), 3.95 (s, 2H), 3.81 (s, 3H), 2.89-3.00 (m, 2H), 2.42 (s, 3H), 2.34 (s, 3H), 1.92-2.02 (m, 1H), 1.76 (br d, *J =* 14.0 Hz, 2H), 1.60-1.68 (m, 3H), 0.90-0.99 (m, 1H), 0.86 (br d, *J =* 5.6 Hz, 3H)
LCMS: m/z = 428.2 (M+H)⁺, Rt = 0.927 min

### 5. General steps for preparation of 4-methyl-2-[[(3S)-3-methyl-1-piperidyl]methyl]-1-(p-tolylsulfonyl)-6H -pyrrolo[2,3-c]pyridin-7-one

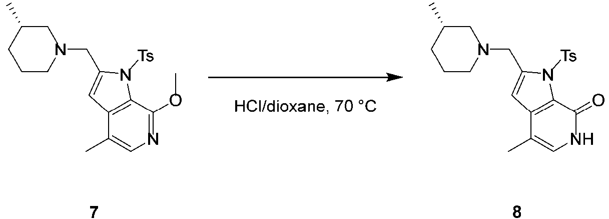

7-methoxy-4-methyl-2-[[(3S)-3-methyl-1-piperidyl]methyl]-1-(p-tolylsulfonyl)pyrrolo[2,3-c]pyridine (170 mg, 397 µmol) was added to a round-bottom flask, followed by HCl/dioxane (4 M, 3 mL). The mixture was stirred under N₂ at 70 °C for 3 hrs. TLC (DCM/ MeOH = 10/ 1, product 1 R_{f} = 0.30) indicated new spot formed. The reaction mixture was diluted with EtOAc (50.0 mL) poured into saturated aqueous NaHCO₃ (30.0 mL) slowly, adjust pH = 8 (saturated aqueous NaHCO₃). The organic phase was washed with brine (50.0 mL), dried over Na₂SO₄. The organic phase was concentrated in vacuum. The crude product was purified by column chromatography (SiO₂, DCM/ MeOH = 1/ 0 to 12/ 1). To afford 4-methyl-2-[[(3S)-3-methyl-1-piperidyl]methyl]-1-(p-tolylsulfonyl)-6H-pyrrolo[2,3-c]pyridin-7-one (150 mg, 323 µmol, 81.2% yield, 89.1% purity) as a yellow solid which was confirmed by H NMR and LCMS.
¹H NMR: (400 MHz, CDCl₃)
*δ* 9.96 (s, 1H), 8.49 (d, *J =* 8.4 Hz, 2H), 7.27 (s, 1H), 7.25 (s, 1H), 6.71 (s, 1H), 6.39 (s, 1H), 3.93 (s, 2H), 2.88-3.01 (m, 2H), 2.40 (s, 3H), 2.17 (d, *J =* 0.8 Hz, 3H), 1.96 (br t, *J =* 10.0 Hz, 1H), 1.65-1.79 (m, 4H), 1.51-1.60 (m, 1H), 0.91 (d, *J =* 12.8 Hz, 1H), 0.86 (d, *J =* 5.6 Hz, 3H)
LCMS: m/z = 414.2 (M+H)⁺, Rt = 1.030 min

### 6. General steps for preparation of 4-methyl-2-[[(3S)-3-methyl-1-piperidyl]methyl]-6-[3-[3-[(4-methyl-1,2,4-triazol-3-yl)methyl]oxetan-3-yl]phenyl]-1-(p-tolylsulfonyl)pyrrolo[2,3-c]pyridin-7-one

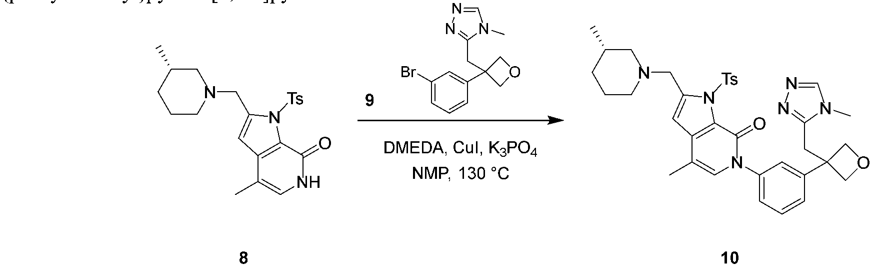

To a solution of 4-methyl-2-[[(3S)-3-methyl-1-piperidyl]methyl]-1-(p-tolylsulfonyl)-6H-pyrrolo[2,3-c]pyridin-7-one (88.0 mg, 212 µmol) and 3-[[3-(3-bromophenyl)oxetan-3-yl]methyl]-4-methyl-1,2,4-triazole (65.6 mg, 212 µmol) in NMP (1.00 mL) was added K₃PO₄ (135 mg, 635 µmol), DMEDA (37.5 mg, 425 µmol) and CuI (40.6 mg, 213 µmol). The suspension was degassed under vacuum and purged with N₂ several times. The mixture was stirred under N₂ at 130 °C for 12 hrs. TLC (DCM/ MeOH = 10/ 1, product 1 R_{f} = 0.35) indicated new spot formed. The reaction mixture was diluted with EtOAc (50.0 mL). The mixture was filtered and the filter cake was washed with EtOAc (10 mL*3). The filter was washed with brine (50.0 mL), dried over Na₂SO₄. The organic phase was concentrated in vacuum. The crude product was purified by column chromatography (SiO₂, DCM/ MeOH = 1/ 0 to 15/ 1). To afford
4-methyl-2-[[(3S)-3-methyl-1-piperidyl]methyl]-6-[3-[3-[(4-methyl-1,2,4-triazol-3-yl)methyl]oxetan-3-yl]phenyl]-1-(p-tolylsulfonyl)pyrrolo[2,3-c]pyridin-7-one (90.0 mg, 130 µmol, 61.1% yield, 92.7% purity) as a white solid which was confirmed by LCMS.
LCMS: m/z = 641.1 (M+H)⁺, Rt = 1.020 min

### 7. General steps for preparation of 4-methyl-2-[[(3S)-3-methyl-1-piperidyl]methyl]-6-[3-[3-[(4-methyl-1,2,4-triazol-3-yl)methyl]oxetan-3-yl]phenyl]-1H-pyrrolo[2,3-c]pyridin-7-one (compound T003)

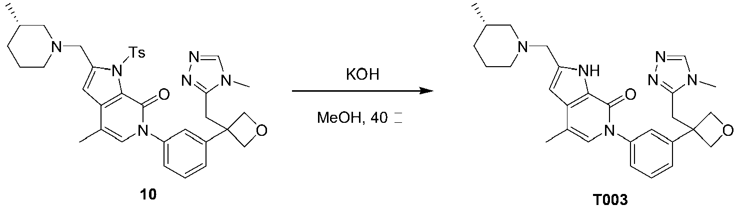

To a solution of 4-methyl-2-[[(3S)-3-methyl-1-piperidyl]methyl]-6-[3-[3-[(4-methyl-1,2,4-triazol-3-yl)methyl]oxetan-3-yl]phenyl]-1-(p-tolylsulfonyl)pyrrolo[2,3-c]pyridin-7-one (40.0 mg, 62.4 µmol) in MeOH (2.00 mL) was added KOH (105 mg, 1.87 mmol). The mixture was stirred under N₂ at 40 °C for 2 hrs. LCMS showed desired mass was detected. The reaction mixture was adjust pH = 8 (HCl, 2 M). The mixture was filtered to get a filtrate. The filtrate was purified by Prep-HPLC (column: Xtimate C18 150*40 mm*10 um; mobile phase: [water (NH₄HCO₃)-ACN]; gradient: 16.0%-56.0% B over 36 min). To afford 4-methyl-2-[[(3S)-3-methyl-1-piperidyl]methyl]-6-[3-[3-[(4-methyl-1,2,4-triazol-3-yl)methyl]oxetan-3-yl]phenyl]-1H-pyrrolo[2,3-c]pyridin-7-one (14.1 mg, 28.6 µmol, 45.9% yield, 99.02% purity) as a white solid which was confirmed by H NMR and LCMS.
¹H NMR: (400 MHz, DMSO-d6)
*δ* 11.94 (s, 1H), 8.20 (s, 1H), 7.34-7.40 (m, 1H), 7.26-7.32 (m, 1H), 6.98 (s, 1H), 6.90 (d, *J =* 7.6 Hz, 1H), 6.78 (d, *J* = 1.2 Hz, 1H), 6.21 (s, 1H), 4.92-4.96 (m, 2H), 4.88 (d, *J =* 6.0 Hz, 2H), 3.56 (s, 2H), 3.50 (s, 2H), 2.95 (s, 3H), 2.73-2.81 (m, 2H), 2.17 (s, 3H), 1.84-1.92 (m, 1H), 1.54-1.64 (m, 4H), 1.40-1.49 (m, 1H), 0.81 (d, *J =* 5.6 Hz, 3H), 0.72-0.80 (m, 1H)
LCMS: m/z = 487.3 (M+H)⁺, Rt = 1.628 min

### Example 4: Synthesis of compound T004

### 1. General steps for preparation of 4-bromo-6-(3-((1s,3R)-3-methyl-1-(4-methyl-4H-1,2,4-triazol-3-yl)cyclobutyl)phenyl)-2-(((S)-3-methylpiperidin-1 -yl)methyl)-1-tosyl-1,6-dihydro-7H-pyrrolo[2,3-c]pyridin-7-one

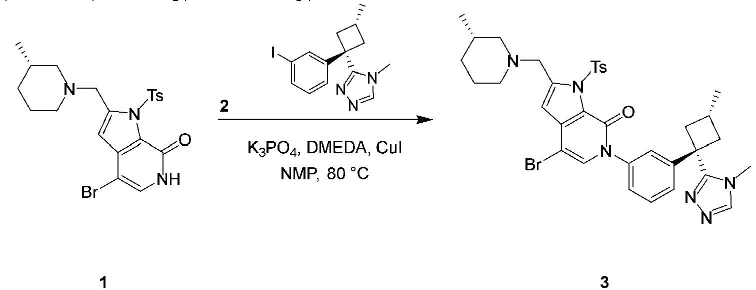

To a solution of 4-bromo-2-[[(3S)-3-methyl-1-piperidyl]methyl]-1-(p-tolylsulfonyl)-6H-pyrrolo[2,3-c]pyridin-7-one (500 mg, 1.05 mmol) and 3-((1s,3s)-1-(3-iodophenyl)-3-methylcyclobutyl)-4-methyl-4H-1,2,4-triazole (369 mg, 1.04 mmol) in NMP (5.00 mL) was added K₃PO₄ (665 mg, 3.13 mmol), CuI (398 mg, 2.09 mmol) and DMEDA (92.1 mg, 1.04 mmol, 112 µL). The suspension was degassed under vacuum and purged with N₂ several times. The mixture was stirred under N₂ at 80 °C for 4 hrs. TLC (Dichloromethane/ Methanol = 10/ 1, product 1 R_{f} = 0.35) indicated new spot formed. The reaction mixture was poured into saturated aqueous NH₄Cl (50.0 mL) slowly. The mixture was extracted with Ethyl acetate (10.0 mL*2). The organic phase was washed with brine (20.0 mL*2), dried over Na₂SO₄ and concentrated in vacuum. The crude product was purified by column chromatography (SiO₂, Dichloromethane/ Methanol = 1/ 0 to 32.3/ 1). To afford 4-bromo-6-(3-((1s,3R)-3-methyl-1-(4-methyl-4H-1,2,4-triazol-3-yl)cyclobutyl)phenyl)-2-(((S)-3-methylpiperidin-1 -yl)methyl)-1-tosyl-1,6-dihydro-7H-pyrrolo[2,3-c]pyridin-7-one (350 mg, 417 µmol, 39.9% yield, 84.0% purity) as a yellow solid which was confirmed by H NMR and LCMS.
¹H NMR: (400 MHz, CDCl₃)
*δ* 8.48 (d, *J =* 8.0 Hz, 2H), 7.56-7.76 (m, 1H), 7.28-7.45 (m, 3H), 7.16-7.24 (m, 4H), 6.26-6.51 (m, 1H), 3.94 (s, 2H), 3.18-3.26 (m, 3H), 2.88-2.97 (m, 2H), 2.79-2.87 (m, 3H), 2.61-2.70 (m, 4H), 2.42 (s, 3H), 1.97 (t, J= 10.4 Hz, 1H), 1.55-1.66 (m, 3H), 1.13 (d, *J =* 5.6 Hz, 3H), 0.90-0.99 (m, 1H), 0.87 (d, *J =* 5.2 Hz, 3H)
LCMS: m/z = 704.8 (M+H)⁺, Rt = 1.275 min

### 2. General steps for preparation of 6-(3-((1s,3R)-3-methyl-1-(4-methyl-4H-1,2,4-triazol-3-yl)cyclobutyl)phenyl)-2-(((S)-3-methylpiperidin-1-yl)methy l)-1-tosyl-4-vinyl-1,6-dihydro-7H-pyrrolo[2,3-c]pyridin-7-one

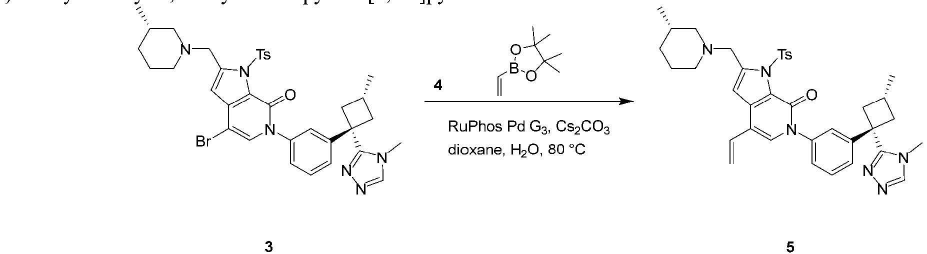

To a solution of 4-bromo-6-(3-((1s,3R)-3-methyl-1-(4-methyl-4H-1,2,4-triazol-3-yl)cyclobutyl)phenyl)-2-(((S)-3-methylpiperidin-1 -yl)methyl)-1-tosyl-1,6-dihydro-7H-pyrrolo[2,3-c]pyridin-7-one (50.0 mg, 71.0 µmol), 4,4,5,5-tetramethyl-2-vinyl-1,3,2-dioxaborolane (32.8 mg, 212 µmol) in dioxane (1.00 mL), H₂O (0.30 mL) was added Cs₂CO₃ (46.3 mg, 142 µmol), RuPhos Pd G3 (11.9 mg, 14.2 µmol). The mixture was stirred at 80 °C for 2 hrs. TLC (Dichloromethane/ Methanol = 10/ 1, product 1 R_{f} = 0.37) indicated new spot formed. The reaction mixture was diluted with Ethyl acetate (20.0 mL). The mixture was filtered and the filter cake was washed with Ethyl acetate (10.0 mL*3). The filtrate was washed with brine (50.0 mL), dried over Na₂SO₄. The organic phase was concentrated in vacuum. The crude product was purified by column chromatography (SiO₂, Dichloromethane/ Methanol = 1/ 0 to 32.3/ 1). To afford 6-(3-((1s,3R)-3-methyl-1-(4-methyl-4H-1,2,4-triazol-3-yl)cyclobutyl)phenyl)-2-(((S)-3-methylpiperidin-1-yl)methy l)-1-tosyl-4-vinyl-1,6-dihydro-7H-pyrrolo[2,3-c]pyridin-7-one (45.0 mg, 67.6 µmol, 95.2% yield, 97.9% purity) as a yellow solid which was confirmed LCMS.
LCMS: m/z = 651.2 (M+H)⁺, Rt = 1.202 min

### 3. General steps for preparation of 6-(3-((1s,3R)-3-methyl-1-(4-methyl-4H-1,2,4-triazol-3-yl)cyclobutyl)phenyl)-2-(((S)-3-methylpiperidin-1-yl)methy l)-4-vinyl-1,6-dihydro-7H-pyrrolo[2,3-c]pyridin-7-one (compound T004)

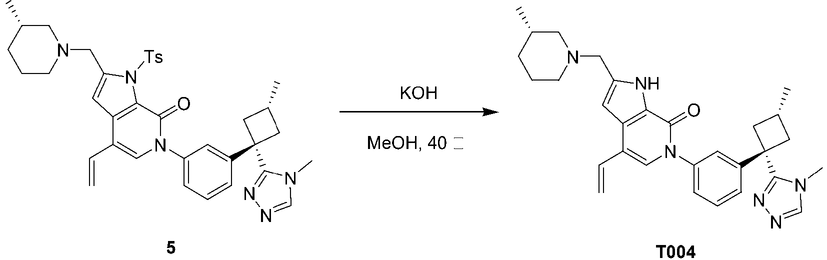

To a solution of 6-(3-((1s,3R)-3-methyl-1-(4-methyl-4H-1,2,4-triazol-3-yl)cyclobutyl)phenyl)-2-(((S)-3-methylpiperidin-1-yl)methy l)-1-tosyl-4-vinyl-1,6-dihydro-7H-pyrrolo[2,3-c]pyridin-7-one (45.0 mg, 69.1 µmol) in MeOH (3.00 mL) was added KOH (116 mg, 2.07 mmol). The mixture was stirred under N₂ at 40 °C for 1hr. LCMS showed desired mass was detected. The reaction mixture was diluted with brine (20.0 mL), extracted with EtOAc (20.0 mL*2). The organic phase washed with KOH (1 M, 20 mL), dried over Na₂SO₄ and concentrated in vacuum. The crude product was purified by Prep-HPLC (column: Xtimate C18 150*40 mm*10 um; mobile phase: [water (NH₃H₂O+NH₄HCO₃)-ACN]; gradient:28.0%-68.0% B over 32 mins). To afford 6-(3-((1s,3R)-3-methyl-1-(4-methyl-4H-1,2,4-triazol-3-yl)cyclobutyl)phenyl)-2-(((S)-3-methylpiperidin-1-yl)methy l)-4-vinyl-1,6-dihydro-7H-pyrrolo[2,3-c]pyridin-7-one (9.40 mg, 18.9 µmol, 27.3% yield, 100% purity) as a white solid which was confirmed by H NMR and LCMS.
¹H NMR: (400 MHz, DMSO-d₆)
*δ* 12.10 (s, 1H), 8.28 (s, 1H), 7.48-7.55 (m, 1H), 7.43-7.47 (m, 1H), 7.34 (s, 1H), 7.29-7.33 (m, 2H), 6.70 (dd, *J =* 17.6, 11.6 Hz, 1H), 6.56 (s, 1H), 5.73 (d, *J =* 17.6 Hz, 1H), 5.19 (d, *J =* 11.6 Hz, 1H), 3.59 (s, 2H), 3.25 (s, 3H), 2.88 (d, *J =* 3.2 Hz, 2H), 2.73-2.81 (m, 2H), 2.53 (d, *J =* 6.4 Hz, 3H), 1.88 (t, *J =* 10.4 Hz, 1H), 1.55-1.66 (m, 4H), 1.40-1.49 (m, 1H), 1.07 (d, *J =* 5.2 Hz, 3H), 0.80 (d, *J =* 5.6 Hz, 3H), 0.68-0.79 (m, 1H)
LCMS: m/z = 497.3 (M+H)⁺, Rt = 1.525 min

### Example 5: Synthesis of compound T005

### 1. General steps for preparation of 2-(diethylaminomethyl)-6-[3-[1-(4-methyl-1,2,4-triazol-3-yl)cyclobutyl]phenyl]-4-(trifluoromethyl)-1H-pyrrolo[2, 3-c]pyridin-7-one (compound T005)

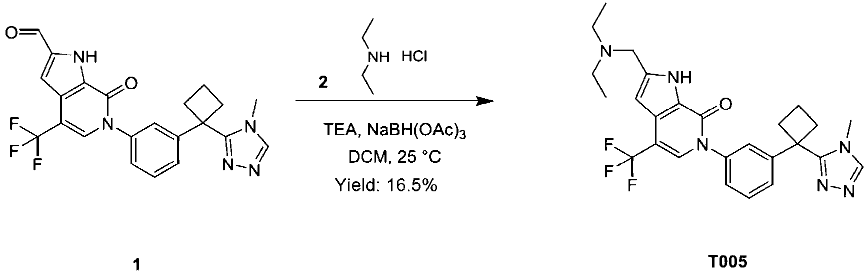

To a solution of 6-[3-[1-(4-methyl-1,2,4-triazol-3-yl)cyclobutyl]phenyl]-7-oxo-4-(trifluoromethyl)-1H-pyrrolo[2,3-c]pyridine-2-car baldehyde (50.0 mg, 113 µmol) in dichloromethane (2.50 mL) was added dropwise TEA (11.4 mg, 113 µmol, 15.7 µL) adjust pH = 7, N-ethylethanamine (18.6 mg, 169 µmol, 26.2 µL, HCl) was added to the mixture, stirred at 25 °C for 0.5 hr, then NaBH(OAc)₃ (60.0 mg, 283 µmol) was added to the mixture, stirred at 25 °C for 1 hr. LCMS showed desired compound was detected. The reaction mixture was diluted with aqueous NaHCO₃ (8.00 mL), extracted with dichloromethane (12.0 mL * 3), the combined organic layers were washed with H₂O (8.00 mL), dried over Na₂SO₄, filtered and concentrated under reduced pressure to give a residue. The residue was purified by prep-HPLC (column: Welch Xtimate C18 40 * 200 mm 7 um; mobile phase: [water (HCl)-ACN]; gradient:0%-38% B over 20.5 min). Compound 2-(diethylaminomethyl)-6-[3-[1-(4-methyl-1,2,4-triazol-3-yl)cyclobutyl]phenyl]-4-(trifluoromethyl)-1H-pyrrolo[2, 3-c]pyridin-7-one (10.1 mg, 18.7 µmol, 16.5% yield, 99.3% purity, HCl) was obtained as a white solid. Confirmed by H NMR and LCMS.
¹H NMR: (DMSO-d₆, 400 MHz)
*δ* 12.84 (s, 1H), 9.26 (s, 1H), 7.83 (d, *J=* 1.2 Hz, 1H), 7.54-7.60 (m, 1H), 7.52 (s, 1H), 7.42-7.46 (m, 1H), 7.39 (d, *J* = 8.0 Hz, 1H), 6.79 (s, 1H), 4.45 (d, *J* = 4.8 Hz, 2H), 3.40 (s, 3H), 3.05-3.15 (m, 4H), 2.94-3.02 (m, 2H), 2.72-2.81 (m, 2H), 2.04-2.10 (m, 1H), 1.98-2.03 (m, 1H), 1.27 (t, *J =* 7.2 Hz, 6H)
LCMS: m/z = 499.2 (M+H)⁺, Rt = 1.333 min

### Example 6: Synthesis of compound T006

### 1. General steps for preparation of 4-bromo-7-methoxy-2-(1-piperidylmethyl)-1-(p-tolylsulfonyl)pyrrolo[2,3-c]pyridine

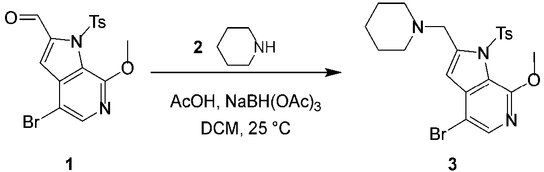

To a solution of piperidine (124 mg, 1.47 mmol, 144 µL) in DCM (4 mL) was added dropwise AcOH (5.87 mg, 97.7 µmol, 5.60 µL) adjust pH = 6, then 4-bromo-7-methoxy-1-(p-tolylsulfonyl)pyrrolo[2,3-c]pyridine-2-carbaldehyde (400 mg, 977 µmol) was added to the mixture, the mixture was stirred under N₂ at 25 °C for 30 min, and then NaBH(OAc)₃ (517 mg, 2.44 mmol) was added to the mixture. The resulting mixture was stirred under N₂ at 25 °C for 2 hrs. LCMS showed desired compound was detected. The reaction mixture was diluted with H₂O (30 mL), extracted with EtOAc 60 mL (20 mL * 3), the combined organic layers were washed with H₂O (20 mL * 3), dried over Na₂SO₄, filtered and concentrated under reduced pressure to give a residue. The residue was purified by column chromatography (SiO₂, PE/ EA =0% to 10%), (Plate 1, PE/ EA = 3/ 1, R_{f} (product) = 0.6). Compound 4-bromo-7-methoxy-2-(1-piperidylmethyl)-1-(p-tolylsulfonyl)pyrrolo[2,3-c]pyridine (364 mg, 677 µmol, 69.2% yield, 89.0% purity) was obtained as a white solid. Confirmed by H NMR and LCMS.
¹H NMR: (CDCl₃, 400 MHz)
*δ* 8.40 (d, *J =* 8.4 Hz, 2H), 7.89 (s, 1H), 7.30 (d, *J =* 8.0 Hz, 2H), 6.57 (s, 1H), 3.95 (s, 2H), 3.83 (s, 3H), 2.51 (br s, 4H), 2.44 (s, 3H), 1.58-1.64 (m, 4H), 1.47-1.54 (m, 2H)
LCMS: m/z = 480.0 (M+3)⁺, Rt = 1.872 min

### 2. General steps for preparation of 4-cyclopropyl-7-methoxy-2-(1-piperidylmethyl)-1-(p-tolylsulfonyl)pyrrolo[2,3-c]pyridine:

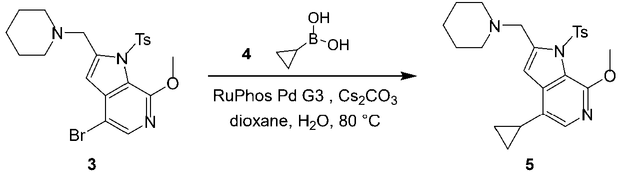

To a solution of 4-bromo-7-methoxy-2-(1-piperidylmethyl)-1-(p-tolylsulfonyl)pyrrolo[2,3-c]pyridine (364 mg, 760 µmol), cyclopropylboronic acid (653 mg, 7.61 mmol) in dioxane (15 mL) was added Cs₂CO₃ (495 mg, 1.52 mmol) in H₂O (4 mL) and RuPhos Pd G3 (63.6 mg, 76.0 µmol). After addition, the mixture was stirred under N₂ at 80 °C for 2 hrs. LCMS showed desired compound was detected. Filtered and the reaction mixture was diluted with H₂O (30 mL), extracted with EtOAc (20 mL * 3), the combined organic layers were washed with H₂O (20 mL * 3), dried over Na₂SO₄, concentrated under reduced pressure to give a residue. The residue was purified by column chromatography (SiO₂, PE/ EA = 0% to 40%), (Plate 1, PE/ EA = 1/ 1, R_{f} (product) = 0.53). Compound 4-cyclopropyl-7-methoxy-2-(1-piperidylmethyl)-1-(p-tolylsulfonyl)pyrrolo[2,3-c]pyridine (190 mg, 381 µmol, 50.1% yield, 88.2% purity) was obtained as a white solid. Confirmed by H NMR and LCMS.
¹H NMR: (CDCl₃, 400 MHz)
*δ* 8.40 (br d, *J* = 8.0 Hz, 2H), 7.55 (d, *J =* 0.4 Hz, 1H), 7.29 (d, *J =* 8.4 Hz, 2H), 6.67 (s, 1H), 3.96 (s, 2H), 3.80 (s, 3H), 2.46-2.61 (m, 4H), 2.42 (s, 3H), 1.89-1.99 (m, 1H), 1.58-1.64 (m, 4H), 1.50 (br d, *J* = 4.4 Hz, 2H), 0.89-0.96 (m, 2H), 0.65-0.70 (m, 2H)
LCMS: m/z = 440.1 (M+H)⁺, Rt = 0.817 min

### 3. General steps for preparation of 4-cyclopropyl-2-(1-piperidylmethyl)-1-(p-tolylsulfonyl)-6H-pyrrolo[2,3-clpyridin-7-one

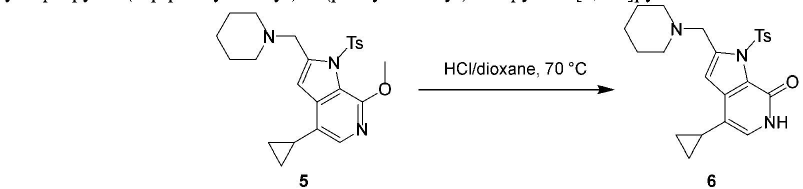

A mixture of 4-cyclopropyl-7-methoxy-2-(1-piperidylmethyl)-1-(p-tolylsulfonyl)pyrrolo[2,3-c]pyridine (190 mg, 432 µmol) in HCl/ dioxane (4 M, 1 mL) was degassed and purged with N₂ for 3 times, and then the mixture was stirred at 70 °C for 2.5 hrs under N₂ atmosphere. LCMS showed desired compound was detected. The reaction mixture was diluted with aqueous NaHCO₃ (15 mL), extracted with EtOAc (20 mL * 3), the combined organic layers were washed with H₂O (20 mL * 3), dried over Na₂SO₄, filtered and concentrated under reduced pressure to give a residue. The residue was purified by column chromatography (SiO₂, DCM/ MeOH=0% to 10%), (Plate 1, DCM/ MeOH = 10/ 1, R (produt) = 0.45). Compound 4-cyclopropyl-2-(1-piperidylmethyl)-1-(p-tolylsulfonyl)-6H-pyrrolo[2,3-c]pyridin-7-one (174 mg, 385 µmol, 89.1% yield, 94.2% purity) was obtained as a white solid. Confirmed by H NMR and LCMS.
1H NMR: (DMSO-d6, 400 MHz)
*δ* 10.90 (br d, *J =* 5.6 Hz, 1H), 8.45 (d, *J =* 8.4 Hz, 2H), 7.38 (d, *J =* 8.4 Hz, 2H), 6.67-6.74 (m, 2H), 3.84 (s, 2H), 2.35-2.45 (m, 7H), 1.72-1.85 (m, 1H), 1.51 (br d, *J* = 4.4 Hz, 4H), 1.44 (br s, 2H), 0.74-0.83 (m, 2H), 0.44-0.55 (m, 2H)
LCMS: m/z = 425.8 (M+H)⁺, Rt = 0.990 min

### 4. General steps for preparation of 4-cyclopropyl-6-[3-[3-[(4-methyl-1,2,4-triazol-3-yl)methyl]oxetan-3-yl]phenyl]-2-(1-piperidylmethyl)-1-(p-tolylsul fonyl)pyrrolo[2,3-c]pyridin-7-one

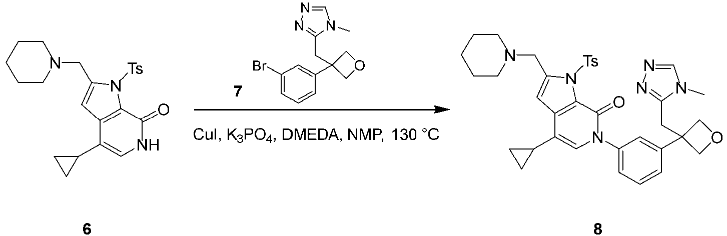

To a solution of 4-cyclopropyl-2-(1-piperidylmethyl)-1-(p-tolylsulfonyl)-6H-pyrrolo[2,3-c]pyridin-7-one (80 mg, 188 µmol) and 3-[[3-(3-bromophenyl)oxetan-3-yl]methyl]-4-methyl-1,2,4-triazole (57.9 mg, 188 µmol) and CuI (35.8 mg, 188 umol), K₃PO₄ (119 mg, 563 µmol) in NMP (1 mL) was added DMEDA (33.1 mg, 375 µmol, 40.4 µL). After addition, the mixture was stirred at 130 °C for 12 hrs under N₂. LCMS showed desired compound was detected. The reaction mixture was diluted with brine (30 mL), extracted with EtOAc (20 mL * 3), the combined organic layers were washed with brine (20 mL * 3), dried over Na₂SO₄, filtered and concentrated under reduced pressure to give a residue. The residue was purified by column chromatography (SiO₂, PE/ EA = 0% to 15%), (Plate 1, PE/ EA = 10/ 1, R_{f} (product) = 0.42). Compound 4-cyclopropyl-6-[3-[3-[(4-methyl-1,2,4-triazol-3-yl)methyl]oxetan-3-yl]phenyl]-2-(1-piperidylmethyl)-1-(p-tolylsul fonyl)pyrrolo[2,3-c]pyridin-7-one (95.0 mg, 106 µmol, 56.7% yield, 73.3% purity) was obtained as a yellow oil. Confirmed by LCMS.
LCMS: m/z = 653.1 (M+H)⁺, Rt = 1.043 min

### 5. General steps for preparation of 4-cyclopropyl-2-(1-piperidylmethyl)-1-(p-tolylsulfonyl)-6H-pyrrolo[2,3-c]pyridin-7-one (compound T006)

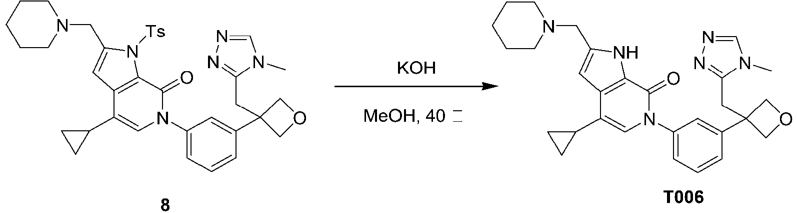

A mixture of 4-cyclopropyl-6-[3-[3-[(4-methyl-1,2,4-triazol-3-yl)methyl]oxetan-3-yl]phenyl]-2-(1-piperidylmethyl)-1-(p-tolylsul fonyl)pyrrolo[2,3-c]pyridin-7-one (95.0 mg, 145 µmol), KOH (122 mg, 2.18 mmol) in MeOH (4.0 mL) was degassed and purged with N₂ for 3 times, and then the mixture was stirred at 40 °C for 4 hrs under N₂ atmosphere. LCMS showed desired compound was detected. Filtered and concentrated under reduced pressure to give a residue. The residue was purified by Prep-HPLC (column: Xtimate C18 150 * 40 mm * 10 um; mobile phase: [water (NH₄HCO₃)-ACN]; gradient: 18%-58% B over 36 min). Compound 4-cyclopropyl-6-[3-[3-[(4-methyl-1,2,4-triazol-3-yl)methyl]oxetan-3-yl]phenyl]-2-(1-piperidylmethyl)-1H-pyrrolo[ 2,3-c]pyridin-7-one (9.40 mg, 18.7 µmol, 12.9% yield, 99.61% purity) was obtained as a white solid. Confirmed by H NMR and LCMS.
¹H NMR: (DMSO-d₆, 400 MHz)
*δ* 11.94 (br s, 1H), 8.19 (s, 1H), 7.37 (t, *J =* 7.6 Hz, 1H), 7.27 (d, *J =* 8.0 Hz, 1H), 6.97 (s, 1H), 6.93 (d, *J =* 7.6 Hz, 1H), 6.63 (s, 1H), 6.31 (s, 1H), 4.91-4.97 (m, 2H), 4.88 (d, *J =* 6.0 Hz, 2H), 3.56 (s, 2H), 3.50 (s, 2H), 2.97 (s, 3H), 2.29-2.43 (m, 4H), 1.82-1.95 (m, 1H), 1.45-1.55 (m, 4H), 1.30-1.41 (m, 2H), 0.75-0.84 (m, 2H), 0.61-0.71 (m, 2H)
LCMS: m/z = 499.2 (M+H)⁺, Rt = 1.223 min

### Example 7: Synthesis of compound T007

### 1. General steps for preparation of 2-((3-azabicyclo[3.1.0]hexan-3-yl)methyl)-6-(3-(1-(4-methyl-4H-1,2,4-triazol-3-yl)cyclobutyl)phenyl)-4-(trifluoro methyl)-1,6-dihydro-7H-pyrrolo[2,3-c]pyridin-7-one (compound T007):

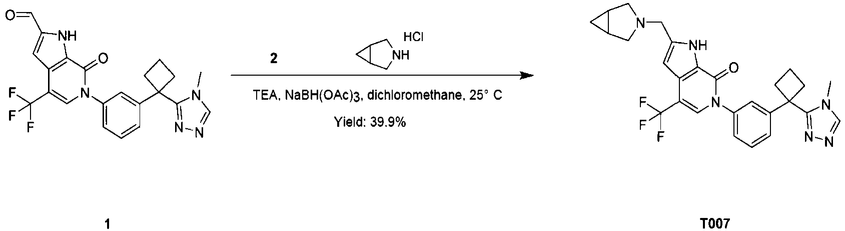

To a solution of 6-[3-[1-(4-methyl-1,2,4-triazol-3-yl)cyclobutyl]phenyl]-7-oxo-4-(trifluoromethyl)-1H-pyrrolo[2,3-c]pyridine-2-car baldehyde (50.0 mg, 113 µmol) in dichloromethane (2.50 mL) was added dropwise TEA (11.4 mg, 113 µmol, 15.7 µL) adjust pH = 7, 3-azabicyclo[3.1.0]hexane (14.1 mg, 118 µmol, HCl) was added to the mixture, stirred at 25 °C for 0.5 hr, then NaBH(OAc)₃ (60.0 mg, 283 µmol) was added to the mixture, stirred at 25 °C for 1 hr. LCMS showed desired compound was detected. The reaction mixture was diluted with aqueous NaHCO₃ (8.00 mL), extracted with dichloromethane (12.0 mL * 3), the combined organic layers were washed with H₂O (8.00 mL), dried over Na₂SO₄, filtered and concentrated under re duced pressure to give a residue. The residue was purified by prep-HPLC (column: Welch Xtimate C18 40 * 200 mm 7 um; mobile phase: [water (HCl)-ACN]; gradient:0%-38% B over 30 min). Compound 2-((3-azabicyclo[3.1.0]hexan-3-yl)methyl)-6-(3-(1-(4-methyl-4H-1,2,4-triazol-3-yl)cyclobutyl)phenyl)-4-(trifluoro methyl)-1,6-dihydro-7H-pyrrolo[2,3-c]pyridin-7-one (24.8 mg, 45.2 µmol, 39.9% yield, 99.5% purity, HCl) was obtained as a white solid. Confirmed by H NMR and LCMS.
¹H NMR: (DMSO-d₆, 400 MHz)
*δ* 12.72 (s, 1H), 9.19 (s, 1H), 7.82 (d, *J =* 1.2 Hz, 1H), 7.54-7.60 (m, 1H), 7.51 (s, 1H), 7.43 (d, *J =* 7.6 Hz, 1H), 7.38 (d, *J* = 7.6 Hz, 1H), 6.74 (s, 1H), 4.47 (d, *J* = 2.8 Hz, 2H), 3.41 (s, 1H), 3.39 (s, 3H), 3.34 (s, 2H), 2.94-3.02 (m, 2H), 2.70-2.80 (m, 2H), 1.94-2.08 (m, 2H), 1.75 (s, 2H), 1.23 (s, 1H), 1.06 (q, *J* = 4.4 Hz, 1H), 0.50-0.73 (m, 1H)
LCMS: m/z =509.2 (M+H)⁺, Rt = 1.337 min

### Example 8: Synthesis of compound T008

### 1. General steps for preparation of 2-[(2-cyclopropylethylamino)methyl]-6-[3-[1-(4-methyl-1,2,4-triazol-3-yl)cyclobutyl|phenyl]-4-(trifluoromethyl)-1 H-pyrrolo[2,3-c]pyridin-7-one (compound T008)

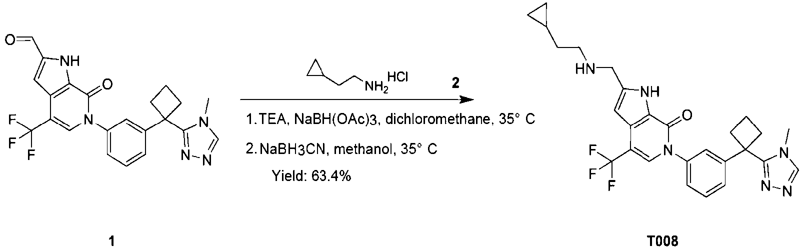

To a mixture of 2-cyclopropylethanamine (27.6 mg, 226 µmol, HCl) in Dichloromethane (3.00 mL) was added TEA (57.3 mg, 566 µmol) adjust pH = 8. Then 6-[3-[1-(4-methyl-1,2,4-triazol-3-yl)cyclobutyl]phenyl]-7-oxo-4-(trifluoromethyl)-1H-pyrrolo[2,3-c]pyridine-2-car baldehyde (50.0 mg, 113 µmol) was added to the mixture. The mixture was stirred under N₂ at 35 °C for 0.5 hr. NaBH(OAc)₃ (60.0 mg, 283 µmol) was added to the mixture. The mixture was stirred under N₂ at 35 °C for 2.5 hrs. Methanol (1.00 mL) and NaBH₃CN (7.12 mg, 113 µmol) was added to the mixture. The mixture was stirred under N₂ at 35 °C for 2 hrs. LCMS showed desired mass was detected. The reaction mixture was diluted with NaHCO₃ (3.00 mL), extracted with Dichloromethane (2.00 mL*2). The organic phase dried over Na₂SO₄ and concentrated in vacuum. The crude product was purified by Prep-HPLC (column: Welch Xtimate C18 40*200 mm 7 um; mobile phase: [water (NH₄HCO₃)-ACN]; gradient: 22.0%-62.0% B over 25 min). To afford 2-[(2-cyclopropylethylamino)methyl]-6-[3-[1-(4-methyl-1,2,4-triazol-3-yl)cyclobutyl]phenyl]-4-(trifluoromethyl)-1 H-pyrrolo[2,3-c]pyridin-7-one (36.7 mg, 71.8 µmol, 63.4% yield, 100% purity) as an off-white solid which was confirmed by H NMR and LCMS.
¹H NMR: (400 MHz, DMSO-d₆)
*δ* 12.00 (s, 1H), 8.35 (s, 1H), 7.70 (s, 1H), 7.47-7.54 (m, 1H), 7.43 (s, 1H), 7.33-7.39 (m, 1H), 7.26 (d, *J =* 8.0 Hz, 1H), 6.33 (s, 1H), 3.80 (s, 2H), 3.27 (s, 3H), 2.90-2.97 (m, 2H), 2.66-2.73 (m, 2H), 2.52-2.55 (m, 2H), 1.93-2.03 (m, 2H), 1.31 (q, *J* = 7.2 Hz, 2H), 0.63-0.77 (m, 1H), 0.33-0.39 (m, 2H), -0.01 (q, *J =* 4.8 Hz, 2H)
LCMS: m/z = 511.3 (M+H)⁺, Rt = 1.480 min

### Example 9: Synthesis of compound T009

### 1. General steps for preparation of 2-[[(1-methylcyclobutyl)amino]methyl]-6-[3-[1-(4-methyl-1,2,4-triazol-3-yl)cyclobutyl]phenyl]-4-(trifluoromethyl )-1H-pyrrolo[2,3-c]pyridin-7-one (compound T009)

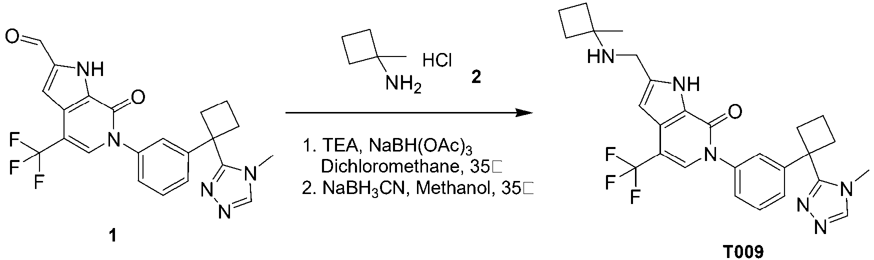

To a mixture of 1-methylcyclobutanamine (276 mg, 2.27 mmol, HCl) in Dichloromethane (15.0 mL) was added TEA (573 mg, 5.66 mmol) adjust pH = 8. Then 6-[3-[1-(4-methyl-1,2,4-triazol-3-yl)cyclobutyl]phenyl]-7-oxo-4-(trifluoromethyl)-1H-pyrrolo[2,3-c]pyridine-2-car baldehyde (500 mg, 1.13 mmol) was added to the mixture. The mixture was stirred under N₂ at 35 °C for 0.5 hr. NaBH(OAc)₃ (600 mg, 2.83 mmol) was added to the mixture. The mixture was stirred under N₂ at 35 °C for 2.5 hrs. Then Methanol (4.00 mL) and NaBH₃CN (214 mg, 3.41 mmol) was added to the mixture. The mixture was stirred under N₂ at 35 °C for 2 hrs. LCMS showed desired mass was detected. The reaction mixture was diluted with NaHCO₃ (3.00 mL), extracted with Dichloromethane (2.00 mL*2). The organic phase dried over Na₂SO₄ and concentrated in vacuum. The crude product was purified by Prep-HPLC (column: Welch Xtimate C18 40*200 mm 7 um; mobile phase: [water (FA)-ACN]; gradient: 0%-38.0% B over 25 min). To afford 2-[[(1-methylcyclobutyl)amino]methyl]-6-[3-[1-(4-methyl-1,2,4-triazol-3-yl)cyclobutyl]phenyl]-4-(triffuoromethyl )-1H-pyrrolo[2,3-c]pyridin-7-one (302 mg, 552 µmol, 48.7% yield, 100% purity, HCl) as a white solid which was confirmed by H NMR and LCMS.
¹H NMR: (400 MHz, DMSO-d₆)
*δ* 12.75 (s, 1H), 9.80 (s, 1H), 9.21 (s, 1H), 7.81 (s, 1H), 7.53-7.60 (m, 1H), 7.51 (s, 1H), 7.43 (d, *J =* 7.6 Hz, 1H), 7.38 (d, *J =* 7.6 Hz, 1H), 6.75 (s, 1H), 4.18-4.22 (m, 2H), 3.40 (s, 3H), 2.94-3.03 (m, 2H), 2.72-2.81 (m, 2H), 2.44-2.49 (m, 2H), 1.96-2.10 (m, 2H), 1.80-1.92 (m, 4H), 1.54 (s, 3H)
LCMS: m/z = 511.2 (M+H)⁺, Rt = 1.358 min

### Example 10: Synthesis of compound T010

### 1. General steps for preparation of ethyl 2-[1-(3-bromophenyl)cyclobutyl]acetate

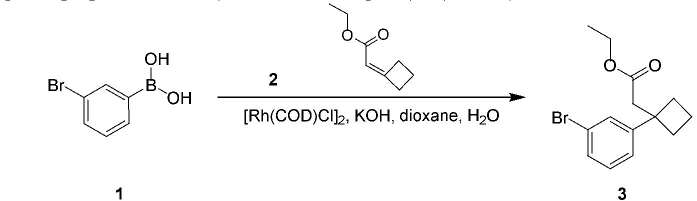

To a solution of ethyl 2-cyclobutylideneacetate (10.0 g, 71.3 mmol) in dioxane (80.0 mL) was added KOH (6.00 g, 107 mmol) in H₂O (6.00 mL) then add [Rh(COD)Cl]₂ (1.76 g, 3.57 mmol), then add (3-bromophenyl)boronic acid (18.6 g, 92.7 mmol) to the mixture in 10 potions in 1 hr and keep the inner temperature below 15 °C. The mixture was stirred at 30 °C for 12 hrs. LCMS showed desired compound was detected. The reaction mixture was concentrated under reduced pressure and EtOAc (100 mL) was added to the dry residue. The solution was washed with brine (80.0 mL * 3), dried over Na₂SO₄, filtered and concentrated under reduced pressure to give a residue. The residue was purified by column chromatography (SiO₂, PE/ EA = 0% to 10%), (Plate 1, PE/ EA = 10/ 1, R_{f} (product) = 0.74). Compound ethyl 2-[1-(3-bromophenyl)cyclobutyl]acetate (18.6 g, crude) was obtained as a yellow oil. Confirmed by H NMR and LCMS.
¹H NMR: (DMSO-d₆, 400 MHz)
*δ* 7.34-7.38 (m, 1H), 7.30 (t, *J* = 1.6 Hz, 1H), 7.25 (t*, J =* 7.6 Hz, 1H), 7.16 (dt, *J* = 7.6*,* 1.2 Hz, 1H), 3.86 (q, *J* = 7.2 Hz, 2H), 2.81 (s, 2H), 2.27-2.34 (m, 4H), 1.99-2.08 (m, 1H), 1.70-1.82 (m, 1H), 0.98 (t, *J =* 7.2 Hz, 3H)
LCMS: m/z = 296.9 (M+H)⁺, Rt = 1.720 min

### 2. General steps for preparation of 2-[1-(3-bromophenyl)cyclobutyl]acetohydrazide

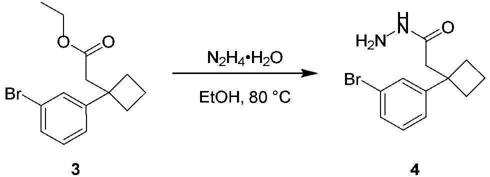

To a solution of ethyl 2-[1-(3-bromophenyl)cyclobutyl]acetate (3.00 g, 10.0 mmol) in EtOH (20.0 mL) was added dropwise N₂H₄·H₂O (5.16 g, 100 mmol, 5.00 mL, 98.0% purity) at 15 °C. After addition, the mixture was stirred at 80 °C for 12 hrs. The mixture was cooled to 25 °C, N₂H₄·H₂O (2.58 g, 50.4 mmol, 2.50 mL, 98.0% purity) was added to the mixture slowly at 15 °C, the mixture was stirred at 80 °C for 12 hrs. LCMS showed desired compound was detected. The reaction mixture was diluted with H₂O (30.0 mL), extracted with EtOAc (20.0 mL * 3), the combined organic layers were washed with H₂O (20.0 mL * 3), dried over Na₂SO₄, filtered and concentrated under reduced pressure to give a residue. Compound 2-[1-(3-bromophenyl)cyclobutyl]acetohydrazide (2.70 g, crude) was obtained as a yellow solid. Confirmed by H NMR and LCMS.
¹H NMR: (DMSO-d₆, 400 MHz)
*δ* 8.76 (br s, 1H), 7.33 (br d, *J* = 8.0 Hz, 1H), 7.27 (t, *J =* 1.6 Hz, 1H), 7.23 (t, *J*= 7.6 Hz, 1H), 7.12 (d, *J* = 7.6 Hz, 1H), 4.01 (br d, *J* = 3.6 Hz, 2H), 2.47 (s, 2H), 2.34-2.42 (m, 2H), 2.19-2.30 (m, 2H), 1.96-2.08 (m, 1H), 1.74 (s, 1H)
LCMS: m/z = 282.8 (M+H)⁺, Rt = 1.295 min

### 3. General steps for preparation of 1-[[2-[1-(3-bromophenyl)cyclobutyl]acetyl]amino]-3-methyl-thiourea

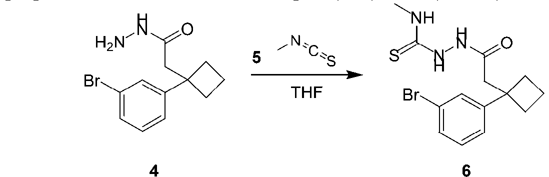

A mixture of 2-[1-(3-bromophenyl)cyclobutyl]acetohydrazide (2.70 g, 9.54 mmol), methylimino(thioxo)methane (1.39 g, 19.0 mmol, 1.30 mL) in THF (30.0 mL) was degassed and purged with N₂ for 3 times, and then the mixture was stirred at 25 °C for 4 hrs under N₂ atmosphere. LCMS showed desired compound was detected. The solvent was removed under vacuum. Compound 1-[[2-[1-(3-bromophenyl)cyclobutyl]acetyl]amino]-3-methyl-thiourea (3.50 g, crude) was obtained as a white solid.

### 4. General steps for preparation of 5-[[1-(3-bromophenyl)cyclobutyl]methyl]-4-methyl]-1,2,4-triazole-3-thiol:

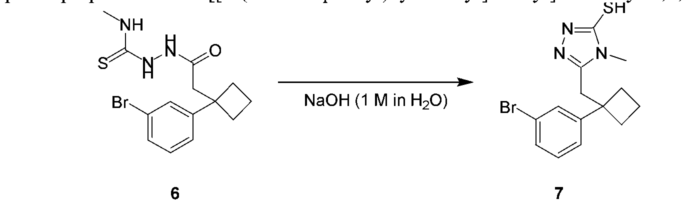

A mixture of 1-[[2-[1-(3-bromophenyl)cyclobutyl]acetyl]amino]-3-methyl-thiourea (3.40 g, 9.54 mmol), NaOH (1 M) in H₂O (30.0 mL) was degassed and purged with N₂ for 3 times, and then the mixture was stirred at 25 °C for 12 hrs under N₂ atmosphere. LCMS showed desired compound was detected. The reaction mixture was diluted with water, then the pH value of the solution was adjust to 1 with HCl (1 M, 30.0 mL) and extracted with EtOAc (40.0 mL * 3), the combined organic layers were washed with H₂O (50.0 mL * 3), dried over Na₂SO₄, filtered and concentrated under reduced pressure to give a residue. Compound 5-[[1-(3-bromophenyl)cyclobutyl]methyl]-4-methyl-1,2,4-triazole-3-thiol (3.26 g, crude) was obtained as a white solid. Confirmed by H NMR and LCMS.
¹H NMR: (DMSO-d₆, 400 MHz)
δ 13.48 (s, 1H), 7.36-7.40 (m, 1H), 7.19-7.25 (m, 2H), 6.96-7.02 (m, 1H), 3.20 (s, 2H), 2.74 (s, 3H), 2.35-2.43 (m, 2H), 2.30-2.35 (m, 2H), 2.25-2.30 (m, 2H)
LCMS: m/z = 339.8 (M+H)⁺, Rt = 1.693 min

### 5. General steps for preparation of 3-[[1-(3-bromophenyl)cyclobutyl]methyl]-4-methyl-1,2,4-triazole

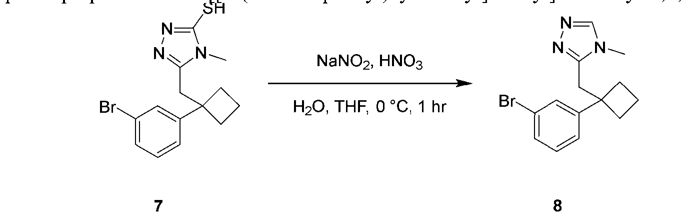

To a solution of 5-[[1-(3-bromophenyl)cyclobutyl]methyl]-4-methyl-1,2,4-triazole-3-thiol (3.26 g, 9.64 mmol) in THF (15.0 mL) and H₂O (15.0 mL) was added NaNO₂ (6.65 g, 96.3 mmol) and HNO₃ (9.81 g, 101 mmol, 7.01 mL, 65.0% purity) at 0 °C slowly. After addition, the mixture was stirred at 0 °C for 1 hr. LCMS showed desired compound was detected. The mixture was basified by saturated aqueous sodium bicarbonate solution then extracted with DCM (50.0 mL * 3). The combined organic layers were dried over Na₂SO₄, filtered and concentrated under reduced pressure to give a residue. Compound 3-[[1-(3-bromophenyl)cyclobutyl]methyl]-4-methyl-1,2,4-triazole (2.14 g, crude) was obtained as a yellow solid. Confirmed by H NMR and LCMS.
¹H NMR: (DMSO-d₆, 400 MHz)
*δ* 8.18 (s, 1H), 7.34-7.38 (m, 1H), 7.19 (t, *J =* 7.6 Hz, 1H), 7.02 (t, *J =* 1.6 Hz, 1H), 6.86 (dt, *J =* 7.6 1.2 Hz, 1H), 3.17 (s, 2H), 2.77 (s, 3H), 2.45-2.49 (m, 2H), 2.21-2.33 (m, 2H), 2.07-2.20 (m, 1H), 1.73-1.87 (m, 1H)
LCMS: m/z = 307.9 (M+H)⁺, Rt = 1.110 min

### 6 General steps for preparation of 4-cyclopropyl-2-[[(3S)-3-methyl-1-piperidyl]methyl]-6-[3-[1-[(4-methyl-1,2,4-triazol-3-yl)methyl]cyclobutyl]phen yl]-1-(p-tolylsulfonyl)pyrrolo[2,3-c]pyridin-7-one

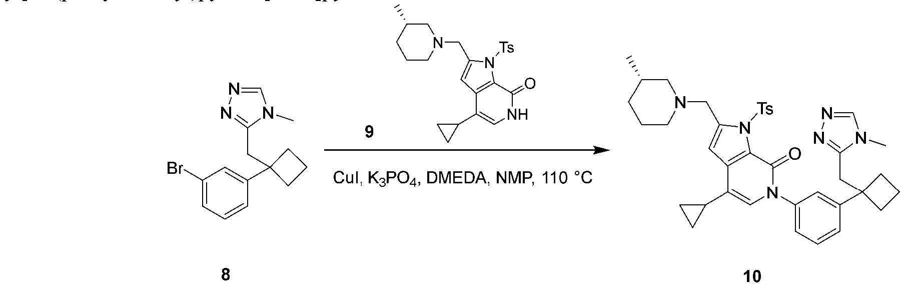

To a solution of 3-[[1-(3-bromophenyl)cyclobutyl]methyl]-4-methyl-1,2,4-triazole (121 mg, 395 µmol), 4-cyclopropyl-2-[[(3S)-3-methyl-1-piperidyl]methyl]-1-(p-tolylsulfonyl)-6H-pyrrolo[2,3-c]pyridin-7-one (116 mg, 263 µmol), CuI (50.2 mg, 263 µmol), K₃PO₄ (168 mg, 791 µmol) in NMP (1.50 mL) was added dropwise DMEDA (46.5 mg, 527 µmol, 56.8 µL). The mixture was stirred at 110 °C for 12 hrs. LCMS showed desired compound was detected. The reaction mixture was filtered and diluted with brine (30.0 mL), extracted with EtOAc (20.0 mL * 3), the combined organic layers were washed with brine (20.0 mL * 3), dried over Na₂SO₄, filtered and concentrated under reduced pressure to give a residue. The residue was purified by column chromatography (SiO₂, DCM/ MeOH = 0% to 3%), (Plate 1, DCM/ MeOH = 10/ 1, R_{f} (product) = 0.42). Compound 4-cyclopropyl-2-[[(3S)-3-methyl-1-piperidyl]methyl]-6-[3-[1-[(4-methyl-1,2,4-triazol-3-yl)methyl]cyclobutyl]phen yl]-1-(p-tolylsulfonyl)pyrrolo[2,3-c]pyridin-7-one (56.0 mg, 84.2 µmol, 31.9% yield) was obtained as a colorless oil. Confirmed by LCMS.
LCMS: m/z = 665.4 (M+H)⁺, Rt = 0.517 min

### 7. General steps for preparation of 4-cyclopropyl-7-methoxy-2-[[(3S)-3-methyl-1-piperidyl]methyl]-1-(p-tolylsulfonyl)pyrrolo[2,3-c]pyridine

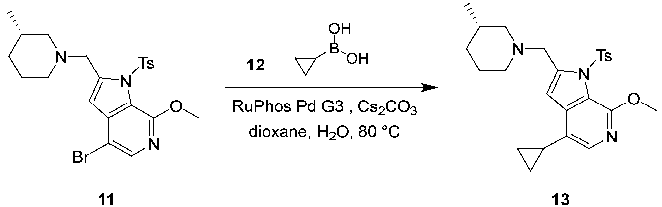

To a solution of 4-bromo-7-methoxy-2-[[(3S)-3-methyl-1-piperidyl]methyl]-1-(p-tolylsulfonyl)pyrrolo[2,3-c]pyridine (300 mg, 609 µmol) and cyclopropylboronic acid (523 mg, 6.09 mmol) in dioxane (3.00 mL) and H₂O (0.75 mL) was added RuPhos Pd G3 (101 mg, 121 µmol) and Cs₂CO₃ (595 mg, 1.83 mmol) under N₂. After addition, the mixture was stirred at 80 °C for 2 hrs under N₂. LCMS (EB6214-238-P1A1) showed desired compound was detected. The reaction mixture was diluted with brine (30.0 mL), extracted with EtOAc (20.0 mL * 3), the combined organic layers were washed with brine (20.0 mL * 3), dried over Na₂SO₄, filtered and concentrated under reduced pressure to give a residue. The residue was purified by column chromatography (SiO₂, PE/ EA = 0% to 20%), (Plate 1, PE/ EA = 10/ 1, R_{f} (product) = 0.53). Compound 4-cyclopropyl-7-methoxy-2-[[(3S)-3-methyl-1-piperidyl]methyl]-1-(p-tolylsulfonyl)pyrrolo[2,3-c]pyridine (215 mg, 464 µmol, 76.1% yield, 97.9% purity) was obtained as a yellow oil. Confirmed by LCMS.
LCMS: m/z = 453.9 (M+H)⁺, Rt = 1.350 min

### 8. General steps for preparation of 4-cyclopropyl-2-[[(3S)-3-methyl-1-piperidyl]methyl]-1-(p-tolylsulfonyl)-6H-pyrrolo[2,3-c]pyridin-7-one

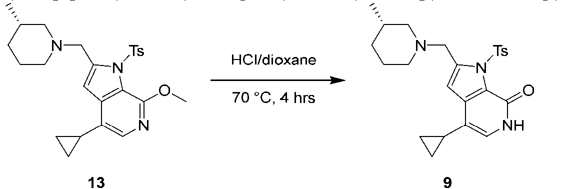

To a solution of 4-cyclopropyl-7-methoxy-2-[[(3S)-3-methyl-1-piperidyl]methyl]-1-(p-tolylsulfonyl)pyrrolo[2,3-c]pyridine (215 mg, 473 µmol) in HCl/ dioxane (4 M, 5 mL) was stirred at 70 °C for 4 hrs. LCMS showed desired compound was detected. The reaction mixture was diluted with water, then the pH value of the solution was adjust to 5 with aqueous NaHCO₃ (20 mL) and extracted with EtOAc (40 mL * 3), the combined organic layers were washed with brine (50 mL * 3), dried over Na₂SO₄, filtered and concentrated under reduced pressure to give a residue. The residue was purified by column chromatography (SiO₂, PE/ EA = 0% to 20%), (Plate 1, PE/ EA = 10/ 1, R_{f} (product) = 0.41). Compound 4-cyclopropyl-2-[[(3S)-3-methyl-1-piperidyl]methyl]-1-(p-tolylsulfonyl)-6H-pyrrolo[2,3-c]pyridin-7-one (116 mg, 198 µmol, 41.8% yield, 75.2% purity) was obtained as a white solid. Confirmed by H NMR and LCMS.
¹H NMR: (DMSO-*d*₆, 400 MHz)
*δ* 10.90 (br d, *J =* 6.0 Hz, 1H), 8.43 (d, *J =* 8.4 Hz, 2H), 7.37 (d, *J =* 8.0 Hz, 2H), 6.66-6.75 (m, 2H), 3.85 (s, 2H), 2.79 (br d, *J =* 8.4 Hz, 2H), 2.38 (s, 3H), 1.88-2.00 (m, 1H), 1.58-1.80 (m, 5H), 1.43-1.52 (m, 1H), 0.85-0.93 (m, 1H), 0.83 (d, *J =* 6.4 Hz, 3H), 0.76-0.81 (m, 2H), 0.44-0.56 (m, 2H)
LCMS: m/z = 439.8 (M+H)⁺, Rt = 1.073 min

### 9. General steps for preparation of 4-cyclopropyl-2-[[(3S)-3-methyl-1-piperidyl]methyl]-6-[3-[1-[(4-methyl-1,2,4-triazol-3-yl)methyl]cyclobutyl]phen yl]-1H-pyrrolo[2,3-c]pyridin-7-one (compound T010)

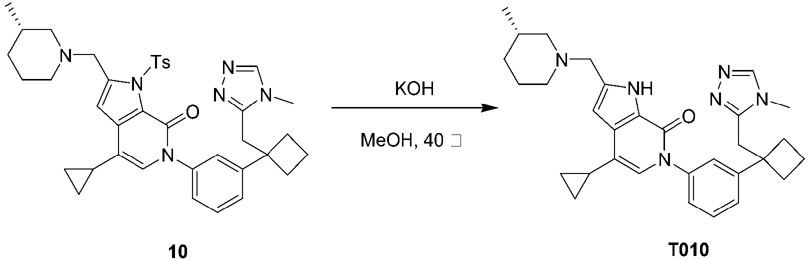

A mixture of 4-cyclopropyl-2-[[(3S)-3-methyl-1-piperidyl]methyl]-6-[3-[1-[(4-methyl-1,2,4-triazol-3-yl)methyl]cyclobutyl]phen yl]-1-(p-tolylsulfonyl)pyrrolo[2,3-c]pyridin-7-one (56.0 mg, 84.2 µmol), KOH (94.5 mg, 1.68 mmol) in MeOH (2.00 mL) was degassed and purged with N₂ for 3 times, and then the mixture was stirred at 40 °C for 4 hrs under N₂ atmosphere. LCMS showed desired compound was detected. Concentrated under reduced pressure to give a residue. The residue was purified by Prep-HPLC (column: Xtimate C 18 150 * 40 mm * 10 um; mobile phase: [water (NH₄HCO₃)-ACN]; gradient: 34.0% - 74.0% B over 36 min). Compound 4-cyclopropyl-2-[[(3S)-3-methyl-1-piperidyl]methyl]-6-[3-[1-[(4-methyl-1,2,4-triazol-3-yl)methyl]cyclobutyl]phen yl]-1H-pyrrolo[2,3-c]pyridin-7-one (10.0 mg, 18.7 µmol, 22.2% yield, 95.62% purity) was obtained as a white solid. Confirmed by H NMR and LCMS.
¹H NMR: (DMSO-d₆, 400 MHz)
*δ* 11.81-12.31 (m, 1H), 8.13 (s, 1H), 7.30-7.37 (m, 1H), 7.17-7.23 (m, 1H), 6.86 (br d, *J* = 7.6 Hz, 1H), 6.78 (s, 1H), 6.61 (s, 1H), 6.31-6.55 (m, 1H), 3.19 (s, 3H), 2.78 (s, 3H), 2.52 (br d, *J =* 1.6 Hz, 4H), 2.28-2.46 (m, 3H), 2.07-2.28 (m, 2H), 1.76-1.96 (m, 3H), 1.65 (br d, *J =* 10.4 Hz, 4H), 0.84 (br d, *J =* 6.4 Hz, 4H), 0.77-0.83 (m, 2H), 0.64-0.69 (m, 2H)
LCMS: m/z = 511.3 (M+H)⁺, Rt = 1.693 min

### Example 11: Synthesis of compound T011

### 1. General steps for preparation of 4-cyclopropyl-6-(3-((1s,3R)-3-methyl-1-(4-methyl-4H-1,2,4-triazol-3-yl)cyclobutyl)phenyl)-2-(((S)-3-methylpiperi din-1-yl)methyl)-1-tosyl-1,6-dihydro-7H-pyrrolo[2,3-c]pyridin-7-one:

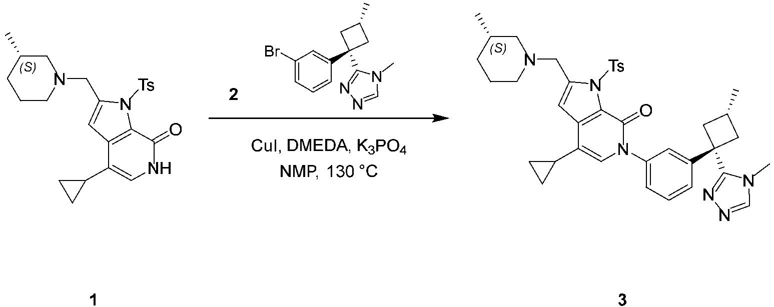

To a solution of 4-cyclopropyl-2-[[(3S)-3-methyl-1-piperidyl]methyl]-1-(p-tolylsulfonyl)-6H-pyrrolo[2,3-c]pyridin-7-one (50.0 mg, 113 µmol), 3-((1s,3s)-1-(3-bromophenyl)-3-methylcyclobutyl)-4-methyl-4H-1,2,4-triazole (31.3 mg, 102 µmol), CuI (21.6 mg, 113 µmol), K₃PO₄ (72.4 mg, 341.2 µmol) in NMP (1 mL) was added dropwise DMEDA (20.0 mg, 227 µmol, 24.4 µL). The mixture was stirred under N₂ at 130 °C for 4 hrs. LCMS showed desired compound was detected. The reaction mixture was diluted with H₂O (30 mL), extracted with EtOAc (20 mL * 3), the combined organic layers were washed with H₂O (20 mL * 3), dried over Na₂SO₄, filtered and concentrated under reduced pressure to give a residue. The residue was purified by column chromatography (SiO₂, PE/ EA = 0% to 15%), (Plate 1, PE/ EA = 5/ 1, R_{f} (product) = 0.45). Compound 4-cyclopropyl-6-(3-((1s,3R)-3-methyl-1-(4-methyl-4H-1,2,4-triazol-3-yl)cyclobutyl)phenyl)-2-(((S)-3-methylpiperi din-1-yl)methyl)-1-tosyl-1,6-dihydro-7H-pyrrolo[2,3-c]pyridin-7-one (72.0 mg, 98.5 µmol, 86.6% yield, 91.0% purity) was obtained as a white solid. Confirmed by LCMS.
LCMS: m/z = 665.3 (M+1)⁺, Rt = 1.778 min

### 2. General steps for preparation of 4-cyclopropyl-6-(3-((1s,3R)-3-methyl-1-(4-methyl-4H-1,2,4-triazol-3-yl)cyclobutyl)phenyl)-2-(((S)-3-methylpiperi din-1-yl)methyl)-1,6-dihydro-7H-pyrrolo[2,3-c]pyridin-7-one (compound T011)

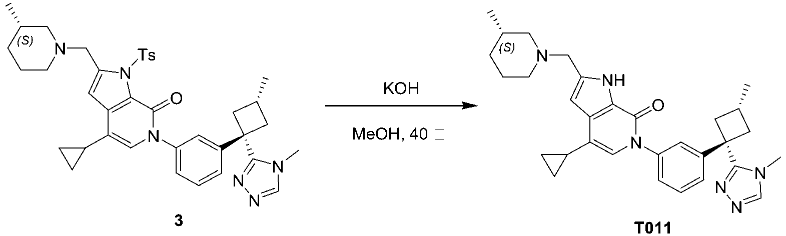

A mixture of 4-cyclopropyl-6-(3-((1s,3R)-3-methyl-1-(4-methyl-4H-1,2,4-triazol-3-yl)cyclobutyl)phenyl)-2-(((S)-3-methylpiperi din-1-yl)methyl)-1-tosyl-1,6-dihydro-7H-pyrrolo[2,3-c]pyridin-7-one (72.0 mg, 108 µmol), KOH (121 mg, 2.17 mmol) in MeOH (4 mL) was degassed and purged with N₂ for 3 times, and then the mixture was stirred at 40 °C for 4 hrs under N₂ atmosphere. LCMS showed desired compound was detected. Filtered and concentrated under reduced pressure to give a residue. The residue was purified by Prep-HPLC (column: Xtimate C18 150 * 40 mm * 10 um; mobile phase: [water (NH₄HCO₃)-ACN]; gradient: 32%-72% B over 36 min). Compound 4-cyclopropyl-6-(3-((1s,3R)-3-methyl-1-(4-methyl-4H-1,2,4-triazol-3-yl)cyclobutyl)phenyl)-2-(((S)-3-methylpiperi din-1-yl)methyl)-1,6-dihydro-7H-pyrrolo[2,3-c]pyridin-7-one (17.4 mg, 32.9 µmol, 30.4% yield, 96.62% purity) was obtained as a white solid. Confirmed by H NMR and LCMS.
¹H NMR: (DMSO-d₆, 400 MHz)
*δ* 11.88-12.18 (m, 1H), 8.29 (s, 1H), 7.45-7.51 (m, 1H), 7.32-7.36 (m, 2H), 7.27 (br d, *J* = 7.2 Hz, 1H), 6.81 (s, 1H), 6.43 (br s, 1H), 3.77 (br s, 2H), 3.25 (s, 3H), 2.86 (br d, *J =* 3.2 Hz, 5H), 2.53 (br s, 2H), 2.09 (br d, *J =* 16.4 Hz, 1H), 1.79-1.95 (m, 2H), 1.42-1.75 (m, 5H), 1.06 (br d, *J =* 5.2 Hz, 3H), 0.82 (br d, *J =* 6.4 Hz, 3H), 0.77-0.81 (m, 2H), 0.65 (br d, *J =* 3.6 Hz, 2H)
LCMS: m/z = 511.3 (M+H)⁺, Rt = 1.640 min

### Examples 12 and 13: Synthesis of compounds T012 & T013

### 1. General steps for preparation of methyl 2-(3-bromophenyl)-2-cyclobutyl-acetate

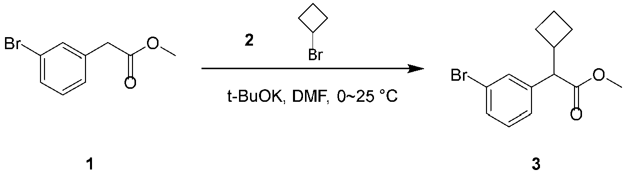

To a solution of methyl 2-(3-bromophenyl) acetate (5.00 g, 21.8 mmol) in DMF (50.0 mL) was added t-BuOK (3.18 g, 28.3 mmol) in portions at 0 °C and stirred at 0 °C for 30 min, bromocyclobutane (3.54 g, 26.1 mmol, 2.47 mL) was added to the mixture slowly at 0 °C. The mixture was stirred at 25 °C for 16 hrs under N₂. LCMS showed desired compound was detected. The reaction mixture was quenched by aqueous NH₄Cl (15.0 mL), stirred for 15 min. Filtered and the reaction mixture was diluted with brine (30.0 mL), extracted with EtOAc (20.0 mL * 3), the combined organic layers were washed with H₂O (20.0 mL * 3), dried over Na₂SO₄, filtered and concentrated under reduced pressure to give a residue. The residue was purified by column chromatography (SiO₂, PE/ EA = 0% to 4%), (Plate 1, PE/ EA = 10/ 1, R_{f} (product) = 0.61). Compound methyl 2-(3-bromophenyl)-2-cyclobutyl-acetate (4.70 g, 15.9 mmol, 73.0% yield, 96.0% purity) was obtained as a colorless oil. Confirmed by H NMR and LCMS.
¹H NMR: (DMSO-d₆, 400 MHz)
*δ* 7.43-7.50 (m, 2H), 7.29 (d, *J =* 4.8 Hz, 2H), 3.72 (d, *J =* 12.0 Hz, 1H), 3.58 (s, 3H), 2.76-2.95 (m, 1H), 2.00-2.12 (m, 1H), 1.69-1.83 (m, 4H), 1.52-1.62 (m, 1H)
LCMS: m/z = 284.6 (M+H)⁺, Rt = 1.967 min

### 2. General steps for preparation of 2-(3-bromophenyl)-2-cyclobutyl-acetohydrazide

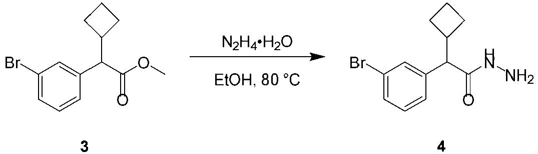

To a solution of methyl 2-(3-bromophenyl)-2-cyclobutyl-acetate (4.70 g, 16.6 mmol) in EtOH (40.0 mL) was added dropwise N₂H₄.H₂O (12.7 g, 248 mmol, 12.3 mL, 98.0% purity). After addition, the mixture was stirred at 80 °C for 12 hrs. LCMS showed desired compound was detected. The reaction mixture was quenched by aqueous Na₂SO₃ (20.0 mL), stirred for 15 min. Diluted with H₂O (30.0 mL), extracted with EtOAc (20.0 mL * 3), the combined organic layers were washed with H₂O (20.0 mL * 3), dried over Na₂SO₄, filtered and concentrated under reduced pressure to give a residue. Compound 2-(3-bromophenyl)-2-cyclobutyl-acetohydrazide (5.00 g, crude) was obtained as a colorless oil. Confirmed by H NMR.
¹H NMR: (DMSO-d₆, 400 MHz)
*δ* 9.21 (s, 1H), 7.49 (s, 1H), 7.40 (d, *J =* 7.2 Hz, 1H), 7.22-7.29 (m, 2H), 4.21 (s, 2H), 3.33-3.36 (m, 1H), 2.83-2.98 (m, 1H), 1.60-1.88 (m, 5H), 1.43-1.53 (m, 1H)

### 3. General steps for preparation of 1-[[2-(3-bromophenyl)-2-cyclobutyl-acetyl]amino]-3-methyl-thiourea

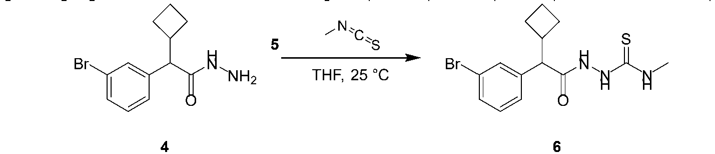

A mixture of 2-(3-bromophenyl)-2-cyclobutyl-acetohydrazide (5.00 g, 17.6 mmol), methylimino(thioxo)methane (2.58 g, 35.3 mmol, 2.42 mL) in THF (50.0 mL) was degassed and purged with N₂ for 3 times, and then the mixture was stirred at 25 °C for 4 hrs under N₂ atmosphere. LCMS showed desired compound was detected. The reaction mixture was diluted with H₂O (30.0 mL), extracted with EtOAc (20.0 mL * 3), the combined organic layers were washed with H₂O (20.0 mL * 3), dried over Na₂SO₄, filtered and concentrated under reduced pressure to give a residue. Compound 1-[[2-(3-bromophenyl)-2-cyclobutyl-acetyl]amino]-3-methyl-thiourea (6.20 g, 17.4 mmol, 98.5% yield) was obtained as a white solid.

### 4. General steps for preparation of 5-[(3-bromophenyl)-cyclobutyl-methyl]-4-methyl-1,2,4-triazole-3-thiol

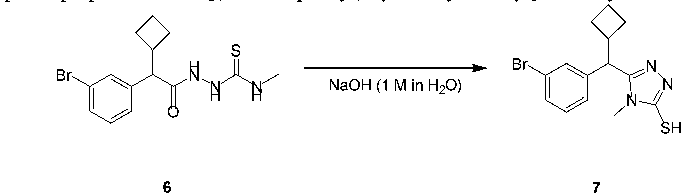

A mixture of 1-[[2-(3-bromophenyl)-2-cyclobutyl-acetyl]amino]-3-methyl-thiourea (6.20 g, 17.4 mmol), NaOH (1 M, 60.0 mL) in H₂O (60.0 mL) was degassed and purged with N₂ for 3 times, and then the mixture was stirred at 25 °C for 4 hrs under N₂ atmosphere. LCMS showed desired compound was detected. The reaction mixture was diluted with aqueous NaHCO₃ (40.0 mL), stirred for 15 min. The reaction mixture was extracted with EtOAc (20.0 mL * 3), the combined organic layers were washed with H₂O (20.0 mL * 3), dried over Na₂SO₄, filtered and concentrated under reduced pressure to give a residue. Compound 5-[(3-bromophenyl)-cyclobutyl-methyl]-4-methyl-1,2,4-triazole-3-thiol (6.00 g, crude) was obtained as a white solid.

### 5. General steps for preparation of 3-[(3-bromophenyl)-cyclobutyl-methyl]-4-methyl-1,2,4-triazole

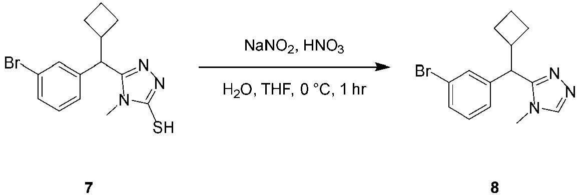

To a solution of 5-[(3-bromophenyl)-cyclobutyl-methyl]-4-methyl-1,2,4-triazole-3-thiol (6.00 g, 17.7 mmol) in THF (30.0 mL) and H₂O (30.0 mL) was added NaNO₂ (12.2 g, 177 mmol), HNO₃ (18.0 g, 186 mmol, 12.9 mL, 65.0% purity) was added to the mixture slowly. After addition, the mixture was stirred at 0 °C for 1 hr. LCMS showed desired compound was detected. The mixture was basified by saturated aqueous sodium bicarbonate solution then extracted with EtOAc (60.0 mL * 3). The combined organic layers were dried over Na₂SO₄, filtered and concentrated under reduced pressure to give a residue. The residue was purified by column chromatography (SiO₂, DCM/ MeOH = 0% to 4%), (Plate 1, DCM/ MeOH = 20/ 1, R_{f} (product) = 0.40). Compound 3-[(3-bromophenyl)-cyclobutyl-methyl]-4-methyl-1,2,4-triazole (4.40 g, 14.2 mmol, 80.1% yield, 98.9% purity) was obtained as a white solid. Confirmed by LCMS.
LCMS: m/z = 305.9 (M+H)⁺, Rt = 1.403 min

### 6 General steps for preparation of 6-(3-(cyclobutyl(4-methyl-4H-1,2,4-triazol-3-yl)methyl)phenyl)-4-cyclopropyl-2-(((S)-3-methylpiperidin-1-yl)met hyl)-1-tosyl-1,6-dihydro-7H-pyrrolo[2,3-c]pyridin-7-one

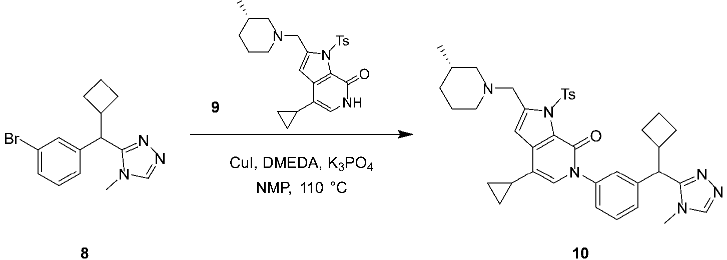

To a solution of 3-[(3-bromophenyl)-cyclobutyl-methyl]-4-methyl-1,2,4-triazole (200 mg, 653 µmol) and (S)-4-cyclopropyl-2-((3-methylpiperidin-1-yl)methyl)-1-tosyl-1,6-dihydro-7H-pyrrolo[2,3-c]pyridin-7-one (287 mg, 653 µmol) in NMP (2.00 mL) was added CuI (124 mg, 653 µmol) and K₃PO₄ (415 mg, 1.96 mmol). DMEDA (115 mg, 1.31 mmol, 140 µL) was added to the mixture. After addition, the mixture was stirred at 110 °C for 4 hrs under N₂. LCMS showed desired compound was detected. The reaction mixture was filtered and diluted with H₂O (30.0 mL), extracted with EtOAc (20.0 mL * 3), the combined organic layers were washed with H₂O (20.0 mL * 3), dried over Na₂SO₄, filtered and concentrated under reduced pressure to give a residue. The residue was purified by column chromatography (SiO₂, DCM/ MeOH = 0% to 4%), (Plate 1, DCM/ MeOH = 20/ 1, R_{f} (product) = 0.34). Compound 6-(3-(cyclobutyl(4-methyl-4H-1,2,4-triazol-3-yl)methyl)phenyl)-4-cyclopropyl-2-(((S)-3-methylpiperidin-1-yl)met hyl)-1-tosyl-1,6-dihydro-7H-pyrrolo[2,3-c]pyridin-7-one (404 mg, 555 µmol, 85.0% yield, 91.4% purity) was obtained as a white solid. Confirmed by LCMS.
LCMS: m/z = 665.2 (M+H)⁺, Rt = 1.225 min

### 7. General steps for preparation of 6-(3-(cyclobutyl(4-methyl-4H-1,2,4-triazol-3-yl)methyl)phenyl)-4-cyclopropyl-2-(((S)-3-methylpiperidin-1-yl)met hyl)-1,6-dihydro-7H-pyrrolo[2,3-c]pyridin-7-one

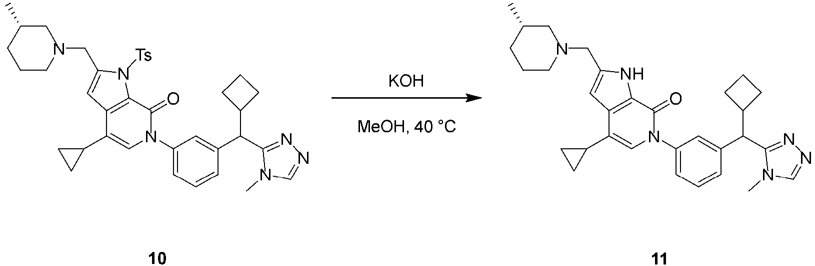

To a mixture of 6-(3-(cyclobutyl(4-methyl-4H-1,2,4-triazol-3-yl)methyl)phenyl)-4-cyclopropyl-2-(((S)-3-methylpiperidin-1-yl)met hyl)-1-tosyl-1,6-dihydro-7H-pyrrolo[2,3-c]pyridin-7-one (260 mg, 391 µmol) in MeOH (5.00 mL) was added KOH (439 mg, 7.83 mmol). The mixture was stirred at 40 °C for 2 hrs. LCMS showed the reaction was completed. The aqueous phase was extracted with ethyl acetate (20.0 mL*2). The combined organic phase was washed with brine (20.0 mL*2), dried with anhydrous Na₂SO₄, filtered and concentrated in vacuum. The residue was purified by Prep-HPLC (column: Xtimate C18 150 * 40 mm * 10 um; mobile phase: [water (HCl)-ACN]; gradient: 6%-46% B over 30 min). Compound 6-(3-(cyclobutyl(4-methyl-4H-1,2,4-triazol-3-yl)methyl)phenyl)-4-cyclopropyl-2-(((S)-3-methylpiperidin-1-yl)met hyl)-1,6-dihydro-7H-pyrrolo[2,3-c]pyridin-7-one (200 mg, 382 µmol, 97.7% yield, 97.5% purity) was obtained as a yellow solid. It was confirmed by LCMS.
LCMS: m/z = 511.3 (M+H)⁺, Rt = 1.525 min

### 8. General steps for preparation of 6-(3-((R)-cyclobutyl(4-methyl-4H-1,2,4-triazol-3-yl)methyl)phenyl)-4-cyclopropyl-2-(((S)-3-methylpiperidin-1-yl) methyl)-1,6-dihydro-7H-pyrrolo[2,3-c]pyridin-7-one (compound T012) and 6-(3-((S)-cyclobutyl(4-methyl-4H-1,2,4-triazol-3-yl)methyl)phenyl)-4-cyclopropyl-2-(((S)-3-methylpiperidin-1-yl) methyl)-1,6-dihydro-7H-pyrrolo[2,3-c]pyridin-7-one (compound T013)

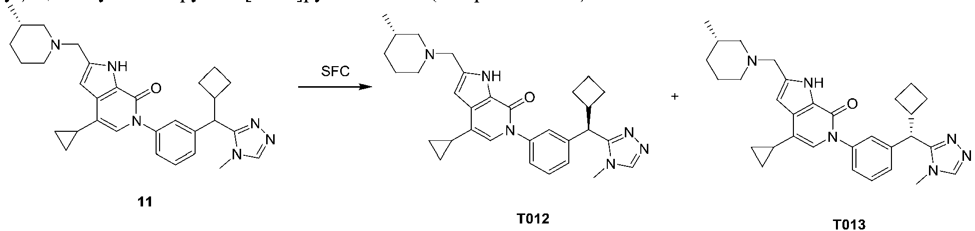

The residue was purified by SFC (column: Daicel Chiralpak IBN 250 mm * 30 mm * 10 um; mobile phase: [CO₂-EtOH (0.1% NH₃H₂O)]; B%:35%, isocratic elution mode). Compound 6-(3-((R)-cyclobutyl(4-methyl-4H-1,2,4-triazol-3-yl)methyl)phenyl)-4-cyclopropyl-2-(((S)-3-methylpiperidin-1-yl) methyl)-1,6-dihydro-7H-pyrrolo[2,3-c]pyridin-7-one (29.2 mg, 56.8 µmol, 14.5% yield, 99.43% purity) was obtained as a white solid. Confirmed by H NMR and LCMS.
¹H NMR: (CD₃CN, 400 MHz)
*δ* 10.73-11.06 (m, 1H), 8.05 (s, 1H), 7.37-7.45 (m, 1H), 7.32 (s, 1H), 7.24-7.31 (m, 2H), 6.73 (d, *J =* 0.4 Hz, 1H), 6.40 (s, 1H), 4.13 (d, *J* = 10.4 Hz, 1H), 3.62 (s, 2H), 3.42 (s, 3H), 3.19-3.31 (m, 1H), 2.71-2.81 (m, 2H), 2.09-2.18 (m, 3H), 1.82-1.91 (m, 5H), 1.70-1.77 (m, 1H), 1.54-1.68 (m, 4H), 1.48 (d, *J =* 12.0 Hz, 1H), 0.82-0.86 (m, 2H), 0.78 (d, *J =* 6.4 Hz, 3H), 0.59-0.64 (m, 2H)
LCMS: m/z = 511.5 (M+H)⁺, Rt = 1.930 min

Compound 6-(3-((S)-cyclobutyl(4-methyl-4H-1,2,4-triazol-3-yl)methyl)phenyl)-4-cyclopropyl-2-(((S)-3-methylpiperidin-1-yl) methyl)-1,6-dihydro-7H-pyrrolo[2,3-c]pyridin-7-one (41.0 mg, 80.0 µmol, 20.4% yield, 99.77% purity) was obtained as a white solid. Confirmed by H NMR and LCMS.
¹H NMR: (CD₃CN, 400 MHz)
*δ* 12.06 (s, 1H), 8.08 (s, 1H), 7.38-7.45 (m, 1H), 7.32 (s, 1H), 7.23-7.30 (m, 2H), 6.75 (s, 1H), 6.65 (s, 1H), 4.25-4.40 (m, 2H), 4.15 (d, *J =* 10.4 Hz, 1H), 3.43 (s, 3H), 3.21-3.38 (m, 3H), 2.72 (t, *J =* 12.0 Hz, 1H), 2.43 (t, *J =* 12.0 Hz, 1H), 2.02-2.18 (m, 3H), 1.80-1.90 (m, 7H), 1.73 (d, *J =* 8.4 Hz, 1H), 1.05-1.16 (m, 1H), 0.89 (d, *J =* 6.4 Hz, 3H), 0.85 (dd, *J =* 8.4, 1.6 Hz, 2H), 0.59-0.66 (m, 2H)
LCMS: m/z = 511.3 (M+H)⁺, Rt = 1.940 min

### Example 14: Synthesis of compound T014

### 1. General steps for preparation of methyl 1-(3-bromophenyl)-3-methyl-cyclobutanecarboxylate

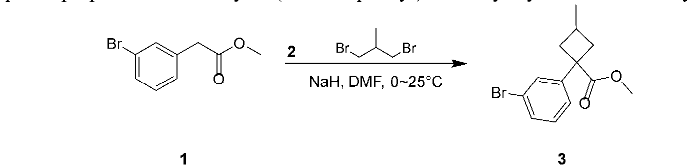

To a solution of methyl 2-(3-bromophenyl)acetate (10.0 g, 43.6 mmol) and 1,3-dibromo-2-methyl-propane (9.43 g, 43.6 mmol) in DMF (100 mL) was added NaH (3.49 g, 87.3 mmol, 60.0% purity) under N₂ at 0 °C. The mixture was stirred under N₂ at 25 °C for 1 hrs. LCMS showed the reaction was completed. TLC (PE/ EtOAc = 10/ 1, product 1 R_{f} = 0.60) indicated new spot formed. The reaction mixture was poured into saturated aqueous NH₄Cl (200 mL) slowly. The reaction mixture was diluted with EtOAc (200 mL), washed with brine (100 mL * 3), dried over Na₂SO₄. The organic phase was concentrated in vacuum. The crude product was purified by column chromatography (SiO₂, PE/ EtOAc = 1/ 0 to 10/ 1). Compound methyl 1-(3-bromophenyl)-3-methyl-cyclobutanecarboxylate (7.60 g, 26.8 mmol, 61.4% yield) was obtained as a colorless oil.

### 2. General steps for preparation of 1-(3-bromophenyl)-3-methyl-cyclobutanecarbohydrazide

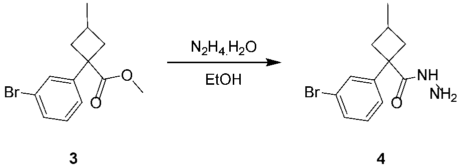

To a solution of methyl 1-(3-bromophenyl)-3-methyl-cyclobutanecarboxylate (7.60 g, 26.8 mmol) in EtOH (80.0 mL) was added N₂H₄·H₂O (9.37 g, 183 mmol, 9.08 mL, 98.0% purity). The mixture was stirred under N₂ at 80°C for 3 hrs. LCMS showed the reaction was completed. The reaction mixture was concentrated in vacuum. The mixture was diluted water (100 mL), extracted with EtOAc (100 mL * 2). The organic phase was dried over Na₂SO₄ and concentrated in vacuum. Compound 1-(3-bromophenyl)-3-methyl-cyclobutanecarbohydrazide (7.60 g, 26.8 mmol, 100% yield) was obtained as a yellow solid.

### 3. General steps for preparation of 1-[[1-(3-bromophenyl)-3-methyl-cyclobutanecarbonyl]amino]-3-methyl-thiourea

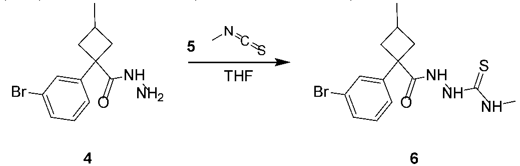

To a solution of 1-(3-bromophenyl)-3-methyl-cyclobutanecarbohydrazide (7.60 g, 26.8 mmol) in THF (20 mL) was added methylimino(thioxo)methane (2.94 g, 40.2 mmol, 2.75 mL). The mixture was stirred under N₂ at 80°C for 3 hrs. LCMS showed the reaction was completed. The reaction mixture was concentrated in vacuum. Compound 1-[[1-(3-bromophenyl)-3-methyl-cyclobutanecarbonyl]amino]-3-methyl-thiourea (8.0 g, 22.4 mmol, 83.6% yield) was obtained as a white solid.

### 4. General steps for preparation of 5-[1-(3-bromophenyl)-3-methyl-cyclobutyl]-4-methyl-1,2,4-triazole-3-thiol

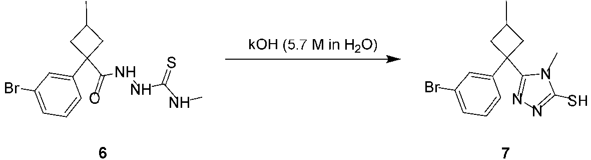

To a solution of 1-[[1-(3-bromophenyl)-3-methyl-cyclobutanecarbonyl]amino]-3-methyl-thiourea (8.0 g, 22.4 mmol) in THF (50 mL) was added KOH (5.7 M, 40 mL). The mixture was stirred under N₂ at 60 °C for 12 hrs. LCMS showed the reaction was completed. The reaction mixture was diluted with brine (200 mL), the water phase was adjusted pH = 5 (HCl, 1 M) and extracted with EtOAc (200 mL * 2), dried over Na₂SO₄. The organic phase was filtered and concentrated in vacuum. The crude product was triturated with MTBE at 25 °C for 12 hrs. Compound 5-[1-(3-bromophenyl)-3-methyl-cyclobutyl]-4-methyl-1,2,4-triazole-3-thiol (7.60 g, crude) was obtained as a yellow solid. It was confirmed by H NMR.
¹H NMR: (400 MHz, DMSO-d₆)
δ 13.71 (s, 1H), 7.52 (s, 1H), 7.45-7.50 (m, 1H), 7.37 (br d, J = 8.0 Hz, 1H), 7.34 (br d, J = 1.6 Hz, 1H), 3.00 (s, 1H), 2.97 (s, 3H), 2.72-2.79 (m, 2H), 2.40-2.46 (m, 2H), 1.06 (br d, J = 5.6 Hz, 3H)

### 5. General steps for preparation of 3-[1-(3-bromophenyl)-3-methyl-cyclobutyl]-4-methyl-1,2,4-triazole

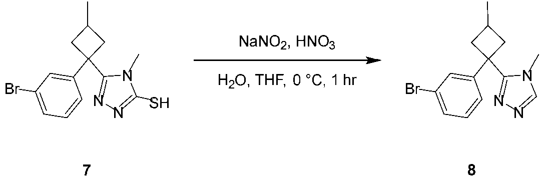

To a solution of 5-[1-(3-bromophenyl)-3-methyl-cyclobutyl]-4-methyl-1,2,4-triazole-3-thiol (7.60 g, 22.4 mmol) in THF (40 mL) and H₂O (40 mL) was added NaNO₂ (15.5 g, 224 mmol) and HNO₃ (20.2 g, 208 mmol, 14.4 mL, 65.0% purity) at 0 °C slowly. After addition, the mixture was stirred at 0 °C for 2 hrs. LCMS showed the reaction was completed. The mixture was basified by saturated aqueous sodium bicarbonate solution then extracted with EtOAc (200 mL * 3). The combined organic layers were dried over Na₂SO₄, filtered and concentrated under reduced pressure to give a residue. The residue was purified by column chromatography (SiO₂, EtOAc/ MeOH = 1/ 0 to 90/ 10). TLC (Plate 1, PE/ EtOAc = 1/ 1, UV 254 nm, R_{f} (product) = 0.3). Compound 3-[1-(3-bromophenyl)-3-methyl-cyclobutyl]-4-methyl-1,2,4-triazole (5.0 g, 16.3 mmol, 72.6% yield) was obtained as a yellow oil.

### 6. General steps for preparation of 3-((1s,3s)-1-(3-bromophenyl)-3-methylcyclobutyl)-4-methyl-4H-1,2,4-triazole&3-((1r,3r)-1-(3-bromophenyl)-3-me thylcyclobutyl)-4-methyl-4H-1,2,4-triazole

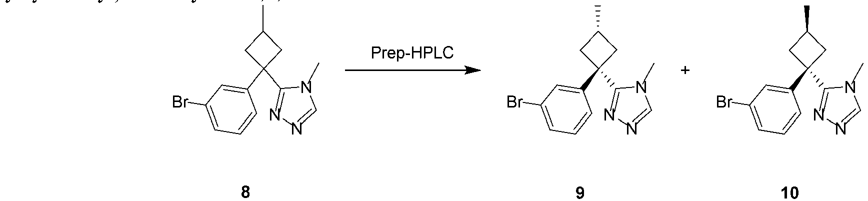

The product was purified by Prep-HPLC (column: Xtimate C18 150 * 40 mm * 10 um; mobile phase: [water (FA)-ACN]; gradient: 16%-56% B over 36 min). Compound 3-((1r,3r)-1-(3-bromophenyl)-3-methylcyclobutyl)-4-methyl-4H-1,2,4-triazole (1.90 g, 6.21 mmol, 38.0% yield, 99.4% purity) was obtained as a yellow solid. It was confirmed by LCMS and H NMR.
LCMS: m/z = 307.5 (M+H)⁺, Rt = 1.212 min
¹H NMR: (400 MHz, DMSO-d₆)
*δ* 8.30 (s, 1H), 7.39-7.51 (m, 2H), 7.28-7.39 (m, 2H), 3.33 (s, 2H), 3.17 (s, 3H), 2.81 (br d, *J =* 3.6 Hz, 2H), 2.43-2.49 (m, 1H), 1.05 (br d, *J* = 5.2 Hz, 3H)

Compound 3-((1s,3s)-1-(3-bromophenyl)-3-methylcyclobutyl)-4-methyl-4H-1,2,4-triazole (4.58 g, 14.9 mmol, 91.6% yield, 89.8% purity) was obtained as a yellow oil. It was confirmed by LCMS.
LCMS: m/z = 307.6 (M+H)⁺, Rt = 1.215 min

### 7. General steps for preparation of 4-cyclopropyl-6-(3-((1r,3S)-3-methyl-1-(4-methyl-4H-1,2,4-triazol-3-yl)cyclobutyl)phenyl)-2-(((S)-3-methylpiperi din-1-yl)methyl)-1-tosyl-1,6-dihydro-7H-pyrrolo[2,3-c]pyridin-7-one

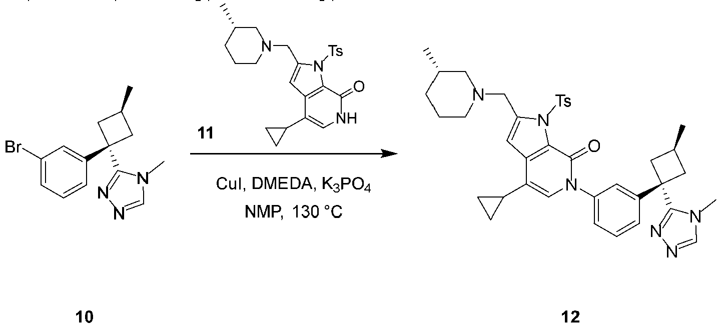

To a mixture of 3-((1r,3r)-1-(3-bromophenyl)-3-methylcyclobutyl)-4-methyl-4H-1,2,4-triazole (50.0 mg, 163 µmol), 4-cyclopropyl-2-[[(3S)-3-methyl-1-piperidyl]methyl]-1-(p-tolylsulfonyl)-6H-pyrrolo[2,3-c]pyridin-7-one (78.9 mg, 179 µmol), CuI (31.1 mg, 163 µmol) and K₃PO₄ (103 mg, 489 µmol) in NMP (1.0 mL) was added DMEDA (28.7 mg, 326 µmol, 35.1 µL). The suspension was degassed under vacuum and purged with N₂ several times. The mixture was stirred under N₂ at 130 °C for 1 hr. LCMS showed the reaction was completed. The mixture was basified by saturated aqueous sodium bicarbonate solution then extracted with EtOAc (10 mL * 3). The combined organic layers were dried over Na₂SO₄, filtered and concentrated under reduced pressure to give a residue. The residue was purified by column chromatography (SiO₂, DCM/ MeOH = 1/ 0 to 96/ 4). TLC (Plate 1, DCM/ MeOH = 10/ 1, UV 254 nm, R_{f} (product) = 0.3). Compound 4-cyclopropyl-6-(3-((1r,3S)-3-methyl-1-(4-methyl-4H-1,2,4-triazol-3-yl)cyclobutyl)phenyl)-2-(((S)-3-methylpiperi din-1-yl)methyl)-1-tosyl-1,6-dihydro-7H-pyrrolo[2,3-c]pyridin-7-one (170 mg, crude) was obtained as a green oil. It was confirmed by LCMS.
LCMS: m/z = 665.3 (M+H)⁺, Rt = 0.800 min

### 8. General steps for preparation of 4-cyclopropyl-6-(3-((1r,3S)-3-methyl-1-(4-methyl-4H-1,2,4-triazol-3-yl)cyclobutyl)phenyl)-2-(((S)-3-methylpiperi din-1-yl)methyl)-1,6-dihydro-7H-pyrrolo[2,3-c]pyridin-7-one (compound T014)

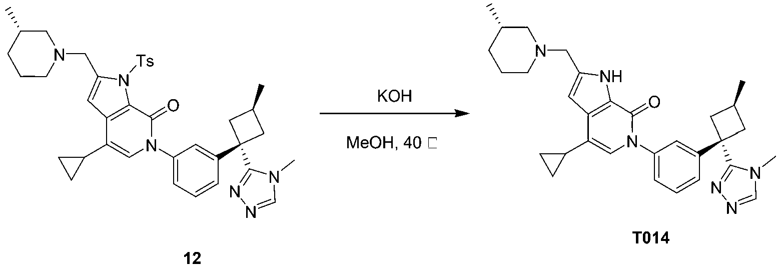

To a mixture of 4-cyclopropyl-6-(3-((1r,3S)-3-methyl-1-(4-methyl-4H-1,2,4-triazol-3-yl)cyclobutyl)phenyl)-2-(((S)-3-methylpiperi din-1-yl)methyl)-1-tosyl-1,6-dihydro-7H-pyrrolo[2,3-c]pyridin-7-one (170 mg, 255 µmol) in MeOH (1.50 mL) was added KOH (286 mg, 5.11 mmol). The mixture was stirred at 25 °C for 2 hrs. LCMS showed the reaction was completed. The reaction mixture was diluted with EtOAc (10 mL), washed with brine (10 mL * 3), dried over Na₂SO₄. The organic phase was concentrated in vacuum. The product was purified by Prep-HPLC (column: Welch Xtimate C18 150 * 30 mm * 5 um; mobile phase: [water (HCl)-ACN]; gradient: 6%-46% B over 25 min). Compound 4-cyclopropyl-6-(3-((1r,3S)-3-methyl-1-(4-methyl-4H-1,2,4-triazol-3-yl)cyclobutyl)phenyl)-2-(((S)-3-methylpiperi din-1-yl)methyl)-1,6-dihydro-7H-pyrrolo[2,3-c]pyridin-7-one (11.6 mg, 22.6 µmol, 8.85% yield, 99.65% purity) was obtained as a white solid. It was confirmed by LCMS and H NMR.
¹H NMR: H NMR, (400 MHz, DMSO-d6)
*δ* 12.35 (s, 1H), 9.41 (br s, 1H), 7.49-7.58 (m, 1H), 7.29-7.39 (m, 2H), 7.27 (br d, *J =* 8.0 Hz, 1H), 6.89 (s, 1H), 6.76 (d, *J =* 1.6 Hz, 1H), 4.36 (br d, *J =* 3.6 Hz, 2H), 3.47 (s, 3H), 3.35 (br d, *J =* 8.8 Hz, 1H), 3.27 (br d, *J =* 11.6 Hz, 1H), 3.15 (br d, *J =* 4.0 Hz, 2H), 2.67-2.77 (m, 1H), 2.46 (br s, 1H), 2.34-2.38 (m, 2H), 1.95-2.04 (m, 1H), 1.87-1.93 (m, 1H), 1.81 (br s, 2H), 1.71 (br d, *J =* 13.6 Hz, 1H), 1.09 (br d, *J =* 5.2 Hz, 3H), 0.96-1.06 (m, 1H), 0.88 (d, *J* = 6.4 Hz, 3H), 0.76-0.85 (m, 2H), 0.70 (q, *J =* 5.2 Hz, 2H)
LCMS: m/z = 511.1 (M+H)⁺, Rt = 1.103 min

### Example 15: Synthesis of compound T015

### 1. General steps for preparation of 2-[[(3R)-3-hydroxypyrrolidin-1-yl]methyl]-6-[3-[1-(4-methyl-1,2,4-triazol-3-yl)cyclobutyl]phenyl]-4-(trifluoromet hyl)-1H-pyrrolo[2,3-c]pyridin-7-one (compound T015)

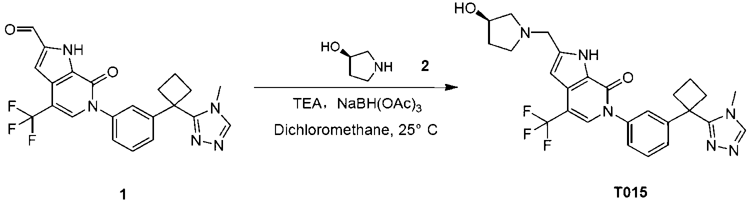

To a mixture of 6-[3-[1-(4-methyl-1,2,4-triazol-3-yl)cyclobutyl]phenyl]-7-oxo-4-(trifluoromethyl)-1H-pyrrolo[2,3-c]pyridine-2-car baldehyde (50.0 mg, 113 µmol) in Dichloromethane (3.00 mL) was added TEA (11.4 mg, 113 µmol) adjust pH = 8. Then (3R)-pyrrolidin-3-ol (19.7 mg, 226 µmol) was added to the mixture. The mixture was stirred under N₂ at 25 °C for 0.5 hr. NaBH(OAc)₃ (60.0 mg, 283 µmol) was added to the mixture. The mixture was stirred under N₂ at 25 °C for 0.5 hr. LCMS showed desired mass was detected. The reaction mixture was diluted with NaHCO₃ (3.00 mL), extracted with Dichloromethane (3.00 mL*2). The organic phase dried over Na₂SO₄ and concentrated in vacuum. The crude product was purified by Prep-HPLC (column: Welch Xtimate C18 40*200 mm 7 um; mobile phase: [water (HCl)-ACN]; gradient: 0%-36.0% B over 30 min). To afford 2-[[(3R)-3-hydroxypyrrolidin-1-yl]methyl]-6-[3-[1-(4-methyl-1,2,4-triazol-3-yl)cyclobutyl]phenyl]-4-(trifluoromet hyl)-1H-pyrrolo[2,3-c]pyridin-7-one (14.1 mg, 24.0 µmol, 21.2% yield, 100% purity, 2HCl) as a white solid which was confirmed by H NMR and LCMS.
¹H NMR: (400 MHz, DMSO-d₆)
*δ* 12.82 (s, 1H), 9.52 (s, 1H), 7.82 (s, 1H), 7.52-7.61 (m, 2H), 7.43 (dd, *J =* 12.8, 8.0 Hz, 2H), 6.70-6.86 (m, 1H), 4.51-4.61 (m, 2H), 4.40 (d, *J =* 2.0 Hz, 1H), 3.48-3.64 (m, 2H), 3.44 (s, 3H), 3.21-3.34 (m, 2H), 3.02-3.18 (m, 1H), 2.93-3.02 (m, 2H), 2.74-2.84 (m, 2H), 2.02-2.28 (m, 2H), 1.84-2.02 (m, 2H)
LCMS: EB6211-700-P1A1, m/z = 513.2 (M+H)⁺, Rt = 0.898 min

### Example 16: Synthesis of compound T016

### 1. General steps for preparation of 2-(1,3-dioxolan-2-yl)-6-[3-[1-(4-methyl-1,2,4-triazol-3-yl)cyclobutyl]phenyl]-1-(p-tolylsulfonyl)-4-(trifluoromethy l)pyrrolo[2,3-c]pyridin-7-one

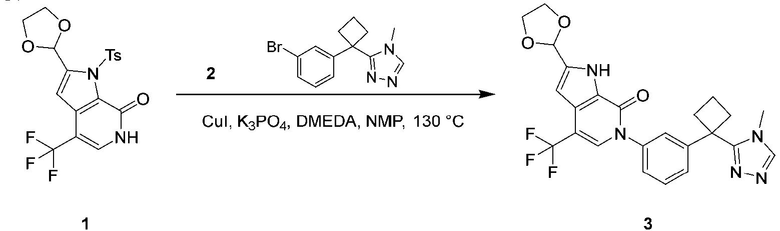

A mixture of 2-(1,3-dioxolan-2-yl)-1-(p-tolylsulfonyl)-4-(trifluoromethyl)-6H-pyrrolo[2,3-c]pyridin-7-one (200 mg, 466 µmol), 3-[1-(3-bromophenyl)cyclobutyl]-4-methyl-1,2,4-triazole (136 mg, 466 µmol), CuI (177 mg, 933 µmol), K₃PO₄ (297 mg, 1.40 mmol) and DMEDA (41.1 mg, 466 µmol, 50.2 µL) in NMP (2.00 mL) was degassed and purged with N₂ for 3 times, and then the mixture was stirred at 130 °C for 1 hr under N₂ atmosphere. LCMS showed desired compound was detected. The reaction mixture was diluted with H₂O (30.0 mL), extracted with ethyl acetate (20.0 mL * 3), the combined organic layers were washed with brine (20.0 mL * 3), dried over Na₂SO₄, filtered and concentrated under reduced pressure to give a residue. The residue was purified by column chromatography (SiO₂, Dichloromethane/ Methyl alcohol = 3.00% to 20.0%), (Plate 1, Dichloromethane/ Methyl alcohol = 10/ 1, R_{f} (product 1) = 0.45, R_{f} (product 2) =0.19). Compound 2-(1,3-dioxolan-2-yl)-6-[3-[1-(4-methyl-1,2,4-triazol-3-yl)cyclobutyl]phenyl]-4-(trifluoromethyl)-1H-pyrrolo[2,3-c ]pyridin-7-one (41.0 mg, 67.8 µmol, 14.5% yield, 80.3% purity) was obtained as a colorless oil. Confirmed by LCMS.
LCMS: m/z = 485.7 (M+H)⁺, Rt = 1.255 min

### 2. General steps for preparation of 6-[3-[1-(4-methyl-1,2,4-triazol-3-yl)cyclobutyl]phenyl]-7-oxo-4-(trifluoromethyl)-1H-pyrrolo[2,3-c]pyridine-2-car baldehyde

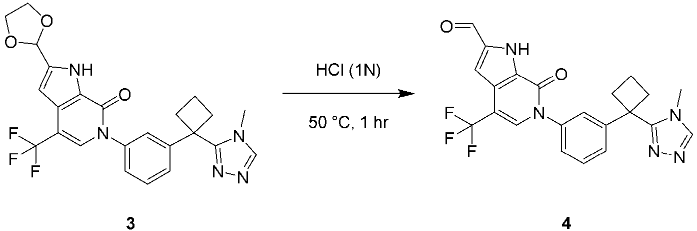

To a solution of 2-(1,3-dioxolan-2-yl)-6-[3-[1-(4-methyl-1,2,4-triazol-3-yl)cyclobutyl]phenyl]-4-(trifluorom ethyl)-1H-pyrrolo[2,3-c]pyridin-7-one (970 mg, 2.00 mmol) in HCl (1 M, 9.00 mL) was stirred at 50 °C f or 1 hr. LCMS showed desired compound was detected. The reaction mixture was diluted with aqueous N aHCO₃ adjust pH = 9, extracted with ethyl acetate (12.0 mL * 3), the combined organic layers were was hed with H₂O (8.00 mL), dried over Na₂SO₄, filtered and concentrated under reduced pressure to give a r esidue. Compound 6-[3-[1-(4-methyl-1,2,4-triazol-3-yl)cyclobutyl]phenyl]-7-oxo-4-(trifluoromethyl)-1H-pyrrolo [2,3-c]pyridine-2-carbaldehyde (940 mg, 2.00 mmol, 99.8% yield, 93.7% purity) was obtained as a colorles s oil. Confirmed by H NMR and LCMS.
¹H NMR: (DMSO-d₆, 400 MHz)
*δ* 13.65 (s, 1H), 9.91 (s, 1H), 8.42-8.69 (m, 1H), 7.87 (d, *J* = 1.2 Hz, 1H), 7.49-7.58 (m, 2H), 7.41 (d, *J* = 7.6 Hz, 1H), 7.29 (d, *J* = 7.6 Hz, 1H), 7.21 (s, 1H), 3.29 (s, 3H), 2.89-2.98 (m, 2H), 2.67-2.75 (m, 2H), 2.01 (s, 1H), 1.96 (s, 1H)
LCMS: EB6214-557-P1C1, m/z = 441.9 (M+H)⁺, Rt = 1.205 min

### 3. General steps for preparation of 2-[[(3S)-3-hydroxypyrrolidin-1-yl]methyl]-6-[3-[1-(4-methyl-1,2,4-triazol-3-yl)cyclobutyl]phenyl]-4-(trifluoromet hyl)-1H-pyrrolo[2,3-c]pyridin-7-one (compound T016)

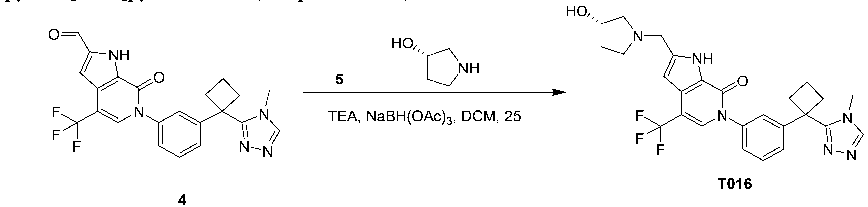

To a solution of 6-[3-[1-(4-methyl-1,2,4-triazol-3-yl)cyclobutyl]phenyl]-7-oxo-4-(trifluoromethyl)-1H-pyrrolo[2,3-c]pyridine-2-car baldehyde (50.0 mg, 113 µmol) in dichloromethane (1.50 mL) was added dropwise TEA (11.4 mg, 113 µmol, 15.7 µL) adjust pH = 7, (3S)-pyrrolidin-3-ol (14.8 mg, 169 µmol, 13.7 µL) was added to the mixture, stirred at 25 °C for 0.5 hr, then NaBH(OAc)₃ (60.0 mg, 283 µmol) was added to the mixture, stirred at 25 °C for 1 hr. LCMS showed desired compound was detected. The reaction mixture was diluted with H₂O (15.0 mL), extracted with ethyl acetate (15.0 mL * 3), the combined organic layers were washed with H₂O (10.0 mL), dried over Na₂SO₄, filtered and concentrated under reduced pressure to give a residue. The residue was purified by Prep-HPLC (column: Welch Xtimate C18 40*200 mm 7 um; mobile phase: [water (HCl)-ACN]; gradient: 0%-36.0% B over 30 mins). Compound 2-[[(3S)-3-hydroxypyrrolidin-1-yl]methyl]-6-[3-[1-(4-methyl-1,2,4-triazol-3-yl)cyclobutyl]phenyl]-4-(trifluoromet hyl)-1H-pyrrolo[2,3-c]pyridin-7-one (6.82 mg, 13.2 µmol, 11.7% yield, 99.9% purity) was obtained as a colorless oil. Confirmed by H NMR and LCMS.
¹H NMR: (DMSO-d₆, 400 MHz)
*δ* 12.81 (s, 1H), 9.41 (s, 1H), 7.82 (s, 1H), 7.55-7.60 (m, 1H), 7.54 (d, *J =* 1.6 Hz, 1H), 7.45 (d, *J =* 8.0 Hz, 1H), 7.41 (d, *J =* 7.6 Hz, 1H), 6.73-6.84 (m, 1H), 4.49-4.57 (m, 2H), 4.39-4.45 (m, 1H), 3.56 (dt, *J =* 12.0, 5.6 Hz, 2H), 3.43 (s, 3H), 3.25 (s, 2H), 3.02-3.09 (m, 1H), 2.91-3.02 (m, 2H), 2.72-2.82 (m, 2H), 2.02-2.27 (m, 2H), 1.80-2.01 (m, 2H)
LCMS: m/z =513.2 (M+H)⁺, Rt = 1.227 min

### Example 17: Synthesis of compound T017

### 1. General steps for preparation of 2-(3-bromophenyl)-2-methyl-propanehydrazide

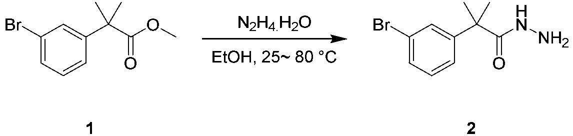

To a solution of methyl 2-(3-bromophenyl)-2-methyl-propanoate (2.64 g, 10.2 mmol) in EtOH (30.0 mL) was added dropwise N₂H₄.H₂O (7.74 g, 151 mmol, 7.50 mL, 98.0% purity) at 25 °C. After addition, the mixture was stirred at 80 °C for 4 hrs. The mixture was cooled to 25 °C, N₂H₄.H₂O (10.3 g, 202 mmol, 10.0 mL, 98.0% purity) was added to the mixture slowly. The mixture was stirred at 80 °C for 4 hrs. LCMS showed desired compound was detected. The reaction mixture was diluted with H₂O (30.0 mL), extracted with ethyl acetate (20.0 mL * 3), the combined organic layers were washed with H₂O (20.0 mL * 3), dried over Na₂SO₄, filtered and concentrated under reduced pressure to give a residue. Compound 2-(3-bromophenyl)-2-methyl-propanehydrazide (2.81 g, 6.97 mmol, 67.9% yield, 63.8% purity) was obtained as a colorless oil.

### 2. General steps for preparation of 1-[[2-(3-bromophenyl)-2-methyl-propanoyl]amino]-3-methyl-thiourea

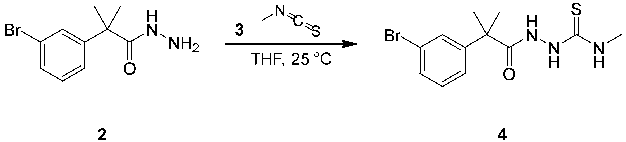

A mixture of 2-(3-bromophenyl)-2-methyl-propanehydrazide (2.64 g, 10.2 mmol), methylimino(thioxo)methane (1.50 g, 20.5 mmol, 1.40 mL) in THF (30.0 mL) was degassed and purged with N₂ for 3 times, and then the mixture was stirred at 25 °C for 6 hrs under N₂ atmosphere. LCMS showed desired compound was detected. The reaction mixture was diluted with H₂O (30.0 mL), extracted with ethyl acetate (20.0 mL * 3), the combined organic layers were washed with H₂O (20.0 mL * 3), dried over Na₂SO₄, filtered and concentrated under reduced pressure to give a residue. Compound 1-[[2-(3-bromophenyl)-2-methyl-propanoyl]amino]-3-methyl-thiourea (3.60 g, 9.36 mmol, 91.2% yield, 85.9% purity) was obtained as a white solid.

### 3. General steps for preparation of 5-[1-(3-bromophenyl)-1-methyl-ethyl]-4-methyl-1,2,4-triazole-3-thiol

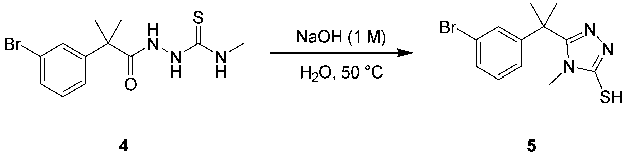

To a solution of 1-[[2-(3-bromophenyl)-2-methyl-propanoyl]amino]-3-methyl-thiourea (3.39 g, 10.2 mmol) in NaOH (1 M, 7.50 mL). The resulting mixture was stirred at 50 °C for 4 hrs. LCMS showed desired compound was detected. The reaction mixture was diluted with water, then the pH value of the solution was adjust to 1 with 1 M HCl and extracted with ethyl acetate (40.0 mL * 3), the combined organic layers were washed with H₂O (50.0 mL * 3), dried over Na₂SO₄, filtered and concentrated under reduced pressure to give a residue. Compound 5-[1-(3-bromophenyl)-1-methyl-ethyl]-4-methyl-1,2,4-triazole-3-thiol (3.20 g, crude) was obtained as a white solid.

### 4. General steps for preparation of 3-[1-(3-bromophenyl)-1-methyl-ethyl]-4-methyl-1,2,4-triazole

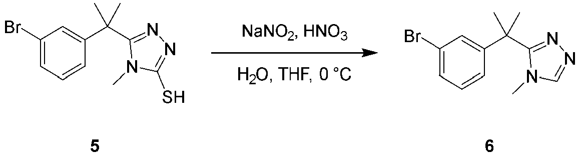

To a solution of 5-[1-(3-bromophenyl)-1-methyl-ethyl]-4-methyl-1,2,4-triazole-3-thiol (3.20 g, 10.2 mmol) in THF (15.0 mL) and H₂O (15.0 mL) was added NaNO₂ (7.07 g, 102 mmol) and HNO₃ (10.4 g, 107 mmol, 7.45 mL, 65.0% purity) slowly at 0 °C. After addition, the mixture was stirred at 0 °C for 1 hr. LCMS showed desired compound was detected. The reaction mixture was diluted with aqueous NaHCO₃ (30.0 mL) adjust pH = 8, extracted with ethyl acetate (40.0 mL * 3), the combined organic layers were washed with H₂O (30.0 mL * 3), dried over Na₂SO₄, filtered and concentrated under reduced pressure to give a residue. The residue was purified by column chromatography (SiO₂, DCM/ MeOH = 0% to 5.00%), (Plate 1, DCM/ MeOH = 10/ 1, R_{f} (product) = 0.34). Compound 3-[1-(3-bromophenyl)-1-methyl-ethyl]-4-methyl-1,2,4-triazole (2.60 g, 8.48 mmol, 82.7% yield, 91.4% purity) was obtained as a colorless oil. Confirmed by H NMR and LCMS.
¹H NMR: (DMSO-d₆, 400 MHz)
*δ* 8.37 (s, 1H), 7.48 (dd, *J= 8.0,* 0.8 Hz, 1H), 7.26-7.35 (m, 2H), 7.02-7.13 (m, 1H), 3.09 (s, 3H), 1.70 (s, 6H)
LCMS: m/z = 279.8 (M+H)⁺, Rt = 1.057 min

### 5. General steps for preparation of (S)-6-(3-(2-(4-methyl-4H-1,2,4-triazol-3-yl)propan-2-yl)phenyl)-2-((3-methylpiperidin-1-yl)methyl)-1-tosyl-4-(trifl uoromethyl)-1,6-dihydro-7H-pyrrolo[2,3-c]pyridin-7-one

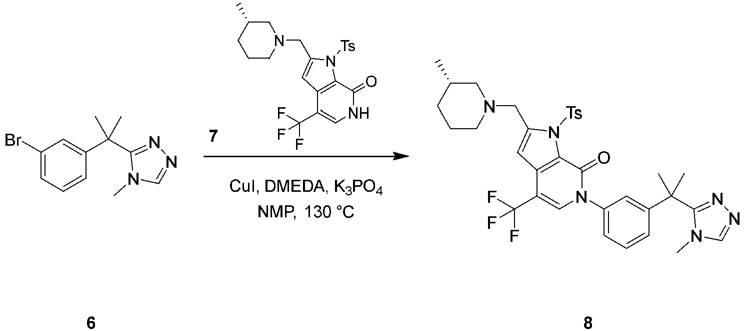

A mixture of 3-[1-(3-bromophenyl)-1-methyl-ethyl]-4-methyl-1,2,4-triazole (29.9 mg, 106 µmol), (S)-2-((3-methylpiperidin-1-yl)methyl)-1-tosyl-4-(trifluoromethyl)-1,6-dihydro-7H-pyrrolo[2,3-c]pyridin-7-one (50.0 mg, 106 µmol), CuI (20.3 mg, 106 µmol), K₃PO₄ (68.1 mg, 320 µmol) and DMEDA (18.8 mg, 213 µmol, 23.0 µL) in NMP (0.50 mL) was degassed and purged with N₂ for 3 times, and then the mixture was stirred at 130 °C for 3 hrs under N₂ atmosphere. LCMS showed desired compound was detected. The reaction mixture was filtered and diluted with H₂O (30.0 mL), extracted with ethyl acetate (20.0 mL * 3), the combined organic layers were washed with H₂O (20.0 mL * 3), dried over Na₂SO₄, filtered and concentrated under reduced pressure to give a residue. The residue was purified by column chromatography (SiO₂, DCM/ MeOH = 0% to 15.0%), (Plate 1, DCM/ MeOH = 10/ 1, R_{f} (product) = 0.34). Compound 6-[3-[1-methyl-1-(4-methyl-1,2,4-triazol-3-yl)ethyl]phenyl]-2-[[(3S)-3-methyl-1-piperidyl]methyl]-1-(p-tolylsulfon yl)-4-(trifluoromethyl)pyrrolo[2,3-c]pyridin-7-one (16.0 mg, 18.2 µmol, 17.0% yield, 76.0% purity) was obtained as a colorless oil. Confirmed by LCMS.
LCMS: m/z = 667.1 (M+H)⁺, Rt = 1.327 min

### 6 General steps for preparation of (S)-6-(3-(2-(4-methyl-4H-1,2,4-triazol-3-yl)propan-2-yl)phenyl)-2-((3-methylpiperidin-1-yl)methyl)-4-(trifluorome thyl)-1,6-dihydro-7H-pyrrolo[2,3-c]pyridin-7-one (compound T017)

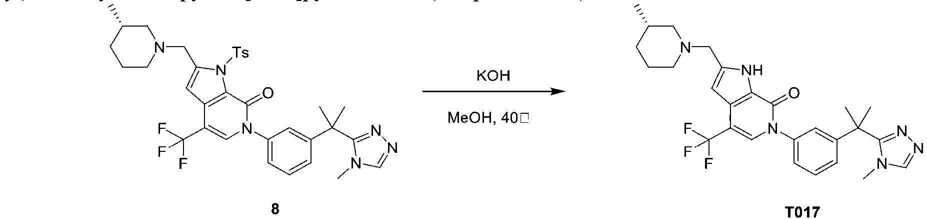

A mixture of (S)-6-(3-(2-(4-methyl-4H-1,2,4-triazol-3-yl)propan-2-yl)phenyl)-2-((3-methylpiperidin-1-yl)methyl)-1-tosyl-4-(trifl uoromethyl)-1,6-dihydro-7H-pyrrolo[2,3-c]pyridin-7-one (16.0 mg, 24.0 µmol), KOH (26.9 mg, 479 µmol) in MeOH (1.00 mL) was degassed and purged with N₂ for 3 times, and then the mixture was stirred at 40 °C for 1 hr under N₂ atmosphere. LCMS showed desired compound was detected. The reaction mixture was diluted with brine (30.0 mL), extracted with ethyl acetate (20.0 mL * 3), the combined organic layers were washed with aqueous KOH (20.0 mL * 2), dried over Na₂SO₄, filtered and concentrated under reduced pressure to give a residue. The residue was purified by Prep-HPLC (column: Xtimate C18 150 * 40 mm * 10 um; mobile phase: [water (FA)-ACN]; gradient: 0%-36.0% B over 25 mins). Compound 6-[3-[1-methyl-1-(4-methyl-1,2,4-triazol-3-yl)ethyl]phenyl]-2-[[(3S)-3-methyl-1-piperidyl]methyl]-4-(trifluoromet hyl)-1H-pyrrolo[2,3-c]pyridin-7-one (2.00 mg, 3.89 µmol, 16.2% yield, 99.7% purity) was obtained as a colorless oil. Confirmed by H NMR and LCMS.
¹H NMR: (CD₃CN, 400 MHz)
*δ* 11.34-12.55 (m, 1H), 8.25 (s, 1H), 7.55 (d, *J =* 1.6 Hz, 1H), 7.46-7.53 (m, 1H), 7.33 (br d, *J =* 8.4 Hz, 1H), 7.23-7.29 (m, 2H), 6.49 (s, 1H), 3.99 (s, 2H), 3.14 (s, 3H), 2.95-3.08 (m, 3H), 2.38 (td, *J* = 10.8, 4.8 Hz, 2H), 2.05-2.12 (m, 1H), 1.78 (s, 6H), 1.75 (br s, 2H), 0.94-1.06 (m, 1H), 0.88 (d, *J =* 6.4 Hz, 3H)
LCMS: m/z = 513.2 (M+H)⁺, Rt = 1.397 min

### Example 18: Synthesis of compound T018

### 1. General steps for preparation of 2-((diethylamino)methyl)-6-(3-((1s,3s)-3-methyl-1-(4-methyl-4H-1,2,4-triazol-3-yl)cyclobutyl)phenyl)-4-(trifluoro methyl)-1,6-dihydro-7H-pyrrolo[2,3-c]pyridin-7-one (compound T018)

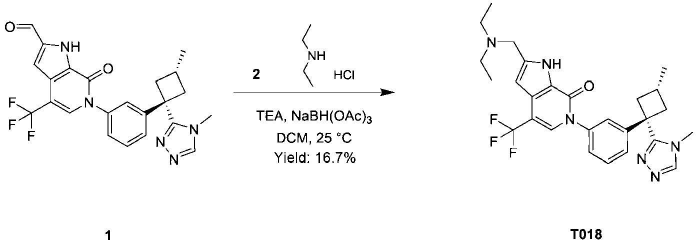

To a solution of 6-(3-((1s,3s)-3-methyl-1-(4-methyl-4H-1,2,4-triazol-3-yl)cyclobutyl)phenyl)-7-oxo-4-(triffuoromethyl)-6,7-dihydro -1H-pyrrolo[2,3-c]pyridine-2-carbaldehyde (45.0 mg, 98.8 µmol) in dichloromethane (2.00 mL) was added dropwise TEA (10.0 mg, 98.8 µmol, 13.7 µL) adjust pH = 7, N-ethylethanamine (12.9 mg, 118 µmol, 18.3 µL, HCl) was added to the mixture, stirred at 25 °C for 0.5 hr, then NaBH(OAc)₃ (52.3 mg, 247 µmol) was added to the mixture, stirred at 25 °C for 1 hr. LCMS showed desired compound was detected. The reaction mixture was diluted with aqueous NaHCO₃ (8.00 mL), extracted with dichloromethane (12.0 mL * 3), the combined organic layers were washed with H₂O (8.00 mL), dried over Na₂SO₄, filtered and concentrated under reduced pressure to give a residue. The residue was purified by Prep-HPLC (column: Welch Xtimate C18 40 * 200 mm 7 um; mobile phase: [water (NH₃H₂O+NH₄HCO₃)-ACN]; gradient:28.0%-68.0% B over 25 mins). Compound 2-((diethylamino)methyl)-6-(3-((1s,3s)-3-methyl-1-(4-methyl-4H-1,2,4-triazol-3-yl)cyclobutyl)phenyl)-4-(trifluoro methyl)-1,6-dihydro-7H-pyrrolo[2,3-c]pyridin-7-one (8.57 mg, 16.5 µmol, 16.7% yield, 99.1% purity) was obtained as a white solid. Confirmed by H NMR and LCMS.
¹H NMR: (DMSO-d₆, 400 MHz)
*δ* 12.12-12.70 (m, 1H), 8.28 (s, 1H), 7.72 (d, *J =* 1.2 Hz, 1H), 7.53 (s, 1H), 7.48-7.52 (m, 1H), 7.38 (s, 1H), 7.33 (s, 1H), 6.27 (s, 1H), 3.69 (s, 2H), 3.25 (s, 3H), 2.84-2.91 (m, 2H), 2.54 (s, 2H), 2.44-2.49 (m, 5H), 1.06 (d, *J* = 5.2 Hz, 3H), 0.99 (t, *J =* 7.2 Hz, 6H)
LCMS: m/z = 513.2 (M+H)⁺, Rt = 1.453 min

### Example 19: Synthesis of compound T019

### 1. General steps for preparation of 2-[[ethyl(isopropyl)amino]methyl]-6-[3-[1-(4-methyl-1,2,4-triazol-3-yl)cyclobutyl]phenyl]-4-(trifluoromethyl)-1H-pyrrolo[2,3-c]pyridin-7-one-pyrrolo[2,3-c]pyridin-7-one (compound T019)

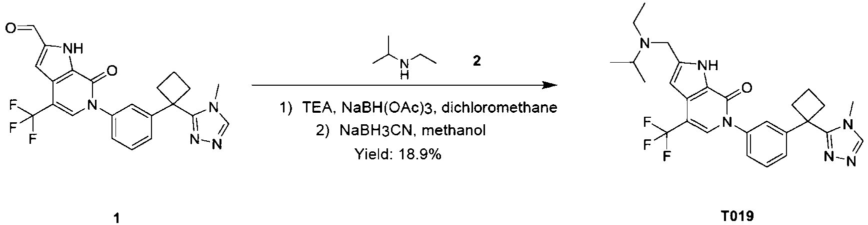

6-[3-[1-(4-methyl-1,2,4-triazol-3-yl)cyclobutyl]phenyl]-7-oxo-4-(trifluoromethyl)-1H-pyrrolo[2,3-c]pyridine-2-carbaldehyde (50.0 mg, 113 µmol) was dissolved in Dichloromethane 2.00 ml, pH was adjusted to 7-8 with TEA. N-ethylpropan-2-amine (14.8 mg, 170 µmol, 20.6 µL) in Dichloromethane 2.00 ml was added to the mixture, stirred at 25 °C for0.5 hrs. NaBH(OAc)₃ (60.0 mg, 283 µmol) was added to the mixture , stirred at 25 °C under N₂ for 3 hrs. N-ethylpropan-2-amine (14.8 mg, 170 µmol, 20.6 µL) in Dichloromethane 2.00 ml was added to the mixture, stirred at 25 °C for0.5 hrs. NaBH(OAc)₃ (60.0 mg, 283 µmol) was added to the mixture , stirred at 35 °C under N₂ for 3 hrs. NaBH(OAc)₃ (60.0 mg, 283 µmol) and Methanol (1.50 mL) was added to the mixture, the mixture was stirred at 35 °C for 12 hrs. NaBH₃CN (10.7 mg, 170 µmol) was added to the mixture, the mixture was stirred at 35 °C for 3 hrs. LCMS shows reactant was consumed completely and desired mass was detected. The reaction mixture was diluted with Dichloromethane 6.00 mL, washed with sat. NaHCO₃ solution 3.00 mL. The water phase extracted with Dichloromethane 12.0 mL (6.00 mL * 2) and Ethyl acetate 12.0 mL (6.0 mL * 2). The combined organic layers were dried over Na₂SO₄, filtered and concentrated under reduced pressure to give a residue. The residue was purified by prep. HPLC (column: Welch Xtimate C18 40 * 200 mm 7 um; mobile phase: [water (FA)-ACN]; gradient: 0%-36% B over 25 min). 2-[[ethyl(isopropyl)amino]methyl]-6-[3-[1-(4-methyl-1,2,4-triazol-3-yl)cyclobutyl]phenyl]-4-(trifluoromethyl)-1H-pyrrolo[2,3-c]pyridin-7-one (11.0 mg, 21.4 µmol, 18.9% yield, 99.9% purity) was obtained as white solid, confirmed by H NMR and LCMS.
¹H NMR: (400 MHz, DMSO-d₆)
*δ* 11.41-13.18 (m, 1H), 8.34 (s, 1H), 8.17 (s, 1H), 7.70 (d, *J =* 1.2 Hz, 1H), 7.48-7.54 (m, 1H), 7.43 (s, 1H), 7.35 (d, *J =* 8.0 Hz, 1H), 7.26 (d, *J =* 8.0 Hz, 1H), 6.29 (s, 1H), 3.67 (s, 2H), 3.27 (s, 3H), 2.89-2.98 (m, 3H), 2.65-2.74 (m, 2H), 2.47 (d, *J* = 7.2 Hz, 2H), 1.90-2.06 (m, 2H), 0.93-1.01 (m, 9H)
LCMS: m/z = 513.2 (M+H)⁺, Rt = 1.401 min

### Example 20: Synthesis of compound T020

### 1. General steps for preparation of 2-[[(3S)-3-fluoropyrrolidin-1-yl]methyl]-6-[3-[1-(4-methyl-1,2,4-triazol-3-yl)cyclobutyl]phenyl]-4-(trifluoromethyl )-1H-pyrrolo[2,3-c]pyridin-7-one (compound T020)

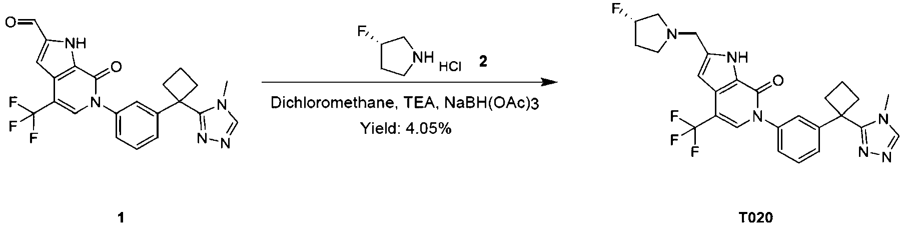

6-[3-[1-(4-methyl-1,2,4-triazol-3-yl)cyclobutyl]phenyl]-7-oxo-4-(trifluoromethyl)-1H-pyrrolo[2,3-c]pyridine-2-carbaldehyde (50.0 mg, 113 µmol) was dissolved in Dichloromethane (1.00 mL), pH was adjust to 7-8 with TEA. (3S)-3-fluoropyrrolidine;hydrochloride (21.3 mg, 170 µmol) in Dichloromethane 0.50 mL was added to the mixture, stirred at 25 °C for 0.5 hr under N₂. Then NaBH(OAc)₃ (60.0 mg, 283 µmol) was added to the mixture, then stirred at 25 °C under N₂ for 2 hrs. LCMS shows reactant was consumed completely and desired mass was detected. The reaction mixture was diluted with Dichloromethane 6.00 mL, washed with sat. NaHCO₃ solution 2.00 mL. The water phase extracted with Dichloromethane 2.00 mL (1.00 mL * 2). The combined organic layers were washed, dried over Na₂SO₄, filtered and concentrated under reduced pressure to give a residue. The residue was purifie by prep.HPLC (column: Welch Xtimate C18 40 * 200 mm 7 um;mobile phase: [water (HCl) - ACN];gradient: 0%-38% B over 30 min). 2-[[(3S)-3-fluoropyrrolidin-1-yl]methyl]-6-[3-[1-(4-methyl-1,2,4-triazol-3-yl)cyclobutyl]phenyl]-4-(trifluoromethyl )-1H-pyrrolo[2,3-c]pyridin-7-one (2.43 mg, 4.59 µmol, 4.05% yield, 97.2% purity) was obtained as off-white solid, confirmed by H NMR and LCMS.
¹H NMR: (400 MHz, DMSO-d₆)
*δ* 12.39 (s, 1H), 8.35 (s, 1H), 7.70 (s, 1H), 7.46-7.56 (m, 1H), 7.42 (s, 1H), 7.35 (d, *J =* 8.0 Hz, 1H), 7.26 (d*, J* = 8.0 Hz, 1H), 6.27 (s, 1H), 3.60 (s, 2H), 3.26 (s, 3H), 2.90-2.97 (m, 2H), 2.66-2.73 (m, 2H), 2.43 (s, 2H), 2.14 (s, 2H), 1.93-2.03 (m, 2H), 1.55-1.62 (m, 2H), 1.23 (d, *J =* 5.2 Hz, 2H), 0.25 (s, 4H)
LCMS: m/z = 515.2 (M+H)⁺, Rt = 1.337 min

### Example 21: Synthesis of compound T021

### 1. General steps for preparation of 2-[[(3R)-3-fluoropyrrolidin-1-yl]methyl]-6-[3-[1-(4-methyl-1,2,4-triazol-3-yl)cyclobutyl]phenyl]-4-(trifluoromethy l)-1H-pyrrolo[2,3-c]pyridin-7-one (compound T021)

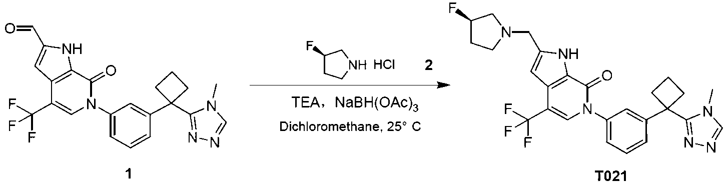

To a mixture of 6-[3-[1-(4-methyl-1,2,4-triazol-3-yl)cyclobutyl]phenyl]-7-oxo-4-(trifluoromethyl)-1H-pyrrolo[2,3-c]pyridine-2-car baldehyde (50.0 mg, 113 µmol) in Dichloromethane (3.00 mL) was added TEA (22.9 mg, 226 µmol) adjust pH = 8. Then (3R)-3-fluoropyrrolidine;hydrochloride (28.4 mg, 226 µmol) was added to the mixture. The mixture was stirred under N₂ at 25 °C for 0.5 hr. NaBH(OAc)₃ (60.0 mg, 283 µmol) was added to the mixture. The mixture was stirred under N₂ at 25 °C for 0.5 hr. LCMS showed desired mass was detected. The reaction mixture was diluted with NaHCO₃ (3.00 mL), extracted with Dichloromethane (3.00 mL*2). The organic phase dried over Na₂SO₄ and concentrated in vacuum. The crude product was purified by Prep-HPLC (column: Welch Xtimate C18 40*200 mm 7 um; mobile phase: [water (HCl)-ACN]; gradient: 0%-38.0% B over 30 min). To afford 2-[[(3R)-3-fluoropyrrolidin-1-yl]methyl]-6-[3-[1-(4-methyl-1,2,4-triazol-3-yl)cyclobutyl]phenyl]-4-(trifluoromethy l)-1H-pyrrolo[2,3-c]pyridin-7-one (19.3 mg, 32.8 µmol, 29.0% yield, 100% purity, 2HCl) as a white solid which was confirmed by H NMR and LCMS.
¹H NMR: (400 MHz, DMSO-d₆)
*δ* 12.85 (s, 1H), 9.48 (s, 1H), 7.83 (s, 1H), 7.50-7.62 (m, 2H), 7.43 (dd, *J =* 13.2, 8.0 Hz, 2H), 6.82 (s, 1H), 5.37-5.55 (m, 1H), 4.57 (br s, 2H), 3.52-3.73 (m, 3H), 3.44 (s, 3H), 3.23-3.38 (m, 1H), 2.95-3.04 (m, 2H), 2.70-2.82 (m, 2H), 2.11-2.46 (m, 2H), 1.95-2.09 (m, 2H)
LCMS: m/z = 515.2 (M+H)⁺, Rt = 1.138 min

### Example 22: Synthesis of compound T022

### 1. General steps for preparation of 4-isopropenyl-7-methoxy-2-[[(3S)-3-methyl-1-piperidyl]methyl]-1-(p-tolylsulfonyl)pyrrolo[2,3-c]pyridine

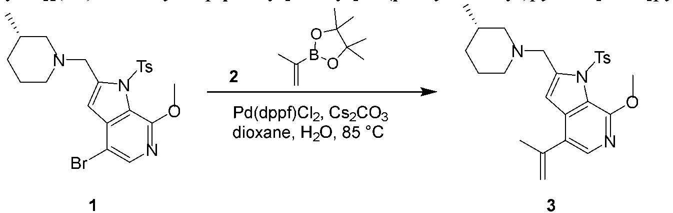

To a solution of 4-bromo-7-methoxy-2-[[(3S)-3-methyl-1-piperidyl]methyl]-1-(p-tolylsulfonyl)pyrrolo[2,3-c]pyridine (300 mg, 609 µmol), 2-isopropenyl-4,4,5,5-tetramethyl-1,3,2-dioxaborolane (307 mg, 1.83 mmol) in dioxane (5.0 mL), H₂O (1.0 mL) was added Pd(dppf)Cl₂ (44.5 mg, 60.9 µmol), Cs₂CO₃ (595 mg, 1.83 mmol) under N₂ at 25 °C. The mixture was stirred at 85 °C for 2 hrs. LCMS showed the reaction was completed. The reaction mixture was diluted with H₂O (10.0 mL), extracted with EtOAc (10.0 mL*2). The organic phase was dried over and concentrated in vacuum. The residue was purified by column chromatography (SiO₂, DCM/ MeOH = 1/ 0 to 97/ 3). TLC (Plate 1, DCM/ MeOH = 20/ 1, UV 254 nm, R_{f} (product) = 0.3). Compound 4-isopropenyl-7-methoxy-2-[[(3S)-3-methyl-1-piperidyl]methyl]-1-(p-tolylsulfonyl)pyrrolo[2,3-c]pyridine (276 mg, 608 µmol, 99.8% yield) was obtained as a yellow oil.

### 2. General steps for preparation of 4-isopropyl-7-methoxy-2-[[(3S)-3-methyl-1-piperidyl]methyl]-1-(p-tolylsulfonyl)pyrrolo[2,3-c]pyridine

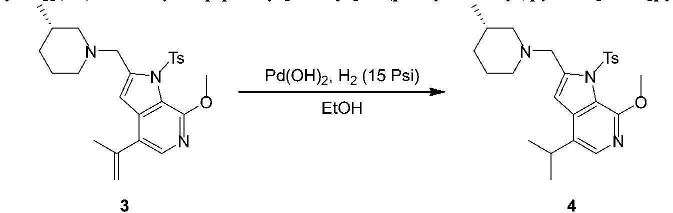

To a mixture of 4-isopropenyl-7-methoxy-2-[[(3S)-3-methyl-1-piperidyl]methyl]-1-(p-tolylsulfonyl)pyrrolo[2,3-c]pyridine (246 mg, 542 µmol) in EtOH (5.0 mL) was added Pd(OH)₂ (200 mg, 284 µmol, 20.0 % purity) under Ar. The suspension was degassed under vacuum and purged with H₂ several times. The mixture was stirred under H₂ (15 psi) at 25°C for 2 hrs. LCMS showed the reaction was completed. The reaction mixture was filtered and concentrated under reduced pressure to give a residue. The residue was purified by column chromatography (SiO₂, DCM/ MeOH = 1/ 0 to 97/ 3). TLC (Plate 1, DCM/ MeOH = 10/ 1, UV 254 nm, R_{f} (product) = 0.3). Compound 4-isopropyl-7-methoxy-2-[[(3S)-3-methyl-1-piperidyl]methyl]-1-(p-tolylsulfonyl)pyrrolo[2,3-c]pyridine (174 mg, 381 µmol, 70.4% yield) was obtained as a colourless oil.

### 3. General steps for preparation of 4-isopropyl-2-[[(3S)-3-methyl-1-piperidyl]methyl]-1-(p-tolylsulfonyl)-6H-pyrrolo[2,3-c]pyridin-7-one

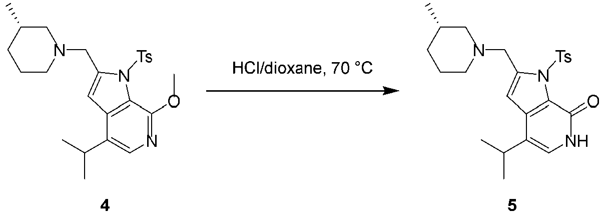

To a mixture of 4-isopropyl-7-methoxy-2-[[(3S)-3-methyl-1-piperidyl]methyl]-1-(p-tolylsulfonyl)pyrrolo[2,3-c]pyridine (80.0 mg, 175 µmol) in HCl/ dioxane (4.0 M, 1.0 mL). The mixture was stirred at 70 °C for 2 hrs. LCMS showed the reaction was completed. EB6215-239 was combined for workup. The reaction mixture was diluted with EtOAc (50.0 mL) poured into saturated aqueous NaHCO₃ (30.0 mL) slowly, adjust pH = 8. The organic phase was washed with brine (50.0 mL), dried over. The organic phase was concentrated in vacuum. The residue was purified by column chromatography (SiO₂, DCM/ MeOH = 1/ 0 to 95/ 5). TLC (Plate 1, DCM/ MeOH = 10/ 1, UV 254 nm, R_{f} (product) = 0.5). Compound 4-isopropyl-2-[[(3S)-3-methyl-1-piperidyl]methyl]-1-(p-tolylsulfonyl)-6H-pyrrolo[2,3-c]pyridin-7-one (80.0 mg, 90.5 µmol, 51.5% yield) was obtained as a white solid.

### 4. General steps for preparation of 4-isopropyl-2-[[(3S)-3-methyl-1-piperidyl]methyl]-6-[3-[3-[(4-methyl-1,2,4-triazol-3-yl)methyl]oxetan-3-yl]phenyl ]-1-(p-tolylsulfonyl)pyrrolo[2,3-c]pyridin-7-one

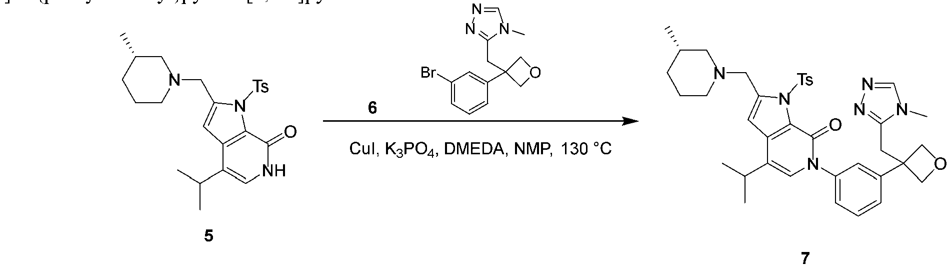

To a mixture of 4-isopropyl-2-[[(3S)-3-methyl-1-piperidyl]methyl]-1-(p-tolylsulfonyl)-6H-pyrrolo[2,3-c]pyridin-7-one (39.0 mg, 88.3 µmol), 3-[[3-(3-bromophenyl)oxetan-3-yl]methyl]-4-methyl-1,2,4-triazole (29.9 mg, 97.1 µmol), K₃PO₄ (56.2 mg, 264 µmol) and CuI (16.8 mg, 88.3 µmol) in NMP (1.0 mL) was added N,N'-dimethylethane-1,2-diamine (15.5 mg, 176 µmol, 19.0 µL). The suspension was degassed under vacuum and purged with N₂ several times. The mixture was stirred under N₂ at 130 °C for 12 hrs. LCMS showed the reaction was completed. EB6215-241 was combined for workup. The reaction mixture was diluted with EtOAc (30.0 mL), washed with brine (30.0 mL * 3), dried over Na₂SO₄. The organic phase was concentrated in vacuum. The residue was purified by column chromatography (SiO₂, DCM/ MeOH = 1/ 0 to 97/ 3). TLC (Plate 1, DCM/ MeOH = 10/ 1, UV 254 nm, R_{f} (product) = 0.6). Compound 4-isopropyl-2-[[(3S)-3-methyl-1-piperidyl]methyl]-6-[3-[3-[(4-methyl-1,2,4-triazol-3-yl)methyl]oxetan-3-yl]phenyl ]-1-(p-tolylsulfonyl)pyrrolo[2,3-c]pyridin-7-one (80.0 mg, 59.8 µmol, 67.7% yield) was obtained as a colourless solid.

### 5. General steps for preparation of 4-isopropyl-2-[[(3S)-3-methyl-1-piperidyl]methyl]-6-[3-[3-[(4-methyl-1,2,4-triazol-3-yl)methyl]oxetan-3-yl]phenyl ]-1H-pyrrolo[2,3-c]pyridin-7-one (compound T022)

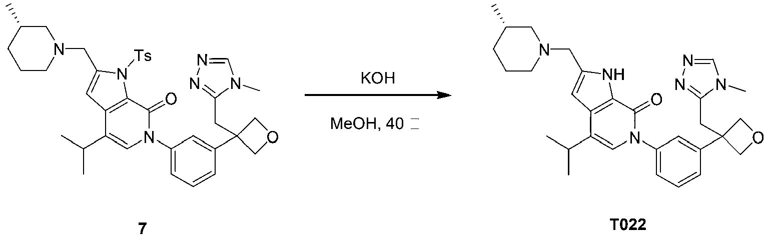

To a mixture of 4-isopropyl-2-[[(3S)-3-methyl-1-piperidyl]methyl]-6-[3-[3-[(4-methyl-1,2,4-triazol-3-yl)methyl]oxetan-3-yl]phenyl ]-1-(p-tolylsulfonyl)pyrrolo[2,3-c]pyridin-7-one (80.0 mg, 119 µmol) in MeOH (2.5 mL) was added KOH (134 mg, 2.39 mmol). The mixture was stirred under N₂ at 25 °C for 2 hrs. LCMS showed the reaction was completed. The reaction mixture was adjust pH = 5 (HCl, 1 M). The mixture was filtered to get a filtrate. The product was purified by Prep-HPLC (column: Welch Xtimate C 18 150 * 30 mm * 5 um; mobile phase: [water (FA) - ACN]; gradient: 0% - 34% B over 25 mins). Compound 4-isopropyl-2-[[(3S)-3-methyl-1-piperidyl]methyl]-6-[3-[3-[(4-methyl-1,2,4-triazol-3-yl)methyl]oxetan-3-yl]phenyl ]-1H-pyrrolo[2,3-c]pyridin-7-one (2.4 mg, 4.58 µmol, 3.83% yield, 98.21% purity) was obtained as a white solid. It was confirmed by LCMS and H NMR.
¹H NMR: (DMSO-d₆, 400 MHz)
*δ* 11.94 (br s, 1H), 8.19 (s, 1H), 7.23-7.47 (m, 2H), 6.87-7.08 (m, 2H), 6.67 (s, 1H), 6.30 (s, 1H), 4.85-4.99 (m, 4H), 3.71-3.80 (m, 2H), 3.32-3.45 (m, 3H), 2.93-3.02 (m, 4H), 2.77 (br s, 2H), 1.91 (br t, *J =* 10.4 Hz, 1H), 1.60 (br s, 4H), 1.45 (br d, *J* = 11.6 Hz, 1H), 1.26 (br d, *J =* 6.8 Hz, 6H), 0.81 (br d, *J* = 4.4 Hz, 3H)
LCMS: m/z = 515.5 (M+H)⁺, Rt = 1.010 min

### Example 23: Synthesis of compound T023

### 1. General steps for preparation of 2-(5-azaspiro[2.4]heptan-5-ylmethyl)-6-[3-[1-(4-methyl-1,2,4-triazol-3-yl)cyclobutyl]phenyl]-4-(trifluoromethyl)-1 H-pyrrolo[2,3-c]pyridin-7-one (compound T023)

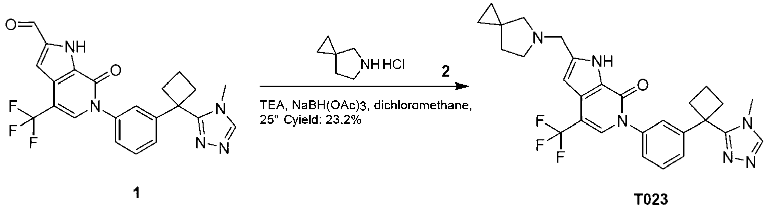

To a mixture of 5-azaspiro[2.4]heptane (22.7 mg, 169 µmol, HCl) in Dichloromethane (3.00 mL) was added TEA (57.3 mg, 566 µmol) adjust pH = 8. Then 6-[3-[1-(4-methyl-1,2,4-triazol-3-yl)cyclobutyl]phenyl]-7-oxo-4-(trifluoromethyl)-1H-pyrrolo[2,3-c]pyridine-2-car baldehyde (50.0 mg, 113 µmol) was added to the mixture. The mixture was stirred under N₂ at 25 °C for 0.5 hr. NaBH(OAc)₃ (60.0 mg, 283 µmol) was added to the mixture. The mixture was stirred under N₂ at 25 °C for 2.5 hrs. LCMS showed desired mass was detected. The reaction mixture was diluted with NaHCO₃ (3.00 mL), extracted with Dichloromethane (2.00 mL*2). The organic phase dried over Na₂SO₄ and concentrated in vacuum. The crude product was purified by Prep-HPLC (column: Welch Xtimate C18 40*200 mm 7 um; mobile phase: [water (FA)-ACN]; gradient: 0%-38.0% B over 25 min). To afford 2-(5-azaspiro[2.4]heptan-5-ylmethyl)-6-[3-[1-(4-methyl-1,2,4-triazol-3-yl)cyclobutyl]phenyl]-4-(trifluoromethyl)-1 H-pyrrolo[2,3-c]pyridin-7-one (15.0 mg, 26.3 µmol, 23.2% yield, 100% purity, FA) as a brown solid which was confirmed by H NMR and LCMS.
¹H NMR: (400 MHz, DMSO-d₆)
*δ* 12.49 (s, 1H), 8.35 (s, 1H), 7.71 (s, 1H), 7.48-7.54 (m, 1H), 7.42 (s, 1H), 7.35 (d, *J =* 8.0 Hz, 1H), 7.26 (d, *J* = 8.0 Hz, 1H), 6.31 (s, 1H), 3.74 (s, 2H), 3.26 (s, 3H), 2.93 (dd, *J =* 6.0, 2.8 Hz, 2H), 2.67-2.73 (m, 4H), 2.48 (s, 2H), 1.94-2.03 (m, 2H), 1.74 (t, *J =* 6.8 Hz, 2H), 0.50 (s, 2H), 0.48 (s, 2H)
LCMS: m/z = 523.2 (M+H)⁺, Rt = 1.415 min

### Examples 24 and 25: Synthesis of compounds T024 & T025

### 1. General steps for preparation of 2-(3-azabicyclo[4.1.0]heptan-3-ylmethyl)-6-[3-[1-(4-methyl-1,2,4-triazol-3-yl)cyclobutyl]phenyl]-4-(trifluorometh yl)-1H-pyrrolo[2,3-c]pyridin-7-one

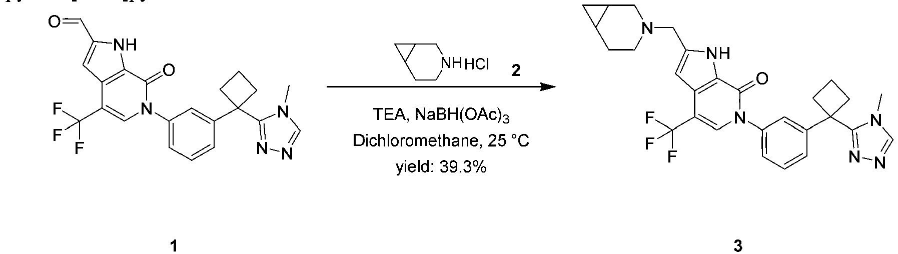

To a mixture of 3-azabicyclo[4.1.0]heptane (60.5 mg, 452 µmol, HCl) in Dichloromethane (5.00 mL) was added TEA (115 mg, 1.14 mmol) adjust pH = 8. Then 6-[3-[1-(4-methyl-1,2,4-triazol-3-yl)cyclobutyl]phenyl]-7-oxo-4-(trifluoromethyl)-1H-pyrrolo[2,3-c]pyridine-2-car baldehyde (100 mg, 226 µmol) was added to the mixture. The mixture was stirred under N₂ at 25 °C for 0.5 hr. NaBH(OAc)₃ (120 mg, 566 µmol) was added to the mixture. The mixture was stirred under N₂ at 25 °C for 2.5 hrs. LCMS showed desired mass was detected. The reaction mixture was diluted with NaHCO₃ (3.00 mL), extracted with Dichloromethane (2.00 mL*2). The organic phase dried over Na₂SO₄ and concentrated in vacuum. The crude product was purified by Prep-HPLC (column: Welch Xtimate C18 40*200 mm 7 um; mobile phase: [water (FA)-ACN]; gradient: 0%-38.0% B over 25 min). To afford 2-(3-azabicyclo[4.1.0]heptan-3-ylmethyl)-6-[3-[1-(4-methyl-1,2,4-triazol-3-yl)cyclobutyl]phenyl]-4-(trifluorometh yl)-1H-pyrrolo[2,3-c]pyridin-7-one (50.0 mg, 89.1 µmol, 39.3% yield, 93.2% purity) as a white solid which was confirmed by LCMS.
LCMS: m/z = 523.3 (M+H)⁺, Rt = 1.478 min

### 2. General steps for preparation of 2-[[(1R,6R)-3-azabicyclo[4.1.0]heptan-3-yl]methyl]-6-[3-[1-(4-methyl-1,2,4-triazol-3-yl)cyclobutyl]phenyl]-4-(trif luoromethyl)-1H-pyrrolo[2,3-c]pyridin-7-one (compound T024) and 2-[[(1S,6S)-3-azabicyclo[4.1.0]heptan-3-yl]methyl]-6-[3-[1-(4-methyl-1,2,4-triazol-3-yl)cyclobutyl]phenyl]-4-(triff uoromethyl)-1H-pyrrolo[2,3-c]pyridin-7-one (compound T025)

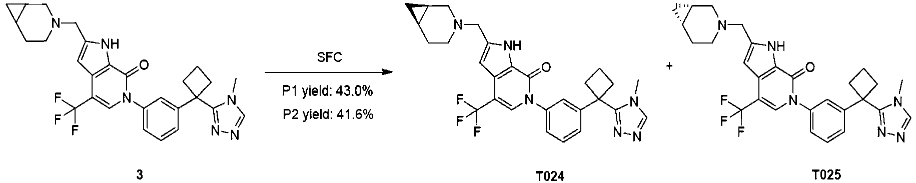

SFC (EB6211-765-P1A) showed two peaks. The crude product was purified by SFC (column: DAICEL CHIRALPAK AD(250mm*30mm,10um);mobile phase: [CO2-EtOH(0.1%NH3H2O)];B%:45%, isocratic elution mode). To afford 2-[[(1R,6R)-3-azabicyclo[4.1.0]heptan-3-yl]methyl]-6-[3-[1-(4-methyl-1,2,4-triazol-3-yl)cyclobutyl]phenyl]-4-(trif luoromethyl)-1H-pyrrolo[2,3-c]pyridin-7-one (21.5 mg, 41.14 µmol, 43.00% yield, 100% purity) as a white solid which was confirmed by H NMR and LCMS.
¹H NMR: (400 MHz, DMSO-d₆)
*δ* 12.40 (s, 1H), 8.35 (s, 1H), 7.71 (s, 1H), 7.47-7.54 (m, 1H), 7.43 (s, 1H), 7.35 (d, *J =* 8.4 Hz, 1H), 7.25 (d*, J* = 8.0 Hz, 1H), 6.25 (s, 1H), 3.49-3.57 (m, 2H), 3.27 (s, 3H), 2.90-2.98 (m, 2H), 2.70 (dd, *J =* 10.4, 6.4 Hz, 3H), 2.55 (d, *J* = 5.2 Hz, 1H), 2.24-2.31 (m, 1H), 1.95-2.04 (m, 3H), 1.90 (dd, *J =* 12.8, 8.4 Hz, 1H), 1.60-1.70 (m, 1H), 0.94-1.03 (m, 1H), 0.84-0.89 (m, 1H), 0.50 (td, *J =* 8.4, 3.6 Hz, 1H), 0.24-0.35 (m, 1H)
LCMS: m/z = 523.3 (M+H)⁺, Rt = 1.488 min

To afford 2-[[(1S,6S)-3-azabicyclo[4.1.0]heptan-3-yl]methyl]-6-[3-[1-(4-methyl-1,2,4-triazol-3-yl)cyclobutyl]phenyl]-4-(triff uoromethyl)-1H-pyrrolo[2,3-c]pyridin-7-one (20.8 mg, 39.80 µmol, 41.60% yield, 100% purity) as a white solid which was confirmed by H NMR and LCMS.
¹H NMR: (400 MHz, DMSO-d₆)
*δ* 12.40 (s, 1H), 8.35 (s, 1H), 7.71 (s, 1H), 7.46-7.54 (m, 1H), 7.43 (s, 1H), 7.36 (d, *J* = 8.0 Hz, 1H), 7.25 (d, *J* = 7.6 Hz, 1H), 6.25 (s, 1H), 3.48-3.57 (m, 2H), 3.27 (s, 3H), 2.90-2.98 (m, 2H), 2.70 (dd, *J* = 10.4, 6.8 Hz, 3H), 2.53-2.57 (m, 1H), 2.27 (dt, *J* = 10.8, 5.2 Hz, 1H), 1.95-2.03 (m, 3H), 1.90 (dd, *J* = 13.2, 8.0 Hz, 1H), 1.61-1.69 (m, 1H), 0.96-1.04 (m, 1H), 0.84-0.90 (m, 1H), 0.50 (td, *J* = 8.4, 3.2 Hz, 1H), 0.30 (q, *J* = 4.8 Hz, 1H)
LCMS: m/z = 523.3 (M+H)⁺, Rt = 1.482 min

### Examples 26 and 27: Synthesis of compounds T026 & T027

### 1. General steps for preparation of 2-(2-azabicyclo[4.1.0]heptan-2-ylmethyl)-6-[3-[1-(4-methyl-1,2,4-triazol-3-yl)cyclobutyl]phenyl]-4-(trifluorometh yl)-1H-pyrrolo[2,3-c]pyridin-7-one

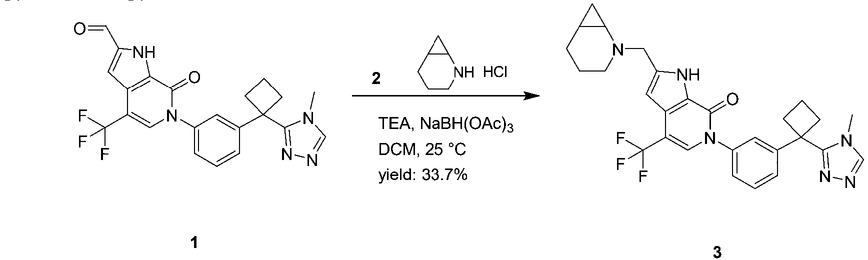

To a solution of 6-[3-[1-(4-methyl-1,2,4-triazol-3-yl)cyclobutyl]phenyl]-7-oxo0-4-(trifluoromethyl)-1H-pyrrolo[2,3-c]pyridine-2-car baldehyde (100 mg, 226 µmol) in dichloromethane (4.00 mL) was added dropwise TEA (22.9 mg, 226 µmol, 31.5 µL) adjust pH = 7, 2-azabicyclo[4.1.0]heptane (39.3 mg, 294 µmol, HCl) was added to the mixture, stirred at 25 °C for 0.5 hr, then NaBH(OAc)₃ (120 mg, 566 µmol) was added to the mixture, stirred at 25 °C for 1 hr. LCMS showed desired compound was detected. The reaction mixture was diluted with aqueous NaHCO₃ (8.00 mL), extracted with dichloromethane (12.0 mL * 3), the combined organic layers were washed with H₂O (8.00 mL), dried over Na₂SO₄, filtered and concentrated under reduced pressure to give a residue. The residue was purified by Prep-HPLC (column: Welch Xtimate C18 40 * 200 mm 7 um; mobile phase: [water (NH₄HCO₃)-ACN]; gradient:28.0%-68.0% B over 25 mins). Compound 2-(2-azabicyclo[4.1.0]heptan-2-ylmethyl)-6-[3-[1-(4-methyl-1,2,4-triazol-3-yl)cyclobutyl]phenyl]-4-(trifluorometh yl)-1H-pyrrolo[2,3-c]pyridin-7-one (40.0 mg, 76.5 µmol, 33.7% yield, 100% purity) was obtained as a white solid..

### 2. General steps for preparation of 2-(((1R,6S)-2-azabicyclo[4.1.0]heptan-2-yl)methyl)-6-(3-(1-(4-methyl-4H-1,2,4-triazol-3-yl)cyclobutyl)phenyl)-4-( trifluoromethyl)-1,6-dihydro-7H-pyrrolo[2,3-c]pyridin-7-one (compound T026) and 2-(((1S,6R)-2-azabicyclo[4.1.0]heptan-2-yl)methyl)-6-(3-(1-(4-methyl-4H-1,2,4-triazol-3-yl)cyclobutyl)phenyl)-4-( trifluoromethyl)-1,6-dihydro-7H-pyrrolo[2,3-c]pyridin-7-one (compound T027)

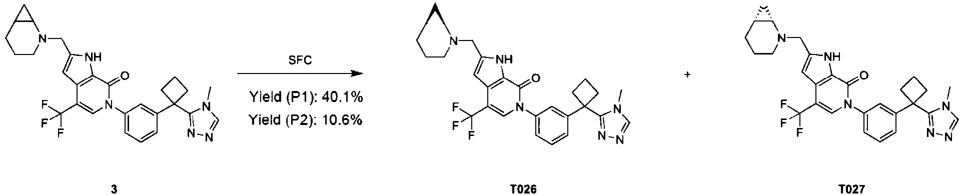

The residue was purified by SFC (column: DAICEL CHIRALPAK AD (250 mm * 30 mm,10 um); mobile phase: [CO₂-EtOH (0.1% NH₃H₂O)]; B%:60.0%, isocratic elution mode). Compound 2-(((1R,6S)-2-azabicyclo[4.1.0]heptan-2-yl)methyl)-6-(3-(1-(4-methyl-4H-1,2,4-triazol-3-yl)cyclobutyl)phenyl)-4-( trifluoromethyl)-1,6-dihydro-7H-pyrrolo[2,3-c]pyridin-7-one (16.5 mg, 30.7 µmol, 40.1% yield, 96.9% purity) was obtained as a white solid. Confirmed by H NMR and LCMS.
1H NMR: (DMSO-d6, 400 MHz)
δ 12.45 (s, 1H), 8.35 (s, 1H), 7.70 (s, 1H), 7.48-7.54 (m, 1H), 7.43 (s, 1H), 7.36 (d, *J* = 8.0 Hz, 1H), 7.25 (d, *J* = 8.0 Hz, 1H), 6.32 (s, 1H), 3.63-3.86 (m, 2H), 3.27 (s, 3H), 2.90-2.98 (m, 2H), 2.66-2.73 (m, 2H), 2.40-2.45 (m, 1H), 2.23 (dd, *J* = 8.0, 6.4 Hz, 1H), 2.10-2.19 (m, 1H), 1.95-2.04 (m, 2H), 1.89 (dd, *J* = 13.2, 7.6 Hz, 1H), 1.37 (d, *J* = 4.4 Hz, 2H), 1.23 (s, 1H), 0.92-1.03 (m, 1H), 0.27-0.33 (m, 1H), 0.22-0.27 (m, 1H)
LCMS: m/z = 523.2 (M+H)⁺, Rt = 1.320 min

The residue (product 2) was purified by Prep-HPLC (column: Welch Xtimate C18 40 * 200 mm 7 um; mobile phase: [water (NH₃H₂O+NH₄HCO₃)-ACN]; gradient:30.0%-70.0% B over 25 mins). Compound 2-(((1S,6R)-2-azabicyclo[4.1.0]heptan-2-yl)methyl)-6-(3-(1-(4-methyl-4H-1,2,4-triazol-3-yl)cyclobutyl)phenyl)-4-( trifluoromethyl)-1,6-dihydro-7H-pyrrolo[2,3-c]pyridin-7-one (4.34 mg, 8.18 µmol, 10.6% yield, 98.5% purity) was obtained as a white solid. Confirmed by H NMR and LCMS.
¹H NMR: (DMSO-*d*₆, 400 MHz)
*δ* 11.61-12.94 (m, 1H), 8.35 (s, 1H), 7.70 (s, 1H), 7.48-7.53 (m, 1H), 7.42 (s, 1H), 7.35 (d, *J* = 7.6 Hz, 1H), 7.26 (d, *J* = 8.0 Hz, 1H), 6.32 (s, 1H), 3.81 (s, 1H), 3.66-3.73 (m, 1H), 3.26 (s, 3H), 2.89-2.97 (m, 2H), 2.66-2.73 (m, 2H), 2.40-2.45 (m, 1H), 2.19-2.26 (m, 1H), 2.11-2.18 (m, 1H), 1.93-2.06 (m, 2H), 1.88 (dd, *J* = 13.2, 7.6 Hz, 1H), 1.41-1.51 (m, 1H), 1.36 (d, *J* = 4.4 Hz, 2H), 0.92-1.06 (m, 1H), 0.18-0.35 (m, 2H)
LCMS: m/z = 523.2 (M+H)⁺, Rt = 1.383 min

### Example 28: Synthesis of compound T028

### 1. General steps for preparation of 2-((3-azabicyclo[3.1.0]hexan-3-yl)methyl)-6-(3-((1s,3s)-3-methyl-1-(4-methyl-4H-1,2,4-triazol-3-yl)cyclobutyl)ph enyl)-4-(trifluoromethyl)-1,6-dihydro-7H-pyrrolo[2,3-c]pyridin-7-one (compound T028)

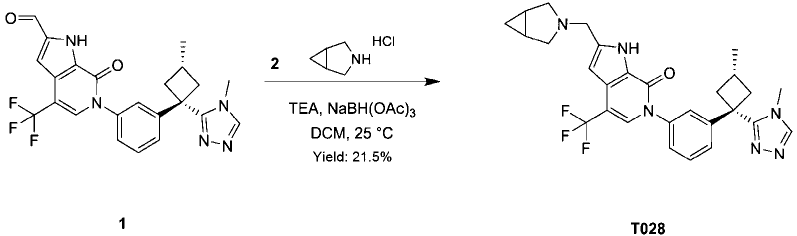

To a solution of 6-(3-((1s,3s)-3-methyl-1-(4-methyl-4H-1,2,4-triazol-3-yl)cyclobutyl)phenyl)-7-oxo-4-(trifluoromethyl)-6,7-dihydro -1H-pyrrolo[2,3-c]pyridine-2-carbaldehyde (50.0 mg, 109 µmol) in dichloromethane (2.00 mL) was added dropwise TEA (11.1 mg, 109 µmol, 15.2 µL) adjust pH = 7, 3-azabicyclo[3.1.0]hexane (17.0 mg, 142 µmol, HCl) was added to the mixture, stirred at 25 °C for 0.5 hr, then NaBH(OAc)₃ (58.1 mg, 274 µmol) was added to the mixture, stirred at 25 °C for 1 hr. LCMS showed desired compound was detected. The reaction mixture was diluted with aqueous NaHCO₃ (8.00 mL), extracted with dichloromethane (12.0 mL * 3), the combined organic layers were washed with H₂O (8.00 mL), dried over Na₂SO₄, filtered and concentrated under reduced pressure to give a residue. The residue was purified by Prep-HPLC (column: Welch Xtimate C18 40 * 200 mm 7 um; mobile phase: [water (NH₄HCO₃)-ACN]; gradient:32.0%-72.0% B over 25 minS). Compound 2-((3-azabicyclo[3.1.0]hexan-3-yl)methyl)-6-(3-((1s,3s)-3-methyl-1-(4-methyl-4H-1,2,4-triazol-3-yl)cyclobutyl)ph enyl)-4-(trifluoromethyl)-1,6-dihydro-7H-pyrrolo[2,3-c]pyridin-7-one (12.4 mg, 23.6 µmol, 21.5% yield, 99.7% purity) was obtained as a white solid. Confirmed by H NMR and LCMS.
¹H NMR: (DMSO-d₆, 400 MHz)
*δ* 12.41 (s, 1H), 8.28 (s, 1H), 7.71 (d, *J* = 1.2 Hz, 1H), 7.47-7.55 (m, 2H), 7.35 (t, *J* = 8. Hz, 2H), 6.24 (s, 1H), 3.71 (s, 2H), 3.25 (s, 3H), 2.87 (d, *J* = 8.6 Hz, 4H), 2.53 (d, *J* = 7.2 Hz, 3H), 2.37 (d, *J* = 8.0 Hz, 2H), 1.31-1.38 (m, 2H), 1.07 (d, *J* = 5.2 Hz, 3H), 0.66 (q, *J* = 3.2 Hz, 1H), 0.30 (td, *J* = 7.6, 3.6 Hz, 1H)
LCMS: m/z = 523.2 (M+H)⁺, Rt = 1.397 min

### Example 29: Synthesis of compound T029

### 1. General steps for preparation of 2-((isopropyl(propyl)amino)methyl)-6-(3-((1s,3s)-3-methyl-1-(4-methyl-4H-1,2,4-triazol-3-yl)cyclobutyl)phenyl)-4 -(trifluoromethyl)-1,6-dihydro-7H-pyrrolo[2,3-c]pyridin-7-one (compound T029)

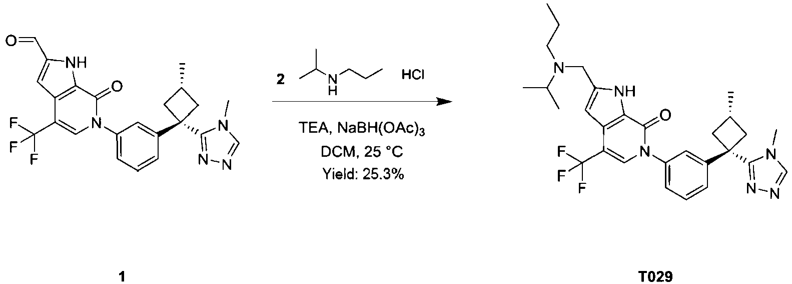

To a solution of 6-(3-((1s,3s)-3-methyl-1-(4-methyl-4H-1,2,4-triazol-3-yl)cyclobutyl)phenyl)-7-oxo-4-(triffuoromethyl)-6,7-dihydro -1H-pyrrolo[2,3-c]pyridine-2-carbaldehyde (50.0 mg, 109 µmol) in dichloromethane (1.00 mL) was added dropwise TEA (11.1 mg, 109 µmol, 15.2 µL) adjust pH = 7, N-isopropylpropan-1-amine (22.6 mg, 164 µmol, HCl) was added to the mixture, stirred at 25 °C for 0.5 hr, then NaBH(OAc)₃ (58.1 mg, 274 µmol) was added to the mixture, stirred at 25 °C for 1 hr. LCMS showed desired compound was detected. The reaction mixture was diluted with aqueous NaHCO₃ (8.00 mL), extracted with dichloromethane (12.0 mL * 3), the combined organic layers were washed with H₂O (8.00 mL), dried over Na₂SO₄, filtered and concentrated under reduced pressure to give a residue. The residue was purified by Prep-HPLC (column: Welch Xtimate C18 40 * 200 mm 7 um; mobile phase: [water (NH₄HCO₃)-ACN]; gradient:46.0%-86.0% B over 25 mins). Compound 2-((isopropyl(propyl)amino)methyl)-6-(3-((1s,3s)-3-methyl-1-(4-methyl-4H-1,2,4-triazol-3-yl)cyclobutyl)phenyl)-4 -(trifluoromethyl)-1,6-dihydro-7H-pyrrolo[2,3-c]pyridin-7-one (15.1 mg, 27.7 µmol, 25.3% yield, 99.5% purity) was obtained as a colorless oil. Confirmed by H NMR and LCMS.
¹H NMR: (DMSO- d₆, 400 MHz)
*δ* 12.36 (s, 1H), 8.28 (s, 1H), 7.70 (d, *J* = 0.8 Hz, 1H), 7.53 (s, 1H), 7.47-7.52 (m, 1H), 7.35 (t, *J* = 9.2 Hz, 2H), 6.29 (s, 1H), 3.65 (s, 2H), 3.25 (s, 3H), 2.84-2.97 (m, 3H), 2.51-2.58 (m, 3H), 2.36 (t, *J* = 7.2 Hz, 2H), 1.31-1.41 (m, 2H), 1.07 (d, *J* = 5.2 Hz, 3H), 0.97 (d, *J* = 6.4 Hz, 6H), 0.81 (t, *J* = 7.2 Hz, 3H)
LCMS: m/z = 541.3 (M+H)⁺, Rt = 1.607 min

### Example 30: Synthesis of compound T030

### 1. General steps for preparation of methyl 5-(3-bromophenyl)spiro[2.3]hexane-5-carboxylate

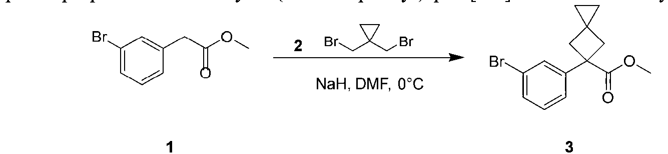

To a solution of NaH (1.05 g, 26.3 mmol, 60.0% purity) in DMF (20 mL) was added 1,1-bis(bromomethyl)cyclopropane (2.0 g, 8.77 mmol) in DMF (1.0 mL) under N₂ at 0 °C. Then methyl 2-(3-bromophenyl)acetate (2.01 g, 8.77 mmol) in DMF (2.0 mL) was added to the mixture. The mixture was stirred at 0 °C for 2 hrs. LCMS showed the reaction was completed. The recation mixture was added dropwise into saturated aqueous NH₄Cl (50 mL) slowly, extracted with EtOAc (50 mL * 2). The combined organic layers were washed with brine, then dried over Na₂SO₄ and filtered. The residue was purified by column chromatography (SiO₂, PE/ EtOAc = 1/ 0 to 90/ 10). TLC (Plate 1, PE/ EtOAc = 5/ 1, UV 254 nm, R_{f} (product) = 0.7). Compound methyl 5-(3-bromophenyl)spiro[2.3]hexane-5-carboxylate (750 mg, 2.54 mmol, 28.9% yield) was obtained as a colourless oil. It was confirmed by H NMR.
¹H NMR: (400 MHz, CDCl₃)
*δ* 7.48 (t, *J* = 1.6 Hz, 1H), 7.39 (dt, *J* = 7.6, 1.6 Hz, 1H), 7.26 (t, *J* = 1.6 Hz, 1H), 7.18-7.24 (m, 1H), 3.69 (s, 3H), 2.88-2.94 (m, 2H), 2.62-2.70 (m, 2H), 0.50-0.59 (m, 2H), 0.37-0.46 (m, 2H)

### 2. General steps for preparation of 5-(3-bromophenyl)spiro[2.3]hexane-5-carbohydrazide

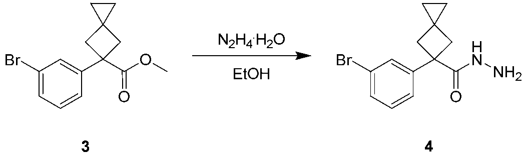

To a solution of methyl 5-(3-bromophenyl)spiro[2.3]hexane-5-carboxylate (750 mg, 2.54 mmol) in EtOH (7.0 mL) was added N₂H₄·H₂O (10.0 g, 196 mmol, 9.72 mL, 98.0% purity). The mixture was stirred at 80 °C for 12 hrs. LCMS showed the reaction was completed. The reaction mixture was diluted with H₂O (30 mL), extracted with EtOAc (30 mL * 3), dried over Na₂SO₄, filtered and concentrated under reduced pressure to give a residue. Compound 5-(3-bromophenyl)spiro[2.3]hexane-5-carbohydrazide (780 mg, crude) was obtained as a colourless oil.

### 3. General steps for preparation of 1-[[5-(3-bromophenyl)spiro[2.3]hexane-5-carbonyl]amino]-3-methyl-thiourea

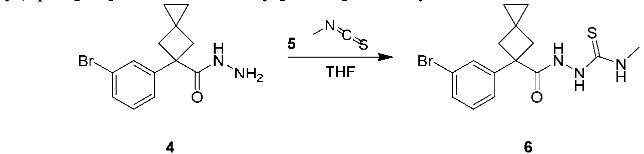

To a solution of 5-(3-bromophenyl)spiro[2.3]hexane-5-carbohydrazide (780 mg, 2.64 mmol) in THF (7.0 mL) was added methylimino(thioxo)methane (289 mg, 3.96 mmol, 271 µL). The mixture was stirred at 25 °C for 6 hrs. LCMS showed the reaction was completed. The reaction mixture was concentrated under reduced pressure to give a residue. Compound 1-[[5-(3-bromophenyl)spiro[2.3]hexane-5-carbonyl]amino]-3-methyl-thiourea (800 mg, 2.17 mmol, 82.2% yield) was obtained as a white solid.

### 4. General steps for preparation of 5-[5-(3-bromophenyl)spiro[2.3hexan-5-yl]-4-methyl-1,2.4-triazole-3-thiol

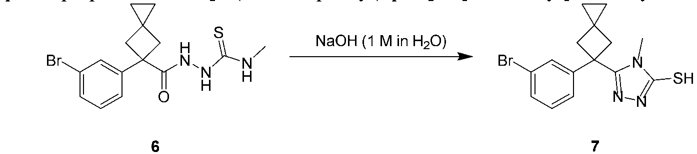

To a solution of 1-[[5-(3-bromophenyl)spiro[2.3]hexane-5-carbonyl]amino]-3-methyl-thiourea (800 mg, 2.17 mmol) was added NaOH (1 M, 10 mL). The mixture was stirred at 25 °C for 2 hrs. LCMS showed the reaction was completed. The reaction mixture was diluted with water, then the pH value of the solution was adjust to 5 with 1 M HCl and extracted with EtOAc (20 mL * 3), the combined organic layers were washed with H₂O (10 mL * 3), dried over Na₂SO₄, filtered and concentrated under reduced pressure to give a residue. Compound 5-[5-(3-bromophenyl)spiro[2.3]hexan-5-yl]-4-methyl-1,2,4-triazole-3-thiol (800 mg, crude) was obtained as a white solid.

### 5. General steps for preparation of 3-[5-(3-bromophenyl)spiro[2.3]hexan-5-yl]-4-methyl-1,2,4-triazole

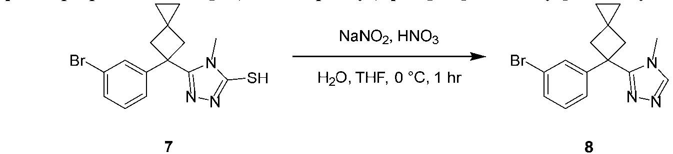

To a solution of 5-[5-(3-bromophenyl)spiro[2.3]hexan-5-yl]-4-methyl-1,2,4-triazole-3-thiol (800 mg, 2.28 mmol) in THF (3.0 mL) and H₂O (3.0 mL) was added NaNO₂ (1.58 g, 22.8 mmol) and HNO₃ (2.62 g, 27.0 mmol, 1.87 mL, 65.0% purity) at 0 °C slowly. After addition, the mixture was stirred at 0 °C for 2 hrs. LCMS showed the reaction was completed. The mixture was basified by saturated aqueous sodium bicarbonate solution then extracted with EtOAc (15 mL * 3). The combined organic layers were dried over Na₂SO₄, filtered and concentrated under reduced pressure to give a residue. The residue was purified by column chromatography (SiO₂, DCM/ MeOH = 1/ 0 to 97/ 3). TLC (Plate 1, DCM/ MeOH = 10/ 1, UV 254 nm, R_{f} (product) = 0.5). Compound 3-[5-(3-bromophenyl)spiro[2.3]hexan-5-yl]-4-methyl-1,2,4-triazole (740 mg, crude) was obtained as a yellow oil. It was confirmed by H NMR.
¹H NMR: (400 MHz, DMSO-d₆)
*δ* 8.39 (s, 1H), 7.47 (br d, *J* = 8.0 Hz, 1H), 7.38 (s, 1H), 7.31-7.37 (m, 1H), 7.24-7.30 (m, 1H), 3.19 (s, 3H), 3.11-3.18 (m, 4H), 0.50-0.60 (m, 2H), 0.39-0.49 (m, 2H)

### 6. General steps for preparation of 4-cyclopropyl-2-[[(3S)-3-methyl-1-piperidyl]methyl]-6-[3-[5-(4-methyl-1,2,4-triazol-3-yl)spiro[2.3]hexan-5-yl]phe nyl]-1-(p-tolylsulfonyl)pyrrolo[2,3-c]pyridin-7-one

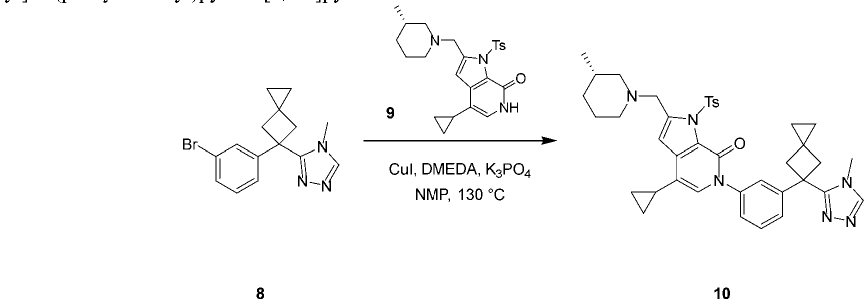

To a mixture of 3-[5-(3-bromophenyl)spiro[2.3]hexan-5-yl]-4-methyl-1,2,4-triazole (100 mg, 314 µmol), 4-cyclopropyl-2-[[(3S)-3-methyl-1-piperidyl]methyl]-1-(p-tolylsulfonyl)-6H-pyrrolo[2,3-c]pyridin-7-one (151 mg, 345 µmol), CuI (59.8 mg, 314 µmol) and K₃PO₄ (200 mg, 942 µmol) in NMP (1.0 mL) was added DMEDA (55.4 mg, 628 µmol, 67.6 µL). The suspension was degassed under vacuum and purged with N₂ several times. The mixture was stirred under N₂ at 130 °C for 1 hr. LCMS showed the reaction was completed. The reaction mixture was diluted with EtOAc (30 mL), washed with brine (30 mL * 3), dried over Na₂SO₄. The organic phase was concentrated in vacuum. The residue was purified by column chromatography (SiO₂, DCM/ MeOH = 1/ 0 to 97/ 3). TLC (Plate 1, DCM/ MeOH = 10/ 1, UV 254 nm, R_{f} (product) = 0.5). Compound 4-cyclopropyl-2-[[(3S)-3-methyl-1-piperidyl]methyl]-6-[3-[5-(4-methyl-1,2,4-triazol-3-yl)spiro[2.3]hexan-5-yl]phe nyl]-1-(p-tolylsulfonyl)pyrrolo[2,3-c]pyridin-7-one (150 mg, 205 µmol, 65.2% yield, 92.5% purity) was obtained as a white solid. It was confirmed by LCMS.
LCMS: m/z = 677.2 (M+H)⁺, Rt = 0.505 min

### 7. General steps for preparation of 4-cyclopropyl-2-[[(3S)-3-methyl-1-piperidyl]methyl]-6-[3-[5-(4-methyl-1,2,4-triazol-3-yl)spiro[2.3]hexan-5-yl]phe nyl]-1H-pyrrolo[2,3-c]pyridin-7-one (compound T030)

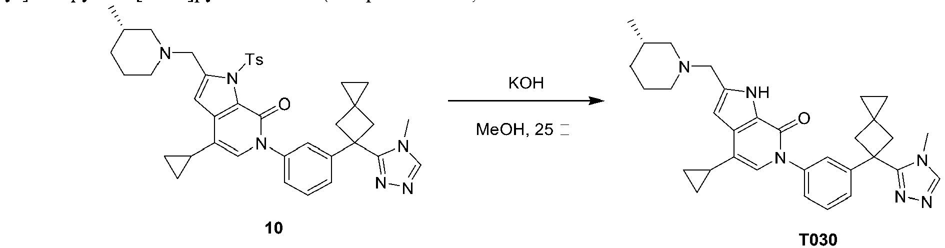

To a mixture of 4-cyclopropyl-2-[[(3S)-3-methyl-1-piperidyl]methyl]-6-[3-[5-(4-methyl-1,2,4-triazol-3-yl)s piro[2.3]hexan-5-yl]phenyl]-1-(p-tolylsulfonyl)pyrrolo[2,3-c]pyridin-7-one (150 mg, 221 µmol) in MeOH (1.5 mL) was added KOH (248 mg, 4.43 mmol). The mixture was stirred at 25 °C for 2 hrs. LCMS showed the reaction was completed. The reaction mixture was diluted with EtOAc (10 mL), washed with brine ( 10 mL * 3), dried over Na₂SO₄. The organic phase was concentrated in vacuum. The product was purifie d by Prep-HPLC (column: Xtimate C18 150 * 40 mm * 10 um; mobile phase: [water(HCl)-ACN]; gradie nt: 8%-48% B over 30 min). Compound 4-cyclopropyl-2-[[(3S)-3-methyl-1-piperidyl]methyl]-6-[3-[5-(4-meth yl-1,2,4-triazol-3-yl)spiro[2.3]hexan-5-yl]phenyl]-1H-pyrrolo[2,3-c]pyridin-7-one (70.0 mg, 133 µmol, 60.2% yield, 99.62% purity) was obtained as a white solid. It was confirmed by LCMS and H NMR.
¹H NMR: (400 MHz, DMSO-d6)
*δ* 12.34 (s, 1H), 9.48 (s, 1H), 7.53-7.63 (m, 1H), 7.42 (br dd, *J* = 3.6, 1.6 Hz, 2H), 7.37 (br d, *J* = 8.0 Hz, 1H), 6.91 (s, 1H), 6.76 (d, *J* = 2.0 Hz, 1H), 4.70-4.77 (m, 1H), 4.36 (br d, *J* = 4.0 Hz, 2H), 3.46 (s, 3H), 3.26-3.39 (m, 2H), 3.23 (br d, *J* = 12.4 Hz, 2H), 2.83 (br d, *J* = 12.8 Hz, 2H), 2.68-2.78 (m, 1H), 1.94-2.06 (m, 1H), 1.86-1.93 (m, 1H), 1.81 (br s, 2H), 1.71 (br d, *J* = 12.4 Hz, 1H), 0.99-1.09 (m, 1H), 0.88 (d, *J* = 6.4 Hz, 3H), 0.80-0.86 (m, 2H), 0.66-0.73 (m, 2H), 0.54-0.62 (m, 2H), 0.44-0.54 (m, 2H)
LCMS: m/z = 523.3 (M+H)⁺, Rt = 1.280 min

### Example 31: Synthesis of compound T031

### 1. General steps for preparation of 6-[3-[1-(4-methyl-1,2,4-triazol-3-yl)cyclobutyl]phenyl]-2-[[(1S,4S)-2-oxa-5-azabicyclo[2.2.1]heptan-5-yl]methyl]-4-(trifluoromethyl)-1H-pyrrolo[2,3-c]pyridin-7-one (compound T031)

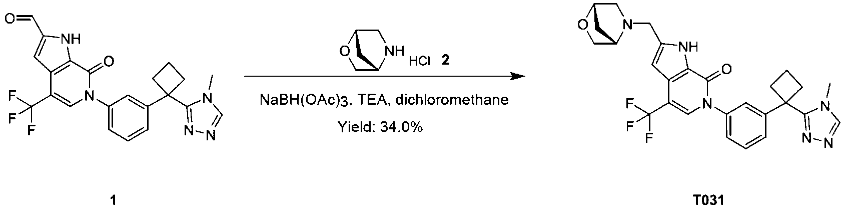

6-[3-[1-(4-methyl-1,2,4-triazol-3-yl)cyclobutyl]phenyl]-7-oxo-4-(trifluoromethyl)-1H-pyrrolo[2,3-c]pyridine-2-carbaldehyde (50.0 mg, 113 µmol) was dissolved in Dichloromethane (1.00 mL), pH was adjust to 7-8 with TEA. (1S,4S)-2-oxa-5-azabicyclo[2.2.1]heptane;hydrochloride (23.0 mg, 170 µmol) in DCM 0.5 mL was added to the mixture, stirred at 25 °C for 0.5 hr under N₂. Then NaBH(OAc)₃ (60.0 mg, 283 µmol) was added to the mixture, then stirred at 25 °C under N₂ for 2 hrs. LCMS shows reactant was consumed completely and desired mass was detected. The reaction mixture was diluted with Dichloromethane 6.00 mL, washed with sat. NaHCO₃ solution 2.00 mL. The water phase extracted with Dichloromethane 2.00 mL (1.00mL * 2). The combined organic layers were washed, dried over Na₂SO₄, filtered and concentrated under reduced pressure to give a residue. The residue was purified by prep.HPLC( column: Welch Xtimate C18 40 * 200 mm 7 um;mobile phase: [water (HCl) - ACN];gradient: 0%-38% B over 30 min). 6-[3-[1-(4-methyl-1,2,4-triazol-3-yl)cyclobutyl]phenyl]-2-[[(1S,4S)-2-oxa-5-azabicyclo[2.2.1]heptan-5-yl]methyl]-4-(trifluoromethyl)-1H-pyrrolo[2,3-c]pyridin-7-one (20.2 mg, 38.5 µmol, 34.0% yield, 100% purity) was obtained as white solid, confirmed by H NMR and LCMS.
¹H NMR: (400 MHz, DMSO-*d*₆)
*δ* 12.70-12.82 (m, 1H), 11.61 (br d, *J* = 2.8 Hz, 1H), 9.44 (s, 1H), 7.83 (s, 1H), 7.55-7.60 (m, 1H), 7.53 (s, 1H), 7.39-7.47 (m, 2H), 6.85 (s, 1H), 4.22-4.73 (m, 6H), 3.43 (s, 3H), 3.34-3.41 (m, 1H), 3.10-3.21 (m, 1H), 2.94-3.05 (m, 2H), 2.73-2.83 (m, 2H), 2.20-2.35 (m, 1H), 1.93-2.16 (m, 3H)
LCMS: m/z = 525.2 (M+H)⁺, Rt = 1.292 min

### Example 32: Synthesis of compound T032

### 1. General steps for preparation of 6-[3-[1-(4-methyl-1,2,4-triazol-3-yl)cyclobutyl]phenyl]-2-[[(1R,4R)-2-oxa-5-azabicyclo[2.2.1]heptan-5-yl]methyl]-4-(trifluoromethyl)-1H-pyrrolo[2,3-c]pyridin-7-one (compound T032)

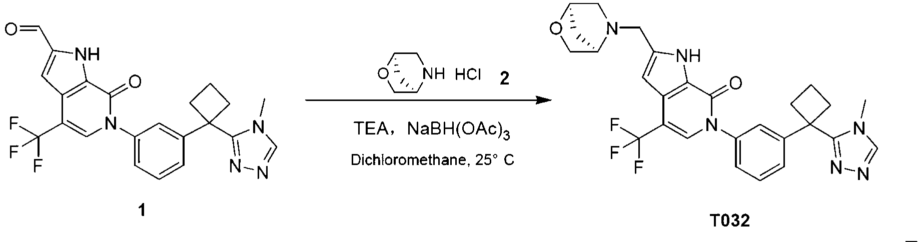

To a mixture of (1R,4R)-2-oxa-5-azabicyclo[2.2.1]heptane (30.7 mg, 226 µmol, HCl) in Dichloromethane (3.00 mL) was added TEA (22.9 mg, 226 µmol) adjust pH = 8. Then 6-[3-[1-(4-methyl-1,2,4-triazol-3-yl)cyclobutyl]phenyl]-7-oxo-4-(trifluoromethyl)-1H-pyrrolo[2,3-c]pyridine-2-car baldehyde (50.0 mg, 113 µmol) was added to the mixture. The mixture was stirred under N₂ at 25 °C for 0.5 hr. NaBH(OAc)₃ (60.0 mg, 283 µmol) was added to the mixture. The mixture was stirred under N₂ at 25 °C for 0.5 hr. LCMS showed desired mass was detected. The reaction mixture was diluted with NaHCO₃ (30.0 mL), extracted with Dichloromethane (20.0 mL*2). The organic phase dried over Na₂SO₄ and concentrated in vacuum. The crude product was purified by Prep-HPLC (column: Welch Xtimate C18 40*200 mm 7 um; mobile phase: [water (FA)-ACN]; gradient: 0%-34.0% B over 25 min). To afford 6-[3-[1-(4-methyl-1,2,4-triazol-3-yl)cyclobutyl]phenyl]-2-[[(1R,4R)-2-oxa-5-azabicyclo[2.2.1]heptan-5-yl]methyl]-4-(trifluoromethyl)-1H-pyrrolo[2,3-c]pyridin-7-one (21.4 mg, 37.5 µmol, 33.1% yield, 100% purity, FA) as a white solid which was confirmed by H NMR AND LCMS.
¹H NMR: (400 MHz, DMSO-*d*₆)
*δ* 12.43 (s, 1H), 8.35 (s, 1H), 7.71 (s, 1H), 7.46-7.55 (m, 1H), 7.42 (s, 1H), 7.35 (d, *J* = 8.0 Hz, 1H), 7.26 (d, *J* = 8.0 Hz, 1H), 6.31 (s, 1H), 4.35 (s, 1H), 3.89 (d, *J* = 7.6 Hz, 1H), 3.82-3.87 (m, 1H), 3.77-3.81 (m, 1H), 3.53 (d, *J* = 7.6 Hz, 2H), 3.49 (s, 1H), 3.26 (s, 3H), 2.90-2.97 (m, 2H), 2.79 (d, *J* = 9.2 Hz, 1H), 2.66-2.73 (m, 2H), 1.93-2.03 (m, 2H), 1.76 (d, *J* = 9.2 Hz, 1H), 1.59 (d, *J* = 9.2 Hz, 1H)
LCMS: m/z = 525.0 (M+H)⁺, Rt = 0.932 min

### Example 33: Synthesis of compound T033

### 1. General steps for preparation of methyl 1-(3-bromophenyl)cyclobutanecarboxylate

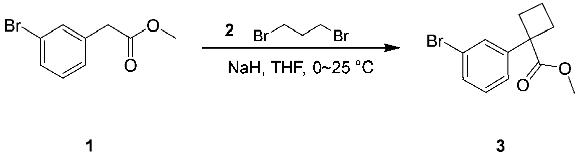

To a solution of methyl 2-(3-bromophenyl)acetate (10.0 g, 43.6 mmol) and 1,3-dibromopropane (9.25 g, 45.8 mmol, 4.67 mL) in DMF (100 mL) was added NaH (3.49 g, 87.3 mmol, 60.0% purity) under N₂ at 0 °C. The mixture was stirred under N₂ at 25 °C for 2 hrs. TLC showed the reaction was completed, TLC (Plate 1, PE/ EtOAc = 5/ 1, I₂, R_{f} (product) = 0.5). The reaction mixture was poured into saturated aqueous NH₄Cl (200 mL) slowly. The reaction mixture was diluted with EtOAc (200 mL), washed with brine (200 mL * 3), dried over Na₂SO₄. The organic phase was concentrated in vacuum. The residue was purified by column chromatography (SiO₂, PE/ EtOAc = 1/ 0 to 94/ 6). Compound methyl 1-(3-bromophenyl)cyclobutanecarboxylate (9.20 g, 34.1 mmol, 78.3% yield) was obtained as a colourless oil. It was confirmed by H NMR.
¹H NMR: (400 MHz, CD₃Cl)
*δ* 7.47-7.52 (m, 1H), 7.39-7.47 (m, 1H), 7.27-7.34 (m, 1H), 7.22-7.27 (m, 1H), 3.72 (s, 3H), 2.85-2.92 (m, 2H), 2.50-2.58 (m, 2H), 2.08-2.15 (m, 1H), 1.88-1.99 (m, 1H)

### 2. General steps for preparation of 1-(3-bromophenyl)cyclobutanecarbohydrazide

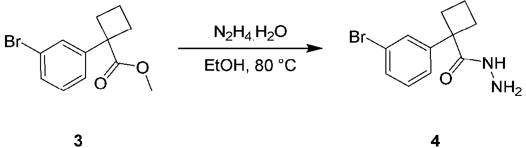

To a solution of methyl 1-(3-bromophenyl)cyclobutanecarboxylate (9.00 g, 33.4 mmol) in EtOH (20.0 mL) was added N₂H₄·H₂O (68.1 g, 1.33 mol, 66.0 mL, 98.0% purity) under N₂. The mixture was stirred under N₂ at 80 °C for 12 hrs. LCMS showed the reaction was completed. The reaction mixture was concentrated in vacuum. The mixture was diluted water(100 mL), extracted with with EtOAc (150 * 2 mL). The organic phase was dried over Na₂SO₄ and concentrated in vacuum. Compound 1-(3-bromophenyl)cyclobutanecarbohydrazide (9.00 g, 33.4 mmol, 100% yield) was obtained as a white solid.

### 3. General steps for preparation of 1-[[1-(3-bromophenyl)cyclobutanecarbonyl]amino]-3-methyl-thiourea

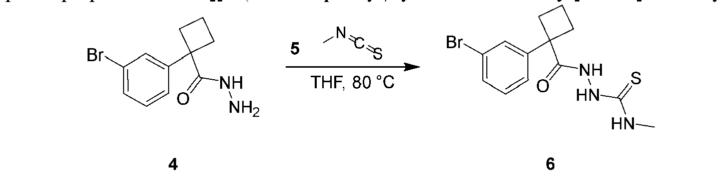

To a solution of 1-(3-bromophenyl)cyclobutanecarbohydrazide (9.00 g, 33.4 mmol) in THF (80.0 mL) was added methylimino(thioxo)methane (3.67 g, 50.1 mmol, 3.43 mL) under N₂ at 25 °C. The mixture was stirred under N₂ at 80 °C for 3 hrs. LCMS showed the reaction was completed. The reaction mixture was concentrated in vacuum. Compound 1-[[1-(3-bromophenyl)cyclobutanecarbonyl]amino]-3-methyl-thiourea (9.00 g, 26.3 mmol, 78.6% yield) was obtained as a yellow solid.

### 4. General steps for preparation of 5-[1-(3-bromophenyl)cyclobutyl]-4-methyl-1,2,4-triazole-3-thiol

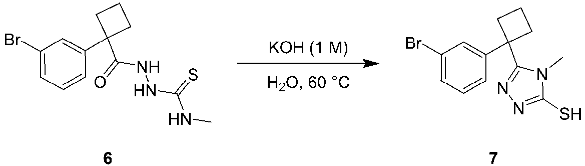

To a solution of 1-[[1-(3-bromophenyl)cyclobutanecarbonyl]amino]-3-methyl-thiourea (8.80 g, 25.7 mmol) in THF (10.0 mL) was added KOH (5.70 M, 22.5 mL) under N₂ at 25 °C. The mixture was stirred under N₂ at 80 °C for 3 hrs. LCMS showed the reaction was completed. The reaction mixture was concentrated in vacuum. Compound 5-[1-(3-bromophenyl)cyclobutyl]-4-methyl-1,2,4-triazole-3-thiol (10.4 g, crude) was obtained as a yellow solid.

### 5. General steps for preparation of 3-[1-(3-bromophenyl)cyclobutyl]-4-methyl-1,2,4-triazole

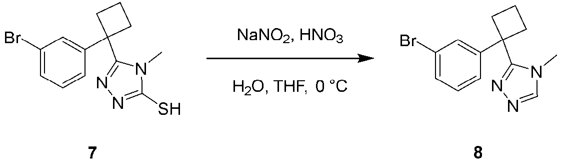

To a solution of 5-[1-(3-bromophenyl)cyclobutyl]-4-methyl-1,2,4-triazole-3-thiol (10.4 g, 32.1 mmol) in H₂O (100 mL), THF (100 mL) was added HNO₃ (32.2 g, 332 mmol, 23.0 mL, 65.0% purity), NaNO₂ (22.1 g, 321 mmol) under N₂ at 0 °C. The mixture was stirred under N₂ at 0 °C for 3 hrs. LCMS showed the reaction was completed. The mixture was quenched by saturated aqueous NaHCO₃ solution then extracted with EtOAc (100 mL * 3). The combined organic layers were dried over Na₂SO₄, filtered and concentrated under reduced pressure to give a residue. The residue was purified by column chromatography (SiO₂, DCM/ MeOH = 1/ 0 to 98/ 2). TLC (Plate 1, DCM/ MeOH = 10/ 1, I₂, R_{f} (product) = 0.4). Compound 3-[1-(3-bromophenyl)cyclobutyl]-4-methyl-1,2,4-triazole (3.90 g, 13.3 mmol, 41.5% yield) was obtained as a white solid. It was confirmed by H NMR.
¹H NMR: (400 MHz, DMSO-*d₆*)
*δ* 8.36 (s, 1H), 7.42-7.51 (m, 1H), 7.36 (t, *J* = 1.6 Hz, 1H), 7.33 (t, *J* = 7.6 Hz, 1H), 7.22-7.27 (m, 1H), 3.16 (s, 3H), 2.86-2.93 (m, 2H), 2.60-2.67 (m, 2H), 1.92-2.00 (m, 2H)

### 6. General steps for preparation of 2-[[(3S)-3-methyl-1-piperidyl]methyl]-6-[3-[1-(4-methyl-1,2,4-triazol-3-yl)cyclobutyl]phenyl]-1-(p-tolylsulfonyl)-4-(trifluoromethyl)pyrrolo[2,3-c]pyridin-7-one

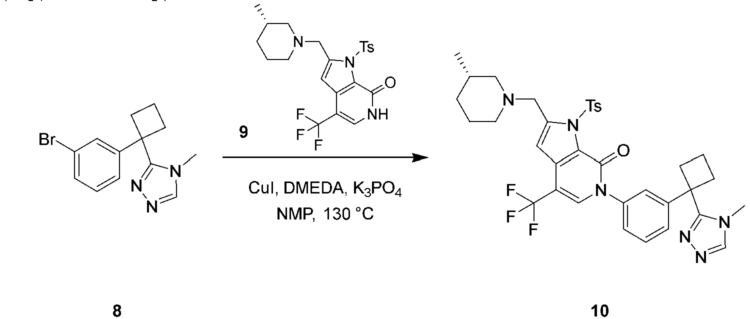

To a solution of 3-[1-(3-bromophenyl)cyclobutyl]-4-methyl-1,2,4-triazole (75.0 mg, 256 µmol), 2-[[(3S)-3-methyl-1-piperidyl]methyl]-1-(p-tolylsulfonyl)-4-(trifluoromethyl)-6H-pyrrolo[2,3-c]pyridin-7-one (100 mg, 213 µmol) in NMP (1.00 mL) was added CuI (122 mg, 641 µmol), K₃PO₄ (45.4 mg, 213 µmol), DMEDA (18.8 mg, 213 µmol, 23.0 µL). The mixture was stirred under N₂ at 130 °C for 2 hrs. LCMS showed the reaction was completed. The reaction mixture was diluted with EtOAc (10.0 mL). The mixture was filtered and the filter cake was washed with EtOAc (10.0 mL * 3). The filtrate was washed with brine (10.0 mL), dried over Na₂SO₄. The organic phase was concentrated in vacuum. Compound 2-[[(3S)-3-methyl-1-piperidyl]methyl]-6-[3-[1-(4-methyl-1,2,4-triazol-3-yl)cyclobutyl]phenyl]-1-(p-tolylsulfonyl)-4-(trifluoromethyl)pyrrolo[2,3-c]pyridin-7-one (50.0 mg, 73.6 µmol, 34.4% yield) was obtained as a green solid.

### 7. General steps for preparation of 2-[[(3S)-3-methyl-1-piperidyl]methyl]-6-[3-[1-(4-methyl-1,2,4-triazol-3-yl)cyclobutyl]phenyl]-4-(trifluoromethyl)-1H-pyrrolo[2,3-c]pyridin-7-one (compound T033)

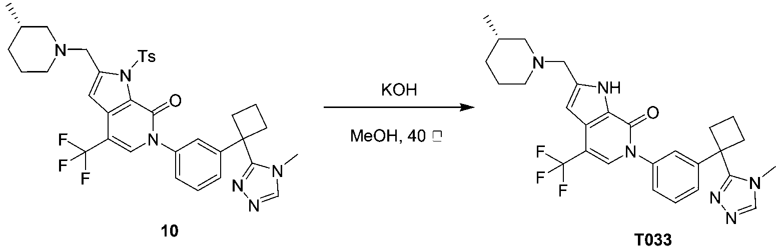

To a solution of 2-[[(3S)-3-methyl-1-piperidyl]methyl]-6-[3-[1-(4-methyl-1,2,4-triazol-3-yl)cyclobutyl]phenyl]-1-(p-tolylsulfonyl)-4-(trifluoromethyl)pyrrolo[2,3-c]pyridin-7-one (100 mg, 147 µmol) in MeOH (2.00 mL) was added KOH (165 mg, 2.95 mmol). The mixture was stirred under N₂ at 40 °C for 1 hr. LCMS showed the reaction was completed. The reaction mixture was diluted with EtOAc (10.0 mL). The mixture was filtered and the filter cake was washed with EtOAc (10.0 mL * 3). The filtrate was washed with brine (10.0 mL), dried over Na₂SO₄. The organic phase was concentrated in vacuum. The product was purified by Prep-HPLC (column: Xtimate C18 150 * 40 mm * 10 um; mobile phase: [water(NH₃H₂O⁺NH₄HCO₃)-ACN]; gradient: 30%-70% B over 32 min). Compound 2-[[(3S)-3-methyl-1-piperidyl]methyl]-6-[3-[1-(4-methyl-1,2,4-triazol-3-yl)cyclobutyl]phenyl]-4-(trifluoromethyl)-1H-pyrrolo[2,3-c]pyridin-7-one (26.8 mg, 50.9 µmol, 34.6% yield, 99.8% purity) was obtained as a green solid. It was confirmed by LCMS and H NMR.
¹H NMR: (400 MHz, CD₃CN)
*δ* 8.06 (s, 1H), 7.54 (d, *J* = 1.2 Hz, 1H), 7.46-7.52 (m, 1H), 7.39 (t, *J* = 2.0 Hz, 1H), 7.28-7.33 (m, 2H), 6.35 (s, 1H), 3.59 (s, 2H), 3.21 (s, 3H), 2.96-3.03 (m, 2H), 2.73-2.78 (m, 2H), 2.67-2.72 (m, 2H), 2.05-2.09 (m, 2H), 2.03 (br d, *J* = 1.2 Hz, 1H), 1.82-1.91 (m, 1H), 1.63-1.72 (m, 2H), 1.60 (br dd, *J* = 8.8, 3.6 Hz, 2H), 1.47-1.55 (m, 1H), 0.82 (d, *J* = 6.0 Hz, 3H)
LCMS: m/z = 525.0 (M+H)⁺, Rt = 1.012 min

### Example 34: Synthesis of compound T034

### 1. General steps for preparation of 2-(((2-cyclobutylethyl)amino)methyl)-6-(3-(1-(4-methyl-4H-1,2,4-triazol-3-yl)cyclobutyl)phenyl)-4-(trifluorometh yl)-1,6-dihydro-7H-pyrrolo[2,3-c]pyridin-7-one (compound T034)

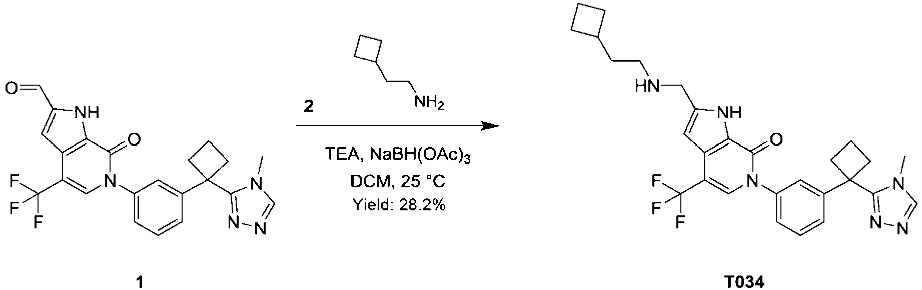

To a solution of 6-(3-(1-(4-methyl-4H-1,2,4-triazol-3-yl)cyclobutyl)phenyl)-7-oxo-4-(trifluoromethyl)-6,7-dihydro-1H-pyrrolo[2,3-c]pyridine-2-carbaldehyde (50.0 mg, 113 µmol) in dichloromethane (2.00 mL) was added dropwise TEA (11.4 mg, 113 µmol, 15.7 µL) adjust pH = 7, 2-cyclobutylethanamine (14.6 mg, 147 µmol) was added to the mixture, stirred at 25 °C for 0.5 hr, then NaBH(OAc)₃ (60.0 mg, 283 µmol) was added to the mixture, stirred at 25 °C for 1 hr. LCMS showed of desired compound was detected. The reaction mixture was diluted with aqueous NaHCO₃ (8.00 mL), extracted with dichloromethane (12.0 mL * 3), the combined organic layers were washed with H₂O (8.00 mL), dried over Na₂SO₄, filtered and concentrated under reduced pressure to give a residue. The residue was purified by Prep-HPLC (column: Welch Xtimate C18 40 * 200 mm 7um; mobile phase: [water (NH₄HCO₃)-ACN]; gradient:28.0%-68.0% B over 25 mins). Compound 2-(((2-cyclobutylethyl)amino)methyl)-6-(3-(1-(4-methyl-4H-1,2,4-triazol-3-yl)cyclobutyl)phenyl)-4-(trifluorometh yl)-1,6-dihydro-7H-pyrrolo[2,3-c]pyridin-7-one (16.8 mg, 32.0 µmol, 28.2% yield, 100% purity) was obtained as a colorless oil. Confirmed by H NMR and LCMS.
¹H NMR: (DMSO- *d*₆, 400 MHz)
*δ* 11.46-13.03 (m, 1H), 8.35 (s, 1H), 7.70 (s, 1H), 7.47-7.54 (m, 1H), 7.43 (s, 1H), 7.36 (d, *J* = 8.0 Hz, 1H), 7.26 (d, *J* = 8.0 Hz, 1H), 6.31 (s, 1H), 3.77 (s, 2H), 3.42-3.49 (m, 1H), 3.27 (s, 3H), 2.90-2.98 (m, 2H), 2.66-2.73 (m, 2H), 2.37 (t, *J* = 7.2 Hz, 2H), 2.25-2.32 (m, 1H), 1.93-2.04 (m, 4H), 1.70-1.83 (m, 2H), 1.46-1.59 (m, 4H)
LCMS: m/z = 525.3 (M+H)⁺, Rt = 1.593 min

### Example 35: Synthesis of compound T035

### 1. General steps for preparation of 6-(3-((1s,3s)-3-methyl-1-(4-methyl-4H-1,2,4-triazol-3-yl)cyclobutyl)phenyl)-2-(((1-methylcyclobutyl)amino)methy 1)-4-(trifluoromethyl)-1,6-dihydro-7H-pyrrolo[2,3-c]pyridin-7-one (compound T035)

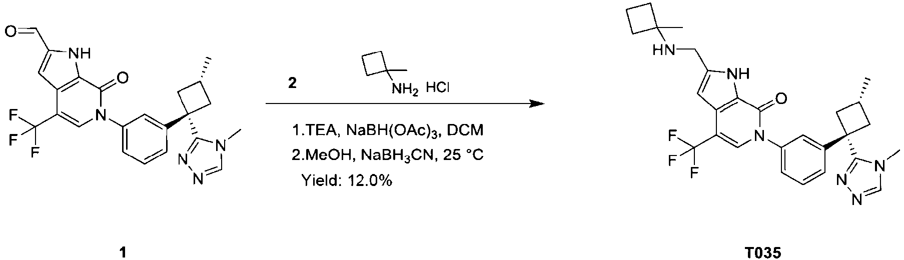

To a solution of 6-[3-[3-methyl-1-(4-methyl-1,2,4-triazol-3-yl)cyclobutyl]phenyl]-7-oxo-4-(trifluoromethyl)-1H-pyrrolo[2,3-c]pyrid ine-2-carbaldehyde (126 mg, 276 µmol) in dichloromethane (2.00 mL) was added dropwise TEA (28.0 mg, 276 µmol, 38.5 µL) adjust pH = 7, 1-methylcyclobutanamine (40.3 mg, 331 µmol, HCl) was added to the mixture, stirred at 25 °C for 0.5 hr, then NaBH(OAc)₃ (146 mg, 691 µmol) was added to the mixture, stirred at 25 °C for 1 hr. Methyl alcohol (1.00 mL) was added to the mixture follow by NaBH₃CN (17.3 mg, 276 µmol). The mixture was stirred at 25 °C for 2 hrs. LCMS showed desired compound was detected. The reaction mixture was diluted with aqueous NaHCO₃ (8.00 mL), extracted with dichloromethane (12.0 mL * 3), the combined organic layers were washed with H₂O (8.00 mL), dried over Na₂SO₄, filtered and concentrated under reduced pressure to give a residue. The residue was purified by Prep-HPLC (column: Welch Xtimate C18 40 * 200 mm 7um; mobile phase: [water (NH₄HCO₃)-ACN]; gradient:28.0%-68.0% B over 25 mins). Compound 6-(3-((1s,3s)-3-methyl-1-(4-methyl-4H-1,2,4-triazol-3-yl)cyclobutyl)phenyl)-2-(((1-methylcyclobutyl)amino)methy 1)-4-(trifluoromethyl)-1,6-dihydro-7H-pyrrolo[2,3-c]pyridin-7-one (17.6 mg, 33.3 µmol, 12.0% yield, 99.5% purity) was obtained as a white solid. Confirmed by H NMR and LCMS.
¹H NMR: (DMSO-*d*₆, 400 MHz)
*δ* 12.74 (s, 1H), 9.83 (s, 2H), 7.82 (d, *J* = 1.2 Hz, 1H), 7.55-7.66 (m, 2H), 7.47 (dd, *J* = 14.8, 8.0 Hz, 2H), 6.76 (s, 1H), 4.16-4.23 (m, 2H), 3.44 (s, 3H), 2.91-3.01 (m, 2H), 2.52-2.68 (m, 4H), 1.86 (d, *J* = 5.2 Hz, 4H), 1.55 (s, 3H), 1.23 (s, 1H), 1.09 (d, *J* = 5.6 Hz, 3H)
LCMS: m/z = 525.3 (M+H)⁺, Rt = 1.477 min

### Example 36: Synthesis of compound T036 &T037

### 1. General steps for preparation of ethyl 2-(3-methylcyclobutylidene)acetate

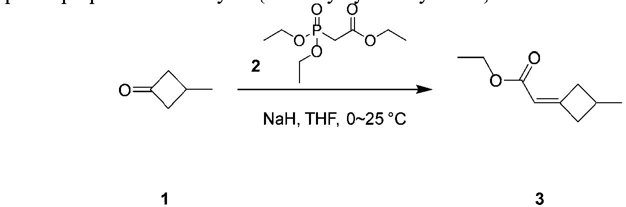

To a solution of NaH (1.43 g, 35.6 mmol, 60.0% purity) in THF (15.0 mL) was added ethyl 2-diethoxyphosphorylacetate (8.00 g, 35.6 mmol, 7.08 mL) in THF (25.0 mL) slowly at 0 °C. The mixture was stirred at 0 °C for 30 min. Then 3-methylcyclobutanone (2.00 g, 23.7 mmol) in THF (15.0 mL) was added dropwise to the mixture. The mixture was stirred at 25 °C for 2 hrs. LCMS showed desired compound was detected. The reaction mixture was quenched by add dropwise H₂O (30.0 mL) at 0 °C, extracted with EtOAc (50.0 mL * 3), the combined organic layers were washed with H₂O (20.0 mL * 3), dried over Na₂SO₄, filtered and concentrated under reduced pressure to give a residue. The residue was purified by column chromatography (SiO₂, PE/ EA = 0% to 4%), (Plate 1, PE/ EA = 20/ 1, R_{f} (product) = 0.34). Compound ethyl 2-(3-methylcyclobutylidene)acetate (1.98 g, 12.2 mmol, 51.5% yield, 95.4% purity) was obtained as a colorless oil. Confirmed by H NMRand LCMS.
¹H NMR: (DMSO-*d*₆, 400 MHz)
*δ* 5.60 (quin, *J* = 2.4 Hz, 1H), 4.04 (q, *J* = 7.2 Hz, 2H), 3.14-3.22 (m, 1H), 2.90-2.97 (m, 1H), 2.58 (br dd, *J* = 5.6, 3.2 Hz, 1H), 2.41-2.47 (m, 1H), 2.34-2.40 (m, 1H), 1.18 (t, *J* = 7.2 Hz, 3H), 1.13 (d, *J* = 6.4 Hz, 3H)

### 2. General steps for preparation of ethyl 2-[1-(3-bromophenyl)-3-methyl-cyclobutyl]acetate

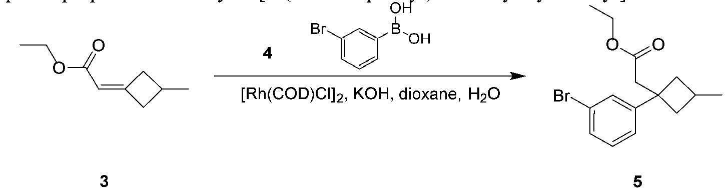

To a solution of chlororhodium;(1Z,5Z)-cycloocta-1,5-diene (31.9 mg, 64.8 µmol) in dioxane (3.50 mL) was added aqueous KOH (1.5 M, 864 µL) and the mixture was stirred for 15 min, then add dropwise a mixture of ethyl 2-(3-methylcyclobutylidene)acetate (200 mg, 1.30 mmol) and (3-bromophenyl)boronic acid (416 mg, 2.08 mmol) in dioxane (3.50 mL) to the mixture. The mixture was stirred at 25 °C for 15 min, then add (3-bromophenyl)boronic acid (130 mg, 648 µmol) in aqueous KOH (1.5 M, 285 µL) to the mixture, the mixture was stirred at 25 °C for 2 hrs. LCMS showed desired compound was detected. The reaction mixture was diluted with H₂O (30.0 mL), extracted with EtOAc (20.0 mL * 3), the combined organic layers were washed with H₂O (20.0 mL * 3), dried over Na₂SO₄, filtered and concentrated under reduced pressure to give a residue. The residue was purified by column chromatography (SiO₂, PE/ EA = 0% to 5%), (Plate 1, PE/ EA = 5/ 1, R_{f} (product) = 0.53). Compound ethyl 2-[1-(3-bromophenyl)-3-methyl-cyclobutyl]acetate (137 mg, 176 µmol, 13.5% yield, 40.0% purity) was obtained as a colorless oil.

### 3. General steps for preparation of 2-[1-(3-bromophenyl)-3-methyl-cyclobutyl]acetohydrazide

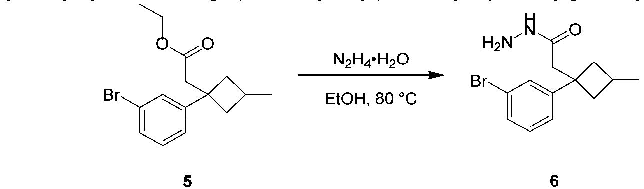

To a solution of ethyl 2-[1-(3-bromophenyl)-3-methyl-cyclobutyl]acetate (137 mg, 440 µmol) in EtOH (1.50 mL) was added dropwise N₂H₄.H₂O (337 mg, 6.60 mmol, 326 µL, 98.0% purity). After addition, the mixture was stirred at 80 °C for 12 hrs. The mixture was cooled to 25 °C, N₂H₄·H₂O (337 mg, 6.60 mmol, 326 µL, 98.0% purity) was added to the mixture slowly, the mixture was stirred at 80 °C for 12 hrs. LCMS showed desired compound was detected. The reaction mixture was diluted with H₂O (30.0 mL), extracted with EtOAc (20.0 mL * 3), the combined organic layers were washed with H₂O (20.0 mL * 3), dried over Na₂SO₄, filtered and concentrated under reduced pressure to give a residue. Compound 2-[1-(3-bromophenyl)-3-methyl-cyclobutyl]acetohydrazide (70.0 mg, crude) was obtained as a white solid. Confirmed by H NMR and LCMS.
¹H NMR: (DMSO-*d*₆, 400 MHz)
*δ* 8.19 (d, *J* = 8.4 Hz, 2H), 7.47 (d, *J* = 8.4 Hz, 2H), 6.83 (s, 1H), 4.03 (s, 2H), 2.40 (s, 3H), 1.87-2.19 (m, 4H), 0.92 (d, *J* = 6.4 Hz, 3H)

### 4. General steps for preparation of 1-[[2-[1-(3-bromophenyl)-3-methyl-cyclobutyl]acetyl]amino]-3-methyl-thiourea

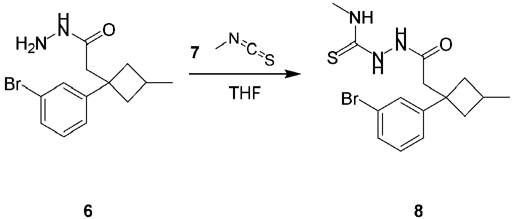

A mixture of 2-[1-(3-bromophenyl)-3-methyl-cyclobutyl]acetohydrazide (70.0 mg, 235 µmol), methylimino(thioxo)methane (34.4 mg, 471 µmol, 32.2 µL) in THF (1.00 mL) was degassed and purged with N₂ for 3 times, and then the mixture was stirred at 25 °C for 4 hrs under N₂ atmosphere. LCMS showed desired compound was detected. The solvent was removed under vacuum. Compound 1-[[2-[1-(3-bromophenyl)-3-methyl-cyclobutyl]acetyl]amino]-3-methyl-thiourea (62.0 mg, crude) was obtained as a yellow solid. Confirmed by H NMR.
¹H NMR: (DMSO-*d*₆, 400 MHz)
*δ* 9.53 (s, 1H), 9.09-9.13 (m, 1H), 7.31-7.44 (m, 2H), 7.20-7.26 (m, 2H), 7.10 (br d, *J* = 7.6 Hz, 1H), 2.85 (br d, *J* = 4.0 Hz, 2H), 2.80 (br d, *J* = 4.4 Hz, 3H), 2.65-2.69 (m, 1H), 1.71-1.83 (m, 4H), 0.99 (br d, *J* = 6.4 Hz, 3H)

### 5. General steps for preparation of 5-[[1-(3-bromophenyl)-3-methyl-cyclobutyl]methyl]-4-methyl-1,2,4-triazole-3-thiol:

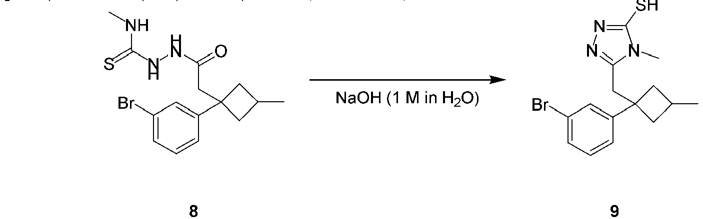

A mixture of 1-[[2-[1-(3-bromophenyl)-3-methyl-cyclobutyl]acetyl]amino]-3-methyl-thiourea (62.0 mg, 167 µmol), NaOH (1 M, 2.00 mL) in H₂O (2.00 mL) was degassed and purged with N₂ for 3 times, and then the mixture was stirred at 25 °C for 12 hrs under N₂ atmosphere. LCMS showed desired compound was detected. The reaction mixture was diluted with water, then the pH value of the solution was adjust to 1 with HCl (1 M, 12.0 mL) and extracted with EtOAc (40.0 mL * 3), the combined organic layers were washed with H₂O (50.0 mL * 3), dried over Na₂SO₄, filtered and concentrated under reduced pressure to give a residue. Compound 5-[[1-(3-bromophenyl)-3-methyl-cyclobutyl]methyl]-4-methyl-1,2,4-triazole-3-thiol (48.0 mg, crude) was obtained as a white solid. Confirmed by H NMR.
¹H NMR: (DMSO-*d*₆, 400 MHz)
*δ* 13.44 (br s, 1H), 7.18-7.28 (m, 2H), 7.14 (s, 1H), 6.91 (br d, *J* = 7.6 Hz, 1H), 3.16 (s, 2H), 2.68 (s, 3H), 1.23 (br s, 1H), 1.05-1.10 (m, 2H), 0.98-1.01 (m, 2H), 0.77-0.93 (m, 3H)

### 6 General steps for preparation of 3-[[1-(3-bromophenyl)-3-methyl-cyclobutyl]methyl]-4-methyl-1,2,4-triazole

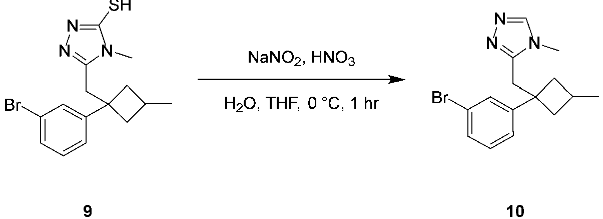

To a solution of 5-[[1-(3-bromophenyl)-3-methyl-cyclobutyl]methyl]-4-methyl-1,2,4-triazole-3-thiol (48.0 mg, 136 µmol) in THF (1.50 mL) and H₂O (1.50 mL) was added dropwise NaNO₂ (94.0 mg, 1.36 mmol) and HNO₃ (138 mg, 1.43 mmol, 27.0 µL, 65.0% purity) slowly at 0 °C, then the mixture was stirred at 0 °C for 1 hr. LCMS showed desired compound was detected. The mixture was basified by saturated aqueous sodium bicarbonate solution then extracted with EtOAc (20.0 mL * 3). The combined organic layers were dried over Na₂SO₄, filtered and concentrated under reduced pressure to give a residue. The residue was purified by column chromatography (SiO₂, DCM/ MeOH = 5/ 1), (Plate 1, DCM/ MeOH = 5/ 1, R_{f} (product) = 0.71). Compound 3-[[1-(3-bromophenyl)-3-methyl-cyclobutyl]methyl]-4-methyl-1,2,4-triazole (20.0 mg, 60.0 µmol, 44.0% yield, 96.1% purity) was obtained as a colorless oil. Confirmed by H NMR and LCMS.
¹H NMR: (CD₃OD, 400 MHz)
*δ* 8.20 (s, 1H), 7.33-7.41 (m, 1H), 7.17-7.27 (m, 1H), 6.95 (t, *J* = 1.6 Hz, 1H), 6.79 (dt, *J* = 7.6, 1.2 Hz, 1H), 2.83 (s, 3H), 2.72 (s, 2H), 2.34 (s, 1H), 2.02-2.04 (m, 2H), 1.84-1.94 (m, 2H), 1.10 (d, *J* = 6.4 Hz, 3H)
LCMS: m/z = 321.6 (M+H)⁺, Rt = 1.257 min

### 7. General steps for preparation of (S)-4-cyclopropyl-6-(3-(3-methyl-1-((4-methyl-4H-1,2,4-triazol-3-yl)methyl)cyclobutyl)phenyl)-2-((3-methylpiper idin-1-yl)methyl)-1-tosyl-1,6-dihydro-7H-pyrrolo[2,3-c]pyridin-7-one

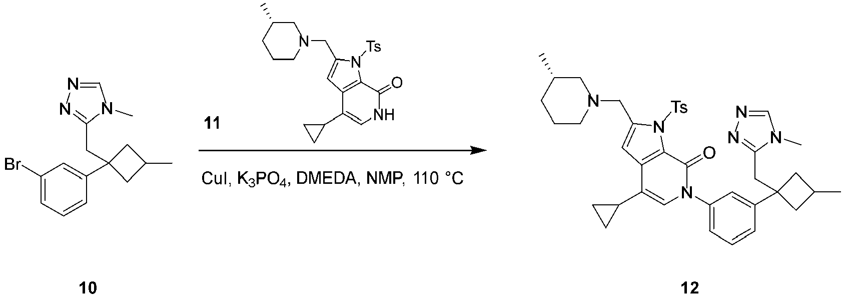

To a solution of 3-[[1-(3-bromophenyl)-3-methyl-cyclobutyl]methyl]-4-methyl-1,2,4-triazole (100 mg, 312 µmol), 4-cyclopropyl-2-[[(3S)-3-methyl-1-piperidyl]methyl]-1-(p-tolylsulfonyl)-6H-pyrrolo[2,3-c]pyridin-7-one (137 mg, 312 µmol), CuI (59.4 mg, 312 µmol), K₃PO₄ (198 mg, 936 µmol) in NMP (1.00 mL) was added DMEDA (55.0 mg, 624 µmol, 67.2 µL). The resulting mixture was stirred at 110 °C for 3 hrs. LCMS showed desired compound was detected. The reaction mixture was diluted with H₂O (30.0 mL), extracted with EtOAc (20.0 mL * 3), the combined organic layers were washed with H₂O (20.0 mL * 3), dried over Na₂SO₄, filtered and concentrated under reduced pressure to give a residue. The residue was purified by column chromatography (SiO₂, PE/ EA = 0% to 3%), (Plate 1, PE/ EA = 10/ 1, R_{f} (product) = 0.45). Compound 4-cyclopropyl-6-[3-[3-methyl-1-[(4-methyl-1,2,4-triazol-3-yl)methyl]cyclobutyl]phenyl]-2-[[(3S)-3-methyl-1-piper idyl]methyl]-1-(p-tolylsulfonyl)pyrrolo[2,3-c]pyridin-7-one (150 mg, 210 µmol, 67.3% yield, 95.2% purity) was obtained as a colorless oil. Confirmed by H NMR and LCMS.
¹H NMR: (CD₃OD, 400 MHz)
*δ* 8.30-8.39 (m, 2H), 7.83 (s, 1H), 7.31-7.43 (m, 3H), 7.07-7.14 (m, 1H), 6.97 (br d, *J* = 7.6 Hz, 1H), 6.76-6.84 (m, 2H), 6.38 (s, 1H), 3.97 (s, 2H), 3.24 (s, 1H), 3.15 (s, 1H), 2.87-2.96 (m, 2H), 2.80 (s, 1H), 2.77 (br d, *J =* 8.8 Hz, 1H), 2.73 (s, 2H), 2.63-2.72 (m, 2H), 2.40 (s, 3H), 2.17 (br d, *J* = 6.4 Hz, 1H), 1.99-2.07 (m, 1H), 1.82-1.95 (m, 3H), 1.55-1.81 (m, 6H), 1.17 (br d, *J* = 5.2 Hz, 1H), 1.10 (d, *J* = 6.0 Hz, 2H), 0.88 (br d, *J* = 6.0 Hz, 6H)
LCMS: m/z = 679.2 (M+H)⁺, Rt = 1.417 min

### 8. General steps for preparation of 4-cyclopropyl-6-(3-((1r,3S)-3-methyl-1-((4-methyl-4H-1,2,4-triazol-3-yl)methyl)cyclobutyl)phenyl)-2-(((S)-3-met hylpiperidin-1-yl)methyl)-1-tosyl-1,6-dihydro-7H-pyrrolo[2,3-c]pyridin-7-one and 4-cyclopropyl-6-(3-((1s,3R)-3-methyl-1-((4-methyl-4H-1,2,4-triazol-3-yl)methyl)cyclobutyl)phenyl)-2-(((S)-3-met hylpiperidin-1-yl)methyl)-1-tosyl-1,6-dihydro-7H-pyrrolo[2,3-c]pyridin-7-one

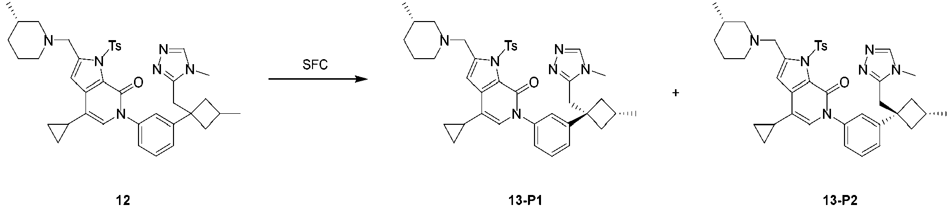

The residue was purified by SFC (column: DAICEL CHIRALPAK AS (250 mm * 30 mm, 10 um); mobile phase: [CO₂-EtOH (0.1% NH₃H₂O)]; B%: 35%, isocratic elution mode). Compound 4-cyclopropyl-6-(3-((1r,3S)-3-methyl-1-((4-methyl-4H-1,2,4-triazol-3-yl)methyl)cyclobutyl)phenyl)-2-(((S)-3-met hylpiperidin-1-yl)methyl)-1-tosyl-1,6-dihydro-7H-pyrrolo[2,3-c]pyridin-7-one (108 mg, 155 µmol, 70.3% yield, 97.7% purity) was obtained as a white solid. Confirmed by LCMS.
LCMS: m/z = 679.6 (M+H)⁺, Rt = 1.237 min

Compound 4-cyclopropyl-6-(3-((1s,3R)-3-methyl-1-((4-methyl-4H-1,2,4-triazol-3-yl)methyl)cyclobutyl)phenyl)-2-(((S)-3-met hylpiperidin-1-yl)methyl)-1-tosyl-1,6-dihydro-7H-pyrrolo[2,3-c]pyridin-7-one (53.0 mg, 72.1 µmol, 32.6% yield, 92.4% purity) was obtained as a white solid. Confirmed by LCMS.
LCMS: m/z = 679.5 (M+H)⁺, Rt = 1.227 min

### 9. General steps for preparation of 4-cyclopropyl-6-(3-((1r,3S)-3-methyl-1-((4-methyl-4H-1,2,4-triazol-3-yl)methyl)cyclobutyl)phenyl)-2-(((S)-3-met hylpiperidin-1-yl)methyl)-1,6-dihydro-7H-pyrrolo[2,3-c]pyridin-7-one (compound T036)

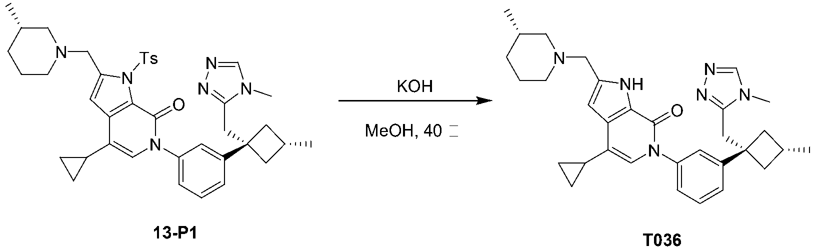

A mixture of 4-cyclopropyl-6-(3-((1r,3S)-3-methyl-1-((4-methyl-4H-1,2,4-triazol-3-yl)methyl)cyclobutyl)phenyl)-2-(((S)-3-met hylpiperidin-1-yl)methyl)-1-tosyl-1,6-dihydro-7H-pyrrolo[2,3-c]pyridin-7-one (108 mg, 159 µmol), KOH (178 mg, 3.18 mmol) in MeOH (4.00 mL) was degassed and purged with N₂ for 3 times, and then the mixture was stirred at 40 °C for 1 hr under N₂ atmosphere. LCMS showed desired compound was detected. Filtered and concentrated under reduced pressure to give a residue. The residue was purified by Prep-HPLC (column: Welch Xtimate C18 150 * 30 mm * 5 um; mobile phase: [water (HCl)-ACN]; gradient: 12% - 52% B over 25 min). Compound 4-cyclopropyl-6-(3-((1r,3S)-3-methyl-1-((4-methyl-4H-1,2,4-triazol-3-yl)methyl)cyclobutyl)phenyl)-2-(((S)-3-met hylpiperidin-1-yl)methyl)-1,6-dihydro-7H-pyrrolo[2,3-c]pyridin-7-one (70.5 mg, 134 µmol, 84.3% yield, 99.91% purity) was obtained as a yellow oil. Confirmed by H NMR and LCMS.
¹H NMR: (CD₃CN, 400 MHz)
*δ* 12.61 (br s, 1H), 8.81 (s, 1H), 7.49-7.55 (m, 1H), 7.29-7.32 (m, 1H), 7.24 (d, *J* = 7.6 Hz, 1H), 7.10 (d, *J* = 2.0 Hz, 1H), 7.07 (s, 1H), 6.87 (t, *J* = 1.6 Hz, 1H), 4.38-4.47 (m, 2H), 3.51 (s, 2H), 3.41 (br d, *J* = 12.0 Hz, 1H), 3.32 (br d, *J* = 12.0 Hz, 1H), 3.01 (s, 3H), 2.72-2.83 (m, 4H), 2.49-2.61 (m, 1H), 2.02-2.15 (m, 2H), 1.95-2.02 (m, 3H), 1.80-1.90 (m, 2H), 1.04-1.12 (m, 4H), 0.97-1.02 (m, 2H), 0.92 (d, J = 6.4 Hz, 3H), 0.76-0.81 (m, 2H)
LCMS: m/z = 525.3 (M+H)⁺, Rt = 1.907 min

### 10. General steps for preparation of 4-cyclopropyl-6-(3-((1s,3R)-3-methyl-1-((4-methyl-4H-1,2,4-triazol-3-yl)methyl)cyclobutyl)phenyl)-2-(((S)-3-met hylpiperidin-1-yl)methyl)-1,6-dihydro-7H-pyrrolo[2,3-c]pyridin-7-one (compound T037)

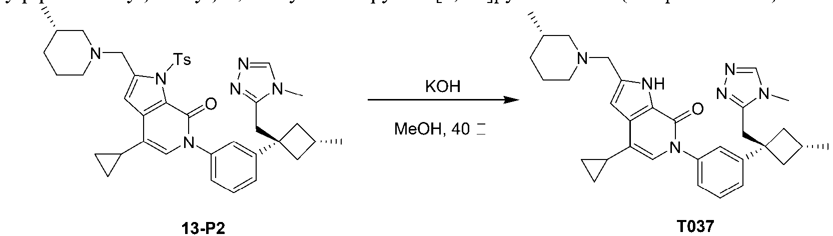

A mixture of 4-cyclopropyl-6-(3-((1s,3R)-3-methyl-1-((4-methyl-4H-1,2,4-triazol-3-yl)methyl)cyclobutyl)phenyl)-2-(((S)-3-met hylpiperidin-1-yl)methyl)-1-tosyl-1,6-dihydro-7H-pyrrolo[2,3-c]pyridin-7-one (53.0 mg, 78.0 µmol), KOH (87.6 mg, 1.56 mmol) in MeOH (2.00 mL) was degassed and purged with N₂ for 3 times, and then the mixture was stirred at 40 °C for 1 hr under N₂ atmosphere. LCMS showed desired compound was detected. Filtered and concentrated under reduced pressure to give a residue. The residue was purified by Prep-HPLC (column: Welch Xtimate C 18 150 * 30 mm * 5 um; mobile phase: [water (HCl)-ACN]; gradient: 12%-52% B over 25 min). Compound 4-cyclopropyl-6-(3-((1s,3R)-3-methyl-1-((4-methyl-4H-1,2,4-triazol-3-yl)methyl)cyclobutyl)phenyl)-2-(((S)-3-met hylpiperidin-1-yl)methyl)-1,6-dihydro-7H-pyrrolo[2,3-c]pyridin-7-one (24.9 mg, 47.3 µmol, 60.6% yield, 99.74% purity) was obtained as a colorless oil. Confirmed by H NMR and LCMS.
¹H NMR: (CD₃CN, 400 MHz)
*δ* 12.62 (br s, 1H), 8.67 (s, 1H), 7.51-7.57 (m, 1H), 7.43-7.48 (m, 1H), 7.32 (br d, *J* = 7.6 Hz, 1H), 7.26 (d, *J* = 1.6 Hz, 1H), 7.11 (s, 1H), 7.06 (s, 1H), 4.41 (br d, *J* = 3.2 Hz, 2H), 3.46 (s, 2H), 3.39 (br d, *J* = 12.8 Hz, 1H), 3.30 (br d, *J* = 10.4 Hz, 1H), 3.15 (s, 3H), 2.75-2.86 (m, 1H), 2.64-2.71 (m, 2H), 2.48-2.59 (m, 1H), 2.24-2.31 (m, 2H), 1.97-2.23 (m, 4H), 1.81-1.91 (m, 2H), 1.16 (d, *J* = 6.4 Hz, 3H), 1.04-1.13 (m, 1H), 0.95-1.01 (m, 2H), 0.92 (d, *J* = 6.4 Hz, 3H), 0.76-0.82 (m, 2H)
LCMS: m/z = 525.4 (M+H)⁺, Rt = 1.897 min

### Example 38: Synthesis of compound T038

### 1. General steps for preparation of 2-[[(3S)-3-hydroxy-1-piperidyl]methyl]-6-[3-[1-(4-methyl-1,2,4-triazol-3-yl)cyclobutyl]phenyl]-4-(trifluoromethyl )-1H-pyrrolo[2,3-c]pyridin-7-one (compound T038)

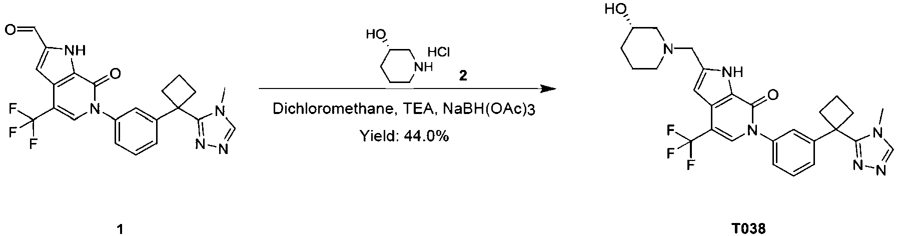

6-[3-[1-(4-methyl-1,2,4-triazol-3-yl)cyclobutyl]phenyl]-7-oxo-4-(trifluoromethyl)-1H-pyrrolo[2,3-c]pyridine-2-carbaldehyde (55.0 mg, 124 µmol) was dissolved in Dichloromethane (1.00 mL), pH was adjust to 7-8 with TEA. (3S)-piperidin-3-ol;hydrochloride (25.7 mg, 187 µmol) in Dichloromethane 1.00 mL was added to the mixture, stirred at 25 °C for 0.5 hr under N₂. Then NaBH(OAc)₃ (66.0 mg, 312 µmol) was added to the mixture, then stirred at 25 °C under N₂ for 12 hrs. LCMS shows reactant was consumed completely and desired mass was detected. The reaction mixture was diluted with Dichloromethane 6.00 mL, washed with sat. NaHCO₃ solution 2.00 mL. The water phase extracted with Dichloromethane 2.00 mL (1.00 mL * 2). The combined organic layers were washed, dried over Na₂SO₄, filtered and concentrated under reduced pressure to give a residue. The residue was purified by prep.HPLC (column: Welch Xtimate C18 40 * 200 mm 7 um;mobile phase: [water (HCl) - ACN];gradient: 0%-38% B over 30 min). 2-[[(3S)-3-hydroxy-1-piperidyl]methyl]-6-[3-[1-(4-methyl-1,2,4-triazol-3-yl)cyclobutyl]phenyl]-4-(trifluoromethyl )-1H-pyrrolo[2,3-c]pyridin-7-one (28.9 mg, 54.9 µmol, 44.0% yield, 100% purity) was obtained as white solid, confirmed by H NMR and LCMS.
¹H NMR: (400 MHz, DMSO-*d*₆)
*δ* 12.75-12.90 (m, 1H), 11.21-11.55 (m, 1H), 9.07-9.37 (m, 1H), 7.84 (s, 1H), 7.55-7.62 (m, 1H), 7.50 (br d, *J* = 12.0 Hz, 1H), 7.44 (br d, *J* = 9.2 Hz, 2H), 6.74 (br d, *J* = 15.6 Hz, 1H), 4.36-4.51 (m, 2H), 4.06 (br s, 1H), 3.41 (br s, 3H), 3.33 (br d, *J* = 14.4 Hz, 2H), 2.70-3.18 (m, 7H), 1.91-2.12 (m, 3H), 1.87 (br d, *J* = 16.4 Hz, 1H), 1.50-1.81 (m, 2H)
LCMS: m/z = 527.2 (M+H)⁺, Rt = 1.296 min

### Example 39: Synthesis of compound T039

### 1. General steps for preparation of 6-[3-[1-(4-methyl-1,2,4-triazol-3-yl)cyclobutyl]phenyl]-7-oxo-4-(trifluoromethyl)-1H-pyrrolo[2,3-c]pyridine-2-car baldehyde

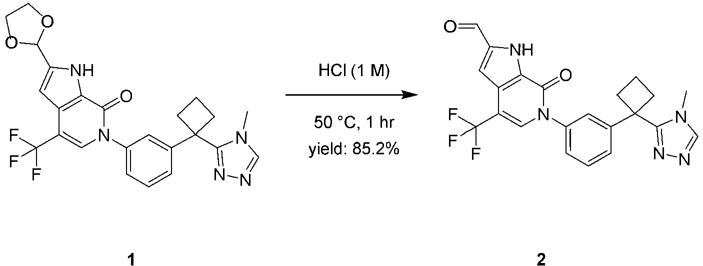

To a solution of 2-(1,3-dioxolan-2-yl)-6-[3-[1-(4-methyl-1,2,4-triazol-3-yl)cyclobutyl]phenyl]-4-(trifluoromethyl)-1H-pyrrolo[2,3-c ]pyridin-7-one (390 mg, 803 µmol) in HCl (1 M, 3.62 mL) was stirred at 50 °C for 1 hr. LCMS showed desired compound was detected. The reaction mixture was diluted with aqueous NaHCO₃ adjust pH = 9, extracted with ethyl acetate (12.0 mL * 3), the combined organic layers were washed with H₂O (8.00 mL), dried over Na₂SO₄, filtered and concentrated under reduced pressure to give a residue. Compound 6-[3-[1-(4-methyl-1,2,4-triazol-3-yl)cyclobutyl]phenyl]-7-oxo-4-(trifluoromethyl)-1H-pyrrolo[2,3-c]pyridine-2-car baldehyde (330 mg, crude) was obtained as a colorless oil. Confirmed by H NMR and LCMS.
¹H NMR: (DMSO-*d*₆, 400 MHz)
*δ* 13.64 (s, 1H), 9.91 (s, 1H), 8.35 (s, 1H), 7.87 (d, *J* = 1.2 Hz, 1H), 7.51-7.56 (m, 1H), 7.49 (s, 1H), 7.40 (d, *J* = 7.6 Hz, 1H), 7.27 (d, *J* = 8.0 Hz, 1H), 7.20 (s, 1H), 3.26 (s, 3H), 2.94 (dt, *J* = 5.6, 3.2 Hz, 2H), 2.67-2.72 (m, 2H), 2.02 (s, 1H), 1.94-1.98 (m, 1H)
LCMS: m/z =441.9 (M+H)⁺, Rt = 1.205 min

### 2. General steps for preparation of 2-[[(3R)-3-hydroxy-1-piperidyl]methyl]-6-[3-[1-(4-methyl-1,2,4-triazol-3-yl)cyclobutyl]phenyl]-4-(trifluoromethyl )-1H-pyrrolo[2,3-c]pyridin-7-one (compound T039)

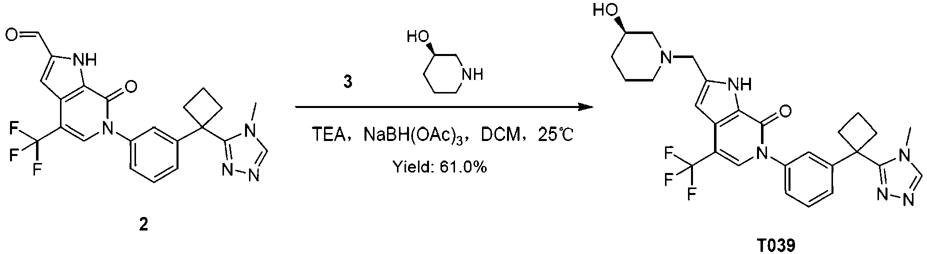

To a solution of 6-[3-[1-(4-methyl-1,2,4-triazol-3-yl)cyclobutyl]phenyl]-7-oxo-4-(trifluoromethyl)-1H-pyrrolo[2,3-c]pyridine-2-car baldehyde (50.0 mg, 113 µmol) in dichloromethane (2.50 mL) was added dropwise TEA (11.4 mg, 113 µmol, 15.7 µL) adjust pH = 9, (3R)-piperidin-3-ol (23.3 mg, 169 µmol, HCl) was added to the mixture, stirred at 25 °C for 0.5 hr, then NaBH(OAc)₃ (60.0 mg, 283 µmol) was added to the mixture, stirred at 25 °C for 1 hr. TEA (11.4 mg, 113 µmol, 15.7 µL) was added to the mixture adjust pH = 9, add (3R)-piperidin-3-ol (12.4 mg, 90.6 µmol, HCl) and stirred at 25 °C for 0.5 hr, NaBH(OAc)₃ (31.2 mg, 147 µmol) was added to the mixture, the mixture was stirred at 35 °C for 3 hrs under N₂. LCMS showed desired compound was detected. The reaction mixture was diluted with aqueous NaHCO₃ (8.00 mL), extracted with dichloromethane (12.0 mL * 3), the combined organic layers were washed with H₂O (8.00 mL), dried over Na₂SO₄, filtered and concentrated under reduced pressure to give a residue. The residue was purified by Prep-HPLC (column: Welch Xtimate C18 40*200 mm 7um; mobile phase: [water (HCl)-ACN]; gradient: 0%-38.0% B over 30 min). Compound 2-[[(3R)-3-hydroxy-1-piperidyl]methyl]-6-[3-[1-(4-methyl-1,2,4-triazol-3-yl)cyclobutyl]phenyl]-4-(trifluoromethyl )-1H-pyrrolo[2,3-c]pyridin-7-one (36.4 mg, 69.1 µmol, 99.1% purity, 61.0% yield) was obtained as a white solid. Confirmed by H NMR and LCMS.
¹H NMR: (DMSO-*d*₆, 400 MHz)
*δ* 12.81 (d, *J* = 13.6 Hz, 1H), 9.25 (d, *J* = 16.0 Hz, 1H), 7.84 (s, 1H), 7.55-7.61 (m, 1H), 7.50 (d, *J* = 12.4 Hz, 1H), 7.42 (t, *J* = 9.6 Hz, 2H), 6.74 (d, *J* = 14.8 Hz, 1H), 4.45 (dd, *J* = 3.6, 2.4 Hz, 2H), 4.29-4.41 (m, 1H), 4.05 (s, 1H), 3.41 (d, *J* = 2.0 Hz, 3H), 3.33 (d, *J* = 14.8 Hz, 2H), 2.91-3.05 (m, 3H), 2.66-2.83 (m, 3H), 1.98-2.07 (m, 2H), 1.83-1.93 (m, 1H), 1.68-1.82 (m, 1H), 1.47-1.67 (m, 1H), 1.11-1.36 (m, 1H)
LCMS: m/z = 527.2 (M+H)⁺, Rt = 1.243 min

### Example 40: Synthesis of compound T040

### 1. General steps for preparation of 2-[[methyl(2-methylbutyl)amino]methyl]-6-[3-[1-(4-methyl-1,2,4-triazol-3-yl)cyclobutyl]phenyl]-4-(trifluorometh yl)-1H-pyrrolo[2,3-c]pyridin-7-one (compound T040)

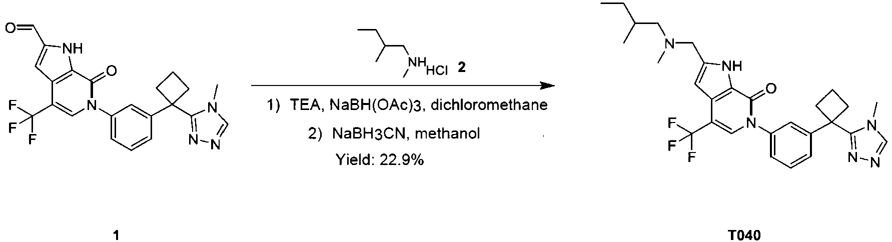

6-[3-[1-(4-methyl-1,2,4-triazol-3-yl)cyclobutyl]phenyl]-7-oxo-4-(trifluoromethyl)-1H-pyrrolo[2,3-c]pyridine-2-carbaldehyde (100 mg, 226 µmol) was dissolved in Dichloromethane 2.00 ml, pH was adjusted to 7-8 with TEA. N,2-dimethylbutan-1-amine (46.8 mg, 340 µmol, HCl) in Dichloromethane 2.00 ml was added to the mixture, stirred at 25 °C for 0.5 hrs. NaBH(OAc)₃ (120 mg, 566 µmol) was added to the mixture, stirred at 25 °C under N₂ for 3 hrs. N,2-dimethylbutan-1-amine (46.8 mg, 340 µmol, HCl) in Dichloromethane 2.00 ml was added to the mixture , stirred at 25 °C for 0.5 hrs. NaBH(OAc)₃ (120 mg, 566 µmol)was added to the mixture , stirred at 35 °C under N₂ for 3 hrs. NaBH(OAc)₃ (120 mg, 566 µmol) and Methanol (2.00 mL) was added to the mixture, the mixture was stirred at 35 °C for 12 hrs. NaBH₃CN (21.4 mg, 340 µmol) was added to the mixture, the mixture was stirred at 35 °C for 3 hrs. LCMS shows reactant was consumed completely and desired mass was detected. The reaction mixture was diluted with Dichloromethane 12.0 mL, washed with sat. NaHCO₃ solution 4.00 mL. The water phase extracted with Dichloromethane 12.0 mL (6.00 mL * 2). The combined organic layers were dried over Na₂SO₄, filtered and concentrated under reduced pressure to give a residue. The residue was purified by prep.HPLC (column: Welch Xtimate C18 40*200mm 7um;mobile phase: [water (FA) - ACN];gradient: 0%-40% B over 25 min).
2-[[methyl(2-methylbutyl)amino]methyl]-6-[3-[1-(4-methyl-1,2,4-triazol-3-yl)cyclobutyl]phenyl]-4-(trifluorometh yl)-1H-pyrrolo[2,3-c]pyridin-7-one (27.4 mg, 51.8 µmol, 22.9% yield, 99.6% purity) was obtained as white solid, confirmed by H NMR and LCMS.
¹H NMR: (400 MHz, DMSO-*d*₆)
*δ* 12.43 (br s, 1H), 8.12-8.66 (m, 1H), 7.71 (s, 1H), 7.47-7.54 (m, 1H), 7.44 (br s, 1H), 7.36 (d, *J* = 7.2 Hz, 1H), 7.25 (d, *J* = 7.6 Hz, 1H), 6.27 (br s, 1H), 3.62 (br s, 2H), 3.27 (s, 3H), 2.93 (br s, 2H), 2.65-2.74 (m, 2H), 2.19 (d, *J* = 7.2 Hz, 1H), 2.15 (s, 3H), 2.05-2.10 (m, 1H), 1.93-2.04 (m, 2H), 1.53-1.63 (m, 1H), 1.37-1.49 (m, 1H), 1.05 (dt, *J* = 13.6, 7.2 Hz, 1H), 0.78-0.91 (m, 6H)
LCMS: m/z = 527.3 (M+H)⁺, Rt = 1.517 min

### Example 41: Synthesis of compound T041

### 1. General steps for preparation of 2-[[ethyl(isobutyl)amino]methyl]-6-[3-[1-(4-methyl-1,2,4-triazol-3-yl)cyclobutyl]phenyl]-4-(trifluoromethyl)-1H-p yrrolo[2,3-c]pyridin-7-one (compound T041)

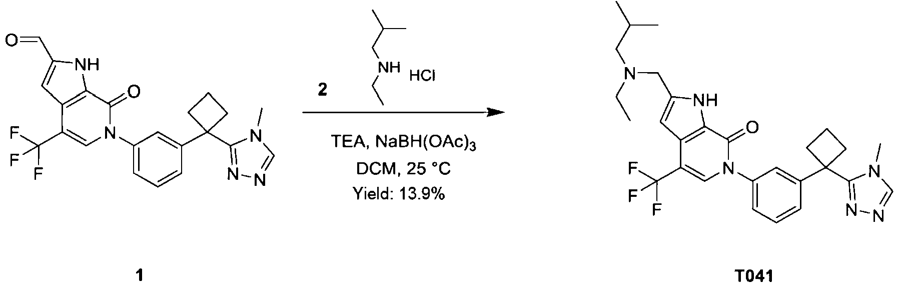

To a solution of6-[3-[1-(4-methyl-1,2,4-triazol-3-yl)cyclobutyl]phenyl]-7-oxo-4-(trifluoromethyl)-1H-pyrrolo[2,3-c]pyridine-2-c arbaldehyde (50.0 mg, 113 µmol) in dichloromethane (2.50 mL) was added dropwise TEA (11.4 mg, 113 µmol, 15.7 µL) adjust pH = 7, N-ethyl-2-methyl-propan-1-amine (18.7 mg, 135 µmol, HCl) was added to the mixture, stirred at 25 °C for 0.5 hr, then NaBH(OAc)₃ (60.0 mg, 283 µmol) was added to the mixture, stirred at 25 °C for 1 hr. LCMS showed desired compound was detected. The reaction mixture was diluted with aqueous NaHCO₃ (8.00 mL), extracted with dichloromethane (12.0 mL * 3), the combined organic layers were washed with H₂O (8.00 mL), dried over Na₂SO₄, filtered and concentrated under reduced pressure to give a residue. The residue was purified by Prep-HPLC (column: Welch Xtimate C18 40 * 200 mm 7 um; mobile phase: [water (NH₄HCO₃)-ACN]; gradient:40.0%-80.0% B over 25 mins). Compound 2-[[ethyl(isobutyl)amino]methyl]-6-[3-[1-(4-methyl-1,2,4-triazol-3-yl)cyclobutyl]phenyl]-4-(trifluoromethyl)-1H-p yrrolo[2,3-c]pyridin-7-one (8.44 mg, 15.8 µmol, 13.9% yield, 98.6% purity) was obtained as a white solid. Confirmed by H NMR and LCMS.
¹H NMR: (DMSO-*d*₆, 400 MHz)
*δ* 12.41 (s, 1H), 8.35 (s, 1H), 7.71 (s, 1H), 7.48-7.54 (m, 1H), 7.44 (s, 1H), 7.36 (d, *J* = 8.0 Hz, 1H), 7.25 (d,*J* = 7.6 Hz, 1H), 6.27 (s, 1H), 3.69 (s, 2H), 3.27 (s, 3H), 2.89-2.97 (m, 2H), 2.65-2.72 (m, 2H), 2.46 (d, *J* = 7.2 Hz, 2H), 2.14 (d, *J* = 7.2 Hz, 2H), 1.92-2.03 (m, 2H), 1.75 (dt, *J* = 13.2, 6.8 Hz, 1H), 0.99 (t, *J* = 7.2 Hz, 3H), 0.85 (d, *J* = 6.4 Hz, 6H)
LCMS: m/z = 527.3 (M+H)⁺, Rt = 1.497 min

### Example 42: Synthesis of compound T042

### 1. General steps for preparation of 2-[[isopropyl(propyl)amino]methyl]-6-[3-[1-(4-methyl-1,2,4-triazol-3-yl)cyclobutyl]phenyl]-4-(trifluoromethyl)-1 H-pyrrolo[2,3-c]pyridin-7-one (compound T042)

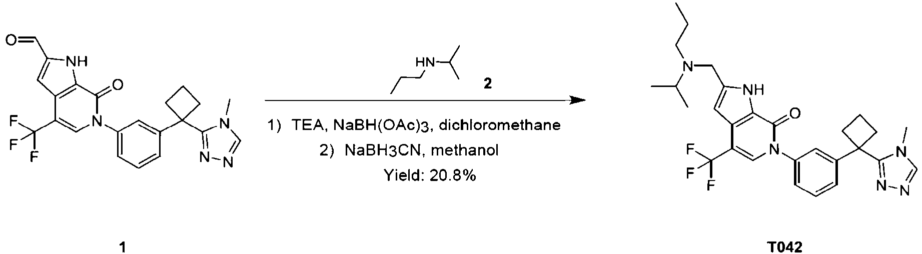

6-[3-[1-(4-methyl-1,2,4-triazol-3-yl)cyclobutyl]phenyl]-7-oxo-4-(trifluoromethyl)-1H-pyrrolo[2,3-c]pyridine-2-carbaldehyde (43.0 mg, 97.4 µmol) was dissolved in Dichloromethane (1.00 mL). pH was adjusted to 7-8 with TEA. N-isopropylpropan-1-amine (14.8 mg, 146.12 µmol) in Dichloromethane (1.00 mL) was added to the mixture, and stirred at 25 °C under N₂ for 0.5 hrs. NaBH(OAc)₃ (51.6 mg, 243 µmol) was added to the mixture the mixture was stirred at 25 °C for 12 hrs. N-isopropylpropan-1-amine (14.8 mg, 146.12 µmol) in Dichloromethane (1.00 mL) was added to the mixture, and stirred at 25 °C under N₂ for 0.5 hrs. NaBH(OAc)₃ (51.6 mg, 243 µmol) was added to the mixture ,the mixture was stirred at 35 °C for 12 hrs. Methanol (1.00 mL) and NaBH₃CN (6.12 mg, 97.4 µmol) was added to the mixture in order. The mixture was stirred at 35 °C for 2 hrs, byproduct was detected. NaBH(OAc)₃ (51.6 mg, 243 µmol) was added to the mixture ,the mixture was stirred at 35 °C for 12 hrs. LCMS shows reactant was consumed completely and desired mass was detected. The reaction mixture was diluted with Dichloromethane 6.00 mL, washed with sat. NaHCO₃ solution 2.00 mL. The water phase extracted with Dichloromethane 2.00 mL (1.00 mL * 2). The combined organic layers were washed, dried over Na₂SO₄, filtered and concentrated under reduced pressure to give a residue. The residue was purified by prep.HPLC (column: Welch Xtimate C18 40 * 200 mm 7 um;mobile phase: [water (FA) - ACN];gradient: 0%-40% B over 25 min). 2-[[isopropyl(propyl)amino]methyl]-6-[3-[1-(4-methyl-1,2,4-triazol-3-yl)cyclobutyl]phenyl]-4-(trifluoromethyl)-1 H-pyrrolo[2,3-c]pyridin-7-one (10.7 mg, 20.3 µmol, 20.8% yield, 99.8% purity was obtained as white solid, confirmed by H NMR and LCMS.
¹H NMR: (400 MHz, DMSO-*d*₆)
*δ* 12.82 (s, 1H), 10.61 (d, *J* = 0.8 Hz, 1H), 9.39 (br s, 1H), 7.83 (s, 1H), 7.52-7.61 (m, 2H), 7.36-7.48 (m, 2H), 6.83 (s, 1H), 4.49 (t, *J* = 6.4 Hz, 2H), 3.50 (br s, 1H), 3.42 (s, 3H), 3.09 (d, *J* = 11.6 Hz, 1H), 2.94-3.03 (m, 2H), 2.89 (dd, *J* = 12.0, 6.0 Hz, 1H), 2.73-2.82 (m, 2H), 1.95-2.11 (m, 2H), 1.55-1.80 (m, 2H), 1.32 (dd, *J* = 11.6, 6.4 Hz, 6H), 0.87 (t, *J* = 7.2 Hz, 3H)
LCMS: m/z = 527.3 (M+H)⁺, Rt = 1.478 min

### Example 43: Synthesis of compound T043

### 1. General steps for preparation of (S)-2-(((3,3-dimethylbutan-2-yl)amino)methyl)-6-(3-(1-(4-methyl-4H-1,2,4-triazol-3-yl)cyclobutyl)phenyl)-4-(trifl uoromethyl)-1,6-dihydro-7H-pyrrolo[2,3-c]pyridin-7-one (compound T043)

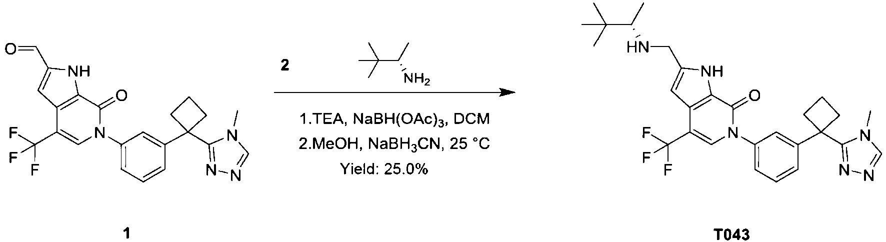

To a solution of 6-(3-(1-(4-methyl-4H-1,2,4-triazol-3-yl)cyclobutyl)phenyl)-7-oxo-4-(trifluoromethyl)-6,7-dihydro-1H-pyrrolo[2,3-c]pyridine-2-carbaldehyde (50.0 mg, 113 µmol) in dichloromethane (2.00 mL) was added dropwise TEA (11.4 mg, 113 µmol, 15.7 µL) adjust pH = 7, rac-(2S)-3,3-dimethylbutan-2-amine (14.9 mg, 147 µmol) was added to the mixture, stirred at 25 °C for 0.5 hr, then NaBH(OAc)₃ (60.0 mg, 283 µmol) was added to the mixture, stirred at 25 °C for 1 hr. Methyl alcohol (1.00 mL) was added to the mixture, followed by NaBH₃CN (7.12 mg, 113 µmol) add slowly, stirred for 1 hr. LCMS showed desired compound was detected. The reaction mixture was diluted with aqueous NaHCO₃ (8.00 mL), extracted with dichloromethane (12.0 mL * 3), the combined organic layers were washed with H₂O (8.00 mL), dried over Na₂SO₄, filtered and concentrated under reduced pressure to give a residue. The residue was purified by Prep-HPLC (column: Welch Xtimate C18 40 * 200 mm 7um; mobile phase: [water (NH₄HCO₃)-ACN]; gradient:32.0%-72.0% B over 25 mins). Compound (S)-2-(((3,3-dimethylbutan-2-yl)amino)methyl)-6-(3-(1-(4-methyl-4H-1,2,4-triazol-3-yl)cyclobutyl)phenyl)-4-(trifl uoromethyl)-1,6-dihydro-7H-pyrrolo[2,3-c]pyridin-7-one (15.0 mg, 28.4 µmol, 25.0% yield, 99.7% purity) was obtained as a white solid. Confirmed by H NMR and LCMS.
¹H NMR: (DMSO- *d*₆, 400 MHz)
*δ* 12.05-12.60 (m, 1H), 8.35 (s, 1H), 7.70 (s, 1H), 7.48-7.54 (m, 1H), 7.43 (s, 1H), 7.36 (d, *J* = 8.0 Hz, 1H), 7.25 (d, *J* = 7.6 Hz, 1H), 6.34 (s, 1H), 3.86-3.93 (m, 1H), 3.73-3.79 (m, 1H), 3.27 (s, 3H), 2.90-2.98 (m, 2H), 2.66-2.73 (m, 2H), 2.14 (q, *J* = 6.4 Hz, 1H), 1.95-2.04 (m, 2H), 0.90 (d, *J* = 6.4 Hz, 3H), 0.83 (s, 9H)
LCMS: m/z = 527.2 (M+H)⁺, Rt = 1.547 min

### Example 44: Synthesis of compound T044

### 1. General steps for preparation of 6-[3-[1-(4-methyl-1,2,4-triazol-3-yl)cyclobutyl]phenyl]-2-[[[rac-(1R)-1,2,2-trimethylpropyl]amino]methyl]-4-(trifl uoromethyl)-1H-pyrrolo[2,3-c]pyridin-7-one (compound T044)

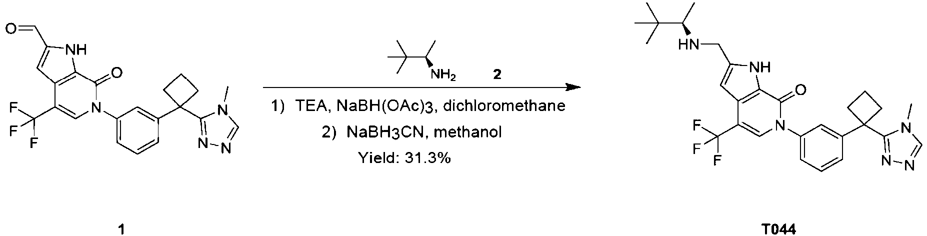

6-[3-[1-(4-methyl-1,2,4-triazol-3-yl)cyclobutyl]phenyl]-7-oxo-4-(trifluoromethyl)-1H-pyrrolo[2,3-c]pyridine-2-carbaldehyde (50.0 mg, 113 µmol) was dissolved in Dichloromethane 2.00 ml, pH was adjusted to 7-8 with TEA. rac-(2R)-3,3-dimethylbutan-2-amine (17.2 mg, 170 µmol, 22.8 µL) in Dichloromethane 2.00 ml was added to the mixture, stirred at 25 °C for0.5 hrs. NaBH(OAc)₃ (60.0 mg, 283 µmol) was added to the mixture , stirred at 25 °C under N₂ for 3 hrs. rac-(2R)-3,3-dimethylbutan-2-amine (17.2 mg, 170 µmol, 22.8 µL) in Dichloromethane 2.00 ml was added to the mixture, stirred at 25 °C for0.5 hrs. NaBH(OAc)₃ (60.0 mg, 283 µmol) was added to the mixture , stirred at 35 °C under N₂ for 3 hrs. NaBH(OAc)₃ (60.0 mg, 283 µmol) and Methanol (1.50 mL) was added to the mixture, the mixture was stirred at 35 °C for 12 hrs. NaBH₃CN (10.7 mg, 170 µmol) was added to the mixture, the mixture was stirred at 35 °C for 12 hrs. LCMS shows reactant was consumed completely and desired mass was detected. The reaction mixture was diluted with Dichloromethane 6.00 mL, washed with sat. NaHCO₃ solution 3.00 mL. The water phase extracted with Dichloromethane 12.0 mL (6.00 mL * 2) and Ethyl acetate 12.0 mL (6.0 mL * 2). The combined organic layers were dried over Na₂SO₄, filtered and concentrated under reduced pressure to give a residue. The residue was purified by prep.HPLC (column: Welch Xtimate C18 40 * 200 mm 7 um;mobile phase: [water (HCl) - ACN];gradient: 0% - 40% B over 25 min). 6-[3-[1-(4-methyl-1,2,4-triazol-3-yl)cyclobutyl]phenyl]-2-[[[rac-(1R)-1,2,2-trimethylpropyl]amino]methyl]-4-(trifl uoromethyl)-1H-pyrrolo[2,3-c]pyridin-7-one (18.7 mg, 35.5 µmol, 31.3% yield, 99.9% purity) was obtained as white solid, confirmed by H NMR and LCMS.
¹H NMR: (400 MHz, DMSO-*d*₆)
*δ* 12.80 (br s, 1H), 9.46-9.65 (m, 1H), 9.05-9.40 (m, 1H), 8.24-8.61 (m, 1H), 7.82 (d, *J* = 1.2 Hz, 1H), 7.48-7.61 (m, 2H), 7.42 (dd, *J* = 18.4, 7.6 Hz, 2H), 6.79 (br s, 1H), 4.47 (d, *J* = 14.0 Hz, 1H), 4.32 (d, *J* = 4.8 Hz, 1H), 3.42 (br s, 3H), 2.93-3.02 (m, 2H), 2.69-2.84 (m, 3H), 1.92-2.14 (m, 2H), 1.23 (d, *J* = 6.8 Hz, 3H), 0.93 (s, 9H)
LCMS: m/z = 527.3 (M+H)⁺, Rt = 1.537 min

### Example 45: Synthesis of compound T045

### 1. General steps for preparation of 2-(((2-cyclopropylethyl)amino)methyl)-6-(3-((1s,3s)-3-methyl-1-(4-methyl-4H-1,2,4-triazol-3-yl)cyclobutyl)pheny 1)-4-(trifluoromethyl)-1,6-dihydro-7H-pyrrolo[2,3-c]pyridin-7-one (compound T045)

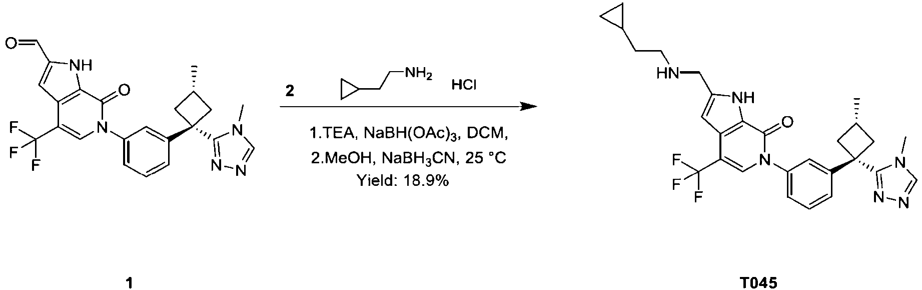

To a solution of 6-(3-((1s,3s)-3-methyl-1-(4-methyl-4H-1,2,4-triazol-3-yl)cyclobutyl)phenyl)-7-oxo-4-(trifluoromethyl)-6,7-dihydro -1H-pyrrolo[2,3-c]pyridine-2-carbaldehyde (50.0 mg, 109 µmol) in dichloromethane (1.00 mL) was added dropwise TEA (11.1 mg, 109 µmol, 15.2 µL) adjust pH = 7, 2-cyclopropylethanamine (20.0 mg, 164 µmol, HCl) was added to the mixture, stirred at 25 °C for 0.5 hr, then NaBH(OAc)₃ (58.1 mg, 274.46 µmol) was added to the mixture, stirred at 25 °C for 1 hr. Methyl alcohol (1.00 mL) and NaBH₃CN (6.90 mg, 109 µmol) was added to the mixture, stirred at 25 °C for 1hr. LCMS showed desired compound was detected. The reaction mixture was diluted with aqueous NaHCO₃ (8.00 mL), extracted with dichloromethane (12.0 mL * 3), the combined organic layers were washed with H₂O (8.00 mL), dried over Na₂SO₄, filtered and concentrated under reduced pressure to give a residue. The residue was purified by Prep-HPLC (column: Welch Xtimate C18 40 * 200 mm 7 um; mobile phase: [water (NH₄HCO₃)-ACN]; gradient:28.0%-68.0% B over 25 mins). Compound 2-(((2-cyclopropylethyl)amino)methyl)-6-(3-((1s,3s)-3-methyl-1-(4-methyl-4H-1,2,4-triazol-3-yl)cyclobutyl)pheny 1)-4-(trifluoromethyl)-1,6-dihydro-7H-pyrrolo[2,3-c]pyridin-7-one (11.0 mg, 20.8 µmol, 18.9% yield, 99.3% purity) was obtained as a colorless oil. Confirmed by H NMR and LCMS.
¹H NMR: (DMSO-*d*₆, 400 MHz)
*δ* 11.67-13.07 (m, 1H), 8.28 (s, 1H), 7.71 (d, *J* = 1.2 Hz, 1H), 7.52-7.54 (m, 1H), 7.48-7.52 (m, 1H), 7.33-7.38 (m, 2H), 6.33 (d, *J* = 0.8 Hz, 1H), 3.80 (s, 2H), 3.25 (s, 3H), 2.88 (d, *J* = 3.6 Hz, 2H), 2.51-2.57 (m, 6H), 1.28-1.34 (m, 2H), 1.07 (d, *J* = 5.2 Hz, 3H), 0.70 (dtd, *J* = 12.0, 7.6, 2.4 Hz, 1H), 0.35-0.39 (m, 2H), -0.02-0.02 (m, 2H)
LCMS: m/z = 525.3 (M+H)⁺, Rt = 1.610 min

### Examples 46 and 48: Synthesis of compounds T046& T048

### 1. General steps for preparation of 2-[(3-fluoro-1-piperidyl)methyl]-6-[3-[1-(4-methyl-1,2,4-triazol-3-yl)cyclobutyl]phenyl]-4-(trifluoromethyl)-1H-py rrolo[2,3-c]pyridin-7-one

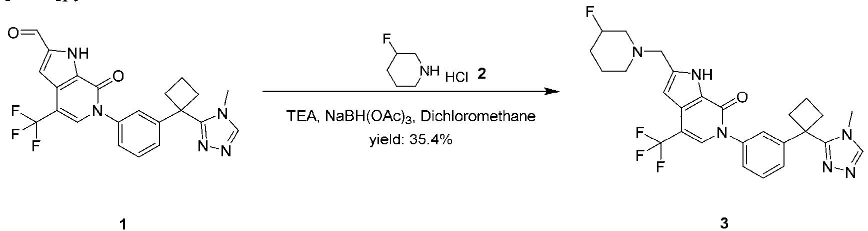

6-[3-[1-(4-methyl-1,2,4-triazol-3-yl)cyclobutyl]phenyl]-7-oxo-4-(trifluoromethyl)-1H-pyrrolo[2,3-c]pyridine-2-carbaldehyde (50.0 mg, 113 µmol) was dissolved in Dichloromethane (1.00 mL), pH was adjust to 7-8 with TEA. 3-fluoropiperidine;hydrochloride (23.7 mg, 170 µmol) in Dichloromethane 1.00 mL was added to the mixture, stirred at 25 °C for 0.5 hr under N₂. Then NaBH(OAc)₃ (60.0 mg, 283 µmol) was added to the mixture, then stirred at 25 °C under N₂ for 12 hrs. LCMS shows reactant was consumed completely and desired mass was detected. The reaction mixture was diluted with Dichloromethane 6.00 mL, washed with sat. NaHCO₃ solution 2.00 mL. The water phase extracted with Dichloromethane 2.00 mL (1.00 mL * 2). The combined organic layers were washed, dried over Na₂SO₄, filtered and concentrated under reduced pressure to give a residue. The residue was purified by prep.HPLC (column: Welch Xtimate C18 40 * 200 mm 7 um;mobile phase: [ water (HCl) - ACN];gradient: 0%-38% B over 30 min). 2-[(3-fluoro-1-piperidyl)methyl]-6-[3-[1-(4-methyl-1,2,4-triazol-3-yl)cyclobutyl]phenyl]-4-(trifluoromethyl)-1H-py rrolo[2,3-c]pyridin-7-one (21.2 mg, 40.1 µmol, 35.4% yield, 100% purity) was obtained as white solid, confirmed by H NMR and LCMS.
¹H NMR: (400 MHz, DMSO-*d*₆)
*δ* 12.85 (br s, 1H), 10.52-10.80 (m, 1H), 9.12-9.30 (m, 1H), 7.85 (d, *J* = 1.6 Hz, 1H), 7.54-7.68 (m, 1H), 7.49 (br s, 1H), 7.42 (br t, *J* = 7.6 Hz, 2H), 6.75 (br s, 1H), 4.91-5.24 (m, 1H), 4.41-4.62 (m, 2H), 3.40 (br d, *J* = 3.2 Hz, 3H), 3.23 (br s, 2H), 2.71-3.11 (m, 6H), 1.87-2.13 (m, 4H), 1.61-1.85 (m, 2H)
LCMS: m/z = 529.2 (M+H)⁺, Rt = 1.355 min

### 2. General steps for preparation of 2-[[(3R)-3-fluoro-1-piperidyl]methyl]-6-[3-[1-(4-methyl-1,2,4-triazol-3-yl)cyclobutyl]phenyl]-4-(trifluoromethyl)-1H-pyrrolo[2,3-c]pyridin-7-one (compound T046) and 2-[[(3S)-3-fluoro-1-piperidyl]methyl]-6-[3-[1-(4-methyl-1,2,4-triazol-3-yl)cyclobutyl]phenyl]-4-(trifluoromethyl)-1 H-pyrrolo[2,3-c]pyridin-7-one (compound T048)

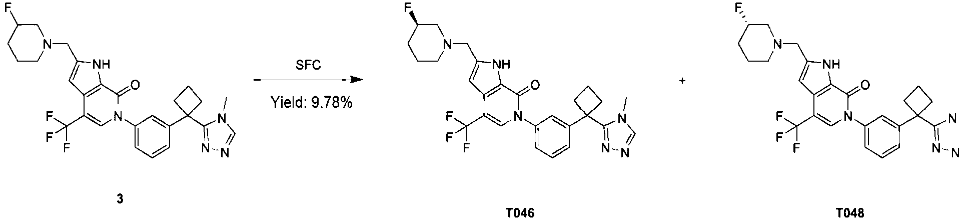

2-[(3-fluoro-1-piperidyl)methyl]-6-[3-[1-(4-methyl-1,2,4-triazol-3-yl)cyclobutyl]phenyl]-4-(trifluoromethyl)-1 H-pyrrolo[2,3-c]pyridin-7-one (21.2 mg, 40.1 µmol) was purified by the following method: column: DAICEL CHIRALPAK AD (250 mm * 30 mm,10 um);mobile phase: [CO2-i-PrOH (0.1%NH₃H₂O)];B%: 40%, isocratic elution mode. 2-[[(3R)-3-fluoro-1-piperidyl]methyl]-6-[3-[1-(4-methyl-1,2,4-triazol-3-yl)cyclobutyl]phenyl]-4-(trifluoromethyl)-1H-pyrrolo[2,3-c]pyridin-7-one (2.03 mg, 3.81 µmol, 9.49% yield, 99.1% purity) was obtained as white solid, confirmed by H NMR and LCMS.
¹H NMR: (400 MHz, DMSO-*d*₆)
*δ* 12.48 (s, 1H), 8.35 (s, 1H), 7.72 (d, *J* = 1.2 Hz, 1H), 7.47-7.55 (m, 1H), 7.44 (d, *J* = 1.6 Hz, 1H), 7.33-7.39 (m, 1H), 7.26 (d, *J* = 8.0 Hz, 1H), 6.30 (s, 1H), 4.53-4.73 (m, 1H), 3.69 (s, 2H), 3.27 (s, 3H), 2.89-3.00 (m, 2H), 2.65-2.75 (m, 3H), 2.35-2.47 (m, 2H), 2.23-2.33 (m, 1H), 1.90-2.06 (m, 2H), 1.64-1.85 (m, 2H), 1.47 (br d, *J* = 7.2 Hz, 2H)
LCMS: m/z = 529.2 (M+H)⁺, Rt = 1.354 min

2-[[(3S)-3-fluoro-1-piperidyl]methyl]-6-[3-[1-(4-methyl-1,2,4-triazol-3-yl)cyclobutyl]phenyl]-4-(trifluoromet hyl)-1H-pyrrolo[2,3-c]pyridin-7-one (2.10 mg, 3.92 µmol, 9.78% yield, 98.7% purity) was obtained as white solid, confirmed by H NMR and LCMS.
¹H NMR: (400 MHz, DMSO-*d*₆)
*δ* 12.49 (br s, 1H), 8.35 (s, 1H), 7.73 (s, 1H), 7.47-7.55 (m, 1H), 7.44 (s, 1H), 7.33-7.39 (m, 1H), 7.26 (br d, *J* = 8.0 Hz, 1H), 6.31 (br s, 1H), 4.53-4.78 (m, 1H), 3.70 (br s, 2H), 3.27 (s, 3H), 2.89-2.99 (m, 2H), 2.65-2.75 (m, 3H), 2.43 (br s, 2H), 2.23-2.33 (m, 1H), 1.92-2.03 (m, 2H), 1.65-1.84 (m, 2H), 1.47 (br s, 2H)
LCMS: m/z = 529.2 (M+H)⁺, Rt = 1.355 min

### Example 47: Synthesis of compound T047

### 1. General steps for preparation of 2-(((S)-3-fluoropyrrolidin-1-yl)methyl)-6-(3-((1s,3R)-3-methyl-1-(4-methyl-4H-1,2,4-triazol-3-yl)cyclobutyl)phen yl)-4-(trifluoromethyl)-1,6-dihydro-7H-pyrrolo[2,3-c]pyridin-7-one (compound T047)

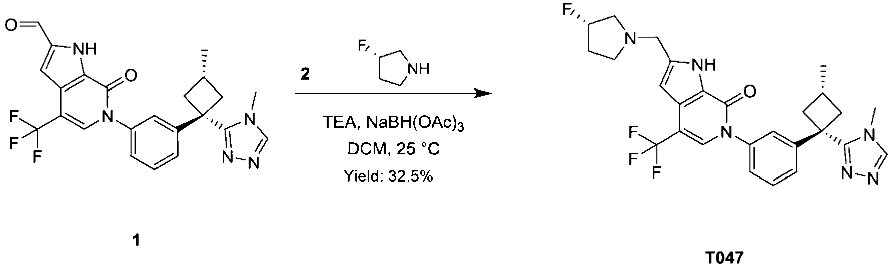

To a solution of 6-(3-((1s,3s)-3-methyl-1-(4-methyl-4H-1,2,4-triazol-3-yl)cyclobutyl)phenyl)-7-oxo-4-(triffuoromethyl)-6,7-dihydro -1H-pyrrolo[2,3-c]pyridine-2-carbaldehyde (50.0 mg, 109 µmol) in dichloromethane (2.00 mL) was added dropwise TEA (11.1 mg, 109 µmol, 15.2 µL) adjust pH = 7, rac-(3S)-3-fluoropyrrolidine (12.7 mg, 142 µmol) was added to the mixture, stirred at 25 °C for 0.5 hr, then NaBH(OAc)₃ (58.1 mg, 274 µmol) was added to the mixture, stirred at 25 °C for 1 hr. LCMS showed desired compound was detected. The reaction mixture was diluted with aqueous NaHCO₃ (8.00 mL), extracted with dichloromethane (12.0 mL * 3), the combined organic layers were washed with H₂O (8.00 mL), dried over Na₂SO₄, filtered and concentrated under reduced pressure to give a residue. The residue was purified by Prep-HPLC (column: Welch Xtimate C18 40 * 200 mm 7 um; mobile phase: [water (NH₄HCO₃)-ACN]; gradient:22.0%-62.0% B over 25 mins). Compound 2-(((S)-3-fluoropyrrolidin-1-yl)methyl)-6-(3-((1s,3R)-3-methyl-1-(4-methyl-4H-1,2,4-triazol-3-yl)cyclobutyl)phen yl)-4-(trifluoromethyl)-1,6-dihydro-7H-pyrrolo[2,3-c]pyridin-7-one (19.0 mg, 35.7 µmol, 32.5% yield, 99.5% purity) was obtained as a white solid. Confirmed by H NMR and LCMS.
¹H NMR: (DMSO-*d*₆, 400 MHz)
*δ* 12.50 (s, 1H), 8.25-8.35 (m, 1H), 7.72 (d, *J* = 1.2 Hz, 1H), 7.49-7.56 (m, 2H), 7.36 (d, *J* = 7.2 Hz, 2H), 6.31 (s, 1H), 5.08-5.29 (m, 1H), 3.76 (s, 2H), 3.25 (s, 3H), 2.88 (s, 3H), 2.80 (s, 2H), 2.61-2.74 (m, 3H), 2.38 (d, *J* = 6.4 Hz, 1H), 2.08-2.21 (m, 1H), 1.80-1.95 (m, 1H), 1.07 (s, 3H)
LCMS: m/z = 529.2 (M+H)⁺, Rt = 1.393 min

### Example 49: Synthesis of compound T049

### 1. General steps for preparation of 2-[[(3R,4S)-3,4-difluoropyrrolidin-1-yl]methyl]-6-[3-[1-(4-methyl-1,2,4-triazol-3-yl)cyclobutyl]phenyl]-4-(trifluor omethyl)-1H-pyrrolo[2,3-c]pyridin-7-one (compound T049)

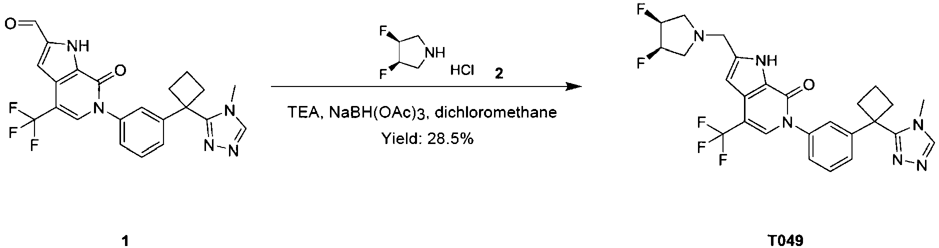

6-[3-[1-(4-methyl-1,2,4-triazol-3-yl)cyclobutyl]phenyl]-7-oxo-4-(trifluoromethyl)-1H-pyrrolo[2,3-c]pyridine-2-carbaldehyde (45.0 mg, 102 µmol) was dissolved in Dichloromethane (1.00 mL), pH was adjust to 7-8 with TEA. (3R,4S)-3,4-difluoropyrrolidine (22.0 mg, 153 µmol, HCl) in Dichloromethane 1.00 mL was added to the mixture, stirred at 25°C for 0.5 hr under N₂. Then NaBH(OAc)₃ (54.0 mg, 255 µmol) was added to the mixture, then stirred at 25 °C under N₂ for 12 hrs. LCMS shows reactant was consumed completely and desired mass was detected. The reaction mixture was diluted with Dichloromethane 6.00 mL, washed with sat. NaHCO₃ solution 2.00 mL. The water phase extracted with Dichloromethane 2.00 mL (1.00 mL * 2). The combined organic layers were washed, dried over Na₂SO₄, filtered and concentrated under reduced pressure to give a residue. The residue was purified by prep.HPLC (column: Welch Xtimate C18 40 * 200 mm 7 um;mobile phase: [ water (HCl) - ACN];gradient: 0%-40% B over 30 min). 2-[[(3R,4S)-3,4-difluoropyrrolidin-1-yl]methyl]-6-[3-[1-(4-methyl-1,2,4-triazol-3-yl)cyclobutyl]phenyl]-4-(trifluor omethyl)-1H-pyrrolo[2,3-c]pyridin-7-one (15.5 mg, 29.1 µmol, 28.5% yield, 99.9% purity) was obtained as white solid, confirmed by H NMR and LCMS.
¹H NMR: (400 MHz, DMSO-*d*₆)
*δ* 12.83 (br s, 1H), 9.41 (s, 1H), 7.83 (s, 1H), 7.55-7.61 (m, 1H), 7.53 (s, 1H), 7.43 (dd, *J* = 13.6, 8.0 Hz, 2H), 6.81 (br s, 1H), 5.31-5.60 (m, 2H), 4.58 (s, 2H), 3.62-3.78 (m, 4H), 3.43 (s, 3H), 2.93-3.06 (m, 2H), 2.70-2.86 (m, 2H), 1.93-2.15 (m, 2H)
LCMS: m/z = 533.2 (M+H)⁺, Rt = 1.420 min

### Example 50: Synthesis of compound T050

### 1. General steps for preparation of 2-[[(2,2-difluoro-3-hydroxy-propyl)amino]methyl]-6-[3-[1-(4-methyl-1,2,4-triazol-3-yl)cyclobutyl]phenyl]-4-(triflu oromethyl)-1H-pyrrolo[2,3-c]pyridin-7-one (compound T050)

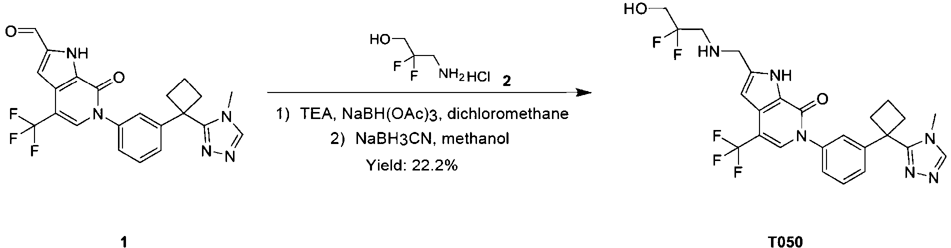

6-[3-[1-(4-methyl-1,2,4-triazol-3-yl)cyclobutyl]phenyl]-7-oxo-4-(trifluoromethyl)-1H-pyrrolo[2,3-c]pyridine-2-carbaldehyde (50.0 mg, 113 µmol) was dissolved in Dichloromethane 2.00 ml, pH was adjusted to 7-8 with TEA. amino-2,2-difluoro-propan-1-ol (25.1 mg, 170 µmol, HCl) in Dichloromethane 2.00 ml was added to the mixture, stirred at 25 °C for0.5 hrs. NaBH(OAc)₃ (60.0 mg, 283 µmol) was added to the mixture , stirred at 25 °C under N₂ for 3 hrs. amino-2,2-difluoro-propan-1-ol (25.1 mg, 170 µmol, HCl) in Dichloromethane 2.00 ml was added to the mixture, stirred at 25 °C for0.5 hrs. NaBH(OAc)₃ (60.0 mg, 283 µmol) was added to the mixture , stirred at 35 °C under N₂ for 3 hrs. NaBH(OAc)₃ (60.0 mg, 283 µmol) and Methanol (1.50 mL) was added to the mixture, the mixture was stirred at 35 °C for 12 hrs. NaBH₃CN (10.7 mg, 170 µmol) was added to the mixture, the mixture was stirred at 35 °C for 12 hrs. LCMS shows reactant was consumed completely and desired mass was detected. The reaction mixture was diluted with Dichloromethane 6.00 mL, washed with sat. NaHCO₃ solution 3.00 mL. The water phase extracted with Dichloromethane 12.0 mL (6.00 mL * 2) and Ethyl acetate 12.0 mL (6.0 mL * 2). The combined organic layers were dried over Na₂SO₄, filtered and concentrated under reduced pressure to give a residue. The residue was purified by prep.HPLC (column: Welch Xtimate C18 40 * 200mm 7 um;mobile phase: [water ( NH₄HCO₃) - ACN];gradient: 14% - 54% B over 25 min). 2-[[(2,2-difluoro-3-hydroxy-propyl)amino]methyl]-6-[3-[1-(4-methyl-1,2,4-triazol-3-yl)cyclobutyl]phenyl]-4-(triflu oromethyl)-1H-pyrrolo[2,3-c]pyridin-7-one (13.5 mg, 25.1 µmol, 22.2% yield, 99.9% purity) was obtained as white solid, confirmed by H NMR and LCMS.
¹H NMR: (400 MHz, DMSO-*d*₆)
*δ* 12.35 (br s, 1H), 8.35 (s, 1H), 7.72 (d, *J* = 0.8 Hz, 1H), 7.47-7.54 (m, 1H), 7.43 (s, 1H), 7.36 (d, *J* = 8.0 Hz, 1H), 7.26 (d, *J* = 8.0 Hz, 1H), 6.36 (s, 1H), 5.41 (t, *J* = 6.0 Hz, 1H), 3.87 (d, *J* = 6.0Hz, 2H), 3.64 (td, *J* = 13.5, 6.0 Hz, 2H), 3.27 (s, 3H), 2.81-3.00 (m, 4H), 2.66-2.74 (m, 2H), 2.54-2.61 (m, 1H), 1.91-2.06 (m, 2H)
LCMS: m/z = 537.2 (M+H)⁺, Rt = 1.248 min

### Example 51: Synthesis of compound T051

### 1. General steps for preparation of 2-(6-azaspiro[2.5]octan-6-ylmethyl)-6-[3-[1-(4-methyl-1,2,4-triazol-3-yl)cyclobutyl]phenyl]-4-(trifluoromethyl)-1 H-pyrrolo[2,3-c]pyridin-7-one (compound T051)

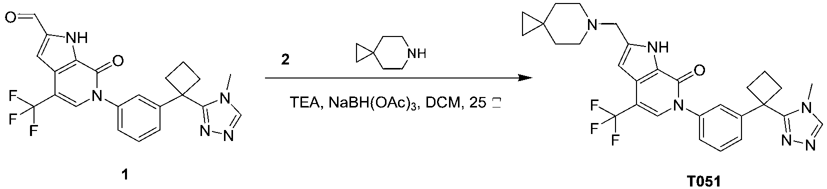

To a solution of6-[3-[1-(4-methyl-1,2,4-triazol-3-yl)cyclobutyl]phenyl]-7-oxo-4-(trifluoromethyl)-1H-pyrrolo[2,3-c]pyridine-2-c arbaldehyde (50.0 mg, 113 µmol) in dichloromethane (1.50 mL) was added dropwise TEA (11.4 mg, 113 µmol, 15.7 µL) adjust pH = 7, 6-azaspiro[2.5]octane (18.8 mg, 169 µmol) was added to the mixture, stirred at 25 °C for 0.5 hr, then NaBH(OAc)₃ (60.0 mg, 283 µmol) was added to the mixture, stirred at 25 °C for 1 hr. LCMS showed desired compound was detected. The reaction mixture was diluted with aqueous NaHCO₃ (8.00 mL), extracted with dichloromethane (12.0 mL * 3), the combined organic layers were washed with H₂O (8.00 mL), dried over Na₂SO₄, filtered and concentrated under reduced pressure to give a residue. The residue was purified by Prep-HPLC (column: Welch Xtimate C18 40*200 mm 7 um; mobile phase: [water (HCl)-ACN]; gradient: 2.00%-42.0% B over 30 mins). Compound 2-(6-azaspiro[2.5]octan-6-ylmethyl)-6-[3-[1-(4-methyl-1,2,4-triazol-3-yl)cyclobutyl]phenyl]-4-(trifluoromethyl)-1 H-pyrrolo[2,3-c]pyridin-7-one (15.3 mg, 28.4 µmol, 25.1% yield, 99.7% purity) was obtained as a white solid. Confirmed by H NMR and LCMS.
¹H NMR: EB6214-559-P1T2 (DMSO-*d*₆, 400 MHz)
*δ* 12.75-12.86 (m, 1H), 8.93-9.09 (m, 1H), 7.83 (s, 1H), 7.56 (t, *J* = 7.6 Hz, 1H), 7.47 (d, *J* = 1.6 Hz, 1H), 7.35-7.45 (m, 2H), 6.74 (s, 1H), 4.48 (s, 2H), 3.36 (d, *J* = 1.6 Hz, 3H), 2.90-3.02 (m, 4H), 2.70-2.78 (m, 2H), 2.53-2.56 (m, 2H), 2.14-2.25 (m, 2H), 1.96-2.08 (m, 2H), 1.13 (d, *J* = 13.6 Hz, 2H), 0.42 (d, *J* = 6.4 Hz, 2H), 0.35 (d, *J* = 5.2 Hz, 2H)
LCMS: m/z =537.2 (M+H)⁺, Rt = 1.497 min

### Example 52: Synthesis of compound T052

### 1. General steps for preparation of 2-(8-azabicyclo[3.2.1]octan-8-ylmethyl)-6-[3-[1-(4-methyl-1,2,4-triazol-3-yl)cyclobutyl]phenyl]-4-(trifluoromethyl )-1H-pyrrolo[2,3-c]pyridin-7-one (compound T052)

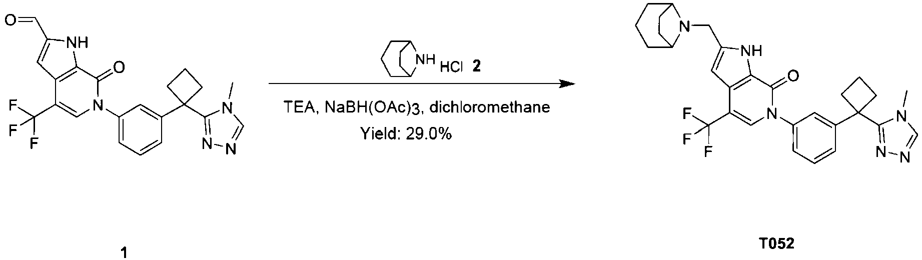

6-[3-[1-(4-methyl-1,2,4-triazol-3-yl)cyclobutyl]phenyl]-7-oxo-4-(trifluoromethyl)-1H-pyrrolo[2,3-c]pyridine-2-carbaldehyde (45.0 mg, 102 µmol)was dissolved in Dichloromethane 1.00 mL, pH was adjust to 7-8 with TEA. 8-azabicyclo[3.2.1]octane (22.6 mg, 153 µmol, HCl) in Dichloromethane 1.00 mL was added to the mixture, stirred at 25 °C for 0.5 hr under N₂. Then NaBH(OAc)₃ (54.0 mg, 255 µmol) was added to the mixture, then stirred at 25 °C under N₂ for 12 hrs. LCMS shows reactant was consumed completely and desired mass was detected. The reaction mixture was diluted with Dichloromethane 6.00 mL, washed with sat. NaHCO₃ solution 2.00 mL. The water phase extracted with Dichloromethane 2.00 mL (1.00 mL * 2). The combined organic layers were dried over Na₂SO₄, filtered and concentrated under reduced pressure to give a residue. The residue was purified by prep.HPLC (column: Welch Xtimate C18 40 * 200 mm 7 um;mobile phase: [water (HCl) - ACN];gradient: 0%-40% B over 30 min).
2-(8-azabicyclo[3.2.1]octan-8-ylmethyl)-6-[3-[1-(4-methyl-1,2,4-triazol-3-yl)cyclobutyl]phenyl]-4-(trifluoromethyl )-1H-pyrrolo[2,3-c]pyridin-7-one (15.9 mg, 29.6 µmol, 29.0% yield, 99.9% purity) was obtained as white solid, confirmed by H NMR and LCMS.
¹H NMR: (400 MHz, DMSO-*d*₆)
*δ* 12.78 (br s, 1H), 10.89-11.14 (m, 1H), 9.28-9.52 (m, 1H), 7.83 (d, *J* = 1.2 Hz, 1H), 7.51-7.61 (m, 2H), 7.42 (br dd, *J* = 14.0, 8.0 Hz, 2H), 6.90 (br s, 1H), 4.34 (br d, *J* = 5.6 Hz, 2H), 3.74 (br s, 2H), 3.40-3.46 (m, 3H), 2.93-3.04 (m, 2H), 2.71-2.83 (m, 2H), 2.24-2.36 (m, 2H), 1.97-2.20 (m, 4H), 1.91 (br d, *J* = 8.8 Hz, 2H), 1.46-1.74 (m, 4H)
LCMS: m/z = 537.2 (M+H)⁺, Rt = 1.405 min

### Example 53: Synthesis of compound T053

### 1. General steps for preparation of 2-(3-azabicyclo[3.2.1]octan-3-ylmethyl)-6-[3-[1-(4-methyl-1,2,4-triazol-3-yl)cyclobutyl]phenyl]-4-(trifluoromethyl )-1H-pyrrolo[2,3-c]pyridin-7-one (compound T053)

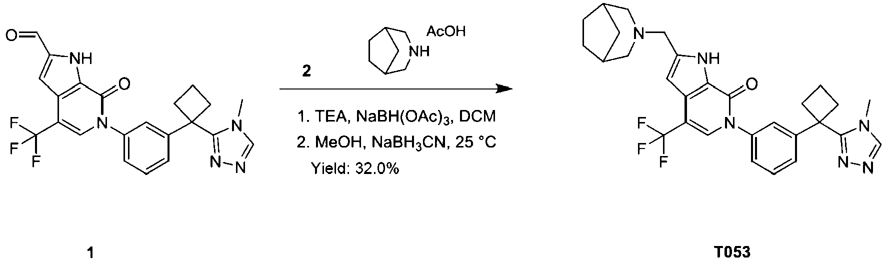

To a solution of6-[3-[1-(4-methyl-1,2,4-triazol-3-yl)cyclobutyl]phenyl]-7-oxo-4-(trifluoromethyl)-1H-pyrrolo[2,3-c]pyridine-2-c arbaldehyde (50.0 mg, 113 µmol) in dichloromethane (2.50 mL) was added dropwise TEA (11.4 mg, 113 µmol, 15.7 µL) adjust pH = 7, 3-azabicyclo[3.2.1]octane (29.1 mg, 169 µmol, AcOH) was added to the mixture, stirred at 25 °C for 0.5 hr, then NaBH(OAc)₃ (60.0 mg, 283 µmol) was added to the mixture, stirred at 25 °C for 1 hr. MeOH (1.00 mL) was added to the mixture, follow by NaBH₃CN (7.12 mg, 113 µmol) was added to the mixture, stirred at 25 °C for 1 hr. LCMS showed desired compound was detected. The reaction mixture was diluted with aqueous NaHCO₃ (8.00 mL), extracted with dichloromethane (12.0 mL * 3), the combined organic layers were washed with H₂O (8.00 mL), dried over Na₂SO₄, filtered and concentrated under reduced pressure to give a residue. The residue was purified by prep-HPLC (column: Welch Xtimate C18 40 * 200 mm 7 um; mobile phase: [water (HCl)-ACN]; gradient:2.00%-42.0% B over 20.5 mins). Compound 2-(3-azabicyclo[3.2.1]octan-3-ylmethyl)-6-[3-[1-(4-methyl-1,2,4-triazol-3-yl)cyclobutyl]phenyl]-4-(trifluoromethyl )-1H-pyrrolo[2,3-c]pyridin-7-one (19.7 mg, 36.3 µmol, 32.0% yield, 99.0% purity) was obtained as a colorless oil. Confirmed by H NMR and LCMS.
¹H NMR: (DMSO-*d*₆, 400 MHz)
*δ* 12.77 (s, 1H), 9.33 (s, 1H), 7.82 (d, *J* = 1.2 Hz, 1H), 7.55-7.60 (m, 1H), 7.52 (s, 1H), 7.41 (dd, *J* = 14.8, 8.0 Hz, 2H), 6.69 (s, 1H), 4.41 (d, *J* = 4.0 Hz, 2H), 3.41 (s, 3H), 3.07-3.13 (m, 2H), 3.04 (d, *J* = 10.0 Hz, 2H), 2.94-3.00 (m, 2H), 2.73-2.81 (m, 2H), 2.37 (s, 2H), 1.98-2.09 (m, 2H), 1.95 (d, *J* = 8.0 Hz, 2H), 1.65-1.75 (m, 2H), 1.52-1.59 (m, 1H), 1.41-1.49 (m, 1H)
LCMS: m/z =537.2 (M+H)⁺, Rt = 1.450 min

### Example 54: Synthesis of compound T054

### 1. General steps for preparation of 2-(5-azaspiro[2.5]octan-5-ylmethyl)-6-[3-[1-(4-methyl-1,2,4-triazol-3-yl)cyclobutyl]phenyl]-4-(trifluoromethyl)-1 H-pyrrolo[2,3-c]pyridin-7-one (compound T054)

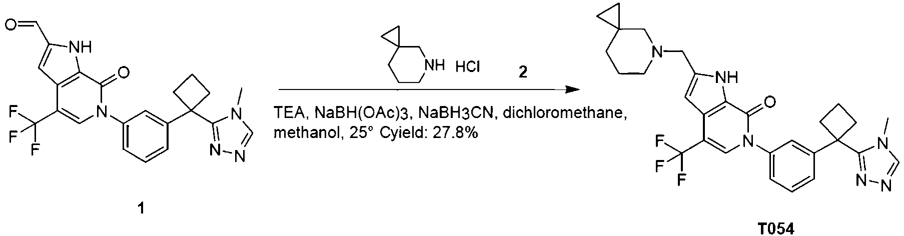

To a mixture of 5-azaspiro[2.5]octane (25.1 mg, 170 µmol, HCl) in Dichloromethane (3.00 mL) was added TEA (45.9 mg, 453 µmol, 63.1 µL) adjust pH = 8. Then 6-[3-[1-(4-methyl-1,2,4-triazol-3-yl)cyclobutyl]phenyl]-7-oxo-4-(trifluoromethyl)-1H-pyrrolo[2,3-c]pyridine-2-car baldehyde (50.0 mg, 113 µmol) was added to the mixture. The mixture was stirred under N₂ at 25 °C for 0.5 hr. NaBH(OAc)₃ (60.0 mg, 283 µmol) was added to the mixture. The mixture was stirred under N₂ at 25 °C for 2.5 hrs. Then Methanol (1.00 mL) and NaBH₃CN (7.12 mg, 113 µmol) was added to the mixture. The mixture was stirred under N₂ at 25 °C for 2 hrs. LCMS showed desired mass was detected. The reaction mixture was diluted with NaHCO₃ (3.00 mL), extracted with Dichloromethane (2.00 mL*2). The organic phase dried over Na₂SO₄ and concentrated in vacuum. The crude product was purified by Prep-HPLC (column: Welch Xtimate C18 40*200 mm 7 um; mobile phase: [water(FA)-ACN]; gradient: 0%-38.0% B over 25 min). To afford 2-(5-azaspiro[2.5]octan-5-ylmethyl)-6-[3-[1-(4-methyl-1,2,4-triazol-3-yl)cyclobutyl]phenyl]-4-(trifluoromethyl)-1 H-pyrrolo[2,3-c]pyridin-7-one (18.4 mg, 31.5 µmol, 27.8% yield, 100% purity, FA) as a white solid which was confirmed by H NMR and LCMS.
¹H NMR: (400 MHz, DMSO-*d*₆)
*δ* 12.39 (s, 1H), 8.35 (s, 1H), 7.70 (s, 1H), 7.46-7.56 (m, 1H), 7.42 (s, 1H), 7.35 (d, *J* = 8.0 Hz, 1H), 7.26 (d,*J* = 8.0 Hz, 1H), 6.27 (s, 1H), 3.60 (s, 2H), 3.26 (s, 3H), 2.90-2.97 (m, 2H), 2.66-2.73 (m, 2H), 2.43 (s, 2H), 2.14 (s, 2H), 1.93-2.03 (m, 2H), 1.55-1.62 (m, 2H), 1.23 (d, *J* = 5.2 Hz, 2H), 0.25 (s, 4H)
LCMS: m/z = 537.2 (M+H)⁺, Rt = 2.248 min

### Examples 55 and 56: Synthesis of compounds T055& T056

### 1. General steps for preparation of 2-((2-azabicyclo[4.1.0]heptan-2-yl)methyl)-6-(3-((1s,3s)-3-methyl-1-(4-methyl-4H-1,2,4-triazol-3-yl)cyclobutyl)ph enyl)-4-(trifluoromethyl)-1,6-dihydro-7H-pyrrolo[2,3-c]pyridin-7-one

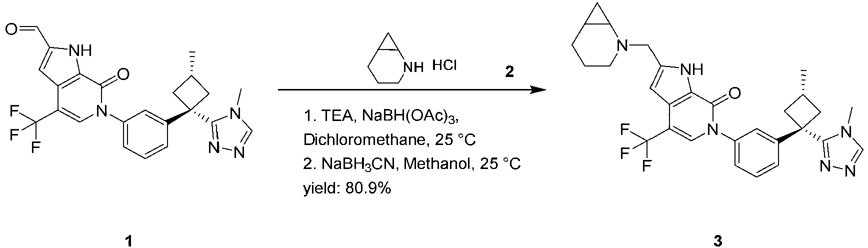

To a mixture of 2-azabicyclo[4.1.0]heptane (44.0 mg, 329 µmol, HCl) in Dichloromethane (6.00 mL) was added TEA (134 mg, 1.32 mmol) adjust pH = 8. Then 6-[3-[3-methyl-1-(4-methyl-1,2,4-triazol-3-yl)cyclobutyl]phenyl]-7-oxo-4-(trifluoromethyl)-1H-pyrrolo[2,3-c]pyrid ine-2-carbaldehyde (100 mg, 219 µmol) was added to the mixture. The mixture was stirred under N₂ at 25 °C for 0.5 hr. NaBH(OAc)₃ (117 mg, 552 µmol) was added to the mixture. The mixture was stirred under N₂ at 25 °C for 2.5 hrs. Then Methanol (1.00 mL) and NaBH₃CN (13.8 mg, 219 µmol) was added to the mixture. The mixture was stirred under N₂ at 25 °C for 4 hrs. LCMS showed desired mass was detected. The reaction mixture was diluted with NaHCO₃ (3.00 mL), extracted with Dichloromethane (2.00 mL*2). The organic phase dried over Na₂SO₄ and concentrated in vacuum. The crude product was purified by Prep-HPLC (column: Welch Xtimate C18 40*200 mm 7 um; mobile phase: [water (NH₃H₂O+NH₄HCO₃)-ACN]; gradient: 34.0%-74.0% B over 25 mins). To afford 2-((2-azabicyclo[4.1.0]heptan-2-yl)methyl)-6-(3-((1s,3s)-3-methyl-1-(4-methyl-4H-1,2,4-triazol-3-yl)cyclobutyl)ph enyl)-4-(trifluoromethyl)-1,6-dihydro-7H-pyrrolo[2,3-c]pyridin-7-one (100 mg, 177 µmol, 80.9% yield, 95.4% purity) as an off-white solid which was confirmed by LCMS.
LCMS: m/z = 537.2 (M+H)⁺, Rt = 1.082 min

### 2. General steps for preparation of 2-(((1R,6S)-2-azabicyclo[4.1.0]heptan-2-yl)methyl)-6-(3-((1s,3S)-3-methyl-1-(4-methyl-4H-1,24-triazol-3-yl)cycl obutyl)phenyl)-4-(trifluoromethyl)-1,6-dihydro-7H-pyrrolo[2,3-c]pyridin-7-one (compound T055) and 2-(((1S,6R)-2-azabicyclo[4.1.0]heptan-2-yl)methyl)-6-(3-((1s,3R)-3-methyl-1-(4-methyl-4H-1,2,4-triazol-3-yl)cycl obutyl)phenyl)-4-(trifluoromethyl)-1,6-dihydro-7H-pyrrolo[2,3-c]pyridin-7-one (compound T056)

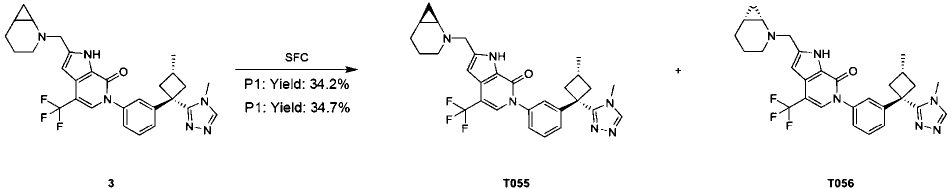

SFC (EB6211-833-P1A) showed two peaks. The crude product was purified by SFC (column: DAICEL CHIRALPAK AD (250 mm*30 mm, 10 um); mobile phase: [CO₂-i-PrOH (0.10% NH₃H₂O)]; B%: 40.0%, isocratic elution mode). To afford 2-(((1R,6S)-2-azabicyclo[4.1.0]heptan-2-yl)methyl)-6-(3-((1s,3S)-3-methyl-1-(4-methyl-4H-1,2,4-triazol-3-yl)cycl obutyl)phenyl)-4-(trifluoromethyl)-1,6-dihydro-7H-pyrrolo[2,3-c]pyridin-7-one (34.2 mg, 63.7 µmol, 34.2% yield, 100% purity) as a white solid which was confirmed by H NMR and LCMS.
¹H NMR: (400 MHz, DMSO-*d*₆)
*δ* 12.46 (s, 1H), 8.28 (s, 1H), 7.71 (s, 1H), 7.48-7.55 (m, 2H), 7.35 (t, *J* = 8.4 Hz, 2H), 6.32 (s, 1H), 3.67-3.83 (m, 2H), 3.25 (s, 3H), 2.88 (br s, 2H), 2.53 (d, *J* = 6.8 Hz, 2H), 2.40-2.46 (m, 1H), 2.23 (ddd, *J* = 8.0, 6.4, 4.4 Hz, 1H), 2.11-2.18 (m, 1H), 1.84-1.94 (m, 1H), 1.42-1.49 (m, 1H), 1.32-1.40 (m, 2H), 1.23 (s, 1H), 1.07 (d, *J* = 4.8 Hz, 3H), 0.93-1.02 (m, 1H), 0.21-0.33 (m, 2H)
LCMS: m/z = 537.3 (M+H)⁺, Rt = 1.532 min

To afford 2-(((1S,6R)-2-azabicyclo[4.1.0]heptan-2-yl)methyl)-6-(3-((1s,3R)-3-methyl-1-(4-methyl-4H-1,2,4-triazol-3-yl)cycl obutyl)phenyl)-4-(trifluoromethyl)-1,6-dihydro-7H-pyrrolo[2,3-c]pyridin-7-one (35.6 mg, 64.7 µmol, 34.7% yield, 97.6% purity) as a white solid which was confirmed by H NMR and LCMS.
¹H NMR: (400 MHz, DMSO-*d*₆)
*δ* 12.46 (s, 1H), 8.29 (s, 1H), 7.71 (s, 1H), 7.48-7.55 (m, 2H), 7.35 (t, *J* = 8.4 Hz, 2H), 6.33 (s, 1H), 3.66-3.84 (m, 2H), 3.25 (s, 3H), 2.84-2.94 (m, 2H), 2.53 (d, *J* = 6.8 Hz, 2H), 2.41-2.47 (m, 1H), 2.21-2.29 (m, 1H), 2.15 (d, *J* = 5.6 Hz, 1H), 1.84-1.94 (m, 1H), 1.42-1.49 (m, 1H), 1.32-1.40 (m, 2H), 1.23 (s, 1H), 1.07 (d, *J* = 5.2 Hz, 3H), 0.98 (t, *J* = 8.0 Hz, 1H), 0.21-0.34 (m, 2H)
LCMS: m/z = 537.3 (M+H)⁺, Rt = 1.532 min

### Example 57: Synthesis of compound T057

### 1. General steps for preparation of 2-((methyl(2,2,2-trifluoroethyl)amino)methyl)-6-(3-(1-(4-methyl-4H-1,2,4-triazol-3-yl)cyclobutyl)phenyl)-4-(triflu oromethyl)-1,6-dihydro-7H-pyrrolo[2,3-c]pyridin-7-one (compound T057)

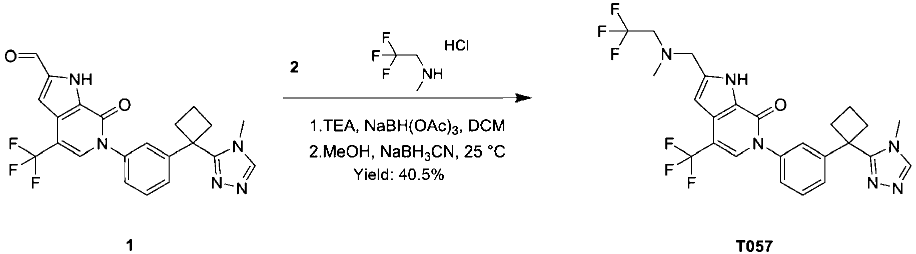

To a solution of 6-(3-(1-(4-methyl-4H-1,2,4-triazol-3-yl)cyclobutyl)phenyl)-7-oxo-4-(trifluoromethyl)-6,7-dihydro-1H-pyrrolo[2,3-c]pyridine-2-carbaldehyde (87.0 mg, 197 µmol) in dichloromethane (2.50 mL) was added dropwise TEA (19.9 mg, 197 µmol, 27.4 µL) adjust pH = 7, 2,2,2-trifluoro-N-methyl-ethanamine (38.3 mg, 256 µmol, HCl) was added to the mixture, stirred at 25 °C for 0.5 hr, then NaBH(OAc)₃ (104 mg, 492 µmol) was added to the mixture, stirred at 25 °C for 1 hr. ThenNaBH₃CN (12.3 mg, 197 µmol) and methyl alcohol (1.00 mL) was add to the mixture and stirred for 1 hr. LCMS showed desired compound was detected. The reaction mixture was diluted with aqueous NaHCO₃ (8.00 mL), extracted with dichloromethane (12.0 mL * 3), the combined organic layers were washed with H₂O (8.00 mL), dried over Na₂SO₄, filtered and concentrated under reduced pressure to give a residue. The residue was purified by Prep-HPLC (column: Welch Xtimate C18 40 * 200 mm 7 um; mobile phase: [water (NH₄HCO₃)-ACN]; gradient: 28.0% - 68.0% B over 25 mins). Compound 2-((methyl(2,2,2-trifluoroethyl)amino)methyl)-6-(3-(1-(4-methyl-4H-1,2,4-triazol-3-yl)cyclobutyl)phenyl)-4-(triflu oromethyl)-1,6-dihydro-7H-pyrrolo[2,3-c]pyridin-7-one (43.5 mg, 79.8 µmol, 40.5% yield, 98.9% purity) was obtained as a white solid. Confirmed by H NMR and LCMS.
¹H NMR: (DMSO-*d*₆, 400 MHz)
*δ* 12.51 (s, 1H), 8.35 (s, 1H), 7.73 (d, *J* = 1.2 Hz, 1H), 7.49-7.54 (m, 1H), 7.44 (t, *J* = 1.6 Hz, 1H), 7.35-7.39 (m, 1H), 7.26 (d, *J* = 7.6 Hz, 1H), 6.38 (d, *J* = 0.8 Hz, 1H), 3.87 (s, 2H), 3.28-3.32 (m, 2H), 3.27 (s, 3H), 2.88-2.97 (m, 2H), 2.67-2.74 (m, 2H), 2.37 (s, 3H), 1.92-2.04 (m, 2H)
LCMS: m/z = 539.1 (M+H)⁺, Rt = 2.247 min

### Example 58: Synthesis of compound T058

### 1. General steps for preparation of 6-(3-((1s,3R)-3-methyl-1-(4-methyl-4H-1,2,4-triazol-3-yl)cyclobutyl)phenyl)-2-(((S)-3-methylpiperidin-1-yl)methy 1)-1-tosyl-4-(trifluoromethyl)-1,6-dihydro-7H-pyrrolo[2,3-c]pyridin-7-one

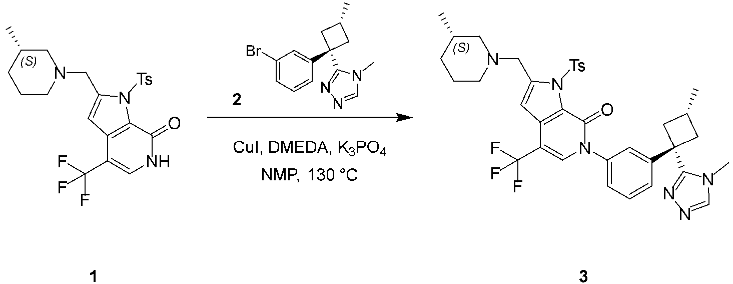

To a solution of 2-[[(3S)-3-methyl-1-piperidyl]methyl]-1-(p-tolylsulfonyl)-4-(trifluoromethyl)-6H-pyrrolo[2,3-c]pyridin-7-one (30.0 mg, 64.1 µmol) and 3-((1s,3s)-1-(3-bromophenyl)-3-methylcyclobutyl)-4-methyl-4H-1,2,4-triazole (21.6 mg, 70.5 µmol) in NMP (1.00 mL) was added K₃PO₄ (40.9 mg, 192 µmol), DMEDA (11.3 mg, 128 µmol) and CuI (12.2 mg, 64.0 µmol). The suspension was degassed under vacuum and purged with N₂ several times. The mixture was stirred under N₂ at 130 °C for 1 hr. TLC (DCM/ MeOH = 10/ 1, product 1 R_{f} = 0.35) indicated new spot formed. The reaction mixture was poured into saturated aqueous NH₄Cl (10 mL) slowly. The mixture was extracted with EtOAc (10.0 mL*2). The organic phase was washed with brine (20.0 mL), dried over Na₂SO₄ and concentrated in vacuum. The crude product was purified by column chromatography (SiO₂, DCM/ MeOH = 1/ 0 to 15/ 1). To afford 6-(3-((1s,3R)-3-methyl-1-(4-methyl-4H-1,2,4-triazol-3-yl)cyclobutyl)phenyl)-2-(((S)-3-methylpiperidin-1-yl)methy 1)-1-tosyl-4-(trifluoromethyl)-1,6-dihydro-7H-pyrrolo[2,3-c]pyridin-7-one (30.0 mg, 33.7 µmol, 52.5% yield, 77.9% purity) as a yellow solid which was confirmed by LCMS.
LCMS: m/z = 693.2 (M+H)⁺, Rt = 1.278 min

### 2. General steps for preparation of 6-(3-((1s,3R)-3-methyl-1-(4-methyl-4H-1,2,4-triazol-3-yl)cyclobutyl)phenyl)-2-(((S)-3-methylpiperidin-1-yl)methy 1)-4-(trifluoromethyl)-1,6-dihydro-7H-pyrrolo[2,3-c]pyridin-7-one (compound T058)

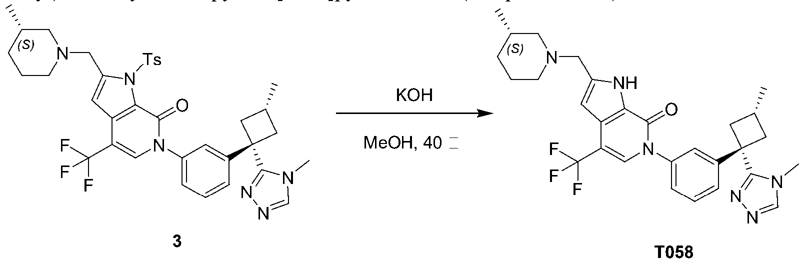

To a solution of 6-(3-((1s,3R)-3-methyl-1-(4-methyl-4H-1,2,4-triazol-3-yl)cyclobutyl)phenyl)-2-(((S)-3-methylpiperidin-1-yl)methy 1)-1-tosyl-4-(trifluoromethyl)-1,6-dihydro-7H-pyrrolo[2,3-c]pyridin-7-one (30.0 mg, 43.3 µmol) in MeOH (3.00 mL) was added KOH (72.9 mg, 1.30 mmol). The mixture was stirred under N₂ at 40 °C for 1hr. LCMS showed desired mass was detected. The reaction mixture was diluted with EtOAc (30.0 mL), washed with brine (30.0 mL), dried over Na₂SO₄. The organic phase was concentrated in vacuum. The crude product was purified by Prep-HPLC (column: Xtimate C18 150*40 mm*10 um; mobile phase: [water (HCl) - ACN]; gradient: 8% - 48% B over 30 min). To afford 6-(3-((1s,3R)-3-methyl-1-(4-methyl-4H-1,2,4-triazol-3-yl)cyclobutyl)phenyl)-2-(((S)-3-methylpiperidin-1-yl)methy 1)-4-(trifluoromethyl)-1,6-dihydro-7H-pyrrolo[2,3-c]pyridin-7-one (11.8 mg, 20.0 µmol, 46.3% yield, 97.71% purity, HCl) as a white solid which was confirmed by H NMR and LCMS.
¹H NMR: (400 MHz, CD₃CN)
*δ* 12.49 (s, 1H), 8.76 (s, 1H), 7.61 (d, *J* = 6.0 Hz, 2H), 7.51-7.59 (m, 2H), 7.43 (d, *J* = 7.2 Hz, 1H), 6.62 (s, 1H), 4.24-4.40 (m, 2H), 3.44 (s, 3H), 3.31-3.36 (m, 1H), 3.23 (br d, *J* = 12.0 Hz, 1H), 2.74-2.90 (m, 2H), 2.55-2.72 (m, 4H), 2.42-2.51 (m, 1H), 2.03-2.14 (m, 1H), 1.80-1.91 (m, 3H), 1.14-1.20 (m, 1H), 1.13 (d, *J* = 5.2 Hz, 3H), 0.92 (d, *J* = 6.4 Hz, 3H)
LCMS: m/z = 539.2 (M+H)⁺, Rt = 1.588 min

### Examples 59 and 60: Synthesis of compounds T059& T060

### 1. General steps for preparation of 6-[3-[cyclobutyl-(4-methyl-1,2,4-triazol-3-yl)methyl]phenyl]-2-[[(3S)-3-methyl-1-piperidyl]methyl]-1-(p-tolylsulfo nyl)-4-(trifluoromethyl)pyrrolo[2,3-c]pyridin-7-one

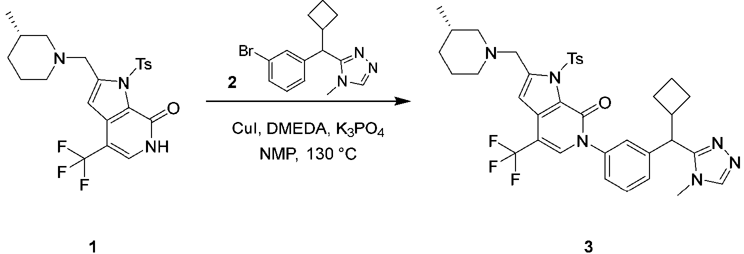

To a solution of 2-[[(3S)-3-methyl-1-piperidyl]methyl]-1-(p-tolylsulfonyl)-4-(trifluoromethyl)-6H-pyrrolo[2,3-c]pyridin-7-one (129 mg, 276 µmol) and 3-[(3-bromophenyl)-cyclobutyl-methyl]-4-methyl-1,2,4-triazole (94.0 mg, 306 µmol), CuI (116 mg, 613 µmol), K₃PO₄ (195 mg, 920 µmol) in NMP (1.00 mL) was added dropwise DMEDA (27.0 mg, 306 µmol, 33.0 µL), the mixture was stirred at 130 °C for 2 hrs under N₂. TLC indicated one major new spot with larger polarity was detected, (DCM/ MeOH = 10/ 1, R_{f} (product) = 0.45). The reaction mixture was diluted with H₂O (30.0 mL), extracted with ethyl acetate (20.0 mL * 3), the combined organic layers were washed with H₂O (20.0 mL * 3), dried over Na₂SO₄, filtered and concentrated under reduced pressure to give a residue. The residue was purified by column chromatography (SiO₂, Dichloromethane/ Methanol = 3.00% to 15.0%), (Plate 1, Dichloromethane/ Methanol = 10/ 1, R_{f} (product) = 0.45). Compound 6-[3-[cyclobutyl-(4-methyl-1,2,4-triazol-3-yl)methyl]phenyl]-2-[[(3S)-3-methyl-1-piperidyl]methyl]-1-(p-tolylsulfo nyl)-4-(trifluoromethyl)pyrrolo[2,3-c]pyridin-7-one (115 mg, 104 µmol, 34.0% yield, 62.9% purity) was obtained as a colorless oil. Confirmed by LCMS.
LCMS: m/z = 693.0 (M+H)⁺, Rt = 1.298 min

### 2. General steps for preparation of 6-[3-[cyclobutyl-(4-methyl-1,2,4-triazol-3-yl)methyl]phenyl]-2-[[(3S)-3-methyl-1-piperidyl]methyl]-4-(trifluorome thyl)-1H-pyrrolo[2,3-c]pyridin-7-one

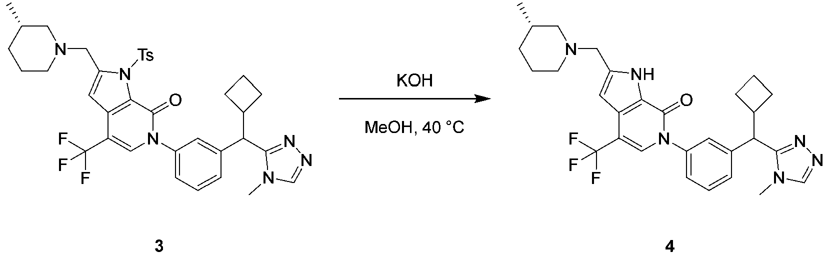

A mixture of 6-[3-[cyclobutyl-(4-methyl-1,2,4-triazol-3-yl)methyl]phenyl]-2-[[(3S)-3-methyl-1-piperidyl]methyl]-1-(p-tolylsulfo nyl)-4-(trifluoromethyl)pyrrolo[2,3-c]pyridin-7-one (115 mg, 166 µmol), KOH (186 mg, 3.32 mmol) in MeOH (1.50 mL) was degassed and purged with N₂ for 3 times, and then the mixture was stirred at 40 °C for 1 hr. LCMS showed desired compound was detected. The reaction mixture was diluted with brine (30.0 mL), extracted with ethyl acetate (20.0 mL * 3), the combined organic layers were washed with aqueous KOH (20.0 mL * 2), dried over Na₂SO₄, filtered and concentrated under reduced pressure to give a residue. The residue was purified by Prep-HPLC (column: Xtimate C18 150 * 40 mm * 10 um; mobile phase: [water (NH₃H₂O+NH₄HCO₃)-ACN]; gradient: 32.0%-72.0% B over 32 mins). Compound 6-[3-[cyclobutyl-(4-methyl-1,2,4-triazol-3-yl)methyl]phenyl]-2-[[(3S)-3-methyl-1-piperidyl]methyl]-4-(trifluorome thyl)-1H-pyrrolo[2,3-c]pyridin-7-one (80.0 mg, 146 µmol, 88.5% yield, 98.9% purity) was obtained as a colorless oil. Confirmed by LCMS.
LCMS: m/z = 539.1 (M+H)⁺, Rt = 0.997 min

### 3. General steps for preparation of 6-(3-((S)-cyclobutyl(4-methyl-4H-1,2,4-triazol-3-yl)methyl)phenyl)-2-(((S)-3-methylpiperidin-1-yl)methyl)-4-(trifl uoromethyl)-1,6-dihydro-7H-pyrrolo[2,3-c]pyridin-7-one (compound T059) and 6-(3-((R)-cyclobutyl(4-methyl-4H-1,2,4-triazol-3-yl)methyl)phenyl)-2-(((S)-3-methylpiperidin-1-yl)methyl)-4-(trifl uoromethyl)-1,6-dihydro-7H-pyrrolo[2,3-c]pyridin-7-one (compound T060)

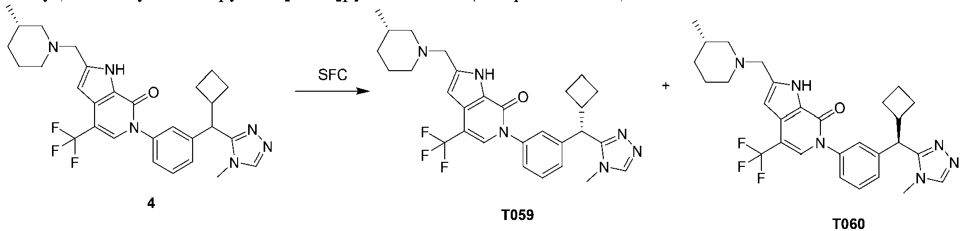

The residue was purified by Prep-HPLC (column: Daicel Chiralpak IBN 250 mm * 30 mm * 10 um; mobile phase: [CO₂-EtOH (0.1% NH₃H₂O)]; B%:35.0%, isocratic elution mode). Compound 6-(3-((S)-cyclobutyl(4-methyl-4H-1,2,4-triazol-3-yl)methyl)phenyl)-2-(((S)-3-methylpiperidin-1-yl)methyl)-4-(trifl uoromethyl)-1,6-dihydro-7H-pyrrolo[2,3-c]pyridin-7-one (10.2 mg, 18.7 µmol, 12.6% yield, 98.9% purity) was obtained as a white solid. Confirmed by H NMR and LCMS.
¹H NMR: (DMSO-*d*₆, 400 MHz)
*δ* 12.43 (s, 1H), 8.33 (s, 1H), 7.66 (d, *J =* 1.2 Hz, 1H), 7.43-7.49 (m, 1H), 7.39 (d, *J =* 1.6 Hz, 1H), 7.33-7.37 (m, 1H), 7.30 (d, *J =* 7.6 Hz, 1H), 6.27 (d, *J =* 1.2 Hz, 1H), 4.28 (d, *J =* 10.4 Hz, 1H), 3.60 (s, 2H), 3.46 (s, 3H), 3.13-3.23 (m, 1H), 2.70-2.80 (m, 2H), 2.01-2.10 (m, 1H), 1.88 (s, 1H), 1.77-1.86 (m, 4H), 1.62-1.74 (m, 2H), 1.58-1.62 (m, 2H), 1.45-1.57 (m, 2H), 1.21-1.44 (m, 1H), 0.81 (d, *J =* 5.6 Hz, 3H)
LCMS: m/z = 539.3 (M+H)⁺, Rt =1.627 min

Compound 6-(3-((R)-cyclobutyl(4-methyl-4H-1,2,4-triazol-3-yl)methyl)phenyl)-2-(((S)-3-methylpiperidin-1-yl)methyl)-4-(trifl uoromethyl)-1,6-dihydro-7H-pyrrolo[2,3-c]pyridin-7-one (10.9 mg, 19.7 µmol, 13.3% yield, 97.7% purity) was obtained as a white solid. Confirmed by H NMR and LCMS.
¹H NMR: (DMSO-*d*₆, 400 MHz)
*δ* 12.31-12.59 (m, 1H), 8.33 (s, 1H), 7.66 (d, *J =* 1.2 Hz, 1H), 7.43-7.48 (m, 1H), 7.39 (d, *J =* 1.6 Hz, 1H), 7.33-7.37 (m, 1H), 7.30 (d, *J =* 7.6 Hz, 1H), 6.27 (s, 1H), 4.28 (d, *J =* 10.4 Hz, 1H), 3.61 (s, 2H), 3.46 (s, 3H), 3.16-3.23 (m, 1H), 2.76 (s, 2H), 2.01-2.09 (m, 1H), 1.77-1.85 (m, 4H), 1.64-1.76 (m, 2H), 1.54-1.64 (m, 4H), 1.45 (d, *J =* 12.0 Hz, 1H), 1.23 (s, 1H), 0.81 (d, *J =* 5.6 Hz, 3H)
LCMS: m/z = 539.3 (M+H)⁺, Rt =1.630 min

### Example 61: Synthesis of compound T061

### 1. General steps for preparation of 4-iodo-7-methoxy-2-[[(3S)-3-methyl-1-piperidyl]methyl]-1-(p-tolylsulfonyl)pyrrolo[2,3-c]pyridine

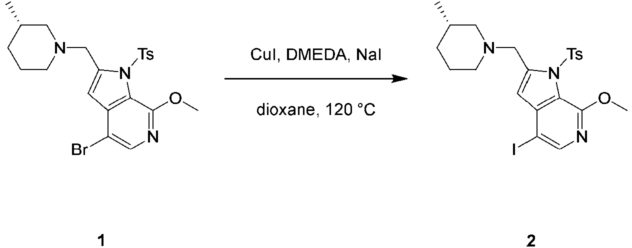

To a solution of 4-bromo-7-methoxy-2-[[(3S)-3-methyl-1-piperidyl]methyl]-1-(p-tolylsulfonyl)pyrrolo[2,3-c]pyridine (500 mg, 1.02 mmol), NaI (913 mg, 6.09 mmol) and CuI (387 mg, 2.03 mmol) in dioxane (10.0 mL) was added DMEDA (179 mg, 2.03 mmol). The mixture was stirred under N₂ at 120 °C for 24 hrs. TLC (PE/ EtOAc = 5/ 1*,* product 1 R_{f} = 0.50) indicated one spot formed. The reaction mixture was diluted with EtOAc (50.0 mL). The mixture was filtered and the filter cake was washed with EtOAc (10.0 mL*3). The filter was washed with brine (50 mL), dried over Na₂SO₄. The organic phase was concentrated in vacuum. The crude product was purified by column chromatography (SiO₂, PE/ EtOAc = 1/ 0 to 10/ 1). To afford 4-iodo-7-methoxy-2-[[(3S)-3-methyl-1-piperidyl]methyl]-1-(p-tolylsulfonyl)pyrrolo[2,3-c]pyridine (450 mg, 790 µmol, 77.8% yield, 94.7% purity) as a yellow solid which was confirmed by H NMR and LCMS.
¹H NMR: (400 MHz, CDCl₃)
*δ* 8.39 (d, *J* = 8.0 Hz, 2H), 8.05 (s, 1H), 7.29 (d, *J =* 8.4 Hz, 2H), 6.46 (s, 1H), 3.95 (s, 2H), 3.81-3.84 (m, 3H), 2.88-2.99 (m, 2H), 2.43 (s, 3H), 1.98 (t, *J =* 10.0 Hz, 1H), 1.70-1.81 (m, 2H), 1.57-1.69 (m, 3H), 0.93 (d, *J* = 12.4 Hz, 1H), 0.87 (d, *J =* 5.6 Hz, 3H)
LCMS: m/z = 539.9 (M+H)⁺, Rt = 1.063 min

### 2. General steps for preparation of 7-methoxy-2-[[(3S)-3-methyl-1-piperidyl]methyl]-1-(p-tolylsulfonyl)-4-(trifluoromethyl)pyrrolo[2,3-c]pyridine

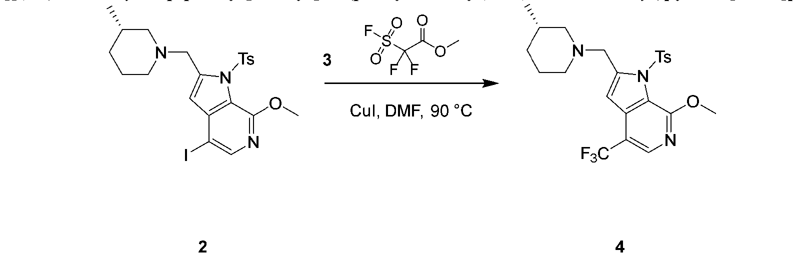

To a solution of 4-iodo-7-methoxy-2-[[(3S)-3-methyl-1-piperidyl]methyl]-1-(p-tolylsulfonyl)pyrrolo[2,3-c]pyridine (200 mg, 370 µmol) in DMF (8 mL) was added methyl 2,2-difluoro-2-fluorosulfonyl-acetate (143 mg, 744 µmol) and CuI (106 mg, 556 µmol). The mixture was stirred under N₂ at 90 °C for 2 hrs. TLC (PE/ EtOAc = 3/ 1, product 1 R_{f} = 0.60) indicated new spot formed. The reaction mixture was poured into saturated aqueous NH₄Cl (10.0 mL) slowly. The mixture was extracted with EtOAc (10.0 mL*2). The organic phase was washed with brine (20.0 mL), dried over Na₂SO₄ and concentrated in vacuum. The crude product was purified by column chromatography (SiO₂, PE/ EtOAc = 1/ 0 to 10/ 1). To afford 7-methoxy-2-[[(3S)-3-methyl-1-piperidyl]methyl]-1-(p-tolylsulfonyl)-4-(trifluoromethyl)pyrrolo[2,3-c]pyridine (60.0 mg, 121 µmol, 32.8% yield, 97.7% purity) as a yellow solid which was confirmed by LCMS.
LCMS: m/z = 482.0 (M+H)⁺, Rt = 1.073 min

### 3. General steps for preparation of 2-[[(3S)-3-methyl-1-piperidyl]methyl]-1-(p-tolylsulfonyl)-4-(trifluoromethyl)-6H-pyrrolo[2,3-c]pyridin-7-one

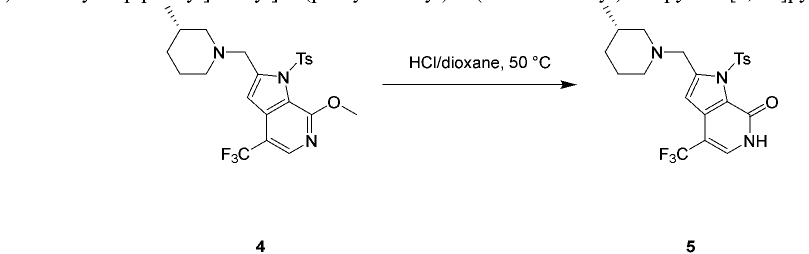

A solution of 7-methoxy-2-[[(3S)-3-methyl-1-piperidyl]methyl]-1-(p-tolylsulfonyl)-4-(trifluoromethyl)pyrrolo[2,3-c]pyridine (60.0 mg, 124 µmol) in HCl/dioxane (4 M, 2 mL). The mixture was stirred under N₂ at 50 °C for 1 hr. TLC (DCM/ MeOH = 10/ 1, product 1 R_{f} = 0.40) indicated new spot formed. The reaction mixture was poured into saturated aqueous NaHCO₃ (30 mL) slowly, adjust pH = 8 (saturated aqueous NaHCO₃). The aqueous phase was extracted with EtOAc (20 mL*2). The organic phase was washed with brine (30 mL), dried over Na₂SO₄ and concentrated in vacuum. The crude product was purified by column chromatography (SiO₂, DCM/ MeOH = 1/ 0 to 19/ 1). To afford
2-[[(3S)-3-methyl-1-piperidyl]methyl]-1-(p-tolylsulfonyl)-4-(trifluoromethyl)-6H-pyrrolo[2,3-c]pyridin-7-one (60.0 mg, 112 µmol, 90.0% yield, 87.4% purity) as a yellow solid which was confirmed by LCMS.
LCMS: m/z = 468.0 (M+H)⁺, Rt = 0.465 min

### 4. General steps for preparation of 2-[[(3S)-3-methyl-1-piperidyl]methyl]-6-[3-[3-[(4-methyl-1,2,4-triazol-3-yl)methyl]oxetan-3-yl]phenyl]-1-(p-tolyls ulfonyl)-4-(trifluoromethyl)pyrrolo[2,3-c]pyridin-7-one

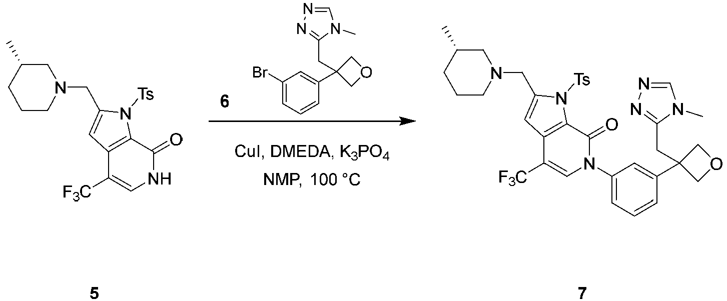

To a solution of 2-[[(3S)-3-methyl-1-piperidyl]methyl]-1-(p-tolylsulfonyl)-4-(trifluoromethyl)-6H-pyrrolo[2,3-c]pyridin-7-one (60.0 mg, 128 µmol and 3-[[3-(3-bromophenyl)oxetan-3-yl]methyl]-4-methyl-1,2,4-triazole (43.5 mg, 141 µmol) in NMP (1.00 mL) was added K₃PO₄ (81.4 mg, 383 µmol), DMEDA (22.6 mg, 256 µmol) and CuI (24.5 mg, 128 µmol). The suspension was degassed under vacuum and purged with N₂ several times. The mixture was stirred under N₂ at 100 °C for 2 hrs. LCMS showed desired mass was detected. The reaction mixture was diluted with EtOAc (50.0 mL). The mixture was filtered and the filter cake was washed with EtOAc (10.0 mL*3). The filter was washed with brine (50.0 mL), dried over Na₂SO₄. The organic phase was concentrated in vacuum. Without purification. To afford 2-[[(3S)-3-methyl-1-piperidyl]methyl]-6-[3-[3-[(4-methyl-1,2,4-triazol-3-yl)methyl]oxetan-3-yl]phenyl]-1-(p-tolyls ulfonyl)-4-(trifluoromethyl)pyrrolo[2,3-c]pyridin-7-one (40.0 mg, 47.0 µmol, 36.6% yield, 81.7% purity) as a yellow solid which was confirmed by LCMS.
LCMS: m/z = 695.1 (M+H)⁺, Rt = 1.143 min

### 5. General steps for preparation of 2-[[(3S)-3-methyl-1-piperidyl]methyl]-6-[3-[3-[(4-methyl-1,2,4-triazol-3-yl)methyl]oxetan-3-yl]phenyl]-4-(trifluor omethyl)-1H-pyrrolo[2,3-c]pyridin-7-one (compound T061)

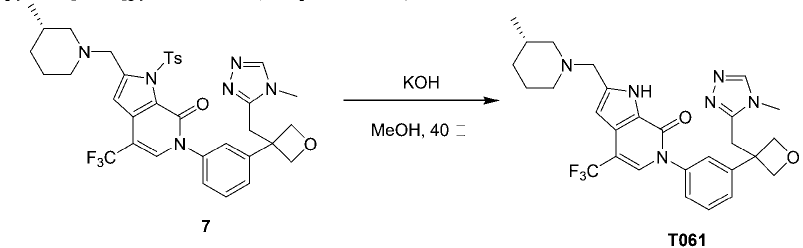

To a solution of 2-[[(3S)-3-methyl-1-piperidyl]methyl]-6-[3-[3-[(4-methyl-1,2,4-triazol-3-yl)methyl]oxetan-3-yl]phenyl]-1-(p-tolyls ulfonyl)-4-(trifluoromethyl)pyrrolo[2,3-c]pyridin-7-one (40.0 mg, 57.5 µmol) in MeOH (3.00 mL) was added KOH (96.9 mg, 1.73 mmol). The mixture was stirred under N₂ at 40 °C for 1 hr. LCMS showed desired mass was detected. The reaction mixture was adjust pH = 8 (HCl, 2 M). The mixture was filtered to get a filtrate. The crude product was purified by Prep-HPLC (column: Welch Xtimate C18 150*30 mm*5 um; mobile phase: [water (FA)-ACN]; gradient:0%-32% B over 25 min). To afford 2-[[(3S)-3-methyl-1-piperidyl]methyl]-6-[3-[3-[(4-methyl-1,2,4-triazol-3-yl)methyl]oxetan-3-yl]phenyl]-4-(trifluor omethyl)-1H-pyrrolo[2,3-c]pyridin-7-one (8.7 mg, 15.9 µmol, 27.6% yield, 98.92% purity) as a white solid which was confirmed by H NMR and LCMS.
¹H NMR: (400 MHz, DMSO-*d*₆)
*δ* 12.54 (s, 1H), 8.18 (s, 1H), 7.59 (d, *J =* 1.2 Hz, 1H), 7.39-7.44 (m, 1H), 7.33-7.38 (m, 1H), 7.12 (d, *J* = 1.6 Hz, 1H), 7.00 (d, *J =* 7.6 Hz, 1H), 6.37 (s, 1H), 4.92-4.96 (m, 2H), 4.87-4.91 (m, 2H), 3.79 (s, 2H), 3.51 (s, 2H), 2.98 (s, 3H), 2.89 (t, *J =* 10.4 Hz, 2H), 2.05-2.16 (m, 1H), 1.83 (t, *J =* 10.4 Hz, 1H), 1.64 (d, *J =* 9.6 Hz, 3H), 1.45-1.56 (m, 1H), 0.84-0.93 (m, 1H), 0.83 (d, *J =* 6.4 Hz, 3H)
LCMS: m/z = 541.4 (M+H)⁺, Rt = 1.928 min

### Examples 62 and 63: Synthesis of compounds T062& T063

### 1. General steps for preparation of 6-[3-[3-hydroxy-1-(4-methyl-1,2,4-triazol-3-yl)cyclobutyl]phenyl]-2-[[(3S)-3-methyl-1-piperidyl]methyl]-1-(p-toly lsulfonyl)-4-(trifluoromethyl)pyrrolo[2,3-c]pyridin-7-one

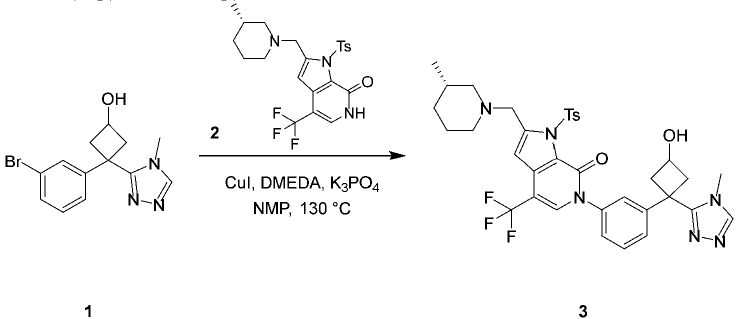

To a solution of 2-[[(3S)-3-methyl-1-piperidyl]methyl]-1-(p-tolylsulfonyl)-4-(trifluoromethyl)-6H-pyrrolo[2,3-c]pyridin-7-one (150 mg, 320 µmol) and 3-(3-bromophenyl)-3-(4-methyl-1,2,4-triazol-3-yl)cyclobutanol (109 mg, 353 µmol) in NMP (1.50 mL) was added K₃PO₄ (204 mg, 961 µmol), CuI (61.1 mg, 320 µmol) and DMEDA (28.3 mg, 321 µmol). The suspension was degassed under vacuum and purged with N₂ several times. The mixture was stirred under N₂ at 130 °C for 1 hr. TLC (DCM/ MeOH = 10/ 1, product 1 R_{f} = 0.35) indicated new spot formed. The reaction mixture was cooled to 20 °C and poured into saturated aqueous NH₄Cl (10 mL) slowly. The mixture was extracted with EtOAc (10 mL*2). The organic phase was washed with brine (20.0 mL*2), dried over Na₂SO₄ and concentrated in vacuum. The crude product was purified by column chromatography (SiO₂, DCM/ MeOH = 1/ 0 to 15/ 1). To afford
6-[3-[3-hydroxy-1-(4-methyl-1,2,4-triazol-3-yl)cyclobutyl]phenyl]-2-[[(3S)-3-methyl-1-piperidyl]methyl]-1-(p-toly lsulfonyl)-4-(trifluoromethyl)pyrrolo[2,3-c]pyridin-7-one (80.0 mg, 115 µmol, 35.8% yield) as a yellow solid which was confirmed by LCMS.
LCMS: m/z = 695.0 (M+H)⁺, Rt = 1.168 min

### 2. General steps for preparation of 6-[3-[3-hydroxy-1-(4-methyl-1,2,4-triazol-3-yl)cyclobutyl]phenyl]-2-[[(3S)-3-methyl-1-piperidyl]methyl]-4-(triflu oromethyl)-1H-pyrrolo[2,3-c]pyridin-7-one:

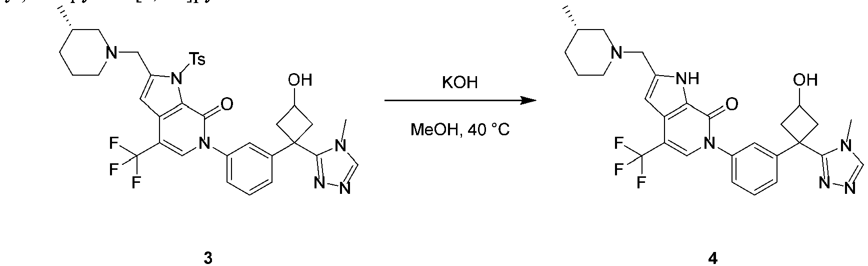

To a solution of 6-[3-[3-hydroxy-1-(4-methyl-1,2,4-triazol-3-yl)cyclobutyl]phenyl]-2-[[(3S)-3-methyl-1-piperidyl]methyl]-1-(p-toly lsulfonyl)-4-(trifluoromethyl)pyrrolo[2,3-c]pyridin-7-one (80.0 mg, 115 µmol) in MeOH (3.00 mL) was added KOH (194 mg, 3.46 mmol). The mixture was stirred under N₂ at 40 °C for 2 hrs. LCMSshowed desired mass was detected. The reaction mixture was diluted with brine (20.0 mL), extracted with EtOAc (20.0 mL*2). The organic phase washed with KOH (1 M, 20 mL), dried over Na₂SO₄ and concentrated in vacuum. The crude product was purified by Prep-HPLC (column: Xtimate C18 150*40 mm*10 um; mobile phase: [water (FA) - ACN]; gradient: 0%-34.0% B over 25 mins). To afford 6-[3-[3-hydroxy-1-(4-methyl-1,2,4-triazol-3-yl)cyclobutyl]phenyl]-2-[[(3S)-3-methyl-1-piperidyl]methyl]-4-(triflu oromethyl)-1H-pyrrolo[2,3-c]pyridin-7-one (60.0 mg, 101 µmol, 87.8% yield, 91.1% purity) as a white solid which was confirmed by LCMS.
LCMS: m/z = 541.3 (M+H)⁺, Rt = 1.248 min

### 3. General steps for preparation of 6-(3-((1s,3R)-3-hydroxy-1-(4-methyl-4H-1,2,4-triazol-3-yl)cyclobutyl)phenyl)-2-(((S)-3-methylpiperidin-1-yl)met hyl)-4-(trifluoromethyl)-1,6-dihydro-7H-pyrrolo[2,3-c]pyridin-7-one (compound T062) and 6-(3-((1r,3S)-3-hydroxy-1-(4-methyl-4H-1,2,4-triazol-3-yl)cyclobutyl)phenyl)-2-(((S)-3-methylpiperidin-1-yl)meth yl)-4-(trifluoromethyl)-1,6-dihydro-7H-pyrrolo[2,3-c]pyridin-7-one (compound T063)

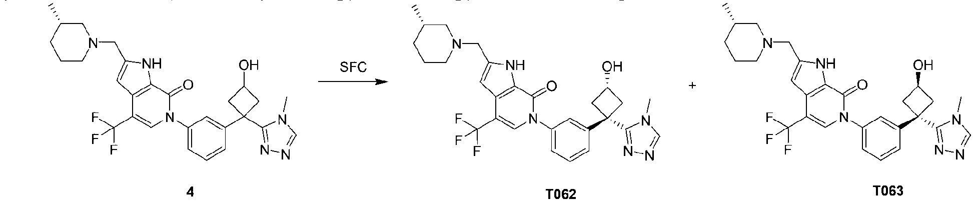

The crude product was purified by SFC (column: DAICEL CHIRALPAK AS (250 mm*30 mm, 10 um); mobile phase: [CO₂-i-PrOH (0.10% NH₃H₂O)]; B%: 25.0%, isocratic elution mode). To afford 6-(3-((1s,3R)-3-hydroxy-1-(4-methyl-4H-1,2,4-triazol-3-yl)cyclobutyl)phenyl)-2-(((S)-3-methylpiperidin-1-yl)met hyl)-4-(trifluoromethyl)-1,6-dihydro-7H-pyrrolo[2,3-c]pyridin-7-one (11.7 mg, 21.2 µmol, 19.1% yield, 98.3% purity) as a white solid which was confirmed by H NMR and LCMS.
¹H NMR: (400 MHz, DMSO-*d*₆)
*δ* 12.10-12.72 (m, 1H), 8.37 (s, 1H), 7.67-7.72 (m, 1H), 7.47-7.53 (m, 1H), 7.33-7.37 (m, 2H), 7.15-7.19 (m, 1H), 6.28 (s, 1H), 5.30 (d, *J =* 5.6 Hz, 1H), 4.02-4.10 (m, 1H), 3.62 (s, 2H), 3.29 (s, 3H), 3.27 (d, *J =* 2.8 Hz, 2H), 2.76 (d, *J =* 5.2 Hz, 2H), 2.45-2.49 (m, 2H), 1.91 (s, 1H), 1.55-1.65 (m, 4H), 1.45 (d, *J =* 12.4 Hz, 1H), 0.82-0.89 (m, 1H), 0.81 (d, *J =* 6.0 Hz, 3H)
LCMS: m/z = 541.3 (M+H)⁺, Rt = 1.178 min

To afford 6-(3-((1r,3S)-3-hydroxy-1-(4-methyl-4H-1,2,4-triazol-3-yl)cyclobutyl)phenyl)-2-(((S)-3-methylpiperidin-1-yl)meth yl)-4-(trifluoromethyl)-1,6-dihydro-7H-pyrrolo[2,3-c]pyridin-7-one (14.3 mg, 25.9 µmol, 23.3% yield, 97.9% purity) as a white solid which was confirmed by H NMR and LCMS.
¹H NMR: (400 MHz, DMSO-*d*₆)
*δ* 12.17-12.59 (m, 1H), 8.28 (s, 1H), 7.72 (d, *J =* 1.2 Hz, 1H), 7.48-7.53 (m, 2H), 7.31-7.37 (m, 2H), 6.27 (s, 1H), 5.14-5.43 (m, 1H), 4.22-4.30 (m, 1H), 3.60 (s, 2H), 3.26 (s, 3H), 3.05-3.11 (m, 2H), 2.71-2.78 (m, 4H), 1.89 (t, *J* = 10.4 Hz, 1H), 1.56-1.66 (m, 4H), 1.40-1.47 (m, 1H), 0.81 (d, *J =* 5.6 Hz, 3H), 0.76 (s, 1H)
LCMS: m/z = 541.3 (M+H)⁺, Rt = 1.185 min

The compound T063 is further purified.

The crude product was purified by SFC (column: DAICEL CHIRALPAK AS (250 mm*30 mm, 10 um); mobile phase: [CO₂-i-PrOH (0.10% NH₃H₂O)]; B%: 25.0%, isocratic elution mode). To afford 6-(3-((1r,3S)-3-hydroxy-1-(4-methyl-4H-1,2,4-triazol-3-yl)cyclobutyl)phenyl)-2-(((S)-3-methylpiperidin-1-yl)meth yl)-4-(trifluoromethyl)-1,6-dihydro-7H-pyrrolo[2,3-c]pyridin-7-one (4.10 mg, 7.43 µmol, 28.1% yield, 98.0% purity) as a white solid which was confirmed by H NMR and LCMS.
¹H NMR: (400 MHz, DMSO-*d*₆)
*δ* 12.17-12.62 (m, 1H), 8.28 (s, 1H), 7.72 (d, *J =* 1.6 Hz, 1H), 7.48-7.53 (m, 2H), 7.31-7.38 (m, 2H), 6.22-6.31 (m, 1H), 5.26-5.34 (m, 1H), 4.25 (d, *J =* 6.8 Hz, 1H), 3.60 (s, 2H), 3.26 (s, 3H), 3.08 (ddd, *J* = 9.6, 7.2, 2.8 Hz, 2H), 2.70-2.78 (m, 4H), 1.85-1.93 (m, 1H), 1.56-1.65 (m, 4H), 1.42-1.48 (m, 1H), 0.84-0.90 (m, 1H), 0.81 (d, *J =* 5.8 Hz, 3H)
LCMS: m/z = 541.2 (M+H)⁺, Rt = 1.185 min

### Example 64: Synthesis of compound T064

### 1. General steps for preparation of (S)-2-((3-hydroxy-3-methylpiperidin-1-yl)methyl)-6-(3-(1-(4-methyl-4H-1,2,4-triazol-3-yl)cyclobutyl)phenyl)-4-(tr ifluoromethyl)-1,6-dihydro-7H-pyrrolo[2,3-c]pyridin-7-one (compound T064)

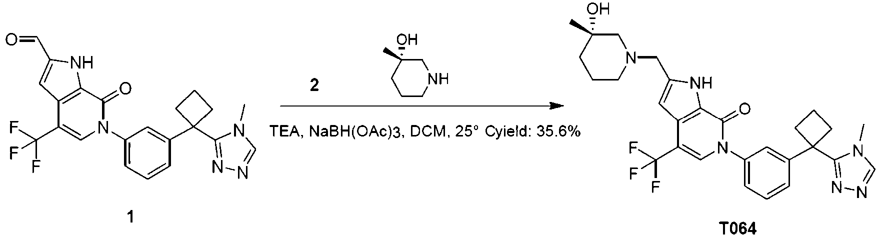

To a solution of6-[3-[1-(4-methyl-1,2,4-triazol-3-yl)cyclobutyl]phenyl]-7-oxo-4-(trifluoromethyl)-1H-pyrrolo[2,3-c]pyridine-2-c arbaldehyde (50.0 mg, 113 µmol) in dichloromethane (2.50 mL) was added dropwise TEA (11.4 mg, 113 µmol, 15.7 µL) adjust pH = 7, (3S)-3-methylpiperidin-3-ol (19.5 mg, 169 µmol) was added to the mixture, stirred at 25 °C for 0.5 hr, then NaBH(OAc)₃ (60.0 mg, 283 µmol) was added to the mixture, stirred at 25 °C for 1 hr. LCMS showed desired compound was detected. The reaction mixture was diluted with aqueous NaHCO₃ (8.00 mL), extracted with dichloromethane (12.0 mL * 3), the combined organic layers were washed with H₂O (8.00 mL), dried over Na₂SO₄, filtered and concentrated under reduced pressure to give a residue. The residue was purified by Prep-HPLC (column: Welch Xtimate C18 40 * 200 mm 7 um; mobile phase: [water (HCl)-ACN]; gradient: 0%-38.0% B over 30 min). Compound (S)-2-((3-hydroxy-3-methylpiperidin-1-yl)methyl)-6-(3-(1-(4-methyl-4H-1,2,4-triazol-3-yl)cyclobutyl)phenyl)-4-(tr ifluoromethyl)-1,6-dihydro-7H-pyrrolo[2,3-c]pyridin-7-one (23.4 mg, 40.3 µmol, 35.6% yield, 98.5% purity, HCl) was obtained as a colorless oil. Confirmed by H NMR and LCMS.
¹H NMR: (DMSO-*d*₆, 400 MHz)
δ 12.82 (s, 1H), 9.11-9.34 (m, 1H), 7.85 (s, 1H), 7.55-7.61 (m, 1H), 7.48 (s, 1H), 7.42 (t, *J =* 6.4 Hz, 2H), 6.73 (s, 1H), 4.44-4.49 (m, 1H), 4.36 (dd, *J =* 13.6, 5.2 Hz, 1H), 3.40 (s, 3H), 3.38 (d, *J =* 1.6 Hz, 1H), 2.95-3.04 (m, 3H), 2.80-2.90 (m, 2H), 2.72-2.78 (m, 2H), 1.97-2.07 (m, 3H), 1.71 (d, *J =* 13.6 Hz, 1H), 1.61 (d, *J =* 12.0 Hz, 1H), 1.40-1.48 (m, 1H), 1.23 (s, 1H), 1.15 (s, 3H)
LCMS: m/z =541.2 (M+H)⁺, Rt = 1.307 min

### Example 65: Synthesis of compound T065

### 1. General steps for preparation of 2-[[(3R)-3-hydroxy-3-methyl-1-piperidyl]methyl]-6-[3-[1-(4-methyl-1,2,4-triazol-3-yl)cyclobutyl]phenyl]-4-(triflu oromethyl)-1H-pyrrolo[2,3-c]pyridin-7-one (compound T065)

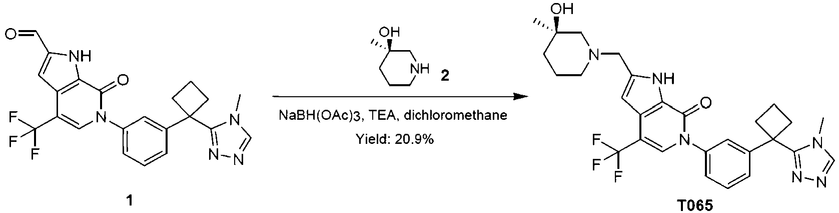

6-[3-[1-(4-methyl-1,2,4-triazol-3-yl)cyclobutyl]phenyl]-7-oxo-4-(trifluoromethyl)-1H-pyrrolo[2,3-c]pyridine-2-carbaldehyde (50.0 mg, 113 µmol) was dissolved in d (1.00 mL), pH was adjust to 7-8 with TEA. (3R)-3-methylpiperidin-3-ol (19.6 mg, 170 µmol) in dichloromethane 0.50 mL was added to the mixture, stirred at 25 °C for 0.5 hr under N₂. Then NaBH(OAc)₃ (60.0 mg, 283 µmol) was added to the mixture, then stirred at 25 °C under N₂ for 2 hrs. LCMS shows reactant was consumed completely and desired mass was detected. The reaction mixture was diluted with dichloromethane 6 mL, washed with sat. NaHCO₃ solution 2.00 mL. The water phase extracted with dichloromethane 2.00 mL (1.00 mL * 2). The combined organic layers were dried over Na₂SO₄, filtered and concentrated under reduced pressure to give a residue. The residue was purified by prep.HPLC (column: Welch Xtimate C18 40 * 200 mm 7um;mobile phase: [water (HCl) - ACN];gradient: 0% - 38% B over 30 min).
2-[[(3R)-3-hydroxy-3-methyl-1-piperidyl]methyl]-6-[3-[1-(4-methyl-1,2,4-triazol-3-yl)cyclobutyl]phenyl]-4-(triflu oromethyl)-1H-pyrrolo[2,3-c]pyridin-7-one (12.8 mg, 23.7 µmol, 20.9% yield, 100% purity) was obtained as white solid, confirmed by H NMR and LCMS.
¹H NMR: (400 MHz, DMSO-*d*₆)
*δ* 12.79 (br s, 1H), 9.40-9.75 (m, 1H), 9.01-9.29 (m, 1H), 7.84 (s, 1H), 7.54-7.61 (m, 1H), 7.38-7.49 (m, 3H), 6.73 (s, 1H), 4.42-4.51 (m, 1H), 4.31-4.40 (m, 1H), 3.38 (br s, 3H), 2.90-3.06 (m, 4H), 2.73-2.88 (m, 4H), 1.93-2.10 (m, 3H), 1.74-1.77 (m, 1H), 1.71 (br d, *J* = 14.4 Hz, 1H), 1.60 (br d, *J* = 13.2 Hz, 1H), 1.40-1.49 (m, 1H), 1.15 (s, 3H)
LCMS: m/z = 541.2 (M+H)⁺, Rt = 1.355 min

### Examples 66 and 67: Synthesis of compounds T066& T067

### 1. General steps for preparation of 3-(3-bromophenyl)-3-(4-methyl-1,2,4-triazol-3-yl)cyclobutanol

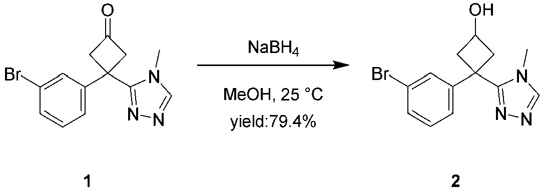

NaBH4 (98.8 mg, 2.61 mmol) was added to the mixture of 3-(3-bromophenyl)-3-(4-methyl-1,2,4-triazol-3-yl)cyclobutanone (400 mg, 1.31 mmol) in Methanol (5.00 mL) at 0 °C under N₂,then the mixture was stirred at 25 °C for 2 hrs. LCMS shows reactant was consumed completely and desired mass was detected. The reaction mixture was quenched by addition water 1.00 mL at 20 °C, and then diluted with Dichloromethane 10.0 mL, dried over Na₂SO₄, dried over Na₂SO₄, filtered and concentrated under reduced pressure to give a residue. The residue was purified by column chromatography (SiO2, Dichloromethane : Methanol = 100 : 0 to 92 : 8). TLC (Plate 1, Dichloromethane : Methanol = 10 : 1, product 1 R_{f} = 0.2). 3-(3-bromophenyl)-3-(4-methyl-1,2,4-triazol-3-yl)cyclobutanol (390 mg, 1.04 mmol, 79.4% yield, 82.0% purity) was obtained as white solid, confirmed by LCMS and H NMR.
¹H NMR: (400 MHz, DMSO-*d*₆)
*δ* 8.24-8.41 (m, 1H), 7.42-7.48 (m, 1H), 7.09-7.41 (m, 3H), 5.30 (dd, *J =* 6.8, 2.4 Hz, 1H), 3.96-4.28 (m, 1H), 3.23 (ddd, *J* = 9.2*,* 6.8, 2.4 Hz, 1H), 3.18 (d, *J =* 1.2 Hz, 3H), 3.01 (ddd, *J =* 9.6, 7.2, 2.8 Hz, 1H), 2.65-2.77 (m, 1H), 2.38-2.46 (m, 1H)
LCMS: m/z = 308.1 (M+H)⁺, Rt = 0.849 min

### 2. General steps for preparation of 3-[1-(3-bromophenyl)-3-methoxy-cyclobutyl]-4-methyl-1,2,4-triazole:

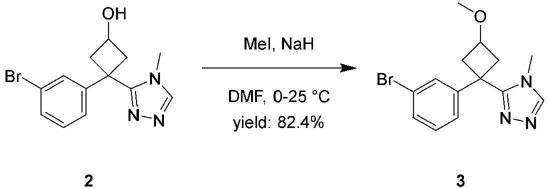

NaH (198 mg, 4.96 mmol, 60% purity) was added to the mixture of 3-(3-bromophenyl)-3-(4-methyl-1,2,4-triazol-3-yl)cyclobutanol (510 mg, 1.65 mmol) in DMF (5 mL) at 0 °C under N₂. The mixture was stirred at 0 °C for 0.5 hr. Then MeI (399 mg, 2.81 mmol, 175 µL) was added to the mixture at 0 °C, the mixture was stirred at 25 °C for 2 hrs. LCMS shows reactant was consumed completely and desired mass was detected. The reaction mixture was quenched by aqueous NH₄Cl (25.0 mL), extracted with ethyl acetate (20.0 mL * 3), the combined organic layers were washed with H₂O (15.0 mL) and brine 15.0 mL, dried over Na₂SO₄, filtered and concentrated under reduced pressure to give a residue. The residue was purified by column chromatography (SiO2, Dichloromethane : Methanol = 100 : 0 to 20 : 1). TLC (Dichloromethane : Methanol = 10 : 1, product 1, R_{f} = 0.4). 3-[1-(3-bromophenyl)-3-methoxy-cyclobutyl]-4-methyl-1,2,4-triazole (480 mg, 1.36 mmol, 82.4% yield, 91.5% purity) was obtained as light yellow oil, confirmed by LCMS and H NMR.
¹H NMR: (400 MHz, DMSO-*d*₆)
*δ* 8.27-8.43 (m, 1H), 7.44-7.50 (m, 1H), 7.15-7.44 (m, 3H), 3.82 (t, *J =* 7.2 Hz, 1H), 3.24-3.29 (m, 1H), 3.19 (s, 3H), 3.15 (d, *J =* 3.2 Hz, 3H), 2.99-3.08 (m, 1H), 2.71-2.80 (m, 1H), 2.42-2.48 (m, 1H)
LCMS: m/z = 324.0 (M+2+H)⁺, Rt = 0.508 min

### 3. General steps for 2-(1,3-dioxolan-2-yl)-6-[3-[3-methoxy-1-(4-methyl-1,2,4-triazol-3-yl)cyclobutyl]phenyl]-4-(trifluoromethyl)-1H-p yrrolo[2,3-c]pyridin-7-one

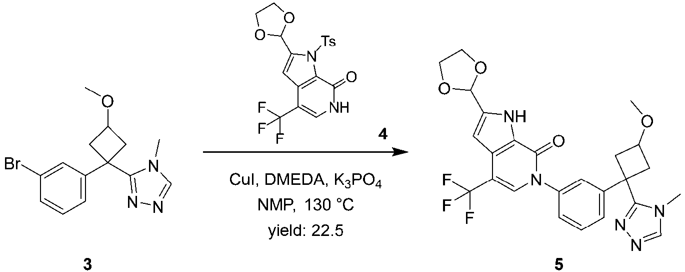

K₃PO₄ (773mg, 3.64 mmol), CuI (463 mg, 2.43 mmol) and DMEDA (107 mg, 1.21 mmol, 131 µL) was added to the solution of 3-[1-(3-bromophenyl)-3-methoxy-cyclobutyl]-4-methyl-1,2,4-triazole (430 mg, 1.33 mmol) and 2-(1,3-dioxolan-2-yl)-1-(p-tolylsulfonyl)-4-(trifluoromethyl)-6H-pyrrolo[2,3-c]pyridin-7-one (520 mg, 1.21 mmol) in NMP (5.00 mL) in order. The mixture was stirred at 130 °C under N₂ for 3 hrs. LCMS shows compound **3** was consumed completely and desired mass was detected. The reaction mixture was diluted with ethyl acetate 30.0 mL, then filtered by celatom. The filter cake was washed with ethyl acetate 90 mL (30 mL * 3). The combined organic phase was washed with NH₃H₂O/sat. NH4Cl (15%, 50.0 mL), sat. NH₄Cl solution (30.0 mL) and brine 120 mL (30.0 mL * 4) in order, dried over Na₂SO₄, filtered and concentrated under reduced pressure to give a residue. The residue was purified by column (SiO2, Dichloromethane : Methanol = 100 : 0 to 85 : 15). TLC (Dichloromethane : Methanol = 10 : 1,product 1, R_{f} = 0.2). 2-(1,3-dioxolan-2-yl)-6-[3-[3-methoxy-1-(4-methyl-1,2,4-triazol-3-yl)cyclobutyl]phenyl]-4-(trifluoromethyl)-1H-p yrrolo[2,3-c]pyridin-7-one (200 mg, 273 µmol, 22.5% yield, 70.4% purity) was obtained as yellow oil, confirmed by LCMS.
LCMS: m/z = 515.9 (M+H)⁺, Rt = 1.224 min;1.241 min

### 4. General steps for preparation of 6-[3-[3-methoxy-1-(4-methyl-1,2,4-triazol-3-yl)cyclobutyl[phenyl]-7-oxo-4-(trifluoromethyl)-1H-pyrrolo[2,3-c]pyr idine-2-carbaldehyde-

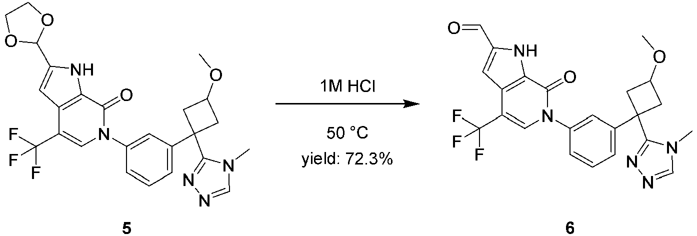

2-(1,3-dioxolan-2-yl)-6-[3-[3-methoxy-1-(4-methyl-1,2,4-triazol-3-yl)cyclobutyl]phenyl]-4-(trifluoromethyl)-1H-pyrrolo[2,3-c]pyridin-7-one (200 mg, 388 µmol) was dissolved in HCl (1 M, 2.00 mL). The mixture was stirred at 50 °C for 1 hr. LCMS shows reactant was consumed completely and desired mass was detected. The pH of the mixture was adjusted to 7-8 with sat. NaHCO₃ solution, extracted with Dichloromethane 30.0 mL (10.0 mL * 3). The combined organic layers were dried over Na₂SO₄, filtered and concentrated under reduced pressure to give a residue.
6-[3-[3-methoxy-1-(4-methyl-1,2,4-triazol-3-yl)cyclobutyl[phenyl]-7-oxo-4-(trifluoromethyl)-1H-pyrrolo[2,3-c]pyr idine-2-carbaldehyde (140 mg, 281 µmol, 72.3% yield, 94.5% purity) was obtained as yellow solid, confirmed by H NMR and LCMS.
¹H NMR: (400 MHz, DMSO-*d*₆)
*δ* 13.53-13.76 (m, 1H), 9.91 (s, 1H), 8.28-8.42 (m, 1H), 7.87 (dd, *J =* 4.0, 1.2 Hz, 1H), 7.34 (br d, *J =* 8.4 Hz, 4H), 7.20 (s, 1H), 3.97-4.10 (m, 1H), 3.27 (d, *J =* 7.2 Hz, 3H), 3.05-3.19 (m, 5H), 2.75-2.82 (m, 1H)
LCMS: m/z = 472.1 (M+H)⁺, Rt = 1.075 min

### 5. General steps for preparation of 6-[3-[3-methoxy-1-(4-methyl-1,2,4-triazol-3-yl)cyclobutyl]phenyl]-2-[[(1-methylcyclobutyl)amino]methyl]-4-(trifl uoromethyl)-1H-pyrrolo[2,3-c]pyridin-7-one

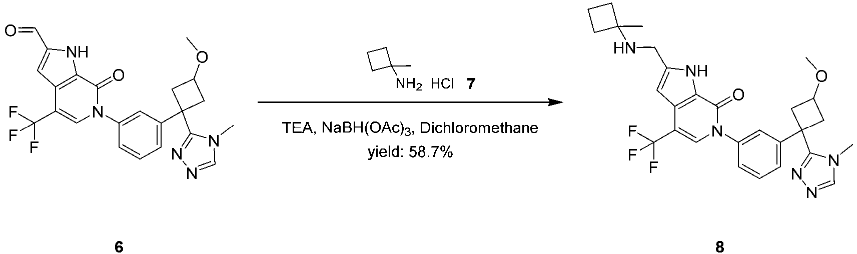

6-[3-[3-methoxy-1-(4-methyl-1,2,4-triazol-3-yl)cyclobutyl]phenyl]-7-oxo-4-(trifluoromethyl)-1H-pyrrolo[2,3-c]pyridine-2-carbaldehyde (60.0 mg, 127 µmol) was dissolved in Dichloromethane (1.00 mL), pH was adjust to 7-8 with TEA. 1-methylcyclobutanamine;hydrochloride (23.2 mg, 191 µmol) in Dichloromethane 0.50 mL was added to the mixture, stirred at 25 °C for 0.5 hr under N₂. Then NaBH(OAc)₃ (67.4 mg, 318 µmol) was added to the mixture, then stirred at 25 °C under N₂ for 12 hrs. LCMS shows reactant was consumed completely and desired mass was detected. The reaction mixture was diluted with Dichloromethane 6.00 mL, washed with sat. NaHCO₃ solution 2.00 mL. The water phase extracted with Dichloromethane 2.00 mL (1.00 mL * 2). The combined organic layers were dried over Na₂SO₄, filtered and concentrated under reduced pressure to give a residue. The residue was purified by Prep.TLC (SiO2, Dichloromethane : Methanol = 10 : 1). TLC (Dichloromethane : Methanol = 10 : 1, R_{f} (P1) = 0.4). 6-[3-[3-methoxy-1-(4-methyl-1,2,4-triazol-3-yl)cyclobutyl]phenyl]-2-[[(1-methylcyclobutyl)amino]methyl]-4-(trifl uoromethyl)-1H-pyrrolo[2,3-c]pyridin-7-one (45.0 mg, 74.7 µmol, 58.7% yield, 89.8% purity) was obtained as light yellow oil, confirmed by H NMR and LCMS.
¹H NMR: (400 MHz, CD₃OD)
*δ* 8.29-8.41 (m, 1H), 7.62 (dd, *J* = 3.2, 1.2 Hz, 1H), 7.56 (q, *J =* 8.0 Hz, 1H), 7.44-7.53 (m, 1H), 7.29-7.39 (m, 2H), 6.52 (s, 1H), 3.93-4.22 (m, 1H), 3.87 (s, 2H), 3.38 (d, *J* = 4.4 Hz, 3H), 3.34-3.36 (m, 1H), 3.27 (d, *J =* 2.8 Hz, 3H), 3.21 (ddd, *J* = 9.6, 7.2, 2.8 Hz, 1H), 2.86 (br s, 1H), 2.61-2.69 (m, 1H), 2.06 (br d, *J =* 10.4 Hz, 2H), 1.72-1.91 (m, 4H), 1.36 (s, 3H)
LCMS: m/z = 541.2 (M+H)⁺, Rt = 1.497 min

### 6. General steps for preparation of 6-(3-((1r,3r)-3-methoxy-1-(4-methyl-4H-1,2,4-triazol-3-yl)cyclobutyl)phenyl)-2-(((1-methylcyclobutyl)amino)met hyl)-4-(trifluoromethyl)-1,6-dihydro-7H-pyrrolo[2,3-c]pyridin-7-one (compound T066) 6-(3-((1s,3s)-3-methoxy-1-(4-methyl-4H-1,2,4-triazol-3-yl)cyclobutyl)phenyl)-2-(((1-methylcyclobutyl)amino)met hyl)-4-(trifluoromethyl)-1,6-dihydro-7H-pyrrolo[2,3-c]pyridin-7-one (compound T067)

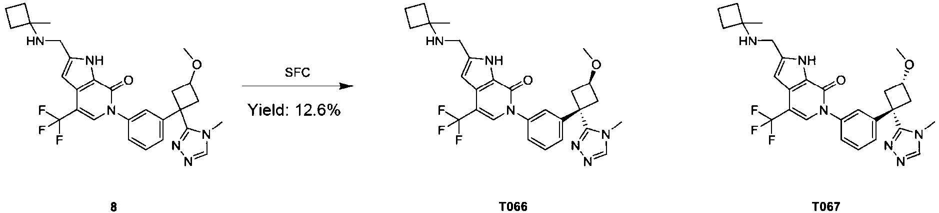

The residue was purified by prep.HPLC (column: Welch Xtimate C18 40 * 200 mm 7 um;mobile phase: [water(FA)-ACN];gradient: 0%-36% B over 25 min) and SFC (column: DAICEL CHIRALPAK IC(250 mm * 3 0mm,10 um);mobile phase: [CO₂ - EtOH (0.1% NH₃H₂O)];B%: 60%, isocratic elution mode). 6-(3-((1r,3r)-3-methoxy-1-(4-methyl-4H-1,2,4-triazol-3-yl)cyclobutyl)phenyl)-2-(((1-methylcyclobutyl)amino)met hyl)-4-(trifluoromethyl)-1,6-dihydro-7H-pyrrolo[2,3-c]pyridin-7-one (5.65 mg, 10.4 µmol, 12.6% yield, 100% purity) was obtained as white solid, confirmed by H NMR and LCMS.
¹H NMR:(400 MHz, DMSO-*d₆*)
*δ* 11.84-12.86 (m, 1H), 8.38 (s, 1H), 8.17-8.24 (m, 1H), 7.71 (d, *J =* 1.2 Hz, 1H), 7.46-7.54 (m, 1H), 7.32-7.41 (m, 2H), 7.19 (d, *J =* 8.0 Hz, 1H), 6.35 (s, 1H), 3.85 (br t, *J* = 7.2 Hz, 1H), 3.76 (s, 2H), 3.33 (br d, *J =* 2.8 Hz, 2H), 3.30 (s, 4H), 3.16 (s, 3H), 2.54 (br d, *J =* 2.4 Hz, 2H), 1.90-2.02 (m, 2H), 1.60-1.74 (m, 4H), 1.24 (s, 3H)
LCMS: m/z = 541.2 (M+H)⁺, Rt = 1.382 min

6-(3-((1s,3s)-3-methoxy-1-(4-methyl-4H-1,2,4-triazol-3-yl)cyclobutyl)phenyl)-2-(((1-methylcyclobutyl)amino )methyl)-4-(trifluoromethyl)-1,6-dihydro-7H-pyrrolo[2,3-c]pyridin-7-one (2.40 mg, 4.44 µmol, 5.33% yield, 99.9% purity) was obtained as white solid, confirmed by H NMR and LCMS.
¹H NMR: (400 MHz, DMSO-*d₆*)
*δ* 11.84-12.87 (m, 1H), 8.30 (s, 1H), 8.20 (s, 1H), 7.72 (s, 1H), 7.47-7.55 (m, 2H), 7.35 (br dd, *J =* 19.2, 8.0 Hz, 2H), 6.35 (s, 1H), 4.06 (br t, *J* = 7.2 Hz, 1H), 3.75 (s, 2H), 3.27 (s, 4H), 3.15 (s, 3H), 3.08-3.14 (m, 2H), 2.74-2.82 (m, 2H), 1.94 (br d, *J =* 8.0 Hz, 2H), 1.60-1.73 (m, 4H), 1.23 (s, 3H)
LCMS: m/z = 541.2 (M+H)⁺, Rt = 1.367 min

### Example 68: Synthesis of compound T068

### 1. General steps for preparation of 4-bromo-7-methoxy-1-(p-tolylsulfonyl)pyrrolo[2,3-c]pyridine

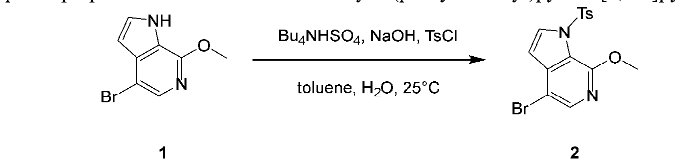

To a solution of 4-bromo-7-methoxy-1H-pyrrolo[2,3-c]pyridine (95.0 g, 418 mmol) in toluene (900 mL) was added Bu₄NHSO₄ (28.4 g, 83.6 mmol) and NaOH (125 g, 1.26 mol, 40.0% purity) at 25 °C under N₂. The mixture was stirred under N₂ at 25 °C for 1 hr. Then 4-methylbenzenesulfonyl chloride (83.7 g, 439 mmol) was added to the mixture at 25 °C under N₂. The mixture was stirred under N₂ at 25 °C under N₂ for 2 hrs. LCMS showed the reaction was completed. TLC (Plate 1, Petroleum ether/ Ethyl acetate = 5/ 1*,* UV 254 nm, R_{f} (product) = 0.5). The reaction mixture was poured into saturated aqueous NH₄Cl (1000 mL) slowly. The mixture was extracted with Ethyl acetate (1000 mL), washed with brine (1000 mL), dried over Na₂SO₄. The organic phase was concentrated in vacuum. The crude product was triturated with Petroleum ether at 25 °C for 12 hrs. Compound 4-bromo-7-methoxy-1-(p-tolylsulfonyl)pyrrolo[2,3-c]pyridine (152 g, 398 mmol, 95.2% yield) was obtained as a yellow solid. It was confirmed by H NMR.
¹H NMR: (400 MHz, CD₃Cl)
*δ* 7.98 (d, *J =* 3.6 Hz, 1H), 7.91 (s, 1H), 7.78 (d, *J =* 8.4 Hz, 2H), 7.30 (br d, *J =* 8.0 Hz, 2H), 6.70 (d, *J =* 3.6 Hz, 1H), 3.89 (s, 3H), 2.42 (s, 3H)

### 2. General steps for preparation of 4-iodo-7-methoxy-1-(p-tolylsulfonyl)pyrrolo[2,3-c]pyridine:

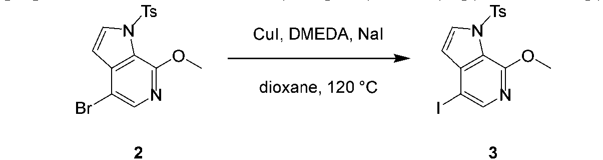

To a solution of 4-bromo-7-methoxy-1-(p-tolylsulfonyl)pyrrolo[2,3-c]pyridine (18.0 g, 47.2 mmol), CuI (4.50 g, 23.6 mmol), NaI (42.4 g, 283 mmol) in dioxane (200 mL) was added DMEDA (4.16 g, 47.2 mmol, 5.08 mL). The mixture was stirred under N₂ at 120 °C for 12 hrs. LCMS showed the reaction was completed. The reaction mixture was diluted with Ethyl acetate (200 mL). The mixture was filtered and the filter cake was washed with Ethyl acetate (200 mL * 3). The filtrate was washed with brine (200 mL), dried over Na₂SO₄. The organic phase was concentrated in vacuum. The crude product was triturated with MTBE at 25 °C for 12 hrs. Compound 4-iodo-7-methoxy-1-(p-tolylsulfonyl)pyrrolo[2,3-c]pyridine (12.6 g, 29.4 mmol, 62.3% yield) was obtained as a white solid. It was confirmed by H NMR.
¹H NMR: (400 MHz, CD₃Cl)
*δ* 7.98 (s, 1H), 7.91 (d, *J* = 3.6 Hz, 1H), 7.70 (d, *J =* 8.4 Hz, 2H), 7.22 (d, *J =* 8.0 Hz, 2H), 6.52 (d, *J =* 3.6 Hz, 1H), 3.79-3.82 (m, 3H), 2.34 (s, 3H)

### 3. General steps for preparation of 7-methoxy-1-(p-tolylsulfonyl)-4-(trifluoromethyl)pyrrolo[2,3-c]pyridine:

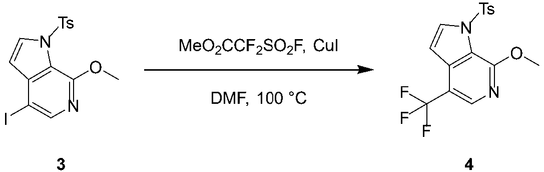

To a solution of 4-iodo-7-methoxy-1-(p-tolylsulfonyl)pyrrolo[2,3-c]pyridine (8.70 g, 20.3 mmol) in DMF (90.0 mL) was added methyl 2,2-difluoro-2-fluorosulfonyl-acetate (23.4 g, 121 mmol, 15.5 mL), CuI (15.4 g, 81.2 mmol). The mixture was stirred under N₂ at 100 °C for 3 hrs. LCMS showed the reaction was completed. The reaction mixture was diluted with Ethyl acetate (100 mL). The mixture was filtered and the filter cake was washed with Ethyl acetate (100 mL * 3). The filtrate was washed with brine (100 mL), dried over Na₂SO₄. The organic phase was concentrated in vacuum. The residue was purified by column chromatography (SiO₂, Petroleum ether/ Ethyl acetate = 1/ 0 to 95/ 5). TLC (Plate 1, Petroleum ether/ Ethyl acetate = 5/ 1, UV 254 nm, R_{f} (product) = 0.25). Compound 7-methoxy-1-(p-tolylsulfonyl)-4-(trifluoromethyl)pyrrolo[2,3-c]pyridine (7.41 g, 20.0 mmol, 98.4% yield) was obtained as a white solid. It was confirmed by H NMR.
¹H NMR: (400 MHz, CD₃Cl)
*δ* 8.14 (d, *J* = 1.2 Hz, 1H), 8.05 (d, *J* = 3.6 Hz, 1H), 7.80 (d, *J* = 8.4 Hz, 2H), 7.32 (d, *J* = 8.0 Hz, 2H), 6.77-6.83 (m, 1H), 3.96 (s, 3H), 2.43 (s, 3H)

### 4. General steps for preparation of methyl 7-methoxy-1-(p-tolylsulfonyl)-4-(trifluoromethyl)pyrrolo[2,3-c]pyridine-2-carboxylate

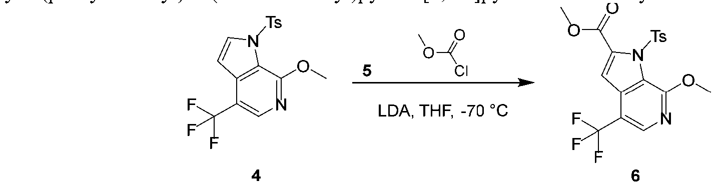

To a solution of 7-methoxy-1-(p-tolylsulfonyl)-4-(trifluoromethyl)pyrrolo[2,3-c]pyridine (1.00 g, 2.70 mmol) in THF (20.0 mL) was added LDA (2 M, 2.03 mL) at -70 °C for 0.5 hr, followed by dropwise addition of methyl carbonochloridate (2.72 g, 28.7 mmol, 2.22 mL) at -70 °C. The mixture was stirred under N₂ at -70 °C for 1.5 hrs. LCMS showed the reaction was completed. The reaction mixture was quenched by NH₄Cl (30.0 mL) at 0 °C, and then diluted with H₂O (30.0 mL). The combined organic layers were washed with Ethyl acetate(30.0 mL * 3), dried over[Na₂SO₄], filtered and concentrated under reduced pressure to give a residue. The organic phase was concentrated in vacuum. The residue was purified by column chromatography (SiO₂, Petroleum ether/ Ethyl acetate = 1/ 0 to 95/ 5). TLC (Plate 1, Petroleum ether/ Ethyl acetate = 5/ 1, UV 254 nm, R_{f} (product) = 0.3). Compound methyl 7-methoxy-1-(p-tolylsulfonyl)-4-(trifluoromethyl)pyrrolo[2,3-c]pyridine-2-carboxylate (960 mg, 2.24 mmol, 82.9% yield) was obtained as a white solid. It was confirmed by H NMR.
¹H NMR: (400 MHz, CD₃Cl)
*δ* 8.28 (d, *J =* 8.4 Hz, 2H), 8.23 (s, 1H), 7.42 (d, *J =* 8.4 Hz, 2H), 7.19 (s, 1H), 4.04 (s, 3H), 3.99 (s, 3H), 2.49 (s, 3H)

### 5. General steps for preparation of dideuterio-[7-methoxy-4-(trifluoromethyl)-1H-pyrrolo[2,3-c]pyridin-2-yl]methanol

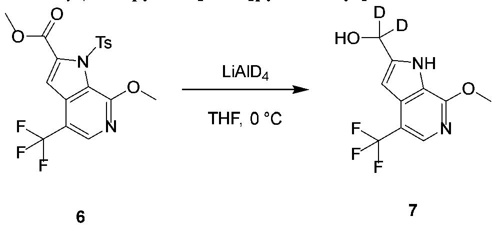

To a solution of methyl 7-methoxy-1-(p-tolylsulfonyl)-4-(trifluoromethyl)pyrrolo[2,3-c]pyridine-2-carboxylate (960 mg, 2.24 mmol) in THF (10.0 mL) was added lithium; tetradeuterioalumanuide (188 mg, 4.48 mmol, 255 µL) dropwise at 0 °C, The mixture was warmed to 25 °C and stirred for 2 hrs under N₂. LCMS showed the reaction was completed. The reaction mixture was quenched by NH₄Cl (20.0 mL) at 0 °C. The combined organic layers were washed with Ethyl acetate (20.0 mL * 3), dried over [Na₂SO₄], filtered and concentrated under reduced pressure to give a residue. The organic phase was concentrated in vacuum. The residue was purified by column chromatography (SiO₂, Petroleum ether/ Ethyl acetate = 1/ 0 to 75/ 25). TLC (Plate 1, Petroleum ether/ Ethyl acetate = 3/ 1, UV 254 nm, R_{f} (product) = 0.2). Compound dideuterio-[7-methoxy-4-(trifluoromethyl)-1H-pyrrolo[2,3-c]pyridin-2-yl]methanol (402 mg, 1.62 mmol, 72.2% yield) was obtained as a white solid. It was confirmed by H NMR.
¹H NMR: (400 MHz, CD₃Cl)
*δ* 9.06 (br s, 1H), 8.06 (s, 1H), 6.52 (s, 1H), 4.14 (s, 3H)

### 6. General steps for preparation of 2-[chloro(dideuterio)methyl]-7-methoxy-4-(trifluoromethyl)-1H-pyrrolo[2,3-c]pyridine

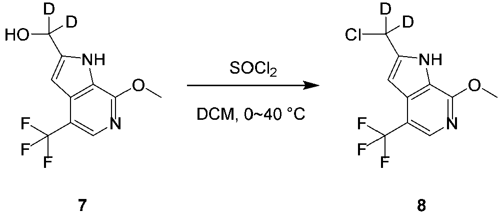

To a solution of dideuterio-[7-methoxy-4-(trifluoromethyl)-1H-pyrrolo[2,3-c]pyridin-2-yl]methanol (375 mg, 1.51 mmol) in DCM (6.00 mL) was added SOCl₂ (1.80 g, 15.1 mmol, 1.10 mL) dropwise at 0 °C. The mixture was warmed to 40 °C and stirred for 1 hr under N₂. LCMS showed the reaction was completed. TLC (Plate 1, Petroleum ether /Ethyl acetate = 2/ 1, UV 254 nm, R_{f} (product) = 0.7). The mixture was concentrated under reduced pressure to give a residue. Compound 2-[chloro(dideuterio)methyl]-7-methoxy-4-(trifluoromethyl)-1H-pyrrolo[2,3-c]pyridine (375 mg, 1.41 mmol, 93.0% yield) was obtained as a white solid.

### 7. General steps for preparation of 2-[dideuterio-[(3S)-3-methyl-1-piperidyl]methyl]-7-methoxy-4-(trifluoromethyl)-1H-pyrrolo[2,3-c]pyridine

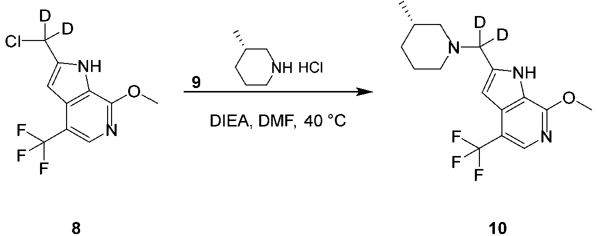

To a solution of (3S)-3-methylpiperidine (408 mg, 3.02 mmol, HCl), DIEA (974 mg, 7.54 mmol, 1.31 mL) in DMF (5.00 mL) was added 2-[chloro(dideuterio)methyl]-7-methoxy-4-(trifluoromethyl)-1H-pyrrolo[2,3-c]pyridine (402 mg, 1.51 mmol) dropwise at 25 °C. The mixture was warmed to 40 °C and stirred for 2 hrs under N₂. LCMS showed the reaction was completed. The mixture was diluted with water (10.0 mL), extracted with ethyl acetate (20.0 * 3 mL). The combined organic layers were washed with brine (20.0 mL), dried over [Na₂SO₄], filtered and concentrated under reduced pressure to give a residue. The organic phase was concentrated in vacuum. The residue was purified by column chromatography (SiO₂, Petroleum ether/ Ethyl acetate = 1/ 0 to 83/ 17). TLC (Plate 1, Petroleum ether/ Ethyl acetate = 2/ 1, UV 254 nm, R_{f} (product) = 0.2). Compound 2-[dideuterio-[(3S)-3-methyl-1-piperidyl]methyl]-7-methoxy-4-(trifluoromethyl)-1H-pyrrolo[2,3-c]pyridine (207 mg, 628 µmol, 41.6% yield) was obtained as a white solid. It was confirmed by H NMR.
¹H NMR: (400 MHz, CD₃Cl)
*δ* 9.27 (br s, 1H), 8.04 (s, 1H), 6.46 (s, 1H), 4.12 (s, 3H), 2.82 (br d, *J =* 12.0 Hz, 1H), 2.77 (br d, *J =* 7.2 Hz, 1H), 1.96-2.02 (m, 1H), 1.75 (br s, 1H), 1.68-1.72 (m, 2H), 1.62-1.68 (m, 2H), 1.54-1.61 (m, 1H), 0.86 (br d, *J =* 5.2 Hz, 3H)

### 8. General steps for preparation of 2-[dideuterio-[(3S)-3-methyl-1-piperidyl]methyl]-4-(trifluoromethyl)-1,6-dihydropyrrolo[2,3-c]pyridin-7-one

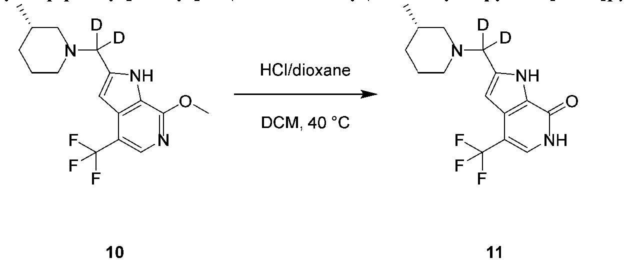

To a solution of 2-[dideuterio-[(3S)-3-methyl-1-piperidyl]methyl]-7-methoxy-4-(trifluoromethyl)-1H-pyrrolo[2,3-c]pyridine (50.0 mg, 151 µmol) in DCM (2.00 mL) was added HCl/ dioxane (4 M, 2.00 mL). The mixture was warmed to 50 °C and stirred for 1 hr under N₂. LCMS showed the reaction was completed. The reaction mixture was diluted with brine (10.0 mL), washed with DCM (10.0 mL * 2), dried over Na₂SO₄. The organic phase was concentrated in vacuum. Compound 2-[dideuterio-[(3S)-3-methyl-1-piperidyl]methyl]-4-(trifluoromethyl)-1,6-dihydropyrrolo[2,3-c]pyridin-7-one (50.0 mg, crude) was obtained as a white solid. It was confirmed by H NMR.
¹H NMR: (400 MHz, DMSO-*d*₆)
*δ* 12.26 (br s, 1H), 11.40 (br d, *J =* 4.0 Hz, 1H), 7.38 (br d, *J =* 5.2 Hz, 1H), 6.21 (br s, 1H), 2.75 (br s, 2H), 1.90 (br d, *J =* 6.4 Hz, 1H), 1.54-1.63 (m, 4H), 1.41-1.49 (m, 1H), 1.23 (s, 1H), 0.80 (br d, *J =* 6.0 Hz, 3H)

### 9. General steps for preparation of 2-[dideuterio-[(3 S)-3-methyl-1-piperidyl]methyl]-6-[3-[3-methyl-1-(4-methyl-1,2,4-triazol-3-yl)cyclobutyl]phenyl] -4-(trifluoromethyl)-1H-pyrrolo[2,3-c]pyridin-7-one (compound T068)

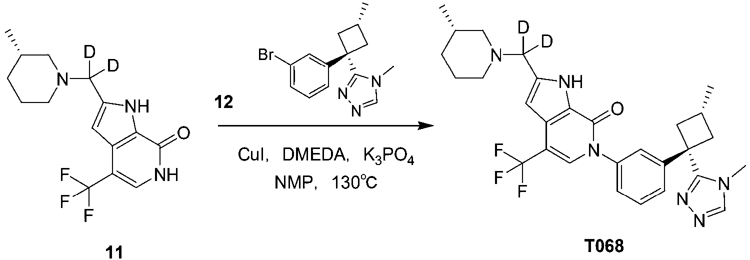

To a mixture of 2-[dideuterio-[(3S)-3-methyl-1-piperidyl]methyl]-4-(trifluoromethyl)-1,6-dihydropyrrolo[2,3-c]pyridin-7-one (160 mg, 507 µmol), 3-[1-(3-bromophenyl)-3-methyl-cyclobutyl]-4-methyl-1,2,4-triazole (155 mg, 507 µmol), CuI (193 mg, 1.01 mmol) and K₃PO₄ (323 mg, 1.52 mmol) in NMP (5.00 mL) was added DMEDA (44.7 mg, 507 µmol, 54.6 µL). The suspension was degassed under vacuum and purged with N₂ several times. The mixture was stirred under N₂ at 130 °C for 3 hrs. LCMS showed the reaction was completed. The reaction mixture was diluted with Ethyl acetate (20.0 mL), washed with brine (20.0 mL * 3), dried over Na₂SO₄. The organic phase was concentrated in vacuum. The product was purified by Prep-HPLC (column: Xtimate C18 150 * 40 mm * 10 um; mobile phase: [water (FA)-ACN]; gradient: 0%-40% B over 25 min). Compound 2-[dideuterio-[(3S)-3-methyl-1-piperidyl]methyl]-6-[3-[3-methyl-1-(4-methyl-1,2,4-triazol-3-yl)cyclobutyl]phenyl] -4-(trifluoromethyl)-1H-pyrrolo[2,3-c]pyridin-7-one (152 mg, 281 µmol, 55.5% yield, 99.7% purity) was obtained as a white solid. It was confirmed by LCMS and H NMR.
¹H NMR: (400 MHz, DMSO-*d*₆)
*δ* 12.34-12.56 (m, 1H), 8.28 (s, 1H), 8.15 (s, 1H), 7.72 (d, *J =* 1.2 Hz, 1H), 7.48-7.54 (m, 2H), 7.35 (br t, *J =* 7.6 Hz, 2H), 6.28 (s, 1H), 3.25 (s, 3H), 2.88 (br s, 1H), 2.77-2.94 (m, 1H), 2.75 (br s, 1H), 2.54-2.61 (m, 1H), 2.52 (br s, 2H), 1.92 (br t, *J =* 10.6 Hz, 1H), 1.55-1.67 (m, 4H), 1.39-1.50 (m, 1H), 1.07 (br d, *J =* 5.2 Hz, 3H), 0.81 (br d, *J=* 6.0 Hz, 3H)
LCMS: m/z = 541.0 (M+H)⁺, Rt = 1.095 min

### Example 69: Synthesis of compound T069

### 1. General steps for preparation of 2-((ethyl(isobutyl)amino)methyl)-6-(3-((1s,3s)-3-methyl-1-(4-methyl-4H-1,2,4-triazol-3-yl)cyclobutyl)phenyl)-4-(t rifluoromethyl)-1,6-dihydro-7H-pyrrolo[2,3-c]pyridin-7-one (compound T069)

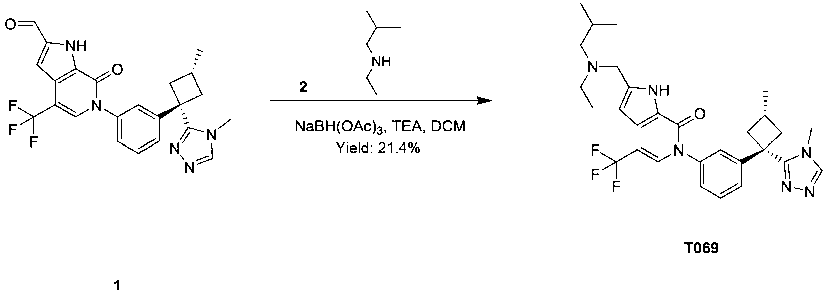

To a solution of 6-(3-((1s,3s)-3-methyl-1-(4-methyl-4H-1,2,4-triazol-3-yl)cyclobutyl)phenyl)-7-oxo-4-(trifluoromethyl)-6,7-dihydro -1H-pyrrolo[2,3-c]pyridine-2-carbaldehyde (50.0 mg, 113 µmol) in dichloromethane (2.00 mL) was added dropwise TEA (11.4 mg, 113 µmol, 15.7 µL) adjust pH = 7, N-ethyl-2-methyl-propan-1-amine (22.6 mg, 164 µmol HCl) was added to the mixture, stirred at 25 °C for 0.5 hr, then NaBH(OAc)₃ (58.1 mg, 274 µmol) was added to the mixture, stirred at 25 °C for 1 hr. LCMS showed desired compound was detected. The reaction mixture was diluted with aqueous NaHCO₃ (8.00 mL), extracted with dichloromethane (12.0 mL * 3), the combined organic layers were washed with H₂O (8.00 mL), dried over Na₂SO₄, filtered and concentrated under reduced pressure to give a residue. The residue was purified by Prep-HPLC (column: Welch Xtimate C18 40 * 200 mm 7 um; mobile phase: [water (HCl)-ACN]; gradient:6.00%-46.0% B over 25 mins). Compound 2-((ethyl(isobutyl)amino)methyl)-6-(3-((1s,3s)-3-methyl-1-(4-methyl-4H-1,2,4-triazol-3-yl)cyclobutyl)phenyl)-4-(t rifluoromethyl)-1,6-dihydro-7H-pyrrolo[2,3-c]pyridin-7-one (13.0 mg, 23.5 µmol, 21.4% yield, 98.1% purity) was obtained as a white solid. Confirmed by H NMR and LCMS.
¹H NMR: (DMSO- *d*₆, 400 MHz)
*δ* 12.90 (s, 1H), 9.43 (s, 1H), 7.84 (d, *J =* 1.2 Hz, 1H), 7.66 (s, 1H), 7.56-7.61 (m, 1H), 7.50 (d, *J =* 8.0 Hz, 1H), 7.46 (d, *J =* 8.0 Hz, 1H), 6.80 (s, 1H), 4.50 (d, *J =* 3.6 Hz, 2H), 3.46 (s, 3H), 3.14-3.24 (m, 1H), 3.08 (dd, *J =* 12.0, 6.0 Hz, 1H), 2.91-3.00 (m, 3H), 2.74-2.86 (m, 1H), 2.54-2.69 (m, 3H), 2.02 (dt, *J =* 13.2, 6.8 Hz, 1H), 1.31 (t, *J =* 7.2 Hz, 3H), 1.08 (d, *J =* 6.0 Hz, 3H), 0.99 (d, *J =* 6.4 Hz, 3H), 0.95 (d, *J =* 6.4 Hz, 3H)
LCMS: m/z = 541.3 (M+H)⁺, Rt = 1.620 min

### Examples 70 and 71: Synthesis of compounds T070& T071

### 1. General steps for preparation of 6-[3-[3-hydroxy-1-(4-methyl-1,2,4-triazol-3-yl)cyclobutyl]phenyl]-2-[[(3S)-3-methyl-1-piperidyl]methyl]-1-(p-toly lsulfonyl)-4-(trifluoromethyl)pyrrolo[2,3-c]pyridin-7-one

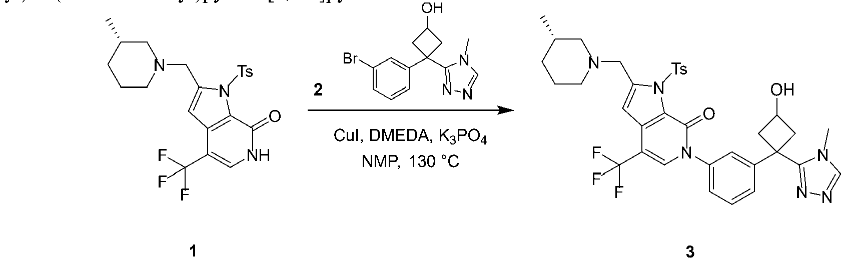

A mixture of 2-[[(3S)-3-methyl-1-piperidyl]methyl]-1-(p-tolylsulfonyl)-4-(trifluoromethyl)-6H-pyrrolo[2,3-c]pyridin-7-one (703 mg, 1.50 mmol), 3-(3-bromophenyl)-3-(4-methyl-1,2,4-triazol-3-yl)cyclobutanol (510 mg, 1.65 mmol), K₃PO₄ (958 mg, 4.51 mmol), CuI (573 mg, 3.01 mmol) in NMP (7.00 mL) was added DMEDA (132 mg, 1.50 mmol, 161 µL) was degassed and purged with N₂ for 3 times, and then the mixture was stirred at 130 °C for 1 hr under N₂ atmosphere. LCMS showed desired compound was detected. The reaction mixture was diluted with H₂O (30.0 mL), extracted with ethyl acetate (20.0 mL * 3), the combined organic layers were washed with H₂O (2.00 mL * 3), dried over Na₂SO₄, filtered and concentrated under reduced pressure to give a residue. The residue was purified by column chromatography (SiO₂, Dichloromethane/ Methanol = 3.00% to 12.0%), (Plate 1, Dichloromethane/ Methanol = 10/ 1, R_{f} (product) = 0.27). Compound 6-[3-[3-hydroxy-1-(4-methyl-1,2,4-triazol-3-yl)cyclobutyl]phenyl]-2-[[(3S)-3-methyl-1-piperidyl]methyl]-1-(p-toly lsulfonyl)-4-(trifluoromethyl)pyrrolo[2,3-c]pyridin-7-one (560 mg, 710 µmol, 47.2% yield, 88.1% purity) was obtained as a colorless oil. Confirmed by H NMR and LCMS.
¹H NMR: (DMSO-*d*₆, 400 MHz)
*δ* 8.41 (d, *J =* 8.4 Hz, 2H), 7.97 (d, *J =* 11.2 Hz, 1H), 7.41 (d, *J =* 8.0 Hz, 2H), 7.30-7.38 (m, 1H), 7.26 (t, *J =* 6.4 Hz, 1H), 7.07-7.23 (m, 1H), 7.06-7.52 (m, 2H), 6.70 (s, 1H), 5.29 (d, *J =* 6.8 Hz, 1H), 3.92 (s, 2H), 3.28 (s, 3H), 3.17-3.26 (m, 3H), 2.99-3.10 (m, 1H), 2.63-2.83 (m, 3H), 2.39 (s, 3H), 1.92-2.03 (m, 1H), 1.72 (t, *J =* 10.4 Hz, 2H), 1.57-1.67 (m, 2H), 1.39-1.54 (m, 1H), 0.87-0.97 (m, 1H), 0.84 (d, *J =* 6.4 Hz, 3H)
LCMS: m/z = 695.1 (M+H)⁺, Rt = 1.065 min

### 2. General steps for preparation of 6-[3-[3-fluoro-1-(4-methyl-1,2,4-triazol-3-yl)cyclobutyl]phenyl]-2-[[(3S)-3-methyl-1-piperidyl]methyl]-1-(p-tolyls ulfonyl)-4-(trifluoromethyl)pyrrolo[2,3-c]pyridin-7-one

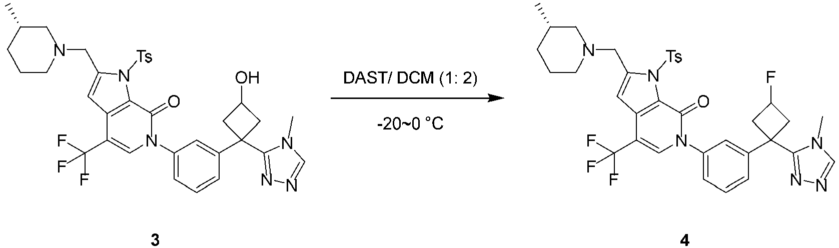

To a solution of 6-[3-[3-hydroxy-1-(4-methyl-1,2,4-triazol-3-yl)cyclobutyl]phenyl]-2-[[(3S)-3-methyl-1-piperidyl]methyl]-1-(p-toly lsulfonyl)-4-(trifluoromethyl)pyrrolo[2,3-c]pyridin-7-one (100 mg, 143 µmol) in DCM (1.00 mL) was added dropwise DAST (580 mg, 3.60 mmol, 475 µL) at -20 °C. The mixture was stirred at -20 °C for 2hrs. LCMS showed desired compound was detected. The reaction mixture was diluted with dichloromethane (30.0 mL) and quenched by aqueous Na₂CO₃ adjust pH =9, extracted with dichloromethane (20.0 mL * 3), the combined organic layers were washed with H₂O (20.0 mL * 3), dried over Na₂SO₄, filtered and concentrated under reduced pressure to give a residue. The residue was purified by column chromatography (SiO₂, Dichloromethane/ Methanol = 0% to 4.00%), (Plate 1, Dichloromethane/ Methanol = 10/ 1, R_{f} (product) = 0.34). Compound 6-[3-[3-fluoro-1-(4-methyl-1,2,4-triazol-3-yl)cyclobutyl]phenyl]-2-[[(3S)-3-methyl-1-piperidyl]methyl]-1-(p-tolyls ulfonyl)-4-(trifluoromethyl)pyrrolo[2,3-c]pyridin-7-one (37.0 mg, 37.0 µmol, 25.7% yield, 69.8% purity) was obtained as a colorless oil. Confirmed by LCMS.
LCMS: m/z = 697.0 (M+H)⁺, Rt = 1.268 min

### 3. General steps for preparation of 6-[3-[3-fluoro-1-(4-methyl-1,2,4-triazol-3-yl)cyclobutyl]phenyl]-2-[[(3S)-3-methyl-1-piperidyl]methyl]-4-(trifluoro methyl)-1H-pyrrolo[2,3-c]pyridin-7-one (compound T070) and 6-[3-[3-fluoro-1-(4-methyl-1,2,4-triazol-3-yl)cyclobutyl]phenyl]-2-[[(3S)-3-methyl-1-piperidyl]methyl]-4-(trifluoro methyl)-1H-pyrrolo[2,3-c]pyridin-7-one (compound T071)

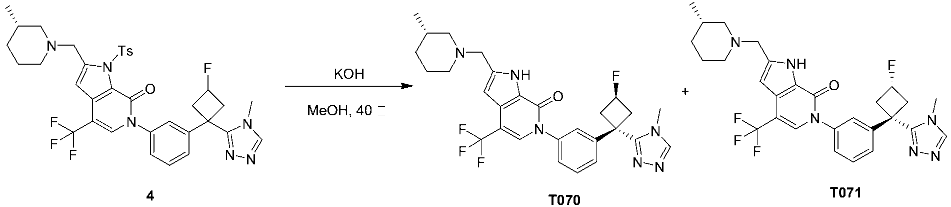

A mixture of 6-[3-[3-fluoro-1-(4-methyl-1,2,4-triazol-3-yl)cyclobutyl]phenyl]-2-[[(3S)-3-methyl-1-piperidyl]methyl]-4-(trifluoro methyl)-1H-pyrrolo[2,3-c]pyridin-7-one (28.8 mg, 53.1 µmol), KOH (59.5 mg, 1.06 mmol) in MeOH (0.50 mL) was degassed and purged with N₂ for 3 times, and then the mixture was stirred at 40 °C for 1 hr. LCMS showed desired compound was detected. The reaction mixture was diluted with H₂O (30.0 mL), extracted with ethyl acetate (20.0 mL * 3), the combined organic layers were washed with aqueous KOH (20.0 mL * 3), dried over Na₂SO₄, filtered and concentrated under reduced pressure to give a residue. The residue was purified by Prep-HPLC (column: Xtimate C18 150*40 mm*10 um;mobile phase: [water(NH₃H₂O+NH₄HCO₃)-ACN];gradient:28%-68% B over 32 mins). Compound 6-[3-[3-fluoro-1-(4-methyl-1,2,4-triazol-3-yl)cyclobutyl]phenyl]-2-[[(3S)-3-methyl-1-piperidyl]methyl]-4-(trifluoro methyl)-1H-pyrrolo[2,3-c]pyridin-7-one (4.53 mg, 8.35 µmol, 15.7% yield, 100% purity) was obtained as a white solid. Confirmed by H NMR and LCMS.
¹H NMR: (CD₃CN, 400 MHz)
*δ* 8.05 (s, 1H), 7.55 (d, *J =* 1.2 Hz, 1H), 7.47-7.53 (m, 1H), 7.41 (s, 1H), 7.34 (t, *J =* 9.2 Hz, 2H), 6.37 (s, 1H), 5.16-5.38 (m, 1H), 3.62 (s, 2H), 3.23 (s, 3H), 3.21 (s, 2H), 3.10-3.20 (m, 2H), 2.72-2.80 (m, 2H), 1.58-1.70 (m, 4H), 1.52 (dd, *J =* 12.0, 3.2 Hz, 1H), 1.27 (s, 1H), 0.88 (s, 1H), 0.83 (d, *J =* 5.6 Hz, 3H)
LCMS: m/z = 543.3 (M+H)⁺, Rt = 1.273min

Compound 6-[3-[3-fluoro-1-(4-methyl-1,2,4-triazol-3-yl)cyclobutyl]phenyl]-2-[[(3S)-3-methyl-1-piperidyl]methyl]-4-(trifluoro methyl)-1H-pyrrolo[2,3-c]pyridin-7-one (2.75 mg, 5.07 µmol, 9.54% yield, 100% purity) was obtained as a white solid. Confirmed by H NMR and LCMS.
¹H NMR: (CD₃CN, 400 MHz)
*δ* 8.09 (s, 1H), 7.55 (s, 1H), 7.47-7.53 (m, 1H), 7.32-7.38 (m, 2H), 7.27 (d, *J =* 7.6 Hz, 1H), 6.36 (s, 1H), 4.90-5.32 (m, 1H), 3.57-3.64 (m, 2H), 3.44-3.53 (m, 2H), 3.22 (s, 3H), 2.81-2.91 (m, 2H), 2.71-2.78 (m, 2H), 1.58-1.69 (m, 4H), 1.46-1.53 (m, 1H), 1.27 (s, 1H), 0.87 (d, *J =* 6.0 Hz, 1H), 0.82 (d, *J =* 5.2 Hz, 3H)
LCMS: m/z = 543.2 (M+H)⁺, Rt = 1.333 min

### Example 72: Synthesis of compound T072

### 1. General steps for preparation of 2-(((3S,4R)-3-fluoro-4-hydroxypiperidin-1-yl)methyl)-6-(3-(1-(4-methyl-4H-1,2,4-triazol-3-yl)cyclobutyl)phenyl)-4-(trifluoromethyl)-1,6-dihydro-7H-pyrrolo[2,3-c]pyridin-7-one (compound T072)

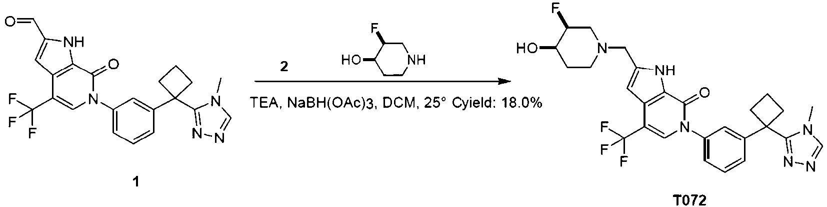

To a solution of 6-[3-[1-(4-methyl-1,2,4-triazol-3-yl)cyclobutyl]phenyl]-7-oxo-4-(trifluoromethyl)-1H-pyrrolo[2,3-c]pyridine-2-car baldehyde (50.0 mg, 113 µmol) in dichloromethane (2.50 mL) was added dropwise TEA (11.4 mg, 113 µmol, 15.7 µL) adjust pH = 7, (3S,4R)-3-fluoropiperidin-4-ol (20.2 mg, 169 µmol) was added to the mixture, stirred at 25 °C for 0.5 hr, then NaBH(OAc)₃ (60.0 mg, 283 µmol) was added to the mixture, stirred at 25 °C for 1 hr. LCMS showed desired compound was detected. The reaction mixture was diluted with aqueous NaHCO₃ (8.00 mL), extracted with dichloromethane (12.0 mL * 3), the combined organic layers were washed with H₂O (8.00 mL), dried over Na₂SO₄, filtered and concentrated under reduced pressure to give a residue. The residue was purified by Prep-HPLC (column: Welch Xtimate C18 40*200 mm 7 um; mobile phase: [water (HCl)-ACN]; gradient: 0%-36% B over 30 min). Compound 2-(((3S,4R)-3-fluoro-4-hydroxypiperidin-1-yl)methyl)-6-(3-(1-(4-methyl-4H-1,2,4-triazol-3-yl)cyclobutyl)phenyl)-4-(trifluoromethyl)-1,6-dihydro-7H-pyrrolo[2,3-c]pyridin-7-one (12.7 mg, 20.4 µmol, 18.0% yield, 99.2% purity, 2HCl) was obtained as a white solid. Confirmed by H NMR and LCMS.
¹H NMR: (DMSO-*d*₆, 400 MHz)
*δ* 12.84 (s, 1H), 9.03-9.22 (m, 1H), 7.84 (d, *J =* 1.2 Hz, 1H), 7.54-7.60 (m, 1H), 7.53 (d, *J =* 3.6 Hz, 1H), 7.37-7.45 (m, 2H), 6.68-6.80 (m, 1H), 4.69-5.08 (m, 2H), 4.49 (s, 2H), 4.43 (s, 1H), 3.55-3.64 (m, 1H), 3.39 (s, 3H), 3.14-3.24 (m, 1H), 2.92-3.03 (m, 3H), 2.72-2.80 (m, 2H), 1.89-2.05 (m, 4H), 1.23 (s, 1H)
LCMS: m/z =545.2 (M+H)⁺, Rt = 1.213 min

### Examples 73 and 74: Synthesis of compounds T073& T074

### 1. General steps for preparation of methyl 3-methyl-1-(2-thienyl)cyclobutanecarboxylate

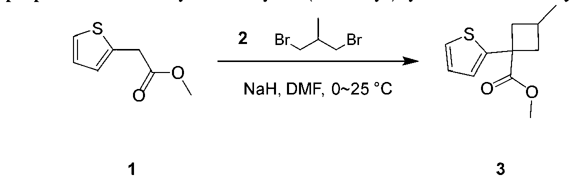

To a solution of methyl 2-(2-thienyl)acetate (10.0 g, 64.0 mmol), 1,3-dibromo-2-methyl-propane (14.5 g, 67.2 mmol) in DMF (200 mL) was added NaH (5.13 g, 128 mmol, 60.0% purity) slowly at 0 °C under N₂. After addition, the mixture was stirred at 25 °C for 2 hrs. LCMS showed desired compound was detected. The reaction mixture was diluted with aqueous NH₄Cl (100 mL), extracted with ethyl acetate (100 mL), the combined organic layers were washed with brine (200 mL * 3), dried over Na₂SO₄, filtered and concentrated under reduced pressure to give a residue. The residue was purified by column chromatography (SiO₂, Petroleum ether/ Ethyl acetate = 0% to 25.0%), (Plate 1, Petroleum ether/ Ethyl acetate = 1/ 1, R_{f} (product) = 0.46). Compound methyl 3-methyl-1-(2-thienyl) cyclobutanecarboxylate (7.10 g, 26.2 mmol, 41.0% yield, 77.8% purity) was obtained as a colorless oil. Confirmed by H NMR and LCMS.
¹H NMR: (CDCl₃, 400 MHz)
*δ* 7.17-7.23 (m, 1H), 6.97-7.03 (m, 1H), 6.83-6.96 (m, 1H), 3.66-3.74 (m, 3H), 2.38-2.65 (m, 4H), 2.03-2.17 (m, 1H), 1.07-1.10 (m, 3H)
LCMS: m/z = 210.9 (M+H)⁺, Rt = 1.653 min

### 2. General steps for preparation of 3-methyl-1-(2-thienyl)cyclobutanecarbohydrazide

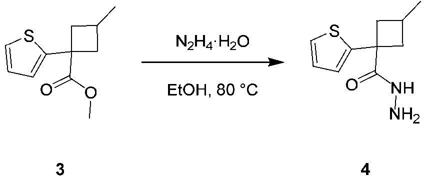

To a solution of methyl 3-methyl-1-(2-thienyl)cyclobutanecarboxylate (5.00 g, 23.7 mmol) in EtOH (50.0 mL) was added dropwise N₂H₄·H₂O (20.6 g, 404 mmol, 20.0 mL, 98.0% purity). After addition, the mixture was stirred at 80 °C for 2 hrs. LCMS showed desired compound was detected. The reaction mixture was diluted with H₂O (30.0 mL), extracted with ethyl acetate (40.0 mL * 3), the combined organic layers were washed with H₂O (30.0 mL * 3), dried over Na₂SO₄, filtered and concentrated under reduced pressure to give a residue. Compound 3-methyl-1-(2-thienyl) cyclobutanecarbohydrazide (5.04 g, crude) was obtained as a white solid.

### 3. General steps for preparation of 1-methyl-3-[[3-methyl-1-(2-thienyl)cyclobutanecarbonyl]amino]thiourea

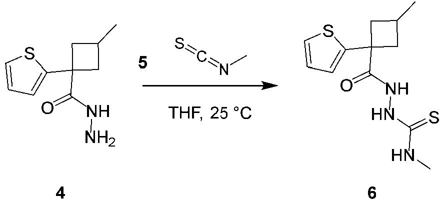

A mixture of 3-methyl-1-(2-thienyl)cyclobutanecarbohydrazide (5.04 g, 23.97 mmol), methylimino(thioxo)methane (3.50 g, 47.9 mmol, 3.28 mL) in THF (50.0 mL) was degassed and purged with N₂ for 3 times, and then the mixture was stirred at 25 °C for 3 hrs. LCMS showed desired compound was detected. Concentrated under reduced pressure to give a residue. Compound 1-methyl-3-[[3-methyl-1-(2-thienyl)cyclobutanecarbonyl]amino]thiourea (6.79 g, crude) was obtained as a white solid.

### 4. General steps for preparation of 4-methyl-5-[3-methyl-1-(2-thienyl)cyclobutyl]-1,2,4-triazole-3-thiol:

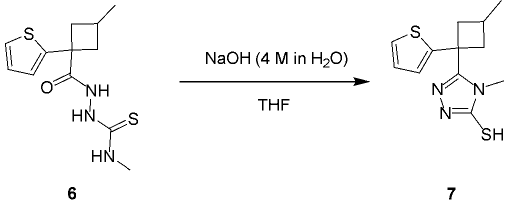

To a solution of 1-methyl-3-[[3-methyl-1-(2-thienyl)cyclobutanecarbonyl]amino]thiourea (6.79 g, 23.9 mmol) in THF (40.0 mL) was added dropwise NaOH (4 M, 10.0 mL). After addition, the mixture was stirred at 40 °C for 9 hrs. LCMS showed desired compound was detected. The reaction mixture was diluted with aqueous 1 N HCl adjust pH = 2, extracted with ethyl acetate (100 mL * 3), the combined organic layers were washed with H₂O (100 mL * 3), dried over Na₂SO₄, filtered and concentrated under reduced pressure to give a residue. Compound 4-methyl-5-[3-methyl-1-(2-thienyl)cyclobutyl]-1,2,4-triazole-3-thiol (1.98 g, 6.92 mmol, 28.8% yield, 92.7% purity) was obtained as a white solid. Confirmed by H NMR and LCMS.
¹H NMR: (DMSO-*d*₆, 400 MHz)
*δ* 13.56-13.87 (m, 1H), 7.46-7.53 (m, 1H), 7.12 (dd, *J =* 3.2, 1.2 Hz, 1H), 6.97-7.06 (m, 1H), 3.04-3.08 (m, 3H), 2.63-2.77 (m, 2H), 2.51-2.56 (m, 2H), 2.17-2.37 (m, 1H), 1.03-1.14 (m, 3H)
LCMS: m/z = 265.9 (M+H)⁺, Rt = 1.400 min

### 5. General steps for preparation of 4-methyl-3-[3-methyl-1-(2-thienyl)cyclobutyl]-1,2,4-triazole

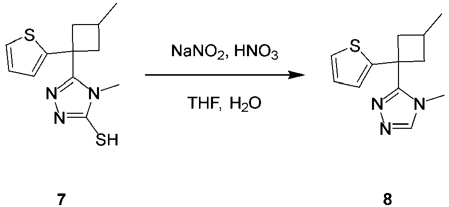

To a solution of 4-methyl-5-[3-methyl-1-(2-thienyl)cyclobutyl]-1,2,4-triazole-3-thiol (850 mg, 3.20 mmol), NaNO₂ (2.21 g, 32.0 mmol) in THF (4.00 mL) and H₂O (4.00 mL) was added dropwise HNO₃ (3.26 g, 33.6 mmol, 2.33 mL, 65.0% purity) at 0 °C. After addition, the mixture was stirred at 0 °C for 1 hr. LCMS showed desired compound was detected. The reaction mixture was diluted with NaHCO₃ adjust pH = 8, extracted with ethyl acetate (20.0 mL * 3), the combined organic layers were washed with H₂O (20.0 mL * 3), dried over Na₂SO₄, filtered and concentrated under reduced pressure to give a residue. The residue was purified by column chromatography (SiO₂, Dichloromethane/ Methyl alcohol = 0% to 10.0%), (Plate 1, Dichloromethane/ Methyl alcohol = 10/ 1, R_{f} (product) = 0.40). Compound 4-methyl-3-[3-methyl-1-(2-thienyl)cyclobutyl]-1,2,4-triazole (516 mg, 2.16 mmol, 67.4% yield, 97.7% purity) was obtained as a colorless oil. Confirmed by H NMR and LCMS.
¹H NMR: (CDCl₃, 400 MHz)
*δ* 7.94-8.11 (m, 1H), 7.16-7.23 (m, 1H), 6.89-6.95 (m, 1H), 6.59-6.85 (m, 1H), 3.24-3.29 (m, 3H), 2.79-2.88 (m, 2H), 2.49-2.78 (m, 2H), 2.26-2.35 (m, 1H), 1.11-1.17 (m, 3H)
LCMS: m/z = 233.7 (M+H)⁺, Rt = 0.995 min

### 6. General steps for preparation of 3-[1-(5-bromo-2-thienyl)-3-methyl-cyclobutyl]-4-methyl-1,2,4-triazole

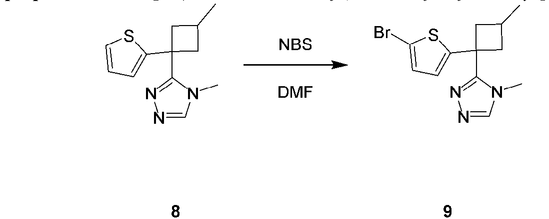

To a solution of 4-methyl-3-[3-methyl-1-(2-thienyl)cyclobutyl]-1,2,4-triazole (466 mg, 2.00 mmol) in DMF (5.00 mL) was added dropwise NBS (391 mg, 2.20 mmol) at 0 °C. After addition, the mixture was stirred at 25 °C for 2 hrs. LCMS showed desired compound was detected. The reaction mixture was diluted with H₂O (30.0 mL), extracted with ethyl acetate (20.0 mL * 3), the combined organic layers were washed with H₂O (20.0 mL * 3), dried over Na₂SO₄, filtered and concentrated under reduced pressure to give a residue. The residue was purified by column chromatography (SiO₂, Petroleum ether/ Ethyl acetate = 0% to 4.00%), (Plate 1, Petroleum ether/ Ethyl acetate = 1/ 1, R_{f} (product) = 0.34). Compound 3-[1-(5-bromo-2-thienyl)-3-methyl-cyclobutyl]-4-methyl-1,2,4-triazole (650 mg, 1.90 mmol, 95.1% yield, 91.3% purity) was obtained as a white solid. Confirmed by H NMR and LCMS.
¹H NMR: (CDCl₃, 400 MHz)
*δ* 7.96-8.11 (m, 1H), 6.84-6.92 (m, 1H), 6.35-6.64 (m, 1H), 3.27-3.38 (m, 3H), 3.12-3.25 (m, 1H), 2.74-2.83 (m, 2H), 2.44-2.74 (m, 2H), 1.12-1.17 (m, 3H)
LCMS: m/z = 311.6 (M+H)⁺, Rt = 1.355 min

### 7. General steps for preparation of 2-(1,3-dioxolan-2-yl)-6-[5-[3-methyl-1-(4-methyl-1,2,4-triazol-3-yl)cyclobutyl]-2-thienyl]-4-(trifluoromethyl)-1H-pyrrolo[2,3-c]pyridin-7-one

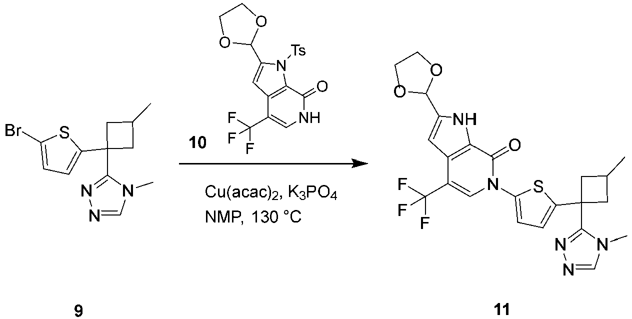

To a solution of 2-(1,3-dioxolan-2-yl)-1-(p-tolylsulfonyl)-4-(trifluoromethyl)-6H-pyrrolo[2,3-c]pyridin-7-one (260 mg, 608 µmol), 3-[1-(5-bromo-2-thienyl)-3-methyl-cyclobutyl]-4-methyl-1,2,4-triazole (190 mg, 608 µ mol), K₃PO₄ (387 mg, 1.83 mmol), Cu(acac)₂ (477 mg, 1.83 mmol) in NMP (2.00 mL) was stirred at 130 °C for 2 hrs under N₂. LCMS showed desired compound was detected. The reaction mixture was diluted with H₂O (15.0 mL), extracted with ethyl acetate (15.0 mL * 3), the combined organic layers were washed with H₂O (10.0 mL * 3), dried over Na₂SO₄, filtered and concentrated under reduced pressure to give a residue. The residue was purified by column chromatography (SiO₂, Dichloromethane/ Methyl alcohol = 0% to 4.00%), (Plate 1, Dichloromethane/ Methyl alcohol = 20/ 1, R_{f} (product) = 0.15). Compound 2-(1,3-dioxolan-2-yl)-6-[5-[3-methyl-1-(4-methyl-1,2,4-triazol-3-yl)cyclobutyl]-2-thienyl]-4-(trifluoromethyl)-1H-pyrrolo[2,3-c]pyridin-7-one (48.0 mg, 84.7 µmol, 13.9% yield, 89.3% purity) was obtained as a colorless oil. Confirmed by LCMS.
LCMS: m/z = 505.8 (M+H)⁺, Rt = 1.375 min

### 8. General steps for preparation of 6-[5-[3-methyl-1-(4-methyl-1,2,4-triazol-3-yl)cyclobutyl]-2-thienyl]-7-oxo-4-(trifluoromethyl)-1H-pyrrolo[2,3-c]p yridine-2-carbaldehyde

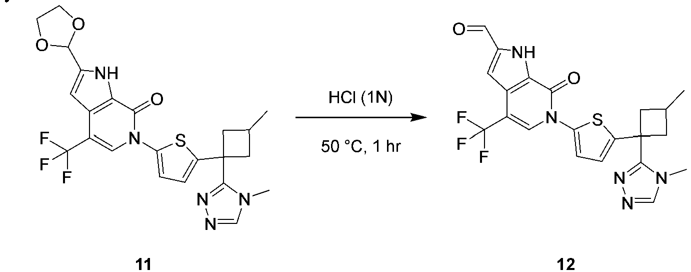

To a solution of 2-(1,3-dioxolan-2-yl)-6-[5-[3-methyl-1-(4-methyl-1,2,4-triazol-3-yl)cyclobutyl]-2-thienyl]-4-(trifluoromethyl)-1H-pyrrolo[2,3-c]pyridin-7-one (48.0 mg, 94.9 µmol) in HCl (1 M, 1.00 mL) was stirred at 50 °C for 1 hr. LCMS showed desired compound was detected. The reaction mixture was diluted with aqueous NaHCO₃ adjust pH = 8, extracted with ethyl acetate (12.0 mL * 3), the combined organic layers were washed with brine (8.00 mL), dried over Na₂SO₄, filtered and concentrated under reduced pressure to give a residue. Compound 6-[5-[3-methyl-1-(4-methyl-1,2,4-triazol-3-yl)cyclobutyl]-2-thienyl]-7-oxo-4-(trifluoromethyl)-1H-pyrrolo[2,3-c]p yridine-2-carbaldehyde (45.3 mg, 92.2 µmol, 97.1% yield, 94.0% purity) was obtained as a yellow oil.

### 9. General steps for preparation of 6-(5-((1r,3S)-3-methyl-1-(4-methyl-4H-1,2,4-triazol-3-yl)cyclobutyl)thiophen-2-yl)-2-(((S)-3-methylpiperidin-1-yl )methyl)-4-(trifluoromethyl)-1,6-dihydro-7H-pyrrolo[2,3-c]pyridin-7-one (compound T073) and 6-(5-((1s,3R)-3-methyl-1-(4-methyl-4H-1,2,4-triazol-3-yl)cyclobutyl)thiophen-2-yl)-2-(((S)-3-methylpiperidin-1-yl )methyl)-4-(trifluoromethyl)-1,6-dihydro-7H-pyrrolo[2,3-c]pyridin-7-one (compound T074)

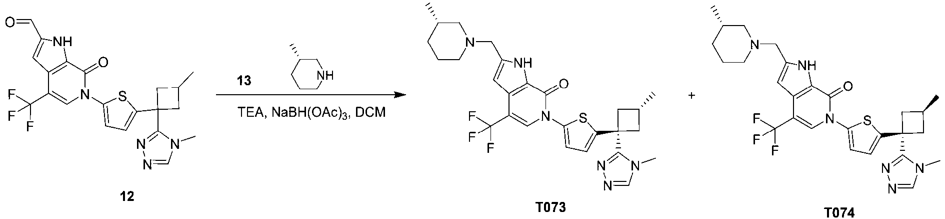

To a solution of 6-[5-[3-methyl-1-(4-methyl-1,2,4-triazol-3-yl)cyclobutyl]-2-thienyl]-7-oxo-4-(trifluoromethyl)-1H-pyrrolo[2,3-c]p yridine-2-carbaldehyde (45.3 mg, 98.1 µmol) in DCM (2.50 mL) was added dropwise TEA (9.93 mg, 98.1 µmol, 13.6 µL) adjust pH = 7, (3S)-3-methylpiperidine (14.6 mg, 107 µmol, HCl) was added to the mixture, stirred at 25 °C for 0.5 hr, then NaBH(OAc)₃ (52.0 mg, 245 µmol) was added to the mixture, stirred at 25 °C for 1 hr. LCMS showed desired compound was detected. The reaction mixture was diluted with aqueous NaHCO₃ (8.00 mL), extracted with DCM (12.0 mL * 3), the combined organic layers were washed with H₂O (8 mL), dried over Na₂SO₄, filtered and concentrated under reduced pressure to give a residue. The residue was purified by Prep-HPLC (column: Welch Xtimate C18 40 * 200 mm 7 um; mobile phase: [water(NH₃H₂O+NH₄HCO₃)-ACN];gradient:36.0%-76.0% B over 25 mins). Compound 6-(5-((1r,3S)-3-methyl-1-(4-methyl-4H-1,2,4-triazol-3-yl)cyclobutyl)thiophen-2-y1)-2-(((S)-3-methylpiperidin-1-yl )methyl)-4-(trifluoromethyl)-1,6-dihydro-7H-pyrrolo[2,3-c]pyridin-7-one (4.58 mg, 8.19 µmol, 8.34% yield, 97.4% purity) was obtained as a white solid. Confirmed by H NMR and LCMS.
¹H NMR: (CD₃OD, 400 MHz)
*δ* 8.36 (s, 1H), 7.75 (d, *J* = 1.2 Hz, 1H), 7.10 (d, *J =* 4.0 Hz, 1H), 6.99 (d, *J =* 4.0 Hz, 1H), 6.45 (s, 1H), 3.69 (s, 2H), 3.48 (s, 3H), 3.32 (s, 1H), 2.93-3.01 (m, 2H), 2.89 (s, 1H), 2.84 (d, *J =* 7.6 Hz, 2H), 2.61-2.69 (m, 2H), 1.99 (td, *J =* 11.6, 2.8 Hz, 1H), 1.69-1.77 (m, 2H), 1.68 (d, *J =* 6.4 Hz, 2H), 1.53-1.64 (m, 1H), 1.16 (d, *J =* 6.4 Hz, 2H), 0.88-0.96 (m, 1H), 0.87 (d, *J =* 5.6 Hz, 3H)
LCMS: m/z =545.2 (M+H)⁺, Rt = 1.627 min

Compound 6-(5-((1s,3R)-3-methyl-1-(4-methyl-4H-1,2,4-triazol-3-yl)cyclobutyl)thiophen-2-yl)-2-(((S)-3-methylpiperidin-1-yl )methyl)-4-(trifluoromethyl)-1,6-dihydro-7H-pyrrolo[2,3-c]pyridin-7-one (5.15 mg, 9.01 µmol, 9.18% yield, 95.3% purity) was obtained as a white solid. Confirmed by H NMR and LCMS
¹H NMR: (CD₃OD, 400 MHz)
*δ* 8.45 (s, 1H), 7.75 (d, *J* = 1.2 Hz, 1H), 7.08 (d, *J =* 4.0 Hz, 1H), 6.76 (d, *J =* 4.0 Hz, 1H), 6.46 (s, 1H), 3.70 (s, 2H), 3.46-3.48 (m, 3H), 3.18-3.25 (m, 2H), 2.84-2.93 (m, 2H), 2.37-2.51 (m, 3H), 1.98-2.06 (m, 1H), 1.67-1.78 (m, 4H), 1.57-1.64 (m, 1H), 1.20 (d, *J=* 6.0 Hz, 3H), 0.90 (s, 1H), 0.87 (d, *J=* 5.6 Hz, 3H)
LCMS: m/z =545.2 (M+H)⁺, Rt = 1.657 min

### Example 75: Synthesis of compound T075

### 1. General steps for preparation of 2-[(4,4-difluoro-1-piperidyl)methyl]-6-[3-[1-(4-methyl-1,2,4-triazol-3-yl)cyclobutyl]phenyl]-4-(trifluoromethyl)-1 H-pyrrolo[2,3-c]pyridin-7-one (compound T075)

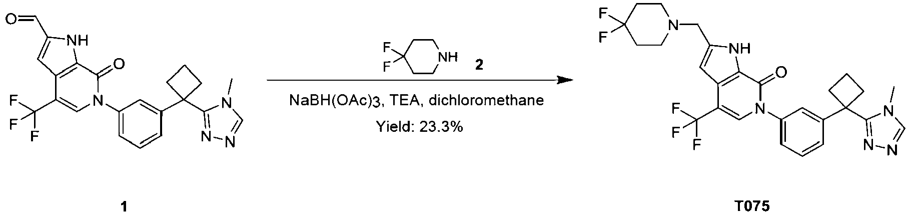

6-[3-[1-(4-methyl-1,2,4-triazol-3-yl)cyclobutyl]phenyl]-7-oxo-4-(trifluoromethyl)-1H-pyrrolo[2,3-c]pyridine-2-carbaldehyde (50.0 mg, 113 µmol) was dissolved in Dichloromethane (1.00 mL), pH was adjust to 7-8 with TEA. 4,4-difluoropiperidine (20.6 mg, 170 µmol) in Dichloromethane 0.50 mL was added to the mixture, stirred at 25°C for 0.5 hr under N₂. Then NaBH(OAc)₃ (60.0 mg, 283 µmol) was added to the mixture, then stirred at 25 °C under N₂ for 2 hrs. LCMS shows reactant was consumed completely and desired mass was detected. The reaction mixture was diluted with Dichloromethane 6.00mL, washed with sat. NaHCO₃ solution 2 mL. The water phase extracted with Dichloromethane 2.00 mL (1.00 mL * 2). The combined organic layers were washed, dried over Na₂SO₄, filtered and concentrated under reduced pressure to give a residue. The residue was purified by prep.HPLC (column: Welch Xtimate C18 40 * 200 mm 7um;mobile phase: [water (HCl)-ACN];gradient: 0% - 40% B over 30 min). 2-[(4,4-difluoro-1-piperidyl)methyl]-6-[3-[1-(4-methyl-1,2,4-triazol-3-yl)cyclobutyl]phenyl]-4-(trifluoromethyl)-1 H-pyrrolo[2,3-c]pyridin-7-one (14.4 mg, 26.4 µmol, 23.3% yield, 100% purity) was obtained as white solid, confirmed by H NMR and LCMS.
¹H NMR: (400 MHz, DMSO-*d*₆)
*δ* 12.78 (s, 1H), 11.39-12.33 (m, 1H), 9.25 (br s, 1H), 7.83 (d, *J =* 1.6 Hz, 1H), 7.54-7.61 (m, 1H), 7.51 (s, 1H), 7.38-7.46 (m, 2H), 6.77 (s, 1H), 4.53 (s, 2H), 3.40 (s, 3H), 3.03-3.34 (m, 4H), 2.92-3.01 (m, 2H), 2.71-2.83 (m, 2H), 2.41 (br s, 4H), 1.95-2.12 (m, 2H)
LCMS: m/z = 547.2 (M+H)⁺, Rt = 1.448 min

### Example 76: Synthesis of compound T076

### 1. General steps for preparation of 2-bromobenzohydrazide

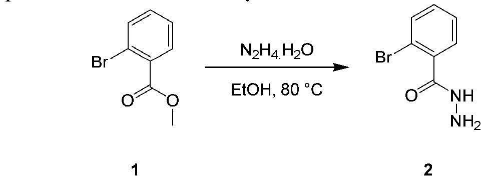

To a solution of methyl 2-bromobenzoate (1.00 g, 4.65 mmol) in EtOH (10.0 mL) was added N₂H₄.H₂O (2.38 g, 46.5 mmol, 2.30 mL, 98.0% purity). The mixture was stirred under N₂ at 80 °C for 12 hrs. TLC (Dichloromethane/ Methanol = 10/ 1, product 1 R_{f} = 0.30) indicated new spot formed. The reaction mixture was concentrated in vacuum. The reaction mixture was diluted with MTBE (10.0 mL). The mixture was stirred under N₂ at 25 °C for 1 hr. The mixture was filtered and the filter cake was washed with MTBE (5.00 mL*3). The filter cake was concentrated in vacuum. To afford 2-bromobenzohydrazide (1.20 g, crude) as a white solid.

### 2. General steps for preparation of 1-[(2-bromobenzoyl)amino]-3-methyl-thiourea

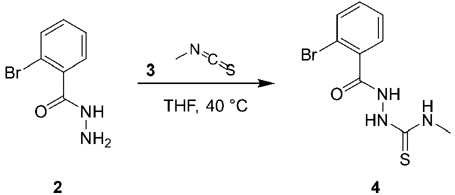

To a solution of 2-bromobenzohydrazide (1.20 g, 5.58 mmol) in THF (12.0 mL) was added methylimino(thioxo)methane (612 mg, 8.37 mmol). The mixture was stirred under N₂ at 40 °C for 2hrs. TLC (Petroleum ether/ Ethyl acetate = 0/ 1, product 1 R_{f} = 0.60) indicated new spot formed. The reaction mixture was concentrated in vacuum. Without purification. To afford 1-[(2-bromobenzoyl)amino]-3-methyl-thiourea (1.30 g, 4.51 mmol, 80.8% yield) as a white solid.

### 3. General steps for preparation of 5-(2-bromophenyl)-4-methyl-1,2,4-triazole-3-thiol

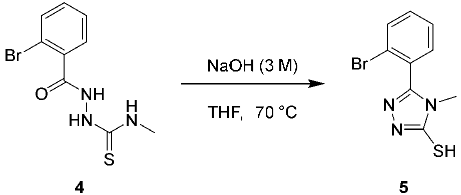

To a solution of 1-[(2-bromobenzoyl)amino]-3-methyl-thiourea (1.30 g, 4.51 mmol) in THF (8.00 mL) was added NaOH (3 M, 4.17 mL). The mixture was stirred under N₂ at 70 °C for 12 hrs. TLC (Petroleum ether/ Ethyl acetate = 0/ 1, product 1 R_{f} = 0.70) indicated new spot formed. The reaction mixture was adjusted pH = 5 (HCl, 1 M) and extracted with Ethyl acetate (20.0 mL*2). The organic phase was dried over Na₂SO₄, filtered and concentrated in vacuum. The reaction mixture was diluted with ACN (3.00 mL). The mixture was stirred under N₂ at 25 °C for 1 hr. The mixture was filtered and the filter cake was washed with ACN (1.00 mL*3). The filter cake was concentrated in vacuum. To afford 5-(2-bromophenyl)-4-methyl-1,2,4-triazole-3-thiol (700 mg, 2.59 mmol, 57.4% yield) as a white solid.

### 4. General steps for preparation of 3-(2-bromophenyl)-4-methyl-1,2,4-triazole

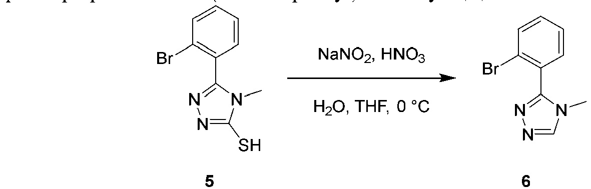

To a solution of 5-(2-bromophenyl)-4-methyl-1,2,4-triazole-3-thiol (700 mg, 2.59 mmol) in THF (3.50 mL) and H₂O (3.50 mL) was added NaNO₂ (1.79 g, 25.9 mmol) and HNO₃ (2.67 g, 27.5 mmol, 1.91 mL, 65.0% purity) at 0 °C slowly. After addition, the mixture was stirred at 0 °C for 2 hrs. TLC (Petroleum ether/ Ethyl acetate = 0/ 1, product 1 R_{f} = 0.20) indicated new spot formed. The reaction mixture was poured into saturated aqueous NaHCO₃ (50.0 mL) slowly, adjust pH = 8 (saturated aqueous NaHCO₃). The mixture was extracted with Ethyl acetate (20.0 mL*2). The organic phase was washed with brine (40.0 mL), dried over Na₂SO₄ and concentrated in vacuum. The crude product was purified by column chromatography (SiO₂, Petroleum ether/ Ethyl acetate = 1/ 0 to 1/ 1). To afford 3-(2-bromophenyl)-4-methyl-1,2,4-triazole (300 mg, 1.26 mmol, 48.6% yield) as a white solid.

### 5. General steps for preparation of 3-[2-(4-methyl-1,2,4-triazol-3-yl)phenyl]aniline

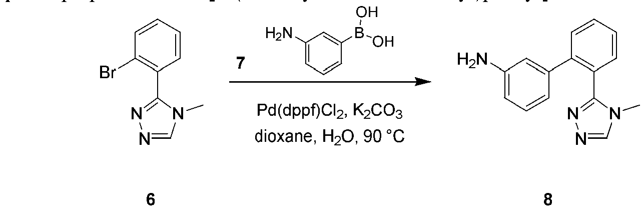

To a solution of 3-(2-bromophenyl)-4-methyl-1,2,4-triazole (300 mg, 1.26 mmol) and (3-aminophenyl)boronic acid (690 mg, 5.04 mmol) in dioxane (8.00 mL) /H₂O (2.00 mL) was added Pd(dppf)Cl₂ (184 mg, 252 µmol) and K₂CO₃ (522 mg, 3.78 mmol). The suspension was degassed under vacuum and purged with N₂ several times. The mixture was stirred under N₂ at 90 °C for 3 hrs. TLC (Dichloromethane/ Methanol = 10/ 1, product 1 R_{f} = 0.40) indicated new spot formed. The reaction mixture was diluted with Ethyl acetate (20.0 mL). The mixture was filtered and the filter cake was washed with Ethyl acetate (10.0 mL*3). The filter was washed with brine (40.0 mL), dried over Na₂SO₄. The organic phase was concentrated in vacuum. The crude product was purified by column chromatography (SiO₂, Dichloromethane/ Methanol = 1/ 0 to 10/ 1). To afford 3-[2-(4-methyl-1,2,4-triazol-3-yl)phenyl]aniline (250 mg, 905 µmol, 71.8% yield, 90.7% purity) as a yellow solid which was confirmed by LCMS.
LCMS: m/z = 250.7 (M+H)⁺, Rt = 0.658 min

### 6. General steps for preparation of 3-[2-(3-bromophenyl)phenyl]-4-methyl-1,2,4-triazole:

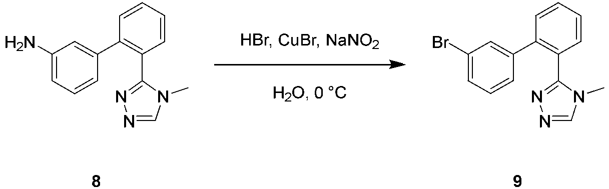

A mixture of 3-[2-(4-methyl-1,2,4-triazol-3-yl)phenyl]aniline (250 mg, 998 µmol) in HBr (2.98 g, 14.7 mmol, 2.00 mL, 40.0% purity) and H₂O (2.0 mL). A solution of NaNO₂ (72.50 mg, 1.05 mmol) in H₂O (0.50 mL) was added dropwise at 0°C. The resulting mixture was stirred at 0 °C for 15 mins. Then a solution of CuBr (129 mg, 899 µmol) in HBr (869 mg, 4.30 mmol, 583 µL, 40.0% purity) was added dropwise at 0 °C. The resulting mixture was stirred at 0°C for 15 mins. TLC (Dichloromethane/ Methanol = 10/ 1, product 1 R_{f} = 0.50) indicated new spot formed. The reaction mixture was diluted with H₂O (10.0 mL), extracted with EtOAc (10.0 mL*2). The organic phase was dried over Na₂SO₄ and concentrated in vacuum. The crude product was purified by column chromatography (SiO₂, Dichloromethane/ Methanol = 1/ 0 to 10/ 1). To afford 3-[2-(3-bromophenyl)phenyl]-4-methyl-1,2,4-triazole (120 mg, 372 µmol, 37.2% yield, 97.4% purity) as a yellow solid which was confirmed by LCMS.
LCMS: m/z = 315.5 (M+H)⁺, Rt = 1.225 min

### 7. General steps for preparation of 2-[[(3S)-3-methyl-1-piperidyl]methyl]-6-[3-[2-(4-methyl-1,2,4-triazol-3-yl)phenyl]phenyl]-1-(p-tolylsulfonyl)-4-(tr ifluoromethyl)pyrrolo[2,3-c]pyridin-7-one

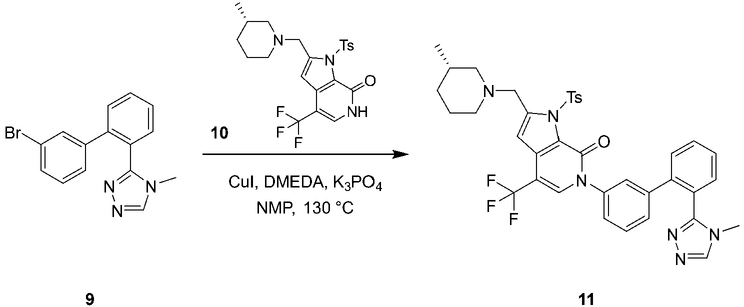

To a solution of 3-[2-(3-bromophenyl)phenyl]-4-methyl-1,2,4-triazole (110 mg, 350 µmol) and 2-[[(3S)-3-methyl-1-piperidyl]methyl]-1-(p-tolylsulfonyl)-4-(trifluoromethyl)-6H-pyrrolo[2,3-c]pyridin-7-one (149 mg, 318 µmol) in NMP (1.50 mL) was added K₃PO₄ (223 mg, 1.05 mmol), CuI (134 mg, 703 µmol) and DMEDA (30.9 mg, 350 µmol, 37.7 µL). The suspension was degassed under vacuum and purged with N₂ several times. The mixture was stirred under N₂ at 130 °C for 2 hrs. TLC (Dichloromethane/ Methanol = 10/ 1, product 1 R_{f} = 0.35) indicated new spot formed. The reaction mixture was poured into saturated aqueous NH₄Cl (10.0 mL) slowly. The mixture was extracted with Ethyl acetate (10.0 mL*2). The organic phase was washed with brine (20.0 mL*2), dried over Na₂SO₄ and concentrated in vacuum. The crude product was purified by column chromatography (SiO₂, DCM/ MeOH = 1/ 0 to 15/ 1). To afford 2-[[(3S)-3-methyl-1-piperidyl]methyl]-6-[3-[2-(4-methyl-1,2,4-triazol-3-yl)phenyl]phenyl]-1-(p-tolylsulfonyl)-4-(tr ifluoromethyl)pyrrolo[2,3-c]pyridin-7-one (100 mg, 58.5 µmol, 16.7% yield, 41.0% purity) as a yellow solid which was confirmed by LCMS.
LCMS: m/z = 350.8 (M+H)⁺, Rt = 1.315 min

### 8. General steps for preparation of 2-[[(3S)-3-methyl-1-piperidyl]methyl]-6-[3-[2-(4-methyl-1,2,4-triazol-3-yl)phenyl]phenyl]-4-(trifluoromethyl)-1H-pyrrolo[2,3-c]pyridin-7-one (compound T076)

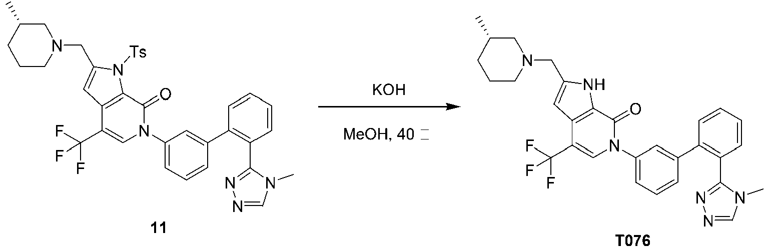

To a solution of 2-[[(3S)-3-methyl-1-piperidyl]methyl]-6-[3-[2-(4-methyl-1,2,4-triazol-3-yl)phenyl]phenyl]-1-(p-tolylsulfonyl)-4-(tr ifluoromethyl)pyrrolo[2,3-c]pyridin-7-one (100 mg, 142 µmol) in MeOH (5.00 mL) was added KOH (240 mg, 4.28 mmol). The mixture was stirred under N₂ at 40 °C for 2hrs. LCMS showed desired mass was detected. The reaction mixture was diluted with brine (20.0 mL), extracted with EtOAc (20.0 mL*2). The organic phase washed with KOH (1.00 M, 20.0 mL), dried over Na₂SO₄ and concentrated in vacuum. The crude product was purified by Prep-HPLC (column: Xtimate C18 150*40 mm*10 um; mobile phase: [water (NH₃H₂O+NH₄HCO₃) - ACN]; gradient: 36.0%-76.0% B over 32 mins). To afford 2-[[(3S)-3-methyl-1-piperidyl]methyl]-6-[3-[2-(4-methyl-1,2,4-triazol-3-yl)phenyl]phenyl]-4-(triffuoromethyl)-1H-pyrrolo[2,3-c]pyridin-7-one (21.9 mg, 39.4 µmol, 27.6% yield, 98.4% purity) as a yellow solid which was confirmed by H NMR and LCMS.
¹H NMR: (400 MHz, DMSO-*d*₆)
*δ* 12.42 (s, 1H), 8.40 (s, 1H), 7.70 (q, *J =* 3.6 Hz, 2H), 7.59 (d, *J =* 3.6 Hz, 2H), 7.51 (d, *J =* 1.2 Hz, 1H), 7.41-7.48 (m, 2H), 7.21 (s, 1H), 7.16-7.19 (m, 1H), 6.27 (s, 1H), 3.59 (s, 2H), 3.07 (s, 3H), 2.72-2.79 (m, 2H), 1.84-1.93 (m, 1H), 1.55-1.66 (m, 4H), 1.41-1.50 (m, 1H), 0.81 (d, *J =* 5.6 Hz, 3H), 0.75 (s, 1H)
LCMS: m/z = 547.0 (M+H)⁺, Rt = 1.145 min

### Example 77: Synthesis of compound T077

### 1. General steps for preparation of ethyl 2-(3-benzyloxycyclobutylidene)acetate

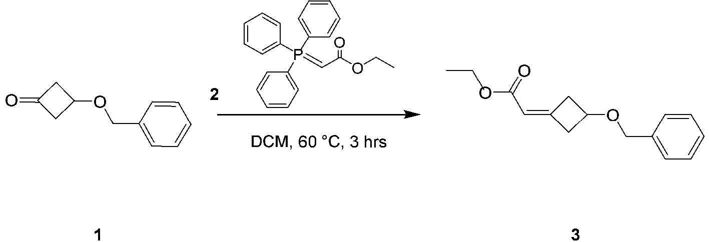

A mixture of 3-benzyloxycyclobutanone (9.59 g, 54.4 mmol), ethyl 2-(triphenyl-phosphanylidene)acetate (15.8 g, 45.3 mmol) in DCM (150 mL) was degassed and purged with N₂ for 3 times, and then the mixture was stirred at 60 °C for 3 hrs under N₂ atmosphere. LCMS showed desired compound was detected. The reaction mixture was diluted with H₂O (30.0 mL), extracted with DCM 150 mL, the combined organic layers were washed with H₂O 60.0 mL, dried over Na₂SO₄, filtered and concentrated under reduced pressure to give a residue. The residue was purified by column chromatography (SiO₂, PE/ EA = 0% to 8%), (Plate 1, Petroleum ether/ Ethyl acetate = 15/ 1, R_{f} (product) = 0.45). Compound ethyl 2-(3-benzyloxycyclobutylidene)acetate (9.80 g, 39.0 mmol, 86.1% yield, 98.2% purity) was obtained as a yellow oil. Confirmed by H NMR and LCMS.
¹H NMR: (DMSO-*d*₆, 400 MHz)
*δ* 7.33-7.35 (m, 3H), 7.25-7.33 (m, 2H), 5.70 (dt, *J =* 4.4, 2.4 Hz, 1H), 4.48 (s, 2H), 4.21 (q, *J =* 6.0 Hz, 1H), 4.05-4.17 (m, 2H), 3.37-3.51 (m, 1H), 3.02-3.12 (m, 1H), 2.97 (ddt, *J =* 17.6, 5.6, 2.8 Hz, 1H), 2.78-2.89 (m, 1H), 1.25 (t, *J =* 7.2 Hz, 3H)
LCMS: m/z = 246.7 (M+H)⁺, Rt = 1.787 min

### 2. General steps for preparation of ethyl 2-[3-benzyloxy-1-(3-bromophenyl)cyclobutyl]acetate

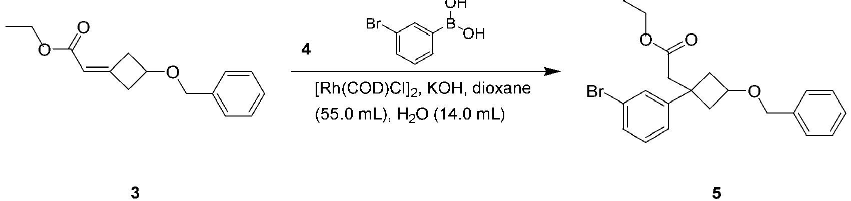

To a solution of [Rh(COD)Cl]₂ (1.12 g, 2.27 mmol) in dioxane (55.0 mL) was added aqueous KOH (1.5 M, 14.0 mL) and the mixture was stirred for 30.0 mins, then add ethyl 2-(3-benzyloxycyclobutylidene)acetate (11.2 g, 45.4 mmol) to the mixture. (3-bromophenyl)boronic acid (14.6 g, 72.7 mmol) was added to the mixture in ten potions during 30.0 mins. The mixture was stirred at 25 °C for 2 hrs. LCMS showed desired compound was detected. The reaction mixture was diluted with H₂O (30.0 mL), extracted with Ethyl acetate (20.0 mL * 3), the combined organic layers were washed with H₂O (20.0 mL * 3), dried over Na₂SO₄, filtered and concentrated under reduced pressure to give a residue. The residue was purified by column chromatography (SiO₂, Petroleum ether/ Ethyl acetate = 0% to 6.00%), (Plate 1, Petroleum ether/ Ethyl acetate = 10/ 1, R_{f} (product) = 0.34). Compound ethyl 2-[3-benzyloxy-1-(3-bromophenyl)cyclobutyl]acetate (8.80 g, 16.8 mmol, 36.9% yield, 77.0% purity) was obtained as a white solid. Confirmed by H NMR and LCMS.
¹H NMR: (CD₃OD, 400 MHz)
*δ* 7.34 (br s, 1H), 7.29-7.33 (m, 6H), 7.20 (t, *J =* 7.6 Hz, 1H), 7.10-7.15 (m, 1H), 4.44 (s, 2H), 4.27 (q, *J* = 7.2 Hz, 1H), 3.89-3.93 (m, 2H), 2.82-2.88 (m, 2H), 2.71 (s, 2H), 2.20-2.32 (m, 2H), 1.02-1.07 (m, 3H)
LCMS: m/z = 404.8 (M+H)⁺, Rt = 1.430 min

### 3. General steps for preparation of ethyl 2-[1-(3-bromophenyl)-3-hydroxy-cyclobutyl]acetate

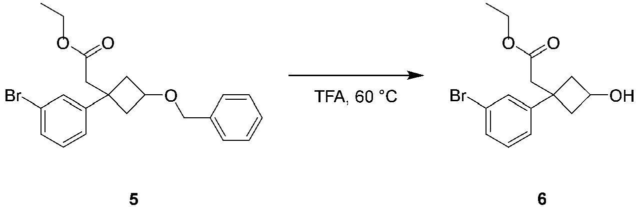

A mixture of ethyl 2-[3-benzyloxy-1-(3-bromophenyl)cyclobutyl]acetate (1.00 g, 2.48 mmol) in TFA (7.68 g, 67.3 mmol, 5.00 mL) was degassed and purged with N₂ for 3 times, and then the mixture was stirred at 60 °C for 12 hrs under N₂ atmosphere. LCMS showed desired compound was detected. The reaction mixture was diluted with H₂O (30.0 mL), extracted with Ethyl acetate (20.0 mL * 3), the combined organic layers were washed with H₂O (20.0 mL * 3), dried over Na₂SO₄, filtered and concentrated under reduced pressure to give a residue. The residue was purified by column chromatography (SiO₂, Petroleum ether/ Ethyl acetate = 0% to 4.00 %) (Plate 1, Petroleum ether/ Ethyl acetate = 10/ 1, R_{f} (product) = 0.66). Compound ethyl 2-[1-(3-bromophenyl)-3-hydroxy-cyclobutyl]acetate (350 mg, crude) was obtained as a yellow oil. Confirmed by H NMR and LCMS.
¹H NMR: (DMSO-*d*₆, 400 MHz)
*δ* 7.33-7.43 (m, 2H), 7.27 (t, *J =* 7.6 Hz, 1H), 7.14-7.22 (m, 1H), 5.46 (q, *J =* 7.2 Hz, 1H), 3.84-3.92 (m, 2H), 2.98 (m, *J =* 10.4 Hz, 2H), 2.87 (s, 2H), 2.64-2.78 (m, 1H), 2.52-2.56 (m, 2H), 1.00 (t*, J =* 7.2 Hz, 3H)
LCMS: m/z = 296.8 (M-OH)⁺, Rt = 1.313 min

### 4. General steps for preparation of ethyl 2-[1-(3-bromophenyl)-3-oxo-cyclobutyl]acetate

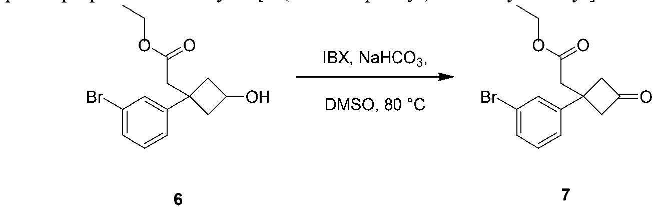

A mixture of ethyl 2-[1-(3-bromophenyl)-3-hydroxy-cyclobutyl]acetate (5.27 g, 16.8 mmol), NaHCO₃ (3.53 g, 42.0 mmol, 1.64 mL), 2-iodylbenzoic acid (7.07 g, 25.2 mmol) in DMSO (50.0 mL) was degassed and purged with N₂ for 3 times, and then the mixture was stirred at 80 °C for 4 hrs under N₂ atmosphere. LCMS showed desired compound was detected. The reaction mixture was diluted with aqueous Na₂SO₃ (30.0 mL), extracted with Ethyl acetate (20.0 mL * 3), the combined organic layers were washed with H₂O (20.0 mL * 3), dried over Na₂SO₄, filtered and concentrated under reduced pressure to give a residue. The residue was purified by column chromatography (SiO₂, Petroleum ether/ Ethyl acetate = 5.00% to 12.0%), (Plate 1, Petroleum ether/ Ethyl acetate = 5/ 1, R_{f} (product) = 0.26). Compound ethyl 2-[1-(3-bromophenyl)-3-oxo-cyclobutyl]acetate (2.34 g, 7.11 mmol, 42.2% yield, 94.6% purity) was obtained as a colorless oil. Confirmed by H NMR and LCMS.
¹H NMR: (DMSO-*d*₆, 400 MHz)
*δ* 7.54 (s, 1H), 7.45 (br d, *J =* 7.6 Hz, 1H), 7.34-7.38 (m, 1H), 7.28-7.33 (m, 1H), 3.92 (q, *J =* 7.2 Hz, 2H), 3.45-3.52 (m, 2H), 3.35-3.41 (m, 2H), 2.95 (s, 2H), 1.03 (t, *J =* 7.2 Hz, 3H)
LCMS: m/z = 310.9 (M+H)⁺, Rt = 0.787 min

### 5. General steps for preparation of ethyl 2-[1-(3-bromophenyl)-3,3-difluoro-cyclobutyl]acetate

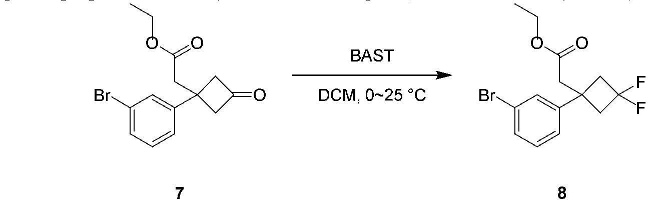

To a solution of ethyl 2-[1-(3-bromophenyl)-3-oxo-cyclobutyl]acetate (500 mg, 1.61 mmol) in DCM (5.00 mL) was added dropwise BAST (5.33 g, 24.1 mmol, 5.28 mL) in Dichloromethane (5.00 mL) at 0 °C. After addition, the mixture was stirred at 25 °C for 12 hrs. LCMS showed desired compound was detected. The reaction mixture was quenched by aqueous NaHCO₃ (15.0 mL), stirred for 15.0 mins, extracted with Ethyl acetate (20.0 mL * 3), the combined organic layers were washed with H₂O (20.0 mL * 3), dried over Na₂SO₄, filtered and concentrated under reduced pressure to give a residue. The residue was purified by column chromatography (SiO₂, Petroleum ether/ Ethyl acetate = 0% to 15.0%), (Plate 1, Petroleum ether/ Ethyl acetate = 5/ 1, R_{f} (product) = 0.65). Compound ethyl 2-[1-(3-bromophenyl)-3,3-difluoro-cyclobutyl]acetate (278 mg, crude) was obtained as a colorless oil. Confirmed by H NMR.
¹H NMR: (DMSO-*d*₆, 400 MHz)
*δ* 7.48 (s, 1H), 7.41-7.45 (m, 1H), 7.30 (d, *J =* 5.2 Hz, 2H), 3.88 (q, *J =* 7.2 Hz, 2H), 3.02 (t, *J =* 12.4 Hz, 4H), 2.87 (s, 2H), 1.00 (t, *J =* 7.2 Hz, 3H)

### 6. General steps for preparation of 2-[1-(3-bromophenyl)-3,3-difluoro-cyclobutyl]acetohydrazide

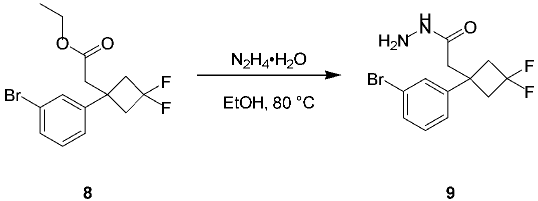

To a solution of ethyl 2-[1-(3-bromophenyl)-3,3-difluoro-cyclobutyl]acetate (278 mg, 834 µmol) in EtOH (3.00 mL) was added dropwise N₂H₄.H₂O (1.04 g, 20.3 mmol, 1.01 mL, 98.0% purity) at 25 °C. The resulting mixture was stirred at 80 °C for 8 hrs. LCMS showed desired compound was detected. The reaction mixture was quenched by aqueous Na₂SO₃ (20.0 mL), stirred for 15.0 mins. Diluted with H₂O (30.0 mL), extracted with Ethyl acetate (20.0 mL * 3), the combined organic layers were washed with H₂O (20.0 mL * 3), dried over Na₂SO₄, filtered and concentrated under reduced pressure to give a residue. Compound 2-[1-(3-bromophenyl)-3,3-difluoro-cyclobutyl]acetohydrazide (281 mg, crude) was obtained as a yellow oil. Confirmed by LCMS.
LCMS: m/z = 318.8 (M+H)⁺, Rt = 1.438 min

### 7. General steps for preparation of 1-[[2-[1-(3-bromophenyl)-3,3-difluoro-cyclobutyl]acetyl]amino]-3-methyl-thiourea

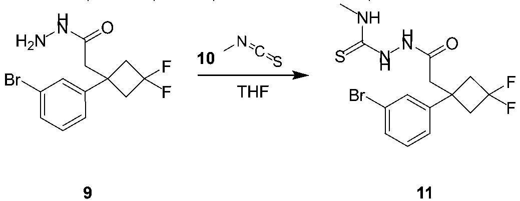

A mixture of 2-[1-(3-bromophenyl)-3,3-difluoro-cyclobutyl]acetohydrazide (266 mg, 833 µmol), methylimino(thioxo)methane (121 mg, 1.67 mmol, 114 µL) in THF (3.00 mL) was degassed and purged with N₂ for 3 times, and then the mixture was stirred at 25 °C for 4 hrs under N₂ atmosphere. LCMS showed desired compound was detected. Concentrated under reduced pressure to give a residue. Compound 1-[[2-[1-(3-bromophenyl)-3,3-difluoro-cyclobutyl]acetyl]amino]-3-methyl-thiourea (282 mg, crude) was obtained as a yellow solid.

### 8. General steps for preparation of 5-[[1-(3-bromophenyl)-3,3-difluoro-cyclobutyl]methyl]-4-methyl-1,2,4-triazole-3-thiol

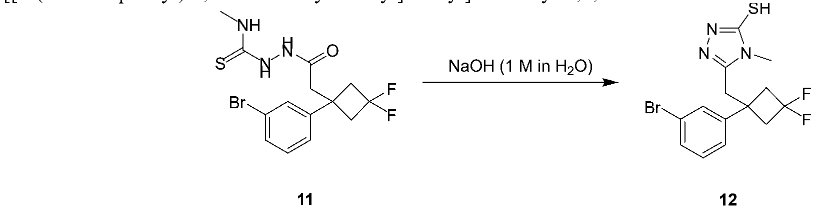

To a solution of 1-[[2-[1-(3-bromophenyl)-3,3-difluoro-cyclobutyl]acetyl]amino]-3-methyl-thiourea (182 mg, 463 µmol) in NaOH (1 M, 3.64 mL) was stirred at 25 °C for 4 hrs under N₂. LCMS showed desired compound was detected. The reaction mixture was diluted with water, then the pH value of the solution was adjust to 1 with HCl (1 M, 12.0 mL) and extracted with Ethyl acetate (40.0 mL * 3), the combined organic layers were washed with H₂O (50.0 mL * 3), dried over Na₂SO₄, filtered and concentrated under reduced pressure to give a residue. Compound 5-[[1-(3-bromophenyl)-3,3-difluoro-cyclobutyl]methyl]-4-methyl-1,2,4-triazole-3-thiol (174 mg, crude) was obtained as a white solid.

### 9. General steps for preparation of 3-[[1-(3-bromophenyl)-3,3-difluoro-cyclobutyl]methyl]-4-methyl-1,2,4-triazole

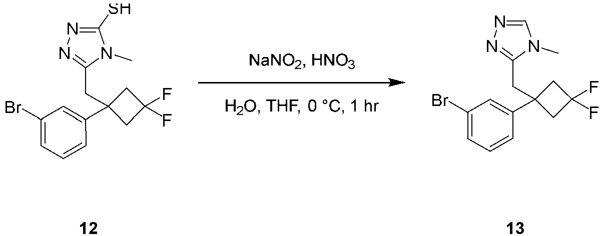

To a solution of 5-[[1-(3-bromophenyl)-3,3-difluoro-cyclobutyl]methyl]-4-methyl-1,2,4-triazole-3-thiol (270 mg, 721 µmol) in THF (1.50 mL) and H₂O (1.50 mL) was added NaNO₂ (497 mg, 7.21 mmol), HNO₃ (734 mg, 7.58 mmol, 524 µL, 65.0% purity) was added dropwise to the mixture. After addition, the mixture was stirred at 0 °C for 1 hr. LCMS showed desired compound was detected. The mixture was basified by saturated aqueous sodium bicarbonate solution then extracted with Ethyl acetate (60.0 mL * 3). The combined organic layers were dried over Na₂SO₄, filtered and concentrated under reduced pressure to give a residue. The residue was purified by column chromatography (SiO₂, Petroleum ether/ Ethyl acetate = 0% to 4.00%), (Plate 1, Petroleum ether/ Ethyl acetate = 20/ 1, R_{f} (product) = 0.34). Compound 3-[[1-(3-bromophenyl)-3,3-difluoro-cyclobutyl]methyl]-4-methyl-1,2,4-triazole (160 mg, crude) was obtained as a colorless oil. Confirmed by H NMR and LCMS.
¹H NMR: (DMSO-*d*₆, 400 MHz)
*δ* 8.22 (s, 1H), 7.43 (dd, *J* = 7.6, 0.8 Hz, 1H), 7.20-7.25 (m, 2H), 7.00 (d, *J* = 7.6 Hz, 1H), 3.22 (s, 2H), 3.15-3.21 (m, 2H), 2.93-3.03 (m, 2H), 2.81 (s, 3H)
LCMS: m/z =343.8 (M+H)⁺, Rt = 1.367 min

### 10. General steps for preparation of 4-cyclopropyl-6-[3-[3,3-difluoro-1-[(4-methyl-1,2,4-triazol-3-yl)methyl]cyclobutyl]phenyl]-2-[[(3S)-3-methyl-1-pi peridyl]methyl]-1-(p-tolylsulfonyl)pyrrolo[2,3-c]pyridin-7-one

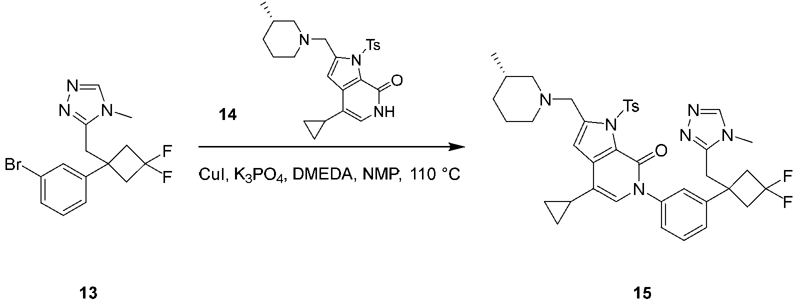

A mixture of 3-[[1-(3-bromophenyl)-3,3-difluoro-cyclobutyl]methyl]-4-methyl-1,2,4-triazole (50.0 mg, 146 µmol), 4-cyclopropyl-2-[[(3S)-3-methyl-1-piperidyl]methyl]-1-(p-tolylsulfonyl)-6H-pyrrolo[2,3-c]pyridin-7-one (64.2 mg, 146 µmol), CuI (27.8 mg, 146 µmol), K₃PO₄ (93.0 mg, 438 µmol) and DMEDA (25.7 mg, 292 µmol, 31.4 µL) in NMP (1.00 mL) was degassed and purged with N₂ for 3 times, and then the mixture was stirred at 110 °C for 2 hrs under N₂ atmosphere. LCMS showed desired compound was detected. The reaction mixture was filtered and diluted with H₂O (30.0 mL), extracted with Ethyl acetate (20.0 mL * 3), the combined organic layers were washed with H₂O (20.0 mL * 3), dried over Na₂SO₄, filtered and concentrated under reduced pressure to give a residue. The residue was purified by column chromatography (SiO₂, Dichloromethane/ Methyl alcohol = 0% to 4.00%), (Plate 1, Dichloromethane/ Methyl alcohol = 10/ 1, R_{f} (product) = 0.5). Compound 4-cyclopropyl-6-[3-[3,3-difluoro-1-[(4-methyl-1,2,4-triazol-3-yl)methyl]cyclobutyl]phenyl]-2-[[(3S)-3-methyl-1-pi peridyl]methyl]-1-(p-tolylsulfonyl)pyrrolo[2,3-c]pyridin-7-one (103 mg, 132 µmol, 90.5% yield, 90.3% purity) was obtained as a colorless oil. Confirmed by LCMS.
LCMS: m/z =701.2 (M+H)⁺, Rt = 1.417 min

### 11. General steps for preparation of 4-cyclopropyl-6-[3-[3,3-difluoro-1-[(4-methyl-1,2,4-triazol-3-yl)methyl]cyclobutyl]phenyl]-2-[[(3S)-3-methyl-1-pi peridyl]methyl]-1H-pyrrolo[2,3-c]pyridin-7-one (compound T077)

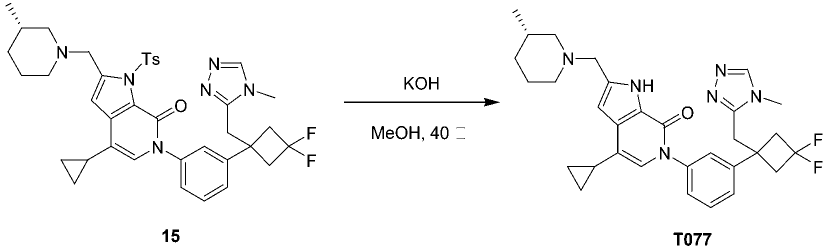

A mixture of 4-cyclopropyl-6-[3-[3,3-difluoro-1-[(4-methyl-1,2,4-triazol-3-yl)methyl]cyclobutyl]phenyl]-2-[[(3S)-3-methyl-1-pi peridyl]methyl]-1-(p-tolylsulfonyl)pyrrolo[2,3-c]pyridin-7-one (103 mg, 146 µmol), KOH (164 mg, 2.94 mmol) in Methyl alcohol (4.00 mL) was degassed and purged with N₂ for 3 times, and then the mixture was stirred at 25 °C for 1 hr. LCMS showed desired compound was detected. The reaction mixture was diluted with brine (30.0 mL), extracted with Ethyl acetate (20.0 mL * 3), the combined organic layers were washed with aqueous KOH (20.0 mL * 2), dried over Na₂SO₄, filtered and concentrated under reduced pressure to give a residue. The residue was purified by Prep-HPLC (column: Xtimate C18 150 * 40 mm * 10 um; mobile phase: [water (HCl)-ACN]; gradient: 2.00%-42.0% B over 36.0 min). Compound 4-cyclopropyl-6-[3-[3,3-difluoro-1-[(4-methyl-1,2,4-triazol-3-yl)methyl]cyclobutyl|phenyl]-2-[[(3S)-3-methyl-1-pi peridyl]methyl]-1H-pyrrolo[2,3-c]pyridin-7-one (19.5 mg, 34.1 µmol, 23.2% yield, 95.7% purity) was obtained as a white solid. Confirmed by H NMR and LCMS.
¹H NMR: (DMSO-*d*₆, 400 MHz)
*δ* 12.32 (s, 1H), 9.36 (s, 1H), 7.41-7.48 (m, 1H), 7.32 (d, *J =* 7.6 Hz, 1H), 7.10-7.19 (m, 2H), 6.75-6.85 (m, 2H), 4.39 (d, *J =* 3.2 Hz, 3H), 3.56 (s, 2H), 3.32 (d, *J* = 9.6 Hz, 3H), 3.25 (d, *J =* 11.4 Hz, 1H), 3.08-3.16 (m, 2H), 3.06 (s, 3H), 2.71-2.78 (m, 1H), 2.40-2.48 (m, 1H), 1.99-2.08 (m, 1H), 1.89-1.89 (m, 1H), 1.87-1.91 (m, 1H), 1.81 (s, 1H), 1.71 (d, *J =* 12.0 Hz, 1H), 0.87 (d, *J =* 6.4 Hz, 3H), 0.79-0.85 (m, 2H), 0.66-0.75 (m, 2H)
LCMS: m/z =547.3 (M+H)⁺, Rt = 2.223 min

### Examples 78 and 79: Synthesis of compounds T078& T079

### 1. General steps for preparation of 4-bromo-2-[(4,4-difluoro-3-methyl-1-piperidyl)methyl]-7-methoxy-1-(p-tolylsulfonyl)pyrrolo[2,3-c]pyridine

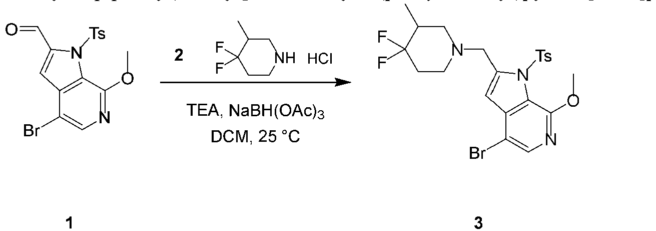

To a solution of 4,4-difluoro-3-methyl-piperidine (745 mg, 4.34 mmol, HCl) in DCM (20.0 mL) was added dropwise TEA (1.46 g, 14.4 mmol, 2.01 mL) and adjust pH = 8, then 4-bromo-7-methoxy-1-(p-tolylsulfonyl)pyrrolo[2,3-c]pyridine-2-carbaldehyde (1.18 g, 2.89 mmol) was added to the mixture and stirred at 25 °C under N₂ for 0.5 hr. NaBH(OAc)₃ (1.53 g, 7.24 mmol) was added to the mixture in 3 points during 1.5 hrs. The resulting mixture was stirred at 25 °C for 2 hrs. LCMS showed desired compound was detected. The reaction mixture was diluted with H₂O (30.0 mL), extracted with DCM (20.0 mL * 3), the combined organic layers were washed with H₂O (20.0 mL * 3), dried over Na₂SO₄, filtered and concentrated under reduced pressure to give a residue. The residue was purified by column chromatography (SiO₂, PE/ EA = 0% to 4%), (Plate 1, PE/ EA = 5/ 1, R_{f} (product) = 0.41). Compound 4-bromo-2-[(4,4-difluoro-3-methyl-1-piperidyl)methyl]-7-methoxy-1-(p-tolylsulfonyl)pyrrolo[2,3-c]pyridine (1.36 g, 2.33 mmol, 80.4% yield, 90.5% purity) was obtained as a white solid. Confirmed by H NMR and LCMS.
¹H NMR: (DMSO-*d*₆, 400 MHz)
*δ* 8.19 (d, *J* = 8.4 Hz, 2H), 7.98-8.04 (m, 1H), 7.47 (d, *J =* 8.0 Hz, 2H), 6.83 (s, 1H), 4.03 (s, 2H), 3.75 (s, 3H), 2.78-2.86 (m, 2H), 2.40 (s, 3H), 2.32-2.38 (m, 1H), 2.01-2.15 (m, 3H), 1.81-1.96 (m, 1H), 0.92 (d, *J =* 6.4 Hz, 3H)
LCMS: m/z = 527.9 (M+H)⁺, Rt = 0.957 min

### 2. General steps for preparation of 4-bromo-2-[(4,4-difluoro-3-methyl-1-piperidyl)methyl]-7-methoxy-1-(p-tolylsulfonyl)pyrrolo[2,3-c]pyridine

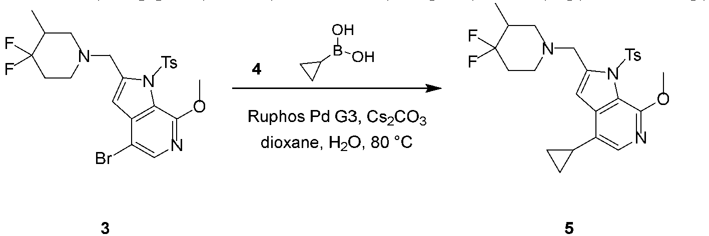

To a solution of4-bromo-2-[(4,4-difluoro-3-methyl-1-piperidyl)methyl]-7-methoxy-1-(p-tolylsulfonyl)pyrrolo[2,3-c]pyridine (1.31 g, 2.48 mmol) and cyclopropylboronic acid (2.13 g, 24.7 mmol) in dioxane (12.0 mL) and H₂O (3.00 mL) was added RuPhos Pd G3 (414 mg, 495 µmol) and Cs₂CO₃ (2.42 g, 7.44 mmol) under N₂. After addition, the mixture was stirred at 80 °C for 2 hrs under N₂. LCMS showed desired compound was detected. The reaction mixture was diluted with H₂O (30.0 mL), extracted with EtOAc (20.0 mL * 3), the combined organic layers were washed with H₂O (20.0 mL * 3), dried over Na₂SO₄, filtered and concentrated under reduced pressure to give a residue. The residue was purified by column chromatography (SiO₂, PE/ EA = 5% to 13%), (Plate 1, PE/ EA = 5/ 1, R_{f} (product) = 0.45). Compound 4-cyclopropyl-2-[(4,4-difluoro-3-methyl-1-piperidyl)methyl]-7-methoxy-1-(p-tolylsulfonyl)pyrrolo[2,3-c]pyridine (1.02 g, 1.79 mmol, 72.1% yield, 85.8% purity) was obtained as a white solid. Confirmed by H NMR and LCMS.
¹H NMR: (CD₃OD, 400 MHz)
*δ* 8.15 (d, *J =* 8.4 Hz, 2H), 7.50 (s, 1H), 7.39 (d, *J =* 8.0 Hz, 2H), 6.90 (s, 1H), 4.61 (s, 1H), 4.05 (s, 2H), 3.74 (s, 3H), 2.79-2.98 (m, 2H), 2.44 (s, 3H), 1.86-2.19 (m, 5H), 0.97-1.04 (m, 3H), 0.97 (s, 2H), 0.67-0.72 (m, 2H)
LCMS: m/z =490.4 (M+H)⁺, Rt = 1.600 min

### 3. General steps for preparation of 4-cyclopropyl-2-[(4,4-difluoro-3-methyl-1-piperidyl)methyl]-1-(p-tolylsulfonyl)-6H-pyrrolo[2,3-c]pyridin-7-one

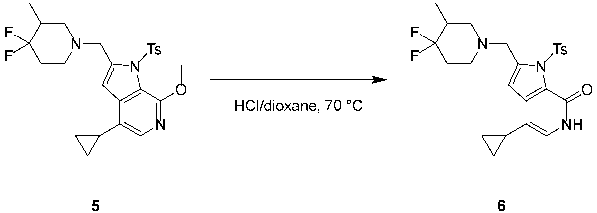

A mixture of 4-cyclopropyl-2-[(4,4-difluoro-3-methyl-1-piperidyl)methyl]-7-methoxy-1-(p-tolylsulfonyl)pyrrolo[2,3-c]pyridine (1.02 g, 2.08 mmol), HCl/ dioxane (4 M, 5.00 mL) was degassed and purged with N₂ for 3 times, and then the mixture was stirred at 70 °C for 1 hr under N₂ atmosphere. LCMS showed desired compound was detected. The reaction mixture was diluted with aqueous NaHCO₃ (15.0 mL), extracted with EtOAc (20.0 mL * 3), the combined organic layers were washed with H₂O (20.0 mL * 3), dried over Na₂SO₄, filtered and concentrated under reduced pressure to give a residue. The residue was purified by column chromatography (SiO₂, DCM/ MeOH = 0% to 4%), (Plate 1, DCM/ MeOH = 10/ 1, R_{f} (product) = 0.54). Compound 4-cyclopropyl-2-[(4,4-difluoro-3-methyl-1-piperidyl)methyl]-1-(p-tolylsulfonyl)-6H-pyrrolo[2,3-c]pyridin-7-one (851 mg, 1.64 mmol, 78.9% yield, 91.9% purity) was obtained as a white solid. Confirmed by H NMR and LCMS.
¹H NMR: (DMSO-*d*₆, 400 MHz)
*δ* 10.93 (br d, *J =* 5.6 Hz, 1H), 8.25 (d, *J =* 8.4 Hz, 2H), 7.38 (d, *J =* 8.0 Hz, 2H), 6.65-6.83 (m, 2H), 3.95 (s, 2H), 2.77-2.85 (m, 2H), 2.38 (s, 3H), 2.29-2.34 (m, 1H), 1.99-2.17 (m, 3H), 1.73-1.90 (m, 2H), 0.92 (d, *J =* 6.4 Hz, 3H), 0.73-0.82 (m, 2H), 0.43-0.54 (m, 2H)
LCMS: m/z =476.2 (M+H)⁺, Rt = 1.247 min

### 4. General steps for preparation of 4-cyclopropyl-2-[(4,4-difluoro-3-methyl-1-piperidyl)methyl]-6-[3-[3-[(4-methyl-1,2,4-triazol-3-yl)methyl]oxetan-3 -yl]phenyl]-1-(p-tolylsulfonyl)pyrrolo[2,3-c]pyridin-7-one

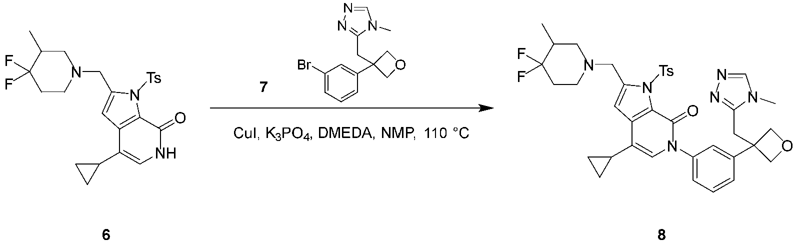

To a solution of 4-cyclopropyl-2-[(4,4-difluoro-3-methyl-1-piperidyl)methyl]-1-(p-tolylsulfonyl)-6H-pyrrolo[2,3-c]pyridin-7-one (200 mg, 420 µmol), 3-[[3-(3-bromophenyl)oxetan-3-yl]methyl]-4-methyl-1,2,4-triazole (129 mg, 420 µmol), CuI (80.1 mg, 420 µmol), K₃PO₄ (267 mg, 1.26 mmol) in NMP (2.00 mL) was added dropwise N,N'-dimethylethane-1,2-diamine (74.1 mg, 841 µmol, 90.5 µL). After addition, the mixture was stirred at 110 °C for 4 hrs. LCMS showed desired compound was detected. The reaction mixture was diluted with brine (30.0 mL), extracted with EtOAc (20.0 mL * 3), the combined organic layers were washed with brine (20.0 mL * 3), dried over Na₂SO₄, filtered and concentrated under reduced pressure to give a residue. The residue was purified by column chromatography (SiO₂, DCM/ MeOH = 0% to 10%), (Plate 1, DCM/ MeOH = 20/ 1, R_{f} (product) = 0.45). Compound 4-cyclopropyl-2-[(4,4-difluoro-3-methyl-1-piperidyl)methyl]-6-[3-[3-[(4-methyl-1,2,4-triazol-3-yl)methyl]oxetan-3 -yl]phenyl]-1-(p-tolylsulfonyl)pyrrolo[2,3-c]pyridin-7-one (215 mg, 275 µmol, 65.5% yield, 90.1% purity) was obtained as a white solid. Confirmed by H NMR and LCMS.
¹H NMR: (DMSO-*d*₆, 400 MHz)
*δ* 8.20-8.29 (m, 2H), 8.08 (s, 1H), 7.33-7.42 (m, 3H), 7.14 (dd, *J =* 8.0, 1.2 Hz, 1H), 7.01 (br d, *J =* 7.6 Hz, 1H), 6.87 (s, 2H), 6.70 (s, 1H), 4.91 (d, *J =* 6.0 Hz, 2H), 4.84 (d, *J =* 6.0 Hz, 2H), 3.88-4.13 (m, 2H), 3.48 (s, 2H), 2.93 (s, 3H), 2.79-2.91 (m, 2H), 2.63-2.70 (m, 1H), 2.36 (s, 3H), 2.00-2.23 (m, 3H), 1.76-1.98 (m, 2H), 0.94 (br d, *J* = 6.0 Hz, 3H), 0.76-0.86 (m, 2H), 0.55-0.66 (m, 2H)
LCMS: m/z = 703.1 (M+H)⁺, Rt = 1.175 min

### 5. General steps for preparation of 4-cyclopropyl-2-[(4,4-difluoro-3-methyl-1-piperidyl)methyl]-6-[3-[3-[(4-methyl-1,2,4-triazol-3-yl)methyl]oxetan-3 -yl]phenyl]-1H-pyrrolo[2,3-c]pyridin-7-one

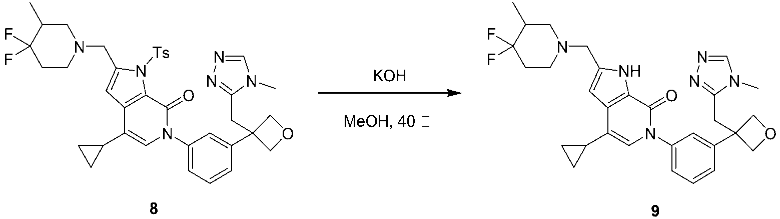

To a solution of 4-cyclopropyl-2-[(4,4-difluoro-3-methyl-1-piperidyl)methyl]-6-[3-[3-[(4-methyl-1,2,4-triazol-3-yl)methyl]oxetan-3 -yl]phenyl]-1-(p-tolylsulfonyl)pyrrolo[2,3-c]pyridin-7-one (210 mg, 298 µmol) in MeOH (8.00 mL) was added KOH (335 mg, 5.98 mmol). After addition, the mixture was stirred at 40 °C for 1 hr. LCMS showed desired compound was detected. The reaction mixture was dilut with aqueous NaHCO₃ (15.0 mL) adjust pH = 7, filtered and concentrated under reduced pressure to give a residue. The residue was purified by Prep-HPLC (column: Xtimate C18 150 * 40 mm * 10 um; mobile phase: [water (NH₄HCO₃)-ACN]; gradient: 22% - 62% B over 30 min). Compound 4-cyclopropyl-2-[(4,4-difluoro-3-methyl-1-piperidyl)methyl]-6-[3-[3-[(4-methyl-1,2,4-triazol-3-yl)methyl]oxetan-3 -yl]phenyl]-1H-pyrrolo[2,3-c]pyridin-7-one (90.0 mg, 163 µmol, 54.7% yield, 99.72% purity) was obtained as a white solid. 10.0 mg was deliver, 80.0 mg for next step. Confirmed by H NMR and LCMS.
¹H NMR: (DMSO-*d*₆, 400 MHz)
*δ* 12.00 (s, 1H), 8.19 (s, 1H), 7.33-7.41 (m, 1H), 7.28 (br d, *J =* 8.0 Hz, 1H), 6.98 (s, 1H), 6.94 (br d, *J =* 7.6 Hz, 1H), 6.64 (s, 1H), 6.35 (s, 1H), 4.92-4.96 (m, 2H), 4.88 (d, *J =* 6.0 Hz, 2H), 3.69 (s, 2H), 3.50 (s, 2H), 2.97 (s, 3H), 2.79 (br t, *J =* 12.0 Hz, 2H), 2.23-2.33 (m, 1H), 1.98-2.14 (m, 3H), 1.81-1.98 (m, 2H), 0.93 (d, *J =* 6.4 Hz, 3H), 0.76-0.84 (m, 2H), 0.61-0.71 (m, 2H)
LCMS: m/z = 549.3 (M+H)⁺, Rt = 1.503 min

### 6 General steps for preparation of (R)-4-cyclopropyl-2-((4,4-difluoro-3-methylpiperidin-1-yl)methyl)-6-(3-(3-((4-methyl-4H-1,2,4-triazol-3-yl)methy l)oxetan-3-yl)phenyl)-1,6-dihydro-7H-pyrrolo[2,3-c]pyridin-7-one (compound T078) and (S)-4-cyclopropyl-2-((4,4-difluoro-3-methylpiperidin-1-yl)methyl)-6-(3-(3-((4-methyl-4H-1,2,4-triazol-3-yl)methyl )oxetan-3-yl)phenyl)-1,6-dihydro-7H-pyrrolo[2,3-c]pyridin-7-one (compound T079):

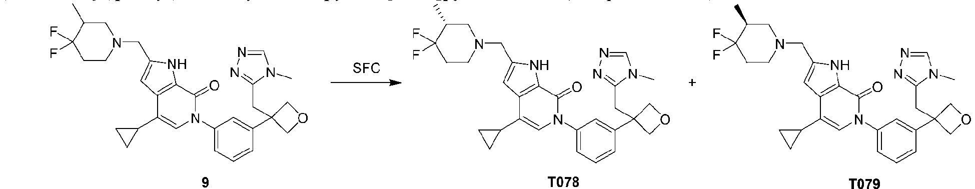

The residue was purified by SFC (column: DAICEL CHIRALPAK AD (250 mm * 30 mm, 10 um); mobile phase: [CO₂ - I - PrOH (0.1% NH₃H₂O)]; B%:30%, isocratic elution mode). Compound (R)-4-cyclopropyl-2-((4,4-difluoro-3-methylpiperidin-1-yl)methyl)-6-(3-(3-((4-methyl-4H-1,2,4-triazol-3-yl)methy l)oxetan-3-yl)phenyl)-1,6-dihydro-7H-pyrrolo[2,3-c]pyridin-7-one (19.9 mg, 36.2 µmol, 24.8% yield, 99.91% purity) was obtained as a white solid. Confirmed by H NMR and LCMS.
¹H NMR: (DMSO-*d*₆, 400 MHz)
*δ* 12.00 (s, 1H), 8.19 (s, 1H), 7.32-7.44 (m, 1H), 7.27 (br d, *J* = 8.0 Hz, 1H), 6.98 (s, 1H), 6.93 (br d, *J* = 7.6 Hz, 1H), 6.64 (s, 1H), 6.35 (d, *J =* 1.6 Hz, 1H), 4.91-4.99 (m, 2H), 4.88 (d, *J =* 6.0 Hz, 2H), 3.68 (s, 2H), 3.50 (s, 2H), 2.97 (s, 3H), 2.78 (br t, *J =* 11.6 Hz, 2H), 2.26 (br t, *J* = 10.0 Hz, 1H), 1.98-2.12 (m, 3H), 1.81-1.97 (m, 2H), 0.92 (d, *J* = 6.4 Hz, 3H), 0.78-0.85 (m, 2H), 0.61-0.70 (m, 2H)
LCMS: m/z = 549.3 (M+H)⁺, Rt = 1.590 min

Compound (S)-4-cyclopropyl-2-((4,4-difluoro-3-methylpiperidin-1-yl)methyl)-6-(3-(3-((4-methyl-4H-1,2,4-triazol-3-yl)methyl )oxetan-3-yl)phenyl)-1,6-dihydro-7H-pyrrolo[2,3-c]pyridin-7-one (7.80 mg, 14.1 µmol, 9.73% yield, 99.81% purity) was obtained as a white solid. Confirmed by H NMR and LCMS.
¹H NMR: (DMSO-*d*₆, 400 MHz)
*δ* 12.02 (br s, 1H), 8.19 (s, 1H), 7.32-7.43 (m, 1H), 7.27 (br d, *J* = 7.6 Hz, 1H), 6.88-7.02 (m, 2H), 6.64 (s, 1H), 6.37 (br s, 1H), 4.94 (br d, *J =* 5.6 Hz, 2H), 4.88 (br d, *J =* 5.6 Hz, 2H), 3.72 (br s, 2H), 3.50 (br s, 2H), 2.97 (s, 3H), 2.82 (br d, *J* = 2.0 Hz, 2H), 2.26-2.38 (m, 1H), 2.02 (br d, *J =* 7.6 Hz, 3H), 1.87 (br s, 2H), 0.93 (br d, *J =* 5.6 Hz, 3H), 0.81 (br d, *J* = 7.2 Hz, 2H), 0.66 (br d, *J =* 4.0 Hz, 2H)
LCMS: m/z = 549.3 (M+H)⁺, Rt = 1.600 min

### Examples 80 and 81: Synthesis of compounds T080& T081

### 1. General steps for preparation of 2-[1-[[6-[3-[1-(4-methyl-1,2,4-triazol-3-yl)cyclobutyl]phenyl]-7-oxo-4-(trifluoromethyl)-1H-pyrrolo[2,3-c]pyridin-2-yl]methyl]-3-piperidyl]acetonitrile

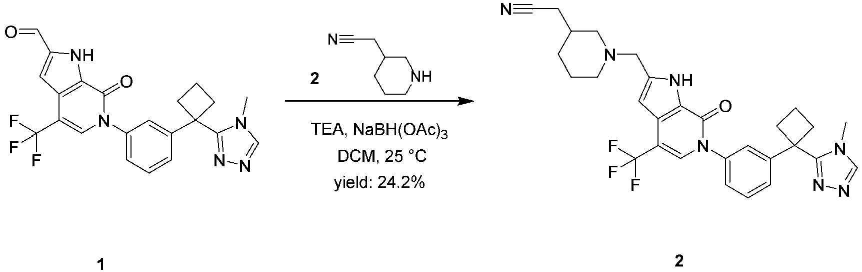

To a solution of6-[3-[1-(4-methyl-1,2,4-triazol-3-yl)cyclobutyl]phenyl]-7-oxo-4-(trifluoromethyl)-1H-pyrrolo[2,3-c]pyridine-2-c arbaldehyde (80.0 mg, 181 µmol) in dichloromethane (2.50 mL) was added dropwise TEA (18.3 mg, 181 µmol, 25.2 µL) adjust pH = 7, 2-(3-piperidyl)acetonitrile (34.9 mg, 217 µmol, HCl) was added to the mixture, stirred at 25 °C for 0.5 hr, thenNaBH(OAc)₃ (96.0 mg, 453 µmol) was added to the mixture, stirred at 25 °C for 1 hr. LCMS showed desired compound was detected. The reaction mixture was diluted with aqueous NaHCO₃ (8.00 mL), extracted with dichloromethane (12.0 mL * 3), the combined organic layers were washed with H₂O (8.00 mL), dried over Na₂SO₄, filtered and concentrated under reduced pressure to give a residue. The residue was purified by Prep-HPLC (column: Welch Xtimate C18 40 * 200 mm 7um; mobile phase: [water (NH₄HCO₃)-ACN]; gradient:22.0%-62.0% B over 25 min). Compound 2-[1-[[6-[3-[1-(4-methyl-1,2,4-triazol-3-yl)cyclobutyl]phenyl]-7-oxo-4-(trifluoromethyl)-1H-pyrrolo[2,3-c]pyridin-2-yl]methyl]-3-piperidyl]acetonitrile (25.0 mg, 43.8 µmol, 24.2% yield, 96.4% purity) was obtained as a colorless oil. Confirmed by LCMS .
LCMS: m/z = 550.2 (M+H)⁺, Rt = 1.337 min

### 2. General steps for preparation of (S)-2-(1-((6-(3-(1-(4-methyl-4H-1,2,4-triazol-3-yl)cyclobutyl)phenyl)-7-oxo-4-(trifluoromethyl)-6,7-dihydro-1H-p yrrolo[2,3-c]pyridin-2-yl)methyl)piperidin-3-yl)acetonitrile (compound T080) and (R)-2-(1-((6-(3-(1-(4-methyl-4H-1,2,4-triazol-3-yl)cyclobutyl)phenyl)-7-oxo-4-(trifluoromethyl)-6,7-dihydro-1H-p yrrolo[2,3-c]pyridin-2-yl)methyl)piperidin-3-yl)acetonitrile (compound T081)

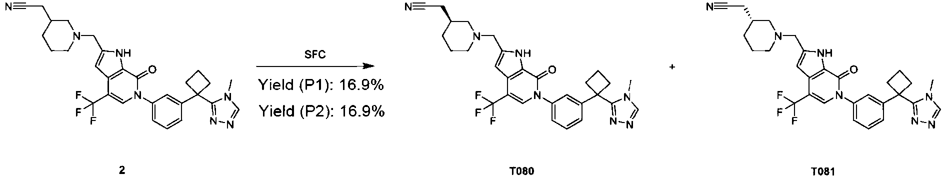

The residue was purified by SFC(column: DAICEL CHIRALPAK AD(250mm*30mm,10um);mobile phase: [CO2-EtOH(0.1%NH3H2O)];B%:45%, isocratic elution mode). Compound (S)-2-(1-((6-(3-(1-(4-methyl-4H-1,2,4-triazol-3-yl)cyclobutyl)phenyl)-7-oxo-4-(trifluoromethyl)-6,7-dihydro-1H-p yrrolo[2,3-c]pyridin-2-yl)methyl)piperidin-3-yl)acetonitrile (4.45 mg, 7.72 µmol, 16.9% yield, 95.4% purity) was obtained as a white solid. Confirmed by H NMR and LCMS.
¹H NMR: (DMSO-*d*₆, 400 MHz)
*δ* 12.46 (s, 1H), 8.35 (s, 1H), 7.71 (s, 1H), 7.48-7.54 (m, 1H), 7.43 (s, 1H), 7.36 (d, *J =* 7.6 Hz, 1H), 7.26 (d, *J* = 8.0 Hz, 1H), 6.29 (s, 1H), 3.65 (s, 2H), 3.27 (s, 3H), 2.90-2.97 (m, 2H), 2.80 (d, *J =* 6.0 Hz, 1H), 2.66-2.73 (m, 3H), 1.94-2.06 (m, 4H), 1.85 (d, *J* = 4.4 Hz, 2H), 1.59-1.72 (m, 2H), 1.23 (s, 3H)
LCMS: m/z = 550.2 (M+H)⁺, Rt = 1.282 min

Compound (R)-2-(1-((6-(3-(1-(4-methyl-4H-1,2,4-triazol-3-yl)cyclobutyl)phenyl)-7-oxo-4-(trifluoromethyl)-6,7-dihydro-1H-p yrrolo[2,3-c]pyridin-2-yl)methyl)piperidin-3-yl)acetonitrile (4.46 mg, 7.71 µmol, 16.9% yield, 95.0% purity) was obtained as a white solid. Confirmed by H NMR and LCMS.
¹H NMR: (DMSO-*d*₆, 400 MHz)
*δ* 12.46 (s, 1H), 8.35 (s, 1H), 7.72 (s, 1H), 7.48-7.54 (m, 1H), 7.43 (s, 1H), 7.36 (d, *J=* 7.6 Hz, 1H), 7.26 (d, *J* = 8.0 Hz, 1H), 6.29 (s, 1H), 3.65 (s, 2H), 3.27 (s, 3H), 2.90-2.97 (m, 2H), 2.80 (d, *J =* 7.2 Hz, 1H), 2.70-2.73 (m, 1H), 2.68 (s, 2H), 1.87-2.10 (m, 4H), 1.85 (d, *J =* 5.6 Hz, 2H), 1.59-1.72 (m, 2H), 1.43-1.51 (m, 1H), 1.23 (s, 1H), 0.98-1.07 (m, 1H)
LCMS: m/z = 550.3 (M+H)⁺, Rt = 1.340 min

### Examples 82 and 83: Synthesis of compounds T082& T083

### 1. General steps for preparation of (S)-2-((3-fluoropyrrolidin-1-yl)methyl)-6-(3-(3-methoxy-1-(4-methyl-4H-1,2,4-triazol-3-yl)cyclobutyl)phenyl)-4-(t rifluoromethyl)-1,6-dihydro-7H-pyrrolo[2,3-c]pyridin-7-one

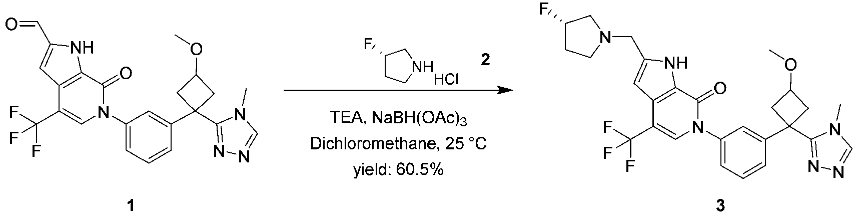

To a mixture of (3S)-3-fluoropyrrolidine (53.2 mg, 424 µmol, HCl) in Dichloromethane (6.00 mL) was added TEA (128 mg, 1.27 mmol, 177 µL) adjust pH = 8.00. Then 6-[3-[3-methoxy-1-(4-methyl-1,2,4-triazol-3-yl) cyclobutyl] phenyl]-7-oxo-4-(trifluoromethyl)-1H-pyrrolo[2,3-c] pyridine-2-carbaldehyde (100 mg, 212 µmol) was added to the mixture. The mixture was stirred under N₂ at 25.0 °C for 0.50 hr. sodium triacetoxyboranuide (112 mg, 530 µmol) was added to the mixture. The mixture was stirred under N₂ at 25.0 °C for 11.5 hrs. LCMS showed desired mass was detected. The mixture was concentrated under reduced pressure. Then it was diluted with Methanol (7.00 mL). The crude product was purified by Prep-HPLC (column: Welch Xtimate C18 40 * 200 mm 7.00 um; mobile phase: [water (NH₃H₂O+NH₄HCO₃)-ACN]; gradient: 18.0%-58.0% B over 25.0 mins). Compound (S)-2-((3-fluoropyrrolidin-1-yl) methyl)-6-(3-(3-methoxy-1-(4-methyl-4H-1,2,4-triazol-3-yl) cyclobutyl) phenyl)-4-(trifluoromethyl)-1,6-dihydro-7H-pyrrolo[2,3-c] pyridin-7-one (70.2 mg, 128 µmol, 60.5% yield, 99.5% purity) was obtained as a white solid, which was confirmed by H NMR and LCMS.
¹H NMR: (400 MHz, DMSO-*d*₆)
*δ* 12.50 (br s, 1H), 8.23-8.42 (m, 1H), 7.72 (dd, *J =* 3.6, 1.6 Hz, 1H), 7.50 (q, *J =* 7.6 Hz, 1H), 7.29-7.40 (m, 2H), 7.18 (d, *J =* 8.0 Hz, 1H), 6.31 (s, 1H), 5.26 (br t, *J=* 5.6 Hz, 1H), 5.12 (br t, *J =* 5.6 Hz, 1H), 4.00-4.14 (m, 1H), 3.85 (quin, *J* = 7.2 Hz, 1H), 3.77 (s, 2H), 3.27-3.30 (m, 3H), 3.14-3.18 (m, 3H), 3.10 (ddd, *J* = 10.0, 7.2, 2.4 Hz, 1H), 2.82-2.88 (m, 1H), 2.75-2.82 (m, 2H), 2.58-2.73 (m, 1H), 2.34-2.44 (m, 1H), 2.04-2.22 (m, 1H), 1.78-1.96 (m, 1H)
LCMS: m/z = 545.1 (M+H)⁺, Rₜ = 0.887 min

### 2. General steps for preparation of 2-(((S)-3-fluoropyrrolidin-1-yl)methyl)-6-(3-((1s,3R)-3-methoxy-1-(4-methyl-4H-1,2,4-triazol-3-yl)cyclobutyl)phe nyl)-4-(trifluoromethyl)-1,6-dihydro-7H-pyrrolo[2,3-c]pyridin-7-one (compound T082) 2-(((S)-3-fluoropyrrolidin-1-yl)methyl)-6-(3-((1r,3S)-3-methoxy-1-(4-methyl-4H-1,2,4-triazol-3-yl)cyclobutyl)phe nyl)-4-(trifluoromethyl)-1,6-dihydro-7H-pyrrolo[2,3-c]pyridin-7-one- (compound T083)

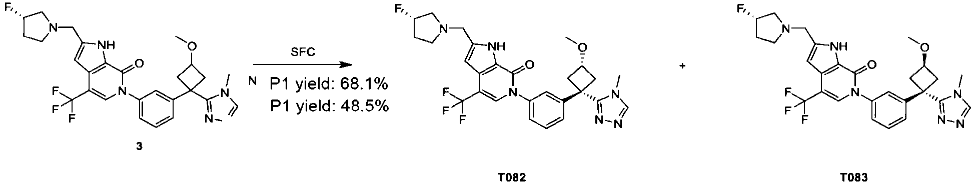

SFC (EB12379-22-P1A1) showed two peaks. The crude product was purified by SFC (column: DAICEL CHIRALPAK IG (250 mm * 30.0 mm, 10.0 um); mobile phase: [CO₂-EtOH (0.10% NH₃H₂O)]; B%: 55.0%, isocratic elution mode). To afford 2-(((S)-3-fluoropyrrolidin-1-yl)methyl)-6-(3-((1s,3R)-3-methoxy-1-(4-methyl-4H-1,2,4-triazol-3-yl)cyclobutyl)phe nyl)-4-(trifluoromethyl)-1,6-dihydro-7H-pyrrolo[2,3-c]pyridin-7-one (33.9 mg, 62.2 µmol, 48.2% yield, 100% purity) as a white solid which was confirmed by H NMR and LCMS.
¹H NMR: (400 MHz, DMSO-*d*₆)
*δ* 12.50 (br s, 1H), 8.38 (s, 1H), 7.72 (d, *J =* 1.2 Hz, 1H), 7.45-7.55 (m, 1H), 7.31-7.40 (m, 2H), 7.18 (br d, *J =* 8.0 Hz, 1H), 6.31 (s, 1H), 5.07-5.32 (m, 1H), 3.85 (quin, *J* = 7.2 Hz, 1H), 3.76 (s, 2H), 3.31-3.38 (m, 4H), 3.29 (s, 3H), 3.16 (s, 3H), 2.75-2.88 (m, 2H), 2.58-2.73 (m, 1H), 2.33-2.43 (m, 1H), 2.04-2.23 (m, 1H), 1.77-1.96 (m, 1H)
LCMS: m/z = 545.2 (M+H)⁺, Rt = 1.309 min

To afford 2-(((S)-3-fluoropyrrolidin-1-yl)methyl)-6-(3-((1r,3S)-3-methoxy-1-(4-methyl-4H-1,2,4-triazol-3-yl)cyclobutyl)phe nyl)-4-(trifluoromethyl)-1,6-dihydro-7H-pyrrolo[2,3-c]pyridin-7-one (23.8 mg, 43.7 µmol, 33.9% yield, 100% purity) as a white solid which was confirmed by H NMR and LCMS.
¹H NMR: (400 MHz, DMSO-*d*₆)
*δ* 12.51 (br s, 1H), 8.30 (s, 1H), 7.73 (d*, J =* 1.2 Hz, 1H), 7.47-7.57 (m, 2H), 7.36-7.40 (m, 1H), 7.32 (br d, J= 8.4 Hz, 1H), 6.31 (s, 1H), 5.08-5.31 (m, 1H), 4.06 (quin, *J* = 7.2 Hz, 1H), 3.76 (s, 2H), 3.27 (s, 3H), 3.15 (s, 3H), 3.10 (ddd, *J =* 10.0, 7.2, 2.4 Hz, 2H), 2.75-2.87 (m, 4H), 2.59-2.72 (m, 1H), 2.33-2.42 (m, 1H), 2.04-2.24 (m, 1H), 1.77-1.96 (m, 1H)
LCMS: m/z = 545.2 (M+H)⁺, Rt = 1.287 min

### Example 84: Synthesis of compound T084

### 1. General steps for preparation of 2-[[(3S)-3-methyl-1-piperidyl]methyl]-6-[3-[5-(4-methyl-1,2,4-triazol-3-yl)spiro[2.3]hexan-5-yl]phenyl]-1-(p-tolyl sulfonyl)-4-(trifluoromethyl)pyrrolo[2,3-c]pyridin-7-one

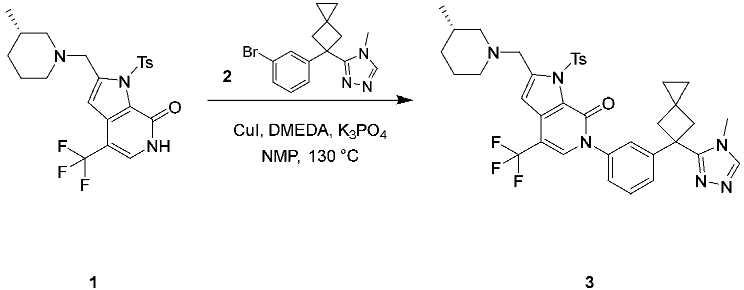

To a mixture of 3-[5-(3-bromophenyl)spiro[2.3]hexan-5-yl]-4-methyl-1,2,4-triazole (74.8 mg, 235 µmol), 2-[[(3S)-3-methyl-1-piperidyl]methyl]-1-(p-tolylsulfonyl)-4-(trifluoromethyl)-6H-pyrrolo[2,3-c]pyridin-7-one (100 mg, 213 µmol), CuI (81.4 mg, 427µmol) and K₃PO₄ (136 mg, 641 µmol) in NMP (1.00 mL) was added DMEDA (18.8 mg, 213 µmol, 23.0 µL). The suspension was degassed under vacuum and purged with N₂ several times. The mixture was stirred under N₂ at 130 °C for 1 hrs. LCMS showed the reaction was completed. The reaction mixture was diluted with Ethyl acetate (20.0 mL), washed with brine (20.0 mL * 3), dried over Na₂SO₄. The organic phase was concentrated in vacuum. The residue was purified by column chromatography (SiO₂, Dichloromethane/ Methanol = 1/ 0 to 97/ 3). TLC (Plate 1, Dichloromethane/ Methanol = 10/ 1, UV 254 nm, R_{f} (product) = 0.5). Compound 2-[[(3S)-3-methyl-1-piperidyl]methyl]-6-[3-[5-(4-methyl-1,2,4-triazol-3-yl)spiro[2.3]hexan-5-yl]phenyl]-1-(p-tolyl sulfonyl)-4-(trifluoromethyl)pyrrolo[2,3-c]pyridin-7-one (80.0 mg, 88.8 µmol, 41.5% yield, 78.3% purity) was obtained as a white solid. It was confirmed by LCMS.
LCMS: m/z = 705.2 (M+H)⁺, Rt = 0.538 min

### 2. General steps for preparation of 2-[[(3S)-3-methyl-1-piperidyl]methyl]-6-[3-[5-(4-methyl-1,2,4-triazol-3-yl)spiro[2.3]hexan-5-yl]phenyl]-4-(trifluor omethyl)-1H-pyrrolo[2,3-c]pyridin-7-one (compound T084)

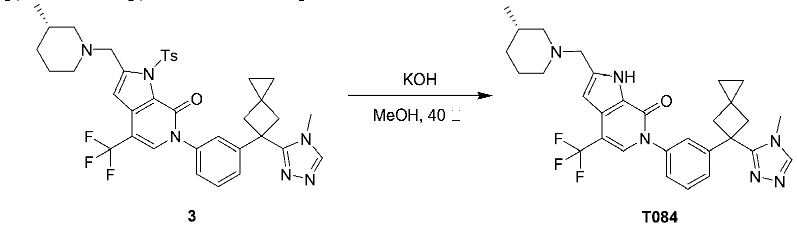

To a mixture of 2-[[(3S)-3-methyl-1-piperidyl]methyl]-6-[3-[5-(4-methyl-1,2,4-triazol-3-yl)spiro[2.3]hexan-5-yl]phenyl]-1-(p-tolyl sulfonyl)-4-(trifluoromethyl)pyrrolo[2,3-c]pyridin-7-one (80.0 mg, 113 µmol) KOH (127 mg, 2.27 mmol) in MeOH (2.00 mL). The suspension was degassedunder vacuum and purged with N₂ several times. The mixture was stirred under N₂ at 40 °C for 1 hr. LCMS showed the reaction was completed. The reaction mixture was diluted with Ethyl acetate (30.0 mL), washed with brine (30.0 mL * 3), dried over Na₂SO₄. The organic phase was concentrated in vacuum. The product was purified by Prep-HPLC (column: Xtimate C18 150 * 40 mm * 10 um; mobile phase: [water(NH₃H₂O⁺·NH₄HCO₃)-ACN]; gradient: 34%-74% B over 32 min). Compound 2-[[(3S)-3-methyl-1-piperidyl]methyl]-6-[3-[5-(4-methyl-1,2,4-triazol-3-yl)spiro[2.3]hexan-5-yl]phenyl]-4-(trifluor omethyl)-1H-pyrrolo[2,3-c]pyridin-7-one (14.8 mg, 26.6 µmol, 23.4% yield, 99.2% purity) was obtained as a white solid. It was confirmed by LCMS and H NMR.
¹H NMR: (400 MHz, CD₃CN)
*δ* 8.09 (s, 1H), 7.55 (d, *J =* 1.6 Hz, 1H), 7.48-7.53 (m, 1H), 7.36-7.41 (m, 2H), 7.34 (d, *J =* 8.0 Hz, 1H), 6.35 (s, 1H), 3.57 (s, 2H), 3.23 (s, 3H), 3.20 (s, 2H), 2.78 (d, *J =* 12.8 Hz, 2H), 2.66-2.74 (m, 2H), 1.85-1.93 (m, 2H), 1.60-1.70 (m, 2H), 1.55 (br s, 2H), 1.42-1.52 (m, 1H), 0.80 (d, *J* = 6.0 Hz, 3H), 0.53-0.59 (m, 2H), 0.45-0.51 (m, 2H)
LCMS: m/z = 551.0 (M+H)⁺, Rt = 1.062 min

### Example 85: Synthesis of compound T085

### 1. General steps for preparation of 6-[3-[3-ethylidene-1-(4-methyl-1,2,4-triazol-3-yl)cyclobutyl]phenyl]-2-[[(3S)-3-methyl-1-piperidyl]methyl]-4-(trifl uoromethyl)-1H-pyrrolo[2,3-c]pyridin-7-one (compound T085)

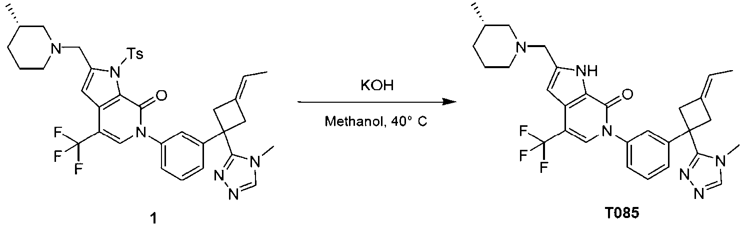

To a solution of 6-[3-[3-ethylidene-1-(4-methyl-1,2,4-triazol-3-yl)cyclobutyl]phenyl]-2-[[(3S)-3-methyl-1-piperidyl]methyl]-1-(p-to lylsulfonyl)-4-(trifluoromethyl)pyrrolo[2,3-c]pyridin-7-one (40.0 mg, 56.7 µmol) in Methanol (5.00 mL) was added KOH (63.7 mg, 1.14 mmol). The mixture was stirred under N₂ at 40 °C for 1hr. LCMS showed desired mass was detected. The reaction mixture was diluted with brine (20.0 mL), extracted with Ethyl acetate (20.0 mL*2). The organic phase washed with KOH (1 M, 20.0 mL), dried over Na₂SO₄ and concentrated in vacuum. The crude product was purified by Prep-HPLC (column: Xtimate C18 150*40 mm*10 um; mobile phase: [water (FA)-ACN]; gradient: 0%-38.0% B over 25 mins). To afford 6-[3-[3-ethylidene-1-(4-methyl-1,2,4-triazol-3-yl)cyclobutyl]phenyl]-2-[[(3S)-3-methyl-1-piperidyl]methyl]-4-(trifl uoromethyl)-1H-pyrrolo[2,3-c]pyridin-7-one (21.0 mg, 34.9 µmol, 61.5% yield, 99.3% purity, FA) as a yellow solid which was confirmed by H NMR and LCMS.
¹H NMR: (400 MHz, DMSO-*d*₆)
*δ* 12.43 (m, 1H), 8.38 (s, 1H), 7.70 (s, 1H), 7.47-7.58 (m, 1H), 7.33-7.41 (m, 2H), 7.27 (d, *J* = 7.6 Hz, 1H), 6.27 (s, 1H), 5.19-5.34 (m, 1H), 3.64 (d, *J* = 16.0 Hz, 2H), 3.60 (s, 2H), 3.44-3.46 (m, 2H), 3.32 (s, 3H), 2.71-2.79 (m, 2H), 1.89 (t, *J* = 10.4 Hz, 1H), 1.54-1.65 (m, 4H), 1.51 (d, *J =* 6.4 Hz, 3H), 1.38-1.48 (m, 1H), 0.81 (d, *J* = 5.6 Hz, 3H), 0.75 (s, 1H)
LCMS: m/z = 551.3 (M+H)⁺, Rt = 1.177 min

### Example 86: Synthesis of compound T086

### 1. General steps for preparation of 2-[[(3aS,7aR)-1,3,3a,4,5,6,7,7a-octahydroisoindol-2-yl]methyl]-6-[3-[1-(4-methyl-1,2,4-triazol-3-yl)cyclobutyl]phe nyl]-4-(trifluoromethyl)-1H-pyrrolo[2,3-c]pyridin-7-one (compound T086)

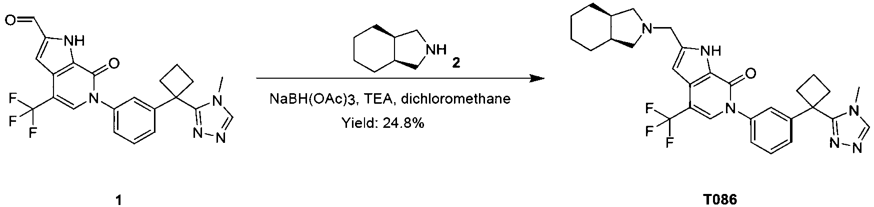

6-[3-[1-(4-methyl-1,2,4-triazol-3-yl)cyclobutyl]phenyl]-7-oxo-4-(triffuoromethyl)-1H-pyrrolo[2,3-c]pyridine-2-carbaldehyde (50.0 mg, 113 µmol) was dissolved in dichloromethane (1.00 mL), pH was adjust to 7-8 with TEA. (3aR,7aS)-2,3,3a,4,5,6,7,7a-octahydro-1H-isoindole (21.3 mg, 170 µmol) in dichloromethane 0.50 mL was added to the mixture, stirred at 25 °C for 0.5 hr under N₂. Then NaBH(OAc)₃ (60.0 mg, 283 µmol) was added to the mixture, then stirred at 25 °C under N₂ for 2 hrs. LCMS shows reactant was consumed completely and desired mass was detected. The reaction mixture was diluted with dichloromethane 6.00 mL, washed with sat. NaHCO₃ solution 2.00 mL. The water phase extracted with dichloromethane 2.00 mL (1.00 mL * 2). The combined organic layers were washed, dried over Na₂SO₄, filtered and concentrated under reduced pressure to give a residue. The residue was purified by prep.HPLC (column: Welch Xtimate C18 40 * 200 mm 7 um;mobile phase: [water (HCl) -ACN];gradient: 4%-44% B over 30 min). 2-[[(3aS,7aR)-1,3,3a,4,5,6,7,7a-octahydroisoindol-2-yl]methyl]-6-[3-[1-(4-methyl-1,2,4-triazol-3-yl)cyclobutyl]phe nyl]-4-(trifluoromethyl)-1H-pyrrolo[2,3-c]pyridin-7-one (15.5 mg, 28.2 µmol, 24.8% yield, 100.0% purity)was obtained as white solid, confirmed by H NMR and LCMS.
¹H NMR: (400 MHz, DMSO-*d*₆)
*δ* 12.70-12.86 (m, 1H), 11.52-11.73 (m, 1H), 9.39 (br s, 1H), 7.82 (d, *J =* 1.2 Hz, 1H), 7.49-7.62 (m, 2H), 7.42 (br dd, *J* = 16.8, 8.0 Hz, 2H), 6.80 (s, 1H), 4.47-4.53 (m, 2H), 3.43 (s, 3H), 3.36-3.41 (m, 1H), 3.25 (br t, *J* = 5.6 Hz, 2H), 3.08-3.17 (m, 1H), 2.93-3.04 (m, 2H), 2.73-2.84 (m, 2H), 2.38 (br d, *J* = 4.4 Hz, 1H), 2.27 (br s, 1H), 1.97-2.10 (m, 2H), 1.60 (br d, *J =* 5.2 Hz, 1H), 1.38-1.56 (m, 5H), 1.23-1.37 (m, 2H)
LCMS: m/z = 551.3 (M+H)⁺, Rt = 1.560 min

### Example 87: Synthesis of compound T087

### 1. General steps for preparation of 2-((3-azabicyclo[3.2.1]octan-3-yl)methyl)-6-(3-((1s,3s)-3-methyl-1-(4-methyl-4H-1,2,4-triazol-3-yl)cyclobutyl)phe nyl)-4-(trifluoromethyl)-1,6-dihydro-7H-pyrrolo[2,3-c]pyridin-7-one (compound T087)

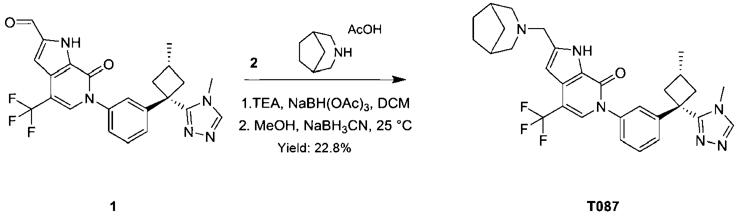

To a solution of 6-(3-((1s,3s)-3-methyl-1-(4-methyl-4H-1,2,4-triazol-3-yl)cyclobutyl)phenyl)-7-oxo-4-(triffuoromethyl)-6,7-dihydro -1H-pyrrolo[2,3-c]pyridine-2-carbaldehyde (50.0 mg, 109 µmol) in dichloromethane (2.00 mL) was added dropwise TEA (11.1 mg, 109 µmol, 15.2 µL) adjust pH = 7, 3-azabicyclo[3.2.1]octane (15.8 mg, 92.6 µmol, HOAC) was added to the mixture, stirred at 25 °C for 0.5 hr, then NaBH(OAc)₃ (58.1 mg, 274 µmol) was added to the mixture, stirred at 25 °C for 1 hr. Methyl alcohol (1.00 mL) was added to the mixture, followed by NaBH₃CN (6.90 mg, 109 µmol) add slowly, stirred for 1 hr. LCMS showed desired compound was detected. The reaction mixture was diluted with aqueous NaHCO₃ (8.00 mL), extracted with dichloromethane (12.0 mL * 3), the combined organic layers were washed with H₂O (8.00 mL), dried over Na₂SO₄, filtered and concentrated under reduced pressure to give a residue. The residue was purified by Prep-HPLC (column: Welch Xtimate C18 40*200mm 7um;mobile phase: [water( NH₄HCO₃)-ACN];gradient:46.0%-86.0% B over 25 mins). Compound 2-(3-azabicyclo[3.2.1]octan-3-ylmethyl)-6-[3-[3-methyl-1-(4-methyl-1,2,4-triazol-3-yl)cyclobutyl]phenyl]-4-(triflu oromethyl)-1H-pyrrolo[2,3-c]pyridin-7-one (13.8 mg, 25.0 µmol, 22.8% yield, 100% purity) was obtained as a white solid. Confirmed by H NMR and LCMS.
¹H NMR: (DMSO-*d*₆, 400 MHz)
*δ* 12.40 (s, 1H), 8.28 (s, 1H), 7.71 (d, *J* = 1.2 Hz, 1H), 7.48-7.56 (m, 2H), 7.35 (t, *J =* 9.2 Hz, 2H), 6.26 (s, 1H), 3.59 (s, 2H), 3.25 (s, 3H), 2.88 (d, *J* = 3.6 Hz, 2H), 2.59-2.64 (m, 2H), 2.52-2.57 (m, 3H), 2.08 (s, 2H), 2.06 (d, *J* = 4.4 Hz, 2H), 1.63 (d, *J =* 6.4 Hz, 2H), 1.47-1.54 (m, 2H), 1.36-1.42 (m, 1H), 1.25 (d, *J =* 10.8 Hz, 1H), 1.06 (d, *J =* 5.2 Hz, 3H)
LCMS: m/z = 551.2 (M+H)⁺, Rt = 1.580 min

### Example 88: Synthesis of compound T088

### 1. General steps for preparation of 2-((5-azaspiro[2.5]octan-5-yl)methyl)-6-(3-((1s,3s)-3-methyl-1-(4-methyl-4H-1,2,4-triazol-3-yl)cyclobutyl)phenyl) -4-(trifluoromethyl)-1,6-dihydro-7H-pyrrolo[2,3-c]pyridin-7-one (compound T088)

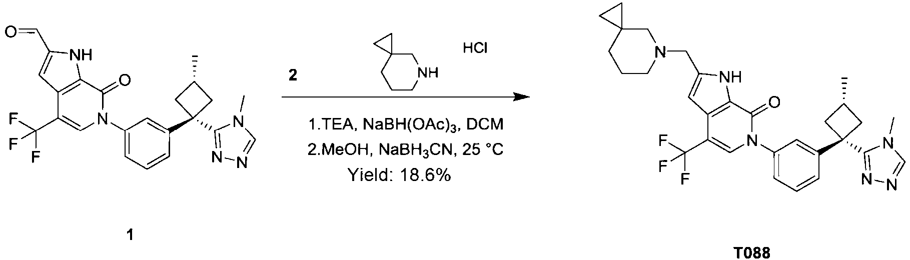

To a solution of 6-(3-((1s,3s)-3-methyl-1-(4-methyl-4H-1,2,4-triazol-3-yl)cyclobutyl)phenyl)-7-oxo-4-(triffuoromethyl)-6,7-dihydro -1H-pyrrolo[2,3-c]pyridine-2-carbaldehyde (50.0 mg, 109 µmol) in dichloromethane (2.00 mL) was added dropwise TEA (11.1 mg, 109 µmol, 15.2 µL) adjust pH = 7, 5-azaspiro[2.5]octane (15.8 mg, 107.48 µmol, 9.79e-1 eq, HCl) was added to the mixture, stirred at 25 °C for 0.5 hr, then NaBH(OAc)3 (58.1 mg, 274 µmol) was added to the mixture, stirred at 25 °C for 1 hr. Methyl alcohol (1.00 mL) was added to the mixture, followed by NaBH₃CN (6.90 mg, 109 µmol) add slowly, stirred for 1 hr. LCMS showed desired compound was detected. The reaction mixture was diluted with aqueous NaHCO₃ (8.00 mL), extracted with dichloromethane (12.0 mL * 3), the combined organic layers were washed with H₂O (8.00 mL), dried over Na₂SO₄, filtered and concentrated under reduced pressure to give a residue. The residue was purified by Prep-HPLC (column: Welch Xtimate C18 40 * 200 mm 7 um; mobile phase: [water (NH₄HCO₃)-ACN]; gradient:36.0%-76.0% B over 25 mins). Compound 2-((5-azaspiro[2.5]octan-5-yl)methyl)-6-(3-((1s,3s)-3-methyl-1-(4-methyl-4H-1,2,4-triazol-3-yl)cyclobutyl)phenyl) -4-(trifluoromethyl)-1,6-dihydro-7H-pyrrolo[2,3-c]pyridin-7-one (11.3 mg, 20.5 µmol, 18.6% yield, 100% purity) was obtained as a white solid. Confirmed by H NMR and LCMS.
¹H NMR: (DMSO-*d*₆, 400 MHz)
*δ* 12.19-12.64 (m, 1H), 8.28 (s, 1H), 7.71 (s, 1H), 7.47-7.55 (m, 2H), 7.35 (t, *J* = 7.6 Hz, 2H), 6.26 (s, 1H), 3.58 (s, 2H), 3.25 (s, 3H), 2.88 (d, *J =* 3.2 Hz, 2H), 2.53 (d, *J =* 6.4 Hz, 3H), 2.42 (s, 2H), 2.13 (s, 2H), 1.53-1.62 (m, 2H), 1.23 (s, 2H), 1.06 (d, *J =* 4.8 Hz, 3H), 0.25 (s, 4H)
LCMS: m/z = 551.3 (M+H)⁺, Rt = 1.603 min

### Example 89: Synthesis of compound T089

### 1. General steps for preparation of 7-methoxy-2-[[(3S)-3-methyl-1-piperidyl]methyl]-4-methylsulfonyl-1-(p-tolylsulfonyl)pyrrolo[2,3-c]pyridine

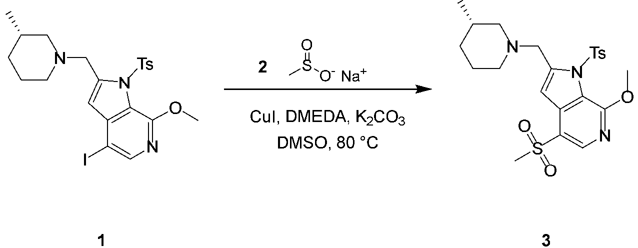

To a solution of 4-iodo-7-methoxy-2-[[(3S)-3-methyl-1-piperidyl]methyl]-1-(p-tolylsulfonyl)pyrrolo[2,3-c]pyridine (150 mg, 278 µmol) and sodium;methanesulfinate (56.8 mg, 556 µmol) in DMSO (6.00 mL) was added K₂CO₃ (76.9 mg, 556 µmol), DMEDA (24.5 mg, 278 µmol) and CuI (26.5 mg, 139 µmol). The suspension was degassed under vacuum and purged with N₂ several times. The mixture was stirred under N₂ at 80 °C for 2 hrs. TLC (DCM/ MeOH = 10/ 1, product 1 R_{f} = 0.40) indicated new spot formed. The reaction mixture was diluted with EtOAc (20.0 mL). The mixture was filtered and the filter cake was washed with EtOAc (10.0 mL*3). The filtrate was washed with brine (50.0 mL), dried over Na₂SO₄. The organic phase was concentrated in vacuum. The crude product was purified by column chromatography (SiO₂, DCM/ MeOH = 1/ 0 to 15/ 1). To afford 7-methoxy-2-[[(3S)-3-methyl-1-piperidyl]methyl]-4-methylsulfonyl-1-(p-tolylsulfonyl)pyrrolo[2,3-c]pyridine (100 mg, 178 µmol, 64.0% yield, 87.6% purity) as a yellow solid which was confirmed by H NMR and LCMS.
¹H NMR: (400 MHz, CDCl₃)
*δ* 8.46 (d, *J* = 8.4 Hz, 2H), 8.42 (s, 1H), 7.33 (d, *J* = 8.0 Hz, 2H), 6.97 (s, 1H), 3.98 (s, 2H), 3.93 (s, 3H), 3.09 (s, 3H), 2.88-2.95 (m, 2H), 2.45 (s, 3H), 1.95-2.02 (m, 1H), 1.71-1.81 (m, 2H), 1.63-1.71 (m, 3H), 0.91-0.97 (m, 1H), 0.86 (d, *J =* 6.0 Hz, 3H)
LCMS: m/z = 492.0 (M+H)⁺, Rt = 0.777 min

### 2. General steps for preparation of 2-[[(3S)-3-methyl-1-piperidyl]methyl]-4-methylsulfonyl-1-(p-tolylsulfonyl)-6H-pyrrolo[2,3-c]pyridin-7-one

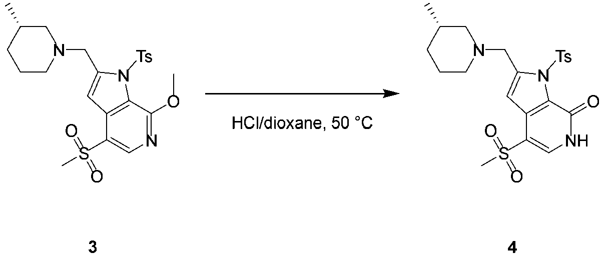

A solution of 7-methoxy-2-[[(3S)-3-methyl-1-piperidyl]methyl]-4-methylsulfonyl-1-(p-tolylsulfonyl)pyrrolo[2,3-c]pyridine (100 mg, 203 µmol) in HCl/dioxane (4 M, 4 mL). The mixture was stirred under N₂ at 50 °C for 2 hrs. TLC (DCM/ MeOH = 10/ 1, product 1 R_{f} = 0.35) indicated new spot formed. The reaction mixture was poured into saturated aqueous NaHCO₃ (30.0 mL) slowly, adjust pH = 8 (saturated aqueous NaHCO₃). The aqueous phase was extracted with EtOAc (20 mL*2). The organic phase was washed with brine (30.0 mL), dried over Na₂SO₄ and concentrated in vacuum. The crude product was purified by column chromatography (SiO₂, DCM/ MeOH = 1/ 0 to 15/ 1). To afford 2-[[(3S)-3-methyl-1-piperidyl]methyl]-4-methylsulfonyl-1-(p-tolylsulfonyl)-6H-pyrrolo[2,3-c]pyridin-7-one (60.0 mg, 109 µmol, 53.9% yield, 87.4% purity) as a yellow solid which was confirmed by LCMS.
LCMS: m/z = 477.8 (M+H)⁺, Rt = 0.970 min

### 3. General steps for preparation of 2-[[(3S)-3-methyl-1-piperidyl]methyl]-4-methylsulfonyl-6-[3-[3-[(4-methyl-1,2,4-triazol-3-yl)methyl]oxetan-3-yl] phenyl]-1-(p-tolylsulfonyl)pyrrolo[2,3-c]pyridin-7-one

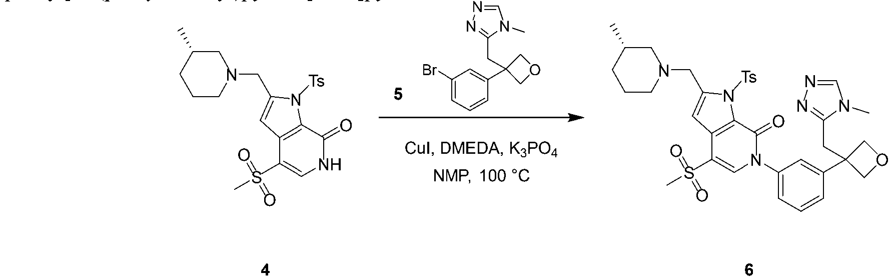

To a solution of 2-[[(3S)-3-methyl-1-piperidyl]methyl]-4-methylsulfonyl-1-(p-tolylsulfonyl)-6H-pyrrolo[2,3-c]pyridin-7-one (60.0 mg, 125 µmol) and 3-[[3-(3-bromophenyl)oxetan-3-yl]methyl]-4-methyl-1,2,4-triazole (46.5 mg, 150 µmol) in NMP (1.00 mL) was added K₃PO₄ (80.0 mg, 376 µmol), N,N'-dimethylethane-1,2-diamine (22.1 mg, 250 µmol) and CuI (23.9 mg, 125 µmol). The suspension was degassed under vacuum and purged with N₂ several times. The mixture was stirred under N₂ at 100 °C for 2 hrs. LCMS showed desired mass was detected. The reaction mixture was diluted with EtOAc (50.0 mL). The mixture was filtered and the filter cake was washed with EtOAc (10.0 mL*3). The filter was washed with brine (50.0 mL), dried over Na₂SO₄. The organic phase was concentrated in vacuum. Without purification. To afford 2-[[(3S)-3-methyl-1-piperidyl]methyl]-4-methylsulfonyl-6-[3-[3-[(4-methyl-1,2,4-triazol-3-yl)methyl]oxetan-3-yl] phenyl]-1-(p-tolylsulfonyl)pyrrolo[2,3-c]pyridin-7-one (40.0 mg, 27.5 µmol, 21.9% yield, 48.6% purity) as a yellow solid which was confirmed by LCMS.
LCMS: m/z = 705.0 (M+H)⁺, Rt = 1.020 min

### 4. General steps for preparation of 2-[[(3S)-3-methyl-1-piperidyl]methyl]-4-methylsulfonyl-6-[3-[3-[(4-methyl-1,2,4-triazol-3-yl)methyl]oxetan-3-yl] phenyl]-1H-pyrrolo[2,3-c]pyridin-7-one (compound T089)

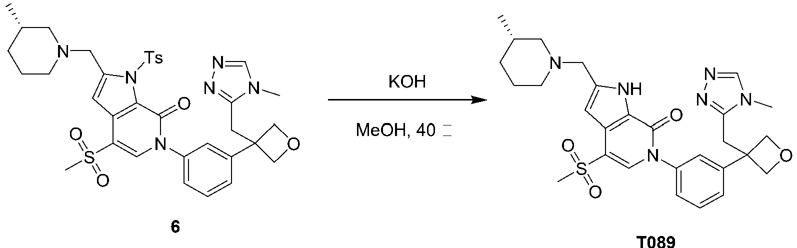

To a solution of 2-[[(3S)-3-methyl-1-piperidyl]methyl]-4-methylsulfonyl-6-[3-[3-[(4-methyl-1,2,4-triazol-3-yl)methyl]oxetan-3-yl] phenyl]-1-(p-tolylsulfonyl)pyrrolo[2,3-c]pyridin-7-one (25.0 mg, 35.4 µmol) in MeOH (3.00 mL) was added KOH (59.7 mg, 1.06 mmol). The mixture was stirred under N₂ at 40 °C for 1 hr. LCMS showed desired mass was detected. The reaction mixture was adjust pH = 8 (HCl, 2 M). The mixture was filtered to get a filtrate. The crude product was purified by Prep-HPLC (column: Welch Xtimate C18 150*30 mm*5 um; mobile phase: [water (FA) - ACN]; gradient: 0%-24% B over 25 min). To afford 2-[[(3S)-3-methyl-1-piperidyl]methyl]-4-methylsulfonyl-6-[3-[3-[(4-methyl-1,2,4-triazol-3-yl)methyl]oxetan-3-yl] phenyl]-1H-pyrrolo[2,3-c]pyridin-7-one (2.3 mg, 4.12 µmol, 11.6% yield, 98.66% purity) as a yellow solid which was confirmed by H NMR and LCMS.
¹H NMR: (400 MHz, CD₃CN)
*δ* 7.98 (s, 1H), 7.59 (s, 1H), 7.39-7.47 (m, 1H), 7.28-7.36 (m, 1H), 7.06 (d, *J =* 7.6 Hz, 1H), 6.94 (s, 1H), 6.68 (s, 1H), 4.96-5.00 (m, 2H), 4.91-4.96 (m, 2H), 4.03 (s, 2H), 3.52 (s, 2H), 3.13 (s, 3H), 3.01-3.08 (m, 2H), 2.93 (s, 3H), 2.37-2.43 (m, 1H), 2.10 (t, *J =* 11.6 Hz, 1H), 1.80-1.89 (m, 1H), 1.67-1.78 (m, 3H), 1.00 (qd, *J =* 11.6, 4.8 Hz, 1H), 0.86 (d, *J =* 6.4 Hz, 3H)
LCMS: m/z = 551.4 (M+H)⁺, Rt = 1.165 min

### Example 90: Synthesis of compound T090

### 1. General steps for preparation of (S)-4-bromo-2-((3-methylpiperidin-1-yl)methyl)-1-tosyl-1,6-dihydro-7H-pyrrolo[2,3-c]pyridin-7-one

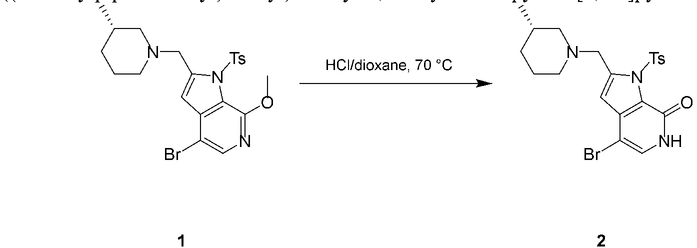

To a mixture of 4-bromo-7-methoxy-2-[[(3S)-3-methyl-1-piperidyl]methyl]-1-(p-tolylsulfonyl)pyrrolo[2,3-c]pyridine (500 mg, 1.02 mmol) in HCl/ dioxane (4 M, 5.00 mL). The mixture was stirred at 70°C for 1 hr. LCMS showed the reaction was completed. The reaction mixture was diluted with EtOAc (50.0 mL) poured into saturated aqueous NaHCO₃ (30.0 mL) slowly, adjust pH = 8 (saturated aqueous NaHCO₃). The organic phase was washed with brine (50.0 mL), dried over Na₂SO₄. The organic phase was concentrated in vacuum. The residue was purified by column chromatography (SiO₂, DCM/ MeOH = 1/ 0 to 95/ 5). TLC (Plate 1, DCM/ MeOH = 10/ 1, UV 254 nm, R_{f} (product) = 0.6). Compound (S)-4-bromo-2-((3-methylpiperidin-1-yl)methyl)-1-tosyl-1,6-dihydro-7H-pyrrolo[2,3-c]pyridin-7-one (295 mg, 616 µmol, 60.7% yield) was obtained as a yellow solid. It was confirmed by H NMR.
¹H NMR: (DMSO-*d*₆, 400 MHz)
*δ* 11.41 (br s, 1H), 8.42 (d, *J* = 8.4 Hz, 2H), 7.39 (d, *J =* 8.4 Hz, 2H), 7.36 (br d, *J =* 4.4 Hz, 1H), 6.54 (s, 1H), 3.86 (s, 2H), 2.77 (br d, *J* = 8.4 Hz, 2H), 2.39 (s, 3H), 1.90-1.98 (m, 1H), 1.65-1.76 (m, 2H), 1.56-1.63 (m, 2H), 1.42-1.51 (m, 1H), 0.85-0.93 (m, 1H), 0.82 (d, *J =* 6.4 Hz, 3H)

### 2. General steps for preparation of (S)-4-bromo-6-(3-(3-((4-methyl-4H-1,2,4-triazol-3-yl)methyl)oxetan-3-yl)phenyl)-2-((3-methylpiperidin-1-yl)meth yl)-1-tosyl-1,6-dihydro-7H-pyrrolo[2,3-c]pyridin-7-one and (S)-6-(3-(3-((4-methyl-4H-1,2,4-triazol-3-yl)methyl)oxetan-3-yl)phenyl)-2-((3-methylpiperidin-1-yl)methyl)-1-tos yl-1,6-dihydro-7H-pyrrolo[2,3-c]pyridin-7-one

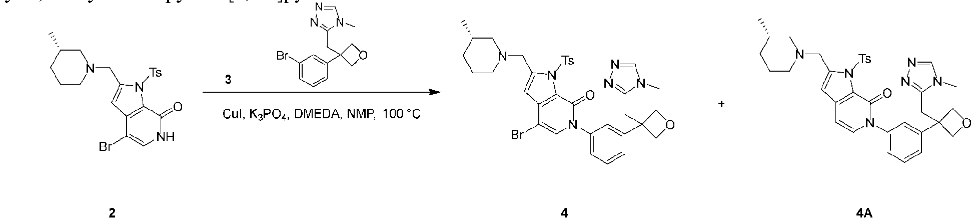

To a mixture of (S)-4-bromo-2-((3-methylpiperidin-1-yl)methyl)-1-tosyl-1,6-dihydro-7H-pyrrolo[2,3-c]pyridin-7-one (100 mg, 209 µmol), 3-[[3-(3-bromophenyl)oxetan-3-yl]methyl]-4-methyl-1,2,4-triazole (64.4 mg, 209 µmol), K₃PO₄ (133 mg, 627 µmol) and CuI (39.8 mg, 209 µmol) in NMP (1.50 mL) was added DMEDA (36.8 mg, 418 µmol, 45.0 µL). The suspension was degassed under vacuum and purged with N₂ several times. The mixture was stirred under N₂ at 100 °C for 2 hrs. LCMS showed the reaction was completed. The reaction mixture was diluted with EtOAc (30.0 mL), washed with brine (30.0 mL * 3), dried over Na₂SO₄. The organic phase was concentrated in vacuum. The residue was purified by column chromatography (SiO₂, DCM/ MeOH = 1/ 0 to 91/ 9). TLC (Plate 1, DCM/ MeOH = 10/ 1, UV 254 nm, R_{f} (product) = 0.4). Compound (S)-4-bromo-6-(3-(3-((4-methyl-4H-1,2,4-triazol-3-yl)methyl)oxetan-3-yl)phenyl)-2-((3-methylpiperidin-1-yl)meth yl)-1-tosyl-1,6-dihydro-7H-pyrrolo[2,3-c]pyridin-7-one (22.0 mg, 31.1 µmol, 14.9% yield) was obtained as a white solid, compound (S)-6-(3-(3-((4-methyl-4H-1,2,4-triazol-3-yl)methyl)oxetan-3-yl)phenyl)-2-((3-methylpiperidin-1-yl)methyl)-1-tos yl-1,6-dihydro-7H-pyrrolo[2,3-c]pyridin-7-one (120 mg, 191 µmol, 91.5% yield) was obtained as a white solid.

### 3. General steps for preparation of (S)-4-bromo-6-(3-(3-((4-methyl-4H-1,2,4-triazol-3-yl)methyl)oxetan-3-yl)phenyl)-2-((3-methylpiperidin-1-yl)meth yl)-1,6-dihydro-7H-pyrrolo[2,3-c]pyridin-7-one (compound T090)

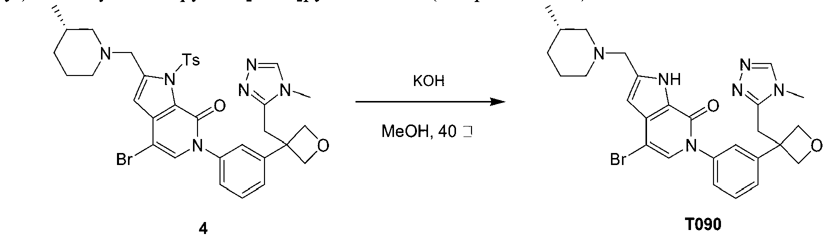

To a solution of (S)-4-bromo-6-(3-(3-((4-methyl-4H-1,2,4-triazol-3-yl)methyl)oxetan-3-yl)phenyl)-2-((3-methylpiperidin-1-yl)meth yl)-1-tosyl-1,6-dihydro-7H-pyrrolo[2,3-c]pyridin-7-one (22.0 mg, 31.1 µmol) in MeOH (0.8 mL) was added KOH (34.9 mg, 623 µmol). The resulting mixture was stirred at 40 °C for 1 hr. LCMS showed desired compound was detected. The reaction mixture was diluted with aqueous NaHCO₃ (10.0 mL). The residue was purified by Prep-HPLC (column: Welch Xtimate C 18 150 * 30 mm * 5 um; mobile phase: [water (FA)-ACN]; gradient: 0%-30% B over 25 min). Compound (S)-4-bromo-6-(3-(3-((4-methyl-4H-1,2,4-triazol-3-yl)methyl)oxetan-3-yl)phenyl)-2-((3-methylpiperidin-1-yl)meth yl)-1,6-dihydro-7H-pyrrolo[2,3-c]pyridin-7-one (6.6 mg, 11.9 µmol, 38.2% yield, 99.72% purity) was obtained as a white solid. Confirmed by H NMR and LCMS.
¹H NMR: (CD₃CN, 400 MHz)
*δ* 10.39-13.86 (m, 1H), 7.94 (s, 1H), 7.35-7.43 (m, 1H), 7.28-7.33 (m, 1H), 7.19 (s, 1H), 6.93-6.99 (m, 2H), 6.37 (s, 1H), 4.97-5.00 (m, 2H), 4.93-4.96 (m, 2H), 3.90 (br d, *J* = 2.0 Hz, 2H), 3.51 (s, 2H), 2.99 (br s, 2H), 2.93 (s, 3H), 2.22-2.29 (m, 1H), 1.96-2.00 (m, 1H), 1.65-1.78 (m, 4H), 0.91-0.99 (m, 1H), 0.85 (d, *J =* 6.4 Hz, 3H)
LCMS: m/z =553.1 (M+H)⁺, Rt = 1.557 min

### Example 91: Synthesis of compound T091

### 1. General steps for preparation of 6-[3-[1-(4-methyl-l,2,4-triazol-3-yl)cyclobutyl]phenyl]-2-[(4,4,4-trifluorobutylamino)methyl]-4-(trifluoromethyl)-1H-pyrrolo[2,3-c]pyridin-7-one (compound T091)

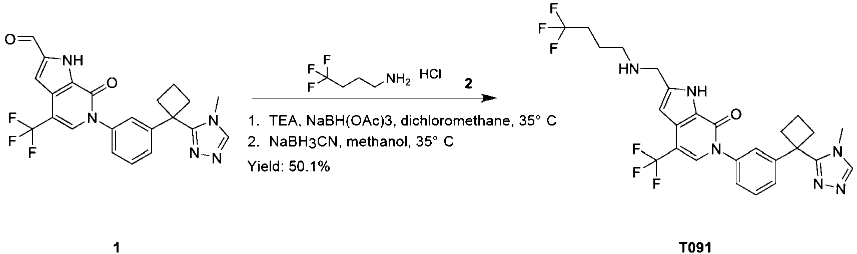

To a mixture of 4,4,4-trifluorobutan-1-amine (28.8 mg, 176 µmol, HCl) in Dichloromethane (3.00 mL) was added TEA (68.8 mg, 679 µmol) adjust pH = 8. Then 6-[3-[1-(4-methyl-1,2,4-triazol-3-yl)cyclobutyl]phenyl]-7-oxo-4-(trifluoromethyl)-1H-pyrrolo[2,3-c]pyridine-2-car baldehyde (50.0 mg, 113 µmol) was added to the mixture. The mixture was stirred under N₂ at 35 °C for 0.5 hr. NaBH(OAc)₃ (60.0 mg, 283 µmol) was added to the mixture. The mixture was stirred under N₂ at 35 °C for 2.5 hrs. Methanol (1.00 mL) and NaBH₃CN (7.12 mg, 113 µmol) was added to the mixture. The mixture was stirred under N₂ at 35 °C for 2 hrs. LCMS showed desired mass was detected. The reaction mixture was diluted with NaHCO₃ (3.00 mL), extracted with Dichloromethane (2.00 mL*2). The organic phase dried over Na₂SO₄ and concentrated in vacuum. The crude product was purified by Prep-HPLC (column: Welch Xtimate C18 40*200 mm 7 um; mobile phase: [water (NH4HCO3)-ACN]; gradient: 24.0%-64.0% B over 25 mins). To afford 6-[3-[1-(4-methyl-l,2,4-triazol-3-yl)cyclobutyl]phenyl]-2-[(4,4,4-trifluorobutylamino)methyl]-4-(trifluoromethyl)-1H-pyrrolo[2,3-c]pyridin-7-one (31.4 mg, 56.8 µmol, 50.1% yield, 100% purity) as an off-white solid which was confirmed by H NMR and LCMS.
¹H NMR: (400 MHz, DMSO-*d*₆)
*δ* 12.31 (d, *J* = 1.2 Hz, 1H), 8.35 (s, 1H), 7.70 (s, 1H), 7.48-7.54 (m, 1H), 7.41 (s, 1H), 7.35 (d, *J* = 7.6 Hz, 1H), 7.27 (d, *J* = 7.6 Hz, 1H), 6.34 (s, 1H), 3.80 (s, 2H), 3.27 (s, 3H), 2.90-2.97 (m, 2H), 2.66-2.73 (m, 2H), 2.25-2.33 (m, 2H), 1.95-2.05 (m, 2H), 1.91 (d, *J* = 11.6 Hz, 1H), 1.64-1.75 (m, 1H), 1.55-1.64 (m, 2H)
LCMS: EB6211-800-P1B1 LCMS, m/z = 553.3 (M+H)⁺, Rt = 1.325 min

### Examples 92 and 93: Synthesis of compounds T092& T093

### 1. General steps for preparation of 3-[1-(3-bromophenyl)-3-ethylidene-cyclobutyl]-4-methyl-1,2,4-triazole

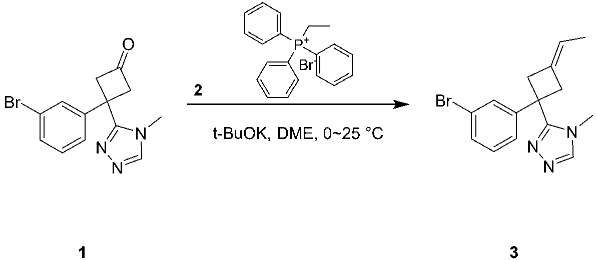

To a solution of ethyl(triphenyl)phosphonium;bromide (9.70 g, 26.1 mmol) and 3-(3-bromophenyl)-3-(4-methyl-1,2,4-triazol-3-yl)cyclobutanone (1.00 g, 3.27 mmol) in DME (50.0 mL) was added t-BuOK (1 M, 19.6 mL) at 0 °C. The mixture was stirred at 25 °C for 4 hrs. TLC (Dichloromethane/ Ethyl acetate = 1/ 1, product 1 R_{f} = 0.30) indicated new spot formed. The reaction mixture was poured into saturated aqueous NH₄Cl (20.0 mL) slowly. The mixture was extracted with Ethyl acetate (20.0 mL*2), washed with brine (20.0 mL), dried over Na₂SO₄. The organic phase was concentrated in vacuum. The crude product was purified by column chromatography (SiO₂, Dichloromethane/ Ethyl acetate = 1/ 0 to 1/ 1) and Prep-HPLC (column: Welch Xtimate C18 250*50 mm*10 um; mobile phase: [water (FA)-ACN]; gradient: 20.0%-60.0% B over 20 mins). To afford 3-[1-(3-bromophenyl)-3-ethylidene-cyclobutyl]-4-methyl-1,2,4-triazole (650 mg, 1.93 mmol, 59.2% yield, 94.7% purity) as yellow oil which was confirmed by H NMR and LCMS.
¹H NMR: (400 MHz, DMSO-*d*₆)
*δ* 8.40 (s, 1H), 7.44-7.50 (m, 1H), 7.31-7.35 (m, 2H), 7.20 (dd, *J =* 7.2, 1.2 Hz, 1H), 5.23-5.32 (m, 1H), 3.61 (s, 2H), 3.28 (d, *J* = 8.8 Hz, 2H), 3.22 (s, 3H), 1.49-1.53 (m, 3H)
LCMS: m/z = 319.8 (M+H)⁺, Rt = 1.327 min

### 2. General steps for preparation of 6-[3-[3-ethylidene-1-(4-methyl-1,2,4-triazol-3-yl)cyclobutyl]phenyl]-2-[[(3S)-3-methyl-1-piperidyl]methyl]-1-(p-to lylsulfonyl)-4-(trifluoromethyl)pyrrolo[2,3-c]pyridin-7-one

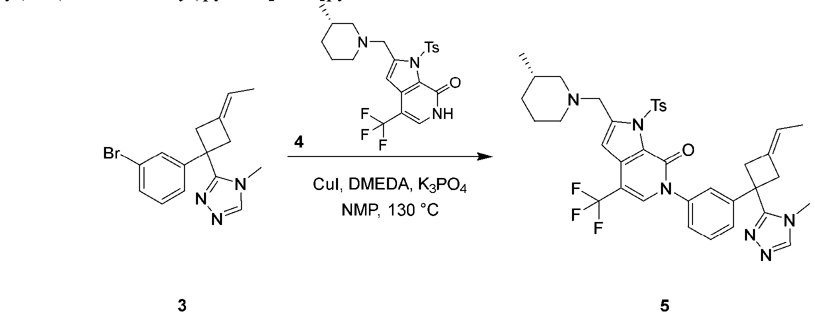

To a solution of 3-[1-(3-bromophenyl)-3-ethylidene-cyclobutyl]-4-methyl-1,2,4-triazole (300 mg, 942 µmol) and 2-[[(3S)-3-methyl-1-piperidyl]methyl]-1-(p-tolylsulfonyl)-4-(trifluoromethyl)-6H-pyrrolo[2,3-c]pyridin-7-one (397 mg, 849 µmol) in NMP (3.00 mL) was added K₃PO₄ (600 mg, 2.83 mmol), CuI (359 mg, 1.89 mmol) and DMEDA (83.5 mg, 947 µmol). The suspension was degassed under vacuum and purged with N₂ several times. The mixture was stirred under N₂ at 130 °C for 1 hr. TLC (Dichloromethane/ Methanol = 10/ 1, product 1 R_{f} = 0.38) indicated new spot formed. The reaction mixture was poured into saturated aqueous NH₄Cl (10 mL) slowly. The mixture was extracted with Ethyl acetate (10.0 mL*2). The organic phase was washed with brine (20.0 mL*3), dried over Na₂SO₄ and concentrated in vacuum. The crude product was purified by column chromatography (SiO₂, Dichloromethane/ Methanol = 1/ 0 to 32/ 1). To afford 6-[3-[3-ethylidene-1-(4-methyl-1,2,4-triazol-3-yl)cyclobutyl]phenyl]-2-[[(3S)-3-methyl-1-piperidyl]methyl]-1-(p-to lylsulfonyl)-4-(trifluoromethyl)pyrrolo[2,3-c]pyridin-7-one (260 mg, 310 µmol, 32.9% yield, 84.2% purity) as a yellow solid which was confirmed by H NMR and LCMS.
¹H NMR: (400 MHz, DMSO-*d*₆)
*δ* 8.43 (s, 1H), 8.41 (s, 1H), 8.39 (s, 1H), 7.98 (s, 1H), 7.46-7.52 (m, 1H), 7.41 (d, *J =* 8.4 Hz, 2H), 7.33-7.38 (m, 1H), 7.29 (s, 1H), 7.19 (d, *J =* 7.6 Hz, 1H), 6.70 (s, 1H), 5.26 (dd, *J =* 4.4, 2.0 Hz, 1H), 3.83-3.98 (m, 2H), 3.57-3.66 (m, 3H), 3.17 (d, *J =* 4.8 Hz, 3H), 2.80 (d, *J* = 9.6 Hz, 2H), 2.40 (s, 3H), 1.99 (d, *J =* 11.2 Hz, 1H), 1.72 (t, *J* = 10.8 Hz, 2H), 1.61-1.67 (m, 2H), 1.51 (s, 5H), 0.87-0.91 (m, 1H), 0.83 (d, *J =* 6.4 Hz, 3H)
LCMS: m/z = 705.0 (M+H)⁺, Rt = 1.347 min

### 3. General steps for preparation of 6-[3-[3-ethyl-1-(4-methyl-1,2,4-triazol-3-yl)cyclobutyl]phenyl]-2-[[(3S)-3-methyl-1-piperidyl]methyl]-1-(p-tolylsu lfonyl)-4-(trifluoromethyl)pyrrolo[2,3-c]pyridin-7-one

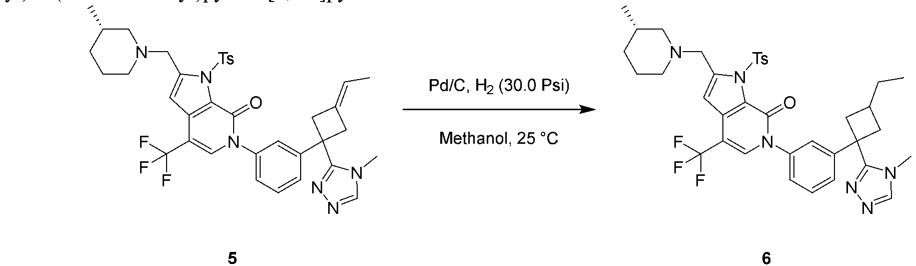

To a solution of 6-[3-[3-ethylidene-1-(4-methyl-1,2,4-triazol-3-yl)cyclobutyl]phenyl]-2-[[(3S)-3-methyl-1-piperidyl]methyl]-1-(p-to lylsulfonyl)-4-(trifluoromethyl)pyrrolo[2,3-c]pyridin-7-one (260 mg, 368 µmol,) in Methanol (10.0 mL) was added Pd/C (118 mg, 110 µmol, 10.0% purity) under Ar. The suspension was degassed under vacuum and purged with H₂ several times. The mixture was stirred under H₂ (30.0 Psi) at 25 °C for 12hrs. LCMS showed desired mass was detected. The reaction mixture was diluted with Methanol (15.0 mL). The mixture was filtered and the filter cake was washed with Methanol (15.0 mL*3). The filtrate was concentrated in vacuum. Without purification. To afford 6-[3-[3-ethyl-1-(4-methyl-1,2,4-triazol-3-yl)cyclobutyl]phenyl]-2-[[(3S)-3-methyl-1-piperidyl]methyl]-1-(p-tolylsu lfonyl)-4-(trifluoromethyl)pyrrolo[2,3-c]pyridin-7-one (230 mg, crude) as a yellow solid.

### 4. General steps for preparation of 6-(3-((1r,3R)-3-ethyl-1-(4-methyl-4H-1,2,4-triazol-3-yl)cyclobutyl)phenyl)-2-(((S)-3-methylpiperidin-1-yl)methyl) -4-(trifluoromethyl)-1,6-dihydro-7H-pyrrolo[2,3-c]pyridin-7-one (compound T092) and 6-(3-((1s,3S)-3-ethyl-1-(4-methyl-4H-1,2,4-triazol-3-yl)cyclobutyl)phenyl)-2-(((S)-3-methylpiperidin-1-yl)methyl) -4-(trifluoromethyl)-1,6-dihydro-7H-pyrrolo[2,3-c]pyridin-7-one (compound T093)

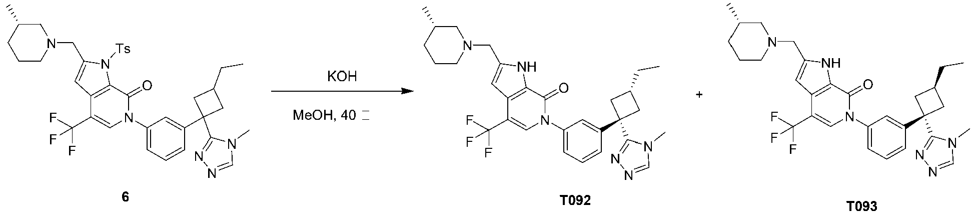

To a solution of 6-[3-[3-ethyl-1-(4-methyl-1,2,4-triazol-3-yl)cyclobutyl]phenyl]-2-[[(3S)-3-methyl-1-piperidyl]methyl]-1-(p-tolylsu lfonyl)-4-(trifluoromethyl)pyrrolo[2,3-c]pyridin-7-one (230 mg, 325 µmol) in Methanol (5.00 mL) was added KOH (365 mg, 6.51 mmol). The mixture was stirred under N₂ at 40 °C for 1 hr. LCMS showed desired mass was detected. The reaction mixture was diluted with brine (10.0 mL) and KOH (1 M, 10.0 mL), extracted with Ethyl acetate (20.0 mL*2). The organic phase washed with KOH (1 M, 20.0 mL), dried over Na₂SO₄ and concentrated in vacuum. The crude product was purified by Prep-HPLC (column: Xtimate C18 150*40 mm*10 um; mobile phase: [water (FA)-ACN]; gradient: 0%-40.0% B over 25 mins). To afford 6-(3-((1r,3R)-3-ethyl-1-(4-methyl-4H-1,2,4-triazol-3-yl)cyclobutyl)phenyl)-2-(((S)-3-methylpiperidin-1-yl)methyl) -4-(trifluoromethyl)-1,6-dihydro-7H-pyrrolo[2,3-c]pyridin-7-one (38.7 mg, 60.7 µmol, 18.6% yield, 94.0% purity, FA) as a white solid which was confirmed by H NMR, F NMR, LCMS, HPLC and SFC.
¹H NMR: (400 MHz, DMSO-*d*₆)
*δ* 12.45 (s, 1H), 8.29 (s, 1H), 7.72 (d, *J* = 1.2 Hz, 1H), 7.47-7.56 (m, 2H), 7.36 (t, *J* = 6.4 Hz, 2H), 6.28 (s, 1H), 3.61 (s, 2H), 3.25 (s, 3H), 2.84 (t, *J =* 10.0 Hz, 2H), 2.71-2.80 (m, 2H), 2.51-2.58 (m, 2H), 2.32 (dt, *J* = 16.4, 8.0 Hz, 1H), 1.90 (t, *J =* 10.4 Hz, 1H), 1.53-1.66 (m, 4H), 1.34-1.49 (m, 3H), 0.82-0.90 (m, 1H), 0.79-0.81 (m, 3H), 0.73-0.79 (m, 3H)
LCMS: m/z = 553.0 (M+H)⁺, Rt = 1.182 min

To afford 6-(3-((1s,3S)-3-ethyl-1-(4-methyl-4H-1,2,4-triazol-3-yl)cyclobutyl)phenyl)-2-(((S)-3-methylpiperidin-1-yl)methyl) -4-(trifluoromethyl)-1,6-dihydro-7H-pyrrolo[2,3-c]pyridin-7-one (11.7 mg, 19.4 µmol, 5.99% yield, 99.7% purity, FA) as a white solid which was confirmed by H NMR and LCMS.
¹H NMR: (400 MHz, DMSO-*d*₆)
*δ* 12.21-12.62 (m, 1H), 8.36 (s, 1H), 7.70 (s, 1H), 7.44-7.52 (m, 1H), 7.37 (s, 1H), 7.33 (d, *J* = 8.0 Hz, 1H), 7.17 (d, *J* = 7.6 Hz, 1H), 6.27 (s, 1H), 3.60 (s, 2H), 3.30 (s, 3H), 3.08 (t, *J* = 9.2 Hz, 2H), 2.75 (s, 2H), 2.22-2.31 (m, 2H), 2.16 (dq, *J* = 16.0, 7.6 Hz, 1H), 1.89 (t, *J =* 10.4 Hz, 1H), 1.53-1.67 (m, 4H), 1.39-1.50 (m, 3H), 0.82-0.90 (m, 1H), 0.81 (br d, *J* = 3.2 Hz, 3H), 0.71-0.80 (m, 3H)
LCMS: m/z = 553.0 (M+H)⁺, Rt = 1.198 min

### Example 94: Synthesis of compound T094

### 1. General steps for preparation of tert-butyl (3R)-4-[[4-bromo-7-methoxy-1-(p-tolylsulfonyl)pyrrolo[2,3-c]pyridin-2-yl]methyl]-3-isopropyl-piperazine-1-carbo xylate

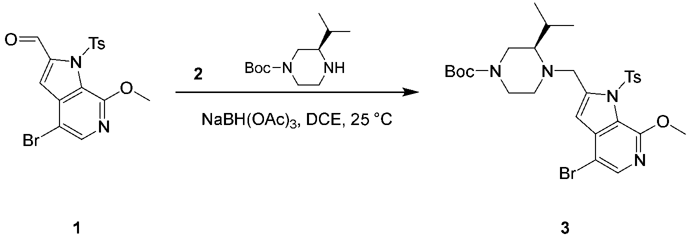

A solution of 4-bromo-7-methoxy-1-(p-tolylsulfonyl)pyrrolo[2,3-c]pyridine-2-carbaldehyde (2.00 g, 4.89 mmol) and tert-butyl (3R)-3-isopropylpiperazine-1-carboxylate (1.17 g, 5.12 mmol) in DCE (30.0 mL). The mixture was stirred under N₂ at 25 °C for 1 hr. Then NaBH(OAc)₃ (3.11 g, 14.7 mmol) was added to the mixture in portions. The mixture was stirred under N₂ at 25 °C for 2 hrs. TLC (PE/ EtOAc = 3/ 1, product 1 R_{f} = 0.50) indicated new spot formed. The reaction mixture was diluted with DCM (100 mL) and poured into brine (100 mL) slowly. The mixture was washed with brine (100 mL), dried over Na₂SO₄. The organic phase was concentrated in vacuum. The crude product was purified by column chromatography (SiO₂, PE/ EtOAc = 1/ 0 to 10/ 1). To afford tert-butyl (3R)-4-[[4-bromo-7-methoxy-1-(p-tolylsulfonyl)pyrrolo[2,3-c]pyridin-2-yl]methyl]-3-isopropyl-piperazine-1-carbo xylate (2.40 g, 3.46 mmol, 70.7% yield, 89.6% purity) as a yellow solid which was confirmed by H NMR and LCMS.
¹H NMR: (400 MHz, CDCl₃)
*δ* 7.92 (d, *J* = 8.4 Hz, 2H), 7.90 (s, 1H), 7.30 (d, *J* = 8.0 Hz, 2H), 6.78 (s, 1H), 4.46-4.71 (m, 1H), 3.84-3.95 (m, 1H), 3.81 (s, 3H), 3.59-3.76 (m, 2H), 3.04-3.30 (m, 2H), 2.88 (d, *J* = 2.4 Hz, 1H), 2.43 (s, 3H), 2.18-2.36 (m, 3H), 1.45-1.50 (m, 9H), 1.02 (d, *J =* 6.4 Hz, 3H), 0.96 (d, *J =* 6.4 Hz, 3H)
LCMS: m/z = 622.9 (M+H)⁺, Rt = 1.547 min

### 2. General steps for preparation of tert-butyl (3R)-4-[[4-cyclopropyl-7-methoxy-1-(p-tolylsulfonyl)pyrrolo[2,3-c]pyridin-2-yl]methyl]-3-isopropyl-piperazine-1-carboxylate

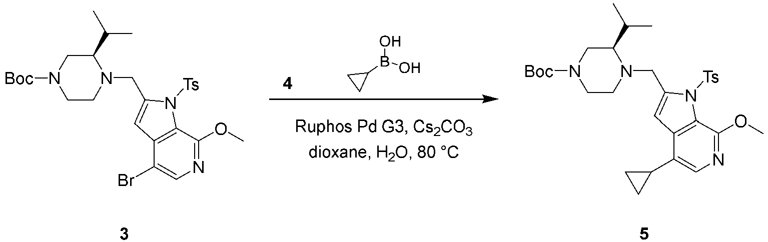

To a solution of tert-butyl (3R)-4-[[4-bromo-7-methoxy-1-(p-tolylsulfonyl)pyrrolo[2,3-c]pyridin-2-yl]methyl]-3-isopropyl-piperazine-1-carbo xylate (1.0 g, 1.61 mmol) and cyclopropylboronic acid (1.38 g, 16.0 mmol) in dioxane (10.0 mL) /H₂O (2.5 mL) was added RuPhos Pd G3 (269 mg, 321 µmol) and Cs₂CO₃ (1.57 g, 4.82 mmol). The suspension was degassed under vacuum and purged with N₂ several times. The mixture was stirred under N₂ at 80 °C for 3hrs. TLC (PE/ EtOAc = 3/ 1, product 1 R_{f} = 0.40) indicated new spot formed. The reaction mixture was diluted with EtOAc (50.0 mL). The mixture was filtered and the filter cake was washed with EtOAc (50.0 mL*3). The filtrate was washed with brine (100 mL), dried over Na₂SO₄. The organic phase was concentrated in vacuum. The crude product was purified by column chromatography (SiO₂, PE/ EtOAc = 1/ 0 to 10/ 1). To afford tert-butyl (3R)-4-[[4-cyclopropyl-7-methoxy-1-(p-tolylsulfonyl)pyrrolo[2,3-c]pyridin-2-yl]methyl]-3-isopropyl-piperazine-1-carboxylate (840 mg, 1.29 mmol, 80.2% yield, 89.5% purity) as a yellow solid which was confirmed by H NMR and LCMS.
¹H NMR: (400 MHz, CDCl₃)
*δ* 7.96 (d, *J* = 8.4 Hz, 2H), 7.55 (s, 1H), 7.28 (d, *J* = 8.4 Hz, 2H), 6.87 (s, 1H), 4.59-4.70 (m, 1H), 3.87-3.99 (m, 1H), 3.78 (s, 3H), 3.59-3.75 (m, 2H), 3.04-3.28 (m, 2H), 2.85-2.93 (m, 1H), 2.42 (s, 3H), 2.18-2.35 (m, 3H), 1.91-1.98 (m, 1H), 1.47 (s, 9H), 1.02 (d, *J =* 6.4 Hz, 3H), 0.96 (d, *J =* 6.4 Hz, 3H), 0.91-0.95 (m, 2H), 0.66-0.70 (m, 2H)
LCMS: m/z = 583.2 (M+H)⁺, Rt = 1.383 min

### 3. General steps for preparation of 4-cyclopropyl-2-[[(2R)-2-isopropylpiperazin-1-yl]methyl]-1-(p-tolylsulfonyl)-6H-pyrrolo[2,3-c]pyridin-7-one

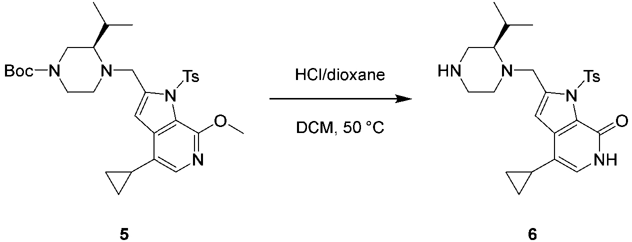

To a solution of tert-butyl (3R)-4-[[4-cyclopropyl-7-methoxy-1-(p-tolylsulfonyl)pyrrolo[2,3-c]pyridin-2-yl]methyl]-3-isopropyl-piperazine-1-carboxylate (840 mg, 1.44 mmol) in DCM (3.00 mL) was added HCl/dioxane (4 M, 10.0 mL). The mixture was stirred under N₂ at 50 °C for 2 hrs. LCMS showed desired mass was detected. The reaction mixture was concentrated in vacuum. Without purification. To afford 4-cyclopropyl-2-[[(2R)-2-isopropylpiperazin-1-yl]methyl]-1-(p-tolylsulfonyl)-6H-pyrrolo[2,3-c]pyridin-7-one (740 mg, crude, HCl) as a yellow solid.

### 4. General steps for preparation of 4-cyclopropyl-2-[[(2R)-2-isopropyl-4-methyl-piperazin-1-yl]methyl]-1-(p-tolylsulfonyl)-6H-pyrrolo[2,3-c]pyridin-7-one

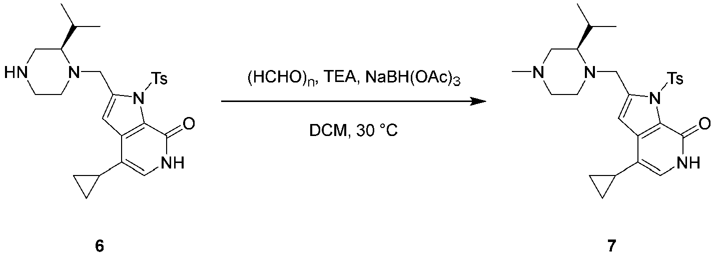

To a solution of 4-cyclopropyl-2-[[(2R)-2-isopropylpiperazin-1-yl]methyl]-1-(p-tolylsulfonyl)-6H-pyrrolo[2,3-c]pyridin-7-one (670 mg, 1.33 mmol, HCl) in DCM (20.0 mL) was added TEA (671 mg, 6.63 mmol) and (HCHO)n (398 mg). The mixture was stirred under N₂ at 30 °C for 0.5 hr. Then NaBH(OAc)₃ (704 mg, 3.32 mmol) was added to the mixture. The mixture was stirred under N₂ at 30 °C for 2 hrs. TLC (DCM/ MeOH = 10/ 1, product 1 R_{f} = 0.30) indicated new spot formed. The reaction mixture was diluted with DCM (50.0 mL) and poured into saturated aqueous NaHCO₃ (50.0 mL) slowly. The mixture was extracted with DCM (50.0 mL), washed with brine (100 mL), dried over Na₂SO₄. The organic phase was concentrated in vacuum. The crude product was purified by column chromatography (SiO₂, DCM/ MeOH = 1/ 0 to 15/ 1). To afford 4-cyclopropyl-2-[[(2R)-2-isopropyl-4-methyl-piperazin-1-yl]methyl]-1-(p-tolylsulfonyl)-6H-pyrrolo[2,3-c]pyridin-7-one (540 mg, 952 µmol, 71.7% yield, 85.1% purity) as a yellow solid which was confirmed by LCMS.
LCMS: m/z = 482.5 (M+H)⁺, Rt = 1.115 min

### 5. General steps for preparation of 4-cyclopropyl-2-(((R)-2-isopropyl-4-methylpiperazin-1-yl)methyl)-6-(3-((1s,3S)-3-methyl-1-(4-methyl-4H-1,2,4-tr iazol-3-yl)cyclobutyl)phenyl)-1-tosyl-1,6-dihydro-7H-pyrrolo[2,3-c]pyridin-7-one

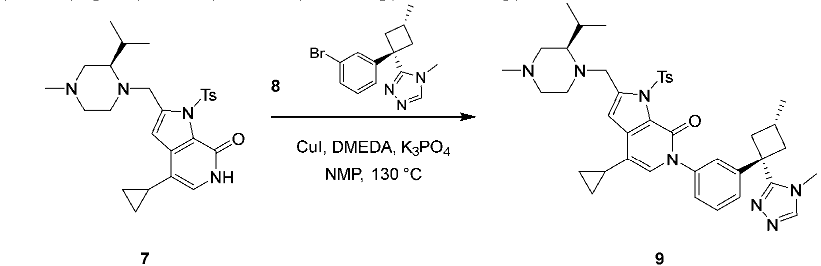

To a solution of 4-cyclopropyl-2-[[(2R)-2-isopropyl-4-methyl-piperazin-1-yl]methyl]-1-(p-tolylsulfonyl)-6H-pyrrolo[2,3-c]pyridin-7-one (100 mg, 207 µmol) and 3-((1s,3s)-1-(3-bromophenyl)-3-methylcyclobutyl)-4-methyl-4H-1,2,4-triazole (63.5 mg, 207 µmol) in NMP (2.00 mL) was added K₃PO₄ (132 mg, 621 µmol), DMEDA (36.5 mg, 414 µmol) and CuI (39.5 mg, 207 µmol). The suspension was degassed under vacuum and purged with N₂ several times. The mixture was stirred under N₂ at 130 °C for 1 hr. TLC (DCM/ MeOH = 10/ 1, product 1 R_{f} = 0.25) indicated new spot formed. The reaction mixture was poured into saturated aqueous NH₄Cl (20.0 mL) slowly. The mixture was extracted with EtOAc (10.0 mL*2). The organic phase was washed with brine (20.0 mL), dried over Na₂SO₄ and concentrated in vacuum. The crude product was purified by column chromatography (SiO₂, DCM/ MeOH = 1/ 0 to 13/ 1). To afford 4-cyclopropyl-2-(((R)-2-isopropyl-4-methylpiperazin-1-yl)methyl)-6-(3-((1s,3S)-3-methyl-1-(4-methyl-4H-1,2,4-tr iazol-3-yl)cyclobutyl)phenyl)-1-tosyl-1,6-dihydro-7H-pyrrolo[2,3-c]pyridin-7-one (30.0 mg, 38.6 µmol, 18.6% yield, 91.2% purity) as a yellow solid which was confirmed by LCMS.
LCMS: m/z = 708.1 (M+H)⁺, Rt = 1.298 min

### 6. General steps for preparation of 4-cyclopropyl-2-((2-isopropyl-4-methylpiperazin-1-yl)methyl)-6-(3-((1s,3s)-3-methyl-1-(4-methyl-4H-1,2,4-triazol -3-yl)cyclobutyl)phenyl)-1,6-dihydro-7H-pyrrolo[2,3-c]pyridin-7-one (compound T094)

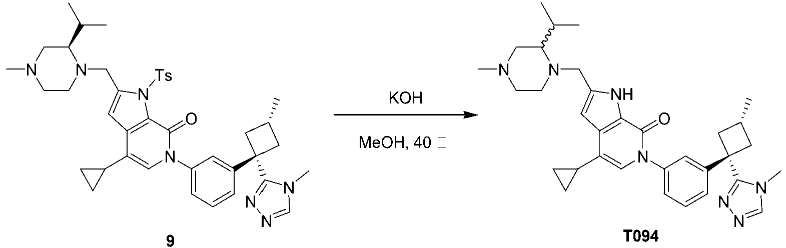

To a solution of 4-cyclopropyl-2-(((R)-2-isopropyl-4-methylpiperazin-1-yl)methyl)-6-(3-((1s,3S)-3-methyl-1-(4-methyl-4H-1,2,4-tr iazol-3-yl)cyclobutyl)phenyl)-1-tosyl-1,6-dihydro-7H-pyrrolo[2,3-c]pyridin-7-one (30.0 mg, 42.4 µmol) in MeOH (4.00 mL) was added KOH (71.3 mg, 1.27 mmol). The mixture was stirred under N₂ at 40 °C for 1hr. LCMS showed desired mass was detected. The reaction mixture was diluted with EtOAc (30.0 mL), washed with brine (30.0 mL), dried over Na₂SO₄. The organic phase was concentrated in vacuum. The crude product was purified by Prep-HPLC (column: Welch Xtimate C18 150*30 mm*5 um; mobile phase: [water (HCl)-ACN]; gradient: 6%-46% B over 25 min). To afford 4-cyclopropyl-2-((2-isopropyl-4-methylpiperazin-1-yl)methyl)-6-(3-((1s,3s)-3-methyl-1-(4-methyl-4H-1,2,4-triazol -3-yl)cyclobutyl)phenyl)-1,6-dihydro-7H-pyrrolo[2,3-c]pyridin-7-one (2.6 mg, 4.20 µmol, 9.90% yield, 95.24% purity, HCl) as a white solid which was confirmed by H NMR and LCMS.
¹H NMR: (400 MHz, CD₃CN)
*δ* 11.95 (s, 1H), 8.86 (s, 1H), 7.53-7.57 (m, 3H), 7.31-7.39 (m, 1H), 6.85 (s, 1H), 6.79 (s, 1H), 4.90 (d, *J =* 13.6 Hz, 1H), 4.47 (d, *J =* 14.0 Hz, 1H), 4.10 (d, *J =* 11.6 Hz, 1H), 3.47-3.78 (m, 6H), 3.44 (s, 3H), 3.36 (d, *J* = 12.0 Hz, 1H), 2.97 (dd, *J =* 10.4, 7.2 Hz, 3H), 2.82 (s, 3H), 2.64 (s, 2H), 1.86-1.91 (m, 1H), 1.22 (d, *J* = 6.4 Hz, 3H), 1.10 (d, *J* = 6.0 Hz, 6H), 0.85-0.90 (m, 2H), 0.66-0.71 (m, 2H)
LCMS: m/z = 554.3 (M+H)⁺, Rt = 1.648 min

### Example 95: Synthesis of compound T095

### 1. General steps for preparation of tert-butyl (3R)-4-[[4-bromo-7-methoxy-1-(p-tolylsulfonyl)pyrrolo[2,3-c]pyridin-2-yl]methyl]-3-isopropyl-piperazine-1-carbo xylate

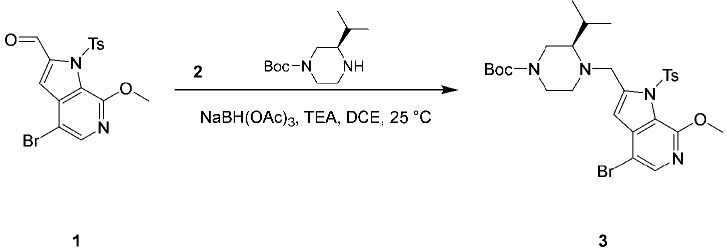

To a solution of 4-bromo-7-methoxy-1-(p-tolylsulfonyl)pyrrolo[2,3-c]pyridine-2-carbaldehyde (1.00 g, 2.44 mmol) and tert-butyl (3R)-3-isopropylpiperazine-1-carboxylate (586 mg, 2.57 mmol) in DCE (15.0 mL) was added TEA (742 mg, 7.33 mmol). The mixture was stirred under N₂ at 25 °C for 1 hr. Then NaBH(OAc)₃ (1.55 g, 7.34 mmol) was added to the mixture in portions. The mixture was stirred under N₂ at 25 °C for 2 hrs. TLC (PE/ EtOAc = 3/ 1, product 1 R_{f} = 0.40) indicated one spot formed. The reaction mixture was diluted with DCM (100 mL) and poured into brine (100 mL) slowly. The mixture was washed with brine (100 mL), dried over Na₂SO₄. The organic phase was concentrated in vacuum. The crude product was purified by column chromatography (SiO₂, PE/ EtOAc = 1/ 0 to 10/ 1). To afford tert-butyl (3R)-4-[[4-bromo-7-methoxy-1-(p-tolylsulfonyl)pyrrolo[2,3-c]pyridin-2-yl]methyl]-3-isopropyl-piperazine-1-carbo xylate (1.30 g, 1.99 mmol, 81.4% yield, 95.1% purity) as a yellow solid which was confirmed by H NMR and LCMS.
¹H NMR: (400 MHz, CDCl₃)
*δ* 7.93 (d, *J =* 8.4 Hz, 2H), 7.89-7.91 (s, 1H), 7.31 (d, *J =* 8.4 Hz, 2H), 6.78 (s, 1H), 4.61 (d, *J =* 16.4 Hz, 1H), 3.85-3.94 (m, 1H), 3.80-3.82 (s, 3H), 3.59-3.79 (m, 2H), 3.00-3.30 (m, 2H), 2.84-2.94 (m, 1H), 2.43 (s, 3H), 2.23-2.40 (m, 3H), 1.47 (s, 9H), 1.02 (d, *J =* 6.4 Hz, 3H), 0.96 (d, *J =* 6.4 Hz, 3H)
LCMS: m/z = 622.8 (M+H)⁺, Rt = 1.540 min

### 2. General steps for preparation of tert-butyl (3R)-4-[[4-cyclopropyl-7-methoxy-1-(p-tolylsulfonyl)pyrrolo[2,3-c]pyridin-2-yl]methyl]-3-isopropyl-piperazine-1-carboxylate

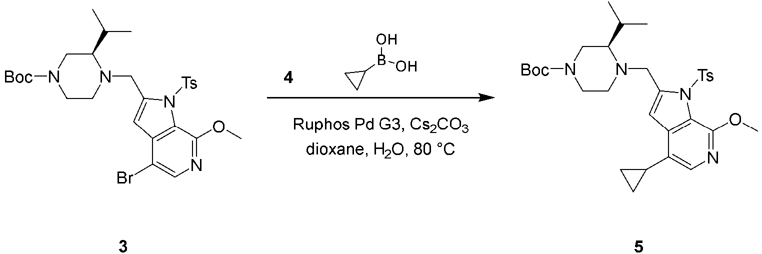

To a solution of tert-butyl (3R)-4-[[4-bromo-7-methoxy-1-(p-tolylsulfonyl)pyrrolo[2,3-c]pyridin-2-yl]methyl]-3-isopropyl-piperazine-1-carbo xylate (1.28 g, 2.06 mmol) and cyclopropylboronic acid (1.77 g, 20.6 mmol) in dioxane (12.0 mL) /H₂O (3.00 mL) was added RuPhos Pd G3 (344 mg, 411 µmol) and Cs₂CO₃ (2.01 g, 6.18 mmol). The suspension was degassed under vacuum and purged with N₂ several times. The mixture was stirred under N₂ at 80 °C for 3 hrs. TLC (PE/ EtOAc = 3/ 1, product 1 R_{f} = 0.40) indicated new spot formed. The reaction mixture was diluted with EtOAc (50 mL). The mixture was filtered and the filter cake was washed with EtOAc (50.0 mL*3). The filtrate was washed with brine (100 mL), dried over Na₂SO₄. The organic phase was concentrated in vacuum. The crude product was purified by column chromatography (SiO₂, PE/ EtOAc = 1/ 0 to 10/ 1). To afford tert-butyl (3R)-4-[[4-cyclopropyl-7-methoxy-1-(p-tolylsulfonyl)pyrrolo[2,3-c]pyridin-2-yl]methyl]-3-isopropyl-piperazine-1-carboxylate (1.10 g, 1.80 mmol, 87.5% yield, 95.5% purity) as a yellow solid which was confirmed by LCMS.
LCMS: m/z = 583.1 (M+H)⁺, Rt = 1.387 min

### 3. General steps for preparation of 4-cyclopropyl-2-[[(2R)-2-isopropylpiperazin-1-yl]methyl]-1-(p-tolylsulfonyl)-6H-pyrrolo[2,3-c]pyridin-7-one

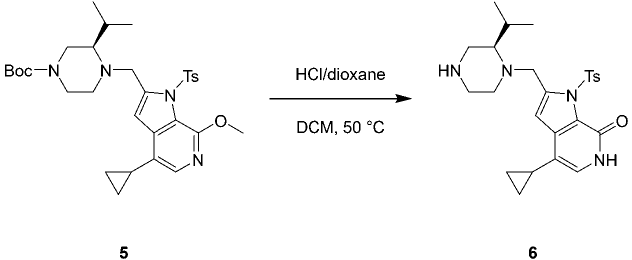

To a solution of tert-butyl (3R)-4-[[4-cyclopropyl-7-methoxy-1-(p-tolylsulfonyl)pyrrolo[2,3-c]pyridin-2-yl]methyl]-3-isopropyl-piperazine-1-carboxylate (1.10 g, 1.89 mmol) in DCM (3.00 mL) was added HCl/dioxane (4 M, 10.0 mL). The mixture was stirred under N₂ at 50 °C for 2 hrs. LCMS showed desired mass was detected. The reaction mixture was concentrated in vacuum. Without purification. To afford 4-cyclopropyl-2-[[(2R)-2-isopropylpiperazin-1-yl]methyl]-1-(p-tolylsulfonyl)-6H-pyrrolo[2,3-c]pyridin-7-one (953 mg, crude, HCl) as a yellow solid.

### 4. General steps for preparation of 4-cyclopropyl-2-[[(2R)-2-isopropyl-4-methyl-piperazin-1-yl]methyl]-1-(p-tolylsulfonyl)-6H-pyrrolo[2,3-c]pyridin-7-one

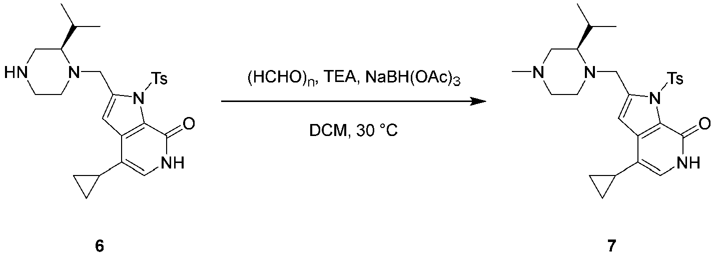

To a solution of 4-cyclopropyl-2-[[(2R)-2-isopropylpiperazin-1-yl]methyl]-1-(p-tolylsulfonyl)-6H-pyrrolo[2,3-c]pyridin-7-one (953 mg, 1.89 mmol, HCl) in DCM (20.0 mL) was added TEA (954 mg, 9.43 mmol) and (HCHO)ₙ (567 mg). The mixture was stirred under N₂ at 30 °C for 0.5 hr. Then NaBH(OAc)₃ (1.00 g, 4.72 mmol) was added to the mixture. The mixture was stirred under N₂ at 30 °C for 12 hrs. TLC (DCM/ MeOH = 10/ 1, product 1 R_{f} = 0.30) indicated new spot formed. The reaction mixture was diluted with DCM (50.0 mL) and poured into saturated aqueous NaHCO₃ (50.0 mL) slowly. The mixture was extracted with DCM (50.0 mL), washed with brine (100 mL), dried over Na₂SO₄. The organic phase was concentrated in vacuum. The crude product was purified by column chromatography (SiO₂, DCM/ MeOH = 1/ 0 to 10/ 1). To afford 4-cyclopropyl-2-[[(2R)-2-isopropyl-4-methyl-piperazin-1-yl]methyl]-1-(p-tolylsulfonyl)-6H-pyrrolo[2,3-c]pyridin-7-one (400 mg, 755 µmol, 40.0% yield, 91.1% purity) as a yellow solid which was confirmed by H NMR and LCMS.
¹H NMR: (400 MHz, CDCl₃)
*δ* 9.35 (s, 1H), 8.21 (d, *J =* 8.4 Hz, 2H), 7.29 (d, *J =* 8.4 Hz, 2H), 6.78 (s, 1H), 6.66 (s, 1H), 4.69 (d, *J =* 16.4 Hz, 1H), 3.66 (d, *J =* 16.4 Hz, 1H), 2.86 (d, *J =* 11.6 Hz, 1H), 2.59-2.80 (m, 2H), 2.41 (s, 3H), 2.33 (s, 3H), 2.05-2.30 (m, 3H), 1.74-1.82 (m, 1H), 1.55-1.67 (s, 2H), 0.98 (d, *J* = 6.8 Hz, 3H), 0.94 (d, *J* = 6.8 Hz, 3H), 0.85-0.90 (m, 2H), 0.48-0.54 (m, 2H)
LCMS: m/z = 483.0 (M+H)⁺, Rt = 0.650 min

### 5. General steps for preparation of 4-cyclopropyl-2-(((R)-2-isopropyl-4-methylpiperazin-1-yl)methyl)-6-(3-((1s,3S)-3-methyl-1-(4-methyl-4H-1,2,4-tr iazol-3-yl)cyclobutyl)phenyl)-1-tosyl-1,6-dihydro-7H-pyrrolo[2,3-c]pyridin-7-one

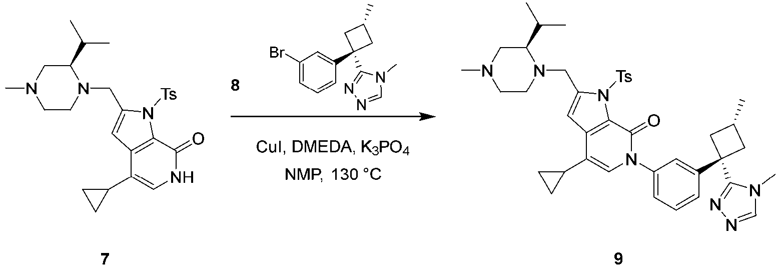

To a solution of 4-cyclopropyl-2-[[(2R)-2-isopropyl-4-methyl-piperazin-1-yl]methyl]-1-(p-tolylsulfonyl)-6H-pyrrolo[2,3-c]pyridin-7-one (100 mg, 207 µmol) and 3-((1s,3s)-1-(3-bromophenyl)-3-methylcyclobutyl)-4-methyl-4H-1,2,4-triazole (69.8 mg, 227 µmol) in NMP (2.00 mL) was added K₃PO₄ (132 mg, 621 µmol), DMEDA (36.5 mg, 414 µmol) and CuI (39.5 mg, 207 µmol). The suspension was degassed under vacuum and purged with N₂ several times. The mixture was stirred under N₂ at 130 °C for 1 hr. TLC (DCM/ MeOH = 10/ 1, product 1 R_{f} = 0.25) indicated new spot formed. The reaction mixture was poured into saturated aqueous NH₄Cl (20.0 mL) slowly. The mixture was extracted with EtOAc (10.0 mL*2). The organic phase was washed with brine (20.0 mL*2), dried over Na₂SO₄ and concentrated in vacuum. The crude product was purified by column chromatography (SiO₂, DCM/ MeOH = 1/ 0 to 13/ 1). To afford 4-cyclopropyl-2-(((R)-2-isopropyl-4-methylpiperazin-1-yl)methyl)-6-(3-((1s,3S)-3-methyl-1-(4-methyl-4H-1,2,4-tr iazol-3-yl)cyclobutyl)phenyl)-1-tosyl-1,6-dihydro-7H-pyrrolo[2,3-c]pyridin-7-one (120 mg, 165 µmol, 80.0% yield, 97.8% purity) as a yellow solid which was confirmed by LCMS.
LCMS: m/z = 708.3 (M+H)⁺, Rt = 1.308 min

### 6. General steps for preparation of 4-cyclopropyl-2-(((R)-2-isopropyl-4-methylpiperazin-1-yl)methyl)-6-(3-((1s,3S)-3-methyl-1-(4-methyl-4H-1,2,4-tr iazol-3-yl)cyclobutyl)phenyl)-1,6-dihydro-7H-pyrrolo[2,3-c]pyridin-7-one (compound T095)

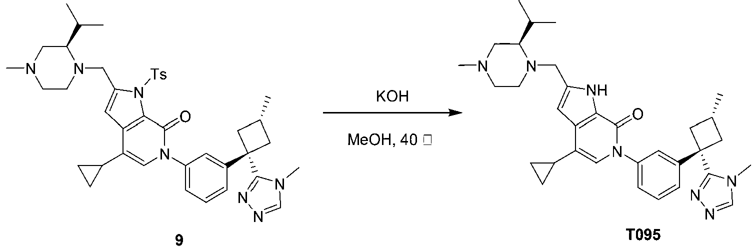

To a solution of 4-cyclopropyl-2-(((R)-2-isopropyl-4-methylpiperazin-1-yl)methyl)-6-(3-((1s,3S)-3-methyl-1-(4-methyl-4H-1,2,4-tr iazol-3-yl)cyclobutyl)phenyl)-1-tosyl-1,6-dihydro-7H-pyrrolo[2,3-c]pyridin-7-one (100 mg, 141 µmol) in MeOH (3.00 mL) was added KOH (238 mg, 4.24 mmol). The mixture was stirred under N₂ at 40 °C for 2 hrs. LCMS showed desired mass was detected. The reaction mixture was diluted with brine (20.0 mL), extracted with EtOAc (20.0 mL*2). The organic phase washed with KOH (1 M, 20 mL), dried over Na₂SO₄ and concentrated in vacuum. The crude product was purified by Prep-HPLC (column: Xtimate C18 150*40 mm*10 um; mobile phase: [water (HCl)-ACN]; gradient: 2.00%-42.0% B over 36 min). To afford 4-cyclopropyl-2-(((R)-2-isopropyl-4-methylpiperazin-1-yl)methyl)-6-(3-((1s,3S)-3-methyl-1-(4-methyl-4H-1,2,4-tr iazol-3-yl)cyclobutyl)phenyl)-1,6-dihydro-7H-pyrrolo[2,3-c]pyridin-7-one (57.4 mg, 92.3 µmol, 65.3% yield, 94.96% purity, HCl) as a white solid which was confirmed by H NMR and LCMS.
¹H NMR: (400 MHz, DMSO-*d*₆)
*δ* 12.38 (s, 1H), 9.38 (s, 1H), 7.52-7.60 (m, 2H), 7.44-7.50 (m, 1H), 7.38 (d, *J =* 8.4 Hz, 1H), 6.92 (s, 1H), 6.66 (s, 1H), 4.17-4.38 (m, 3H), 3.54-3.77 (m, 3H), 3.48 (s, 3H), 3.12-3.40 (m, 4H), 2.97 (dd, *J* = 10.4, 7.2 Hz, 2H), 2.81 (s, 3H), 2.57-2.68 (m, 3H), 1.85-1.94 (m, 1H), 1.09 (d, *J* = 6.4 Hz, 6H), 1.00-1.06 (m, 3H), 0.80-0.86 (m, 2H), 0.68-0.75 (m, 2H)
LCMS: m/z = 554.5 (M+H)⁺, Rt = 1.598 min

### Example 96: Synthesis of compound T096

### 1. General steps for preparation of 6-[3-[1-(4-methyl-l,2,4-triazol-3-yl)cyclobutyl]phenyl]-2-[[2-(trifluoromethoxy)ethylamino]methyl]-4-(trifluorome thyl)-1H-pyrrolo[2,3-c]pyridin-7-one (compound T096)

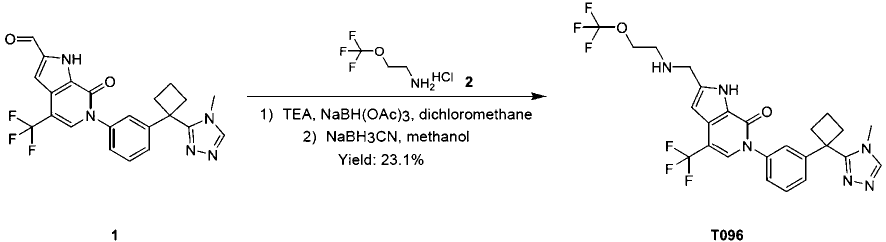

6-[3-[1-(4-methyl-1,2,4-triazol-3-yl)cyclobutyl]phenyl]-7-oxo-4-(trifluoromethyl)-1H-pyrrolo[2,3-c]pyridine-2-carbaldehyde (50.0 mg, 113 µmol) was dissolved in Dichloromethane 2.00 ml, pH was adjusted to 7-8 with TEA. 2-(trifluoromethoxy)ethanamine (28.1 mg, 170 µmol, HCl) in Dichloromethane 2.00 ml was added to the mixture, stirred at 25 °C for0.5 hrs. NaBH(OAc)₃ (60.0 mg, 283 µmol) was added to the mixture , stirred at 25 °C under N₂ for 3 hrs. 2-(trifluoromethoxy)ethanamine (28.1 mg, 170 µmol, HCl) in Dichloromethane 2.00 ml was added to the mixture, stirred at 25 °C for0.5 hrs. NaBH(OAc)₃ (60.0 mg, 283 µmol) was added to the mixture , stirred at 35 °C under N₂ for 3 hrs. NaBH(OAc)₃ (60.0 mg, 283 µmol) and Methanol (1.50 mL) was added to the mixture, the mixture was stirred at 35 °C for 12 hrs. NaBH₃CN (10.68 mg, 169.91 µmol) was added to the mixture, the mixture was stirred at 35 °C for 3 hrs. LCMS shows reactant was consumed completely and desired mass was detected. The reaction mixture was diluted with Dichloromethane 6.00 mL, washed with sat. NaHCO₃ solution 3.00 mL. The water phase extracted with Dichloromethane 12.0 mL (6.00 mL * 2) and Ethyl acetate 12.0 mL (6.0 mL * 2). The combined organic layers were dried over Na₂SO₄, filtered and concentrated under reduced pressure to give a residue. The residue was purified by prep.HPLC (column: Welch Xtimate C18 40 * 200 mm 7 um;mobile phase: [water (FA) - ACN];gradient: 0% - 38% B over 25 min). 6-[3-[1-(4-methyl-1,2,4-triazol-3-yl)cyclobutyl]phenyl]-2-[[2-(trifluoromethoxy)ethylamino]methyl]-4-(trifluorome thyl)-1H-pyrrolo[2,3-c]pyridin-7-one (14.9 mg, 26.2 µmol, 23.1% yield, 97.3% purity) was obtained as white solid, confirmed by LCMS and H NMR.
¹H NMR: (400 MHz, DMSO-*d*₆)
*δ* 12.37 (br s, 1H), 8.35 (s, 1H), 8.14 (s, 1H), 7.72 (d, *J =* 0.8 Hz, 1H), 7.47-7.55 (m, 1H), 7.42 (s, 1H), 7.36 (d, *J* = 8.0 Hz, 1H), 7.26 (d, *J =* 8.0 Hz, 1H), 6.35 (s, 1H), 4.10 (t, *J =* 5.6 Hz, 2H), 3.85 (s, 2H), 3.27 (s, 3H), 2.89-2.98 (m, 2H), 2.77 (t, *J =* 5.6 Hz, 2H), 2.66-2.74 (m, 2H), 1.91-2.07 (m, 2H)
LCMS: m/z = 555.2 (M+H)⁺, Rt = 1.494 min

### Example 97: Synthesis of compound T097

### 1. General steps for preparation of 2-[[2-(cyclopentoxy)ethylamino|methyl]-6-[3-[1-(4-methyl-1,2,4-triazol-3-yl)cyclobutyl|phenyl]-4-(trifluoromethy l)-1H-pyrrolo[2,3-c]pyridin-7-one (compound T097)

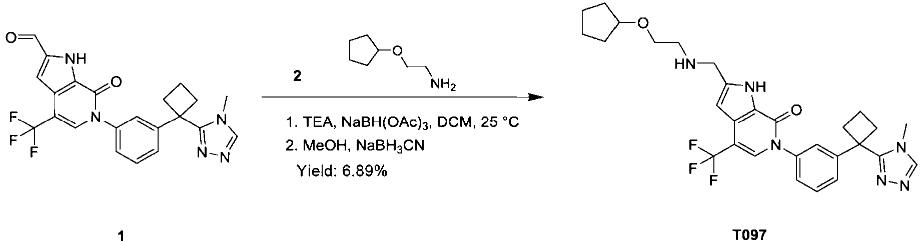

To a solution of 6-[3-[1-(4-methyl-1,2,4-triazol-3-yl)cyclobutyl]phenyl]-7-oxo-4-(trifluoromethyl)-1H-pyrrolo[2,3-c]pyridine-2-car baldehyde (50.0 mg, 113 µmol) in dichloromethane (2.50 mL) was added dropwise TEA (11.4 mg, 113 µmol, 15.7 µL) adjust pH = 7, 2-(cyclopentoxy)ethanamine (16.1 mg, 124 µmol) was added to the mixture, stirred at 25 °C for 0.5 hr, then NaBH(OAc)₃ (60.0 mg, 283 µmol) was added to the mixture, stirred at 25 °C for 1 hr. Methyl alcohol (1.00 mL) was added to the mixture, followed by NaBH₃CN (7.12 mg, 113 µmol) was added slowly, stirred for 1 hr. LCMS showed desired compound was detected. The reaction mixture was diluted with aqueous NaHCO₃ (8.00 mL), extracted with dichloromethane (12.0 mL * 3), the combined organic layers were washed with H₂O (8.00 mL), dried over Na₂SO₄, filtered and concentrated under reduced pressure to give a residue. The residue was purified by prep-HPLC (column: Welch Xtimate C18 40 * 200 mm 7 um; mobile phase: [water (NH₄HCO₃)-ACN]; gradient:26.0%-66.0% B over 25 mins). Compound 2-[[2-(cyclopentoxy)ethylamino]methyl]-6-[3-[1-(4-methyl-1,2,4-triazol-3-yl)cyclobutyl]phenyl]-4-(trifluoromethy l)-1H-pyrrolo[2,3-c]pyridin-7-one (4.33 mg, 7.81 µmol, 6.89% yield) was obtained as a white solid. Confirmed by H NMR and LCMS.
¹H NMR: (DMSO-*d*₆, 400 MHz)
*δ* 11.86-12.84 (m, 1H), 8.35 (s, 1H), 7.71 (s, 1H), 7.46-7.54 (m, 1H), 7.43 (s, 1H), 7.36 (d, *J* = 7.6 Hz, 1H), 7.26 (d, *J* = 7.6 Hz, 1H), 6.32 (s, 1H), 3.83 (s, 1H), 3.81 (s, 2H), 3.37-3.39 (m, 3H), 3.27 (s, 3H), 2.90-2.96 (m, 2H), 2.67-2.73 (m, 2H), 2.58 (t, *J* = 5.6 Hz, 2H), 1.93-2.02 (m, 2H), 1.60-1.67 (m, 2H), 1.50-1.59 (m, 4H), 1.45 (d, *J =* 5.2 Hz, 2H)
LCMS: m/z = 555.3 (M+H)⁺, Rt = 1.372 min

### Example 98: Synthesis of compound T098

### 1. General steps for preparation of 2-[(diisobutylamino)methyl]-6-[3-[1-(4-methyl-1,2,4-triazol-3-yl)cyclobutyl]phenyl]-4-(trifluoromethyl)-1H-pyrrol o[2,3-c]pyridin-7-one (compopund T098)

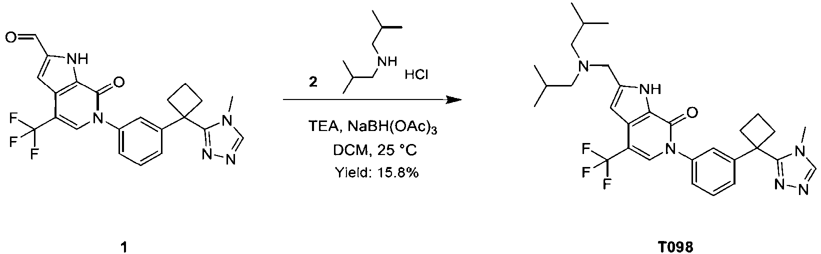

To a solution of6-[3-[1-(4-methyl-1,2,4-triazol-3-yl)cyclobutyl]phenyl]-7-oxo-4-(trifluoromethyl)-1H-pyrrolo[2,3-c]pyridine-2-c arbaldehyde (50.0 mg, 113 µmol) in dichloromethane (2.50 mL) was added dropwise TEA (11.4 mg, 113 µmol, 15.7 µ) adjust pH = 7, N-isobutyl-2-methyl-propan-1-amine (26.2 mg, 158 µmol, 35.5 µL, HCl) was added to the mixture, stirred at 25 °C for 0.5 hr, then NaBH(OAc)₃ (60.0 mg, 283 µmol) was added to the mixture, stirred at 25 °C for 1 hr. LCMS showed desired compound was detected. The reaction mixture was diluted with aqueous NaHCO₃ (8.00 mL), extracted with dichloromethane (12.0 mL * 3), the combined organic layers were washed with H₂O (8.00 mL), dried over Na₂SO₄, filtered and concentrated under reduced pressure to give a residue. The residue was purified by Prep-HPLC (column: Welch Xtimate C18 40 * 200 mm 7 um; mobile phase: [water (NH₄HCO₃)-ACN]; gradient:52.0%-92.0% B over 25 mins). Compound 2-[(diisobutylamino)methyl]-6-[3-[1-(4-methyl-1,2,4-triazol-3-yl)cyclobutyl]phenyl]-4-(trifluoromethyl)-1H-pyrrol o[2,3-c]pyridin-7-one (10.0 mg, 17.9 µmol, 15.8% yield, 99.5% purity) was obtained as a white solid. Confirmed by H NMR and LCMS.
¹H NMR: (DMSO-*d*₆, 400 MHz)
*δ* 12.40 (s, 1H), 8.34 (s, 1H), 7.70 (s, 1H), 7.48-7.53 (m, 1H), 7.45 (s, 1H), 7.36 (d, *J =* 7.6 Hz, 1H), 7.25 (d, *J* = 7.6 Hz, 1H), 6.27 (s, 1H), 3.67 (s, 2H), 3.27 (s, 3H), 2.89-2.97 (m, 2H), 2.65-2.72 (m, 2H), 2.11 (d, *J* = 7.0 Hz, 4H), 1.94-2.03 (m, 2H), 1.77 (dt, *J =* 13.2, 6.6 Hz, 2H), 0.85 (d, *J =* 6.4 Hz, 12H)
LCMS: m/z = 555.3 (M+H)⁺, Rt = 1.677 min

### Examples 99 and 100: Synthesis of compounds T099& T100

### 1. General steps for preparation of 2-[[[1-(methoxymethyl)cyclopropyl]amino]methyl]-6-[3-[3-methoxy-1-(4-methyl-1,2,4-triazol-3-yl)cyclobutyl]phe nyl]-4-(trifluoromethyl)-1H-pyrrolo[2,3-c]pyridin-7-one

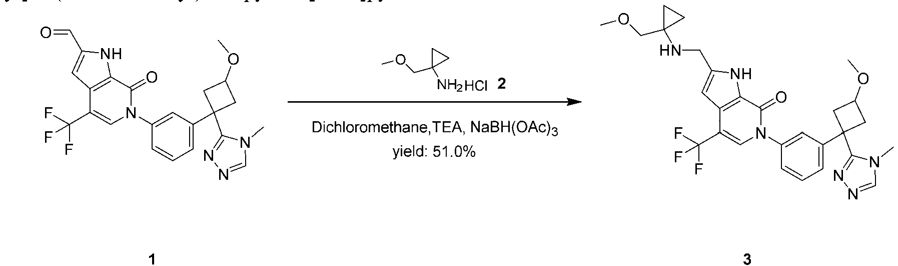

6-[3-[3-methoxy-1-(4-methyl-1,2,4-triazol-3-yl)cyclobutyl]phenyl]-7-oxo-4-(trifluoromethyl)-1H-pyrrolo[2,3-c]pyridine-2-carbaldehyde (70.0 mg, 148 µmol) was dissolved in Dichloromethane (1.00 mL), pH was adjust to 7-8 with TEA. 1-(methoxymethyl)cyclopropanamine (51.1 mg, 371 µmol, HCl) in Dichloromethane 1.00 mL was added to the mixture, stirred at 25 °C for 0.5 hr under N₂. Then NaBH(OAc)₃ (78.7 mg, 371 µmol, 2.5 *eq*) was added to the mixture, then stirred at 25 °C under N₂ for 12 hrs. LCMS shows reactant was consumed completely and desired mass was detected. The reaction mixture was diluted with Dichloromethane 6.00 mL,washed with sat. NaHCO₃ solution 2.00 mL. The water phase extracted with Dichloromethane 2.00 mL (1.00 mL * 2). The combined organic layers were dried over Na₂SO₄, filtered and concentrated under reduced pressure to give a residue. The residue was purified by Prep.TLC (SiO₂, Dichloromethane : Methanol = 10 : 1). TLC (Plate 1, Dichloromethane : Methanol = 10 : 1, product 1 R_{f} = 0.4). 2-[[[1-(methoxymethyl)cyclopropyl]amino]methyl]-6-[3-[3-methoxy-1-(4-methyl-1,2,4-triazol-3-yl)cyclobutyl]phe nyl]-4-(trifluoromethyl)-1H-pyrrolo[2,3-c]pyridin-7-one (45.0 mg, 75.7 µmol, 51.0% yield, 93.6% purity) was obtained as light yellow oil, confirmed by H NMR and LCMS.
¹H NMR: (400 MHz, CD₃OD)
*δ* 8.31-8.39 (m, 1H), 7.61 (dd, *J* = 3.6, 1.6 Hz, 1H), 7.57 (q*, J =* 8.4 Hz, 1H), 7.44-7.52 (m, 1H), 7.28-7.39 (m, 2H), 6.45 (s, 1H), 4.17 (quin, *J =* 7.2 Hz, 1H), 4.03 (s, 2H), 3.93-4.02 (m, 1H), 3.37-3.39 (m, 4H), 3.37 (s, 3H), 3.36-3.37 (m, 1H), 3.33-3.36 (m, 1H), 3.27 (d, *J* = 2.8 Hz, 3H), 3.21 (ddd, *J* = 9.8, 7.2, 2.8 Hz, 1H), 2.83-2.92 (m, 1H), 2.62-2.70 (m, 1H), 0.67-0.72 (m, 2H), 0.60-0.65 (m, 1H), 0.53-0.59 (m, 3H)
LCMS: m/z = 557.3 (M+H)⁺, Rt = 0.914 min

### 2. General steps for preparation of 6-(3-((1r,3r)-3-methoxy-1-(4-methyl-4H-1,2,4-triazol-3-yl)cyclobutyl)phenyl)-2-(((1-(methoxymethyl)cyclopropyl) amino)methyl)-4-(trifluoromethyl)-1,6-dihydro-7H-pyrrolo[2,3-c]pyridin-7-one (compound T099) and 6-(3-((1s,3s)-3-methoxy-1-(4-methyl-4H-1,2,4-triazol-3-yl)cyclobutyl)phenyl)-2-(((1-(methoxymethyl)cyclopropyl )amino)methyl)-4-(trifluoromethyl)-1,6-dihydro-7H-pyrrolo[2,3-c]pyridin-7-one (compound T100)

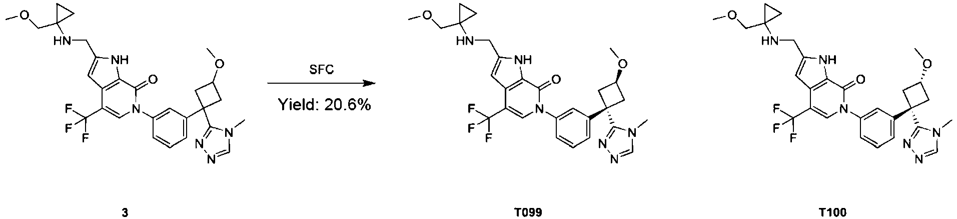

2-[[[1-(methoxymethyl)cyclopropyl]amino]methyl]-6-[3-[3-methoxy-1-(4-methyl-1,2,4-triazol-3-yl)cyclobuty l]phenyl]-4-(trifluoromethyl)-1H-pyrrolo[2,3-c]pyridin-7-one (45.0 mg, 80.8 µmol) was purified by SFC (column: DAICEL CHIRALPAK IC (250 mm * 30 mm,10 um);mobile phase: [CO₂-EtOH(0.1%NH3H2O)];B%: 60%, isocratic elution mode),then purified by prep.HPLC (column: Welch Xtimate C18 40 * 200 mm 7 um;mobile phase: [water (FA) - ACN];gradient: 0% - 36% B over 30 min). 6-(3-((1r,3r)-3-methoxy-1-(4-methyl-4H-1,2,4-triazol-3-yl)cyclobutyl)phenyl)-2-(((1-(methoxymethyl)cyclopropyl) amino)methyl)-4-(trifluoromethyl)-1,6-dihydro-7H-pyrrolo[2,3-c]pyridin-7-one (9.26 mg, 16.6 µmol, 20.6% yield, 100% purity) was obtained as white solid, confirmed by H NMR and LCMS.
¹H NMR: (400 MHz, DMSO-*d*₆)
*δ* 12.30 (br s, 1H), 8.38 (s, 1H), 8.16 (s, 1H), 7.70 (d, *J =* 1.2 Hz, 1H), 7.44-7.55 (m, 1H), 7.30-7.40 (m, 2H), 7.18 (d, *J =* 8.0 Hz, 1H), 6.29 (s, 1H), 3.88 (s, 2H), 3.85 (s, 1H), 3.32 (br d, *J =* 2.0 Hz, 4H), 3.30 (s, 3H), 3.27 (s, 2H), 3.25 (s, 3H), 3.16 (s, 3H), 2.52-2.55 (m, 1H), 0.53-0.60 (m, 2H), 0.40-0.47 (m, 2H)
LCMS: m/z = 557.2 (M+H)⁺, Rt = 1.361 min

6-(3-((1s,3s)-3-methoxy-1-(4-methyl-4H-1,2,4-triazol-3-yl)cyclobutyl)phenyl)-2-(((1-(methoxymethyl)cyclopr opyl)amino)methyl)-4-(trifluoromethyl)-1,6-dihydro-7H-pyrrolo[2,3-c]pyridin-7-one (7.6 mg, 13.64 µmol, 16.9% yield, 99.9% purity) was obtained as white solid, confirmed by H NMR and LCMS.
¹H NMR: (400 MHz, DMSO-*d*₆)
*δ* 12.32 (br s, 1H), 8.31 (s, 1H), 8.20 (s, 1H), 7.71 (s, 1H), 7.48-7.55 (m, 2H), 7.30-7.40 (m, 2H), 6.31 (s, 1H), 4.06 (br t, *J* = 7.2 Hz, 1H), 3.89 (s, 2H), 3.28 (s, 5H), 3.25 (s, 4H), 3.16 (s, 3H), 3.08-3.14 (m, 2H), 2.75-2.82 (m, 2H), 0.53-0.60 (m, 2H), 0.40-0.47 (m, 2H)
LCMS: m/z = 557.2 (M+H)⁺, Rt = 1.336 min

### Example 101: Synthesis of compound T101

### 1. General steps for preparation of 6-(3-fluoro-5-((1s,3R)-3-methyl-1-(4-methyl-4H-1,2,4-triazol-3-yl)cyclobutyl)phenyl)-2-(((S)-3-methylpiperidin-1-yl)methyl)-1-tosyl-4-(trifluoromethyl)-1,6-dihydro-7H-pyrrolo[2,3-c]pyridin-7-one

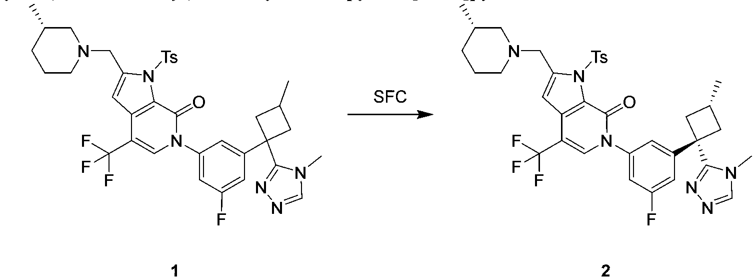

The crude product was purified by SFC (column: DAICEL CHIRALCEL OX (250 mm*30 mm, 10 um); mobile phase: [CO₂ - Methanol (0.10% NH₃H₂O)]; B%:35.0%, isocratic elution mode). To afford 6-(3-fluoro-5-((1s,3R)-3-methyl-1-(4-methyl-4H-1,2,4-triazol-3-yl)cyclobutyl)phenyl)-2-(((S)-3-methylpiperidin-1-yl)methyl)-1-tosyl-4-(trifluoromethyl)-1,6-dihydro-7H-pyrrolo[2,3-c]pyridin-7-one (75.0 mg, 103 µmol, 73.6% yield, 98.2% purity) as a white solid which was confirmed by LCMS.
LCMS: m/z = 711.1 (M+H)⁺, Rt = 1.317 min

### 2. General steps for preparation of 6-(3-fluoro-5-((1s,3R)-3-methyl-1-(4-methyl-4H-1,2,4-triazol-3-yl)cyclobutyl)phenyl)-2-(((S)-3-methylpiperidin-1-yl)methyl)-4-(trifluoromethyl)-1,6-dihydro-7H-pyrrolo[2,3-c]pyridin-7-one (compund T101)

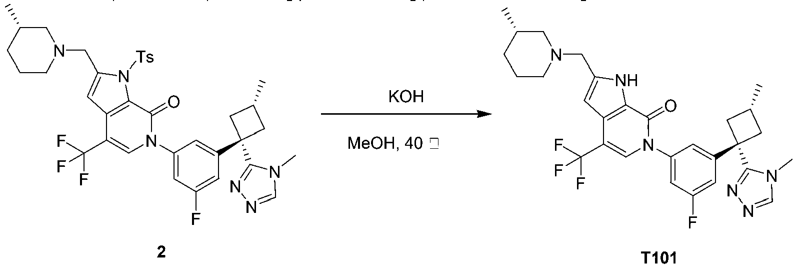

To a solution of 6-(3-fluoro-5-((1s,3R)-3-methyl-1-(4-methyl-4H-1,2,4-triazol-3-yl)cyclobutyl)phenyl)-2-(((S)-3-methylpiperidin-1-yl)methyl)-1-tosyl-4-(trifluoromethyl)-1,6-dihydro-7H-pyrrolo[2,3-c]pyridin-7-one (70.0 mg, 98.4 µmol) in Methanol (3.00 mL) was added KOH (111 mg, 1.98 mmol). The mixture was stirred under N₂ at 40 °C for 1 hr. LCMS showed desired mass was detected. The reaction mixture was diluted with brine (10.0 mL) and KOH (1 M, 10.0 mL), extracted with Ethyl acetate (20.0 mL*2). The organic phase washed with KOH (1 M, 20.0 mL), dried over Na₂SO₄ and concentrated in vacuum. The crude product was purified by Prep-HPLC (column: Xtimate C18 150*40 mm*10 um; mobile phase: [water (FA)-ACN]; gradient: 0%-38.0% B over 25 mins). To afford 6-(3-fluoro-5-((1s,3R)-3-methyl-1-(4-methyl-4H-1,2,4-triazol-3-yl)cyclobutyl)phenyl)-2-(((S)-3-methylpiperidin-1-yl)methyl)-4-(trifluoromethyl)-1,6-dihydro-7H-pyrrolo[2,3-c]pyridin-7-one (28.0 mg, 48.4 µmol, 49.2% yield, 96.4% purity) as a white solid which was confirmed by H NMR and LCMS.
¹H NMR: (400 MHz, DMSO-*d*₆)
*δ* 12.20-12.74 (m, 1H), 8.31 (s, 1H), 7.75 (d, *J* = 1.2 Hz, 1H), 7.37 (dd, *J* = 9.2*,* 2.0 Hz, 1H), 7.35 (s, 1H), 7.18 (d, *J* = 10.0 Hz, 1H), 6.27 (s, 1H), 3.60 (s, 2H), 3.27 (s, 3H), 2.89 (d, *J =* 3.6 Hz, 2H), 2.72-2.81 (m, 2H), 2.53 (d, *J* = 6.4 Hz, 3H), 1.84-1.94 (m, 1H), 1.54-1.64 (m, 4H), 1.39-1.49 (m, 1H), 1.06 (d, *J* = 5.2 Hz, 3H), 0.80 (d, *J* = 5.6 Hz, 3H), 0.75 (s, 1H)
LCMS: m/z = 557.1 (M+H)⁺, Rt = 1.110 min

### Example 102: Synthesis of compound T102

### 1. General steps for preparation of 2-((ethylamino)methyl)-6-(3-(1-(4-methyl-4H-1,2,4-triazol-3-yl)cyclobutyl)phenyl)-4-(trifluoromethyl)-1,6-dihydr o-7H-pyrrolo[2,3-c]pyridin-7-one (compound T102)

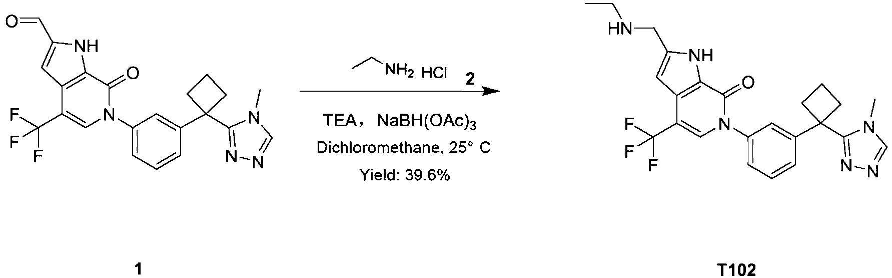

To a mixture of ethanamine (18.4 mg, 226 µmol, 26.8 µL, HCl) in Dichloromethane (3.00 mL) was added TEA (68.7 mg, 679 µmol, 94.6 µL) adjust pH = 8.00. Then 6-[3-[1-(4-methyl-1,2,4-triazol-3-yl)cyclobutyl]phenyl]-7-oxo-4-(trifluoromethyl)-1H-pyrrolo[2,3-c]pyridine-2-car baldehyde (50.0 mg, 113 µmol) was added to the mixture. The mixture was stirred under N₂ at 25.0 °C for 0.50 hr. sodium triacetoxyboranuide (60.0 mg, 283 µmol) was added to the mixture. The mixture was stirred under N₂ at 25.0 ° C for 11.5 hrs. LCMS showed desired mass was detected. The mixture was concentrated under reduced pressure. Then it was diluted with Methanol (7.00 mL). The crude product was purified by Prep-HPLC (column: Welch Xtimate C18 40.0 * 200 mm 7.00 um; mobile phase: [water (NH₃H₂O+NH₄HCO₃)-ACN]; gradient: 16.0%-56.0% B over 25.0 mins). Compound 2-((ethylamino)methyl)-6-(3-(1-(4-methyl-4H-1,2,4-triazol-3-yl)cyclobutyl)phenyl)-4-(trifluoromethyl)-1,6-dihydr o-7H-pyrrolo[2,3-c]pyridin-7-one (21.1 mg, 44.8 µmol, 39.6% yield) was obtained as a white solid, which was confirmed by H NMR and LCMS.
¹H NMR: (400 MHz, DMSO-*d*₆)
*δ* 8.35 (s, 1H), 7.71 (d, *J =* 1.2 Hz, 1H), 7.48-7.55 (m, 1H), 7.43 (t, *J =* 1.6 Hz, 1H), 7.34-7.39 (m, 1H), 7.26 (d, *J* = 8.4 Hz, 1H), 6.32 (s, 1H), 3.79 (s, 2H), 3.27 (s, 3H), 2.90-2.98 (m, 2H), 2.65-2.74 (m, 2H), 2.45-2.49 (m, 2H), 1.90-2.08 (m, 2H), 1.01 (t, *J =* 7.2 Hz, 3H)
LCMS: m/z = 471.2 (M+H)⁺, Rₜ = 1.291 min

### Example 103: Synthesis of compound T103

### 1. General steps for preparation of ethyl 2-(3-bromo-5-fluoro-phenyl)acetate

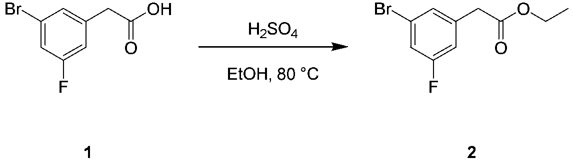

To a solution of 2-(3-bromo-5-fluoro-phenyl)acetic acid (3.00 g, 12.8 mmol) in EtOH (30.0 mL) was added H₂SO₄ (258 mg, 2.58 mmol, 98.0% purity). The mixture was stirred under N₂ at 80 °C for 3 hrs. TLC (Petroleum ether/ Ethyl acetate = 5/ 1, product 1 R_{f} = 0.40) indicated new spot formed. The reaction mixture was diluted with saturated aqueous NaHCO₃ (20.0 mL) slowly, adjust pH = 8 (saturated aqueous NaHCO₃). The mixture was extracted with Ethyl acetate (20.0 mL*2). The organic phase was washed with brine (50.0 mL), dried over Na₂SO₄ and concentrated in vacuum. The crude product was purified by column chromatography (SiO₂, Petroleum ether/ Ethyl acetate = 1/ 0 to 20/ 1). To afford ethyl 2-(3-bromo-5-fluoro-phenyl)acetate (3.30 g, 12.6 mmol, 97.8% yield, 99.7% purity) as colorless oil which was confirmed by H NMR and LCMS.
¹H NMR: (400 MHz, CDCl₃)
*δ* 7.23 (s, 1H), 7.16 (dt, *J =* 8.0, 2.0 Hz, 1H), 6.97 (dd, *J* = 9.2*,* 1.6 Hz, 1H), 4.17 (q, *J =* 7.2 Hz, 2H), 3.57 (s, 2H), 1.27 (t, *J =* 7.2 Hz, 3H)
LCMS: m/z = 262.8 (M+H)⁺, Rt = 0.923 min

### 2. General steps for preparation of ethyl 1-(3-bromo-5-fluoro-phenyl)-3-methyl-cyclobutanecarboxylate

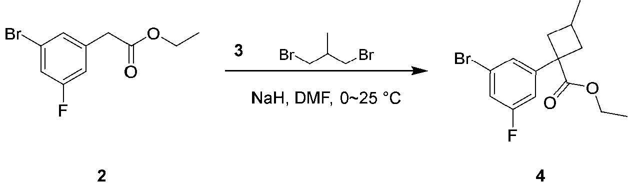

To a solution of ethyl 2-(3-bromo-5-fluoro-phenyl)acetate (2.80 g, 10.7 mmol), 1,3-dibromo-2-methyl-propane (2.43 g, 11.2 mmol) in DMF (28.0 mL) was added NaH (857 mg, 21.4 mmol, 60.0% purity) at 0°C. The mixture was stirred under N₂ at 25 °C for 2 hrs. TLC (Petroleum ether/ Ethyl acetate = 5/ 1, product 1 R_{f} = 0.20) indicated new spot formed. The reaction mixture was poured into saturated aqueous NH₄Cl (50.0 mL) slowly. The reaction mixture was extracted with Ethyl acetate (50.0 mL*2). The organic phase was washed with brine (50.0 mL*3), dried over Na₂SO₄ and concentrated in vacuum. The crude product was purified by column chromatography (SiO₂, Petroleum ether/ Ethyl acetate = 1/ 0 to 10/ 1). To afford ethyl 1-(3-bromo-5-fluoro-phenyl)-3-methyl-cyclobutanecarboxylate (2.10 g, 5.75 mmol, 53.6% yield, 86.3% purity) as colorless oil which was confirmed by LCMS.
LCMS: m/z = 314.8 (M+H)⁺, Rt = 1.443 min

### 3. General steps for preparation of 1-(3-bromo-5-fluoro-phenyl)-3-methyl-cyclobutanecarboxylic acid

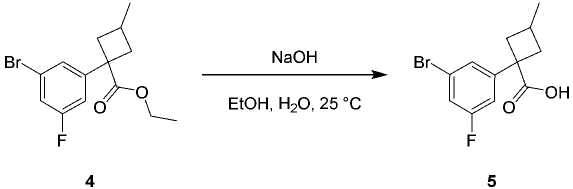

A mixture of ethyl 1-(3-bromo-5-fluoro-phenyl)-3-methyl-cyclobutanecarboxylate (1.90 g, 6.03 mmol) in EtOH (20.0 mL). Then a solution of NaOH (1.45 g, 36.1 mmol) in H₂O (5.00 mL) was added to the mixture. The mixture was stirred under N₂ at 25 °C for 2 hrs. LCMS showed desired mass was detected. The reaction mixture was concentrated in vacuum to remove EtOH. The residue was diluted with water (20.0 mL), adjust pH = 5 (HCl, 2 M). The mixture was extracted with Ethyl acetate (50.0 mL*2). The organic phase was dried over Na₂SO₄ and concentrated in vacuum. Without purification. To afford 1-(3-bromo-5-fluoro-phenyl)-3-methyl-cyclobutanecarboxylic acid (2.80 g, crude) as a white solid.

### 4. General steps for preparation of 1-[[1-(3-bromo-5-fluoro-phenyl)-3-methyl-cyclobutanecarbonyl]amino]-3-methyl-thiourea

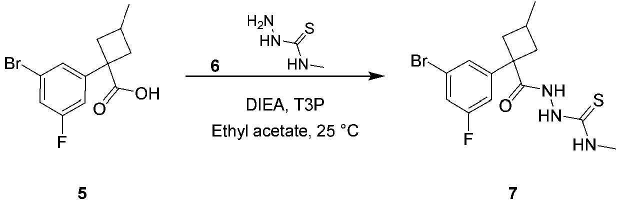

To a solution of 1-(3-bromo-5-fluoro-phenyl)-3-methyl-cyclobutanecarboxylic acid (2.80 g, 9.75 mmol) and 1-amino-3-methyl-thiourea (1.33 g, 12.6 mmol) in Ethyl acetate (28.0 mL) was added DIEA (12.6 g, 97.4 mmol, 16.9 mL), T3P (19.0 g, 29.9 mmol, 17.8 mL, 50.0% purity). The mixture was stirred under N₂ at 25 °C for 2 hrs. LCMS showed desired mass was detected. The reaction mixture was diluted with saturated aqueous NH₄Cl (50.0 mL), extracted with EtOAc (20.0 mL*2). The organic phase washed with brine (50.0 mL), dried over Na₂SO₄ and concentrated in vacuum. Without purification. To afford 1-[[1-(3-bromo-5-fluoro-phenyl)-3-methyl-cyclobutanecarbonyl]amino]-3-methyl-thiourea (4.30 g, crude) as yellow oil.

### 5. General steps for preparation of 5-[1-(3-bromo-5-fluoro-phenyl)-3-methyl-cyclobutyl]-4-methyl-1,2,4-triazole-3-thiol

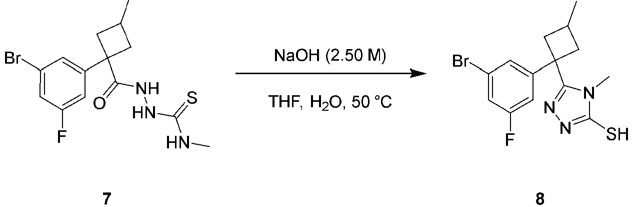

A mixture of 1-[[1-(3-bromo-5-fluoro-phenyl)-3-methyl-cyclobutanecarbonyl]amino]-3-methyl-thiourea (4.00 g, 10.6 mmol) in THF (10.0 mL). Then a solution of NaOH (4.00 g, 100 mmol) in H₂O (30.0 mL) was added to the mixture. The mixture was stirred under N₂ at 50 °C for 12 hrs. TLC (Petroleum ether/ Ethyl acetate = 3/ 1, product 1 R_{f} = 0.40) indicated new spot formed. The reaction mixture was diluted with H₂O (50.0 mL) and adjust pH = 4 (HCl, 2 M), extracted with Ethyl acetate (50.0 mL*2). The organic phase was dried over Na₂SO₄ and concentrated in vacuum. The crude product was purified by column chromatography (SiO₂, Petroleum ether/ Ethyl acetate = 1/ 0 to 4/ 1). To afford 5-[1-(3-bromo-5-fluoro-phenyl)-3-methyl-cyclobutyl]-4-methyl-1,2,4-triazole-3-thiol (1.90 g, 4.51 mmol, 42.2% yield, 84.6% purity) as a yellow solid which was confirmed by H NMR and LCMS.
¹H NMR: (400 MHz, DMSO-*d*₆)
*δ* 13.65-13.89 (m, 1H), 7.45-7.54 (m, 1H), 7.29-7.32 (m, 1H), 7.13-7.27 (m, 1H), 2.99-3.04 (m, 3H), 2.75-2.93 (m, 2H), 2.39-2.47 (m, 2H), 2.17-2.34 (m, 1H), 1.04-1.07 (m, 3H)
LCMS: m/z = 357.8 (M+H)⁺, Rt = 0.893 min

### 6. General steps for preparation of 3-(1-(3-bromo-5-fluorophenyl)-3-methylcyclobutyl)-4-methyl-4H-1,2,4-triazole and 3-((1r,3r)-1-(3-bromo-5-fluorophenyl)-3-methylcyclobutyl)-4-methyl-4H-1,2,4-triazole

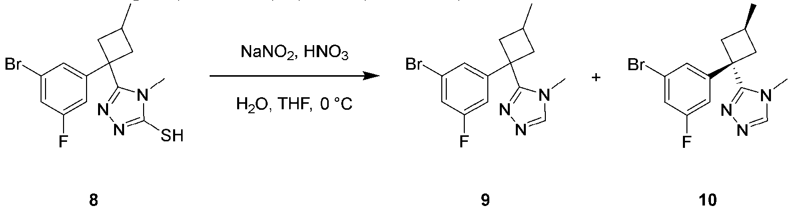

To a solution of 5-[1-(3-bromo-5-fluoro-phenyl)-3-methyl-cyclobutyl]-4-methyl-1,2,4-triazole-3-thiol (1.90 g, 5.33 mmol) in THF (19.0 mL) and H₂O (19.0 mL) was added NaNO₂ (3.68 g, 53.3 mmol). HNO₃ (5.33 g, 54.9 mmol, 3.81 mL, 65.0% purity) was added to the mixture slowly at 0 °C. After addition, the mixture was stirred at 0 °C for 1 hr. TLC (Dichloromethane/ Methanol = 10/ 1, product 1 R_{f} = 0.40) indicated new spot formed. The reaction mixture was poured into saturated aqueous NaHCO₃ (50.0 mL) slowly, adjust pH = 8 (saturated aqueous NaHCO₃). The mixture was extracted with Ethyl acetate (50.0 mL*2). The organic phase was washed with brine (50.0 mL), dried over Na₂SO₄ and concentrated in vacuum. The crude product was purified by column chromatography (SiO₂, Petroleum ether/ Ethyl acetate = 1/ 0 to 19/ 1) and Prep-HPLC (column: Welch Xtimate C18 250*50 mm*10 um; mobile phase: [water (FA)-ACN]; gradient: 15.0%-55.0% B over 25 mins). To afford 3-(1-(3-bromo-5-fluorophenyl)-3-methylcyclobutyl)-4-methyl-4H-1,2,4-triazole (1.30 g, 3.31 mmol, 62.1% yield, 82.6% purity) as a white solid. To afford 3-((1r,3r)-1-(3-bromo-5-fluorophenyl)-3-methylcyclobutyl)-4-methyl-4H-1,2,4-triazole (90.0 mg, 274 µmol, 5.15% yield, 98.9% purity) as a white oil.

### 7. General steps for preparation of 6-(3-fluoro-5-((1r,3S)-3-methyl-1-(4-methyl-4H-1,2,4-triazol-3-yl)cyclobutyl)phenyl)-2-(((S)-3-methylpiperidin-1-yl)methyl)-1-tosyl-4-(trifluoromethyl)-1,6-dihydro-7H-pyrrolo[2,3-c]pyridin-7-one

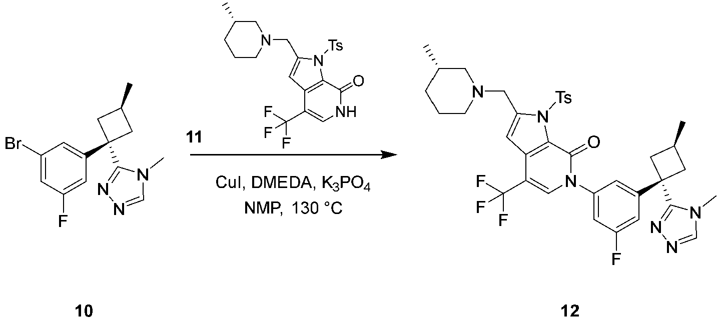

To a solution of 3-((1r,3r)-1-(3-bromo-5-fluorophenyl)-3-methylcyclobutyl)-4-methyl-4H-1,2,4-triazole (80.0 mg, 246 µmol) and 2-[[(3S)-3-methyl-1-piperidyl]methyl]-1-(p-tolylsulfonyl)-4-(trifluoromethyl)-6H-pyrrolo[2,3-c]pyridin-7-one (110 mg, 235 µmol) in NMP (1.50 mL) was added K₃PO₄ (157 mg, 739 µmol), CuI (93.8 mg, 492 µmol) and DMEDA (21.8 mg, 247 µmol). The suspension was degassed under vacuum and purged with N₂ several times. The mixture was stirred under N₂ at 130 °C for 2 hrs. TLC (Dichloromethane/ Methanol = 10/ 1, product 1 R_{f} = 0.37) indicated new spot formed. The reaction mixture was poured into saturated aqueous NH₄Cl (30 mL) slowly. The mixture was extracted with Ethyl acetate (20.0 mL*2). The organic phase was washed with brine (30.0 mL*3), dried over Na₂SO₄ and concentrated in vacuum. The crude product was purified by column chromatography (SiO₂, Dichloromethane/ Methanol = 1/ 0 to 32/ 1). To afford 6-(3-fluoro-5-((1r,3S)-3-methyl-1-(4-methyl-4H-1,2,4-triazol-3-yl)cyclobutyl)phenyl)-2-(((S)-3-methylpiperidin-1-yl)methyl)-1-tosyl-4-(trifluoromethyl)-1,6-dihydro-7H-pyrrolo[2,3-c]pyridin-7-one (60.0 mg, 67.7 µmol, 27.4% yield, 80.3% purity) as a yellow solid which was confirmed by LCMS.
LCMS: m/z = 711.1 (M+H)⁺, Rt = 1.372 min

### 8. General steps for preparation of 6-(3-fluoro-5-((1r,3S)-3-methyl-1-(4-methyl-4H-1,2,4-triazol-3-yl)cyclobutyl)phenyl)-2-(((S)-3-methylpiperidin-1-yl)methyl)-4-(trifluoromethyl)-1,6-dihydro-7H-pyrrolo[2,3-c]pyridin-7-one (compound T103)

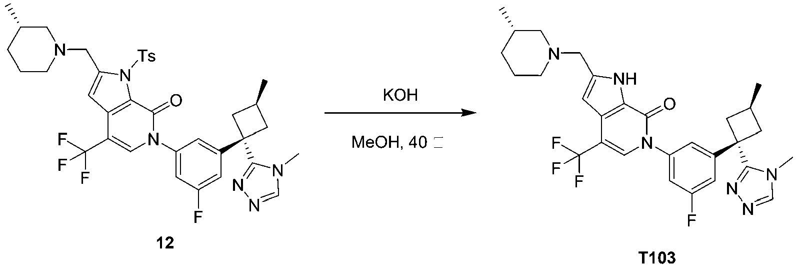

To a solution of 6-(3-fluoro-5-((1r,3S)-3-methyl-1-(4-methyl-4H-1,2,4-triazol-3-yl)cyclobutyl)phenyl)-2-(((S)-3-methylpiperidin-1-yl)methyl)-1-tosyl-4-(trifluoromethyl)-1,6-dihydro-7H-pyrrolo[2,3-c]pyridin-7-one (55.0 mg, 77.3 µmol) in MeOH (3.00 mL) was added KOH (130 mg, 2.32 mmol). The mixture was stirred under N₂ at 40 °C for 1 hr. LCMS showed desired mass was detected. The reaction mixture was diluted with brine (20.0 mL), extracted with Ethyl acetate (20.0 mL*2). The organic phase washed with KOH (1 M, 20.0 mL), dried over Na₂SO₄ and concentrated in vacuum. The crude product was purified by Prep-HPLC (column: Xtimate C18 150*40 mm*10 um; mobile phase: [water (FA) - ACN]; gradient: 0%-40.0% B over 25 mins). To afford 6-(3-fluoro-5-((1r,3S)-3-methyl-1-(4-methyl-4H-1,2,4-triazol-3-yl)cyclobutyl)phenyl)-2-(((S)-3-methylpiperidin-1-yl)methyl)-4-(trifluoromethyl)-1,6-dihydro-7H-pyrrolo[2,3-c]pyridin-7-one (23.0 mg, 37.2 µmol, 48.1% yield, 97.7% purity, FA) as a white solid which was confirmed by H NMR and LCMS.
¹H NMR: (400 MHz, DMSO-*d*₆)
*δ* 12.47 (s, 1H), 8.38 (s, 1H), 7.74 (d, *J* = 0.8 Hz, 1H), 7.35 (d, *J* = 9.2 Hz, 1H), 7.19 (s, 1H), 7.03 (d, *J* = 9.6 Hz, 1H), 6.27 (s, 1H), 3.61 (s, 2H), 3.31 (s, 3H), 3.08-3.13 (m, 2H), 2.73-2.79 (m, 2H), 2.25-2.36 (m, 3H), 1.90 (t, *J* = 10.4 Hz, 1H), 1.55-1.66 (m, 4H), 1.39-1.50 (m, 1H), 1.09 (d, *J* = 5.6 Hz, 3H), 0.81 (d, *J* = 5.6 Hz, 3H), 0.75 (m, 1H)
LCMS: m/z = 557.3 (M+H)⁺, Rt = 1.445 min

### Example 104: Synthesis of compound T104

### 1. General steps for preparation of 2-((isopropylamino)methyl)-6-(3-(1-(4-methyl-4H-1,2,4-triazol-3-yl)cyclobutyl)phenyl)-4-(trifluoromethyl)-1,6-di hydro-7H-pyrrolo[2,3-c]pyridin-7-one (compound T104)

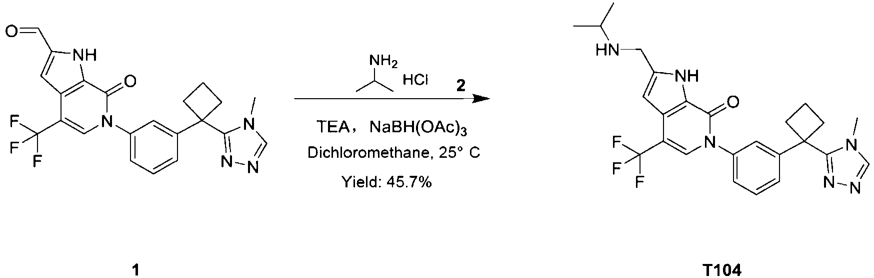

To a mixture of propan-2-amine (21.6 mg, 226 µmol, 31.4 µL, HCl) in Dichloromethane (3.00 mL) was added TEA (68.7 mg, 679 µmol, 94.6 µL) adjust pH = 8.00. Then 6-[3-[1-(4-methyl-1,2,4-triazol-3-yl)cyclobutyl]phenyl]-7-oxo-4-(trifluoromethyl)-1H-pyrrolo[2,3-c]pyridine-2-car baldehyde (50.0 mg, 113 µmol) was added to the mixture. The mixture was stirred under N₂ at 25.0 °C for 0.50 hr. sodium triacetoxyboranuide (60.0 mg, 283 µmol) was added to the mixture. The mixture was stirred under N₂ at 25.0 °C for 11.5 hrs. LCMS showed desired mass was detected. The mixture was concentrated under reduced pressure. Then it was diluted with Methanol (7.00 mL). The crude product was purified by Prep-HPLC (column: Welch Xtimate C18 40.0 * 200 mm 7.00 um; mobile phase: [water(NH₃H₂O+NH₄HCO₃)-ACN]; gradient: 18.0%-58.0% B over 25 mins). Compound 2-((isopropylamino)methyl)-6-(3-(1-(4-methyl-4H-1,2,4-triazol-3-yl)cyclobutyl)phenyl)-4-(trifluoromethyl)-1,6-di hydro-7H-pyrrolo[2,3-c]pyridin-7-one (25.1 mg, 51.8 µmol, 45.7% yield, 100% purity) was obtained as a white solid, which was confirmed by H NMR and LCMS.
¹H NMR: (400 MHz, DMSO-*d*₆)
*δ* 8.35 (s, 1H), 7.71 (d, *J =* 1.2 Hz, 1H), 7.47-7.55 (m, 1H), 7.43 (s, 1H), 7.36 (br d, *J* = 8.0 Hz, 1H), 7.26 (br d, *J* = 8.0 Hz, 1H), 6.34 (s, 1H), 3.80 (s, 2H), 3.27 (s, 3H), 2.90-2.98 (m, 2H), 2.62-2.73 (m, 3H), 1.92-2.05 (m, 2H), 0.97 (d, *J =* 6.0 Hz, 6H)
LCMS: m/z = 485.2 (M+H)⁺, Rₜ = 1.346 min

### Example 105: Synthesis of compound T105

### 1. General steps for preparation of 2-[(5,5-diffuoro-2-azabicyclo[2.2.1]heptan-2-yl)methyl]-6-[3-[1-(4-methyl-1,2,4-triazol-3-yl)cyclobutyl]phenyl]-4-(trifluoromethyl)-1H-pyrrolo[2,3-c]pyridin-7-one (compound T105)

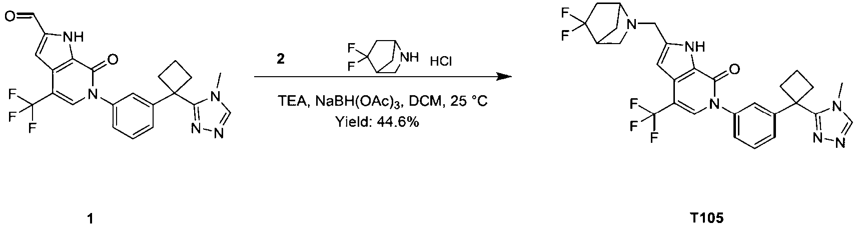

To a solution of 6-[3-[1-(4-methyl-1,2,4-triazol-3-yl)cyclobutyl]phenyl]-7-oxo-4-(trifluoromethyl)-1H-pyrrolo[2,3-c]pyridine-2-car baldehyde (45.0 mg, 101 µmol) in dichloromethane (2.50 mL) was added dropwise TEA (10.3 mg, 101 µmol, 14.1 µL) adjust pH = 7, 5,5-difluoro-2-azabicyclo[2.2.1]heptane (20.3 mg, 120 µmol, HCl) was added to the mixture, stirred at 25 °C for 0.5 hr, then NaBH(OAc)₃ (54.0 mg, 254 µmol) was added to the mixture, stirred at 25 °C for 1 hr. LCMS showed desired compound was detected. The reaction mixture was diluted with aqueous NaHCO₃ (8.00 mL), extracted with dichloromethane (12.0 mL * 3), the combined organic layers were washed with H₂O (8.00 mL), dried over Na₂SO₄, filtered and concentrated under reduced pressure to give a residue. The residue was purified by Prep-HPLC (column: Welch Xtimate C18 40 * 200 mm 7 um; mobile phase: [water (NH₄HCO₃)-ACN]; gradient:26%-66% B over 25 mins). Compound 2-[(5,5-difluoro-2-azabicyclo [2.2.1]heptan-2-yl)methyl]-6-[3-[1-(4-methyl-1,2,4-triazol-3-yl)cyclobutyl]phenyl]-4-(trifluoromethyl)-1H-pyrrolo[ 2,3-c]pyridin-7-one (25.6 mg, 45.5 µmol, 44.6% yield, 99.4% purity) was obtained as a colorless oil. Confirmed by H NMR and LCMS.
¹H NMR: (DMSO-*d*₆, 400 MHz)
*δ* 12.38-12.55 (m, 1H), 8.29-8.48 (m, 1H), 7.73 (s, 1H), 7.48-7.55 (m, 1H), 7.42 (s, 1H), 7.36 (d, *J =* 7.6 Hz, 1H), 7.27 (d, *J =* 7.6 Hz, 1H), 6.21-6.51 (m, 1H), 3.60-4.03 (m, 2H), 3.27 (s, 3H), 2.91-3.00 (m, 2H), 2.63-2.76 (m, 5H), 2.33 (s, 1H), 2.17 (td, *J =* 5.6, 1.6 Hz, 1H), 1.90-2.06 (m, 3H), 1.64-1.86 (m, 2H)
LCMS: m/z =559.2 (M+H)⁺, Rt = 1.370 min

### Example 106: Synthesis of compound T106

### 1. General steps for preparation of 2-[[3-fluoro-5-(hydroxymethyl)-1-piperidyl]methyl]-6-[3-[1-(4-methyl-1,2,4-triazol-3-yl)cyclobutyl]phenyl]-4-(trif luoromethyl)-1H-pyrrolo[2,3-c]pyridin-7-one-one (compound T106)

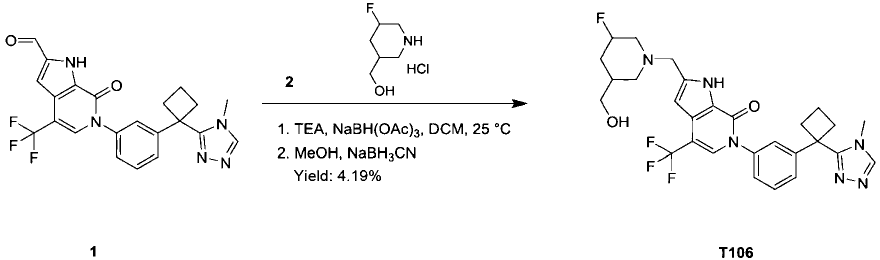

To a solution of 6-[3-[1-(4-methyl-1,2,4-triazol-3-yl)cyclobutyl]phenyl]-7-oxo-4-(trifluoromethyl)-1H-pyrrolo[2,3-c]pyridine-2-car baldehyde (45.0 mg, 101 µmol) in dichloromethane (2.50 mL) was added dropwise TEA (10.3 mg, 101 µmol, 14.1 µL) adjust pH = 7, (5-fluoro-3-piperidyl)methanol (20.36 mg, 120.05 µmol, 1.18 eq, HCl) was added to the mixture, stirred at 25 °C for 0.5 hr, then NaBH(OAc)₃ (54.0 mg, 254 µmol) was added to the mixture, stirred at 25 °C for 1 hr. Methyl alcohol (1.00 mL) was added to the mixture and stirred at 25 °C for 10 min, NaBH₃CN (6.41 mg, 101 µmol) was added to the mixture and stirred at 25 °C for 1 hr. LCMS showed desired compound was detected. The reaction mixture was diluted with aqueous NaHCO₃ (8.00 mL), extracted with dichloromethane (12.0 mL * 3), the combined organic layers were washed with H₂O (8.00 mL), dried over Na₂SO₄, filtered and concentrated under reduced pressure to give a residue. The residue was purified by Prep-HPLC (column: Welch Xtimate C18 40 * 200 mm 7 um; mobile phase: [water (HCl)-ACN]; gradient:0%-36% B over 30 mins). Compound 2-[[3-fluoro-5-(hydroxymethyl)-1-piperidyl]methyl]-6-[3-[1-(4-methyl-l,2,4-triazol-3-yl)cyclobutyl]phenyl]-4-(trif luoromethyl)-1H-pyrrolo[2,3-c]pyridin-7-one (2.41 mg, 4.27 µmol, 4.19% yield, 98.9% purity) was obtained as a white solid. Confirmed by H NMR and LCMS.
¹H NMR: (DMSO-*d*₆, 400 MHz)
*δ* 12.86 (s, 1H), 8.95 (s, 1H), 7.84 (s, 1H), 7.54-7.60 (m, 1H), 7.45 (s, 1H), 7.37-7.43 (m, 2H), 6.72 (s, 1H), 5.09-5.25 (m, 1H), 4.46-4.56 (m, 2H), 3.36 (s, 3H), 3.27 (dd, *J* = 10.8, 6.4 Hz, 2H), 2.94-3.00 (m, 2H), 2.71-2.80 (m, 3H), 2.27-2.39 (m, 1H), 2.22 (s, 1H), 1.91-2.10 (m, 4H), 1.75 (s, 1H), 1.23 (s, 2H)
LCMS: m/z =559.2 (M+H)⁺, Rt = 1.253 min

### Example 107: Synthesis of compound T107

### 1. General steps for preparation of 2-(((3S,4R)-3-fluoro-4-methoxypiperidin-1-yl)methyl)-6-(3-(1-(4-methyl-4H-1,2,4-triazol-3-yl)cyclobutyl)phenyl)-4-(trifluoromethyl)-1,6-dihydro-7H-pyrrolo[2,3-c]pyridin-7-one (compound T107)

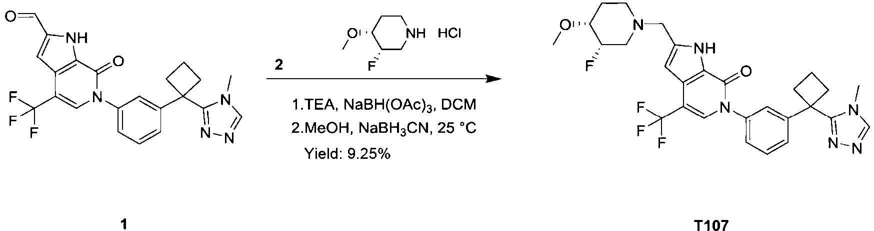

To a solution of6-[3-[1-(4-methyl-1,2,4-triazol-3-yl)cyclobutyl]phenyl]-7-oxo-4-(trifluoromethyl)-1H-pyrrolo[2,3-c]pyridine-2-c arbaldehyde (50.0 mg, 113 µmol) in dichloromethane (2.50 mL) was added dropwise TEA (11.4 mg, 113 µmol, 15.7 µL) adjust pH = 7, (3S,4R)-3-fluoro-4-methoxy-piperidine (24.9 mg, 147 µmol, HCl) was added to the mixture, stirred at 25 °C for 0.5 hr, then NaBH(OAc)₃ (60.0 mg, 283 µmol) was added to the mixture, stirred at 25 °C for 1 hr. Then NaBH₃CN (7.12 mg, 113 µmol) and methyl alcohol (1.00 mL) was added to the mixture and stirred for 1 hr. LCMS showed desired compound was detected. The reaction mixture was diluted with aqueous NaHCO₃ (8.00 mL), extracted with dichloromethane (12.0 mL * 3), the combined organic layers were washed with H₂O (8.00 mL), dried over Na₂SO₄, filtered and concentrated under reduced pressure to give a residue. The residue was purified by Prep-HPLC (column: Welch Xtimate C18 40 * 200 mm 7.00 um; mobile phase: [water (NH₄HCO₃)-ACN]; gradient:18.0%-58.0% B over 25 mins). Compound 2-(((3S,4R)-3-fluoro-4-methoxypiperidin-1-yl)methyl)-6-(3-(1-(4-methyl-4H-1,2,4-triazol-3-yl)cyclobutyl)phenyl)-4-(trifluoromethyl)-1,6-dihydro-7H-pyrrolo[2,3-c]pyridin-7-one (5.85 mg, 10.4 µmol, 9.25% yield) was obtained as a white solid. Confirmed by H NMR and LCMS.
¹H NMR: (DMSO-*d*₆, 400 MHz)
*δ* 12.46 (s, 1H), 8.35 (s, 1H), 7.72 (d, *J =* 1.2 Hz, 1H), 7.47-7.55 (m, 1H), 7.44 (s, 1H), 7.34-7.39 (m, 1H), 7.26 (d, *J* = 8.0 Hz, 1H), 6.29 (s, 1H), 4.60-4.86 (m, 1H), 3.69 (s, 2H), 3.26 (d, *J* = 3.2 Hz, 6H), 2.85-2.99 (m, 3H), 2.63-2.76 (m, 3H), 2.29-2.47 (m, 2H), 2.21 (t, *J =* 8.4 Hz, 1H), 1.95-2.04 (m, 2H), 1.63-1.78 (m, 2H)
LCMS: m/z = 559.2 (M+H)⁺, Rt = 1.307 min

### Example 108: Synthesis of compound T108

### 1. General steps for preparation of 2-(((3R,4S)-3-fluoro-4-methoxypiperidin-1-yl)methyl)-6-(3-(1-(4-methyl-4H-1,2,4-triazol-3-yl)cyclobutyl)phenyl)-4-(trifluoromethyl)-1,6-dihydro-7H-pyrrolo[2,3-c]pyridin-7-one (compound T108)

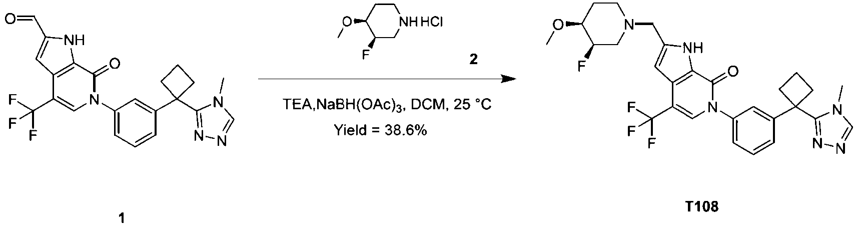

A mixture of 6-[3-[1-(4-methyl-1,2,4-triazol-3-yl)cyclobutyl]phenyl]-7-oxo-4-(trifluoromethyl)-1H-pyrrolo[2,3-c]pyridine-2-car baldehyde (50.0 mg, 113 µmol), (3R,4S)-3-fluoro-4-methoxy-piperidine (22.6 mg, 133 µmol, HCl) , TEA (34.3 mg, 339 µmol, 47.3 µL) in DCM (1.20 mL) was degassed and purged with N₂ for 3 times, and then the mixture was stirred at 25 °C for 30 min under N₂ atmosphere. then NaBH(OAc)₃ (48.0 mg, 226 µmol) was added and stirred at 25 °C for 30 min. LCMS showed desired mass was detected. The reaction mixture was quenched by addition NaHCO₃ 5.00 mL at 25 °C, and then diluted with DCM 5.00 mL and extracted with DCM (5.00 mL * 3). The combined organic layers were washed with brine 20.0 mL, dried over Na₂SO₄, filtered and concentrated under reduced pressure to give a residue. The residue was purified by prep-HPLC (column: Welch Xtimate C18 40*200mm 7um;mobile phase: [water(HCl)-ACN];gradient:0%-38% B over 30 min). To afford Compound 2-[[(3R,4S)-3-fluoro-4-methoxy-1-piperidyl]methyl]-6-[3-[1-(4-methyl-1,2,4-triazol-3-yl)cyclobutyl]phenyl]-4-(trif luoromethyl)-1H-pyrrolo[2,3-c]pyridin-7-one (25.0 mg, 43.7 µmol, 38.6% yield, 97.8% purity) was obtained as a white solid. which confirmed by LCMS and HNMR).
¹H NMR: (400 MHz, DMSO-*d*₆)
*δ* (ppm) 12.67-12.99 (m, 1H), 11.79-12.53 (m, 1H), 10.50-11.17 (m, 1H), 9.67 (s, 1H), 7.84 (s, 1H), 7.51-7.66 (m, 2H), 7.45 (br d, J = 7.2 Hz, 2H), 6.66-6.84 (m, 1H), 5.06-5.33 (m, 1H), 4.43-4.60 (m, 3H), 3.87 (br s, 1H), 3.64 (br d, J = 8.0 Hz, 1H), 3.47 (s, 3H), 3.24-3.35 (m, 5H), 2.91-3.12 (m, 2H), 2.72-2.87 (m, 2H), 1.84-2.25 (m, 4H)
LCMS: m/z = 559.0 (M+H)⁺, Rt = 0.979 min

### Example 109: Synthesis of compound T109

### 1. General steps for preparation of 1-(3-bromophenyl)-3,3-dimethoxy-cyclobutanecarbonitrile

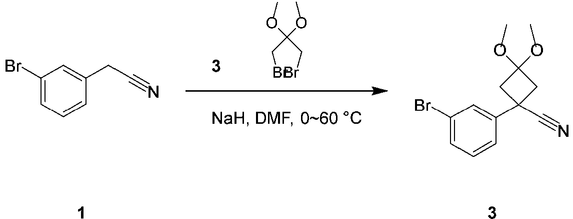

To a solution of NaH (20.4 g, 510 mmol, 60.0% purity) in DMF (425 mL) was added 2-(3-bromophenyl)acetonitrile (50.0 g, 255 mmol) at 0 °C under N₂ atmosphere. After addition, 1,3-dibromo-2,2-dimethoxy-propane (66.8 g, 255 mmol) in DMF (75.0 mL) was added dropwise to the mixture at 60 °C. The resulting mixture was stirred at 60 °C for 2 hrs. LCMS showed desired compound was detected. The reaction mixture was diluted with NH₄Cl (100 mL) at 0 °C, extracted with ethyl acetate (500 mL * 3), the combined organic layers were washed with brine (400 mL * 3), dried over Na₂SO₄, filtered and concentrated under reduced pressure to give a residue. The residue was purified by column chromatography (SiO₂, Petroleum ether/ Ethyl acetate = 0% to 5.00%), (Plate 1, Petroleum ether/ Ethyl acetate = *5*/ 1, R_{f} (product) = 0.4). Compound 1-(3-bromophenyl)-3,3-dimethoxy-cyclobutanecarbonitrile (38.0 g, 60.4 mmol, 23.7% yield, 47.1% purity) was obtained as a yellow oil. Confirmed by H NMR and LCMS.
LCMS: m/z = 265.8 (M+H-32)⁺, Rt = 1.733 min
¹H NMR: (CDCl₃, 400 MHz)
*δ* 7.55 (t, *J* = 2.0 Hz, 1H), 7.38-7.42 (m, 1H), 7.33-7.37 (m, 1H), 7.21-7.25 (m, 1H), 3.21 (s, 3H), 3.11 (s, 3H), 3.00-3.06 (m, 2H), 2.60-2.67 (m, 2H)

### 2. General steps for preparation of 1-(3-bromophenyl)-3,3-dimethoxy-cyclobutanecarbonitrile

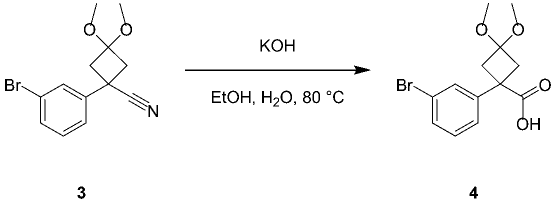

To a solution of 1-(3-bromophenyl)-3,3-dimethoxy-cyclobutanecarbonitrile (25.0 g, 84.4 mmol) in H₂O (250 mL) and EtOH (250 mL) was added KOH (142 g, 2.53 mol). After addition, the mixture was stirred at 80 °C for 16 hrs. LCMS showed desired compound was detected. The reaction mixture was diluted with H₂O (300 mL), extracted with ethyl acetate (300 mL*2). The aqueous phase was adjust pH = 2 (HCl, 2 M), extracted with ethyl acetate (300 mL*2). The organic phase was washed with brine (300 mL), dried over Na₂SO₄ and concentrated in vacuum. Compound 1-(3-bromophenyl)-3,3-dimethoxy-cyclobutanecarboxylic acid (30.0 g, 48.0 mmol, 56.9% yield, 50.5% purity) was obtained as a colorless oil. Confirmed by LCMS.
LCMS: m/z = 282.9 (M+H-32)⁺, Rt = 1.333 min

### 3. General steps for preparation of 1-[[1-(3-bromophenyl)-3,3-dimethoxy-cyclobutanecarbonyl]amino]-3-methyl-thiourea:

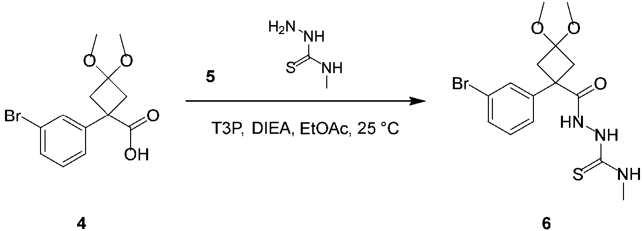

To a solution of 1-(3-bromophenyl)-3,3-dimethoxy-cyclobutanecarboxylic acid (27.0 g, 85.6 mmol) in ethyl acetate (270 mL) was added dropwise DIEA (166 g, 1.29 mol, 223 mL), and then 1-amino-3-methyl-thiourea (11.7 g, 111 mmol) was added to the mixture, T3P (170 g, 267 mmol, 159 mL, 50.0% purity) was added to the mixture at 0 °C. The resulting mixture was stirred at 25 °C for 2 hrs. LCMS showed desired compound was detected. The reaction mixture was diluted with aqueous NH₄Cl (200 mL), the combined organic layers were extracted with ethyl acetate (300 mL *2), dried over Na₂SO₄, filtered and concentrated under reduced pressure to give a residue. Compound 1-[[1-(3-bromophenyl)-3,3-dimethoxy-cyclobutanecarbonyl]amino]-3-methyl-thiourea (32.5 g, 18.0 mmol, 21.0% yield, 22.3% purity) was obtained as a colorless oil. Confirmed by LCMS.
LCMS: m/z = 383.6 (M+H-18)⁺, Rt = 1.435 min

### 4. General steps for preparation of 5-[1-(3-bromophenyl)-3,3-dimethoxy-cyclobutyl]-4-methyl-1,2,4-triazole-3-thiol

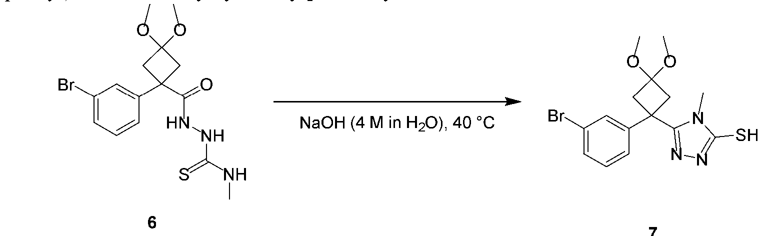

To a solution of 1-[[1-(3-bromophenyl)-3,3-dimethoxy-cyclobutanecarbonyl]amino]-3-methyl-thiourea (33.0 g, 82.0 mmol) in THF (80.0 mL) was added dropwise a solution of NaOH (52.4 g, 1.31 mol) in H₂O (330 mL). After addition, the mixture was stirred at 40 °C for 12 hrs. LCMS showed desired compound was detected. The reaction mixture was diluted with H₂O (100 mL), extracted with ethyl acetate (100 mL * 3), the combined organic layers were washed with H₂O (100 mL * 3), dried over Na₂SO₄, filtered and concentrated under reduced pressure to give a residue. The residue was purified by column chromatography (SiO₂, Petroleum ether/ Ethyl acetate = 0% to 50.0%), (Plate 1, Petroleum ether/ Ethyl acetate = 1/ 1, R_{f} (product) = 0.45). Compound 5-[1-(3-bromophenyl)-3,3-dimethoxy-cyclobutyl]-4-methyl-1,2,4-triazole-3-thiol (8.50 g, 18.8 mmol, 23.0% yield, 85.4% purity) was obtained as a colorless oil. Confirmed by H NMR and LCMS.
¹H NMR: (CDCl₃, 400 MHz)
δ 11.38 (s, 1H), 7.39-7.44 (m, 2H), 7.19-7.24 (m, 1H), 7.11 (d, *J* = 8.0 Hz, 1H), 3.22 (s, 1H), 3.20 (s, 3H), 3.19 (s, 3H), 3.16-3.18 (m, 3H), 3.14 (s, 1H), 2.81 (d, *J=* 13.6 Hz, 2H)
LCMS: m/z = 383.5 (M+H)⁺, Rt = 1.448 min

### 5. General steps for preparation of 3-[1-(3-bromophenyl)-3,3-dimethoxy-cyclobutyl]-4-methyl-1,2,4-triazole

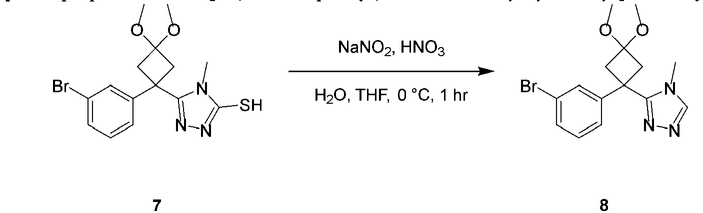

To a solution of 5-[1-(3-bromophenyl)-3,3-dimethoxy-cyclobutyl]-4-methyl-1,2,4-triazole-3-thiol (8.30 g, 21.6 mmol) in THF (40.0 mL) and H₂O (40.0 mL) was added NaNO₂ (14.9 g, 215 mmol). HNO₃ (28.4 g, 293 mmol, 20.3 mL, 65.0% purity) was added to the mixture slowly at 0 °C. After addition, the mixture was stirred at 0 °C for 1 hrs. LCMS showed desired compound was detected. The reaction mixture was diluted with aqueous NaHCO₃ (200 mL) adjust pH = 8, extracted with ethyl acetate (80.0 mL * 3), the combined organic layers were washed with H₂O (80.0 mL * 3), dried over Na₂SO₄, filtered and concentrated under reduced pressure to give a residue. The residue was purified by column chromatography (SiO₂, DCM/ MeOH = 0% to 8.00%), (Plate 1, DCM/ MeOH = 101 1, R_{f} (product) = 0.40). Compound 3-[1-(3-bromophenyl)-3,3-dimethoxy-cyclobutyl]-4-methyl-1,2,4-triazole (6.30 g, 16.6 mmol, 77.1% yield, 93.2% purity) was obtained as a colorless oil. Confirmed by H NMR and LCMS.
¹H NMR: (CDCl₃, 400 MHz)
*δ* 8.04-8.13 (m, 1H), 7.42 (s, 1H), 7.37 (d, *J= 7.6* Hz, 1H), 7.12-7.20 (m, 2H), 3.27-3.32 (m, 2H), 3.26 (s, 3H), 3.19 (s, 3H), 3.16 (s, 3H), 2.84-2.89 (m, 2H)
LCMS: m/z = 353.4 (M+H)⁺, Rt = 1.062 min

### 6. General steps for preparation of 3-(3-bromophenyl)-3-(4-methyl-1,2,4-triazol-3-yl)cyclobutanone

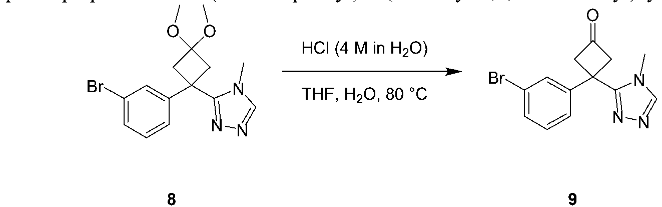

To a solution of 3-[1-(3-bromophenyl)-3,3-dimethoxy-cyclobutyl]-4-methyl-1,2,4-triazole (4.83 g, 13.7 mmol) in THF (10.0 mL) and H₂O (40.0 mL) was added dropwise HCl (4 M, 19.3 mL) at 25 °C. After addition, the mixture was stirred at 80 °C for 2 hrs. LCMS showed desired compound was detected. The reaction mixture was diluted with NaHCO₃ adjust pH = 8, extracted with ethyl acetate (35.0 mL * 3), the combined organic layers were dried over Na₂SO₄, filtered and concentrated under reduced pressure to give a residue. The residue was purified by column chromatography (SiO₂, DCM/ MeOH = 0% to 6.00%), (Plate 1, DCM/ MeOH = *5*/ 1, R_{f}(product) = 0.49). Compound 3-(3-bromophenyl)-3-(4-methyl-1,2,4-triazol-3-yl)cyclobutanone (4.75 g, 12.6 mmol, 91.8% yield, 81.2% purity) was obtained as a colorless oil. Confirmed by H NMR and LCMS.
¹H NMR: (CDCl₃, 400 MHz)
δ 8.13 (s, 1H), 7.46 (dt, *J* = 8.0, 1.2 Hz, 1H), 7.41 (t, *J* = 2.0 Hz, 1H), 7.26-7.29 (m, 1H), 7.16-7.20 (m, 1H), 4.17-4.24 (m, 2H), 3.69-3.76 (m, 2H), 3.31 (s, 3H)
LCMS: m/z = 307.4 (M+H)⁺, Rt = 1.105 min

### 7. General steps for preparation of 3-[-(3-bromophenyl)-3,3-difluoro-cyclobutyl]-4-methyl-1,2,4-triazole

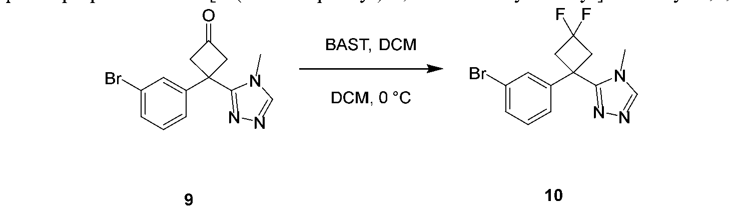

To a solution of 3-(3-bromophenyl)-3-(4-methyl-1,2,4-triazol-3-yl)cyclobutanone (500 mg, 1.63 mmol) in DCM (5.00 mL) was added dropwise a solution of BAST (2.89 g, 13.0 mmol, 2.86 mL) in DCM (5.00 mL) at 0 °C. After addition, the mixture was stirred at 25 °C for 24 hrs. LCMS showed desired compound was detected. The reaction mixture was diluted with NaHCO₃ adjust pH = 8, extracted with ethyl acetate (20.0 mL * 3), the combined organic layers were washed with saturated aqueous NaHCO₃ (20.0 mL * 3), dried over Na₂SO₄, filtered and concentrated under reduced pressure to give a residue. The residue was purified by column chromatography (SiO₂, DCM/ MeOH = 0% to 2.00%), (Plate 1, DCM/ MeOH = 10/ 1, R_{f} (product) = 0.42). Compound 3-[1-(3-bromophenyl)-3,3-difluoro-cyclobutyl]-4-methyl-1,2,4-triazole (50.0 mg, 119 µmol, 7.31% yield, 78.4% purity) was obtained as a colorless oil. Confirmed by H NMR and LCMS.
¹H NMR: (CDCl₃, 400 MHz)
δ 8.06 (s, 1H), 7.40-7.47 (m, 2H), 7.15-7.25 (m, 2H), 3.66-3.76 (m, 2H), 3.47-3.56 (m, 2H), 3.25 (s, 3H)
LCMS: m/z =327.4 (M+H)⁺, Rt = 1.182 min

### 8. General steps for preparation of 6-[3-[3,3-difluoro-1-(4-methyl-1,2,4-triazol-3-yl)cyclobutyl]phenyl]-2-[[(3S)-3-methyl-1-piperidyl]methyl]-1-(p-to lylsulfonyl)-4-(trifluoromethyl)pyrrolo[2,3-c]pyridin-7-one

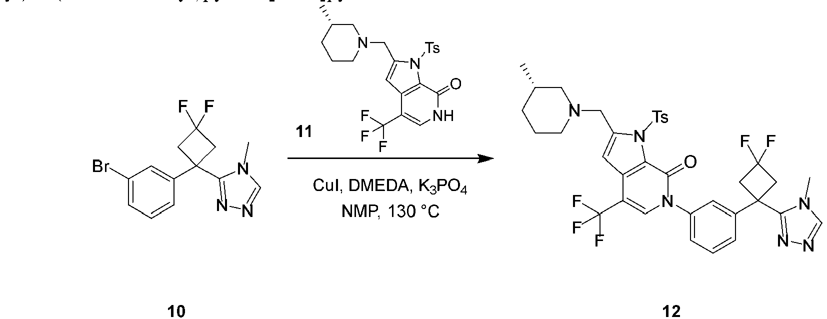

A mixture of 3-[1-(3-bromophenyl)-3,3-difluoro-cyclobutyl]-4-methyl-1,2,4-triazole (50.0 mg, 152 µmol), 2-[[(3S)-3-methyl-1-piperidyl]methyl]-1-(p-tolylsulfonyl)-4-(trifluoromethyl)-6H-pyrrolo[2,3-c]pyridin-7-one (71.2 mg, 152 µmol), CuI (58.0 mg, 304 µmol), K₃PO₄ (97.0 mg, 457 µmol) and DMEDA (13.4 mg, 152 µmol, 16.4 µL) in NMP (0.50 mL) was degassed and purged with N₂ for 3 times, and then the mixture was stirred at 130 °C for 2 hrs under N₂ atmosphere. LCMS showed desired compound was detected. The reaction mixture was diluted with H₂O (30.0 mL), extracted with ethyl acetate (20.0 mL * 3), the combined organic layers were washed with H₂O (20.0 mL * 3), dried over Na₂SO₄, filtered and concentrated under reduced pressure to give a residue. The residue was purified by column chromatography (SiO₂, DCM/ MeOH = 3.00% to 20.0%), (Plate 1, DCM/ MeOH = 10/ 1, R_{f} (product) = 0.34). Compound 6-[3-[3,3-difluoro-1-(4-methyl-1,2,4-triazol-3-yl)cyclobutyl]phenyl]-2-[[(3 S)-3-methyl-1-piperidyl]methyl]-1-(p-to lylsulfonyl)-4-(trifluoromethyl)pyrrolo[2,3-c]pyridin-7-one (46.3 mg, 36.6 µmol, 24.0% yield, 56.6% purity) was obtained as a colorless oil. Confirmed by H NMR and LCMS.
¹H NMR: (CDCl₃, 400 MHz)
*δ* 8.26-8.64 (m, 1H), 7.37-7.55 (m, 2H), 7.35 (d, *J* = 1.6 Hz, 3H), 6.91-7.23 (m, 3H), 6.43-6.61 (m, 1H), 3.90-4.00 (m, 1H), 3.68-3.89 (m, 2H), 3.54-3.68 (m, 2H), 3.45-3.54 (m, 3H), 3.32-3.35 (m, 2H), 2.86-2.98 (m, 2H), 2.45 (s, 2H), 2.04-2.14 (m, 2H), 1.25 (s, 5H), 0.92-0.99 (m, 1H), 0.88 (s, 3H)
LCMS: m/z =715.0 (M+H)⁺, Rt = 1.225 min

### 9. General steps for preparation of 6-[3-[3,3-difluoro-1-(4-methyl-1,2,4-triazol-3-yl)cyclobutyl]phenyl]-2-[[(3S)-3-methyl-1-piperidyl]methyl]-4-(trifl uoromethyl)-1H-pyrrolo[2,3-c]pyridin-7-one (compound T109)

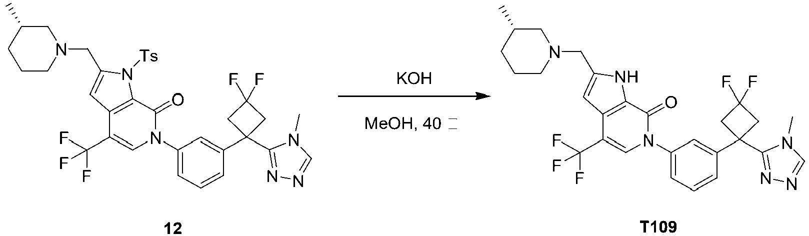

A mixture of 6-[3-[3,3-difluoro-1-(4-methyl-1,2,4-triazol-3-yl)cyclobutyl]phenyl]-2-[[(3 S)-3-methyl-1-piperidyl]methyl]-1-(p-to lylsulfonyl)-4-(trifluoromethyl)pyrrolo[2,3-c]pyridin-7-one (46.3 mg, 64.7 µmol), KOH (72.6 mg, 1.30 mmol) in MeOH (0.50 mL) was degassed and purged with N₂ for 3 times, and then the mixture was stirred at 40 °C for 1 hr. LCMS showed desired compound was detected. The reaction mixture was extracted with ethyl acetate (20.0 mL * 3), the combined organic layers were washed with aqueous KOH (20.0 mL * 3), dried over Na₂SO₄, filtered and concentrated under reduced pressure to give a residue. The residue was purified by Prep-HPLC (column: Xtimate C18 150 * 40 mm * 10 um; mobile phase: [water (NH₃H₂O + NH₄HCO₃)-ACN]; gradient: 32.0%-72.0% B over 32 mins). Compound 6-[3-[3,3-difluoro-1-(4-methyl-1,2,4-triazol-3-yl)cyclobutyl]phenyl]-2-[[(3S)-3-methyl-1-piperidyl]methyl]-4-(trifl uoromethyl)-1H-pyrrolo[2,3-c]pyridin-7-one (3.75 mg, 6.62 µmol, 10.2% yield, 99.0% purity) was obtained as a yellow solid. Confirmed by H NMR and LCMS.
¹H NMR: (DMSO-*d*₆, 400 MHz)
δ 12.32-12.57 (m, 1H), 8.40 (s, 1H), 7.74 (s, 1H), 7.52-7.57 (m, 2H), 7.42 (d, *J= 7.6* Hz, 1H), 7.32 (d, *J= 7.6* Hz, 1H), 6.27 (s, 1H), 3.66-3.78 (m, 3H), 3.60 (s, 2H), 3.44-3.53 (m, 4H), 2.71-2.77 (m, 2H), 1.88 (t, *J* = 10.4 Hz, 1H), 1.55-1.62 (m, 4H), 1.41-1.48 (m, 1H), 0.81 (d, *J =* 4.8 Hz, 3H), 0.73-0.79 (m, 1H)
LCMS: m/z =561.3 (M+H)⁺, Rt = 1.587 min

### Example 110: Synthesis of compound T110

### 1. General steps for preparation of 2-[[(3S)-4,4-difluoro-3-methyl-1-piperidyl]methyl]-6-[3-[1-(4-methyl-1,2,4-triazol-3-yl)cyclobutyl]phenyl]-4-(trifl uoromethyl)-1H-pyrrolo[2,3-c]pyridin-7-one (compound T110)

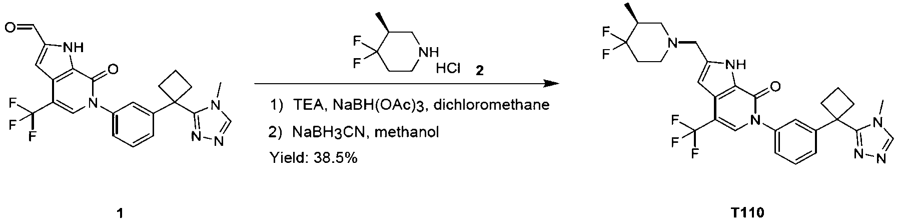

6-[3-[1-(4-methyl-1,2,4-triazol-3-yl)cyclobutyl]phenyl]-7-oxo-4-(trifluoromethyl)-1H-pyrrolo[2,3-c]pyridine-2-carbaldehyde (50.0 mg, 113 µmol) was dissolved in Dichloromethane 1.00 mL, pH was adjust to 7-8 with TEA. (3S)-4,4-difluoro-3-methyl-piperidine (29.2 mg, 170 µmol, HCl) in Dichloromethane 1 mL was added to the mixture, stirred at 25 °C for 0.5 hr under N₂. Then NaBH(OAc)₃ (60.0 mg, 283 µmol) was added to the mixture, then stirred at 25 °C under N₂ for 12 hrs. pH was adjust to 6-7 with TEA. (3S)-4,4-difluoro-3-methyl-piperidine (15.3 mg, 89.2 µmol, HCl) was added to the mixture and stirred at 25 °C for 0.5 hr, then NaBH(OAc)₃ (60.0 mg, 283 µmol) was added to the mixture, stirred at 35 °C under N₂ for 2 hrs. NaBH₃CN (10.7 mg, 170 µmol) and methyl alcohol 1.00 mLwas added to the mixture, stirred at 35 °C under N₂ for 2 hrs. LCMS shows reactant was consumed completely and desired mass was detected. The reaction mixture was diluted with Dichloromethane 6.00 mL, washed with sat. NaHCO₃ solution 2.00 mL. The water phase extracted with Dichloromethane 2.00 mL (1.00 mL * 2). The combined organic layers were washed, dried over Na₂SO₄, filtered and concentrated under reduced pressure to give a residue. The residue was purified by prep.HPLC (column: Welch Xtimate C18 40 * 200 mm 7um;mobile phase: [water (HCl) - ACN];gradient: 2%-42% B over 20.5 min). 2-[[(3S)-4,4-difluoro-3-methyl-1-piperidyl]methyl]-6-[3-[1-(4-methyl-1,2,4-triazol-3-yl)cyclobutyl]phenyl]-4-(trifl uoromethyl)-1H-pyrrolo[2,3-c]pyridin-7-one (24.5 mg, 43.71 µmol, 38.58% yield, 100.0% purity) was obtained as white solid, confirmed by H NMR and LCMS.
¹H NMR: (400 MHz, DMSO-*d*₆)
δ 12.77 (s, 1H), 9.04-9.33 (m, 1H), 7.84 (d, *J* = 1.2 Hz, 1H), 7.53-7.61 (m, 1H), 7.49 (s, 1H), 7.41 (br dd, *J* = 14.0, 7.6 Hz, 2H), 6.76 (s, 1H), 4.50 (br s, 2H), 3.39 (br s, 3H), 3.03-3.19 (m, 2H), 2.93-3.00 (m, 2H), 2.82-2.92 (m, 1H), 2.72-2.80 (m, 2H), 2.57-2.66 (m, 1H), 2.40 (br s, 3H), 1.93-2.11 (m, 2H), 0.99 (br d, *J =* 6.0 Hz, 3H)
LCMS: m/z = 561.3 (M+H)⁺, Rt = 1.536 min

### Example 111: Synthesis of compound T111

### 1. General steps for preparation of (R)-2-((4,4-difluoro-3-methylpiperidin-1-yl)methyl)-6-(3-(1-(4-methyl-4H-1,2,4-triazol-3-yl)cyclobutyl)phenyl)-4-(trifluoromethyl)-1,6-dihydro-7H-pyrrolo[2,3-c]pyridin-7-one (compound T111)

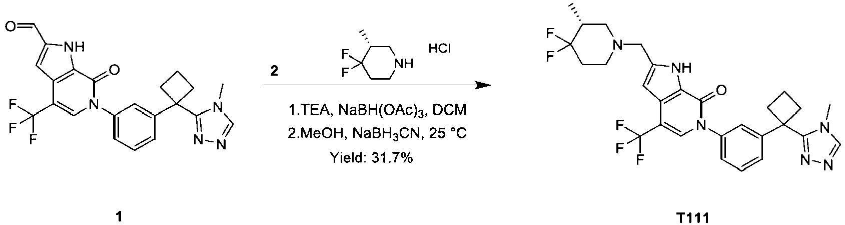

To a solution of 6-(3-(1-(4-methyl-4H-1,2,4-triazol-3-yl)cyclobutyl)phenyl)-7-oxo-4-(trifluoromethyl)-6,7-dihydro-1H-pyrrolo[2,3-c]pyridine-2-carbaldehyde (50.0 mg, 113 µmol) in dichloromethane (2.50 mL) was added dropwise TEA (11.4 mg, 113 µmol, 15.7 µL) adjust pH = 7, rac-(3R)-4,4-difluoro-3-methyl-piperidine (25.2 mg, 147 µmol, HCl) was added to the mixture, stirred at 25 °C for 0.5 hr, then NaBH(OAc)₃ (60.0 mg, 283 µmol) was added to the mixture, stirred at 25 °C for 1 hr. Methyl alcohol (1.00 mL) was added to the mixture, followed by NaBH₃CN (7.12 mg, 113 µmol) add slowly, stirred for 1 hr. LCMS showed desired compound was detected. The reaction mixture was diluted with aqueous NaHCO₃ (8.00 mL), extracted with dichloromethane (12.0 mL * 3), the combined organic layers were washed with H₂O (8.00 mL), dried over Na₂SO₄, filtered and concentrated under reduced pressure to give a residue. The residue was purified by Prep-HPLC (column: Welch Xtimate C18 40 * 200 mm 7 um; mobile phase: [water (NH₄HCO₃)-ACN]; gradient:30.0%-70.0% B over 25 mins). Compound 6-[3-[1-(4-methyl-1,2,4-triazol-3-yl)cyclobutyl]phenyl]-2-[[rac-(3R)-4,4-difluoro-3-methyl-1-piperidyl]methyl]-4-( trifluoromethyl)-1H-pyrrolo[2,3-c]pyridin-7-one (20.8 mg, 35.9 µmol, 31.7% yield, 97.0% purity) was obtained as a white solid. Confirmed by H NMR and LCMS.
¹H NMR: (DMSO-*d*₆, 400 MHz)
δ 12.47 (s, 1H), 8.35 (s, 1H), 7.72 (s, 1H), 7.48-7.53 (m, 1H), 7.44 (s, 1H), 7.36 (d, *J=* 8.0 Hz, 1H), 7.26 (d, *J* = 8.0 Hz, 1H), 6.31 (s, 1H), 3.71 (s, 2H), 3.27 (s, 3H), 2.90-2.98 (m, 2H), 2.74-2.84 (m, 2H), 2.65-2.72 (m, 2H), 2.22-2.32 (m, 1H), 2.03-2.17 (m, 2H), 2.00 (d, *J =* 3.2 Hz, 2H), 1.87-1.98 (m, 2H), 0.92 (d, *J =* 6.4 Hz, 3H)
LCMS: m/z = 561.2 (M+H)⁺, Rt = 1.530 min

### Example 112: Synthesis of compound T112

### 1. General steps for preparation of 2-[[4-(difluoromethyl)-1-piperidyl]methyl]-6-[3-[1-(4-methyl-1,2,4-triazol-3-yl)cyclobutyl]phenyl]-4-(trifluoromet hyl)-1H-pyrrolo[2,3-c]pyridin-7-one (compound T112)

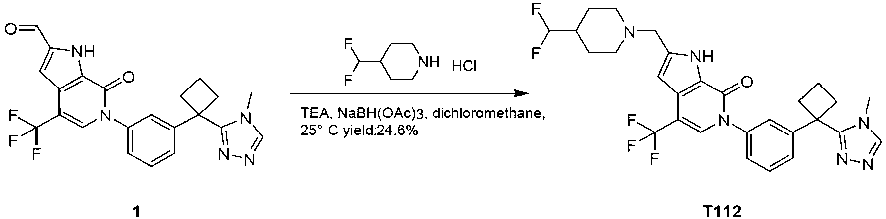

To a mixture of 4-(difluoromethyl)piperidine (29.2 mg, 170 µmol, HCl) in Dichloromethane (3.00 mL) was added TEA (57.3 mg, 566 µmol) adjust pH = 8. Then 6-[3-[1-(4-methyl-1,2,4-triazol-3-yl)cyclobutyl]phenyl]-7-oxo-4-(trifluoromethyl)-1H-pyrrolo[2,3-c]pyridine-2-car baldehyde (50.0 mg, 113 µmol) was added to the mixture. The mixture was stirred under N₂ at 25 °C for 0.5 hr. NaBH(OAc)₃ (60.0 mg, 283 µmol) was added to the mixture. The mixture was stirred under N₂ at 25 °C for 2.5 hrs. LCMS showed desired mass was detected. The reaction mixture was diluted with NaHCO₃ (3.00 mL), extracted with Dichloromethane (2.00 mL*2). The organic phase dried over Na₂SO₄ and concentrated in vacuum. The crude product was purified by Prep-HPLC (column: Welch Xtimate C18 40*200 mm 7 um; mobile phase: [water (FA)-ACN]; gradient: 0%-38.0% B over 25 min). To afford 2-[[4-(difluoromethyl)-1-piperidyl]methyl]-6-[3-[1-(4-methyl-1,2,4-triazol-3-yl)cyclobutyl]phenyl]-4-(trifluoromet hyl)-1H-pyrrolo[2,3-c]pyridin-7-one (16.9 mg, 27.8 µmol, 24.6% yield, 100% purity, FA) as a white solid which was confirmed by H NMR and LCMS.
¹H NMR: (400 MHz, DMSO-*d*₆)
δ 12.45 (s, 1H), 8.35 (s, 1H), 7.71 (d, *J* = 1.2 Hz, 1H), 7.48-7.54 (m, 1H), 7.42 (s, 1H), 7.35 (d, *J* = 8.0 Hz, 1H), 7.26 (d, *J* = 8.0 Hz, 1H), 6.29 (s, 1H), 5.72-6.02 (m, 1H), 3.63 (s, 2H), 3.27 (s, 3H), 2.88-2.97 (m, 4H), 2.66-2.73 (m, 2H), 1.93-2.03 (m, 4H), 1.67-1.78 (m, 1H), 1.64 (d, *J =* 12.4 Hz, 2H), 1.36 (qd, *J* = 12.4, 3.6 Hz, 2H)
LCMS: m/z = 561.2 (M+H)⁺, Rt = 1.398 min

### Examples 113 and 114: Synthesis of compounds T113& T114

### 1. General steps for preparation of 2-[[3-(difluoromethyl)-1-piperidyl]methyl]-6-[3-[1-(4-methyl-1,2,4-triazol-3-yl)cyclobutyl]phenyl]-4-(trifluoromet hyl)-1H-pyrrolo[2,3-c]pyridin-7-one

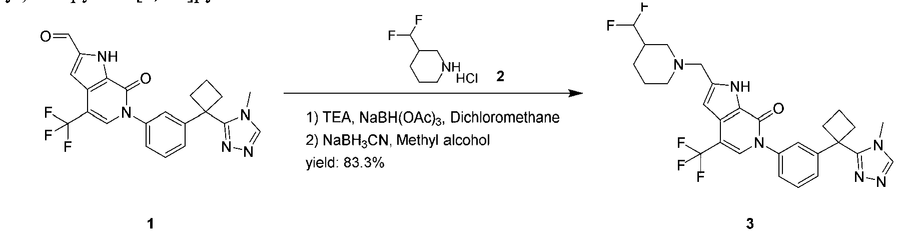

6-[3-[1-(4-methyl-1,2,4-triazol-3-yl)cyclobutyl]phenyl]-7-oxo-4-(trifluoromethyl)-1H-pyrrolo[2,3-c]pyridine-2-carbaldehyde (85.0 mg, 193 µmol) was dissolved in Dichloromethane (2.00 mL). pH was adjusted to 7-8 with TEA. 3-(difluoromethyl)piperidine (49.6 mg, 289 µmol, HCl) in Dichloromethane (2.00 mL) was added to the mixture, and stirred at 25 °C under N₂ for 0.5 hrs. NaBH(OAc)₃ (102 mg, 481 µmol) was added to the mixture the mixture was stirred at 25 °C for 12 hrs. 3-(difluoromethyl)piperidine (49.6 mg, 289 µmol, HCl) in Dichloromethane (2.00 mL) was added to the mixture, and stirred at 25 °C under N₂ for 0.5 hrs. NaBH(OAc)₃ (102 mg, 481 µmol) was added to the mixture the mixture was stirred at 30 °C for 2 hrs. Methyl alcohol (2.00 mL) and NaBH₃CN (12.1 mg, 193 µmol) was added to the mixture in order. The mixture was stirred at 35 °C for 2 hrs. LCMS shows reactant was consumed completely and desired mass was detected. The reaction mixture was diluted with Dichloromethane 12.0 mL, washed with sat. NaHCO₃ solution 4.00 mL. The water phase extracted with Dichloromethane 4.00 mL (2.00 mL * 2). The combined organic layers were washed, dried over Na₂SO₄, filtered and concentrated under reduced pressure to give a residue. The residue was purified by prep.HPLC (column: Welch Xtimate C18 40 * 200 mm 7 um;mobile phase: [water (FA) - ACN];gradient: 0%-40% B over 25 min). 2-[[3-(difluoromethyl)-1-piperidyl]methyl]-6-[3-[1-(4-methyl-1,2,4-triazol-3-yl)cyclobutyl]phenyl]-4-(trifluoromet hyl)-1H-pyrrolo[2,3-c]pyridin-7-one (90.0 mg, 160 µmol, 83.3% yield, 99.9% purity) was obtained as white solid, confirmed by LCMS and HPLC.
LCMS: m/z = 561.3 (M+H)⁺, Rt = 1.458 min

### 2. General steps for preparation of 6-[3-[1-(4-methyl-1,2,4-triazol-3-yl)cyclobutyl]phenyl]-2-[[rac-(3R)-3-(difluoromethyl)-1-piperidyl]methyl]-4-(trif luoromethyl)-1H-pyrrolo[2,3-c]pyridin-7-one (compound T113) and 6-[3-[1-(4-methyl-1,2,4-triazol-3-yl)cyclobutyl]phenyl]-2-[[rac-(3S)-3-(difluoromethyl)-1-piperidyl]methyl]-4-(trifl uoromethyl)-1H-pyrrolo[2,3-c]pyridin-7-one (compound T114)

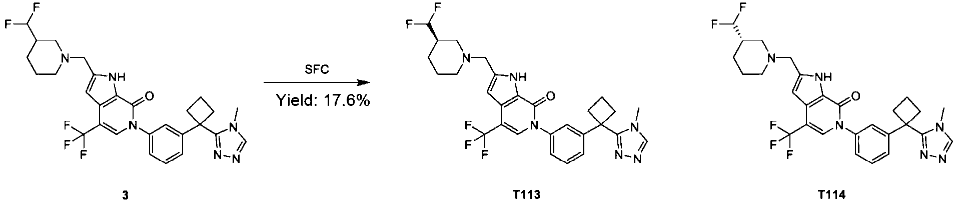

2-[[3-(difluoromethyl)-1-piperidyl]methyl]-6-[3-[1-(4-methyl-1,2,4-triazol-3-yl)cyclobutyl]phenyl]-4-(trifluor omethyl)-1H-pyrrolo[2,3-c]pyridin-7-one (90.0 mg, 160 µmol) was purified by SFC (column: DAICEL CHIRALPAK AD (250 mm * 30 mm, 10 um);mobile phase: [CO₂ - i-PrOH (0.1% NH₃H₂O)];B%: 25%, isocratic elution mode). 6-[3-[1-(4-methyl-1,2,4-triazol-3-yl)cyclobutyl]phenyl]-2-[[rac-(3R)-3-(difluoromethyl)-1-piperidyl]methyl]-4-(trif luoromethyl)-1H-pyrrolo[2,3-c]pyridin-7-one (16.0 mg, 28.7 µmol, 17.6% yield, 99.0% purity) was obtained as white solid, confirmed by H NMR and LCMS.
¹H NMR: (400 MHz, DMSO-*d*₆)
δ 12.27-12.86 (m, 1H), 8.35 (s, 1H), 7.73 (br s, 1H), 7.47-7.56 (m, 1H), 7.42 (br s, 1H), 7.36 (br d, *J =* 7.8 Hz, 1H), 7.27 (br d, *J* = 7.2 Hz, 1H), 6.34 (br s, 1H), 5.81-6.17 (m, 1H), 3.45-3.86 (m, 2H), 3.27 (s, 3H), 2.90-2.99 (m, 2H), 2.59-2.89 (m, 4H), 1.90-2.11 (m, 4H), 1.68 (br d, *J =* 8.0 Hz, 1H), 1.43-1.56 (m, 1H), 1.33-1.35 (m, 1H), 1.23 (br s, 3H)
LCMS: m/z = 561.2 (M+H)⁺, Rt = 1.490 min

6-[3-[1-(4-methyl-1,2,4-triazol-3-yl)cyclobutyl]phenyl]-2-[[rac-(3S)-3-(difluoromethyl)-1-piperidyl]methyl]-4 -(trifluoromethyl)-1H-pyrrolo[2,3-c]pyridin-7-one (10.2 mg, 18.2 µmol, 11.3% yield, 99.9% purity)was obtained as white solid, confirmed by H NMR and LCMS.
¹H NMR: (400 MHz, DMSO-*d*₆)
δ 12.47 (br s, 1H), 8.36 (s, 1H), 7.67-7.86 (m, 1H), 7.48-7.57 (m, 1H), 7.44 (s, 1H), 7.37 (d, *J=* 7.2 Hz, 1H), 7.26 (d, *J= 7.6* Hz, 1H), 6.30 (s, 1H), 5.80-6.14 (m, 1H), 3.68 (s, 2H), 3.27 (s, 3H), 2.89-3.00 (m, 2H), 2.62-2.87 (m, 4H), 1.94-2.11 (m, 4H), 1.67 (d, *J =* 10.4 Hz, 2H), 1.48 (d, *J = 9.6* Hz, 1H)
LCMS: m/z = 561.2 (M+H)⁺, Rt = 1.477 min

### Examples 115 and 116: Synthesis of compounds T115& T116

### 1. General steps for preparation of 2-[[(4R)-3,3-difluoro-4-methyl-1-piperidyl]methyl]-6-[3-[1-(4-methyl-1,2,4-triazol-3-yl)cyclobutyl]phenyl]-4-(trifl uoromethyl)-1H-pyrrolo[2,3-c]pyridin-7-one

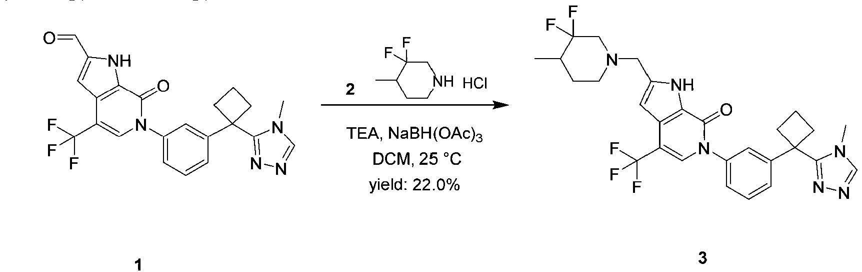

To a solution of 6-[3-[1-(4-methyl-1,2,4-triazol-3-yl)cyclobutyl]phenyl]-7-oxo-4-(trifluoromethyl)-1H-pyrrolo[2,3-c]pyridine-2-car baldehyde (100 mg, 226 µmol) in dichloromethane (2.50 mL) was added dropwise TEA (22.9 mg, 226 µmol, 31.5 µL) adjust pH = 7, (4R)-3,3-difluoro-4-methyl-piperidine (50.5 mg, 294 µmol, HCl) was added to the mixture, stirred at 25 °C for 0.5 hr, then NaBH(OAc)₃ (120 mg, 566 µmol) was added to the mixture, stirred at 25 °C for 1 hr. LCMS showed desired compound was detected. The reaction mixture was diluted with aqueous NaHCO₃ (8.00 mL), extracted with dichloromethane (12.0 mL * 3), the combined organic layers were washed with H₂O (8.00 mL), dried over Na₂SO₄, filtered and concentrated under reduced pressure to give a residue. The residue was purified by Prep-HPLC (column: Welch Xtimate C18 40 * 200 mm 7 um; mobile phase: [water (FA)-ACN]; gradient:16.0%-56.0% B over 25 mins). Compound 2-[[(4R)-3,3-difluoro-4-methyl-1-piperidyl]methyl]-6-[3-[1-(4-methyl-1,2,4-triazol-3-yl)cyclobutyl]phenyl]-4-(trifl uoromethyl)-1H-pyrrolo[2,3-c]pyridin-7-one (30.0 mg, 49.9 µmol, 22.0% yield, 93.3% purity) was obtained as a colorless oil.

### 2. General steps for preparation of (R)-2-((3,3-difluoro-4-methylpiperidin-1-yl)methyl)-6-(3-(1-(4-methyl-4H-1,2,4-triazol-3-yl)cyclobutyl)phenyl)-4-(trifluoromethyl)-1,6-dihydro-7H-pyrrolo[2,3-c]pyridin-7-one (compound T115) and (S)-2-((3,3-difluoro-4-methylpiperidin-1-yl)methyl)-6-(3-(1-(4-methyl-4H-1,2,4-triazol-3-yl)cyclobutyl)phenyl)-4-( trifluoromethyl)-1,6-dihydro-7H-pyrrolo[2,3-c]pyridin-7-one (compound T116)

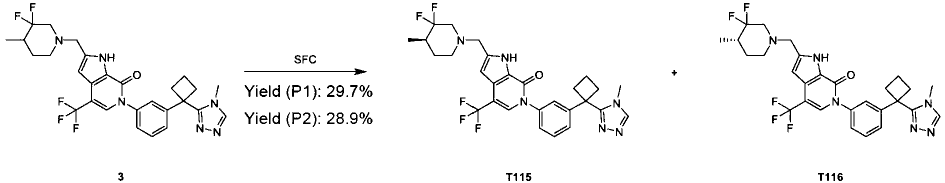

The residue was purified by SFC (column: DAICEL CHIRALPAK AD (250 mm * 30.0 mm, 10.0 um);mobile phase: [CO₂-i-PrOH(0.1% NH₃H₂O)]; B%:45.0%, isocratic elution mode). Compound (R)-2-((3,3-difluoro-4-methylpiperidin-1-yl)methyl)-6-(3-(1-(4-methyl-4H-1,2,4-triazol-3-yl)cyclobutyl)phenyl)-4-(trifluoromethyl)-1,6-dihydro-7H-pyrrolo[2,3-c]pyridin-7-one (8.93 mg, 15.9 µmol, 29.7% yield) was obtained as a white solid. Confirmed by H NMR and LCMS.
¹H NMR: (DMSO-*d*₆, 400 MHz)
*δ* 12.51 (s, 1H), 8.35 (s, 1H), 7.73 (s, 1H), 7.49-7.53 (m, 1H), 7.44 (s, 1H), 7.36 *(d, J=* 7.6 Hz, 1H), 7.26 (d*, J* = 7.6 Hz, 1H), 6.32 (s, 1H), 3.75 (s, 2H), 3.27 (s, 3H), 3.03-3.10 (m, 1H), 2.90-2.97 (m, 2H), 2.82 (d, *J* = 11.2 Hz, 1H), 2.70 (d, *J =* 10.4 Hz, 2H), 2.24-2.34 (m, 1H), 2.11 (t, *J* = 10.8 Hz, 1H), 1.95-2.03 (m, 2H), 1.62-1.72 (m, 1H), 1.33-1.44 (m, 1H), 1.23 (s, 1H), 0.95 (d, *J=* 6.4 Hz, 3H)
LCMS: m/z = 561.3 (M+H)⁺, Rt = 1.078 min

Compound (S)-2-((3,3-difluoro-4-methylpiperidin-1-yl)methyl)-6-(3-(1-(4-methyl-4H-1,2,4-triazol-3-yl)cyclobutyl)phenyl)-4-( trifluoromethyl)-1,6-dihydro-7H-pyrrolo[2,3-c]pyridin-7-one (8.69 mg, 15.5 µmol, 28.9% yield) was obtained as a white solid. Confirmed by H NMR and LCMS.
¹H NMR: (DMSO-*d*₆, 400 MHz)
δ 12.39-12.59 (m, 1H), 8.35 (s, 1H), 7.73 (s, 1H), 7.48-7.55 (m, 1H), 7.44 (s, 1H), 7.36 (d, *J* = 6.8 Hz, 1H), 7.26 (d, *J* = 8.0 Hz, 1H), 6.32 (s, 1H), 3.75 (s, 2H), 3.27 (s, 3H), 3.03-3.09 (m, 1H), 2.90-2.97 (m, 2H), 2.79-2.85 (m, 1H), 2.67 (dd, *J* = 5.6, 1.6 Hz, 2H), 2.33 (s, 1H), 2.07-2.14 (m, 1H), 1.93-2.02 (m, 2H), 1.63-1.72 (m, 1H), 1.32-1.41 (m, 1H), 1.23 (s, 1H), 0.95 (d, *J=* 6.4 Hz, 3H)
LCMS: m/z = 561.4 (M+H)⁺, Rt = 1.071 min

### Examples 117 and 118: Synthesis of compounds T117& T118

### 1. General steps for preparation of 2-(diethylaminomethyl)-6-[3-[3-methoxy-1-(4-methyl-1,2,4-triazol-3-yl)cyclobutyl]phenyl]-4-(trifluoromethyl)-1H -pyrrolo[2,3-c]pyridin-7-one

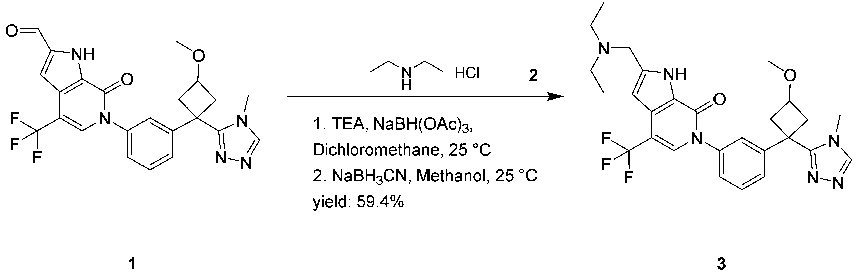

To a mixture of N-ethylethanamine (69.7 mg, 635 µmol, HCl) in Dichloromethane (5.00 mL) was added TEA (193 mg, 1.91 mmol) adjust pH = 8. Then 6-[3-[3-methoxy-1-(4-methyl-1,2,4-triazol-3-yl)cyclobutyl]phenyl]-7-oxo-4-(trifluoromethyl)-1H-pyrrolo[2,3-c]pyr idine-2-carbaldehyde (150 mg, 318 µmol) was added to the mixture. The mixture was stirred under N₂ at 25 °C for 0.5 hr. NaBH(OAc)₃ (169 mg, 797 µmol) was added to the mixture. The mixture was stirred under N₂ at 25 °C for 2.5 hrs. Then Methanol (1.00 mL) and NaBH₃CN (20.0 mg, 318 µmol) was added to the mixture. The mixture was stirred under N₂ at 25 °C for 3 hrs. LCMS showed desired mass was detected. The reaction mixture was diluted with NaHCO₃ (5.00 mL), extracted with Dichloromethane (5.00 mL*2). The organic phase dried over Na₂SO₄ and concentrated in vacuum. The crude product was purified by Prep-HPLC (column: Welch Xtimate C18 40*200 mm 7 um; mobile phase: [water(FA)-ACN]; gradient:0%-36.0% B over 25 min). To afford 2-(diethylaminomethyl)-6-[3-[3-methoxy-1-(4-methyl-1,2,4-triazol-3-yl)cyclobutyl]phenyl]-4-(trifluoromethyl)-1H -pyrrolo[2,3-c]pyridin-7-one (100 mg, 189 µmol, 59.4% yield) as a white solid.

### 2. General steps for preparation of 2-((diethylamino)methyl)-6-(3-((1s,3s)-3-methoxy-1-(4-methyl-4H-1,2,4-triazol-3-yl)cyclobutyl)phenyl)-4-(triffuor omethyl)-1,6-dihydro-7H-pyrrolo[2,3-c]pyridin-7-one (compound T117) and 2-((diethylamino)methyl)-6-(3-((1r,3r)-3-methoxy-1-(4-methyl-4H-1,2,4-triazol-3-yl)cyclobutyl)phenyl)-4-(trifluor omethyl)-1,6-dihydro-7H-pyrrolo[2,3-c]pyridin-7-one (compound T118)

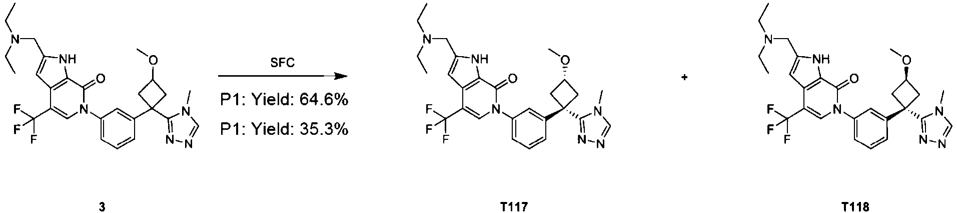

SFC (EB6211-847-P1A) showed two peaks. The crude product was purified by SFC (column: DAICEL CHIRALPAK IG (250 mm*30 mm, 10 um); mobile phase: [CO₂-EtOH (0.10%NH₃H₂O)]; B%:45.0%, isocratic elution mode). To afford 2-((diethylamino)methyl)-6-(3-((1s,3s)-3-methoxy-1-(4-methyl-4H-1,2,4-triazol-3-yl)cyclobutyl)phenyl)-4-(triffuor omethyl)-1,6-dihydro-7H-pyrrolo[2,3-c]pyridin-7-one (40.0 mg, 70.6 µmol, 37.3% yield, 93.3% purity) as a white solid. The crude product was purified by Prep-HPLC (column: Welch Xtimate C18 40*200 mm 7 um; mobile phase: [water (NH₃H₂O+NH₄HCO₃)-ACN]; gradient: 24.0%-64.0% B over 25 min). To afford 2-(diethylaminomethyl)-6-[3-[3-methoxy-1-(4-methyl-1,2,4-triazol-3-yl)cyclobutyl]phenyl]-4-(trifluoromethyl)-1H -pyrrolo[2,3-c]pyridin-7-one (19.4 mg, 36.7 µmol, 64.6% yield, 100% purity) as a white solid which was confirmed by H NMR and LCMS.
¹H NMR: (400 MHz, DMSO-*d*₆)
δ 12.41 (s, 1H), 8.38 (s, 1H), 7.71 (s, 1H), 7.45-7.53 (m, 1H), 7.34-7.40 (m, 2H), 7.18 (d, *J=* 8.0 Hz, 1H), 6.27 (s, 1H), 3.85 (quin, *J* = 7.2 Hz, 1H), 3.69 (s, 2H), 3.30 (s, 3H), 3.16 (s, 3H), 2.54 (d, *J* = 2.4 Hz, 4H), 2.44-2.49 (m, 4H), 0.99 (t, *J* = 7.2 Hz, 6H)
LCMS: m/z = 529.3 (M+H)⁺, Rt = 1.232 min

To afford 2-((diethylamino)methyl)-6-(3-((1r,3r)-3-methoxy-1-(4-methyl-4H-1,2,4-triazol-3-yl)cyclobutyl)phenyl)-4-(trifluor omethyl)-1,6-dihydro-7H-pyrrolo[2,3-c]pyridin-7-one (40.0 mg, 63.4 µmol, 33.5% yield, 83.8% purity) as a white solid. The crude product was purified by Prep-HPLC (column: Welch Xtimate C18 40*200 mm 7 um; mobile phase: [water (NH₃H₂O+NH₄HCO₃)-ACN]; gradient: 24.0%-64.0% B over 25 min). To afford 2-(diethylaminomethyl)-6-[3-[3-methoxy-1-(4-methyl-1,2,4-triazol-3-yl)cyclobutyl]phenyl]-4-(trifluoromethyl)-1H -pyrrolo[2,3-c]pyridin-7-one (10.6 mg, 20.0 µmol, 35.3% yield, 100% purity) as a white solid which was confirmed by H NMR and LCMS.
¹H NMR: (400 MHz, DMSO-*d*₆)
δ 12.41 (s, 1H), 8.30 (s, 1H), 7.72 (d, *J* = 1.2 Hz, 1H), 7.53 (d, *J* = 2.0 Hz, 1H), 7.49-7.52 (m, 1H), 7.36-7.40 (m, 1H), 7.32 (br d, *J* = 8.0 Hz, 1H), 6.27 (s, 1H), 4.06 (quin, *J* = 7.2 Hz, 1H), 3.69 (s, 2H), 3.27 (s, 3H), 3.15 (s, 3H), 3.11 (ddd, *J =* 10.0, 7.2, 2.4 Hz, 2H), 2.75-2.82 (m, 2H), 2.44-2.49 (m, 4H), 0.99 (t, *J* = 7.2 Hz, 6H)
LCMS: m/z = 529.3 (M+H)⁺, Rt = 1.215 min

### Examples 119 and 120: Synthesis of compounds T119& T120

### 1. General steps for preparation of 6-(3-(3,3-difluoro-1-((R)-fluoro(4-methyl-4H-1,2,4-triazol-3-yl)methyl)cyclobutyl)phenyl)-2-(((S)-3-methylpiperid in-1-yl)methyl)-4-(trifluoromethyl)-1,6-dihydro-7H-pyrrolo[2,3-c]pyridin-7-one (compound T119) and 6-(3-(3,3-difluoro-1-((S)-fluoro(4-methyl-4H-1,2,4-triazol-3-yl)methyl)cyclobutyl)phenyl)-2-(((S)-3-methylpiperid in-1-yl)methyl)-4-(trifluoromethyl)-1,6-dihydro-7H-pyrrolo[2,3-c]pyridin-7-one (compound T120)

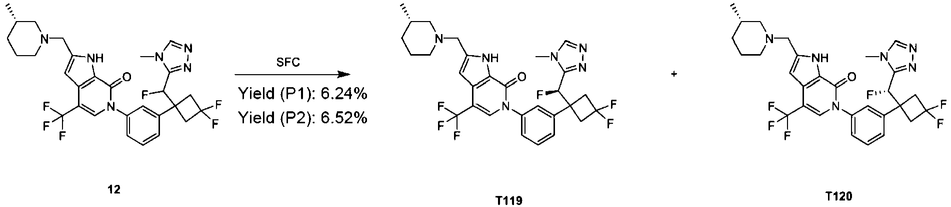

The residue was purified by Prep-HPLC (column: Welch Xtimate C18 40 * 200 mm 7 um; mobile phase: [water(FA)-ACN];gradient:2.00%-42.0% B over 25 mins) and SFC (column: DAICEL CHIRALCEL OD (250 mm * 30 mm,10 um);mobile phase: [CO₂-EtOH(0.1%NH₃H₂O)]; B%:25.0%, isocratic elution mode). Compound 6-(3-(3,3-difluoro-1-((R)-fluoro(4-methyl-4H-1,2,4-triazol-3-yl)methyl)cyclobutyl)phenyl)-2-(((S)-3-methylpiperid in-1-yl)methyl)-4-(trifluoromethyl)-1,6-dihydro-7H-pyrrolo[2,3-c]pyridin-7-one (12.4 mg, 20.2 µmol, 6.24% yield, 96.6% purity) was obtained as a white solid. Confirmed by H NMR and LCMS.
¹H NMR: (DMSO-*d*₆, 400 MHz)
δ 12.39-12.47 (m, 1H), 7.58 (d, *J* = 1.2 Hz, 1H), 7.40-7.45 (m, 2H), 7.31 (s, 1H), 7.16-7.20 (m, 1H), 6.27 (s, 1H), 6.04-6.17 (m, 1H), 3.60 (s, 2H), 3.53-3.59 (m, 1H), 3.40-3.46 (m, 2H), 3.12-3.17 (m, 1H), 3.06 (s, 3H), 2.72-2.78 (m, 2H), 1.85-1.93 (m, 1H), 1.56-1.66 (m, 4H), 1.43-1.48 (m, 1H), 1.23 (s, 2H), 0.81 (d, J = 5.4 Hz, 3H)
LCMS: m/z =593.2 (M+H)⁺, Rt = 1.760 min

Compound 6-(3-(3,3-difluoro-1-((S)-fluoro(4-methyl-4H-1,2,4-triazol-3-yl)methyl)cyclobutyl)phenyl)-2-(((S)-3-methylpiperid in-1-yl)methyl)-4-(trifluoromethyl)-1,6-dihydro-7H-pyrrolo[2,3-c]pyridin-7-one (12.9 mg, 21.1 µmol, 6.52% yield, 97.1% purity) was obtained as a white solid. Confirmed by H NMR and LCMS.
¹H NMR: (DMSO-*d*₆, 400 MHz)
δ 12.31-12.58 (m, 1H), 7.58 (d, *J =* 1.2 Hz, 1H), 7.40-7.45 (m, 2H), 7.31 (s, 1H), 7.16-7.20 (m, 1H), 6.27 (s, 1H), 6.04-6.17 (m, 1H), 3.60 (s, 2H), 3.53-3.59 (m, 1H), 3.40-3.46 (m, 2H), 3.12-3.17 (m, 1H), 3.06 (s, 3H), 2.72-2.78 (m, 2H), 1.85-1.93 (m, 1H), 1.56-1.66 (m, 4H), 1.43-1.48 (m, 1H), 1.23 (s, 2H), 0.81 (d, *J =* 5.4 Hz, 3H)
LCMS: m/z =593.2 (M+H)⁺, Rt = 1.777 min

### Example 121: Synthesis of compound T121

### 1. General steps for preparation of ethyl 2-(3,5-dibromophenyl)acetate

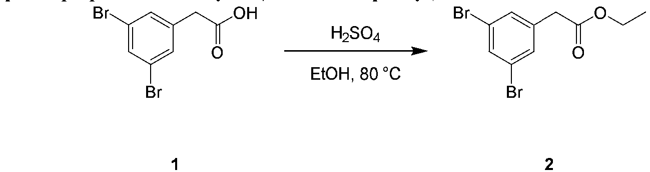

To a solution of 2-(3,5-dibromophenyl)acetic acid (25.0 g, 85.0 mmol) in EtOH (250 mL) was added H₂SO₄ (1.67 g, 17.0 mmol). The mixture was stirred under N₂ at 80 °C for 2 hrs. TLC (Petroleum ether/ Ethyl acetate = *5*/ 1, product 1 R_{f} = 0.40) indicated new spot formed. The reaction mixture was diluted with saturated aqueous NaHCO₃ (200 mL) slowly, adjust pH = 8 (saturated aqueous NaHCO₃). The mixture was extracted with Ethyl acetate (200 mL*2). The organic phase was washed with brine (500 mL), dried over Na₂SO₄ and concentrated in vacuum. The crude product was purified by column chromatography (SiO₂, Petroleum ether/ Ethyl acetate = 1/ 0 to *5*/ *1).* To afford ethyl 2-(3,5-dibromophenyl)acetate (27.0 g, 83.6 mmol, 98.2% yield, 99.7% purity) as yellow oil which was confirmed by H NMR and LCMS.
¹H NMR: (400 MHz, CDCl₃)
*δ* 7.58 (t, *J=* 1.6 Hz, 1H), 7.38 (d, *J=* 1.6 Hz, 2H), 4.17 (q, *J =* 7.2 Hz, 2H), 3.55 (s, 2H), 1.27 (t, *J=* 7.2 Hz, 3H)
LCMS: m/z = 322.6 (M+H)⁺, Rt = 1.140 min

### 2. General steps for preparation of ethyl 2-(3-bromo-5-cyano-phenyl)acetate

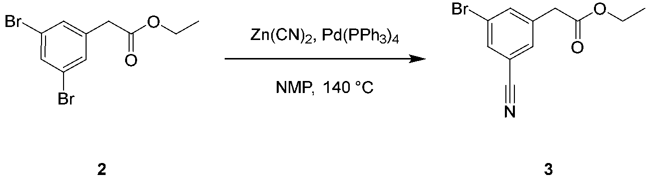

To a solution of ethyl 2-(3,5-dibromophenyl)acetate (10.0 g, 31.0 mmol) and Pd(PPh₃)₄ (3.60 g, 3.12 mmol) in NMP (100 mL) was adde Zn(CN)₂ (1.64 g, 13.9 mmol). The suspension was purged with N₂ several times. The mixture was stirred at 140 °C for 2 hrs under N₂. TLC (Petroleum ether/ Ethyl acetate = *5*/ *1,* product 1 R_{f} = 0.40) indicated new spot formed. The reaction mixture was diluted with Ethyl acetate (300 mL). The mixture was filtered and the filter cake was washed with Ethyl acetate (100 mL*3). The filtrate was washed with brine (500 mL*3), dried over Na₂SO₄. The organic phase was concentrated in vacuum. The crude product was purified by column chromatography (SiO₂, Petroleum ether/ Ethyl acetate = 1/ 0 to 10/ 1). To afford ethyl 2-(3-bromo-5-cyano-phenyl)acetate (2.90 g, 10.7 mmol, 34.6% yield, 99.4% purity) as a white solid which was confirmed by H NMR and LCMS.
¹H NMR: (400 MHz, CDCl₃)
δ 7.63-7.75 (m, 2H), 7.53 (s, 1H), 4.18 (q, *J = 7.2* Hz, 2H), 3.62 (s, 2H), 1.28 (t, *J=* 7.2 Hz, 3H)
LCMS: m/z = 266.1 (M+H)⁺, Rt = 1.312 min

### 3. General steps for preparation of ethyl 1-(3-bromo-5-cyano-phenyl)-3-methyl-cyclobutanecarboxylate

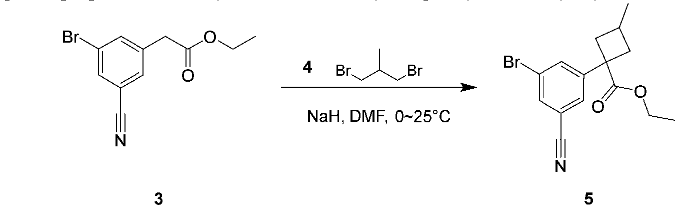

To a solution of ethyl 2-(3-bromo-5-cyano-phenyl)acetate (2.60 g, 9.70 mmol), 1,3-dibromo-2-methyl-propane (2.10 g, 9.71 mmol) in DMF (52.0 mL) was added NaH (776 mg, 19.4 mmol, 60.0% purity) at 0°C. The mixture was stirred under N₂ at 25 °C for 2 hrs. TLC (Petroleum ether/ Ethyl acetate = *5*/ 1*,* product 1 R_{f} = 0.50) indicated new spot formed. The reaction mixture was poured into saturated aqueous NH₄Cl (50.0 mL) slowly. The reaction mixture was extracted with Ethyl acetate (80.0 mL*2). The organic phase was washed with brine (100 mL*3), dried over Na₂SO₄ and concentrated in vacuum. The crude product was purified by column chromatography (SiO₂, Petroleum ether/ Ethyl acetate = 1/ 0 to 10/ 1). To afford ethyl 1-(3-bromo-5-cyano-phenyl)-3-methyl-cyclobutanecarboxylate (2.30 g, 6.87 mmol, 70.8% yield, 96.2% purity) as colorless oil which was confirmed by H NMR and LCMS.
¹H NMR: (400 MHz, CDCl₃)
*δ* 7.75 (t, *J* = 1.6 Hz, 1H), 7.63-7.69 (m, 1H), 7.56-7.62 (m, 1H), 4.07-4.13 (m, 2H), 2.58-3.03 (m, 2H), 2.24-2.50 (m, 2H), 1.93-2.04 (m, 1H), 1.17-1.21 (m, 3H), 1.04-1.13 (m, 3H)
LCMS: m/z = 323.8 (M+H)⁺, Rt = 1.280 min

### 4. General steps for preparation of 1-(3-bromo-5-cyano-phenyl)-3-methyl-cyclobutanecarboxylic acid

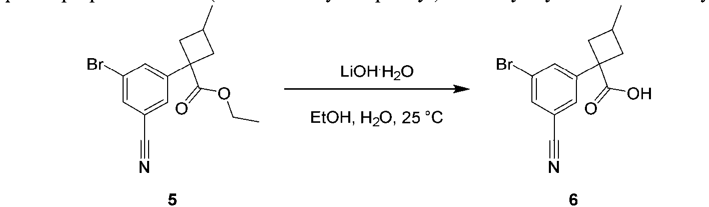

A mixture of ethyl 1-(3-bromo-5-cyano-phenyl)-3-methyl-cyclobutanecarboxylate (2.20 g, 6.83 mmol) in EtOH (22.0 mL). Then a solution of LiOH.H₂O (1.72 g, 41.0 mmol) in H₂O (5.50 mL) was added to the mixture. The mixture was stirred under N₂ at 25 °C for 2 hrs. LCMS showed desired mass was detected. The reaction mixture was diluted with brine (20.0 mL), adjust pH = 5 (HCl, 2 M). The mixture was extracted with Ethyl acetate (50.0 mL*2). The organic phase was dried over Na₂SO₄ and concentrated in vacuum. Without purification. To afford 1-(3-bromo-5-cyano-phenyl)-3-methyl-cyclobutanecarboxylic acid (2.00 g, crude) as a white solid.

### 5. General steps for preparation of 1-[[1-(3-bromo-5-cyano-phenyl)-3-methyl-cyclobutanecarbonyl]amino]-3-methyl-thiourea

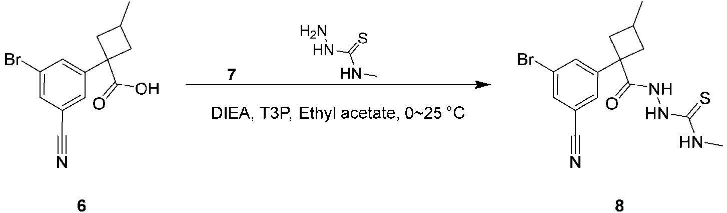

To a mixture of 1-(3-bromo-5-cyano-phenyl)-3-methyl-cyclobutanecarboxylic acid (2.00 g, 6.80 mmol) and 1-amino-3-methyl-thiourea (929 mg, 8.83 mmol) in Ethyl acetate (20.0 mL) was added DIEA (8.79 g, 67.9 mmol). Then T3P (13.5 g, 21.2 mmol, 12.6 mL, 50.0% purity) was added to the mixture slowly at 0 °C. The mixture was stirred under N₂ at 25 °C for 2 hrs. LCMS showed desired mass was detected. The reaction mixture was diluted with saturated aqueous NH₄Cl (50.0 mL), extracted with Ethyl acetate (50.0 mL*2). The organic phase was dried over Na₂SO₄ and concentrated in vacuum. Without purification. To afford 1-[[1-(3-bromo-5-cyano-phenyl)-3-methyl-cyclobutanecarbonyl]amino]-3-methyl-thiourea (3.50 g, crude) as a yellow solid.

### 6. General steps for preparation of 3-bromo-5-[3-methyl-1-(4-methyl-5-sulfanyl-1,2,4-triazol-3-yl)cyclobutyl]benzoic acid:

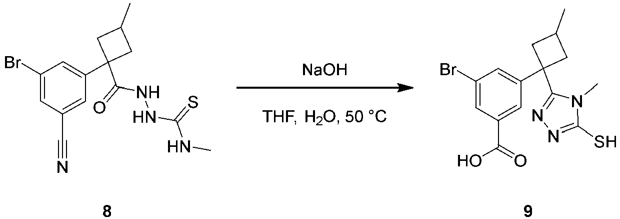

A mixture of 1-[[1-(3-bromo-5-cyano-phenyl)-3-methyl-cyclobutanecarbonyl]amino]-3-methyl-thiourea (2.90 g, 7.61 mmol) in THF (14.5 mL). Then a solution of NaOH (4.56 g, 114 mmol) in H₂O (29.0 mL) was added to the mixture. The mixture was stirred under N₂ at 50 °C for 12 hrs. LCMS showed desired mass was detected. The reaction mixture was diluted with NaOH (30.0 mL, 2 M), extracted with Ethyl acetate (50.0 mL*2). The aqueous phase was diluted with Ethyl acetate (20.0 mL), adjust pH= 4 (HCl, 2 M) and extracted with Ethyl acetate (50.0 mL*2). The second organic phase was dried over Na₂SO₄ and concentrated in vacuum. Without purification. To afford 3-bromo-5-[3-methyl-1-(4-methyl-5-sulfanyl-1,2,4-triazol-3-yl)cyclobutyl]benzoic acid (2.50 g, 5.95 mmol, 78.2% yield, 91.0% purity) as a yellow solid which was confirmed by LCMS.
LCMS: m/z = 383.5 (M+H)⁺, Rt = 1.288 min

### 7. General steps for preparation of 3-bromo-5-[3-methyl-1-(4-methyl-1,2,4-triazol-3-yl)cyclobutyl]benzoic acid

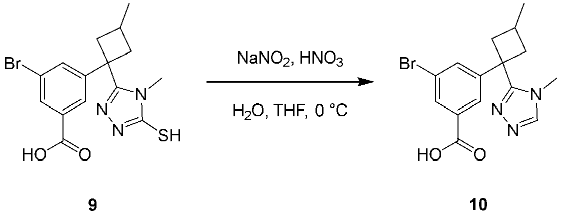

To a solution of 3-bromo-5-[3-methyl-1-(4-methyl-5-sulfanyl-1,2,4-triazol-3-yl)cyclobutyl]benzoic acid (2.50 g, 6.54 mmol) in THF (25.0 mL) and H₂O (25.0 mL) was added NaNO₂ (4.51 g, 65.3 mmol). HNO₃ (6.49 g, 66.9 mmol, 4.64 mL, 65.0% purity) was added to the mixture slowly at 0 °C. After addition, the mixture was stirred at 0 °C for 1 hr. LCMS showed desired mass was detected. The reaction mixture was poured into brine (20.0 mL) slowly, adjust pH = 6 (NaHCO₃, solid). The mixture was extracted with Ethyl acetate (50.0 mL*3). The organic phase was dried over Na₂SO₄ and concentrated in vacuum. The crude product was purified by reversed-phase HPLC (0.1% HCl condition). To afford 3-bromo-5-[3-methyl-1-(4-methyl-1,2,4-triazol-3-yl)cyclobutyl]benzoic acid (1.50 g, 3.94 mmol, 60.2% yield, 92.0% purity) as a yellow solid which was confirmed by H NMR and LCMS.
¹H NMR: (400 MHz, DMSO-*d*₆)
δ 13.11-13.71 (s, 1H), 8.29-8.43 (m, 1H), 7.86-7.98 (m, 1H), 7.53-7.81 (m, 2H), 3.15-3.20 (m, 3H), 2.81-3.13 (m, 2H), 2.52-2.58 (m, 2H), 2.18-2.41 (m, 1H), 1.00-1.13 (m, 3H)
LCMS: m/z = 351.5 (M+H)⁺, Rt = 1.145 min

### 8. General steps for preparation of 3-[3-methyl-1-(4-methyl-1,2,4-triazol-3-yl)cyclobutyl]-5-[2-[[(3S)-3-methyl-1-piperidyl]methyl]-7-oxo-1-(p-tolyls ulfonyl)-4-(trifluoromethyl)pyrrolo[2,3-c]pyridin-6-yl]benzoic acid

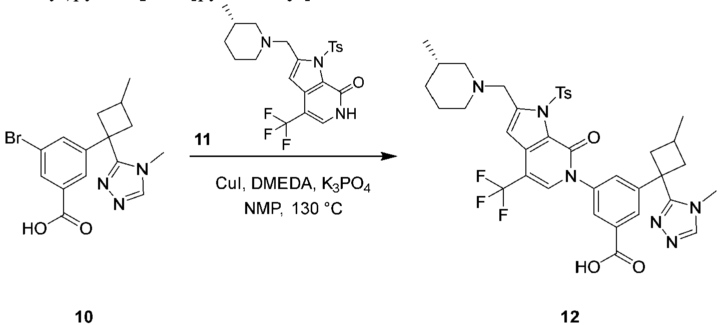

To a solution of 3-bromo-5-[3-methyl-1-(4-methyl-1,2,4-triazol-3-yl)cyclobutyl]benzoic acid (500 mg, 1.43 mmol) and 2-[[(3S)-3-methyl-1-piperidyl]methyl]-1-(p-tolylsulfonyl)-4-(trifluoromethyl)-6H-pyrrolo[2,3-c]pyridin-7-one (634 mg, 1.36 mmol) in NMP (5.00 mL) was added K₃PO₄ (909 mg, 4.28 mmol), CuI (544 mg, 2.86 mmol) and DMEDA (126 mg, 1.43 mmol). The suspension was degassed under vacuum and purged with N₂ several times. The mixture was stirred under N₂ at 130 °C for 1 hr. LCMS showed desired mass was detected. The reaction mixture was diluted with saturated aqueous NH₄Cl (50.0 mL) and Ethyl acetate (20.0 mL). The mixture was filtered and the filter cake was washed with Ethyl acetate (10.0 mL*3). The filtrate was extracted with Ethyl acetate (50.0 mL*2), dried over Na₂SO₄. The organic phase was concentrated in vacuum. The crude product was purified by Prep-HPLC (column: Xtimate C18 150*40 mm*10 um; mobile phase: [water (HCl)-ACN]; gradient: 14.0%-44.0% B over 36 mins). To afford 3-[3-methyl-1-(4-methyl-1,2,4-triazol-3-yl)cyclobutyl]-5-[2-[[(3S)-3-methyl-1-piperidyl]methyl]-7-oxo-1-(p-tolyls ulfonyl)-4-(trifluoromethyl)pyrrolo[2,3-c]pyridin-6-yl]benzoic acid (460 mg, 531 µmol, 37.2% yield, 85.2% purity) as a yellow solid which was confirmed by H NMR and LCMS.
¹H NMR: (400 MHz, DMSO-*d*₆)
δ 12.92-13.89 (s, 1H), 9.23 (s, 1H), 8.13-8.19 (m, 1H), 7.97-8.03 (d, *J=* 8.4 Hz, 2H), 7.89-7.94 (m, 1H), 7.79 (s, 1H), 7.67-7.74 (m, 1H), 7.51 (s, 1H), 7.41-7.45 (d, *J =* 8.4 Hz, 2H), 4.91 (s, 2H), 3.39-3.55 (m, 2H), 3.36-3.39 (m, 3H), 2.99-3.10 (m, 1H), 2.88-2.94 (m, 2H), 2.73-2.84 (m, 1H), 2.59-2.66 (m, 2H), 2.51-2.57 (m, 1H), 2.40 (s, 3H), 2.04-2.15 (m, 1H), 1.74-1.96 (m, 3H), 1.11-1.25 (m, 1H), 1.09 (d, *J* = 6.4 Hz, 3H), 0.90 (d, *J* = 6.4 Hz, 3H)
LCMS: m/z = 737.1 (M+H)⁺, Rt = 1.183 min

### 9. General steps for preparation of 3-[3-methyl-1-(4-methyl-1,2,4-triazol-3-yl)cyclobutyl]-5-[2-[[(3S)-3-methyl-1-piperidyl]methyl]-7-oxo-4-(trifluoro methyl)-1H-pyrrolo[2,3-c]pyridin-6-yl]benzoic acid (compound T128)

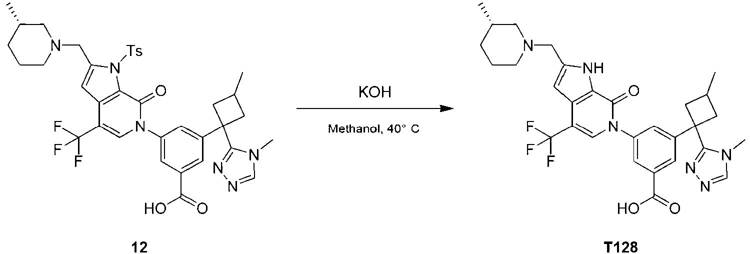

To a solution of 3-[3-methyl-1-(4-methyl-1,2,4-triazol-3-yl)cyclobutyl]-5-[2-[[(3S)-3-methyl-1-piperidyl]methyl]-7-oxo-1-(p-tolyls ulfonyl)-4-(trifluoromethyl)pyrrolo[2,3-c]pyridin-6-yl]benzoic acid (400 mg, 542 µmol) in Methanol (5.00 mL) was added KOH (610 mg, 10.8 mmol). The mixture was stirred under N₂ at 40 °C for 1 hr. LCMS showed desired mass was detected. The reaction mixture was adjust pH = 5 (HCl, 2 M) and concentrated in vacuum. The crude product was purified by reversed-phase HPLC (0.10% HCl condition). To afford 3-[3-methyl-1-(4-methyl-1,2,4-triazol-3-yl)cyclobutyl]-5-[2-[[(3S)-3-methyl-1-piperidyl]methyl]-7-oxo-4-(trifluoro methyl)-1H-pyrrolo[2,3-c]pyridin-6-yl]benzoic acid (330 mg, 530 µmol, 97.6% yield, 93.6% purity) as a yellow solid which was confirmed by H NMR and LCMS.
¹H NMR: (400 MHz, DMSO-*d*₆)
δ 12.83 (s, 1H), 9.11-9.34 (m, 1H), 7.91-7.96 (m, 3H), 7.48 (d, *J = 8.0* Hz, 1H), 7.11 (d, *J=* 7.6 Hz, 1H), 6.77 (s, 1H), 4.41 (s, 2H), 3.39-3.44 (m, 3H), 3.28 (d, *J =* 10.4 Hz, 1H), 3.01-3.25 (m, 1H), 2.83-3.01 (m, 2H), 2.67-2.83 (m, 1H), 2.57-2.67 (m, 2H), 2.51-2.57 (m, 1H), 2.31-2.42 (m, 1H), 1.99 *(d, J=* 5.2 Hz, 1H), 1.82 (s, 2H), 1.71 (d, *J* = 12.8 Hz, 1H), 1.09 (d, *J =* 6.0 Hz, 3H), 0.97-1.06 (m, 1H), 0.88 (d, *J=* 6.4 Hz, 3H)
LCMS: m/z = 583.1 (M+H)⁺, Rt = 1.010 min

### 10. General steps for preparation of 3-[3-methyl-1-(4-methyl-1,2,4-triazol-3-yl)cyclobutyl]-5-[2-[[(3S)-3-methyl-1-piperidyl]methyl]-7-oxo-4-(trifluoro methyl)-1H-pyrrolo[2,3-c]pyridin-6-yl]benzamide (compound T127)

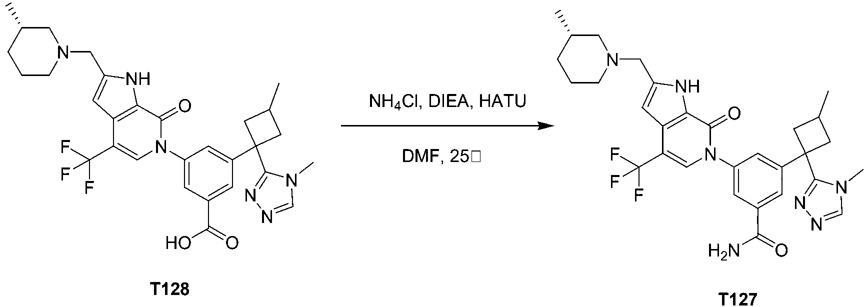

To a solution of 3-[3-methyl-1-(4-methyl-1,2,4-triazol-3-yl)cyclobutyl]-5-[2-[[(3S)-3-methyl-1-piperidyl]methyl]-7-oxo-4-(trifluoro methyl)-1H-pyrrolo[2,3-c]pyridin-6-yl]benzoic acid (255 mg, 437 µmol) and DIEA (170 mg, 1.32 mmol) in DMF (5.00 mL) was added and HATU (250 mg, 657 µmol) and NH₄Cl (234 mg, 4.38 mmol). The mixture was stirred under N₂ at 25 °C for 0.5 hr. LCMS showed desired mass was detected. The reaction mixture was diluted with Ethyl acetate (20.0 mL) and poured into saturated aqueous Na₂CO₃ (20.0 mL) slowly, adjust pH = 8 (saturated aqueous Na₂CO₃). The mixture was extracted with Ethyl acetate (30.0 mL). The organic phase washed with saturated aqueous Na₂CO₃/brine (30.0 mL*3, 1/ 1), dried over Na₂SO₄. The organic phase was concentrated in vacuum. The crude product was purified by Prep-HPLC (column: Xtimate C18 150*40 mm*10 um; mobile phase: [water(FA)-ACN]; gradient:0%-34.0% B over 25 mins). To afford 3-[3-methyl-1-(4-methyl-1,2,4-triazol-3-yl)cyclobutyl]-5-[2-[[(3S)-3-methyl-1-piperidyl]methyl]-7-oxo-4-(trifluoro methyl)-1H-pyrrolo[2,3-c]pyridin-6-yl]benzamide (55.0 mg, 83.4 µmol, 19.0% yield, 95.2% purity, FA) as a white solid which was confirmed by H NMR and LCMS
¹H NMR: (400 MHz, DMSO-*d*₆)
δ 12.28-12.63 (s, 1H), 8.29 (s, 1H), 8.12 (s, 1H), 7.87 (s, 1H), 7.80-7.84 (m, 2H), 7.69 (s, 1H), 7.52 (s, 1H), 6.28 (s, 1H), 3.61 (s, 2H), 3.25 (s, 3H), 2.88-2.93 (m, 2H), 2.73-2.79 (m, 2H), 2.53-2.60 (m, 3H), 1.90 (t, *J* = 10.0 Hz, 1H), 1.56-1.66 (m, 4H), 1.40-1.50 (m, 1H), 1.07 (d, *J=* 5.6 Hz, 3H), 0.81 (d, *J =* 5.6 Hz, 3H), 0.76 (m, 1H)
LCMS: m/z = 582.1 (M+H)⁺, Rt = 0.983 min

### 11. General steps for preparation of 3-((1s,3R)-3-methyl-1-(4-methyl-4H-1,2,4-triazol-3-yl)cyclobutyl)-5-(2-(((S)-3-methylpiperidin-1-yl)methyl)-7-ox o-4-(trifluoromethyl)-1,7-dihydro-6H-pyrrolo[2,3-c]pyridin-6-yl)benzonitrile (compound T121)

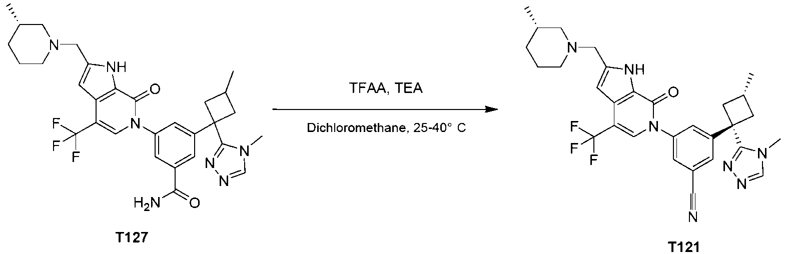

To a mixture of 3-[3-methyl-1-(4-methyl-1,2,4-triazol-3-yl)cyclobutyl]-5-[2-[[(3S)-3-methyl-1-piperidyl]methyl]-7-oxo-4-(trifluoro methyl)-1H-pyrrolo[2,3-c]pyridin-6-yl]benzamide (40.0 mg, 68.7 µmol) in Dichloromethane (5.00 mL) was added TEA (41.8 mg, 413 µmol). Then a solution of TFAA (57.8 mg, 275 µmol) in Dichloromethane (0.50 mL) was added to the mixture at 25 °C slowly. The mixture was stirred under N₂ at 40 °C for 4 hrs. LCMS showed desired mass was detected. The reaction mixture was diluted with Dichloromethane (10.0 mL), washed with saturated aqueous NaHCO₃ (10.0 mL*2). The organic phase was concentrated in vacuum. The crude product was purified by Prep-HPLC (column: Xtimate C18 150*40 mm*10 um; mobile phase: [water (FA)-ACN]; gradient:0%-38.0% B over 25 mins). To afford 3-((1s,3R)-3-methyl-1-(4-methyl-4H-1,2,4-triazol-3-yl)cyclobutyl)-5-(2-(((S)-3-methylpiperidin-1-yl)methyl)-7-ox o-4-(trifluoromethyl)-1,7-dihydro-6H-pyrrolo[2,3-c]pyridin-6-yl)benzonitrile (24.3 mg, 42.8 µmol, 62.2% yield, 99.3% purity) as a white solid which was confirmed by H NMR and LCMS.
¹H NMR: (400 MHz, DMSO-*d*₆)
δ 12.48 (s, 1H), 8.31 (s, 1H), 7.99 (d, *J* = 1.2 Hz, 1H), 7.81-7.87 (m, 3H), 6.27 (s, 1H), 3.60 (s, 2H), 3.26 (s, 3H), 2.90-2.96 (m, 2H), 2.72-2.79 (m, 2H), 2.52-2.59 (m, 3H), 1.88 (t, *J* = 10.4 Hz, 1H), 1.55-1.67 (m, 4H), 1.40-1.49 (m, 1H), 1.07 (d, *J* = 5.2 Hz, 3H), 0.81 (br d, *J =* 5.6 Hz, 3H), 0.71-0.80 (m, 1H)
LCMS: m/z = 564.1 (M+H)⁺, Rt = 1.163 min

### Example 122: Synthesis of compound T122

### 1. General steps for preparation of 3-((1r,3 S)-3-methyl-1-(4-methyl-4H-1,2,4-triazol-3-yl)cyclobutyl)-5-(2-(((S)-3-methylpiperidin-1-yl)methyl)-7-ox o-4-(trifluoromethyl)-1,7-dihydro-6H-pyrrolo[2,3-c]pyridin-6-yl)benzamide

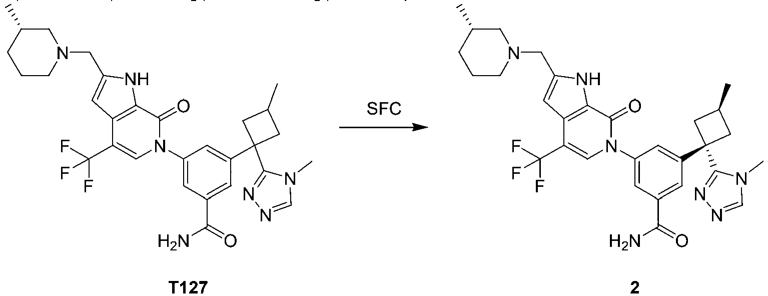

The T127 crude product of the Example 121 was purified by SFC (column: DAICEL CHIRALPAK IG (250 mm*30 mm, 10 um); mobile phase: [CO₂-EtOH (0.10%NH₃H₂O)]; B%:45.0%, isocratic elution mode). To afford 3-((1r,3S)-3-methyl-1-(4-methyl-4H-1,2,4-triazol-3-yl)cyclobutyl)-5-(2-(((S)-3-methylpiperidin-1-yl)methyl)-7-ox o-4-(trifluoromethyl)-1,7-dihydro-6H-pyrrolo[2,3-c]pyridin-6-yl)benzamide (65.0 mg, 92.7 µmol, 26.9% yield, 83.0% purity) as a yellow solid which was confirmed by H NMR and LCMS.
¹H NMR: (400 MHz, DMSO-*d*₆)
δ 12.48 (s, 1H), 8.37 (s, 1H), 8.11 (s, 1H), 7.80 (s, 2H), 7.70 (s, 1H), 7.51-7.59 (m, 2H), 6.28 (s, 1H), 3.60 (s, 2H), 3.30 (s, 3H), 3.14 (dd, *J* = 8.8, 5.6 Hz, 2H), 2.71-2.80 (m, 2H), 2.25-2.34 (m, 3H), 1.86-1.93 (m, 1H), 1.56-1.67 (m, 4H), 1.42-1.51 (m, 1H), 1.09 (d, *J=* 6.0 Hz, 3H), 0.81 (d, *J =* 5.6 Hz, 3H), 0.75 (m, 1H)
LCMS: m/z = 582.2 (M+H)⁺, Rt = 1.047 min

### 2. General steps for preparation of 3-((1r,3S)-3-methyl-1-(4-methyl-4H-1,2,4-triazol-3-yl)cyclobutyl)-5-(2-(((S)-3-methylpiperidin-1-yl)methyl)-7-ox o-4-(trifluoromethyl)-1,7-dihydro-6H-pyrrolo[2,3-c]pyridin-6-yl)benzonitrile (compound T122)

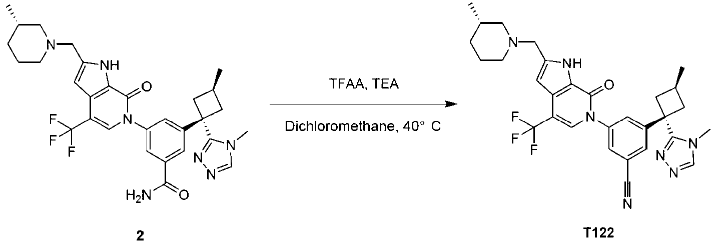

To a mixture of 3-((1r,3 S)-3-methyl-1-(4-methyl-4H-1,2,4-triazol-3-yl)cyclobutyl)-5-(2-(((S)-3-methylpiperidin-1-yl)methyl)-7-ox o-4-(trifluoromethyl)-1,7-dihydro-6H-pyrrolo[2,3-c]pyridin-6-yl)benzamide (60.0 mg, 103 µmol) in dichloromethane (5.00 mL) was added TEA (62.7 mg, 619 µmol, 86.2 µL) and TFAA (86.7 mg, 412 µmol). The mixture was stirred under N₂ at 40 °C for 2 hrs. LCMS showed desired mass was detected. The reaction mixture was diluted with dichloromethane (10.0 mL), washed with saturated aqueous NaHCO₃ (10.0 mL*2). The organic phase was concentrated in vacuum. The crude product was purified by Prep-HPLC (column: Xtimate C18 150*40 mm*10 um; mobile phase: [water (FA)-ACN]; gradient: 0%-40.0% B over 25 mins). To afford 3-((1r,3 S)-3-methyl-1-(4-methyl-4H-1,2,4-triazol-3-yl)cyclobutyl)-5-(2-(((S)-3-methylpiperidin-1-yl)methyl)-7-ox o-4-(trifluoromethyl)-1,7-dihydro-6H-pyrrolo[2,3-c]pyridin-6-yl)benzonitrile (22.0 mg, 35.8 µmol, 34.7% yield, 99.2% purity, FA) as a white solid which was confirmed by H NMR and LCMS.
¹H NMR: (400 MHz, DMSO-*d*₆)
δ 12.48 (s, 1H), 8.39 (s, 1H), 7.95 (d, *J* = 1.6 Hz, 1H), 7.82 (d, *J* = 1.2 Hz, 1H), 7.68 (d, *J* = 1.6 Hz, 2H), 6.27 (s, 1H), 3.60 (s, 2H), 3.30 (s, 3H), 3.11 (d, *J* = 3.6 Hz, 2H), 2.72-2.79 (m, 2H), 2.29-2.36 (m, 3H), 1.88 (t, J= 10.4 Hz, 1H), 1.55-1.65 (m, 4H), 1.39-1.49 (m, 1H), 1.09 (d, *J* = 5.6 Hz, 3H), 0.80 (d, *J* = 5.6 Hz, 3H), 0.75 (s, 1H)
LCMS: m/z = 563.9 (M+H)⁺, Rt = 1.148 min

### Examples 123 and 124: Synthesis of compounds T123& T124

### 1. General steps for preparation of 2-((2-oxa-7-azaspiro[4.5]decan-7-yl)methyl)-6-(3-(1-(4-methyl-4H-1,2,4-triazol-3-yl)cyclobutyl)phenyl)-4-(trifluor omethyl)-1,6-dihydro-7H-pyrrolo[2,3-c]pyridin-7-one

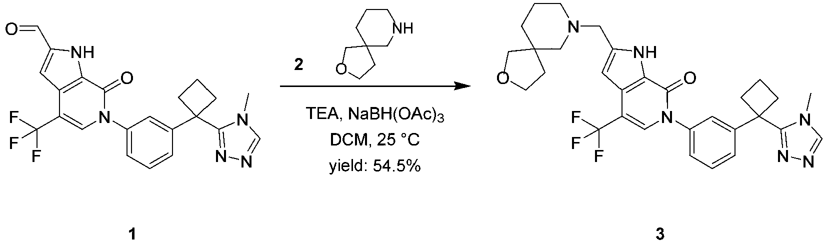

To a solution of 6-[3-[1-(4-methyl-1,2,4-triazol-3-yl)cyclobutyl]phenyl]-7-oxo-4-(trifluoromethyl)-1H-pyrrolo[2,3-c]pyridine-2-car baldehyde (100 mg, 226 µmol) in dichloromethane (2.00 mL) was added dropwise TEA (22.9 mg, 226 µmol, 31.5 µL) adjust pH = 7, 2-oxa-9-azaspiro[4.5]decane (52.3 mg, 294 µmol, HCl) was added to the mixture, stirred at 25 °C for 0.5 hr, then NaBH(OAc)₃ (240 mg, 1.13 mmol) was added to the mixture, stirred at 25 °C for 1 hr. LCMS showed desired compound was detected. The reaction mixture was diluted with aqueous NaHCO₃ (8.00 mL), extracted with dichloromethane (12.0 mL * 3), the combined organic layers were washed with H₂O (8.00 mL), dried over Na₂SO₄, filtered and concentrated under reduced pressure to give a residue. The residue was purified by Prep-HPLC (column: Welch Xtimate C18 40 * 200 mm 7 um; mobile phase: [water (NH₄HCO₃)-ACN]; gradient:26.0%-66.0% B over 25 mins). Compound 2-((2-oxa-7-azaspiro[4.5]decan-7-yl)methyl)-6-(3-(1-(4-methyl-4H-1,2,4-triazol-3-yl)cyclobutyl)phenyl)-4-(trifluor omethyl)-1,6-dihydro-7H-pyrrolo[2,3-c]pyridin-7-one (70.0 mg, 123 µmol, 54.5% yield, 100% purity) was obtained as a white solid. Confirmed by LCMS.
LCMS: m/z = 567.2 (M+H)⁺, Rt = 1.317 min

### 2. General steps for preparation of (S)-2-((2-oxa-7-azaspiro[4.5]decan-7-yl)methyl)-6-(3-(1-(4-methyl-4H-1,2,4-triazol-3-yl)cyclobutyl)phenyl)-4-(trif luoromethyl)-1,6-dihydro-7H-pyrrolo[2,3-c]pyridin-7-one (compound T123) and (R)-2-((2-oxa-7-azaspiro[4.5]decan-7-yl)methyl)-6-(3-(1-(4-methyl-4H-1,2,4-triazol-3-yl)cyclobutyl)phenyl)-4-(trif luoromethyl)-1,6-dihydro-7H-pyrrolo[2,3-c]pyridin-7-one (compound T124)

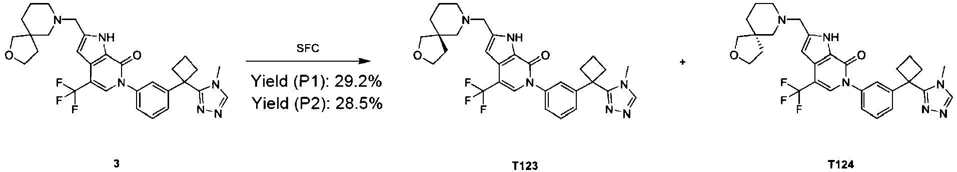

The residue was purified by Prep-HPLC (column: DAICEL CHIRALPAK AD(250 mm*30 mm,10 um); mobile phase: [CO₂-EtOH (0.1% NH₃H₂O)]; B%:55.0%, isocratic elution mode). Compound (S)-2-((2-oxa-7-azaspiro[4.5]decan-7-yl)methyl)-6-(3-(1-(4-methyl-4H-1,2,4-triazol-3-yl)cyclobutyl)phenyl)-4-(trif luoromethyl)-1,6-dihydro-7H-pyrrolo[2,3-c]pyridin-7-one (20.5 mg, 36.1 µmol, 29.2% yield, 100% purity) was obtained as a white solid. Confirmed by H NMR and LCMS.
¹H NMR: (DMSO-*d*₆, 400 MHz)
δ 12.43 (s, 1H), 8.35 (s, 1H), 7.71 (s, 1H), 7.48-7.54 (m, 1H), 7.44 (s, 1H), 7.36 *(d, J=* 8.0 Hz, 1H), 7.25 (d, *J* = 7.6 Hz, 1H), 6.28 (s, 1H), 3.63-3.68 (m, 2H), 3.59-3.63 (m, 2H), 3.31 (d, *J =* 8.4 Hz, 2H), 3.27 (s, 3H), 2.89-2.97 (m, 2H), 2.66-2.73 (m, 2H), 2.23-2.33 (m, 2H), 1.95-2.02 (m, 2H), 1.70-1.83 (m, 1H), 1.49-1.59 (m, 2H), 1.38-1.48 (m, 2H), 1.34 (dd, *J =* 8.4, 4.0 Hz, 1H), 1.23 (s, 2H)
LCMS: m/z = 567.2 (M+H)⁺, Rt = 1.377 min

Compound (R)-2-((2-oxa-7-azaspiro[4.5]decan-7-yl)methyl)-6-(3-(1-(4-methyl-4H-1,2,4-triazol-3-yl)cyclobutyl)phenyl)-4-(trif luoromethyl)-1,6-dihydro-7H-pyrrolo[2,3-c]pyridin-7-one (20.0 mg, 35.3 µmol, 28.5% yield, 100% purity) was obtained as a white solid. Confirmed by H NMR and LCMS.
¹H NMR: (DMSO-*d*₆, 400 MHz)
δ 12.44 (s, 1H), 8.35 (s, 1H), 7.71 (s, 1H), 7.48-7.53 (m, 1H), 7.44 (s, 1H), 7.36 (d, *J* = 8.4 Hz, 1H), 7.25 (d, *J* = 8.0 Hz, 1H), 6.28 (s, 1H), 3.65 (t, *J = 7.2* Hz, 2H), 3.59-3.63 (m, 2H), 3.30-3.33 (m, 2H), 3.27 (s, 3H), 2.90-2.97 (m, 2H), 2.66-2.73 (m, 2H), 2.23-2.36 (m, 2H), 2.18 (d, *J* = 8.4 Hz, 1H), 1.93-2.02 (m, 2H), 1.71-1.80 (m, 1H), 1.49-1.58 (m, 2H), 1.36-1.48 (m, 2H), 1.19-1.35 (m, 2H)
LCMS: m/z = 567.3 (M+H)⁺, Rt = 1.370 min

### Example 125: Synthesis of compound T125

### 1. General steps for preparation of 2-((2-oxa-8-azaspiro[4.5]decan-8-yl)methyl)-6-(3-(1-(4-methyl-4H-1,2,4-triazol-3-yl)cyclobutyl)phenyl)-4-(triffuor omethyl)-1,6-dihydro-7H-pyrrolo[2,3-c]pyridin-7-one (compound T125)

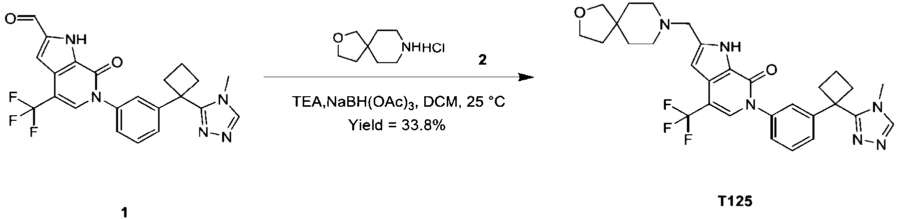

A mixture of 6-[3-[1-(4-methyl-1,2,4-triazol-3-yl)cyclobutyl]phenyl]-7-oxo-4-(trifluoromethyl)-1H-pyrrolo[2,3-c]pyridine-2-car baldehyde (50.0 mg, 113 µmol) , 2-oxa-8-azaspiro[4.5]decane (30.1 mg, 169 µmol, HCl) , TEA (34.3 mg, 339 µmol, 47.3 µL) in DCM (1.20 mL) was degassed and purged with N₂ for 3 times, and then the mixture was stirred at 25 °C for 30 min under N₂ atmosphere. then NaBH(OAc)₃ (48.0 mg, 226 µmol) was added and stirred for 30 min. LCMS showed desired mass was detected. The reaction mixture was quenched by addition NaHCO₃ 5.00 mL at 25 °C, and then diluted with DCM 5.00 mL and extracted with DCM (10.0 mL * 3). The combined organic layers were washed with brine 20.0 mL, dried over Na₂SO₄, filtered and concentrated under reduced pressure to give a residue. The residue was purified by prep-HPLC (column: Welch Xtimate C18 40*200mm 7um;mobile phase: [water(HCl)-ACN];gradient:0%-38% B over 30 min). To afford Compound 6-[3-[1-(4-methyl-1,2,4-triazol-3-yl)cyclobutyl]phenyl]-2-(2-oxa-8-azaspiro[4.5]decan-8-ylmethyl)-4-(trifluoromet hyl)-1H-pyrrolo[2,3-c]pyridin-7-one (23.0 mg, 38.3 µmol, 33.8% yield, 94.5% purity) was obtained as a white solid which confirmed by LCMS and HNMR.
¹H NMR: (400 MHz, DMSO-*d*₆):
δ (ppm) 12.72 (br d, *J* = 3.2 Hz, 1H), 11.19 (br s, 1H), 9.29 (s, 1H), 7.83 (d, *J* = 1.2 Hz, 1H), 7.53-7.62 (m, 1H), 7.51 (s, 1H), 7.35-7.46 (m, 2H), 6.74 (s, 1H), 4.47 (br d, *J* = 6.4 Hz, 2H), 3.70-3.78 (m, 2H), 3.56 (s, 1H), 3.41 (s, 3H), 3.40 (br s, 1H), 3.31 (br t, *J =* 11.6 Hz, 2H), 2.86-3.05 (m, 4H), 2.69-2.83 (m, 2H), 1.97-2.12 (m, 2H), 1.83-1.95 (m, 2H), 1.65-1.82 (m, 4H)
LCMS: m/z = 567.0 (M+H)⁺, Rt = 0.981 min

### Example 126: Synthesis of compound T126

### 1. General steps for preparation of 6-[3-[3,3-difluoro-1-[(4-methyl-1,2,4-triazol-3-yl)methyl]cyclobutyl]phenyl]-2-[[(3S)-3-methyl-1-piperidyl]methyl 1-1-(p-tolylsulfonyl)-4-(trifluoromethyl)pyrrolo[2,3-c]pyridin-7-one

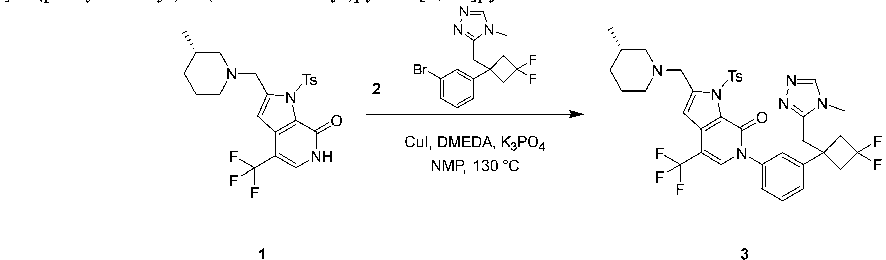

A mixture of 2-[[(3S)-3-methyl-1-piperidyl]methyl]-1-(p-tolylsulfonyl)-4-(trifluoromethyl)-6H-pyrrolo[2,3-c]pyridin-7-one (70.0 mg, 149 µmol), 3-[[1-(3-bromophenyl)-3,3-difluoro-cyclobutyl]methyl]-4-methyl-1,2,4-triazole (57.0 mg, 166 µmol), CuI (63.4 mg, 333 µmol), K₃PO₄ (106 mg, 499 µmol) in NMP (0.50 mL) was added DMEDA (14.6 mg, 166 µmol, 17.9 µL) was degassed and purged with N₂ for 3 times, and then the mixture was stirred at 130 °C for 2 hrs. LCMS showed desired compound was detected. The reaction mixture was diluted with H₂O (30.0 mL), extracted with ethyl acetate (20.0 mL * 3), the combined organic layers were washed with H₂O (20.0 mL * 3), dried over Na₂SO₄, filtered and concentrated under reduced pressure to give a residue. The residue was purified by column chromatography (SiO₂, Dichloromethane/ Methanol=3.00% to 18.0%), (Plate 1, Dichloromethane/ Methanol = 10/ 1, R_{f} (product) = 0.31). Compound 6-[3-[3,3-difluoro-1-[(4-methyl-1,2,4-triazol-3-yl)methyl]cyclobutyl]phenyl]-2-[[(3S)-3-methyl-1-piperidyl]methyl ]-1-(p-tolylsulfonyl)-4-(trifluoromethyl)pyrrolo[2,3-c]pyridin-7-one (55.0 mg, 65.8 µmol, 39.5% yield, 87.2% purity) was obtained as a colorless oil. Confirmed by LCMS.
LCMS: m/z = 729.1 (M+H)⁺, Rt = 1.268 min

### 2. General steps for preparation of 6-[3-[3,3-difluoro-1-[(4-methyl-1,2,4-triazol-3-yl)methyl]cyclobutyl]phenyl]-2-[[(3S)-3-methyl-1-piperidyl]methyl ]-4-(trifluoromethyl)-1H-pyrrolo[2,3-c]pyridin-7-one (compound T126)

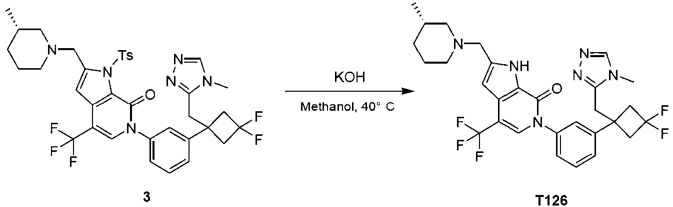

A mixture of 6-[3-[3,3-difluoro-1-[(4-methyl-1,2,4-triazol-3-yl)methyl]cyclobutyl]phenyl]-2-[[(3S)-3-methyl-1-piperidyl]methyl ]-1-(p-tolylsulfonyl)-4-(trifluoromethyl)pyrrolo[2,3-c]pyridin-7-one (55.0 mg, 75.4 µmol), KOH (84.6 mg, 1.51 mmol) in MeOH (0.50 mL) was degassed and purged with N₂ for 3 times, and then the mixture was stirred at 40 °C for 1 hr. LCMS showed desired compound was detected. The reaction mixture was diluted with brine (30.0 mL), extracted with ethyl acetate (20.0 mL * 3), the combined organic layers were washed with aqueous KOH (20.0 mL * 2), dried over Na₂SO₄, filtered and concentrated under reduce pressure to give a residue. The residue was purified by Prep-HPLC (column: Xtimate C18 150 * 40 mm * 10 um; mobile phase: [water (FA)-ACN]; gradient: 0%-40.0% B over 25 mins). Compound 6-[3-[3,3-difluoro-1-[(4-methyl-1,2,4-triazol-3-yl)methyl]cyclobutyl]phenyl]-2-[[(3S)-3-methyl-1-piperidyl]methyl ]-4-(trifluoromethyl)-1H-pyrrolo[2,3-c]pyridin-7-one (15.7 mg, 27.0 µmol, 35.8% yield, 99.1% purity) was obtained as a colorless oil. Confirmed by H NMR and LCMS.
¹H NMR: (DMSO-*d*₆, 400 MHz)
δ 12.21-12.69 (m, 1H), 8.15 (s, 1H), 7.56 (d, *J* = 1.2 Hz, 1H), 7.32-7.45 (m, 2H), 7.13 (s, 1H), 6.99 (d, *J* = 7.6 Hz, 1H), 6.27 (s, 1H), 3.60 (s, 3H), 3.29 (s, 1H), 3.24 (s, 2H), 3.04 (q, *J* = 14.4 Hz, 3H), 2.81 (s, 3H), 2.75 (d, *J* = 6.0 Hz, 2H), 1.89 (t, *J* = 10.4 Hz, 1H), 1.63 (s, 1H), 1.55-1.61 (m, 3H), 1.45 (d, *J* = 12.0 Hz, 1H), 0.81 (d, *J* = 5.6 Hz, 3H)
LCMS: m/z =575.3 (M+H)⁺, Rt = 1.443 min

### Example 127: Synthesis of compound T127

The synthesis of compound T127 is described in the Example 121.

### Example 128: Synthesis of compound T128

The synthesis of compound T128 is described in the Example 121.

### Example 129: Synthesis of compound T129

### 1. General steps for preparation of methyl 2-(3-bromophenyl)-3-hydroxy-2-(hydroxymethyl)propanoate

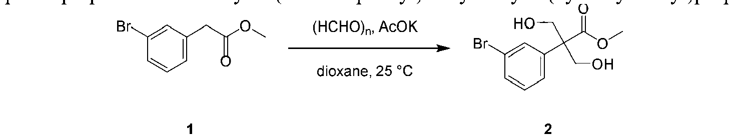

To a solution of methyl 2-(3-bromophenyl)acetate (15.0 g, 65.4 mmol) in dioxane (150 mL) was added AcOK (2.57 g, 26.1 mmol) and (HCHO)ₙ (6.09 g, 65.4 mmol, 5.58 mL) slowly. After addition, the mixture was stirred at 25 °C for 8 hrs. LCMS showed desired compound was detected. The reaction mixture was filtered and diluted with HCl (1 N, 20.0 mL) adjust pH = 7, extracted with Ethyl acetate (150 mL * 3), the combined organic layers were washed with H₂O (150 mL * 3), dried over Na₂SO₄, filtered and concentrated under reduced pressure to give a residue. The residue was purified by column chromatography (SiO₂, DCM/ MeOH = 0% to 4.00%), (Plate 1, DCM/ MeOH = 20/ 1, R_{f} (product) = 0.35). Compound methyl 2-(3-bromophenyl)-3-hydroxy-2-(hydroxymethyl)propanoate (15.7 g, crude) was obtained as a colorless oil. Confirmed by H NMR and LCMS.
¹H NMR: (DMSO-*d*₆, 400 MHz)
δ 7.43-7.49 (m, 1H), 7.17-7.37 (m, 3H), 4.87 (t, *J* = 4.8 Hz, 1H), 4.45-4.67 (m, 1H), 3.90-4.00 (m, 4H), 3.56-3.65 (m, 3H)
LCMS: m/z = 290.4 (M+H)⁺, Rt = 1.015 min

### 2. General steps for preparation of methyl 3-(3-bromophenyl)oxetane-3-carboxylate

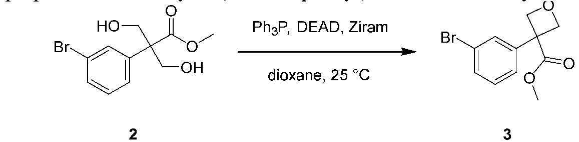

To a solution of methyl 2-(3-bromophenyl)-3-hydroxy-2-(hydroxymethyl)propanoate (15.7 g, 54.3 mmol) in dioxane (160 mL) was added dropwise PPh₃ (28.4 g, 108 mmol), Ziram (24.9 g, 81.4 mmol), then add DEAD (18.9 g, 108 mmol, 19.7 mL) at 25 °C. The resulting mixture was stirred at 30 °C for 12 hrs. TLC indicated one major new spot with lower polarity was detected. The reaction mixture was diluted with Ethyl acetate (30.0 mL), filtered and washed with H₂O (20.0 mL * 3), dried over Na₂SO₄, filtered and concentrated under reduced pressure to give a residue. The residue was purified by column chromatography (SiO₂, Petroleum ether/ Ethyl acetate = 5.00% to 8.00%), (Plate 1, Petroleum ether/ Ethyl acetate = *5*/ 1, R_{f} (product) = 0.42). Compound methyl 3-(3-bromophenyl)oxetane-3-carboxylate (1.50 g, 4.14 mmol, 7.62% yield, 74.8% purity) was obtained as a colorless oil. Confirmed by H NMR.
¹H NMR: (CDCl₃, 400 MHz)
δ 7.44-7.48 (m, 1H), 7.40 (t, *J =* 1.6Hz, 1H), 7.26 (s, 1H), 7.15-7.20 (m, 1H), 5.24 (d, *J =* 6.4 Hz, 2H), 4.98 (d, *J= 6.4* Hz, 2H), 3.77 (s, 3H)

### 3. General steps for preparation of 3-(3-bromophenyl)oxetane-3-carbohydrazide

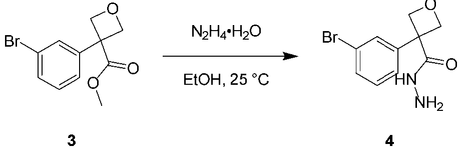

To a solution of methyl 3-(3-bromophenyl)oxetane-3-carboxylate (1.00 g, 3.69 mmol) in EtOH (10.0 mL) was added dropwise N₂H₄·H₂O (3.10 g, 49.4 mmol, 3.00 mL, 80.0% purity). After addition, the mixture was stirred at 25 °C for 3 hrs. LCMS showed desired compound was detected. The reaction mixture was diluted with H₂O (30.0 mL), extracted with Ethyl acetate (20.0 mL * 3), the combined organic layers were washed with H₂O (20.0 mL * 3), dried over Na₂SO₄, filtered and concentrated under reduced pressure to give a residue. Compound 3-(3-bromophenyl)oxetane-3-carbohydrazide (970 mg, crude) was obtained as a white solid. Confirmed by H NMR and LCMS.
¹H NMR: (CDCl₃, 400 MHz)
δ 7.48-7.53 (m, 1H), 7.35 (t, *J = 1.6* Hz, 1H), 7.31 (t, *J =* 8.0 Hz, 1H), 7.10-7.17 (m, 1H), 5.25 (d, *J=* 5.6 Hz, 2H), 4.96 (d, *J = 6.0* Hz, 2H)
LCMS: m/z = 272.5 (M+H)⁺, Rt = 0.825 min

### 4. General steps for preparation of 1-[[3-(3-bromophenyl)oxetane-3-carbonyl]aminol-3-methyl-thiourea

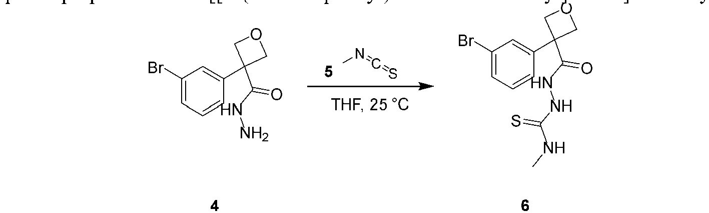

A mixture of 3-(3-bromophenyl)oxetane-3-carbohydrazide (970 mg, 3.58 mmol), methylimino(thioxo)methane (523 mg, 7.16 mmol, 489 µL) in THF (10.0 mL) was degassed and purged with N₂ for 3 times, and then the mixture was stirred at 25 °C for 8 hrs under N₂ atmosphere. LCMS showed desired compound was detected. The reaction mixture was diluted with H₂O (30.0 mL), extracted with Ethyl acetate (20.0 mL * 3), the combined organic layers were washed with H₂O (20.0 mL * 3), dried over Na₂SO₄, filtered and concentrated under reduced pressure to give a residue. Compound 1-[[3-(3-bromophenyl)oxetane-3-carbonyl]amino]-3-methyl-thiourea (1.18 g, crude) was obtained as a white solid.

### 5. General steps for preparation of 5-[3-(3-bromophenyl)oxetan-3-yl]-4-methyl-1,2,4-triazole-3-thiol

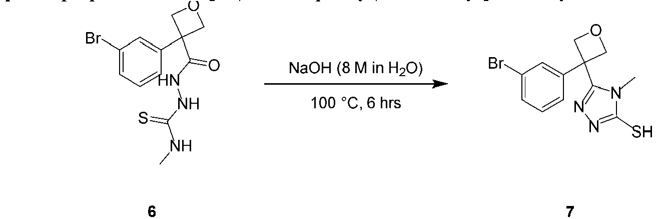

1-[[3-(3-bromophenyl)oxetane-3-carbonyl]amino]-3-methyl-thiourea (1.18 g, 3.43 mmol) was added to NaOH (8 M, 10.0 mL) solution, stirred at 100 °C for 6 hrs. LCMS showed desired compound was detected. The reaction mixture was diluted with water, then the pH value of the solution was adjust to 1 with HCl (1 M, 25.0 mL) and extracted with Ethyl acetate (40.0 mL * 3), the combined organic layers were washed with H₂O (50.0 mL * 3), dried over Na₂SO₄, filtered and concentrated under reduced pressure to give a residue. Compound 5-[3-(3-bromophenyl)oxetan-3-yl]-4-methyl-1,2,4-triazole-3-thiol (1.20 g, crude) was obtained as a white solid. Confirmed by LCMS.
LCMS: m/z = 327.8 (M+H)⁺, Rt = 1.192 min

### 6. General steps for preparation of 3-[3-(3-bromophenyl)oxetan-3-yl]-4-methyl-1,2,4-triazole

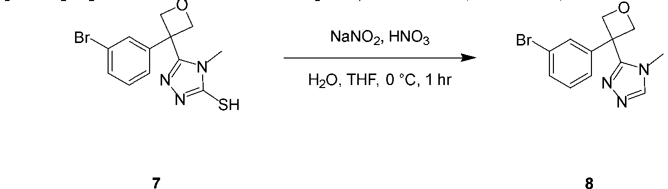

To a solution of 5-[3-(3-bromophenyl)oxetan-3-yl]-4-methyl-1,2,4-triazole-3-thiol (1.12 g, 3.43 mmol) in THF (5.00 mL) and H₂O (5.00 mL) was added NaNO₂ (2.37 g, 34.3 mmol) and HNO₃ (3.49 g, 36.0 mmol, 2.50 mL, 65.0% purity) slowly at 0 °C. The resulting mixture was stirred at 0 °C for 1 hr. LCMS showed desired compound was detected. The mixture was basified by saturated aqueous sodium bicarbonate solution (20.0 mL) then extracted with Ethyl acetate (60.0 mL * 3). The combined organic layers were dried over Na₂SO₄, filtered and concentrated under reduced pressure to give a residue. The residue was purified by column chromatography (SiO₂, DCM/ MeOH = 0% to 3.00%), (Plate 1, DCM/ MeOH = 10/ 1, R_{f} (product) = 0.54). Compound 3-[3-(3-bromophenyl)oxetan-3-yl]-4-methyl-1,2,4-triazole (571 mg, 1.91 mmol, 55.5% yield, 98.3% purity) was obtained as a yellow oil. Confirmed by H NMR and LCMS.
¹H NMR: (DMSO-*d*₆, 400 MHz)
δ 7.56 (dd, *J* = 8.0, 0.8 Hz, 1H), 7.47 (s, 1H), 7.39 (t, *J* = 8.0 Hz, 1H), 7.23 (br d, *J* = 8.0 Hz, 1H), 5.34 (d, *J* = 6.4 Hz, 2H), 5.05 (d, *J* = 6.4 Hz, 2H), 3.21 (s, 3H)
LCMS: m/z = 295.8 (M+H)⁺, Rt = 1.153 min

### 7. General steps for preparation of (S)-6-(3-(3-(4-methyl-4H-1,2,4-triazol-3-yl)oxetan-3-yl)phenyl)-2-((3-methylpiperidin-1-yl)methyl)-1-tosyl-4-(trifl uoromethyl)-1,6-dihydro-7H-pyrrolo[2,3-c]pyridin-7-one

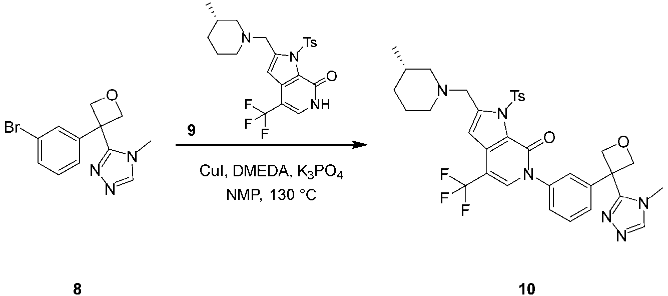

A mixture of 3-[3-(3-bromophenyl)oxetan-3-yl]-4-methyl-1,2,4-triazole (100 mg, 339 µmol), (S)-2-((3-methylpiperidin-1-yl)methyl)-1-tosyl-4-(trifluoromethyl)-1,6-dihydro-7H-pyrrolo[2,3-c]pyridin-7-one (150 mg, 320 µmol), CuI (61.1 mg, 320 µmol), K₃PO₄ (204 mg, 962 µmol) and DMEDA (56.5 mg, 641 µmol, 69.0 µL) in NMP (1.00 mL) was degassed and purged with N₂ for 3 times, and then the mixture was stirred at 130 °C for 3 hrs under N₂ atmosphere. TLC indicated ~0% of Reactant 1 was remained, and one major new spot with larger polarity was detected. The reaction mixture was diluted with H₂O (30.0 mL), extracted with Ethyl acetate (20.0 mL * 3), the combined organic layers were washed with H₂O (20.0 mL * 3), dried over Na₂SO₄, filtered and concentrated under reduced pressure to give a residue. The residue was purified by column chromatography (SiO₂, DCM/ MeOH = 3.00% to 18.0%), (Plate 1, DCM/ MeOH = 10/ 1, R_{f} (product) = 0.35). Compound (S)-6-(3-(3-(4-methyl-4H-1,2,4-triazol-3-yl)oxetan-3-yl)phenyl)-2-((3-methylpiperidin-1-yl)methyl)-1-tosyl-4-(trifl uoromethyl)-1,6-dihydro-7H-pyrrolo[2,3-c]pyridin-7-one (40.0 mg, 46.4 µmol, 14.4% yield, 79.0% purity) was obtained as a colorless oil. Confirmed by LCMS.
LCMS: m/z = 681.1 (M+H)⁺, Rt = 1.303 min

### 8. General steps for preparation of (S)-6-(3-(3-(4-methyl-4H-1,2,4-triazol-3-yl)oxetan-3-yl)phenyl)-2-((3-methylpiperidin-1-yl)methyl)-4-(trifluorome thyl)-1,6-dihydro-7H-pyrrolo[2,3-c]pyridin-7-one (compound T129)

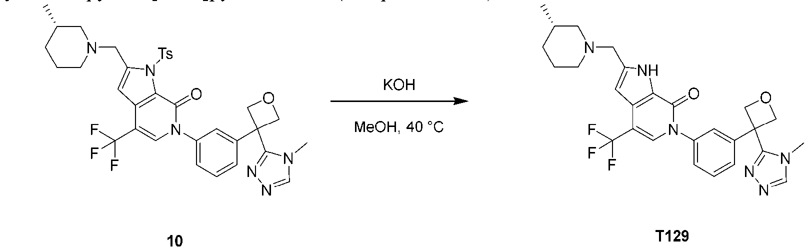

A mixture of (S)-6-(3-(3-(4-methyl-4H-1,2,4-triazol-3-yl)oxetan-3-yl)phenyl)-2-((3-methylpiperidin-1-yl)methyl)-1-tosyl-4-(trifl uoromethyl)-1,6-dihydro-7H-pyrrolo[2,3-c]pyridin-7-one (40.0 mg, 58.7 µmol), KOH (65.9 mg, 1.18 mmol) in MeOH (1.00 mL) was degassed and purged with N₂ for 3 times, and then the mixture was stirred at 40 °C for 1 hr under N₂ atmosphere. LCMS showed desired compound was detected. The reaction mixture was diluted with brine (30.0 mL), extracted with Ethyl acetate (20.0 mL * 3), the combined organic layers were washed with aqueous KOH (20.0 mL * 2), dried over Na₂SO₄, filtered and concentrated under reduced pressure to give a residue. The residue was purified by Prep-HPLC (column: Xtimate C18 150 * 40 mm * 10 um; mobile phase: [water (NH₃.H₂O+NH₄HCO₃)-ACN]; gradient: 28.0%-68.0% B over 32 mins). Compound (S)-6-(3-(3-(4-methyl-4H-1,2,4-triazol-3-yl)oxetan-3-yl)phenyl)-2-((3-methylpiperidin-1-yl)methyl)-4-(trifluorome thyl)-1,6-dihydro-7H-pyrrolo[2,3-c]pyridin-7-one (10.0 mg, 18.4 µmol, 31.3% yield, 98.1% purity) was obtained as a white solid. Confirmed by H NMR and LCMS.
¹H NMR: (DMSO-*d*₆, 400 MHz)
δ 12.25-12.59 (m, 1H), 8.47 (s, 1H), 7.75 (d, *J=* 1.2 Hz, 1H), 7.55-7.61 (m, 1H), 7.44-7.49 (m, 2H), 7.33 (d, *J* = 7.6 Hz, 1H), 6.27 (s, 1H), 5.39 (d, *J* = 6.4 Hz, 2H), 5.13 (d, *J* = 6.4 Hz, 2H), 3.60 (s, 2H), 3.30 (s, 3H), 2.72-2.79 (m, 2H), 1.84-1.95 (m, 1H), 1.53-1.64 (m, 4H), 1.19-1.52 (m, 2H), 0.81 (br d, *J* = 5.6 Hz, 3H)
LCMS: m/z =527.3 (M+H)⁺, Rt = 1.290 min

### Examples 130 and 131: Synthesis of compounds T130& T131

### 1. General steps for preparation of 3-(3-bromophenyl)-3-(4-methyl-1,2,4-triazol-3-yl)cyclobutano

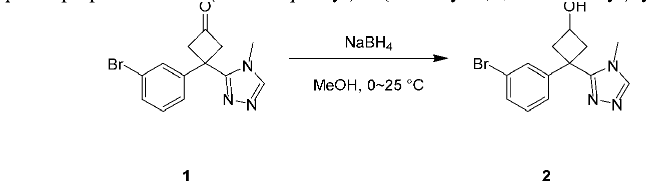

To a solution of 3-(3-bromophenyl)-3-(4-methyl-1,2,4-triazol-3-yl)cyclobutanone (1.50 g, 4.90 mmol) in MeOH (30.0 mL) was added NaBH₄ (370 mg, 9.80 mmol) slowly at 0 °C. After addition, the mixture was stirred at 25 °C for 2 hrs. LCMS showed desired compound was detected. The reaction mixture was quenched by saturated aqueous NH₄Cl (20.0 mL) slowly. The mixture was extracted with ethyl acetate (30.0 mL*3), dried over Na₂SO₄. The organic phase was concentrated in vacuum. The residue was purified by column chromatography (SiO₂, Dichloromethane/ Methanol = 0% to 14.0%), (Plate 1, Dichloromethane/ Methanol = 10/ 1, R_{f} (product) = 0.18). Compound 3-(3-bromophenyl)-3-(4-methyl-1,2,4-triazol-3-yl)cyclobutanol (1.17 g, 3.78 mmol, 77.1% yield, 99.5% purity) was obtained as a white solid. Confirmed by H NMR and LCMS.
¹H NMR: (CDCl₃, 400 MHz)
δ 7.96-8.08 (m, 1H), 7.36-7.48 (m, 2H), 7.09-7.23 (m, 2H), 4.43-4.59 (m, 1H), 3.34-3.39 (m, 1H), 3.18-3.22 (m, 3H), 3.07-3.15 (m, 1H), 2.95-3.03 (m, 1H), 2.57-2.63 (m, 1H)
LCMS: m/z = 309.5 (M+H)⁺, Rt = 0.942 min

### 2. General steps for preparation of 3-[1-(3-bromophenyl)-3-(trifluoromethoxy)cyclobutyl]-4-methyl-1,2,4-triazole

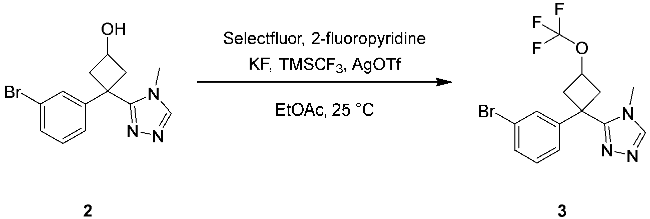

To a mixture of AgOTf (1.67 g, 6.49 mmol) and Selectfluor (862 mg, 2.43 mmol) and KF (377 mg, 6.49 mmol, 152 µL) was added a solution of 3-(3-bromophenyl)-3-(4-methyl-1,2,4-triazol-3-yl)cyclobutanol (500 mg, 1.62 mmol) in ethyl acetate (10.0 mL). 2-fluoropyridine (630 mg, 6.49 mmol, 557 µL) and TMSCF₃ (576 mg, 4.06 mmol) was added dropwise to the mixture at 25 °C. After addition, the mixture was stirred at 25 °C for 12 hrs. LCMS showed desired compound was detected. The reaction mixture was diluted with H₂O (30.0 mL), extracted with ethyl acetate (20.0 mL * 3), dried over Na₂SO₄, filtered and concentrated under reduced pressure to give a residue. The residue was purified by column chromatography (SiO₂, Dichloromethane/ Methanol = 0% to 5.00%), (Plate 1, Dichloromethane/ Methanol = 10/ 1, R_{f} (product) = 0.48). Compound 3-[1-(3-bromophenyl)-3-(trifluoromethoxy)cyclobutyl]-4-methyl-1,2,4-triazole (300 mg, 610 µmol, 37.6% yield, 76.6% purity) was obtained as a colorless oil. Confirmed by H NMR and LCMS.
¹H NMR: (DMSO-*d*₆, 400 MHz)
*δ* 8.38-8.50 (m, 1H), 7.47-7.51 (m, 1H), 7.37 (d, *J =* 6.8 Hz, 1H), 7.32-7.36 (m, 1H), 7.21 (d, *J =* 7.6 Hz, 1H), 4.67-5.09 (m, 1H), 3.39-3.52 (m, 2H), 3.20 (d, *J=* 6.0 Hz, 3H), 3.17 (d, *J=* 5.2 Hz, 1H), 2.83-2.90 (m, 1H)

### 3. General steps for preparation of 2-[[(3S)-3-methyl-1-piperidyl]methyl]-6-[3-[1-(4-methyl-1,2,4-triazol-3-yl)-3-(trifluoromethoxy)cyclobutyl]phenyl ]-1-(p-tolylsulfonyl)-4-(trifluoromethyl)pyrrolo[2,3-c]pyridin-7-one

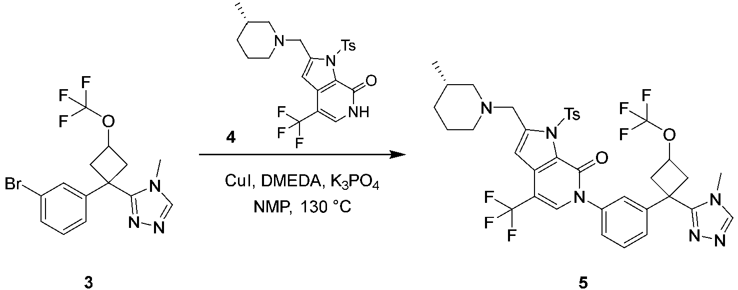

To a solution of 3-[1-(3-bromophenyl)-3-(trifluoromethoxy)cyclobutyl]-4-methyl-1,2,4-triazole (150 mg, 398 µmol), 2-[[(3S)-3-methyl-1-piperidyl]methyl]-1-(p-tolylsulfonyl)-4-(trifluoromethyl)-6H-pyrrolo[2,3-c]pyridin-7-one (149 mg, 319 µmol), CuI (151 mg, 797 µmol), K₃PO₄ (253 mg, 1.20 mmol) in NMP (0.70 mL) was added dropwise DMEDA (35.1 mg, 398 µmol, 42.9 µL). After addition, the mixture was stirred at 130 °C for 2 hrs. LCMS showed desired compound was detected. The reaction mixture was diluted with H₂O (30.0 mL), extracted with ethyl acetate (20.0 mL * 3), the combined organic layers were washed with H₂O (20.0 mL * 3), dried over Na₂SO₄, filtered and concentrated under reduced pressure to give a residue. Compound 2-[[(3S)-3-methyl-1-piperidyl]methyl]-6-[3-[1-(4-methyl-1,2,4-triazol-3-yl)-3-(trifluoromethoxy)cyclobutyl]phenyl ]-1-(p-tolylsulfonyl)-4-(trifluoromethyl)pyrrolo[2,3-c]pyridin-7-one (100 mg, crude) was obtained as a colorless oil. Confirmed by LCMS.
LCMS: m/z = 763.0 (M+H)⁺, Rt = 1.342 min

### 4. General steps for preparation of 6-(3-((1s,3R)-1-(4-methyl-4H-1,2,4-triazol-3-yl)-3-(trifluoromethoxy)cyclobutyl)phenyl)-2-(((S)-3-methylpiperidin -1-yl)methyl)-4-(trifluoromethyl)-1,6-dihydro-7H-pyrrolo[2,3-c]pyridin-7-one (compound T130) and 6-(3-((1r,3S)-1-(4-methyl-4H-1,2,4-triazol-3-yl)-3-(trifluoromethoxy)cyclobutyl)phenyl)-2-(((S)-3-methylpiperidin -1-yl)methyl)-4-(trifluoromethyl)-1,6-dihydro-7H-pyrrolo[2,3-c]pyridin-7-one (compound T131)

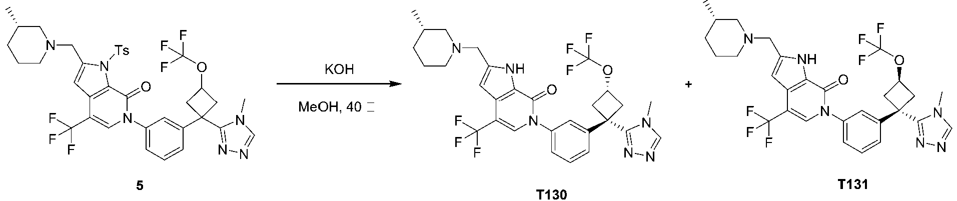

A mixture of 2-[[(3S)-3-methyl-1-piperidyl]methyl]-6-[3-[1-(4-methyl-1,2,4-triazol-3-yl)-3-(trifluoromethoxy)cyclobutyl]phenyl ]-1-(p-tolylsulfonyl)-4-(trifluoromethyl)pyrrolo[2,3-c]pyridin-7-one (100 mg, 131 µmol), KOH (147 mg, 2.62 mmol) in MeOH (1.00 mL) was degassed and purged with N₂ for 3 times, and then the mixture was stirred at 40 °C for 1 hr. LCMS showed desired compound was detected. The reaction mixture was extracted with ethyl acetate (20.0 mL * 3), the combined organic layers were washed with aqueous KOH (20.0 mL * 3), dried over Na₂SO₄, filtered and concentrated under reduced pressure to give a residue. The residue was purified by Prep-HPLC (column: Xtimate C18 150 * 40 mm * 10 um; mobile phase: [water (NH₃H₂O+NH₄HCO₃)-ACN]; gradient: 36.0%-76.0% B over 32 mins). Compound 6-(3-((1s,3R)-1-(4-methyl-4H-1,2,4-triazol-3-yl)-3-(trifluoromethoxy)cyclobutyl)phenyl)-2-(((S)-3-methylpiperidin -1-yl)methyl)-4-(trifluoromethyl)-1,6-dihydro-7H-pyrrolo[2,3-c]pyridin-7-one (6.44 mg, 10.3 µmol, 7.91% yield, 98.0% purity) was obtained as a white solid. Confirmed by H NMR and LCMS.
¹H NMR: (DMSO-*d*₆, 400 MHz)
δ 12.18-12.65 (m, 1H), 8.35 (s, 1H), 7.75 (d, *J* = 1.2 Hz, 1H), 7.61 (s, 1H), 7.49-7.55 (m, 1H), 7.41 (d, *J* = 8.4 Hz, 1H), 7.31 (d, *J* = 8.0 Hz, 1H), 6.27 (s, 1H), 5.02 (q, *J* = 7.2 Hz, 1H), 3.60 (s, 2H), 3.28 (s, 3H), 3.14-3.21 (m, 4H), 2.72-2.79 (m, 2H), 1.89 (t, *J* = 10.4 Hz, 1H), 1.54-1.65 (m, 4H), 1.44 (d, *J* = 12.4 Hz, 1H), 0.81 (d, *J* = 5.6 Hz, 3H), 0.78 (s, 1H)
LCMS: m/z =609.2 (M+H)⁺, Rt = 1.797 min

Compound 6-(3-((1r,3S)-1-(4-methyl-4H-1,2,4-triazol-3-yl)-3-(trifluoromethoxy)cyclobutyl)phenyl)-2-(((S)-3-methylpiperidin -1-yl)methyl)-4-(trifluoromethyl)-1,6-dihydro-7H-pyrrolo[2,3-c]pyridin-7-one (5.23 mg, 8.42 µmol, 6.42% yield, 98.0% purity) was obtained as a white solid. Confirmed by H NMR and LCMS.
¹H NMR: (DMSO-*d*₆, 400 MHz)
δ 12.29-12.58 (m, 1H), 8.43 (s, 1H), 7.73 (s, 1H), 7.49-7.54 (m, 1H), 7.47 (s, 1H), 7.40 (d, *J* = 8.0 Hz, 1H), 7.20 (d, *J =* 8.0 Hz, 1H), 6.27 (s, 1H), 4.76 (q, *J* = 7.2 Hz, 1H), 3.60 (s, 2H), 3.49-3.53 (m, 2H), 3.29 (s, 3H), 2.89-2.94 (m, 2H), 2.71-2.78 (m, 2H), 1.88 (t, *J=* 10.4 Hz, 1H), 1.54-1.64 (m, 4H), 1.44 (d, *J=* 12.4 Hz, 1H), 0.81 (d, *J=* 5.2 Hz, 3H), 0.71-0.79 (m, 1H)
LCMS: m/z =609.2 (M+H)⁺, Rt = 1.873 min

### Examples 132 and 133: Synthesis of compounds T132& T133

### 1. General steps for preparation of 4-cyclopropyl-6-[3-[3-hydroxy-1-(4-methyl-1,2,4-triazol-3-yl)cyclobutyl]phenyl]-2-[[(3S)-3-methyl-1-piperidyl]m ethyl]-1-(p-tolylsulfonyl)pyrrolo[2,3-c]pyridin-7-one

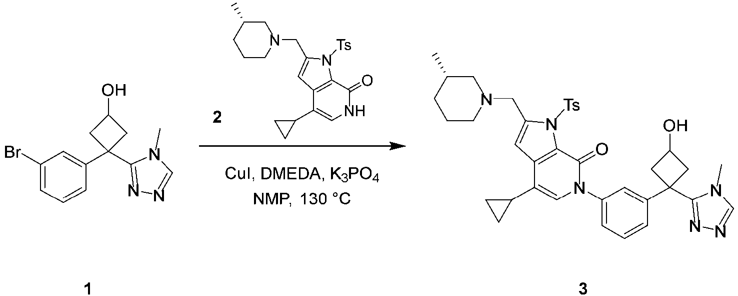

To a solution of 3-(3-bromophenyl)-3-(4-methyl-1,2,4-triazol-3-yl)cyclobutanol (120 mg, 389 µmol) and 4-cyclopropyl-2-[[(3S)-3-methyl-1-piperidyl]methyl]-1-(p-tolylsulfonyl)-6H-pyrrolo[2,3-c]pyridin-7-one (154 mg, 350 µmol) in NMP (5.00 mL) was added K₃PO₄ (248 mg, 1.17 mmol), DMEDA (34.3 mg, 389 µmol) and CuI (74.2 mg, 389 µmol). The suspension was degassed under vacuum and purged with N₂ several times. The mixture was stirred under N₂ at 130 °C for 1 hr. TLC (DCM/ MeOH = 10/ 1, product 1 R_{f} = 0.35) indicated new spot formed. The reaction mixture was poured into saturated aqueous NH₄Cl (10.0 mL) slowly. The mixture was extracted with EtOAc (10.0 mL*2). The organic phase was washed with brine (20.0 mL*2), dried over Na₂SO₄ and concentrated in vacuum. The crude product was purified by column chromatography (SiO₂, DCM/ MeOH = 1/ 0 to 15/ 1). To afford 4-cyclopropyl-6-[3-[3-hydroxy-1-(4-methyl-1,2,4-triazol-3-yl)cyclobutyl]phenyl]-2-[[(3S)-3-methyl-1-piperidyl]m ethyl]-1-(p-tolylsulfonyl)pyrrolo[2,3-c]pyridin-7-one (210 mg, 298 µmol, 76.5% yield, 94.7% purity) as a yellow solid which was confirmed by H NMR and LCMS.
¹H NMR: (400 MHz, CDCl₃)
δ 8.48 (d, *J* = 7.6 Hz, 2H), 7.29-7.46 (m, 2H), 7.14-7.26 (m, 4H), 7.03 (s, 1H), 6.78 (s, 1H), 6.59 (s, 1H), 4.36-4.63 (m, 1H), 3.85-4.04 (m, 2H), 3.39-3.50 (m, 1H), 3.34 (s, 3H), 3.04-3.26 (m, 2H), 2.83-3.04 (m, 3H), 2.61 (s, 2H), 2.41 (s, 3H), 1.92-2.00 (m, 1H), 1.66-1.84 (m, 5H), 0.81-0.95 (m, 6H), 0.52 (q, *J* = 5.2 Hz, 2H)
LCMS: m/z = 667.1 (M+H)⁺, Rt = 1.062 min

### 2. General steps for preparation of 4-cyclopropyl-6-[3-[3-hydroxy-1-(4-methyl-1,2,4-triazol-3-yl)cyclobutyl]phenyl]-2-[[(3S)-3-methyl-1-piperidyl]m ethyl]-1H-pyrrolo[2,3-c]pyridin-7-one

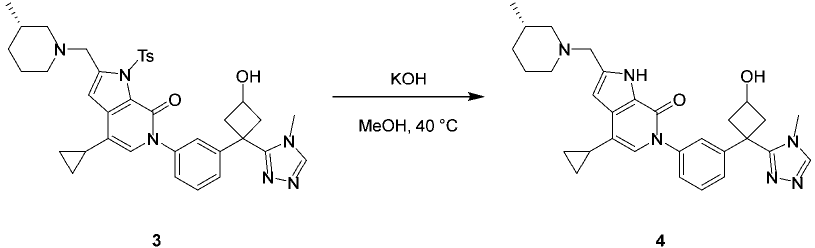

To a solution of 4-cyclopropyl-6-[3-[3-hydroxy-1-(4-methyl-1,2,4-triazol-3-yl)cyclobutyl]phenyl]-2-[[(3S)-3-methyl-1-piperidyl]m ethyl]-1-(p-tolylsulfonyl)pyrrolo[2,3-c]pyridin-7-one (210 mg, 314 µmol) in MeOH (4.00 mL) was added KOH (353 mg, 6.29 mmol). The mixture was stirred under N₂ at 40 °C for 2 hrs. LCMS showed desired mass was detected. The reaction mixture was diluted with brine (20.0 mL), extracted with EtOAc (20.0 mL*2). The organic phase washed with KOH (1 M, 20.0 mL), dried over Na₂SO₄ and concentrated in vacuum. The crude product was purified by Prep-HPLC (column: Xtimate C18 150*40 mm*10 um; mobile phase: [water (HCl) - ACN]; gradient: 0%-34.0% B over 36 mins). To afford 4-cyclopropyl-6-[3-[3-hydroxy-1-(4-methyl-1,2,4-triazol-3-yl)cyclobutyl]phenyl]-2-[[(3S)-3-methyl-1-piperidyl]m ethyl]-1H-pyrrolo[2,3-c]pyridin-7-one (160 mg, 312 µmol, 99.1% yield) as a yellow solid.

### 3. General steps for preparation of 4-cyclopropyl-6-(3-((1s,3R)-3-hydroxy-1-(4-methyl-4H-1,2,4-triazol-3-yl)cyclobutyl)phenyl)-2-(((S)-3-methylpipe ridin-1-yl)methyl)-1,6-dihydro-7H-pyrrolo[2,3-c]pyridin-7-one (compound T132) and 4-cyclopropyl-6-(3-((1r,3S)-3-hydroxy-1-(4-methyl-4H-1,2,4-triazol-3-yl)cyclobutyl)phenyl)-2-(((S)-3-methylpiper idin-1-yl)methyl)-1,6-dihydro-7H-pyrrolo[2,3-c]pyridin-7-one (compound T133)

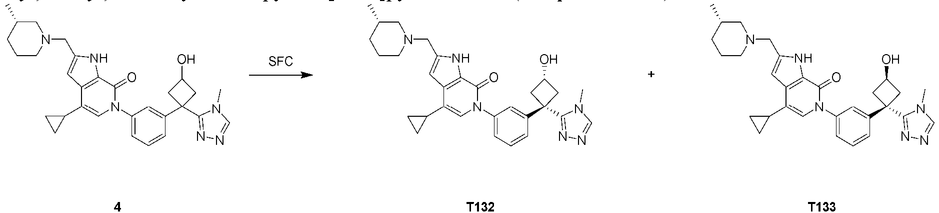

The crude product was purified by SFC (column: DAICEL CHIRALPAK AD (250 mm*30 mm, 10 um); mobile phase: [CO₂-EtOH (0.10% NH₃H₂O)]; B%: 35.0%, isocratic elution mode). To afford 4-cyclopropyl-6-(3-((1s,3R)-3-hydroxy-1-(4-methyl-4H-1,2,4-triazol-3-yl)cyclobutyl)phenyl)-2-(((S)-3-methylpipe ridin-1-yl)methyl)-1,6-dihydro-7H-pyrrolo[2,3-c]pyridin-7-one (18.9 mg, 36.8 µmol, 12.5% yield, 99.8% purity) as a yellow solid which was confirmed by H NMR and LCMS.
¹H NMR: (400MHz, DMSO-*d*₆)
δ 11.94 (s, 1H), 8.37 (s, 1H), 7.42-7.50 (m, 1H), 7.25 (d, *J=* 8.0 Hz, 1H), 7.19 (s, 1H), 7.12-7.18 (m, 1H), 6.77 (s, 1H), 6.32 (s, 1H), 5.30 (br d, *J* = 6.8 Hz, 1H), 4.04 (sxt, *J* = 7.2 Hz, 1H), 3.57 (s, 2H), 3.31 (s, 3H), 3.27 (d, *J* = 2.8 Hz, 2H), 2.73-2.83 (m, 2H), 2.43-2.48 (m, 2H), 1.84-1.93 (m, 2H), 1.55-1.66 (m, 4H), 1.41-1.50 (m, 1H), 0.77-0.84 (m, 6H), 0.62-0.67 (m, 2H)
LCMS: m/z = 513.3 (M+H)⁺, Rt = 1.128 min

To afford 4-cyclopropyl-6-(3-((1r,3S)-3-hydroxy-1-(4-methyl-4H-1,2,4-triazol-3-yl)cyclobutyl)phenyl)-2-(((S)-3-methylpiper idin-1-yl)methyl)-1,6-dihydro-7H-pyrrolo[2,3-c]pyridin-7-one (30.1 mg, 58.4 µmol, 19.9% yield, 99.5% purity) as a yellow solid which was confirmed by H NMR and LCMS.
¹H NMR: (400 MHz, DMSO-*d*₆)
δ 11.94 (s, 1H), 8.28 (s, 1H), 7.47 (t, *J* = 8.0 Hz, 1H), 7.35 (s, 1H), 7.31 (d, *J=* 8.0 Hz, 1H), 7.27 (d, *J* = 7.6 Hz, 1H), 6.79 (s, 1H), 6.31 (s, 1H), 5.22-5.45 (m, 1H), 4.21-4.31 (m, 1H), 3.57 (s, 2H), 3.27 (s, 3H), 3.03-3.09 (m, 2H), 2.70-2.78 (m, 4H), 1.84-1.91 (m, 2H), 1.56-1.65 (m, 4H), 1.47 (s, 1H), 0.81 (d, *J* = 4.0 Hz, 6H), 0.62-0.67 (m, 2H)
LCMS: m/z = 513.4 (M+H)⁺, Rt = 1.122min

### Examples 134 and 135: Synthesis of compounds T134& T135

### 1. General steps for preparation of 1-(3-bromophenyl)-3,3-dimethoxy-cyclobutanecarbonitrile

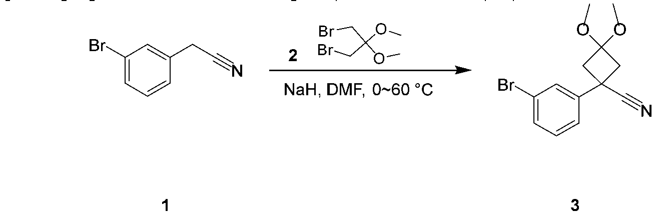

To a solution of NaH (8.16 g, 204 mmol, 60.0% purity) in DMF (170 mL) was added 2-(3-bromophenyl)acetonitrile (20.0 g, 102 mmol) at 0 °C under N₂ atmosphere. After addition, 1,3-dibromo-2,2-dimethoxy-propane (26.7 g, 102 mmol) in DMF (30.0 mL) was added dropwise to the mixture at 60 °C. The resulting mixture was stirred at 60 °C for 2 hrs. LCMS showed desired compound was detected. The reaction mixture was diluted with NH₄Cl (30.0 mL) at 0 °C, extracted with Ethyl acetate (20.0 mL * 3), the combined organic layers were washed with brine (20.0 mL * 3), dried over Na₂SO₄, filtered and concentrated under reduced pressure to give a residue. The residue was purified by column chromatography (SiO₂, Petroleum ether/ Ethyl acetate = 0% to 4.00%), (Plate 1, Petroleum ether/ Ethyl acetate = *5*/ 1, R_{f} (product) = 0.4). Compound 1-(3-bromophenyl)-3,3-dimethoxy-cyclobutanecarbonitrile (12.9 g, 33.6 mmol, 33.0% yield, 77.3% purity) was obtained as a colorless oil. Confirmed by H NMR and LCMS.
¹H NMR: (CDCl₃, 400 MHz)
δ 7.59 (t, *J* = 2.0 Hz, 1H), 7.41-7.45 (m, 1H), 7.36-7.40 (m, 1H), 7.24 (s, 1H), 3.24 (s, 3H), 3.15 (s, 3H), 3.04-3.08 (m, 2H), 2.64-2.69 (m, 2H)
LCMS: m/z = 265.8 (M+H-32)⁺, Rt = 1.740 min

### 2. General steps for preparation of 1-(3-bromophenyl)-3,3-dimethoxy-cyclobutanecarboxylic acid

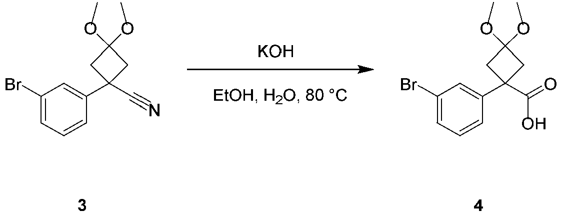

To a solution of 1-(3-bromophenyl)-3,3-dimethoxy-cyclobutanecarbonitrile (12.9 g, 43.5 mmol) in H₂O (130 mL) and EtOH (130 mL) was added KOH (73.3 g, 1.31 mol). After addition, the mixture was stirred at 80 °C for 16 hrs. LCMS showed desired compound was detected. The reaction mixture was diluted with H₂O (300 mL), extracted with Ethyl acetate (300 mL*2). The aqueous phase was adjust pH = 2 (HCl, 2 M), extracted with Ethyl acetate (300 mL*2). The organic phase was washed with brine (300 mL), dried over Na₂SO₄ and concentrated in vacuum. Compound 1-(3-bromophenyl)-3,3-dimethoxy-cyclobutanecarboxylic acid (10.3 g, 29.8 mmol, 68.5% yield, 91.4% purity) was obtained as a colorless oil. Confirmed by H NMR.
¹H NMR: (CDCl₃, 400 MHz)
*δ* 9.63-11.81 (m, 1H), 7.46 (s, 1H), 7.37-7.42 (m, 1H), 7.22-7.26 (m, 1H), 7.17-7.22 (m, 1H), 3.19 (s, 3H), 3.14-3.16 (m, 1H), 3.12 (s, 3H), 3.11-3.12 (m, 1H), 2.55-2.57 (m, 1H), 2.52-2.54 (m, 1H)

### 3. General steps for preparation of 1-1-(3-bromophenyl)-3,3-dimethoxy-cyclobutanecarbonyl]amino]-3-methyl-thiourea

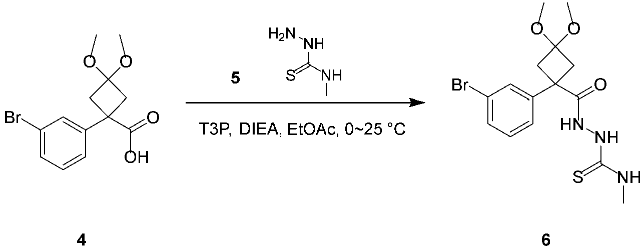

To a solution of 1-(3-bromophenyl)-3,3-dimethoxy-cyclobutanecarboxylic acid (10.3 g, 32.6 mmol) in Ethyl acetate (110 mL) was added dropwise DIEA (63.3 g, 490 mmol, 85.3 mL), and then 1-amino-3-methyl-thiourea (4.47 g, 42.4 mmol) was added to the mixture, T3P (64.8 g, 101 mmol, 60.7 mL, 50.0% purity) was added to the mixture at 0 °C. The resulting mixture was stirred at 25 °C for 2 hrs. LCMS showed desired compound was detected. The reaction mixture was diluted with Ethyl acetate (50.0 mL), the combined organic layers were washed with aqueous NaHCO₃ (50.0 mL * 2) and aqueous NH₄Cl (50.0 mL * 2), dried over Na₂SO₄, filtered and concentrated under reduced pressure to give a residue. Compound 1-[[1-(3-bromophenyl)-3,3-dimethoxy-cyclobutanecarbonyl]amino]-3-methyl-thiourea (11.9 g, 25.8 mmol, 79.0% yield, 86.7% purity) was obtained as a yellow oil. Confirmed by H NMR.
¹H NMR: (CDCl₃, 400 MHz)
δ 10.91-11.88 (m, 1H), 7.44-7.52 (m, 1H), 7.34 (d, *J=* 7.6 Hz, 1H), 7.26-7.31 (m, 1H), 7.26 (s, 2H), 7.15-7.19 (m, 1H), 3.18-3.29 (m, 3H), 3.17 (d, *J = 5.2* Hz, 2H), 3.15 (s, 3H), 3.03-3.08 (m, 3H), 2.52-2.63 (m, 2H)

### 4. General steps for preparation of 5-[1-(3-bromophenyl)-3,3-dimethoxy-cyclobutyl]-4-methyl-1,2,4-triazole-3-thiol

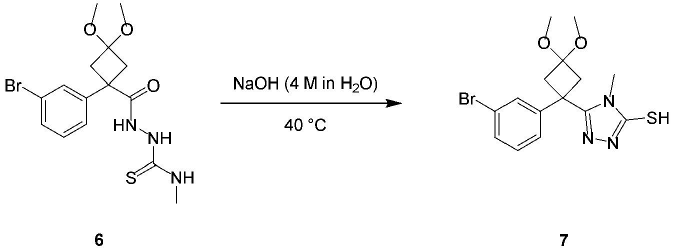

To a solution of NaOH (18.9 g, 473 mmol) in H₂O (120 mL) was added dropwisel-[[1-(3-bromophenyl)-3,3-dimethoxy-cyclobutanecarbonyl]amino]-3-methyl-thiourea (11.9 g, 29.5 mmol). After addition, the mixture was stirred at 40 °C for 12 hrs. LCMS showed desired compound was detected. The reaction mixture was diluted with H₂O (100 mL), extracted with Ethyl acetate (100 mL * 3), the combined organic layers were washed with H₂O (100 mL * 3), dried over Na₂SO₄, filtered and concentrated under reduced pressure to give a residue. Compound 5-[1-(3-bromophenyl)-3,3-dimethoxy-cyclobutyl]-4-methyl-1,2,4-triazole-3-thiol (7.92 g, crude) was obtained as a colorless oil.

### 5. General steps for preparation of 3-[1-(3-bromophenyl)-3,3-dimethoxy-cyclobutyl]-4-methyl-1,2,4-triazole:

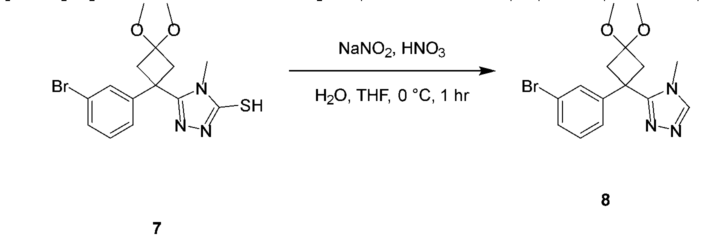

To a solution of 5-[1-(3-bromophenyl)-3,3-dimethoxy-cyclobutyl]-4-methyl-1,2,4-triazole-3-thiol (7.92 g, 20.6 mmol) in THF (40.0 mL) and H₂O (40.0 mL) was added NaNO₂ (14.2 g, 206 mmol). HNO₃ (27.1 g, 280 mmol, 19.4 mL, 65.0% purity) was added to the mixture slowly at 0 °C. After addition, the mixture was stirred at 0 °C for 1 hr. LCMS showed desired compound was detected. The reaction mixture was diluted with aqueous NaHCO₃ (200 mL) adjust pH = 8, extracted with Ethyl acetate (80.0 mL * 3), the combined organic layers were washed with H₂O (80.0 mL * 3), dried over Na₂SO₄, filtered and concentrated under reduced pressure to give a residue. The residue was purified by column chromatography (SiO₂, DCM/ MeOH = 0% to 10.0%), (Plate 1, DCM/ MeOH = 10/ 1, R_{f} (product) = 0.34). Compound 3-[1-(3-bromophenyl)-3,3-dimethoxy-cyclobutyl]-4-methyl-1,2,4-triazole (6.00 g, 14.3 mmol, 69.4% yield, 84.0% purity) was obtained as a white solid. Confirmed by H NMR.
¹H NMR: (CDCl₃, 400 MHz)
*δ* 8.02 (s, 1H), 7.42 (s, 1H), 7.35-7.39 (m, 1H), 7.16-7.21 (m, 1H), 7.12-7.16 (m, 1H), 3.27-3.31 (m, 2H), 3.25 (s, 3H), 3.20 (s, 3H), 3.16 (s, 3H), 2.86-2.90 (m, 2H)

### 6. General steps for preparation of 3-(3-bromophenyl)-3-(4-methyl-1,2,4-triazol-3-yl)cyclobutanone

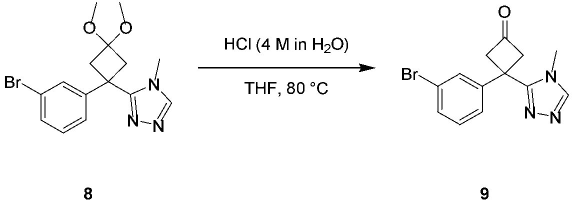

To a solution of 3-[1-(3-bromophenyl)-3,3-dimethoxy-cyclobutyl]-4-methyl-1,2,4-triazole (6.00 g, 17.0 mmol) in THF (60.0 mL) was added dropwise HCl (4 M, 45.0 mL). The resulting mixture was stirred at 80 °C for 3 hrs. LCMS showed desired compound was detected. The reaction mixture was diluted with aqueous Na₂CO₃ (250 mL), adjust the pH = 8, extracted with Ethyl acetate (200 mL * 2), the combined organic layers were washed with H₂O (200 mL), dried over Na₂SO₄, filtered and concentrated under reduced pressure to give a residue. The residue was purified by column chromatography (SiO₂, DCM/ MeOH = 0% to 6.00%), (Plate 1, DCM/ MeOH = 10/ 1, R_{f} (product) = 0.4). Compound 3-(3-bromophenyl)-3-(4-methyl-1,2,4-triazol-3-yl)cyclobutanone (3.50 g, 11.3 mmol, 66.7% yield, 99.4% purity) was obtained as a colorless oil. Confirmed by H NMR and LCMS.
¹H NMR: (CDCl₃, 400 MHz)
*δ* 8.23 (s, 1H), 7.46 *(d, J=* 7.6 Hz, 1H), 7.41 (t*, J=* 1.6 Hz, 1H), 7.26 (s, 1H), 7.15-7.21 (m, 1H), 4.15-4.24 (m, 2H), 3.65-3.75 (m, 2H), 3.32 (s, 3H)
LCMS: m/z = 307.9 (M+H)⁺, Rt = 1.190 min

### 7. General steps for preparation of 3-(3-bromophenyl)-3-(4-methyl-1,2,4-triazol-3-yl)cyclobutanol

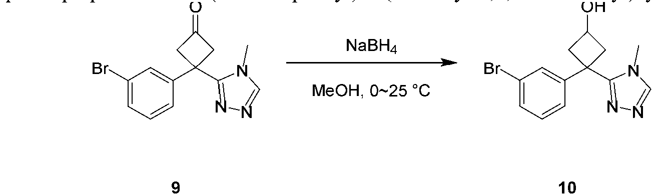

To a solution of 3-(3-bromophenyl)-3-(4-methyl-1,2,4-triazol-3-yl)cyclobutanone (1.50 g, 4.90 mmol) in MeOH (30.0 mL) was added NaBH₄ (370 mg, 9.80 mmol) slowly at 0 °C. After addition, the mixture was stirred at 25 °C for 2 hrs. LCMS showed desired compound was detected. The reaction mixture was quenched by saturated aqueous NH₄Cl (20.0 mL) slowly. The mixture was extracted with Ethyl acetate (30.0 mL*3), dried over Na₂SO₄. The organic phase was concentrated in vacuum. The residue was purified by column chromatography (SiO₂, DCM/ MeOH = 0% to 14.0%), (Plate 1, DCM/ MeOH = 10/ 1, R_{f} (product) = 0.18). Compound 3-(3-bromophenyl)-3-(4-methyl-1,2,4-triazol-3-yl)cyclobutanol (1.17 g, 3.78 mmol, 77.1% yield, 99.5% purity) was obtained as a white solid. Confirmed by H NMR and LCMS.
¹H NMR: (CDCl₃, 400 MHz)
*δ* 7.96-8.08 (m, 1H), 7.36-7.48 (m, 2H), 7.09-7.23 (m, 2H), 4.43-4.59 (m, 1H), 3.34-3.39 (m, 1H), 3.18-3.22 (m, 3H), 3.07-3.15 (m, 1H), 2.95-3.03 (m, 1H), 2.57-2.63 (m, 1H)
LCMS: m/z = 309.5 (M+H)⁺, Rt = 0.942 min

### 8. General steps for preparation of 3-[1-(3-bromophenyl)-3-methoxy-cyclobutyl]-4-methyl-1,2,4-triazole

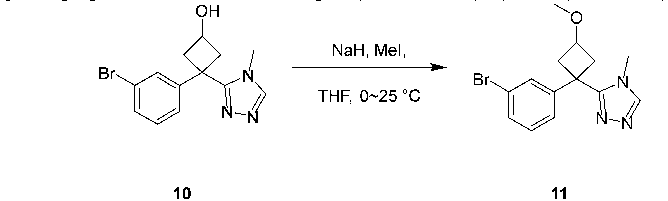

To a solution of 3-(3-bromophenyl)-3-(4-methyl-1,2,4-triazol-3-yl)cyclobutanol (150 mg, 486 µmol) in DMF (1.50 mL) was added NaH (60.0 mg, 1.50 mmol, 60.0% purity) at 0 °C. After addition, the mixture was stirred at 0 °C for 1 hr, and then MeI (83.0 mg, 584 µmol, 36.4 µL) was added to the mixture at 0 °C, the mixture was stirred at 25 °C for 12 hrs. LCMS showed desired compound was detected. The reaction mixture was quenched by aqueous NH₄Cl (30.0 mL), extracted with Ethyl acetate (20.0 mL * 3), the combined organic layers were washed with H₂O (20.0 mL * 3), dried over Na₂SO₄, filtered and concentrated under reduced pressure to give a residue. The residue was purified by column chromatography (SiO₂, DCM/ MeOH = 0% to 4.00%), (Plate 1, DCM/ MeOH = 20/ 1, R_{f} (product) = 0.34). Compound 3-[1-(3-bromophenyl)-3-methoxy-cyclobutyl]-4-methyl-1,2,4-triazole (140 mg, crude) was obtained as a colorless oil. Confirmed by H NMR.
¹H NMR: (CDCl₃, 400 MHz)
δ 8.09 (br d, *J =* 20.0 Hz, 1H), 7.35-7.50 (m, 2H), 7.07-7.24 (m, 2H), 4.00-4.14 (m, 1H), 3.34 (m, *J* = 9.2, 6.8, 2.8 Hz, 1H), 3.26 (d, *J* = 5.2 Hz, 3H), 3.22 (d, *J=* 8.0 Hz, 3H), 2.92-3.10 (m, 2H), 2.52-2.60 (m, 1H)

### 9. General steps for preparation of 6-[3-[3-methoxy-1-(4-methyl-1,2,4-triazol-3-yl)cyclobutyl]phenyl]-2-[[(3S)-3-methyl-1-piperidyl]methyl]-1-(p-tol ylsulfonyl)-4-(trifluoromethyl)pyrrolo[2,3-c]pyridin-7-one:

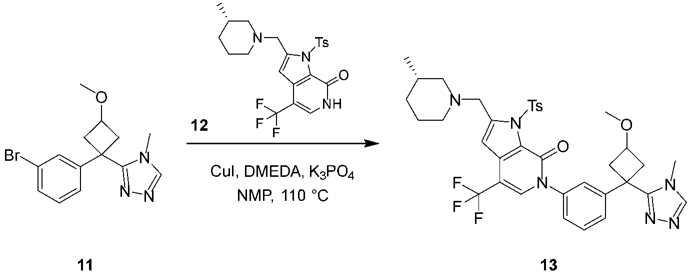

To a solution of 3-[1-(3-bromophenyl)-3-methoxy-cyclobutyl]-4-methyl-1,2,4-triazole (80.0 mg, 248 µmol) and2-[[(3S)-3-methyl-1-piperidyl]methyl]-1-(p-tolylsulfonyl)-4-(trifluoromethyl)-6H-pyrrolo [2,3-c]pyridin-7-one (116 mg, 248 µmol) in NMP (1.00 mL) was added CuI (47.2 mg, 248 µmol), K₃PO₄ (158 mg, 744 µmol), DMEDA (43.7 mg, 496 µmol, 53.4 µL). After addition, the mixture was stirred at 110 °C for 4 hrs under N₂. LCMS showed desired compound was detected. The reaction mixture was filtered and diluted with H₂O (30.0 mL), extracted with Ethyl acetate (20.0 mL * 3), the combined organic layers were washed with H₂O (20.0 mL * 3), dried over Na₂SO₄, filtered and concentrated under reduced pressure to give a residue. The residue was purified by column chromatography (SiO₂, DCM/ MeOH = 3% to 20.0%), (Plate 1, DCM/ MeOH = 10/ 1, R_{f} (product) = 0.25). Compound 6-[3-[3-methoxy-1-(4-methyl-1,2,4-triazol-3-yl)cyclobutyl]phenyl]-2-[[(3S)-3-methyl-1-piperidyl]methyl]-1-(p-tol ylsulfonyl)-4-(trifluoromethyl)pyrrolo[2,3-c]pyridin-7-one (71.0 mg, 84.4 µmol, 34.0% yield, 84.3% purity) was obtained as a colorless oil. Confirmed by LCMS.
LCMS: m/z =709.2 (M+H)⁺, Rt = 1.125 min

### 10. General steps for preparation of 6-[3-[3-methoxy-1-(4-methyl-1,2,4-triazol-3-yl)cyclobutyl]phenyl]-2-[[(3S)-3-methyl-1-piperidyl]methyl]-4-(triflu oromethyl)-1H-pyrrolo[2,3-c]pyridin-7-one

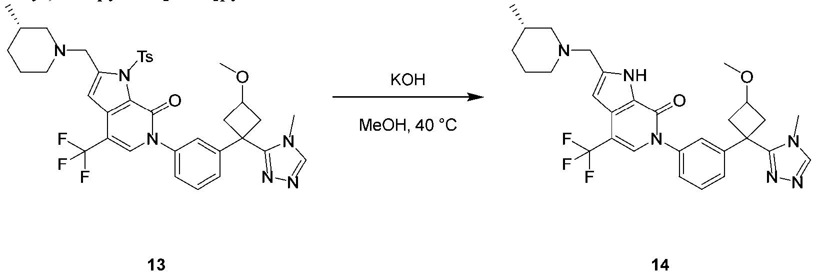

A mixture of 6-[3-[3-methoxy-1-(4-methyl-1,2,4-triazol-3-yl)cyclobutyl]phenyl]-2-[[(3S)-3-methyl-1-piperidyl]methyl]-1-(p-tol ylsulfonyl)-4-(trifluoromethyl)pyrrolo[2,3-c]pyridin-7-one (61.0 mg, 86.0 µmol), KOH (96.5 mg, 1.72 mmol) in MeOH (1.00 mL) was degassed and purged with N₂ for 3 times, and then the mixture was stirred at 40 °C for 1 hr under N₂ atmosphere. LCMS showed desired compound was detected. The reaction mixture was diluted with Brine (30.0 mL), extracted with Ethyl acetate (20.0 mL * 3), the combined organic layers were washed with aqueous KOH (20.0 mL * 2), dried over Na₂SO₄, filtered and concentrated under reduced pressure to give a residue. The residue was purified by Prep-HPLC (column: Welch Xtimate C18 150 * 40.0 mm * 10.0 um; mobile phase: [water (FA)-ACN]; gradient: 0%-36.0% B over 25 mins). (SFC: Whelk0_EtOH_DEA_5_40_28 ML_4 mins A), (Plate 1, Rt (peak 1) = 2.533, Rₜ (peak 2) = 2.677). Compound 6-[3-[3-methoxy-1-(4-methyl-1,2,4-triazol-3-yl)cyclobutyl]phenyl]-2-[[(3S)-3-methyl-1-piperidyl]methyl]-4-(triflu oromethyl)-1H-pyrrolo[2,3-c]pyridin-7-one (45.0 mg, 79.7 µmol, 92.6% yield, 98.3% purity) was obtained as a white solid. Confirmed by LCMS.
LCMS: m/z = 555.1 (M+H)⁺, Rt = 1.005 min

### 11. General steps for preparation of 6-(3-((1s,3R)-3-methoxy-1-(4-methyl-4H-1,2,4-triazol-3-yl)cyclobutyl)phenyl)-2-(((S)-3-methylpiperidin-1-yl)met hyl)-4-(trifluoromethyl)-1,6-dihydro-7H-pyrrolo[2,3-c]pyridin-7-one (compound T134) and 6-(3-((1r,3S)-3-methoxy-1-(4-methyl-4H-1,2,4-triazol-3-yl)cyclobutyl)phenyl)-2-(((S)-3-methylpiperidin-1-yl)met hyl)-4-(trifluoromethyl)-1,6-dihydro-7H-pyrrolo[2,3-c]pyridin-7-one (compound T135)

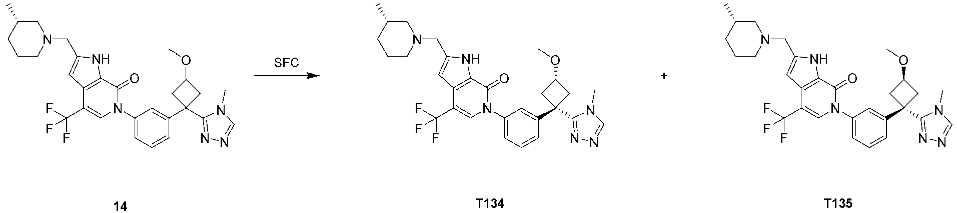

The residue was purified by SFC: (Whelk0_EtOH_DEA_5_40_28 ML_4 min A), (Plate 1, Rt (peak 1) = 2.533, Rt (peak 2) = 2.677). Compound 6-(3-((1s,3R)-3-methoxy-1-(4-methyl-4H-1,2,4-triazol-3-yl)cyclobutyl)phenyl)-2-(((S)-3-methylpiperidin-1-yl)met hyl)-4-(trifluoromethyl)-1,6-dihydro-7H-pyrrolo[2,3-c]pyridin-7-one (19.9 mg, 35.4 µmol, 43.7% yield, 98.9% purity) was obtained as a white solid. Confirmed by H NMR and LCMS.
¹H NMR: (DMSO-*d*₆, 400 MHz)
*δ* 12.17-12.61 (m, 1H), 8.38 (s, 1H), 7.71 (d, *J=* 1.2 Hz, 1H), 7.44-7.54 (m, 1H), 7.32-7.40 (m, 2H), 7.18 (br d, *J =* 7.6 Hz, 1H), 6.27 (s, 1H), 3.81-3.88 (m, 1H), 3.61 (s, 2H), 3.32-3.34 (m, 2H), 3.30 (s, 3H), 3.16 (s, 3H), 2.71-2.80 (m, 2H), 2.54 (br d, *J =* 2.6 Hz, 2H), 1.90 (br t, J = 10.0 Hz, 1H), 1.54-1.65 (m, 4H), 1.18-1.49 (m, 2H), 0.81 (br d, *J =* 5.6 Hz, 3H)
LCMS: m/z =555.3 (M+H)⁺, Rt = 2.383 min

Compound 6-(3-((1r,3S)-3-methoxy-1-(4-methyl-4H-1,2,4-triazol-3-yl)cyclobutyl)phenyl)-2-(((S)-3-methylpiperidin-1-yl)met hyl)-4-(trifluoromethyl)-1,6-dihydro-7H-pyrrolo[2,3-c]pyridin-7-one (7.0 mg, 12.5 µmol, 15.4% yield, 99.3% purity) was obtained as a white solid. Confirmed by H NMR and LCMS.
¹H NMR: (DMSO-*d*₆, 400 MHz)
*δ* 12.29-12.59 (m, 1H), 8.30 (s, 1H), 7.72 (s, 1H), 7.47-7.56 (m, 2H), 7.30-7.39 (m, 2H), 6.27 (s, 1H), 4.06 (dt, *J=* 14.4, 7.2 Hz, 1H), 3.60 (s, 2H), 3.27 (s, 3H), 3.15 (s, 3H), 3.07-3.12 (m, 2H), 2.72-2.83 (m, 4H), 1.89 (br t, *J=* 10.8 Hz, 1H), 1.60 (br s, 3H), 1.45 (br d, *J =* 12.0 Hz, 2H), 1.23 (br s, 1H), 0.81 (br d, *J =* 5.2 Hz, 3H)
LCMS: m/z =555.3 (M+H)⁺, Rt = 2.373 min

### Examples 136 and 137: Synthesis of compounds T136& T137

### 1. General steps for preparation of [3-(3-bromophenyl)-3-(4-methyl-1,2,4-triazol-3-yl)cyclobutyl] methanesulfonate

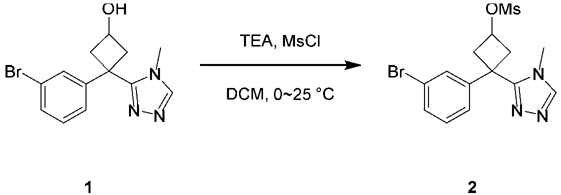

To a solution of 3-(3-bromophenyl)-3-(4-methyl-1,2,4-triazol-3-yl)cyclobutanol (500 mg, 1.62 mmol) in DCM (10.0 mL) was added TEA (329 mg, 3.25 mmol). Then a solution of MsCl (240 mg, 2.10 mmol) in DCM (2.00 mL) was added to the mixture at 0 °C. The mixture was stirred under N₂ at 25 °C for 2 hrs. TLC (DCM/ MeOH = 10/ 1, product 1 R_{f} = 0.50) indicated new spot formed. The reaction mixture was poured into saturated aqueous NH₄Cl (20 mL) slowly at 0 °C. The mixture was extracted with DCM (20.0 mL*2). The organic phase was washed with brine (20.0 mL), dried over Na₂SO₄ and concentrated in vacuum. The crude product was purified by column chromatography (SiO₂, DCM/ MeOH = 1/ 0 to 15/ 1). To afford [3-(3-bromophenyl)-3-(4-methyl-1,2,4-triazol-3-yl)cyclobutyl] methanesulfonate (450 mg, 1.15 mmol, 70.6% yield, 98.4% purity) as a white solid which was confirmed by H NMR and LCMS.
¹H NMR: (400 MHz, CDCl₃)
*δ* 8.10 (d, *J =* 20.0 Hz, 1H), 7.45 (dd, *J =* 4.0, 2.0 Hz, 1H), 7.29-7.43 (m, 1H), 7.23-7.26 (m, 1H), 7.08-7.23 (m, 1H), 5.03-5.27 (m, 1H), 3.54 (ddd, *J* = 9.6, 7.2, 2.8 Hz, 1H), 3.29-3.48 (m, 1H), 3.23-3.29 (m, 1H), 3.22 (d, *J =* 4.0 Hz, 3H), 3.02 (d, *J=* 4.4 Hz, 3H), 2.93-2.99 (m, 1H)
LCMS: m/z = 385.5, 387.5 (M+H)⁺, Rt = 1.022, 1.092 min

### 2. General steps for preparation of 3-(3-bromophenyl)-3-(4-methyl-1,2,4-triazol-3-yl)cyclobutanecarbonitrile

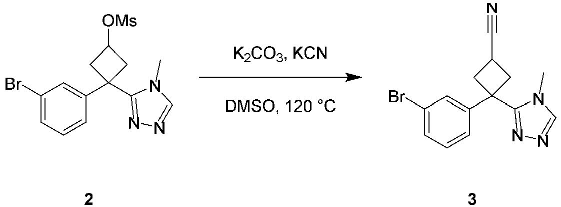

To a solution of [3-(3-bromophenyl)-3-(4-methyl-1,2,4-triazol-3-yl)cyclobutyl] methanesulfonate (450 mg, 1.17 mmol) in DMSO (10.0 mL) was added K₂CO₃ (322 mg, 2.33 mmol) and KCN (160 mg, 2.46 mmol). The mixture was stirred under N₂ at 120 °C for 12 hrs. TLC (DCM/ MeOH = 10/ 1, product 1 R_{f} = 0.45) indicated new spot formed. The reaction mixture was diluted with Ethyl acetate (20.0 mL), washed with brine (20.0 mL*3), dried over Na₂SO₄. The organic phase was concentrated in vacuum. The aqueous phase was adjust pH = 11 (NaOH, 3 M), quenched by saturated aqueous NaClO (100 mL). The crude product was purified by column chromatography (SiO₂, DCM/ MeOH = 1/ 0 to 15/ 1). To afford 3-(3-bromophenyl)-3-(4-methyl-1,2,4-triazol-3-yl)cyclobutanecarbonitrile (250 mg, 687 µmol, 59.0% yield, 87.2% purity) as a yellow solid which was confirmed by H NMR and LCMS.
¹H NMR: (400 MHz, CDCl₃)
*δ* 7.97-8.20 (m, 1H), 7.44-7.48 (m, 1H), 7.27-7.34 (m, 1H), 7.23-7.26 (m, 1H), 7.08-7.23 (m, 1H), 3.42-3.53 (m, 1H), 3.27-3.42 (m, 2H), 3.20 (d, *J=* 7.2 Hz, 3H), 3.01-3.13 (m, 2H)
LCMS: m/z = 316.5, 318.5 (M+H)⁺, Rt = 1.028, 1.068 min

### 3. General steps for preparation of 3-[3-[2-[[(3S)-3-methyl-1-piperidyl]methyl]-7-oxo-1-(p-tolylsulfonyl)-4-(trifluoromethyl)pyrrolo[2,3-c]pyridin-6-y l]phenyl]-3-(4-methyl-1,2,4-triazol-3-yl)cyclobutanecarbonitrile

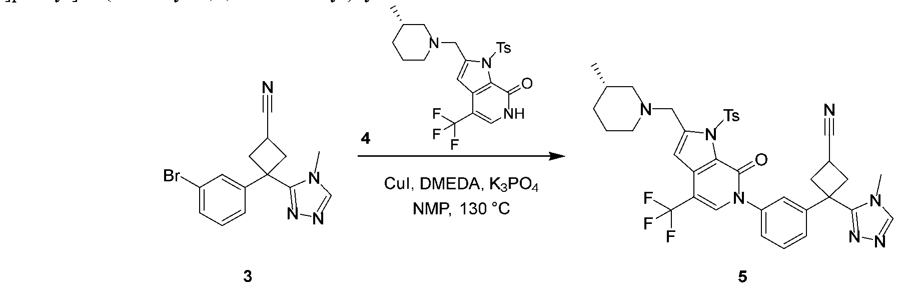

To a solution of 3-(3-bromophenyl)-3-(4-methyl-1,2,4-triazol-3-yl)cyclobutanecarbonitrile (180 mg, 567 µmol) and 2-[[(3S)-3-methyl-1-piperidyl]methyl]-1-(p-tolylsulfonyl)-4-(trifluoromethyl)-6H-pyrrolo[2,3-c]pyridin-7-one (239 mg, 511 µmol) in NMP (1.80 mL) was added K₃PO₄ (362 mg, 1.71 mmol), CuI (216 mg, 1.13 mmol) and DMEDA (50.0 mg, 567 µmol). The suspension was degassed under vacuum and purged with N₂ several times. The mixture was stirred under N₂ at 130 °C for 2 hrs. TLC (DCM/ MeOH = 10/ 1, product 1 R_{f} = 0.35) indicated new spot formed. The reaction mixture was poured into saturated aqueous NH₄Cl (10 mL) slowly. The mixture was extracted with EtOAc (10.0 mL*2). The organic phase was washed with brine (20.0 mL*2), dried over Na₂SO₄ and concentrated in vacuum. The crude product was purified by column chromatography (SiO₂, DCM/ MeOH = 1/ 0 to 15/ 1). To afford 3-[3-[2-[[(3S)-3-methyl-1-piperidyl]methyl]-7-oxo-1-(p-tolylsulfonyl)-4-(trifluoromethyl)pyrrolo[2,3-c]pyridin-6-y l]phenyl]-3-(4-methyl-1,2,4-triazol-3-yl)cyclobutanecarbonitrile (150 mg, 162 µmol, 28.6% yield, 76.2% purity) as a yellow solid which was confirmed by LCMS.
LCMS: m/z = 703.9, 704.0 (M+H)⁺, Rt = 1.188, 1.208 min

### 4. General steps for preparation of (1S,3r)-3-(4-methyl-4H-1,2,4-triazol-3-yl)-3-(3-(2-(((S)-3-methylpiperidin-1-yl)methyl)-7-oxo-4-(trifluoromethyl)-1,7-dihydro-6H-pyrrolo[2,3-c]pyridin-6-yl)phenyl)cyclobutane-1-carbonitrile (compound T136) and (1R,3S)-3-(4-methyl-4H-1,2,4-triazol-3-yl)-3-(3-(2-(((S)-3-methylpiperidin-1-yl)methyl)-7-oxo-4-(trifluoromethyl)-1,7-dihydro-6H-pyrrolo[2,3-c]pyridin-6-yl)phenyl)cyclobutane-1-carbonitrile (compound T137)

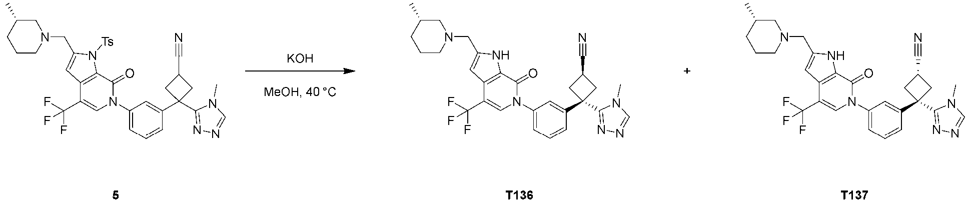

To a solution of 3-[3-[2-[[(3S)-3-methyl-1-piperidyl]methyl]-7-oxo-1-(p-tolylsulfonyl)-4-(trifluoromethyl)pyrrolo[2,3-c]pyridin-6-y l]phenyl]-3-(4-methyl-1,2,4-triazol-3-yl)cyclobutanecarbonitrile (150 mg, 213 µmol) in MeOH (3.00 mL) was added KOH (239 mg, 4.26 mmol). The mixture was stirred under N₂ at 40 °C for 1hr. LCMS showed desired mass was detected. The reaction mixture was diluted with brine (20.0 mL), extracted with EtOAc (20.0 mL*2). The organic phase washed with KOH (1 M, 20 mL), dried over Na₂SO₄ and concentrated in vacuum. The crude product was purified by Prep-HPLC (column: Xtimate C18 150*40 mm*10 um; mobile phase: [water (NH₃H₂O+NH₄HCO₃)-ACN]; gradient: 22.0%-62.0% B over 32 mins). To afford (1S,3r)-3-(4-methyl-4H-1,2,4-triazol-3-yl)-3-(3-(2-(((S)-3-methylpiperidin-1-yl)methyl)-7-oxo-4-(trifluoromethyl)-1,7-dihydro-6H-pyrrolo[2,3-c]pyridin-6-yl)phenyl)cyclobutane-1-carbonitrile (8.00 mg, 14.5 µmol, 99.7% purity) as a white solid. To afford (1R,3S)-3-(4-methyl-4H-1,2,4-triazol-3-yl)-3-(3-(2-(((S)-3-methylpiperidin-1-yl)methyl)-7-oxo-4-(trifluoromethyl)-1,7-dihydro-6H-pyrrolo[2,3-c]pyridin-6-yl)phenyl)cyclobutane-1-carbonitrile (13.0 mg, 23.6 µmol, 100% purity) as a white solid.
¹H NMR: (400 MHz, CD₃CN)
*δ* 8.04-8.14 (s, 1H), 7.56 (d, *J =* 1.6 Hz, 1H), 7.48-7.54 (m, 1H), 7.32-7.40 (m, 2H), 7.26 (d, *J =* 8.0 Hz, 1H), 6.36 (s, 1H), 3.60 (s, 2H), 3.35-3.43 (m, 2H), 3.26-3.34 (m, 1H), 3.16-3.22 (m, 3H), 2.98-3.08 (m, 2H), 2.71-2.78 (m, 2H), 1.59-1.72 (m, 4H), 1.44-1.59 (m, 2H), 0.84-0.92 (m, 1H), 0.82 (d, *J =* 6.0 Hz, 3H)
LCMS: m/z = 550.3 (M+H)⁺, Rt = 1.385 min
¹H NMR: (400 MHz, CD₃CN)
*δ* 8.07 (s, 1H), 7.57 (d, *J =* 1.6 Hz, 1H), 7.50-7.55 (m, 1H), 7.44 (t, *J =* 1.6 Hz, 1H), 7.37-7.41 (m, 1H), 7.30-7.36 (m, 1H), 6.35 (d, *J =* 1.2 Hz, 1H), 3.57 (s, 2H), 3.40-3.47 (m, 1H), 3.33-3.39 (m, 2H), 3.21 (s, 3H), 3.12-3.18 (m, 2H), 2.67-2.73 (m, 2H), 1.87 (td, *J* = 11.2, 2.4 Hz, 1H), 1.53-1.67 (m, 4H), 1.42-1.50 (m, 1H), 0.80-0.88 (m, 1H), 0.79 (d, *J =* 6.0 Hz, 3H)
LCMS: m/z = 550.2 (M+H)⁺, Rt = 1.365 min

### Examples 138 and 139: Synthesis of compounds T138& T139

### 1. General steps for preparation of 2-[3-(3-bromophenyl)-3-(4-methyl-1,2,4-triazol-3-yl)cyclobutylidene]acetonitrile

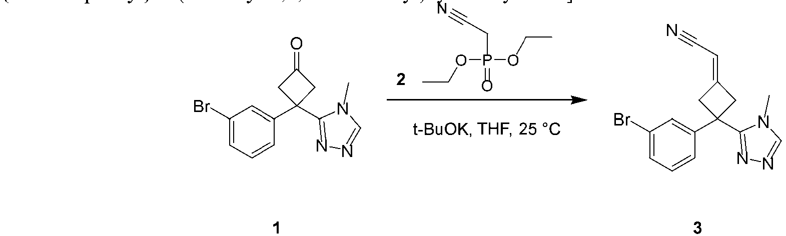

To a solution of 3-(3-bromophenyl)-3-(4-methyl-1,2,4-triazol-3-yl)cyclobutanone (100 mg, 326 µmol), 2-diethoxyphosphorylacetonitrile (69.4 mg, 391 µmol, 63.4 µL) in THF (2.00 mL) was added t-BuOK (1 M, 391 µL). The mixture was stirred under N₂ at 25 °C for 2 hrs. LCMS showed the reaction was completed. TLC (Plate 1, Dichloromethane/ Methanol = 10/ 1, UV 254 nm, R_{f} (product) = 0.5). The reaction mixture was partitioned between water (10.0 mL) and Ethyl acetate (10.0 mL). The organic phase was separated, washed with brine (10.0 mL), dried over [Na₂SO₄], filtered and concentrated under reduced pressure to give a residue. Compound2-[3-(3-bromophenyl)-3-(4-methyl-1,2,4-triazol-3-yl)cyclobutylidene]acetonitrile (110 mg, crude) was obtained as a yellow oil.

### 2. General steps for preparation of 2-[3-(3-bromophenyl)-3-(4-methyl-1,2,4-triazol-3-yl)cyclobutyl]acetonitrile

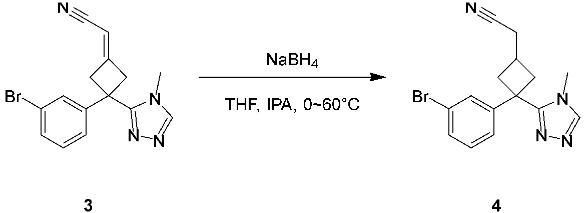

To a solution of 2-[3-(3-bromophenyl)-3-(4-methyl-1,2,4-triazol-3-yl)cyclobutylidene]acetonitrile (110 mg, 334 µmol) in IPA (0.20 mL), THF (1.00 mL) was added NaBH₄ (110 mg, 2.91 mmol) at 0 °C. The mixture was stirred under N₂ at 60 °C for 12 hrs. LCMS showed the reaction was completed. The reaction mixture was diluted with Ethyl acetate (5.00 mL). The filtrate was washed with brine (5.00 mL), dried over Na₂SO₄. The organic phase was concentrated in vacuum. The residue was purified by column chromatography (SiO₂, Dichloromethane/ Methanol = 1/ 0 to 97/ 3). TLC (Plate 1, Dichloromethane/ Methanol = 10/ 1, UV 254 nm, R_{f} (product) = 0.5). Compound 2-[3-(3-bromophenyl)-3-(4-methyl-1,2,4-triazol-3-yl)cyclobutyl]acetonitrile (100 mg, 115 µmol, 34.4% yield, 38.1% purity) was obtained as a white solid.

### 4. General steps for preparation of 2-[3-[3-[2-[[(3S)-3-methyl-1-piperidyl]methyl]-7-oxo-1-(p-tolylsulfonyl)-4-(trifluoromethyl)pyrrolo[2,3-c]pyridin-6-yl]phenyl]-3-(4-methyl-1,2,4-triazol-3-yl)cyclobutyl]acetonitrile

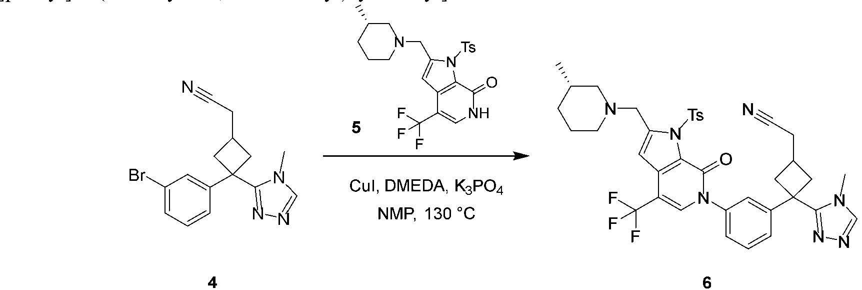

To a solution of 2-[3-(3-bromophenyl)-3-(4-methyl-1,2,4-triazol-3-yl)cyclobutyl]acetonitrile (80.0 mg, 241 µmol), 2-[[(3S)-3-methyl-1-piperidyl]methyl]-1-(p-tolylsulfonyl)-4-(trifluoromethyl)-6H-pyrrolo[2,3-c]pyridin-7-one (118 mg, 253 µmol) in NMP (2.00 mL) was added CuI (46.0 mg, 241 µmol), K₃PO₄ (153 mg, 724 µmol), DMEDA (21.2 mg, 241 µmol, 26.0 µL). The mixture was stirred under N₂ at 130 °C for 2 hrs. LCMS showed the reaction was completed. The reaction mixture was diluted with Ethyl acetate (5.00 mL). The filtrate was washed with brine (5.00 mL), dried over Na₂SO₄. The organic phase was concentrated in vacuum. The residue was purified by column chromatography (SiO₂, Dichloromethane/ Methanol = 1/ 0 to 94/ 6). TLC (Plate 1, Dichloromethane/ Methanol = 10/ 1, UV 254 nm, R_{f} (product) = 0.3). Compound 2-[3-[3-[2-[[(3S)-3-methyl-1-piperidyl]methyl]-7-oxo-1-(p-tolylsulfonyl)-4-(trifluoromethyl)pyrrolo[2,3-c]pyridin-6-yl]phenyl]-3-(4-methyl-1,2,4-triazol-3-yl)cyclobutyl]acetonitrile (90.0 mg, 125 µmol, 51.9% yield) was obtained as a yellow solid.

### 5. General steps for preparation of 2-[3-[3-[2-[[(3S)-3-methyl-1-piperidyl]methyl]-7-oxo-4-(trifluoromethyl)-1H-pyrrolo[2,3-c]pyridin-6-yl]phenyl]-3-(4-methyl-1,2,4-triazol-3-yl)cyclobutyl]acetonitrile

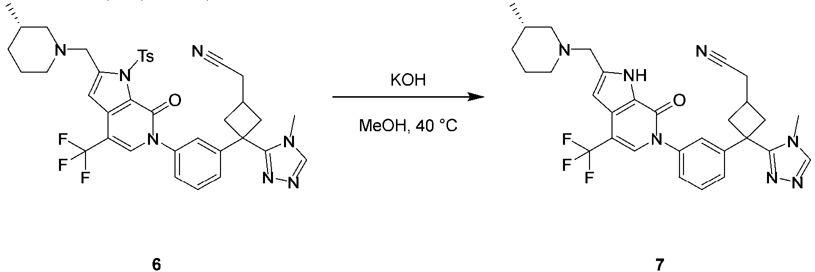

To a solution of 2-[3-[3-[2-[[(3S)-3-methyl-1-piperidyl]methyl]-7-oxo-1-(p-tolylsulfonyl)-4-(trifluoromethyl)pyrrolo[2,3-c]pyridin-6-yl]phenyl]-3-(4-methyl-1,2,4-triazol-3-yl)cyclobutyl]acetonitrile (90.0 mg, 125 µmol) in Methanol (2.00 mL) was added KOH (140 mg, 2.51 mmol). The mixture was stirred under N₂ at 40 °C for 2 hrs. LCMS showed the reaction was completed. The reaction mixture was diluted with Ethyl acetate (5.00 mL). The filtrate was washed with brine (5.00 mL), dried over Na₂SO₄. The organic phase was concentrated in vacuum. The product was purified by Prep-HPLC (column: Welch Xtimate C18 150 * 40 mm * 10 um; mobile phase: [water (FA)-ACN]; gradient: 0%-38% B over 25 min). Compound 2-[3-[3-[2-[[(3S)-3-methyl-1-piperidyl]methyl]-7-oxo-4-(trifluoromethyl)-1H-pyrrolo[2,3-c]pyridin-6-yl]phenyl]-3-(4-methyl-1,2,4-triazol-3-yl)cyclobutyl]acetonitrile (60.0 mg, 106 µmol, 84.6% yield, 99.7% purity) was obtained as a white solid.

### 6. General steps for preparation of 2-((1S,3r)-3-(4-methyl-4H-1,2,4-triazol-3-yl)-3-(3-(2-(((S)-3-methylpiperidin-1-yl)methyl)-7-oxo-4-(triffuorometh yl)-1,7-dihydro-6H-pyrrolo[2,3-c]pyridin-6-yl)phenyl)cyclobutyl)acetonitrile (compound T138) and 2-((1R,3s)-3-(4-methyl-4H-1,2,4-triazol-3-yl)-3-(3-(2-(((S)-3-methylpiperidin-1-yl)methyl)-7-oxo-4-(trifluorometh yl)-1,7-dihydro-6H-pyrrolo[2,3-c]pyridin-6-yl)phenyl)cyclobutyl)acetonitrile (compound T139)

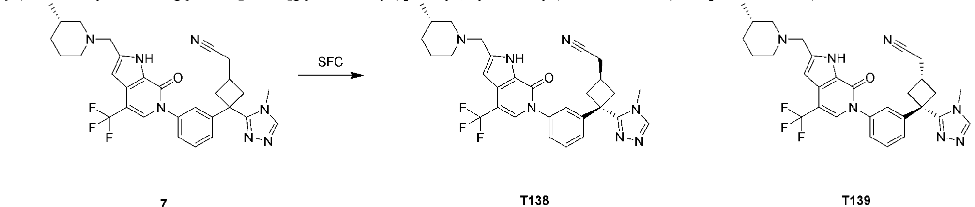

The product was purified by SFC (column: DAICEL CHIRALPAK AD (250 mm * 30 mm, 10 um); mobile phase: [CO₂-EtOH(0.10% NH₃·H₂O)]; B%: 35.0%, isocratic elution mode). Compound 2-((1S,3r)-3-(4-methyl-4H-1,2,4-triazol-3-yl)-3-(3-(2-(((S)-3-methylpiperidin-1-yl)methyl)-7-oxo-4-(triffuorometh yl)-1,7-dihydro-6H-pyrrolo[2,3-c]pyridin-6-yl)phenyl)cyclobutyl)acetonitrile (8.20 mg, 14.5 µmol, 11.6% yield, 98.4% purity) was obtained as a white solid. It was confirmed by LCMS and H NMR.
¹H NMR: (400 MHz, CD₃CN)
*δ* 9.38-12.27 (m, 1H), 8.09 (s, 1H), 7.55 (d, *J=* 1.6 Hz, 1H), 7.46-7.52 (m, 1H), 7.31-7.36 (m, 2H), 7.27 (d, *J=* 8.4 Hz, 1H), 6.35 (s, 1H), 3.57 (s, 2H), 3.24 (s, 3H), 3.15-3.23 (m, 2H), 2.69-2.75 (m, 2H), 2.62-2.69 (m, 1H), 2.58 (d, *J =* 6.4 Hz, 2H), 2.48-2.56 (m, 2H), 2.09-2.17 (m, 1H), 1.84-1.92 (m, 1H), 1.59-1.68 (m, 2H), 1.53-1.59 (m, 2H), 0.82-0.90 (m, 1H), 0.79 (d, *J =* 6.0 Hz, 3H)
LCMS: m/z = 564.3 (M+H)⁺, Rt = 1.227 min

Compound 2-((1R,3s)-3-(4-methyl-4H-1,2,4-triazol-3-yl)-3-(3-(2-(((S)-3-methylpiperidin-1-yl)methyl)-7-oxo-4-(trifluorometh yl)-1,7-dihydro-6H-pyrrolo[2,3-c]pyridin-6-yl)phenyl)cyclobutyl)acetonitrile (10.6 mg, 18.3 µmol, 14.6% yield, 97.7% purity) was obtained as a white solid. It was confirmed by LCMS and H NMR.
¹H NMR: (400 MHz, CD₃CN)
*δ* 9.52-13.77 (m, 1H), 8.05 (s, 1H), 7.58 (d, *J =* 1.6 Hz, 1H), 7.50-7.55 (m, 1H), 7.48 (t, *J =* 1.6 Hz, 1H), 7.38 (s, 1H), 7.32-7.37 (m, 1H), 6.52 (s, 1H), 4.03-4.12 (m, 2H), 3.21 (s, 3H), 3.08 (br d, *J =* 14.8 Hz, 2H), 2.90-2.95 (m, 2H), 2.85 (br dd, *J =* 5.6, 1.6 Hz, 2H), 2.56 (br d, *J =* 5.2 Hz, 2H), 2.42-2.53 (m, 2H), 2.18 (br t, *J =* 11.2 Hz, 1H), 1.86-1.92 (m, 1H), 1.79 (br s, 2H), 1.73-1.76 (m, 1H), 0.99-1.08 (m, 1H), 0.88 (d, *J=* 6.4 Hz, 3H)
LCMS: m/z = 564.1 (M+H)⁺, Rt = 1.223 min

### Example 140: Synthesis of compound T140

### 1. General steps for preparation of 3-((1s,3s)-3-methyl-1-(4-methyl-4H-1,2,4-triazol-3-yl)cyclobutyl)aniline

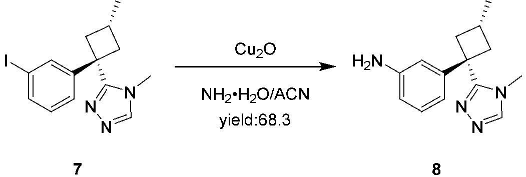

NH₃.H₂O (1.82 g, 15.6 mmol, 2.00 mL, 30% purity) was added to the mixture of 3-((1s,3s)-1-(3-iodophenyl)-3-methylcyclobutyl)-4-methyl-4H-1,2,4-triazole (300 mg, 849 µmol) and CuO (67.6 mg, 849 µmol, 10.7 µL) in ACN (2.00 mL). The mixture was stirred at 100 °C for 16 hrs. LCMS shows reactant was consumed completely and desired mass was detected. The mixture was diluted with water 3.00 mL, extracted with Ethyl acetate 15.0 mL (5.00 mL* 3), dried over Na₂SO₄,filtered and concentrated under reduced pressure to give a residue. The residue was purified by column chromatography (SiO2, Dichloromethane : Methanol = 100 : 0 to 93 : 7). TLC (Dichloromethane : Methanol = 10 : 1,product 1 R_{f} = 0.45). 3-((1s,3s)-3-methyl-1-(4-methyl-4H-1,2,4-triazol-3-yl)cyclobutyl)aniline (180 mg, 580 µmol, 68.3% yield, 78.1% purity) was obtained as white solid, confirmed by LCMS and H NMR.
¹H NMR: (400 MHz, DMSO-*d*₆)
*δ* 8.26 (s, 1H), 6.98 (t, *J =* 8.0 Hz, 1H), 6.44-6.49 (m, 2H), 6.39-6.43 (m, 1H), 5.05 (s, 2H), 3.14 (s, 3H), 2.71 (dd, *J =* 9.6, 5.6 Hz, 2H), 2.51-2.53 (m, 2H), 2.43-2.48 (m, 2H)
LCMS: m/z = 243.1 (M+H)⁺, Rt = 0.689 min

### 2. General steps for preparation of 5-bromo-3-(trifluoromethyl)pyridine-2-carbaldehyde

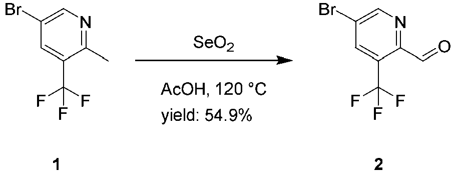

SeO₂ (6.70 g, 60.4 mmol) was added to the solution of 5-bromo-2-methyl-3-(trifluoromethyl)pyridine (5.80 g, 24.2 mmol) in AcOH (58.0 mL). The mixture was stirred at 120 °C under N₂ for 16 hrs. LCMS shows reactant was consumed completely and desired mass was detected. The mixture was concentrated under reduced pressure at 60 °C to remove AcOH, then diluted with sat .NaHCO₃ solution 70.0 mL, and Ethyl acetate 70.0 mL and filtered. The water phase was extracted with Ethyl acetate 160 mL (80.0 mL * 2). The combined organic phase was washed with brine 50.0 mL, dried over Na₂SO₄, filtered and concentrated under reduced pressure to give a residue. The residue was purified by column chromatography (SiO2, Petroleum ether : Ethyl acetate = 100 : 0 to 10 : 1). TLC(Petroleum ether : Ethyl acetate = 5 : 1,product 1 R_{f} = 0.5). 5-bromo-3-(trifluoromethyl)pyridine-2-carbaldehyde (3.61 g, 13.3 mmol, 54.9% yield, 93.4% purity) was obtained as yellow oil, confirmed by LCMS and H NMR.
¹H NMR: (400 MHz, DMSO-*d*₆)
*δ* 10.03 (s, 1H), 9.25 (d, *J =* 1.6 Hz, 1H), 8.69 (d, *J=* 1.2 Hz, 1H)
LCMS: m/z = 253.9 (M+H)⁺, Rt = 0.960 min

### 3. General steps for preparation of N-((5-bromo-3-(trifluoromethyl)pyridin-2-yl)methyl)-3-((1s,3s)-3-methyl-1-(4-methyl-4H-1,2,4-triazol-3-yl)cyclob utyl)aniline

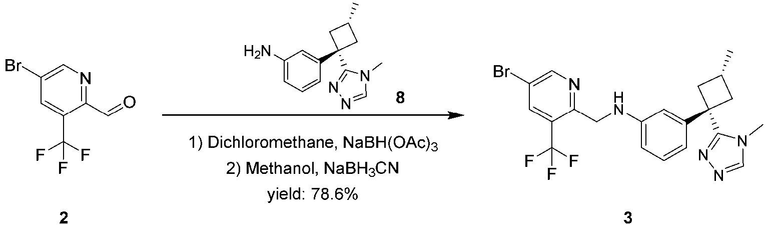

3-((1s,3s)-3-methyl-1-(4-methyl-4H-1,2,4-triazol-3-yl)cyclobutyl)aniline (140 mg, 578 µmol) and 5-bromo-3-(trifluoromethyl)pyridine-2-carbaldehyde (147 mg, 578 µmol) was dissolved in Dichloromethane (3.00 mL). The mixture was stirred at 25 °C under N₂ for 0.5 hr. Then NaBH(OAc)₃ (306 mg, 1.44 mmol) was added to the mixture, stirred at 25 °C for 2 hrs. Methanol (3.00 mL) and NaBH₃CN (36.3 mg, 578 µmol) was added to the mixture, stirred at 30 °C for 12 hrs. LCMS shows reactant was consumed completely and desired mass was detected. The reaction mixture was diluted with Dichloromethane 10.0 mL, washed with water 10.0 mL. The water phase extracted with Dichloromethane 20.0 mL (10.0 mL * 2). The combined organic layers were dried over Na₂SO₄, filtered and concentrated under reduced pressure to give a residue. The residue was purified by column chromatography (SiO2, Dichloromethane : Methanol = 100 : 0 to 25 : 1). TLC (Dichloromethane : Methanol = 20 : 1,product 1 R_{f} = 0.50). N-((5-bromo-3-(trifluoromethyl)pyridin-2-yl)methyl)-3-((1s,3s)-3-methyl-1-(4-methyl-4H-1,2,4-triazol-3-yl)cyclob utyl)aniline (220 mg, 454 µmol, 78.6% yield, 99.1% purity) was obtained as white solid, confirmed by H NMR and LCMS.
¹H NMR: (400 MHz, DMSO-*d*₆)
*δ* 8.92 (s, 1H), 8.44 (d, *J =* 2.0 Hz, 1H), 8.21 (s, 1H), 7.03 (t, *J =* 8.0 Hz, 1H), 6.53 (s, 1H), 6.42-6.51 (m, 2H), 6.26 (t, *J =* 5.6 Hz, 1H), 4.44 (d, *J =* 5.6 Hz, 2H), 3.06 (s, 3H), 2.67 (br s, 2H), 2.42 (d, *J =* 6.8 Hz, 3H), 1.03 (d, *J =* 5.2 Hz, 3H)
LCMS: m/z = 480.0 (M+H)⁺, Rt = 1.297 min

### 4. General steps for preparation of 6-bromo-2-(3-((1s,3s)-3-methyl-1-(4-methyl-4H-1,2,4-triazol-3-yl)cyclobutyl)phenyl)-8-(trifluoromethyl)imidazo[ 1,5-a]pyridin-3(2H)-one

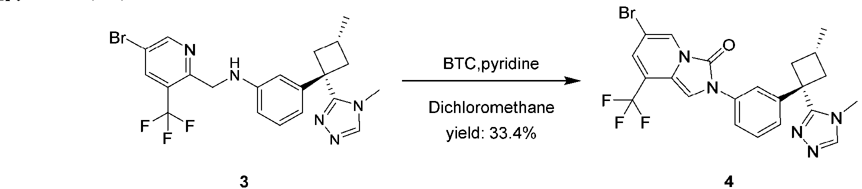

N-((5-bromo-3-(trifluoromethyl)pyridin-2-yl)methyl)-3-((1s,3s)-3-methyl-1-(4-methyl-4H-1,2,4-triazol-3-yl)c yclobutyl)aniline (240 mg, 500 µmol) and pyridine (119 mg, 1.50 mmol, 121 µL)was dissolved in Dichloromethane (6.00 mL). Bis(trichloromethyl) carbonate (74.1 mg, 250 µmol) in Dichloromethane (1.00 mL) was added to the mixture. The mixture was stirred at 20 °C for 12 hrs. LCMS shows reactant was consumed completely and desired mass was detected. The mixture was quenched with NaHCO₃ solution 10.0 mL, extracted with Dichloromethane 15.0 mL (5.00 mL * 3). The combined organic phase was dried over Na₂SO₄, concentrated under reduced pressure to give a residue. The residue was purified by reversed-phase HPLC (0 - 55% ACN/water). 6-bromo-2-(3-((1s,3s)-3-methyl-1-(4-methyl-4H-1,2,4-triazol-3-yl)cyclobutyl)phenyl)-8-(trifluoromethyl)imidazo[ 1,5-a]pyridin-3(2H)-one (85.0 mg, 167 µmol, 33.4% yield, 99.3% purity) was obtained as yellow solid, confirmed by LCMS and H NMR.
¹H NMR: (400 MHz, DMSO-*d*₆)
*δ* 8.29 (s, 1H), 8.03 (s, 1H), 7.82 (t, *J =* 1.6 Hz, 1H), 7.63 (dd, *J =* 8.0, 1.2 Hz, 1H), 7.46-7.55 (m, 2H), 7.26 (d, *J= 8.0* Hz, 1H), 7.16 (s, 1H), 3.20 (s, 3H), 2.87 (d, *J =* 3.6 Hz, 2H), 2.55 (br s, 1H), 2.52 (d, *J=* 2.0 Hz, 2H), 1.08 (d, *J =* 5.4 Hz, 3H)
LCMS: m/z = 506.0 (M+H)⁺, Rt = 1.328 min

### 5. General steps for preparation of 2-(3-((1s,3s)-3-methyl-1-(4-methyl-4H-1,2,4-triazol-3-yl)cyclobutyl)phenyl)-3-oxo-8-(trifluoromethyl)-2,3-dihydro imidazo[1,5-a]pyridine-6-carbaldehyde:

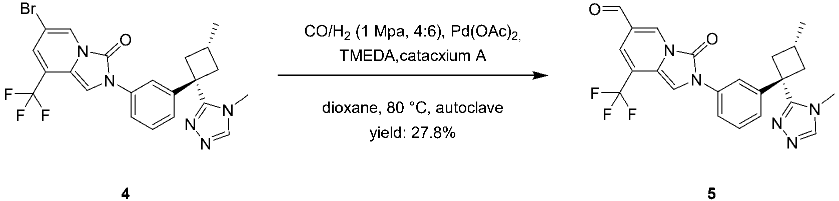

To a solution of 6-bromo-2-(3-((1s,3s)-3-methyl-1-(4-methyl-4H-1,2,4-triazol-3-yl)cyclobutyl)phenyl)-8-(trifluoromethyl)imidazo[ 1,5-a]pyridin-3(2H)-one (40.0 mg, 79.0 µmol) and TMEDA (27.5 mg, 237 µmol, 35.77 µL) in dioxane (4.00 mL) was added Pd(OAc)₂ (8.87 mg, 39.5 µmol) and bis(1-adamantyl)-butyl-phosphane (28.3 mg, 79.0 µmol) at 20 °C. The mixture was stirred at 80 °C in a 50 mL of autoclave under CO/H₂ (0.4 Mpa/0.6 Mpa) for 16 hrs. LCMS shows reactant was consumed completely and desired mass was detected. The mixture was concentrated under reduced pressure to give a residue. The residue was purified by column chromatography (SiO₂, Dichloromethane : Methanol = 100 : 0 to 93 : 7). TLC (Dichloromethane : Methanol = 20 : 1,product 1 R_{f} = 0.3). 2-(3-((1s,3s)-3-methyl-1-(4-methyl-4H-1,2,4-triazol-3-yl)cyclobutyl)phenyl)-3-oxo-8-(trifluoromethyl)-2,3-dihydro imidazo[1,5-a]pyridine-6-carbaldehyde (10.0 mg, 22.0 µmol, 27.8% yield) was obtained as yellow solid, confirmed by H NMR.
¹H NMR: (400 MHz, CDCl₃)
*δ* 9.70 (s, 1H), 8.30 (s, 1H), 7.99 (s, 1H), 7.75 (s, 1H), 7.48-7.55 (m, 2H), 7.34-7.40 (m, 2H), 6.87 (s, 1H), 3.27 (s, 3H), 2.88-2.95 (m, 2H), 2.64-2.75 (m, 3H), 1.17 (d, *J =* 6.0 Hz, 3H)

### 6. General steps for preparation of 2-(3-((1s,3R)-3-methyl-1-(4-methyl-4H-1,2,4-triazol-3-yl)cyclobutyl)phenyl)-6-(((S)-3-methylpiperidin-1-yl)methy l)-8-(trifluoromethyl)imidazo[1,5-a]pyridin-3(2H)-one (compound T140)

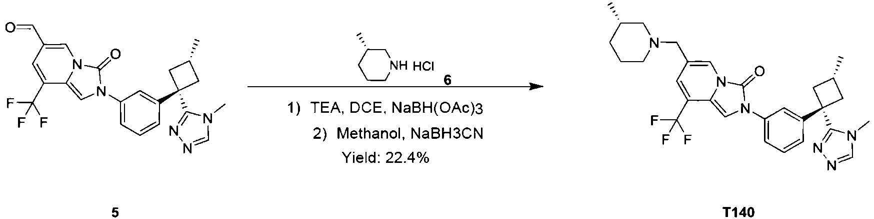

TEA (4.44 mg, 43.9 µmol, 6.11 µL) in DCE 0.50 mL was added to the mixture of 2-(3-((1s,3s)-3-methyl-l-(4-methyl-4H-1,2,4-triazol-3-yl)cyclobutyl)phenyl)-3-oxo-8-(trifluoromethyl)-2,3-dihydro imidazo[1,5-a]pyridine-6-carbaldehyde (10.0 mg, 22.0 µmol) and rac-(3S)-3-methylpiperidine (5.96 mg, 43.9 µmol, HCl) in DCE 0.50 mL. The mixture was stirred at 25 °C for 0.5 hr. NaBH(OAc)₃ (9.31 mg, 43.9 µmol) was added to the mixture, then stirred at 25 °C for 12 hrs. NaBH₃CN (6.90 mg, 110 µmol) and Methanol (1.00 mL) was added to the mixture, stirred at20 °C for 6 hrs. LCMS shows reactant was consumed completely and desired mass was detected. The mixture was quenched with water 2.0 mL, extracted with Dichloromethane 6.00 mL (2.00 mL * 3). Combined organic phase was dried over Na₂SO₄, filtered and concentrated under reduced pressure to give a residue. The residue was purified by prep.HPLC (column: Welch Xtimate C18 40 * 200 mm 7 um;mobile phase: [water ( NH₄HCO₃)-ACN];gradient: 42%-82% B over 25 min). 2-(3-((1s,3R)-3-methyl-1-(4-methyl-4H-1,2,4-triazol-3-yl)cyclobutyl)phenyl)-6-(((S)-3-methylpiperidin-1-yl)methy l)-8-(trifluoromethyl)imidazo[1,5-a]pyridin-3(2H)-one (2.80 mg, 4.91 µmol, 22.4% yield, 94.5% purity) was obtained as yellow solid, confirmed by H NMR and LCMS.
¹H NMR: (400 MHz, CDCl₃)
*δ* 6.79 (br s, 1H), 3.26 (s, 3H), 2.80-2.98 (m, 3H), 2.70 (d, *J =* 8.0 Hz, 4H), 2.02 (d, *J =* 6.4 Hz, 2H), 1.78 (d, *J* = 7.2 Hz, 2H), 1.24-1.39 (m, 3H), 1.16 (d, *J =* 5.6 Hz, 3H), 0.90 (d, *J =* 6.0 Hz, 5H)
LCMS: m/z = 539.3 (M+H)⁺, Rt = 1.564 min

### Example 141: Synthesis of compound T141

### 1. General steps for preparation of tert-butyl (2R)-2-methyl-4-[[6-[3-[3-methyl-1-(4-methyl-1,2,4-triazol-3-yl)cyclobutyl]phenyl]-7-oxo-4-(trifluoromethyl)-1H-pyrrolo[2,3-c]pyridin-2-yl]methyl]piperazine-1-carboxylate (compound T141)

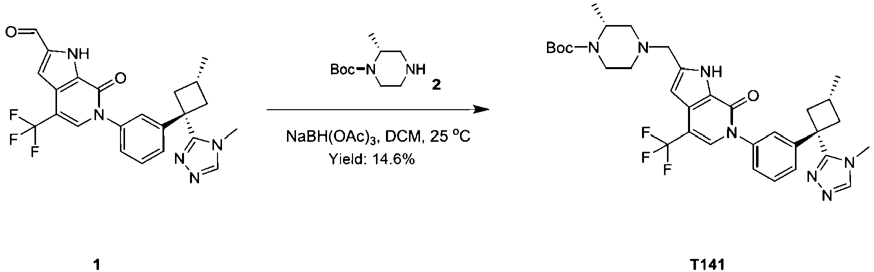

6-[3-[3-methyl-1-(4-methyl-1,2,4-triazol-3-yl)cyclobutyl]phenyl]-7-oxo-4-(trifluoromethyl)-1H-pyrrolo [2,3-c] pyridine-2-carbaldehyde (45.0 mg, 98.8 µmol) was dissolved in Dichloromethane (1.00 mL). pH of the mixture was adjusted to 7 with TEA. Tert-butyl (2R)-2-methylpiperazine-1-carboxylate (29.7 mg, 148 µmol) was added to the mixture and stirred at 25 °C for 0.5 hrs. NaBH(OAc)₃ (52.4 mg, 247 µmol) was added to the mixture and stirred at 25 °C for 1 hrs. LCMS shows reactant was consumed completely and desired mass was detected. The mixture was diluted with dichloromethane 6.00 mL, washed with sat. NaHCO₃ solution 2.00 mL, dried over Na₂SO₄, filtered and concentrated under reduced pressure to give a residue. The residue was purified by prep.HPLC (column: Welch Xtimate C18 40 * 200 mm 7 um;mobile phase: [water (NH₄HCO₃)-ACN];gradient:38%-78% B over 25 min). Tert-butyl (2R)-2-methyl-4-[[6-[3-[3-methyl-1-(4-methyl-1,2,4-triazol-3-yl)cyclobutyl]phenyl]-7-oxo-4-(trifluoromethyl)-1H-pyrrolo[2,3-c]pyridin-2-yl]methyl]piperazine-1-carboxylate (9.30 mg, 14.4 µmol, 14.6% yield, 99.0% purity) was obtained as white solid, confirmed by H NMR and LCMS.
¹H NMR: (400 MHz, DMSO-*d*₆)
*δ* 12.47 (br s, 1H), 8.28 (s, 1H), 7.72 (d, *J =* 1.2 Hz, 1H), 7.46-7.57 (m, 2H), 7.35 (t, *J =* 8.4 Hz, 2H), 6.30 (s, 1H), 4.08 (br d, *J =* 4.0 Hz, 1H), 3.58-3.73 (m, 3H), 3.25 (s, 3H), 2.95-3.04 (m, 1H), 2.88 (br d, *J =* 3.6 Hz, 2H), 2.76-2.83 (m, 1H), 2.65 (br d, *J =* 11.2 Hz, 1H), 2.53 (br d, *J =* 6.8 Hz, 3H), 2.08 (dd, *J =* 11.2, 3.6 Hz, 1H), 1.92 (td, *J* = 11.6, 3.2 Hz, 1H), 1.38 (s, 9H), 1.16 (d, *J* = 6.8 Hz, 3H), 1.07 (br d, *J* = 5.2 Hz, 3H)
LCMS: m/z = 640.3 (M+H)⁺, Rt = 1.798 min

### Example 142: Synthesis of compound T142

### 1. General steps for preparation of 4-iodo-7-methoxy-1-(p-tolylsulfonyl)pyrrolo[2.3-c]pyridine

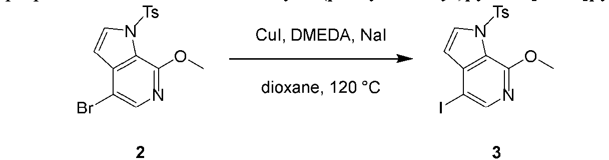

To a solution of 4-bromo-7-methoxy-1-(p-tolylsulfonyl)pyrrolo[2,3-c]pyridine (100 g, 262 mmol) , CuI (29.9 g, 157 mmol) ,NaI (235 g, 1.57 mol) in dioxane (1000 mL) was added DMEDA (23.1 g, 262 mmol, 28.2 mL). The mixture was stirred under N₂ at 120 °C for 12 hrs. LCMS showed the reaction was completed. TLC (Plate 1, Petroleum ether/ Ethyl acetate = 5/ 1, UV 254 nm, R_{f} (product) = 0.3). The reaction mixture was diluted with NH₄Cl (1000 mL). The mixture was filtered and the filter cake was washed with Ethyl acetate (1000 mL * 3). The filtrate was washed with brine (1000 mL), dried over Na₂SO₄. The organic phase was concentrated in vacuum. The crude product was triturated with MTBE at 25 °C for 12 hrs. Compound 4-iodo-7-methoxy-1-(p-tolylsulfonyl)pyrrolo[2,3-c]pyridine (85.0 g, 198 mmol, 75.6% yield) was obtained as a yellow solid. It was confirmed by H NMR.
¹H NMR: (400 MHz, CD₃Cl)
*δ* 8.06 (s, 1H), 7.99 (d, *J =* 3.6 Hz, 1H), 7.78 (d, *J=* 8.4 Hz, 2H), 7.30 (d, *J =* 8.0 Hz, 2H), 6.60 (d, *J=* 3.6 Hz, 1H), 3.89 (s, 3H), 2.42 (s, 3H)

### 2. General steps for preparation of 7-methoxy-1-(p-tolylsulfonyl)-4-(trifluoromethyl)pyrrolo[2,3-c]pyridine-2-carbaldehyde

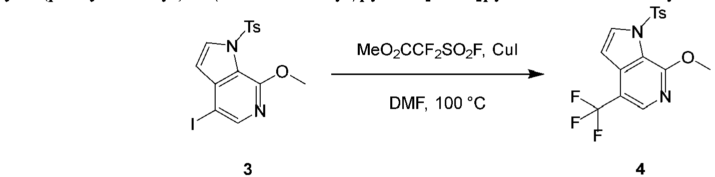

To a solution of 4-iodo-7-methoxy-1-(p-tolylsulfonyl)pyrrolo[2,3-c]pyridine (65.0 g, 151 mmol) in DMF (650 mL) was added methyl 2,2-difluoro-2-fluorosulfonyl-acetate (174 g, 910 mmol, 115 mL), CuI (115 g, 607 mmol). The mixture was stirred under N₂ at 100 °C for 3 hrs. LCMS showed the reaction was completed. The reaction mixture was diluted with Ethyl acetate (100 mL). The mixture was filtered and the filter cake was washed with Ethyl acetate (100 mL * 3). The filtrate was washed with brine (100 mL), dried over Na₂SO₄. The organic phase was concentrated in vacuum. The residue was purified by column chromatography (SiO₂, Petroleum ether/ Ethyl acetate = 1/ 0 to 95/ 5). TLC (Plate 1, Petroleum ether/ Ethyl acetate = 5/ 1, UV 254 nm, R_{f} (product) = 0.25). Compound 7-methoxy-1-(p-tolylsulfonyl)-4-(trifluoromethyl)pyrrolo[2,3-c]pyridine (45.0 g, 119 mmol, 78.4% yield, 98.0% purity) was obtained as a white solid. It was confirmed by H NMR.
¹H NMR: (400 MHz, CD₃Cl)
*δ* 8.13 (s, 1H), 8.03-8.07 (m, 1H), 7.80 (d, *J =* 8.4 Hz, 2H), 7.32 (d, *J =* 8.4 Hz, 2H), 6.79 (dd, *J =* 3.6, 1.4 Hz, 1H), 3.96 (s, 3H), 2.41-2.45 (m, 4H)

### 3. General steps for preparation of 7-methoxy-1-(p-tolylsulfonyl)-4-(trifluoromethyl)pyrrolo[2,3-c]pyridine-2-carbaldehyde

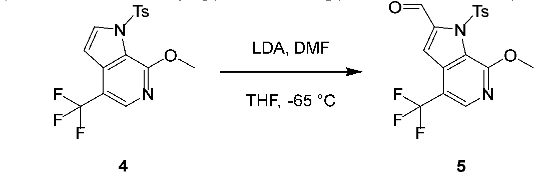

To a solution of 7-methoxy-1-(p-tolylsulfonyl)-4-(trifluoromethyl)pyrrolo[2,3-c]pyridine (5.00 g, 13.5 mmol) in THF (50.0 mL) was added LDA (2 M, 10.2 mL) at -65 °C under N₂. The mixture was stirred under N₂ at -65 °C for 0.5 hr. Then a solution of DMF (2.00 g, 27.2 mmol, 2.10 mL) in THF (10.0 mL) was added to the mixture at -65 °C under N₂. The mixture was stirred at -65 °C under N₂ for 2 hrs. LCMS showed the reaction was completed. The reaction mixture was poured into saturated aqueous NH₄Cl (100 mL) slowly. The mixture was extracted with Ethyl acetate (100 * 3 mL), dried over Na₂SO₄. The organic phase was concentrated in vacuum. The residue was purified by column chromatography (SiO₂, Petroleum ether/ Dichloromethane = 1/ 0 to 55/ 45). TLC (Plate 1, Petroleum ether/ Dichloromethane = 1/ 1, UV 254 nm, R_{f} (product) = 0.3). Compound 7-methoxy-1-(p-tolylsulfonyl)-4-(trifluoromethyl)pyrrolo[2,3-c]pyridine-2-carbaldehyde (1.10 g, 2.76 mmol, 20.4% yield) was obtained as a yellow solid.

### 4. General steps for preparation of 7-oxo-1-(p-tolylsulfonyl)-4-(trifluoromethyl)-6H-pyrrolo[2,3-c]pyridine-2-carbaldehyde

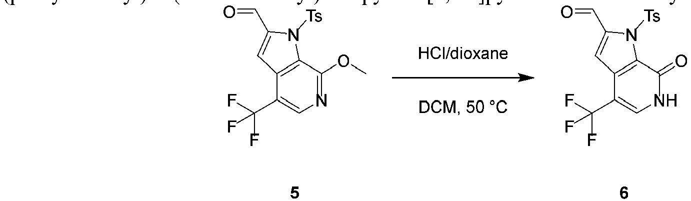

To a solution of 7-methoxy-1-(p-tolylsulfonyl)-4-(trifluoromethyl)pyrrolo[2,3-c]pyridine-2-carbaldehyde (1.00 g, 2.51 mmol) in DCM (2.00 mL) was added HCl/ dioxane (2 M, 10.0 mL). The mixture was stirred under N₂ at *50* °C for 1 hr. LCMS showed the reaction was completed. TLC ( Plate 1, Petroleum ether/ Ethyl acetate = 2/ 1, UV 254 nm, R_{f} (product) =0.4). The reaction mixture was concentrated under reduced pressure to give a residue. Compound 7-oxo-1-(p-tolylsulfonyl)-4-(trifluoromethyl)-6H-pyrrolo[2,3-c]pyridine-2-carbaldehyde (939 mg, 2.44 mmol, 97.3% yield) was obtained as a white solid.

### 5. General steps for preparation of 2-(1,3-dioxolan-2-yl)-1-(p-tolylsulfonyl)-4-(trifluoromethyl)-6H-pyrrolo[2,3-c]pyridin-7-one

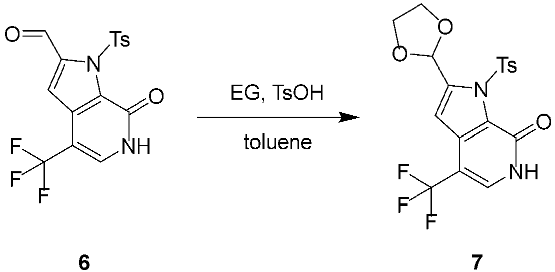

To a solution of 7-oxo-1-(p-tolylsulfonyl)-4-(trifluoromethyl)-6H-pyrrolo[2,3-c]pyridine-2-carbaldehyde (939 mg, 2.44 mmol), EG (5.57 g, 89.6 mmol, 5.00 mL) was added TsOH·H₂O (92.95 mg, 488.64 µmol). The mixture was stirred at 130 °C under N₂ for 1 hr. LCMS showed the reaction was completed. The mixture was quenched with aqueous NaHCO₃ (10.0 mL) and extracted with DCM. The combined organic were rinsed with brine and then dried over Na₂SO₄. The residue was purified by column chromatography (SiO₂, Dichloromethane/ Ethyl acetate = 1/ 0 to 90/ 10). TLC (Plate 1, Dichloromethane/ Ethyl acetate = 1/ 1, UV 254 nm, R_{f} (product) = 0.5). Compound 2-(1,3-dioxolan-2-yl)-1-(p-tolylsulfonyl)-4-(trifluoromethyl)-6H-pyrrolo[2,3-c]pyridin-7-one (442 mg, 1.03 mmol, 42.2% yield) was obtained as a white solid. It was confirmed by H NMR.
¹H NMR: (400 MHz, CD3Cl)
*δ* 11.12 (br dd, *J =* 7.6, 2.4 Hz, 1H), 8.22 (d, *J =* 8.4 Hz, 2H), 7.29-7.36 (m, 3H), 6.87 (s, 1H), 6.81 (s, 1H), 4.07-4.19 (m, 4H), 2.42 (s, 3H)

### 6. General steps for preparation of 2-(1,3-dioxolan-2-yl)-6-[3-[3-methyl-1-(4-methyl-1,2,4-triazol-3-yl)cyclobutyl]phenyl]-1-(p-tolylsulfonyl)-4-(triflu oromethyl)pyrrolo[2,3-c]pyridin-7-one

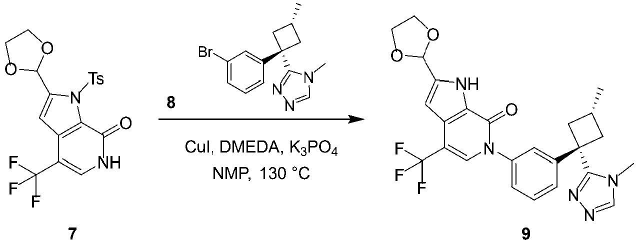

To a solution of 2-(1,3-dioxolan-2-yl)-1-(p-tolylsulfonyl)-4-(trifluoromethyl)-6H-pyrrolo[2,3-c]pyridin-7-one (400 mg, 933 µmol), 3-[1-(3-bromophenyl)-3-methyl-cyclobutyl]-4-methyl-1,2,4-triazole (343 mg, 1.12 mmol), CuI (355 mg, 1.87 mmol), K₃PO₄ (594 mg, 2.80 mmol) in NMP (4.00 mL) was added DMEDA (82.3 mg, 933 µmol, 100 µL). The mixture was stirred under N₂ at 130 °C for 1 hrs. LCMS showed the reaction was completed. The mixture was extracted with Ethyl acetate (300 * 3 mL), brine (300 mL), dried over Na₂SO₄. The organic phase was concentrated in vacuum. The crude product was purified by reversed-phase HPLC(base). Compound 2-(1,3-dioxolan-2-yl)-6-[3-[3-methyl-1-(4-methyl-1,2,4-triazol-3-yl)cyclobutyl]phenyl]-1-(p-tolylsulfonyl)-4-(triflu oromethyl)pyrrolo[2,3-c]pyridin-7-one (202 mg, 185 µmol, 19.8% yield, 60.0% purity) was obtained as a white solid. It was confirmed by LCMS.
LCMS: m/z = 500.1 (M+H)⁺, Rt = 3.068 min

### 7. General steps for preparation of 6-[3-[3-methyl-1-(4-methyl-1,2,4-triazol-3-yl)cyclobutyl]phenyl]-7-oxo-1-(p-tolylsulfonyl)-4-(trifluoromethyl)pyrr olo[2,3-c]pyridine-2-carbaldehyde

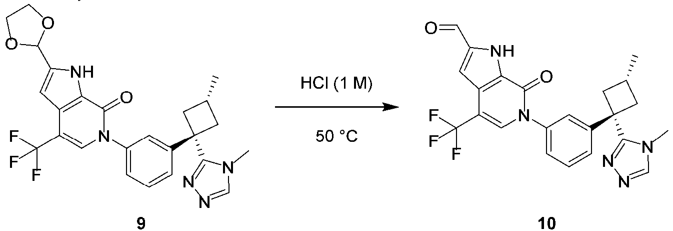

To a solution of 2-(1,3-dioxolan-2-yl)-6-[3-[3-methyl-1-(4-methyl-1,2,4-triazol-3-yl)cyclobutyl]phenyl]-1-(p-tolylsulfonyl)-4-(triflu oromethyl)pyrrolo[2,3-c]pyridin-7-one (180 mg, 275 µmol) was added HCl (1 M, 4.50 mL). The mixture was stirred at 50 °C under N₂ for 3 hrs. LCMS showed the reaction was completed. The reaction mixture was concentrated under reduced pressure to give a residue. Compound 6-[3-[3-methyl-1-(4-methyl-1,2,4-triazol-3-yl)cyclobutyl]phenyl]-7-oxo-1-(p-tolylsulfonyl)-4-(trifluoromethyl)pyrr olo[2,3-c]pyridine-2-carbaldehyde (167 mg, 275 µmol, 100% yield) was obtained as a yellow solid.

### 8. General steps for preparation of tert-butyl (2R)-2-methyl-4-[[6-[3-[3-methyl-1-(4-methyl-1,2,4-triazol-3-yl)cyclobutyl]phenyl]-7-oxo-1-(p-tolylsulfonyl)-4-(tr ifluoromethyl)pyrrolo[2,3-c]pyridin-2-yl]methyl]piperazine-1-carboxylate-N

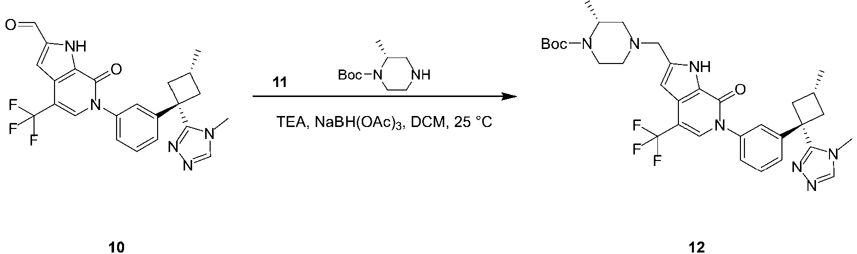

To a solution of 6-[3-[3-methyl-1-(4-methyl-1,2,4-triazol-3-yl)cyclobutyl]phenyl]-7-oxo-1-(p-tolylsulfonyl)-4-(trifluoromethyl)pyrr olo[2,3-c]pyridine-2-carbaldehyde (167 mg, 275 µmol) in DCM (5.00 mL) was added TEA (27.8 mg, 275 µmol, 38.3 µL) adjust pH = 7. The mixture was added tert-butyl (2R)-2-methylpiperazine-1-carboxylate (82.7 mg, 413 µmol) and stirred under N₂ at 25 °C for 0.5 hr. NaBH(OAc)₃ (145 mg, 688 µmol) was added to the mixture. The mixture was stirred under N₂ at 25 °C for 1 hrs. LCMS showed the reaction was completed. The mixture was extracted with Ethyl acetate (10.0 * 3 mL), brine (10.0 mL), dried over Na₂SO₄. The organic phase was concentrated in vacuum. Compound tert-butyl (2R)-2-methyl-4-[[6-[3-[3-methyl-1-(4-methyl-1,2,4-triazol-3-yl)cyclobutyl]phenyl]-7-oxo-1-(p-tolylsulfonyl)-4-(tr ifluoromethyl)pyrrolo[2,3-c]pyridin-2-yl]methyl]piperazine-1-carboxylate (200 mg, 251 µmol, 91.4% yield) was obtained as a yellow solid.

### 9. General steps for preparation of 6-[3-[3-methyl-1-(4-methyl-1,2,4-triazol-3-yl)cyclobutyl]phenyl]-2-[[(3R)-3-methylpiperazin-1-yl]methyl]-4-(triflu oromethyl)-1H-pyrrolo[2,3-c]pyridin-7-one (compound T142)

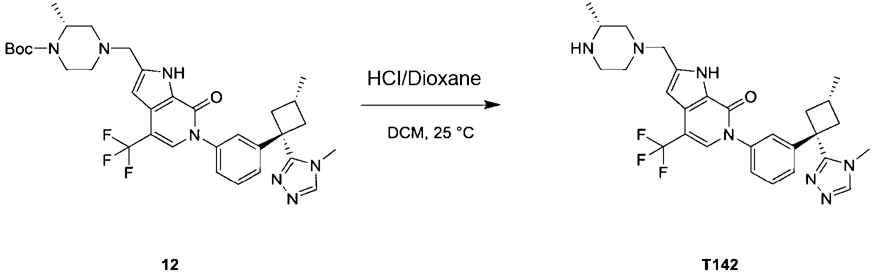

To a solution of tert-butyl (2R)-2-methyl-4-[[6-[3-[3-methyl-1-(4-methyl-1,2,4-triazol-3-yl)cyclobutyl]phenyl]-7-oxo-1-(p-tolylsulfonyl)-4-(tr ifluoromethyl)pyrrolo[2,3-c]pyridin-2-yl]methyl]piperazine-1-carboxylate (200 mg, 251 µmol) in DCM (2.00 mL) was added HCl/ dioxane (2 M, 5.00 mL). The mixture was stirred under N₂ at 25 °C for 1 hr. LCMS showed the reaction was completed. The mixture was extracted with Ethyl acetate (10.0 * 3 mL), brine (10.0 mL), dried over Na₂SO₄. The organic phase was concentrated in vacuum. The residue was purified by prep.HPLC (column: Welch Xtimate C18 40 * 200 mm 7 um;mobile phase: [water (HCl) - ACN];gradient: 0%-38% B over 20 min). 6-[3-[3-methyl-1-(4-methyl-1,2,4-triazol-3-yl)cyclobutyl]phenyl]-2-[[(3R)-3-methylpiperazin-1-yl]methyl]-4-(triflu oromethyl)-1H-pyrrolo[2,3-c]pyridin-7-one (11.4 mg, 20.7 µmol, 5.59% yield, 98.0% purity) was obtained as white solid, confirmed by H NMR and LCMS.
¹H NMR: (400 MHz, DMSO-*d6*)
*δ* 12.86 (s, 1H), 10.16 (br s, 1H), 10.02 (br s, 1H), 9.39 (s, 1H), 7.85 (s, 1H), 7.62 (s, 1H), 7.59 (d, *J =* 8.0 Hz, 1H), 7.49-7.55 (m, 1H), 7.46 (br d, *J =* 7.6 Hz, 1H), 6.81 (s, 1H), 4.40-4.62 (m, 1H), 4.49 (br s, 2H), 3.53 (br d, *J =* 11.6 Hz, 5H), 3.25-3.42 (m, 3H), 3.14 (br t, *J =* 12.0 Hz, 1H), 2.93-3.01 (m, 2H), 2.61 (br d, *J =* 10.4 Hz, 2H), 2.53-2.59 (m, 1H), 1.32 (br d, *J =* 6.4 Hz, 3H), 1.09 (d, *J=* 6.0 Hz, 3H)
LCMS: m/z = 540.3 (M+H)⁺, Rt = 1.477 min

### Examples 143 and 144: Synthesis of compounds T143& T144

### 1. General steps for preparation of 2-((3-azabicyclo[3.1.0]hexan-3-yl)methyl)-6-(3-((1s,3s)-3-methoxy-1-(4-methyl-4H-1,2,4-triazol-3-yl)cyclobutyl)p henyl)-4-(trifluoromethyl)-1,6-dihydro-7H-pyrrolo[2,3-c]pyridin-7-one (compound T143) and 2-((3-azabicyclo[3.1.0]hexan-3-yl)methyl)-6-(3-((1r,3r)-3-methoxy-1-(4-methyl-4H-1,2,4-triazol-3-yl)cyclobutyl)p henyl)-4-(trifluoromethyl)-1,6-dihydro-7H-pyrrolo[2,3-c]pyridin-7-one (compound T144)

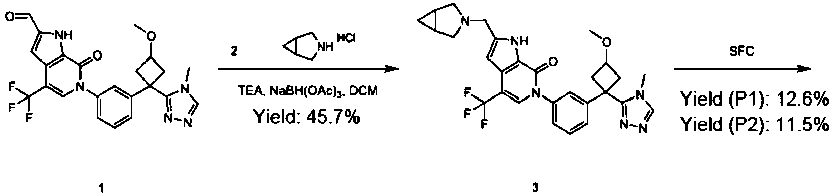

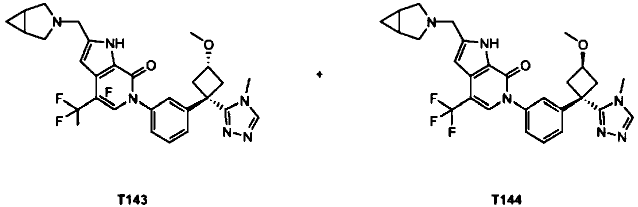

To a solution of 6-(3-(3-methoxy-1-(4-methyl-4H-1,2,4-triazol-3-yl)cyclobutyl)phenyl)-7-oxo-4-(trifluoromethyl)-6,7-dihydro-1H-pyrrolo[2,3-c]pyridine-2-carbaldehyde (150 mg, 318 µmol) in dichloromethane (2.00 mL) was added dropwise TEA (32.2 mg, 318 µmol, 44.2 µL) adjust pH = 7, 3-azabicyclo[3.1.0]hexane (57.0 mg, 477 µmol, HCl) was added to the mixture, stirred at 25 °C for 0.5 hr, then NaBH(OAc)₃ (168 mg, 795 µmol) was added to the mixture, stirred at 25 °C for 1 hr. LCMS showed desired compound was detected. The reaction mixture was diluted with aqueous NaHCO₃ (8.00 mL), extracted with dichloromethane (12.0 mL * 3), the combined organic layers were washed with H₂O (8.00 mL), dried over Na₂SO₄, filtered and concentrated under reduced pressure to give a residue. The residue was purified by Prep-HPLC (column: Welch Xtimate C18 40 * 200 mm 7 um; mobile phase: [water (NH₄HCO₃)-ACN]; gradient:26.0%-66.0% B over 25 mins). Separated by SFC (column: DAICEL CHIRALPAK IC(250mm*30mm,10um);mobile phase: [CO2-ACN/EtOH(0.1% NH3H2O)];B%:55%, isocratic elution mode) to give T143 2-((3-azabicyclo[3.1.0]hexan-3-yl)methyl)-6-(3-((1s,3s)-3-methoxy-1-(4-methyl-4H-1,2,4-triazol-3-yl)cyclobutyl)p henyl)-4-(trifluoromethyl)-1,6-dihydro-7H-pyrrolo[2,3-c]pyridin-7-one (22.2 mg, 40.3 µmol, 12.6% yield, 97.8% purity) (Rt = 1.234 min) and T144 2-((3-azabicyclo[3.1.0]hexan-3-yl)methyl)-6-(3-((1r,3r)-3-methoxy-1-(4-methyl-4H-1,2,4-triazol-3-yl)cyclobutyl)p henyl)-4-(trifluoromethyl)-1,6-dihydro-7H-pyrrolo[2,3-c]pyridin-7-one (20.0 mg, 36.8 µmol, 11.5% yield, 99.3% purity) (Rt = 2.015 min) both as white solid. T143 was confirmed by H NMR and LCMS.
¹H NMR: (DMSO-*d*₆, 400 MHz)
*δ* 12.18-12.63 (m, 1H), 8.38 (s, 1H), 7.71 (d, *J=* 1.2 Hz, 1H), 7.45-7.54 (m, 1H), 7.32-7.40 (m, 2H), 7.18 (d, *J* = 8.0 Hz, 1H), 6.23 (s, 1H), 3.85 (t, *J =* 7.2 Hz, 1H), 3.71 (s, 2H), 3.29 (s, 3H), 3.16 (s, 3H), 2.87 (d, *J =* 8.4 Hz, 2H), 2.54 (s, 2H), 2.36 (d, *J =* 8.0 Hz, 2H), 1.31-1.36 (m, 2H), 1.23 (s, 2H), 0.66 (q, *J =* 3.6 Hz, 1H), 0.30 (td, *J=* 7.6, 3.6 Hz, 1H)
LCMS: m/z =539.3 (M+H)⁺, Rt = 1.317 min

T144 was confirmed by H NMR and LCMS.
¹H NMR: (DMSO-*d*₆, 400 MHz)
*δ* 12.31-12.55 (m, 1H), 8.30 (s, 1H), 7.73 (s, 1H), 7.51-7.55 (m, 1H), 7.50 (s, 1H), 7.36-7.39 (m, 1H), 7.33 (d, *J= 8.0* Hz, 1H), 6.10-6.37 (m, 1H), 4.06 (quin, *J* = 7.2 Hz, 1H), 3.55-3.85 (m, 2H), 3.27 (s, 3H), 3.15 (s, 3H), 3.10 (ddd, *J =* 10.0, 7.2, 2.4 Hz, 2H), 2.88 (d, *J =* 2.4 Hz, 1H), 2.75-2.81 (m, 2H), 2.29-2.48 (m, 2H), 1.29-1.53 (m, 2H), 1.23 (s, 1H), 0.68 (s, 1H), 0.16-0.43 (m, 1H)
LCMS: m/z =539.2 (M+H)⁺, Rt = 1.297 min

### Examples 145 and 146: Synthesis of compounds T145& T146

### 1. General steps for preparation of 2-(3-azabicyclo[3.2.1]octan-3-ylmethyl)-6-[3-[3-methoxy-1-(4-methyl-1,2,4-triazol-3-yl)cyclobutyl]phenyl]-4-(trifl uoromethyl)-1H-pyrrolo[2,3-c]pyridin-7-one

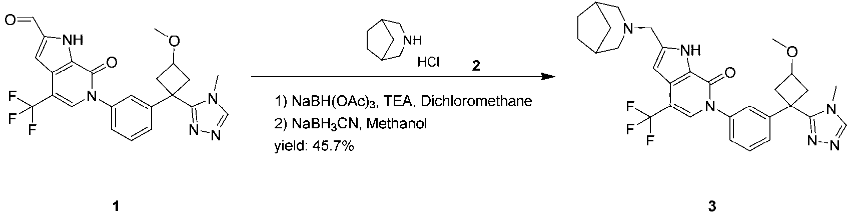

3-azabicyclo[3.2.1]octane (62.6 mg, 424 µmol, HCl) was added to the mixture of 6-[3-[3-methoxy-1-(4-methyl-1,2,4-triazol-3-yl)cyclobutyl]phenyl]-7-oxo-4-(trifluoromethyl)-1H-pyrrolo[2,3-c]pyr idine-2-carbaldehyde (100 mg, 212 µmol) and TEA (42.9 mg, 424 µmol, 59.0 µL) in Dichloromethane (3.00 mL). The mixture was stirred at 25 °C under N₂ for 0.5 hr. NaBH(OAc)₃ (112 mg, 530 µmol) was added to the mixture, stirred at 35 °C under N₂. for 5 hrs. Methanol (1.50 mL) and NaBH₃CN (26.7 mg, 424 µmol) was added to the mixture at 25 °C, the mixture was stirred at 35 °C under N₂ for 12 hrs. LCMS shows reactant was consumed completely and desired mass was detected. The reaction mixture was diluted with Dichloromethane 6.00 mL, washed with sat. NaHCO₃ solution 3.00 mL. The water phase extracted with Dichloromethane 12.0 mL (6.00 mL * 2) and Ethyl acetate 12.0 mL (6.00 mL * 2). The combined organic layers were washed, dried over Na₂SO₄, filtered and concentrated under reduced pressure to give a residue. The mixture was purified by Prep-HPLC (column: Welch Xtimate C18 40 * 200 mm 7um;mobile phase: [water (FA) - ACN];gradient: 0%-38% B over 25 min). 2-(3-azabicyclo[3.2.1]octan-3-ylmethyl)-6-[3-[3-methoxy-1-(4-methyl-1,2,4-triazol-3-yl)cyclobutyl]phenyl]-4-(trifl uoromethyl)-1H-pyrrolo[2,3-c]pyridin-7-one (55 mg, 96.97 µmol, 45.7% yield, 99.9% purity) was obtained as white solid, confirmed by H NMR and LCMS.
¹H NMR: (400 MHz, DMSO-*d*₆)
*δ* 10.48-10.89 (m, 1H), 7.96-8.12 (m, 1H), 7.53-7.56 (m, 1H), 7.46-7.52 (m, 1H), 7.34-7.45 (m, 1H), 7.22-7.34 (m, 2H), 6.36 (s, 1H), 3.86-4.17 (m, 1H), 3.63 (s, 2H), 3.35 (ddd, J = 9.6, 6.8, 2.8 Hz, 1H), 3.22 (dd, J = 6.4, 3.6 Hz, 6H), 3.03-3.11 (m, 1H), 2.83-2.91 (m, 1H), 2.62-2.66 (m, 2H), 2.50-2.58 (m, 3H), 2.16 (br d, J = 10.4 Hz, 2H), 1.68 (br d, J = 7.2 Hz, 2H), 1.53-1.59 (m, 2H), 1.43-1.50 (m, 1H), 1.33-1.39 (m, 1H)
LCMS: m/z = 567.3 (M+H)⁺, Rt =2.133 min;2.176 min

### 2. General steps for preparation of 2-((3-azabicyclo[3.2.1]octan-3-yl)methyl)-6-(3-((1s,3s)-3-methoxy-1-(4-methyl-4H-1,2,4-triazol-3-yl)cyclobutyl)p henyl)-4-(trifluoromethyl)-1,6-dihydro-7H-pyrrolo[2,3-c]pyridin-7-one (compound T145) and 2-((3-azabicyclo[3.2.1]octan-3-yl)methyl)-6-(3-((1r,3r)-3-methoxy-1-(4-methyl-4H-1,2,4-triazol-3-yl)cyclobutyl)ph enyl)-4-(trifluoromethyl)-1,6-dihydro-7H-pyrrolo[2,3-c]pyridin-7-one (compound T146)

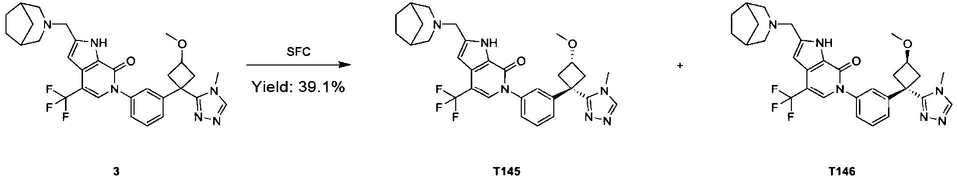

2-(3-azabicyclo[3.2.1]octan-3-ylmethyl)-6-[3-[3-methoxy-1-(4-methyl-1,2,4-triazol-3-yl)cyclobutyl]phenyl]-4 -(trifluoromethyl)-1H-pyrrolo[2,3-c]pyridin-7-one (55.0 mg, 97.1 µmol) was purified by SFC (column: DAICEL CHIRALPAK IG (250mm*30mm,10um);mobile phase: [CO₂-EtOH(0.1% NH₃H₂O)];B%: 60%, isocratic elution mode).
2-((3-azabicyclo[3.2.1]octan-3-yl)methyl)-6-(3-((1s,3s)-3-methoxy-1-(4-methyl-4H-1,2,4-triazol-3-yl)cyclobutyl)p henyl)-4-(trifluoromethyl)-1,6-dihydro-7H-pyrrolo[2,3-c]pyridin-7-one (21.6 mg, 37.9 µmol, 39.1% yield, 99.5% purity) was obtained as white solid, confirmed by H NMR and LCMS.
¹H NMR: (400 MHz, DMSO-*d*₆)
*δ* 12.40 (br s, 1H), 8.38 (s, 1H), 7.71 (d, J = 1.6 Hz, 1H), 7.46-7.53 (m, 1H), 7.32-7.41 (m, 2H), 7.18 (d, J = 8.0 Hz, 1H), 6.25 (s, 1H), 3.85 (quin, J = 7.2 Hz, 1H), 3.59 (s, 2H), 3.35 (br d, J = 2.8 Hz, 1H), 3.30-3.32 (m, 1H), 3.30 (s, 3H), 3.16 (s, 3H), 2.61 (br d, J = 7.2 Hz, 2H), 2.54 (br d, J = 2.4 Hz, 2H), 2.04-2.13 (m, 4H), 1.63 (br d, J = 6.4 Hz, 2H), 1.47-1.55 (m, 2H), 1.39 (br d, J = 4.4 Hz, 1H), 1.26 (br s, 1H)
LCMS: m/z = 567.3 (M+H)⁺, Rt =1.423 min

2-((3-azabicyclo[3.2.1]octan-3-yl)methyl)-6-(3-((1r,3r)-3-methoxy-1-(4-methyl-4H-1,2,4-triazol-3-yl)cyclobut yl)phenyl)-4-(trifluoromethyl)-1,6-dihydro-7H-pyrrolo[2,3-c]pyridin-7-one (16.8 mg, 29.50 µmol, 30.4% yield, 99.5% purity) was obtained as white solid, confirmed by H NMRand LCMS.
¹H NMR: (400 MHz, DMSO-*d*₆)
*δ* 12.41 (s, 1H), 8.30 (s, 1H), 7.72 (d, J = 1.2 Hz, 1H), 7.48-7.55 (m, 2H), 7.35-7.41 (m, 1H), 7.32 (d, J = 8.0 Hz, 1H), 6.26 (s, 1H), 4.05 (quin, J = 7.2 Hz, 1H), 3.60 (s, 2H), 3.27 (s, 3H), 3.15 (s, 3H), 3.10 (ddd, J = 10.0, 7.2, 2.4 Hz, 2H), 2.73-2.83 (m, 2H), 2.57-2.65 (m, 2H), 2.04-2.12 (m, 4H), 1.63 (br d, J = 6.4 Hz, 2H), 1.48-1.55 (m, 2H), 1.35-1.43 (m, 1H), 1.26 (br s, 1H)
LCMS: m/z = 567.3 (M+H)⁺, Rt =1.416 min

### Examples 147 and 148: Synthesis of compounds T147& T148

### 1. General steps for preparation of 2-((5-azaspiro[2.5]octan-5-yl)methyl)-6-(3-((1s,3s)-3-methoxy-1-(4-methyl-4H-1,2,4-triazol-3-yl)cyclobutyl)pheny l)-4-(trifluoromethyl)-1,6-dihydro-7H-pyrrolo[2,3-c]pyridin-7-one (compound T147) & 2-((5-azaspiro[2.5]octan-5-yl)methyl)-6-(3-((1r,3r)-3-methoxy-1-(4-methyl-4H-1,2,4-triazol-3-yl)cyclobutyl)pheny l)-4-(trifluoromethyl)-1,6-dihydro-7H-pyrrolo[2,3-c]pyridin-7-one (compound T148)

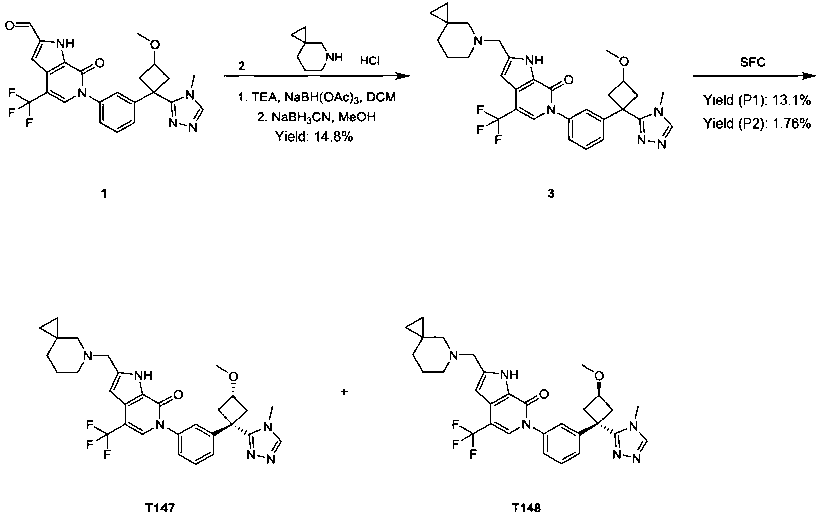

To a solution of 6-[3-[3-methoxy-1-(4-methyl-1,2,4-triazol-3-yl)cyclobutyl]phenyl]-7-oxo-4-(trifluoromethyl)-1H-pyrrolo[2,3-c]pyr idine-2-carbaldehyde (100 mg, 212 µmol) in dichloromethane (2.00 mL) was added dropwise TEA (21.4 mg, 212 µmol, 29.5 µL) adjust pH = 7, 5-azaspiro[2.5]octane (35.3 mg, 239 µmol, HCl) was added to the mixture, stirred at 25 °C for 0.5 hr, then NaBH(OAc)₃ (112 mg, 530 µmol) was added to the mixture, stirred at 25 °C for 1 hr. Methyl alcohol (1.00 mL) and NaBH₃CN (13.3 mg, 212 µmol) was added and stirred at 25 °C for 1 hr. LCMS showed desired compound was detected. The reaction mixture was diluted with aqueous NaHCO₃ (8.00 mL), extracted with dichloromethane (12.0 mL * 3), the combined organic layers were washed with H₂O (8.00 mL), dried over Na₂SO₄, filtered and concentrated under reduced pressure to give a residue. The residue was purified by Prep-HPLC (column: Welch Xtimate C18 40 * 200mm 7 um; mobile phase: [water (NH₄HCO₃)-ACN]; gradient:30.0%-70.0% B over 25 mins). Confirmed by SFC (EB6214-685-P1O1). Separated by SFC (column: DAICEL CHIRALPAK IG (250mm*30mm,10um);mobile phase: [CO₂-EtOH(0.1%NH₃H₂O)];B%:55%, isocratic elution mode) to give T147 2-(5-azaspiro[2.5]octan-5-ylmethyl)-6-[3-[3-methoxy-1-(4-methyl-1,2,4-triazol-3-yl)cyclobutyl]phenyl]-4-(trifluor omethyl)-1H-pyrrolo[2,3-c]pyridin-7-one (16.0 mg, 27.8 µmol, 13.1% yield, 98.5% purity) (Rt = 1.427 min) and T148 2-(5-azaspiro[2.5]octan-5-ylmethyl)-6-[3-[3-methoxy-1-(4-methyl-1,2,4-triazol-3-yl)cyclobutyl]phenyl]-4-(trifluor omethyl)-1H-pyrrolo[2,3-c]pyridin-7-one (2.11 mg, 3.72 µmol, 1.76% yield, 100% purity) (Rt = 1.899 min) both as white solid. T147 was confirmed by H NMR and LCMS.
¹H NMR: (400 MHz, DMSO-*d*₆)
*δ* 12.42 (s, 1H), 8.38 (s, 1H), 7.71 (d, *J =* 1.6 Hz, 1H), 7.45-7.54 (m, 1H), 7.32-7.39 (m, 2H), 7.18 (d, *J =* 8.0 Hz, 1H), 6.26 (s, 1H), 3.85 (quin, *J* = 7.2 Hz, 1H), 3.59 (s, 2H), 3.29 (s, 3H), 3.16 (s, 3H), 2.51-2.58 (m, 4H), 2.42 (s, 2H), 2.13 (s, 2H), 1.55-1.64 (m, 2H), 1.23 (s, 2H), 0.25 (s, 4H)
LCMS: m/z = 567.2 (M+H)⁺, Rt = 1.433 min

T148 was confirmed by H NMR and LCMS.
¹H NMR: (400 MHz, CD₃OD)
*δ* 8.32 (s, 1H), 7.63 (s, 1H), 7.55-7.60 (m, 1H), 7.52 (s, 1H), 7.46 (d, *J =* 8.0 Hz, 1H), 7.37 (d, *J=* 7.2 Hz, 1H), 6.49 (s, 1H), 4.17 (quin, *J* = 7.2 Hz, 1H), 3.78 (s, 2H), 3.39 (s, 3H), 3.27 (s, 3H), 3.18-3.24 (m, 2H), 2.82-2.91 (m, 2H), 2.62 (s, 2H), 2.31 (s, 2H), 1.71-1.82 (m, 2H), 1.34 (d, *J =* 7.2 Hz, 2H), 0.38 (s, 2H), 0.37 (s, 2H).
LCMS: m/z = 567.3 (M+H)⁺, Rt = 1.430 min

### Examples 149 and 150: Synthesis of compounds T149& T150

### 1. General steps for preparation of 2-((ethyl(isobutyl)amino)methyl)-6-(3-(3-methoxy-1-(4-methyl-4H-1,2,4-triazol-3-yl)cyclobutyl)phenyl)-4-(trifluo romethyl)-1,6-dihydro-7H-pyrrolo[2,3-c]pyridin-7-one

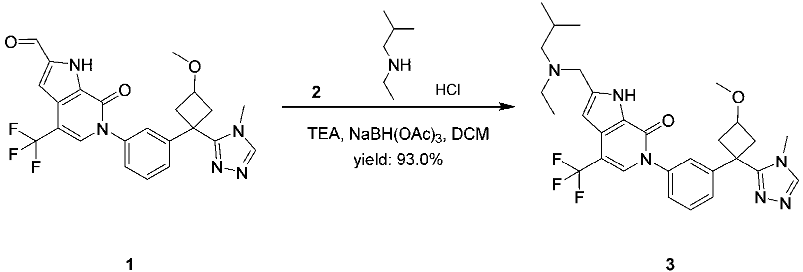

To a solution of 6-(3-(3-methoxy-1-(4-methyl-4H-1,2,4-triazol-3-yl)cyclobutyl)phenyl)-7-oxo-4-(trifluoromethyl)-6,7-dihydro-1H-pyrrolo[2,3-c]pyridine-2-carbaldehyde (100 mg, 212 µmol) in dichloromethane (2.00 mL) was added dropwise TEA (107 mg, 1.06 mmol, 147 µL) adjust pH = 7, N-ethyl-2-methyl-propan-1-amine (43.8 mg, 318 µmol, HCl) was added to the mixture, stirred at 25 °C for 0.5 hr, then NaBH(OAc)₃ (112 mg, 530 µmol) was added to the mixture, stirred at 25 °C for 1 hr. LCMS showed desired compound was detected. The reaction mixture was diluted with aqueous NaHCO₃ (8.00 mL), extracted with dichloromethane (12.0 mL * 3), the combined organic layers were washed with H₂O (8.00 mL), dried over Na₂SO₄, filtered and concentrated under reduced pressure to give a residue. The residue was purified by Prep-HPLC (column: Welch Xtimate C18 40 * 200 mm 7 um; mobile phase: [water (NH₃H₂O+NH₄HCO₃)-ACN]; gradient:40.0%-80.0% B over 25 mins). Compound 2-((ethyl(isobutyl)amino)methyl)-6-(3-(3-methoxy-1-(4-methyl-4H-1,2,4-triazol-3-yl)cyclobutyl)phenyl)-4-(trifluo romethyl)-1,6-dihydro-7H-pyrrolo[2,3-c]pyridin-7-one (110 mg, 93.0% yield, 99.8% purity) was obtained as a white solid. Confirmed by H NMR and LCMS.
¹H NMR: (400 MHz, DMSO-*d*₆)
*δ* 12.41 (s, 1H), 8.27-8.41 (m, 1H), 7.71 (dd, *J =* 4.0, 1.2 Hz, 1H), 7.46-7.54 (m, 2H), 7.36-7.38 (m, 1H), 7.18 (d, *J =* 8.0 Hz, 1H), 6.27 (s, 1H), 3.69 (s, 2H), 3.32 (s, 1H), 3.28 (d, *J =* 9.6 Hz, 3H), 3.16 (d, *J =* 4.0 Hz, 3H), 3.06-3.14 (m, 1H), 2.78 (ddd, *J =* 10.0, 8.0, 2.4 Hz, 1H), 2.54 (s, 1H), 2.42-2.48 (m, 3H), 2.14 (d, *J =* 7.2 Hz, 2H), 1.75 (dt, *J =* 13.2, 6.8 Hz, 1H), 0.99 (t, *J = 7.2* Hz, 3H), 0.84 (d, *J =* 6.8 Hz, 6H)
LCMS: m/z = 557.3 (M+H)⁺, Rt = 1.450 min

### 2. General steps for preparation of 2-((ethyl(isobutyl)amino)methyl)-6-(3-((1s,3s)-3-methoxy-1-(4-methyl-4H-1,2,4-triazol-3-yl)cyclobutyl)phenyl)-4-(trifluoromethyl)-1,6-dihydro-7H-pyrrolo[2,3-c]pyridin-7-one (compound T149) & 2-((ethyl(isobutyl)amino)methyl)-6-(3-((1r,3r)-3-methoxy-1-(4-methyl-4H-1,2,4-triazol-3-yl)cyclobutyl)phenyl)-4-(trifluoromethyl)-1,6-dihydro-7H-pyrrolo[2,3-c]pyridin-7-one (compound T150)

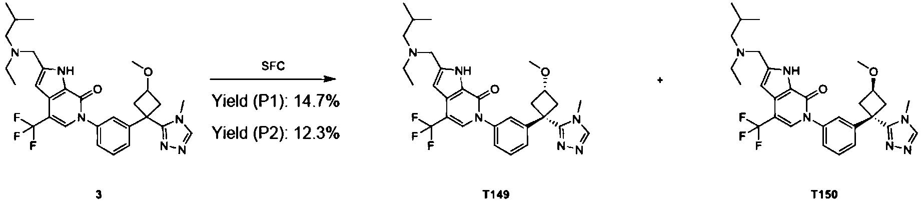

The residue was purified by SFC (column: DAICEL CHIRALPAK IG (250 mm * 30 mm,10 um); mobile phase: [CO₂-EtOH (0.1%NH₃H₂O)]; B%:35.0%, isocratic elution mode). Separated by SFC to give compound T149 and compound T150 both as white solid. compound T149: 2-((ethyl(isobutyl)amino)methyl)-6-(3-((1s,3s)-3-methoxy-1-(4-methyl-4H-1,2,4-triazol-3-yl)cyclobutyl)phenyl)-4-(trifluoromethyl)-1,6-dihydro-7H-pyrrolo[2,3-c]pyridin-7-one (19.7 mg, 34.3 µmol, 14.7% yield, 97.0% purity) was confirmed by H NMR and LCMS.
¹H NMR: (400 MHz, DMSO-*d*₆)
*δ* 12.41 (s, 1H), 8.38 (s, 1H), 7.71 (d, *J =* 1.6 Hz, 1H), 7.45-7.53 (m, 1H), 7.31-7.39 (m, 2H), 7.18 (d, *J =* 8.0 Hz, 1H), 6.27 (s, 1H), 3.80-3.89 (m, 1H), 3.69 (s, 2H), 3.30 (s, 3H), 3.16 (s, 3H), 2.54 (d, *J* = 2.4 Hz, 2H), 2.41-2.49 (m, 4H), 2.14 (d, *J* = 7.2 Hz, 2H), 1.75 (dt, *J* = 13.6, 6.8 Hz, 1H), 0.99 (t, *J =* 7.2 Hz, 3H), 0.85 (d, *J* = 6.4 Hz, 6H)
LCMS: m/z = 557.3 (M+H)⁺, Rt = 1.437 min

Compound T150: 2-((ethyl(isobutyl)amino)methyl)-6-(3-((1r,3r)-3-methoxy-1-(4-methyl-4H-1,2,4-triazol-3-yl)cyclobutyl)phenyl)-4-(trifluoromethyl)-1,6-dihydro-7H-pyrrolo[2,3-c]pyridin-7-one (16.4 mg, 28.7 µmol, 12.3% yield, 97.6% purity) was confirmed by H NMR and LCMS.
¹H NMR: (400 MHz, DMSO-*d*₆)
*δ* 12.42 (s, 1H), 8.25-8.37 (m, 1H), 7.72 (d, *J =* 1.2 Hz, 1H), 7.53 (s, 1H), 7.50 (d, *J =* 7.6 Hz, 1H), 7.38 (d, *J =* 8.0 Hz, 1H), 7.32 (d, *J* = 7.6 Hz, 1H), 6.27 (s, 1H), 4.06 (t, *J =* 7.2 Hz, 1H), 3.69 (s, 2H), 3.27 (s, 3H), 3.15 (s, 3H), 3.08-3.13 (m, 2H), 2.75-2.82 (m, 2H), 2.46 (d, *J =* 7.2 Hz, 2H), 2.14 (d, *J =* 7.2 Hz, 2H), 1.62-1.79 (m, 1H), 0.99 (t, *J =* 7.2 Hz, 3H), 0.85 (d, *J =* 6.4 Hz, 6H).
LCMS: m/z = 557.3 (M+H)⁺, Rt = 1.420 min

### Examples 151 and 152: Synthesis of compounds T151& T152

### 1. General steps for preparation of 2-((isopropyl(propyl)amino)methyl)-6-(3-(3-methoxy-1-(4-methyl-4H-1,2,4-triazol-3-yl)cyclobutyl)phenyl)-4-(trifl uoromethyl)-1,6-dihydro-7H-pyrrolo[2,3-c]pyridin-7-one

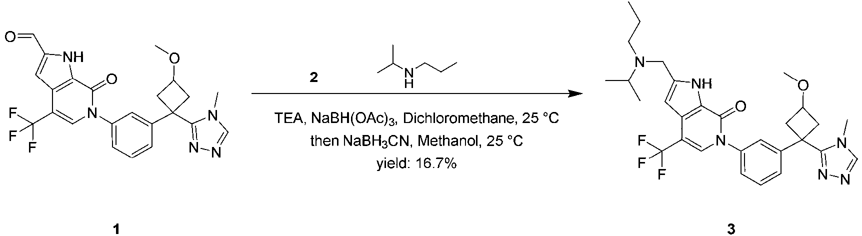

To a mixture of 6-[3-[3-methoxy-1-(4-methyl-1,2,4-triazol-3-yl)cyclobutyl]phenyl]-7-oxo-4-(trifluoromethyl)-1H-pyrrolo[2,3-c]pyr idine-2-carbaldehyde (100 mg, 212 µmol)in Dichloromethane (5.00 mL) was added N-isopropylpropan-1-amine (32.2 mg, 318 µmol). The mixture was stirred under N₂ at 25.0 °C for 0.50 hr. sodium triacetoxyboranuide (112 mg, 530 µmol) was added to the mixture. The mixture was stirred under N₂ at 25.0 °C for 3.50 hrs. Then Methanol (1.00 mL) and sodium cyanoboranuide (13.3 mg, 212 µmol) was added to the mixture. The mixture was stirred under N₂ at 25.0 °C for 8.00 hrs. LCMS showed desired mass was detected. The mixture was concentrated under reduced pressure. Then it was diluted with Methanol (7.00 mL). The crude product was purified by Prep-HPLC (column: Welch Xtimate C18 40.0 * 200 mm 7.00 um; mobile phase: [water (0.05%NH₃H₂O 10.0 mM NH₄HCO₃)-ACN]; gradient: 0.00% - 38.0% B over 25.0 mins). Compound 2-((isopropyl(propyl)amino)methyl)-6-(3-(3-methoxy-1-(4-methyl-4H-1,2,4-triazol-3-yl)cyclobutyl)phenyl)-4-(trifl uoromethyl)-1,6-dihydro-7H-pyrrolo[2,3-c]pyridin-7-one (20.1 mg, 35.3 µmol, 16.6% yield, 97.9% purity) was obtained as a white solid, which was confirmed by H NMR and LCMS.
¹H NMR: (400 MHz, DMSO-*d*₆)
*δ* 12.36 (br s, 1H), 8.26-8.40 (m, 1H), 7.70 (dd, *J =* 3.6, 1.2 Hz, 1H), 7.45-7.55 (m, 2H), 7.36-7.39 (m, 1H), 7.16-7.34 (m, 1H), 6.29 (s, 1H), 3.80-4.12 (m, 1H), 3.65 (s, 2H), 3.28 (d, *J* = 9.6 Hz, 4H), 3.16 (d, *J =* 4.0 Hz, 3H), 3.10 (ddd, *J =* 10.0, 7.2, 2.4 Hz, 1H), 2.91 (dt, *J =* 13.2, 6.4 Hz, 1H), 2.73-2.83 (m, 1H), 2.54 (br d, *J =* 2.4 Hz, 1H), 2.36 (t, *J =* 7.2 Hz, 2H), 1.29-1.42 (m, 2H), 0.97 (d, *J =* 6.4 Hz, 6H), 0.81 (t, *J =* 7.6 Hz, 3H)
LCMS: m/z = 557.2 (M+H)⁺, Rₜ = 0.967 min

### 2. General steps for preparation of 2-((isopropyl(propyl)amino)methyl)-6-(3-((1s,3s)-3-methoxy-1-(4-methyl-4H-1,2,4-triazol-3-yl)cyclobutyl)phenyl) -4-(trifluoromethyl)-1,6-dihydro-7H-pyrrolo[2,3-c]pyridin-7-one (compound T151) and 2-((isopropyl(propyl)amino)methyl)-6-(3-((1r,3r)-3-methoxy-1-(4-methyl-4H-1,2,4-triazol-3-yl)cyclobutyl)phenyl) -4-(trifluoromethyl)-1,6-dihydro-7H-pyrrolo[2,3-c]pyridin-7-one (compound T152)

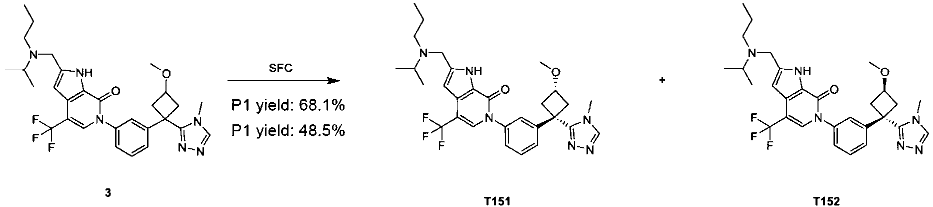

SFC (EB12379-21-P1A1) showed two peaks. The crude product was purified by SFC (column: DAICEL CHIRALPAK IG (250 mm * 30.0 mm, 10.0 um); mobile phase: [CO₂-ethyl alcohol (0.10% NH₃H₂O)]; B%: 55.0%, isocratic elution mode). To afford 2-((isopropyl(propyl)amino)methyl)-6-(3-((1s,3s)-3-methoxy-1-(4-methyl-4H-1,2,4-triazol-3-yl)cyclobutyl)phenyl) -4-(trifluoromethyl)-1,6-dihydro-7H-pyrrolo[2,3-c]pyridin-7-one (13.9 mg, 24.6 µmol, 68.1% yield, 98.5% purity) as a white solid which was confirmed by H NMR and LCMS.
¹H NMR: (400 MHz, DMSO-*d*₆)
*δ* 12.36 (br s, 1H), 8.38 (s, 1H), 7.70 (d, *J =* 1.2 Hz, 1H), 7.45-7.56 (m, 1H), 7.32-7.40 (m, 2H), 7.18 (d, *J* = 8.0 Hz, 1H), 6.28 (s, 1H), 3.85 (quin, *J =* 7.2 Hz, 1H), 3.65 (s, 2H), 3.30-3.33 (m, 2H), 3.30 (s, 3H), 3.16 (s, 3H), 2.91 (dt, *J =* 13.2, 6.4 Hz, 1H), 2.54 (br d, *J =* 2.4 Hz, 1H), 2.36 (t, *J =* 7.2 Hz, 2H), 1.31-1.41 (m, 2H), 0.97 (d, *J =* 6.4 Hz, 6H), 0.81 (t, *J =* 7.2 Hz, 3H)
LCMS: m/z = 557.3 (M+H)⁺, Rt = 1.433 min

To afford 2-((isopropyl(propyl)amino)methyl)-6-(3-((1r,3r)-3-methoxy-1-(4-methyl-4H-1,2,4-triazol-3-yl)cyclobutyl)phenyl) -4-(trifluoromethyl)-1,6-dihydro-7H-pyrrolo[2,3-c]pyridin-7-one (9.76 mg, 17.5 µmol, 48.5% yield, 100% purity) as a white solid which was confirmed by H NMR and LCMS.
¹H NMR: (400 MHz, DMSO-*d*₆)
*δ* 12.38 (br s, 1H), 8.30 (s, 1H), 7.71 (d, *J =* 1.2 Hz, 1H), 7.45-7.57 (m, 2H), 7.26-7.41 (m, 2H), 6.29 (s, 1H), 4.05 (quin, *J =* 7.2 Hz, 1H), 3.65 (s, 2H), 3.27 (s, 3H), 3.15 (s, 3H), 3.10 (ddd, *J =* 10.0, 7.2, 2.4 Hz, 2H), 2.91 (dt, *J* = 13.2, 6.4 Hz, 1H), 2.72-2.83 (m, 2H), 2.36 (br t, *J =* 7.2 Hz, 2H), 1.31-1.41 (m, 2H), 0.97 (d, *J =* 6.4 Hz, 6H), 0.81 (t, *J =* 7.2 Hz, 3H)
LCMS: m/z = 557.3 (M+H)⁺, Rt = 1.423 min

### Examples 153 and 154: Synthesis of compounds T153& T154

### 1. General steps for preparation of 2-(((2-cyclopropylethyl)amino)methyl)-6-(3-(3-methoxy-1-(4-methyl-4H-1,2,4-triazol-3-yl)cyclobutyl)phenyl)-4-(t rifluoromethyl)-1,6-dihydro-7H-pyrrolo[2,3-c]pyridin-7-one

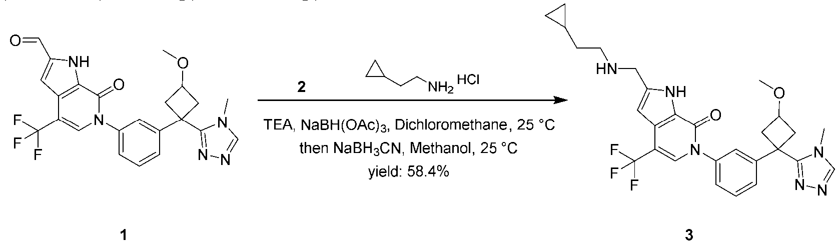

To a mixture of 2-cyclopropylethanamine (38.6 mg, 318 µmol, HCl) in Dichloromethane (5.00 mL) was added TEA (128 mg, 1.27 mmol, 177 µL) adjust pH = 8.00. Then 6-[3-[3-methoxy-1-(4-methyl-1,2,4-triazol-3-yl) cyclobutyl] phenyl]-7-oxo-4-(trifluoromethyl)-1H-pyrrolo[2,3-c] pyridine-2-carbaldehyde (100 mg, 212 µmol) was added to the mixture. The mixture was stirred under N₂ at 25.0 °C for 0.50 hr. sodium triacetoxyboranuide (112 mg, 530 µmol) was added to the mixture. The mixture was stirred under N₂ at 25.0 °C for 3.50 hrs. Then Methanol (1.00 mL), andsodium cyanoboranuide (13.3 mg, 212 µmol) was added to the mixture. The mixture was stirred under N₂ at 25.0 °C for 8.00 hrs. LCMS showed desired mass was detected. The mixture was concentrated under reduced pressure. Then it was diluted with Methanol (7.00 mL). The crude product was purified by Prep-HPLC (column: Welch Xtimate C18 40.0 * 200 mm 7.00 um; mobile phase: [water (HCl) - ACN]; gradient: 0.00% - 38.0% B over 25.0 mins). Compound 2-(((2-cyclopropylethyl) amino) methyl)-6-(3-(3-methoxy-1-(4-methyl-4H-1,2,4-triazol-3-yl) cyclobutyl) phenyl)-4-(trifluoromethyl)-1,6-dihydro-7H-pyrrolo[2,3-c] pyridin-7-one (70.0 mg, 123 µmol, 58.4% yield, 95.7% purity) was obtained as a white solid, which was confirmed by H NMR and LCMS.
¹H NMR: (400 MHz, DMSO-*d*₆)
*δ* 12.70 (br d, *J =* 3.2 Hz, 1H), 9.43 (br d, *J =* 3.2 Hz, 2H), 9.06 (br d, *J =* 15.2 Hz, 1H), 7.77-7.90 (m, 1H), 7.51-7.59 (m, 1H), 7.41-7.46 (m, 1H), 7.25-7.40 (m, 1H), 6.69 (s, 1H), 4.32 (br s, 2H), 4.02-4.15 (m, 1H), 3.86-3.92 (m, 1H), 3.39 (d, *J* = 8.8 Hz, 3H), 3.30-3.36 (m, 1H), 3.17 (d, *J* = 2.4 Hz, 3H), 2.92-3.01 (m, 2H), 2.78-2.90 (m, 1H), 2.58 (ddd, *J =* 9.6, 7.6, 2.4 Hz, 1H), 1.57 (q, *J =* 7.6 Hz, 2H), 0.65-0.80 (m, 1H), 0.34-0.53 (m, 2H), 0.01-0.16 (m, 2H)
LCMS: m/z = 541.2 (M+H)⁺, Rₜ = 0.969 min

### 2. General steps for preparation of 2-(((2-cyclopropylethyl)amino)methyl)-6-(3-((1s,3s)-3-methoxy-1-(4-methyl-4H-1,2,4-triazol-3-yl)cyclobutyl)phen yl)-4-(trifluoromethyl)-1,6-dihydro-7H-pyrrolo[2,3-c]pyridin-7-one (compound T153) and 2-(((2-cyclopropylethyl)amino)methyl)-6-(3-((1r,3r)-3-methoxy-1-(4-methyl-4H-1,2,4-triazol-3-yl)cyclobutyl)phen yl)-4-(trifluoromethyl)-1,6-dihydro-7H-pyrrolo[2,3-c]pyridin-7-one (compound T154)

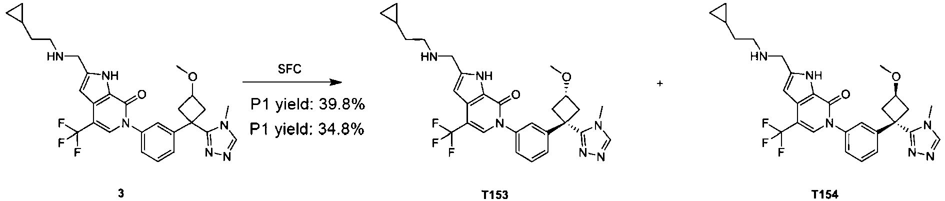

SFC (EB12379-19-P1A1) showed two peaks. The crude product was purified by SFC (column: DAICEL CHIRALCEL OX (250 mm * 30.0 mm, 10.0 um); mobile phase: [CO₂ - Ethyl alcohol (0.10% NH₃H₂O)]; B%: 45.0%, isocratic elution mode). To afford 2-(((2-cyclopropylethyl)amino)methyl)-6-(3-((1s,3s)-3-methoxy-1-(4-methyl-4H-1,2,4-triazol-3-yl)cyclobutyl)phen yl)-4-(trifluoromethyl)-1,6-dihydro-7H-pyrrolo[2,3-c]pyridin-7-one (28.6 mg, 51.5 µmol, 39.8% yield, 97.4% purity) as a white solid which was confirmed by H NMR and LCMS.
¹H NMR: (400 MHz, DMSO-*d*₆)
*δ* 8.38 (s, 1H), 7.71 (d, *J =* 1.2 Hz, 1H), 7.46-7.55 (m, 1H), 7.32-7.40 (m, 2H), 7.18 (br d, *J =* 8.0 Hz, 1H), 6.32 (s, 1H), 3.82-3.90 (m, 1H), 3.80 (s, 2H), 3.31-3.37 (m, 4H), 3.30 (s, 3H), 3.16 (s, 3H), 2.52-2.55 (m, 2H), 1.31 (q, *J = 7.2* Hz, 2H), 0.65-0.77 (m, 1H), 0.33-0.41 (m, 2H), -0.05-0.04 (m, 2H)
LCMS: m/z = 541.2 (M+H)⁺, Rt = 1.437 min

To afford 2-(((2-cyclopropylethyl)amino)methyl)-6-(3-((1r,3r)-3-methoxy-1-(4-methyl-4H-1,2,4-triazol-3-yl)cyclobutyl)phen yl)-4-(trifluoromethyl)-1,6-dihydro-7H-pyrrolo[2,3-c]pyridin-7-one (24.6 mg, 45.0 µmol, 34.8% yield, 99.0% purity) as a white solid which was confirmed by H NMR and LCMS.
¹H NMR: (400 MHz, DMSO-*d*₆)
*δ* 8.31 (s, 1H), 7.63-7.74 (m, 1H), 7.46-7.55 (m, 2H), 7.26-7.41 (m, 2H), 6.19-6.35 (m, 1H), 4.33 (br s, 1H), 4.05 (quin, *J* = 7.2 Hz, 1H), 3.79 (s, 1H), 3.27 (s, 3H), 3.18-3.24 (m, 1H), 3.15 (s, 3H), 3.05-3.13 (m, 2H), 2.72-2.84 (m, 2H), 2.52-2.56 (m, 1H), 1.20-1.27 (m, 3H), 0.26-0.43 (m, 2H), -0.09-0.04 (m, 2H)
LCMS: m/z = 541.3 (M+H)⁺, Rₜ = 1.424 min

### Examples 155 and 156: Synthesis of compounds T155& T156

### 1. General steps for preparation of 2-((3-(difluoromethyl)piperidin-1-yl)methyl)-6-(3-(3-methoxy-1-(4-methyl-4H-1,2,4-triazol-3-yl)cyclobutyl)pheny l)-4-(trifluoromethyl)-1,6-dihydro-7H-pyrrolo[2,3-c]pyridin-7-one

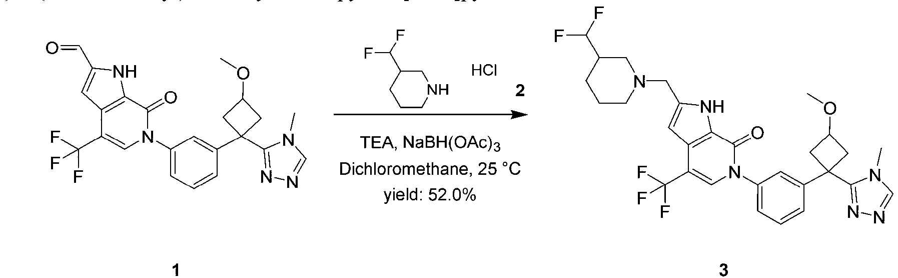

To a mixture of 3-(difluoromethyl) piperidine (109 mg, 636 µmol, HCl) in Dichloromethane (10.0 mL) was added TEA (257 mg, 2.55 mmol, 354 µL) adjust pH = 8.00. Then 6-[3-[3-methoxy-1-(4-methyl-1,2,4-triazol-3-yl) cyclobutyl] phenyl]-7-oxo-4-(trifluoromethyl)-1H-pyrrolo[2,3-c] pyridine-2-carbaldehyde (200 mg, 424 µmol) was added to the mixture. The mixture was stirred under N₂ at 25.0 °C for 0.50 hr. sodium triacetoxyboranuide (224 mg, 1.06 mmol) was added to the mixture. The mixture was stirred under N₂ at 25.0 °C for 11.5 hrs. LCMS showed desired mass was detected. The mixture was concentrated under reduced pressure. Then it was diluted with Methanol (7.00 mL). The crude product was purified by Prep-HPLC (column: Welch Xtimate C18 40.0 * 200 mm 7.00 um; mobile phase: [water (HCl) - ACN]; gradient: 0.00% - 38.0% B over 25.0 mins). Compound 2-((3-(difluoromethyl) piperidin-1-yl) methyl)-6-(3-(3-methoxy-1-(4-methyl-4H-1,2,4-triazol-3-yl) cyclobutyl) phenyl)-4-(trifluoromethyl)-1,6-dihydro-7H-pyrrolo[2,3-c] pyridin-7-one (130 mg, 220 µmol, 52.0% yield, 99.7% purity) was obtained as a white solid, which was confirmed by H NMR and LCMS.
¹H NMR: (400 MHz, DMSO-*d*₆)
*δ* 12.84 (br s, 1H), 11.32-11.59 (m, 1H), 9.09 (br d, *J =* 14.4 Hz, 1H), 7.84 (dt, *J =* 2.8, 1.2 Hz, 1H), 7.52-7.60 (m, 1H), 7.37-7.48 (m, 2H), 7.29 (d, *J =* 8.0 Hz, 1H), 6.77 (br s, 1H), 6.07 (td, *J =* 55.6, 3.2 Hz, 1H), 4.41-4.54 (m, 2H), 4.02-4.15 (m, 1H), 3.88 (br d, *J =* 7.2 Hz, 1H), 3.43-3.52 (m, 2H), 3.39 (d, *J =* 8.4 Hz, 3H), 3.30-3.36 (m, 1H), 3.17 (d, *J =* 2.4 Hz, 3H), 2.80-2.88 (m, 2H), 2.58 (ddd, *J* = 9.6*,* 7.6, 2.4 Hz, 2H), 2.07 (s, 2H), 1.90 (br s, 1H), 1.82 (br d, *J =* 13.6 Hz, 1H)
LCMS: m/z = 591.1 (M+H)⁺, Rₜ = 0.962 min

### 2. General steps for preparation of 2-((3-(difluoromethyl)piperidin-1-yl)methyl)-6-(3-((1s,3s)-3-methoxy-1-(4-methyl-4H-1,2,4-triazol-3-yl)cyclobutyl )phenyl)-4-(trifluoromethyl)-1,6-dihydro-7H-pyrrolo[2,3-c]pyridin-7-one (compound T155) and 2-((3-(difluoromethyl)piperidin-1-yl)methyl)-6-(3-((1r,3r)-3-methoxy-1-(4-methyl-4H-1,2,4-triazol-3-yl)cyclobutyl )phenyl)-4-(trifluoromethyl)-1,6-dihydro-7H-pyrrolo[2,3-c]pyridin-7-one (compound T156)

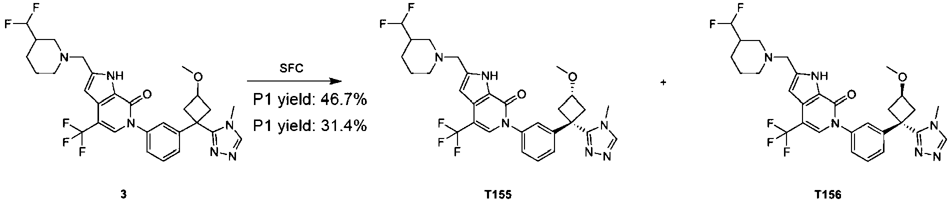

SFC (EB12379-23-P1A1) showed two peaks. The crude product was purified by SFC (column: DAICEL CHIRALPAK IG (250 mm * 30.0 mm, 10.0 um); mobile phase: [CO₂ - Ethyl alcohol (0.10% NH₃H₂O)]; B %: 45.0 %, isocratic elution mode). To afford 2-((3-(difluoromethyl)piperidin-1-yl)methyl)-6-(3-((1s,3s)-3-methoxy-1-(4-methyl-4H-1,2,4-triazol-3-yl)cyclobutyl )phenyl)-4-(trifluoromethyl)-1,6-dihydro-7H-pyrrolo[2,3-c]pyridin-7-one (61.4 mg, 103 µmol, 46.7% yield, 99.5% purity) as a white solid which was confirmed by H NMR and LCMS.
¹H NMR: (400 MHz, DMSO-*d*₆)
*δ* 12.49 (br s, 1H), 8.38 (s, 1H), 7.73 (s, 1H), 7.45-7.56 (m, 1H), 7.31-7.40 (m, 2H), 7.19 (br d, *J =* 7.6 Hz, 1H), 6.31 (br s, 1H), 5.80-6.16 (m, 1H), 3.85 (quin, *J =* 7.2 Hz, 1H), 3.71 (br s, 2H), 3.30 (s, 3H), 3.16 (s, 3H), 2.66-2.92 (m, 2H), 2.54 (br d, *J =* 2.4 Hz, 1H), 1.93-2.18 (m, 3H), 1.67 (br d, *J =* 10.0 Hz, 2H), 1.41-1.56 (m, 1H), 1.20-1.32 (m, 1H), 1.06-1.19 (m, 1H)
LCMS: m/z = 591.3 (M+H)⁺, Rt = 1.428 min

To afford 2-((3-(difluoromethyl)piperidin-1-yl)methyl)-6-(3-((1r,3r)-3-methoxy-1-(4-methyl-4H-1,2,4-triazol-3-yl)cyclobutyl )phenyl)-4-(trifluoromethyl)-1,6-dihydro-7H-pyrrolo[2,3-c]pyridin-7-one (41.4 mg, 69.5 µmol, 31.4% yield, 99.2% purity) as a white solid which was confirmed by H NMR and LCMS.
¹H NMR: (400 MHz, DMSO-*d*₆)
*δ* 12.40-12.56 (m, 1H), 8.30 (s, 1H), 7.73 (s, 1H), 7.47-7.57 (m, 2H), 7.38 (br d, *J =* 7.6 Hz, 1H), 7.32 (br d, *J* = 7.6 Hz, 1H), 6.29 (s, 1H), 5.79-6.15 (m, 1H), 4.06 (quin, *J* = 7.6 Hz, 1H), 3.67 (s, 2H), 3.27 (s, 3H), 3.15 (s, 3H), 3.10 (ddd, *J =* 10.0, 7.2, 2.4 Hz, 2H), 2.79 (br dd, *J* = 7.2, 2.4 Hz, 2H), 2.64-2.77 (m, 2H), 2.02-2.09 (m, 1H), 1.94-2.01 (m, 2H), 1.66 (br d, *J =* 10.4 Hz, 2H), 1.41-1.52 (m, 1H), 1.08-1.17 (m, 1H)
LCMS: m/z = 591. (M+H)⁺, Rt = 1.429 min

### Example 157: Synthesis of compound T157

### 1. General steps for preparation of 2-((3-(difluoromethyl)piperidin-1-yl)methyl)-6-(3-((1s,3s)-3-methyl-1-(4-methyl-4H-1,2,4-triazol-3-yl)cyclobutyl) phenyl)-4-(trifluoromethyl)-1,6-dihydro-7H-pyrrolo[2,3-c]pyridin-7-one (compound T157)

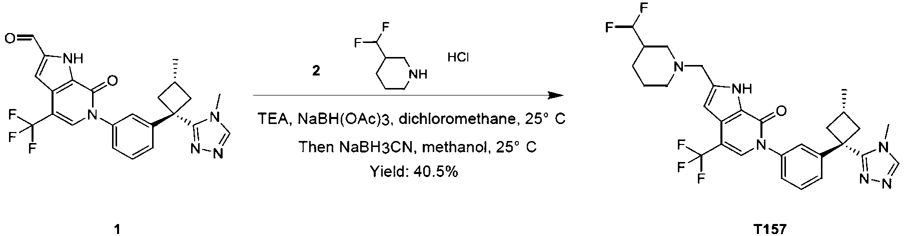

To a mixture of 3-(difluoromethyl) piperidine (56.5 mg, 329 µmol, HCl) in Dichloromethane (5.00 mL) was added TEA (133 mg, 1.32 mmol, 183 µL) adjust pH = 8.00. Then 6-[3-[3-methyl-1-(4-methyl-1,2,4-triazol-3-yl) cyclobutyl] phenyl]-7-oxo-4-(trifluoromethyl)-1H-pyrrolo[2,3-c] pyridine-2-carbaldehyde (100 mg, 219 µmol) was added to the mixture. The mixture was stirred under N₂ at 25.0 °C for 0.50 hr. sodium triacetoxyboranuide (116 mg, 548 µmol) was added to the mixture. The mixture was stirred under N₂ at 25.0 °C for 11.50 hrs. LCMS showed desired mass was detected. The mixture was concentrated under reduced pressure. Then it was diluted with Methanol (7.00 mL). The crude product was purified by Prep-HPLC (column: Welch Xtimate C18 40.0 * 200 mm 7.00 um; mobile phase: [water (NH₃H₂O+NH₄HCO₃) - ACN]; gradient: 34.0% - 74.0% B over 25.0 mins). Compound 2-((3-(difluoromethyl)piperidin-1-yl)methyl)-6-(3-((1s,3s)-3-methyl-1-(4-methyl-4H-1,2,4-triazol-3-yl)cyclobutyl) phenyl)-4-(trifluoromethyl)-1,6-dihydro-7H-pyrrolo[2,3-c]pyridin-7-one (51.2 mg, 89.1 µmol, 40.5% yield, 100% purity) was obtained as a white solid, which was confirmed by H NMR and LCMS.
¹H NMR: (400 MHz, DMSO-*d*₆)
*δ* 12.45 (br s, 1H), 8.28 (s, 1H), 7.72 (d, *J =* 1.2 Hz, 1H), 7.48-7.56 (m, 2H), 7.35 (br t, *J =* 9.2 Hz, 2H), 6.29 (s, 1H), 5.80-6.14 (m, 1H), 3.67 (s, 2H), 3.25 (s, 3H), 2.85-2.92 (m, 2H), 2.81 (br d, *J* = 7.6 Hz, 1H), 2.72 (br d, *J =* 10.8 Hz, 1H), 2.52-2.59 (m, 3H), 2.06 (br d, *J =* 10.8 Hz, 1H), 1.94-2.02 (m, 2H), 1.63-1.71 (m, 2H), 1.41-1.54 (m, 1H), 1.11-1.19 (m, 1H), 1.07 (br d, *J* = 5.2 Hz, 3H)
LCMS: m/z = 575.2 (M+H)⁺, Rₜ = 1.577 min

### Example 158: Synthesis of compound T158

### 1. General steps for preparation of 2-((((S)-3,3-dimethylbutan-2-yl)amino)methyl)-6-(3-((1s,3R)-3-methyl-1-(4-methyl-4H-1,2,4-triazol-3-yl)cyclobut yl)phenyl)-4-(trifluoromethyl)-1,6-dihydro-7H-pyrrolo[2,3-c]pyridin-7-one (compound T158)

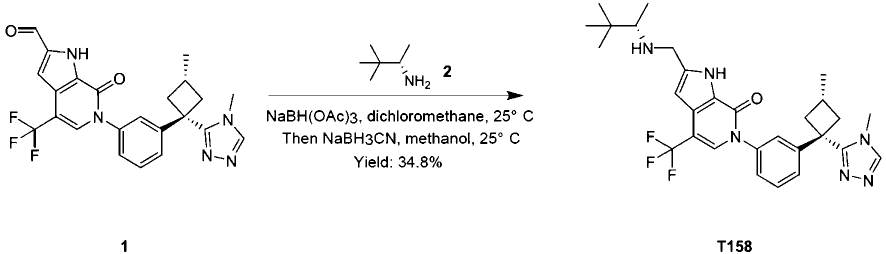

To a mixture of 6-[3-[3-methyl-1-(4-methyl-1,2,4-triazol-3-yl) cyclobutyl] phenyl]-7-oxo-4-(trifluoromethyl)-1H-pyrrolo[2,3-c] pyridine-2-carbaldehyde (50.0 mg, 109 µmol) in Dichloromethane (3.00 mL) was added (2S)-3,3-dimethylbutan-2-amine (22.2 mg, 219 µmol). The mixture was stirred under N₂ at 25.0 °C for 0.50 hr. sodium triacetoxyboranuide (58.1 mg, 274 µmol) was added to the mixture. The mixture was stirred under N₂ at 25.0 °C for 3.50 hrs. Then Methanol (1.00 mL) and sodium cyanoboranuide (6.90 mg, 109 µmol) was added to the mixture. The mixture was stirred under N₂ at 25.0 °C for 8.00 hrs. LCMS showed desired mass was detected. The reaction mixture was diluted with NaHCO₃ (1.00 mL), extracted with Dichloromethane (2.00 mL * 2). The organic phase dried over Na₂SO₄ and concentrated in vacuum. The crude product was purified by Prep-HPLC (column: Welch Xtimate C18 40.0 * 200 mm 7.00 um; mobile phase: [water (NH₃H₂O+NH₄HCO₃) - ACN]; gradient: 38.0% - 78.0% B over 25.0 mins). Compound 2-((((S)-3,3-dimethylbutan-2-yl)amino)methyl)-6-(3-((1s,3R)-3-methyl-1-(4-methyl-4H-1,2,4-triazol-3-yl)cyclobut yl)phenyl)-4-(trifluoromethyl)-1,6-dihydro-7H-pyrrolo[2,3-c]pyridin-7-one (20.8 mg, 38.2 µmol, 34.8% yield, 99.5% purity) was obtained as a white solid, which was confirmed by H NMR and LCMS.
¹H NMR: (400 MHz, DMSO-*d*₆)
*δ* 8.28 (s, 1H), 7.71 (d, *J =* 1.2 Hz, 1H), 7.47-7.55 (m, 2H), 7.28-7.41 (m, 2H), 6.34 (s, 1H), 3.84-3.94 (m, 1H), 3.72-3.80 (m, 1H), 3.25 (s, 3H), 2.84-2.93 (m, 2H), 2.52-2.59 (m, 3H), 2.15 (q, *J =* 6.4 Hz, 1H), 1.07 (br d, *J* = 5.6 Hz, 3H), 0.90 (d, *J =* 6.4 Hz, 3H), 0.83 (s, 9H)
LCMS: m/z = 541.3 (M+H)⁺, Rₜ = 1.690 min

### Example 159: Synthesis of compound T159

### 1. General steps for preparation of 2-((ethyl(isopropyl)amino)methyl)-6-(3-((1s,3s)-3-methyl-1-(4-methyl-4H-1,2,4-triazol-3-yl)cyclobutyl)phenyl)-4-(trifluoromethyl)-1,6-dihydro-7H-pyrrolo[2,3-c]pyridin-7-one (compound T159)

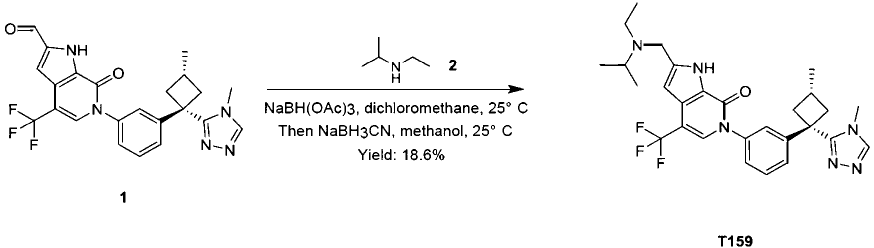

To a mixture of 6-[3-[3-methyl-1-(4-methyl-1,2,4-triazol-3-yl)cyclobutyl]phenyl]-7-oxo-4-(trifluoromethyl)-1H-pyrrolo[2,3-c]pyrid ine-2-carbaldehyde (50.0 mg, 109 µmol) in Dichloromethane (2.00 mL) was added N-ethylpropan-2-amine (14.3 mg, 164 µmol). The mixture was stirred under N₂ at 25.0 °C for 0.50 hr. sodium triacetoxyboranuide (58.1 mg, 274 µmol) was added to the mixture. The mixture was stirred under N₂ at 25.0 °C for 3.50 hrs. Then Methanol (1.00 mL) and sodium cyanoboranuide (6.90 mg, 109 µmol) was added to the mixture. The mixture was stirred under N₂ at 25.0 °C for 8.00 hrs. LCMS showed desired mass was detected. The reaction mixture was diluted with NaHCO₃ (1.00 mL), extracted with Dichloromethane (2.00 mL * 2). The organic phase dried over Na₂SO₄ and concentrated in vacuum. The crude product was purified by Prep-HPLC (column: Welch Xtimate C18 40.0 * 200 mm 7.00 um; mobile phase: [water (NH₃H₂O+NH₄HCO₃) - ACN]; gradient: 38.0% - 78.0% B over 25 mins). Compound 2-((ethyl(isopropyl)amino)methyl)-6-(3-((1s,3s)-3-methyl-1-(4-methyl-4H-1,2,4-triazol-3-yl)cyclobutyl)phenyl)-4-(trifluoromethyl)-1,6-dihydro-7H-pyrrolo[2,3-c]pyridin-7-one (10.8 mg, 20.4 µmol, 18.6% yield, 99.5% purity) was obtained as a white solid, which was confirmed by H NMR and LCMS.
¹H NMR: (400 MHz, DMSO-*d*₆)
*δ* 8.28 (s, 1H), 7.70 (d, *J =* 1.2 Hz, 1H), 7.47-7.55 (m, 2H), 7.35 (br t, *J =* 8.8 Hz, 2H), 6.28 (s, 1H), 3.65 (s, 2H), 3.25 (s, 3H), 2.90-2.98 (m, 1H), 2.88 (br d, *J =* 3.6 Hz, 2H), 2.52-2.57 (m, 3H), 2.43-2.48 (m, 2H), 1.07 (br d, *J= 5.6* Hz, 3H), 0.94-1.00 (m, 9H)
LCMS: m/z = 527.3 (M+H)⁺, Rₜ = 1.536 min

### Example 160: Synthesis of compound T160

### 1. General steps for preparation of 6-(3-(1-(4-methyl-4H-1,2,4-triazol-3-yl)cyclobutyl)phenyl)-2-((propylamino)methyl)-4-(trifluoromethyl)-1,6-dihyd ro-7H-pyrrolo[2,3-c]pyridin-7-one (compound T160)

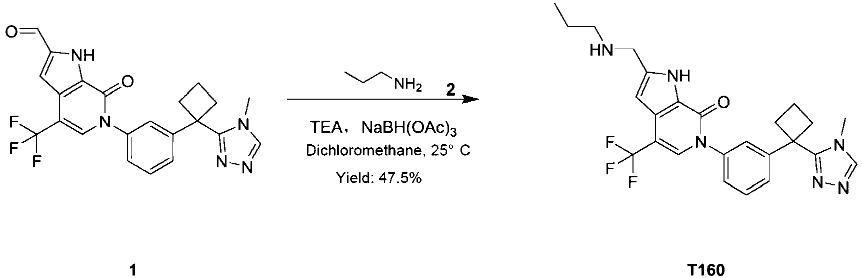

To a mixture of propan-1-amine (13.4 mg, 226 µmol, 18.6 µL) in Dichloromethane (3.00 mL) was added TEA (34.4 mg, 339 µmol, 47.3 µL) adjust pH = 8.00. Then 6-[3-[1-(4-methyl-1,2,4-triazol-3-yl) cyclobutyl] phenyl]-7-oxo-4-(trifluoromethyl)-1H-pyrrolo[2,3-c] pyridine-2-carbaldehyde (50.0 mg, 113 µmol) was added to the mixture. The mixture was stirred under N₂ at 25.0 °C for 0.50 hr. sodium triacetoxyboranuide (60.0 mg, 283 µmol) was added to the mixture. The mixture was stirred under N₂ at 25.0 °C for 11.5 hrs. LCMS showed desired mass was detected. The mixture was concentrated under reduced pressure. Then it was diluted with Methanol (7.00 mL). The crude product was purified by Prep-HPLC (column: Welch Xtimate C18 40.0 * 200 mm 7.00 um; mobile phase: [water (NH₃H₂O+NH₄HCO₃)-ACN]; gradient: 20.0%-60.0% B over 25.0 mins). Compound 6-(3-(1-(4-methyl-4H-1,2,4-triazol-3-yl) cyclobutyl) phenyl)-2-((propylamino)methyl)-4-(trifluoromethyl)-1,6-dihydro-7H-pyrrolo[2,3-c] pyridin-7-one (26.1 mg, 53.8 µmol, 47.5% yield, 100% purity) was obtained as a white solid, which was confirmed by H NMR and LCMS.
¹H NMR: (400 MHz, DMSO-*d*₆)
*δ* 8.35 (s, 1H), 7.71 (d, *J =* 1.2 Hz, 1H), 7.48-7.54 (m, 1H), 7.43 (d, *J =* 1.6 Hz, 1H), 7.34-7.39 (m, 1H), 7.26 (d, *J =* 8.4 Hz, 1H), 6.32 (s, 1H), 3.79 (s, 2H), 3.27 (s, 3H), 2.88-3.00 (m, 2H), 2.63-2.75 (m, 2H), 2.42 (t, *J* = 7.2 Hz, 2H), 1.91-2.05 (m, 2H), 1.41 (sxt, *J* = 7.2 Hz, 2H), 0.86 (t, *J* = 7.2 Hz, 3H)
LCMS: m/z = 485.3 (M+H)⁺, Rₜ = 1.379 min

### Example 161: Synthesis of compound T161

### 1. General steps for preparation of 2-((tert-butylamino)methyl)-6-(3-(1-(4-methyl-4H-1,2,4-triazol-3-yl)cyclobutyl)phenyl)-4-(trifluoromethyl)-1,6-dih ydro-7H-pyrrolo[2,3-c]pyridin-7-one (compound T161)

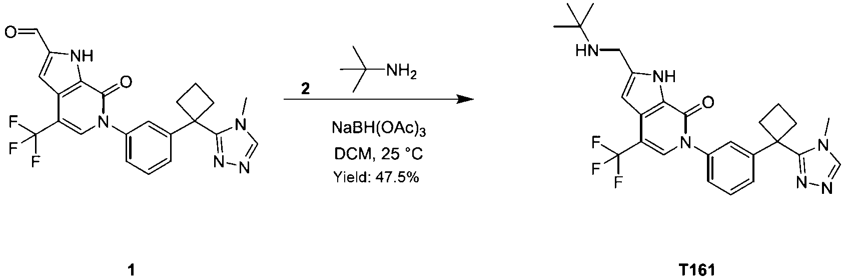

To a solution of 6-(3-(1-(4-methyl-4H-1,2,4-triazol-3-yl)cyclobutyl)phenyl)-7-oxo-4-(trifluoromethyl)-6,7-dihydro-1H-pyrrolo[2,3-c]pyridine-2-carbaldehyde (50.0 mg, 113 µmol) in dichloromethane (2.00 mL) was added 2-methylpropan-2-amine (12.4 mg, 169 µmol, 17.8 µL), stirred at 25 °C for 0.5 hr, then NaBH(OAc)₃ (60.0 mg, 283 µmol) was added to the mixture, stirred at 25 °C for 1 hr. LCMS showed desired compound was detected. The reaction mixture was diluted with aqueous NaHCO₃ (8.00 mL), extracted with dichloromethane (12.0 mL * 3), the combined organic layers were washed with H₂O (8.00 mL), dried over Na₂SO₄, filtered and concentrated under reduced pressure to give a residue. The residue was purified by Prep-HPLC (column: Welch Xtimate C18 40 * 200 mm 7 um; mobile phase: [water(NH₄HCO₃)-ACN]; gradient:24.0%-64.0% B over 25 mins). Compound 2-((tert-butylamino)methyl)-6-(3-(1-(4-methyl-4H-1,2,4-triazol-3-yl)cyclobutyl)phenyl)-4-(trifluoromethyl)-1,6-dih ydro-7H-pyrrolo[2,3-c]pyridin-7-one (27.1 mg, 53.8 µmol, 47.5% yield, 99.1% purity) was obtained as a white solid. Confirmed by H NMR and LCMS.
¹H NMR: (400 MHz, DMSO-*d*₆)
*δ* 10.48-13.60 (m, 1H), 8.35 (s, 1H), 7.70 (d, *J =* 1.2 Hz, 1H), 7.46-7.56 (m, 1H), 7.42 (s, 1H), 7.31-7.39 (m, 1H), 7.26 (d, *J =* 8.0 Hz, 1H), 6.34 (s, 1H), 3.79 (s, 2H), 3.44 (s, 1H), 3.27 (s, 3H), 2.90-2.98 (m, 2H), 2.65-2.75 (m, 2H), 1.87-2.07 (m, 2H), 1.08 (s, 9H)
LCMS: m/z = 499.2 (M+H)⁺, Rt = 1.317 min

### Example 162: Synthesis of compound T162

### 1. General steps for preparation of 6-(3-(1-(4-methyl-4H-1,2,4-triazol-3-yl)cyclobutyl)phenyl)-2-((neopentylamino)methyl)-4-(trifluoromethyl)-1,6-di hydro-7H-pyrrolo[2,3-c]pyridin-7-one (compound T162)

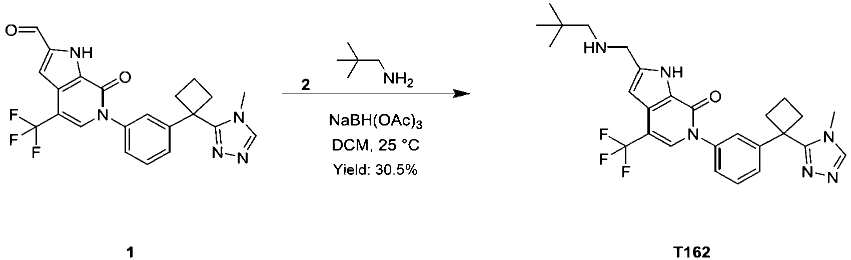

To a solution of 6-(3-(1-(4-methyl-4H-1,2,4-triazol-3-yl)cyclobutyl)phenyl)-7-oxo-4-(trifluoromethyl)-6,7-dihydro-1H-pyrrolo[2,3-c]pyridine-2-carbaldehyde (50.0 mg, 113 µmol) in dichloromethane (2.00 mL) was added 2,2-dimethylpropan-1-amine (14.8 mg, 169 µmol) stirred at 25 °C for 0.5 hr, then NaBH(OAc)₃ (60.0 mg, 283 µmol) was added to the mixture, stirred at 25 °C for 1 hr. LCMS showed desired compound was detected. The reaction mixture was diluted with aqueous NaHCO₃ (8.00 mL), extracted with dichloromethane (12.0 mL * 3), the combined organic layers were washed with H₂O (8.00 mL), dried over Na₂SO₄, filtered and concentrated under reduced pressure to give a residue. The residue was purified by Prep-HPLC (column: Welch Xtimate C18 40 * 200 mm 7 um; mobile phase: [water(NH₄HCO₃)-ACN];gradient:30.0%-70.0% B over 25 mins). Compound 6-(3-(1-(4-methyl-4H-1,2,4-triazol-3-yl)cyclobutyl)phenyl)-2-((neopentylamino)methyl)-4-(trifluoromethyl)-1,6-di hydro-7H-pyrrolo[2,3-c]pyridin-7-one (17.8 mg, 34.5 µmol, 30.5% yield, 99.6% purity) was obtained as a white solid. Confirmed by H NMR and LCMS.
¹H NMR: (400 MHz, DMSO-*d*₆)
*δ* 12.09-12.62 (m, 1H), 8.35 (s, 1H), 7.71 (d, *J =* 1.2 Hz, 1H), 7.47-7.56 (m, 1H), 7.43 (s, 1H), 7.36 (d, *J =* 8.0 Hz, 1H), 7.26 (d, *J =* 8.0 Hz, 1H), 6.32 (s, 1H), 3.81 (s, 2H), 3.27 (s, 3H), 2.89-2.99 (m, 2H), 2.65-2.76 (m, 2H), 2.20 (s, 2H), 1.92-2.03 (m, 2H), 0.86 (s, 9H)
LCMS: m/z = 513.2 (M+H)⁺, Rt = 1.477 min

Other compounds are shown below:

| No. | Structure | Characterization result |
|---|---|---|
| T183 | 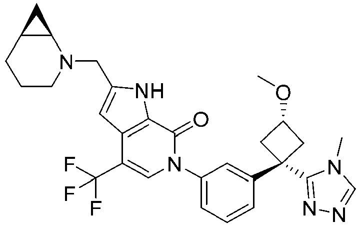 | 1H NMR (DMSO-d6, 400 MHz): *δ* (ppm) 12.45 (s, 1H), 8.38 (s, 1H), 7.70 (d, *J =* 1.6 Hz, 1H), 7.46-7.53 (m, 1H), 7.33-7.38 (m, 2H), 7.18 (d, *J* = 8.0 Hz, 1H), 6.32 (s, 1H), 3.85 (t, *J =* 7.2 Hz, 1H), 3.77-3.81 (m, 1H), 3.67-3.73 (m, 1H), 3.35 (d, *J* = 2.8 Hz, 2H), 3.30 (s, 3H), 3.16 (s, 3H), 2.54 (d, *J =* 2.4 Hz, 2H), 2.40-2.45 (m, 1H), 2.23 (ddd, *J* = 8.4, 6.4, 4.4 Hz, 1H), 2.12-2.18 (m, 1H), 1.84-1.93 (m, 1H), 1.42-1.49 (m, 1H), 1.33-1.40 (m, 2H), 0.92-1.02 (m, 1H), 0.27-0.34 (m, 1H), 0.23-0.27 (m, 1H) |
| | | LCMS: m/z=553.3(M+H)⁺, Rt=1.361 min |
| T184 | 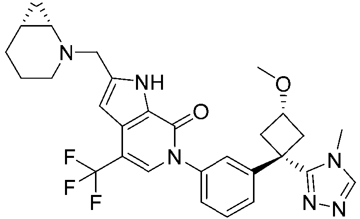 | 1H NMR (DMSO-d6, 400 MHz): *δ* (ppm) 12.45 (s, 1H), 8.38 (s, 1H), 7.70 (d, *J =* 1.6 Hz, 1H), 7.46-7.53 (m, 1H), 7.33-7.38 (m, 2H), 7.18 (d, *J =* 8.0 Hz, 1H), 6.32 (s, 1H), 3.82-3.91 (m, 1H), 3.76-3.82 (m, 1H), 3.67-3.73 (m, 1H), 3.35 (s, 2H), 3.29 (s, 3H), 3.16 (s, 3H), 2.54 (s, 2H), 2.40-2.46 (m, 1H), 2.23 (ddd, *J =* 8.4, 6.4, 4.4 Hz, 1H), 2.15 (ddd, *J* = 11.2, 7.2, 4.4 Hz, 1H), 1.83-1.94 (m, 1H), 1.41-1.49 (m, 1H), 1.37 (d, *J =* 5.2 Hz, 2H), 0.93-1.02 (m, 1H), 0.21-0.34 (m, 2H) |
| | | LCMS: m/z=553.3(M+H)⁺, Rt=1.355min |
| T185 | 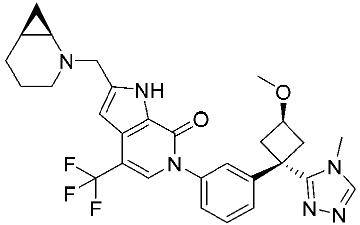 | 1H NMR (DMSO-d6, 400 MHz): *δ* (ppm) 12.46 (s, 1H), 8.30 (s, 1H), 7.71 (d, *J* = 1.2 Hz, 1H), 7.48-7.53 (m, 2H), 7.38 (d, *J =* 8.0 Hz, 1H), 7.32 (d, *J =* 8.0 Hz, 1H), 6.32 (s, 1H), 4.06 (t, *J* = 7.2 Hz, 1H), 3.76-3.83 (m, 1H), 3.67-3.74 (m, 1H), 3.27 (s, 3H), 3.15 (s, 3H), 3.10 (ddd, *J* = 9.6, 7.2, 2.4 Hz, 2H), 2.75-2.81 (m, 2H), 2.40-2.45 (m, 1H), 2.23 (ddd, *J =* 8.4, 6.4, 4.4 Hz, 1H), 2.11-2.18 (m, 1H), 1.83-1.93 (m, 1H), 1.41-1.48 (m, 1H), 1.32-1.40 (m, 2H), 0.98 (t, *J =* 8.4 Hz, 1H), 0.27-0.37 (m, 1H), 0.21-0.27 (m, 1H) |
| | | LCMS: m/z=553.3(M+H)⁺, Rt=1.34 min |
| T186 | 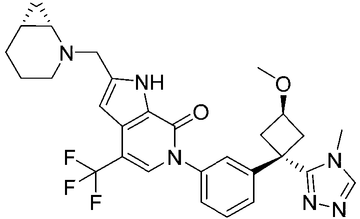 | 1H NMR (DMSO-d6, 400 MHz): *δ* (ppm) 12.46 (s, 1H), 8.30 (s, 1H), 7.71 (d, *J =* 1.2 Hz, 1H), 7.48-7.54 (m, 2H), 7.35-7.40 (m, 1H), 7.32 (d, J = 8.4 Hz, 1H), 6.32 (s, 1H), 4.06 (quin, *J =* 7.2 Hz, 1H), 3.66-3.82 (m, 2H), 3.27 (s, 3H), 3.15 (s, 3H), 3.07-3.13 (m, 2H), 2.78 (ddd, J = 10.0, 7.6, 2.4 Hz, 2H), 2.40-2.46 (m, 1H), 2.23 (ddd, *J* = 8.4, 6.4, 4.4 Hz, 1H), 2.11-2.18 (m, 1H), 1.84-1.94 (m, 1H), 1.41-1.48 (m, 1H), 1.32-1.40 (m, 2H), 0.93-1.02 (m, 1H), 0.28-0.34 (m, 1H), 0.23-0.27 (m, 1H) |
| | | LCMS: m/z=553.3(M+H)⁺, Rt=1.341 min |
| T192 | 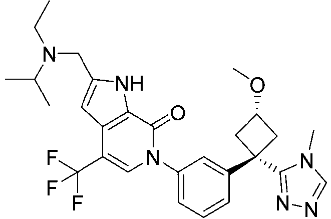 | 1H NMR (DMSO-d6, 400 MHz): *δ* (ppm) 12.34 (br s, 1H), 8.38 (s, 1H), 7.70 (s, 1H), 7.46-7.53 (m, 1H), 7.32-7.40 (m, 2H), 7.18 (d, J = 8.0 Hz, 1H), 6.28 (s, 1H), 3.85 (t, *J =* 7.2 Hz, 1H), 3.65 (s, 2H), 3.31-3.36 (m, 4H), 3.30 (s, 3H), 3.16 (s, 3H), 2.94 (dt, *J =* 13.2, 6.4 Hz, 1H), 2.42-2.49 (m, 2H), 0.94-1.01 (m, 9H) |
| | | LCMS: m/z=543.3(M+H)⁺, Rt=2.197 min |
| T193 | 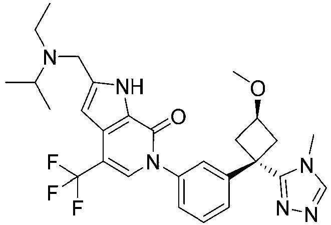 | 1H NMR (DMSO-d6, 400 MHz): δ (ppm) 12.36 (br s, 1H), 8.30 (s, 1H), 7.71 (d, *J* = 1.6 Hz, 1H), 7.48-7.55 (m, 2H), 7.29-7.40 (m, 2H), 6.29 (s, 1H), 4.06 (t, *J =* 7.2 Hz, 1H), 3.65 (s, 2H), 3.27 (s, 3H), 3.15 (s, 3H), 3.10 (ddd, *J =* 10.0, 7.2, 2.4 Hz, 2H), 2.89-2.99 (m, 1H), 2.72-2.85 (m, 2H), 2.43-2.48 (m, 2H), 0.93-1.01 (m, 9H) |
| | | LCMS: m/z=543.3(M+H)⁺, Rt=2.16min |

### Activity Test Examples

### Example 1: CBL-B Binding Experiment

### Experimental Materials:

In the CBL-B binding experiment, HEPES is purchased from Sigma (V900477-500G), BSA from Miltenyi Biotec (Cat#130-091-376), and Streptavidin-Tb cryptate from Cisbio (Cat # 610SADLA). CBL-B protein and Ligand L1 are provided by Zhuhai Yufan.

The structure of L1 is as follows, prepared with reference to Example 168 of Patent Document WO2021021761.

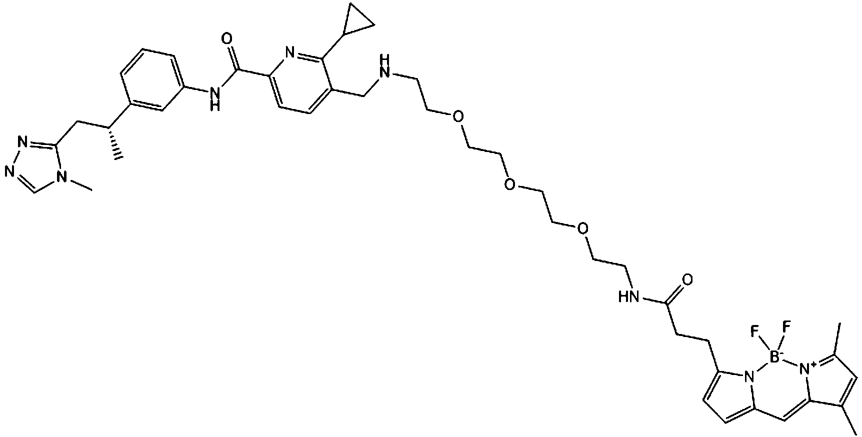

### Experimental Methods:

The experimental reactions are conducted in a 384-well plate (Greiner, Cat #784075), with a total reaction system of 15 ul. The reaction system mainly includes 1× buffer, 20 mM HEPES pH 7.5, 150 mM NaCl, 0.01% Triton X-100, 0.5 mM TCEP, and 0.1% ug/ml BSA. The compound is continuously diluted with DMSO for 10 concentration points, and 150 nL is transferred to the experimental plate. The experimental reaction begins after adding 8 nM of CBL-B protein and the reaction is carried out at 25°C for 60 minutes. After adding 5 µL of Ligand L1, 5 µL of the Streptavidin-Tb cryptate detection reagent is added. After an additional 60 minutes of incubation at room temperature, the experimental plate is read using an EnVision plate.

### Data Analysis:

The luminescence signal values were converted to percentage inhibition rates. Inhibition % = (Sample value - Maximum value)/(Minimum value - Maximum value) × 100. The minimum value refers to the measured value in the wells without CBL-B protein but with DMSO and Ligand, while the maximum value refers to the measured value in the wells with DMSO, CBL-B protein, and Ligand. The IC50 values are calculated by XLFit (version 5.3.1.3) in Excel. Equation: Y = Bottom + (Top - Bottom)/(1 + (IC50/X) ^HillSlope); Ki = Relative IC50/2.

### Results:

According to the above experimental methods, the Ki values of the compounds measured are as follows:

**Table 1 Test Results**

| Compound No. | cbl-b binding Ki/nM | Compound No. | cbl-b binding Ki/nM | Compoun d No. | cbl-b binding Ki/nM | Compou nd No. | cbl-b binding Ki/nM |
|---|---|---|---|---|---|---|---|
| T001 | <50nM | T043 | <50nM | T086 | <50nM | T128 | <50nM |
| T002 | <50nM | T044 | <50nM | T087 | <50nM | T129 | <50nM |
| T003 | <50nM | T045 | <50nM | T088 | <50nM | T130 | <50nM |
| T004 | <50nM | T046 | <50nM | T089 | <50nM | T131 | <50nM |
| T005 | <50nM | T047 | <50nM | T090 | <50nM | T132 | <50nM |
| T006 | <50nM | T048 | <50nM | T091 | <50nM | T133 | <50nM |
| T007 | <50nM | T049 | <50nM | T092 | <50nM | T134 | <50nM |
| T008 | <50nM | T050 | <50nM | T093 | <50nM | T135 | <50nM |
| T009 | <50nM | T051 | <50nM | T094 | <50nM | T136 | <50nM |
| T010 | <50nM | T052 | <50nM | T095 | <50nM | T137 | <50nM |
| T011 | <50nM | T053 | <50nM | T096 | <50nM | T138 | <50nM |
| T012 | <50nM | T054 | <50nM | T097 | <50nM | T139 | <50nM |
| T013 | <50nM | T055 | <50nM | T098 | <50nM | T140 | <50nM |
| T014 | <50nM | T056 | <50nM | T099 | <50nM | T141 | <50nM |
| T015 | <50nM | T057 | <50nM | T100 | <50nM | T142 | <50nM |
| T016 | <50nM | T058 | <50nM | T101 | <50nM | T143 | <50nM |
| T017 | <50nM | T059 | <50nM | T102 | <50nM | T144 | <50nM |
| T018 | <50nM | T060 | <50nM | T103 | <50nM | T145 | <50nM |
| T019 | <50nM | T061 | <50nM | T104 | <50nM | T146 | <50nM |
| T020 | <50nM | T062 | <50nM | T105 | <50nM | T147 | <50nM |
| T021 | <50nM | T063 | <50nM | T106 | <50nM | T148 | <50nM |
| T022 | <50nM | T064 | <50nM | T107 | <50nM | T149 | <50nM |
| T023 | <50nM | T065 | <50nM | T108 | <50nM | T150 | <50nM |
| T024 | <50nM | T066 | <50nM | T109 | <50nM | T151 | <50nM |
| T025 | <50nM | T067 | <50nM | T110 | <50nM | T152 | <50nM |
| T026 | <50nM | T069 | <50nM | T111 | <50nM | T153 | <50nM |
| T027 | <50nM | T070 | <50nM | T112 | <50nM | T154 | <50nM |
| T028 | <50nM | T071 | <50nM | T113 | <50nM | T155 | <50nM |
| T029 | <50nM | T072 | <50nM | T114 | <50nM | T156 | <50nM |
| T030 | <50nM | T073 | <50nM | T115 | 50nM<Ki<100nM | T157 | <50nM |
| T031 | <50nM | T074 | <50nM | T116 | 50nM<Ki<100nM | T158 | <50nM |
| T032 | <50nM | T075 | <50nM | T117 | <50nM | T159 | <50nM |
| T033 | <50nM | T076 | <50nM | T118 | <50nM | T160 | <50nM |
| T034 | <50nM | T077 | <50nM | T119 | <50nM | T161 | <50nM |
| T035 | <50nM | T078 | <50nM | T120 | <50nM | T162 | <50nM |
| T036 | 50nM<Ki<100n M | T079 | <50nM | T121 | <50nM | T183 | <50nM |
| T037 | <50nM | T080 | <50nM | T122 | <50nM | T184 | <50nM |
| T038 | <50nM | T081 | <50nM | T123 | <50nM | T185 | <50nM |
| T039 | <50nM | T082 | <50nM | T124 | 100nM<Ki<500n M | T186 | <50nM |
| T040 | <50nM | T083 | <50nM | T125 | <50nM | T192 | <50nM |
| T041 | <50nM | T084 | <50nM | T126 | <50nM | T193 | <50nM |
| T042 | <50nM | T085 | <50nM | T127 | <50nM | | |

As shown in the above table, the compound of the present disclosure may bind well to the CBL-B protein.

### Example 2: Effect of Compounds on the Level of IL-2 Secretion by Anti-CD3 Activated Human PBMCs

### Experimental Operations

1. Take hPBMCs cultured overnight, centrifuge to discard the supernatant of the culture medium, resuspend the centrifuged cells in fresh complete medium, and adjust the cell concentration to 1.5×10⁶/ml;
2. Inoculate the resuspended cells into a 96-well cell culture plate at 200ul/well of cell suspension, and incubate in a 37°C cell incubator for 1h;
3. Adjust the compound concentration with DMSO, starting at a final concentration of 3 µM, and perform a 3-fold dilution sequentially for 10 concentration gradients, with an additional DMSO control well set up; and
4. Adjust the CD3 antibody to an appropriate concentration to achieve a final concentration of 2 µg/mL, mix thoroughly, and incubate in a 37°C cell incubator for 48h.
5. After 48h, centrifuge at 1500 rpm for 5 min to collect the supernatant of the culture medium for the human IL-2 ELISA test.

### Data Analysis:

The OD values obtained from the ELISA test are converted into the fold change (FC) in IL-2 concentration. Fold change = (IL-2 OD values secreted from cells induced by anti-CD3 at different concentrations of the compound)/(IL-2 OD values secreted from DMSO group cells induced by anti-CD3). The EC₅₀ is calculated using GraphPad Prism. When the fold change = 5, the corresponding compound concentration is C_{FC=5} (in nM).

### Results:

**Table 2 Test Results**

| Compou nd No. | IL-2 C_{FC=5} /nM | Compou nd No. | IL-2 C_{FC=5} /nM | Compou nd No. | IL-2 C_{FC=5} /nM | Compou nd No. | IL-2 C_{FC=5} /nM |
|---|---|---|---|---|---|---|---|
| T002 | <500nM | T039 | 500nM<C_{FC=5} <3000nM | T085 | <500nM | T134 | <500nM |
| T005 | <500nM | T040 | <500nM | T086 | <500nM | T135 | <500nM |
| T007 | <500nM | T041 | <500nM | T087 | <500nM | T136 | <500nM |
| T008 | <500nM | T042 | <500nM | T088 | <500nM | T137 | <500nM |
| T009 | <500nM | T043 | <500nM | T090 | 500nM<C_{FC=5} <3000nM | T138 | 500nM<C_{FC=5} <3000nM |
| T010 | <500nM | T045 | <500nM | T091 | <500nM | T139 | <500nM |
| T011 | <500nM | T047 | <500nM | T092 | <500nM | T140 | <500nM |
| T013 | <500nM | T051 | 500nM<C_{FC=5} <3000nM | T093 | <500nM | T142 | 500nM<C_{FC=5} <3000nM |
| T014 | <500nM | T053 | <500nM | T096 | <500nM | T143 | 500nM<C_{FC=5} <3000nM |
| T016 | 3000nM<C_{FC=5} <5000nM | T054 | <500nM | T097 | <500nM | T144 | 500nM<C_{FC=5} <3000nM |
| T017 | <500nM | T055 | <500nM | T099 | 500nM<C_{FC=5} <3000nM | T145 | <500nM |
| T018 | <500nM | T056 | <500nM | T102 | <500nM | T147 | <500nM |
| T019 | <500nM | T058 | <500nM | T103 | <500nM | T149 | <500nM |
| T020 | <500nM | T060 | <500nM | T104 | <500nM | T151 | <500nM |
| T021 | <500nM | T061 | 500nM<C_{FC=5} <3000nM | T109 | <500nM | T152 | <500nM |
| T023 | <500nM | T062 | 3000nM<C_{FC=5} <5000nM | T111 | <500nM | T154 | <500nM |
| T024 | <500nM | T063 | 3000nM<C_{FC=5} <5000nM | T114 | <500nM | T156 | <500nM |
| T025 | <500nM | T065 | 500nM<C_{FC=5} <3000nM | T117 | 500nM<C_{FC=5} <3000nM | T157 | <500nM |
| T026 | <500nM | T066 | <500nM | T119 | <500nM | T158 | <500nM |
| T027 | <500nM | T067 | <500nM | T120 | <500nM | T159 | <500nM |
| T028 | <500nM | T069 | <500nM | T121 | <500nM | T160 | <500nM |
| T029 | <500nM | T070 | <500nM | T122 | <500nM | T161 | <500nM |
| T030 | <500nM | T071 | <500nM | T126 | <500nM | T162 | <500nM |
| T033 | <500nM | T077 | <500nM | T127 | 500nM<C_{FC=5} <3000nM | T183 | <500nM |
| T034 | <500nM | T078 | 500nM<C_{FC=5} <3000nM | T129 | 500nM<C_{FC=5} <3000nM | T185 | <500nM |
| T035 | <500nM | T080 | <500nM | T130 | <500nM | T192 | <500nM |
| T037 | <500nM | T082 | 500nM<C_{FC=5} <3000nM | T131 | <500nM | | |
| T038 | <500nM | T084 | <500nM | T133 | 500nM<C_{FC=5} <3000nM | | |

As shown in the above table, the compounds of the present disclosure can effectively promote IL-2 secretion at the cellular level.

The above description is only a preferred example of the present disclosure and is not intended to limit the present disclosure. Any modifications, equivalent substitutions, and the like made within the spirit and principles of the present disclosure shall be included in the scope of protection of the present disclosure.

The aforementioned examples and methods described in the present disclosure may vary based on the capabilities, experience, and preferences of those skilled in the art.

The listing of the method steps in a certain order in the present disclosure does not constitute any limitation on the sequence of the method steps.

## Claims

1. A compound, or a pharmaceutically acceptable salt, stereoisomer, ester, prodrug, solvate, or deuterated compound thereof, having the following structure: 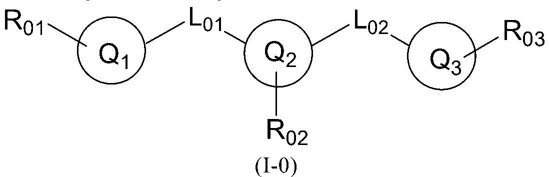 wherein,
Q₁-ring is an aliphatic ring, an aromatic ring, or a heterocyclic ring;
Q₂-ring is an aliphatic ring, an aromatic ring, or a heterocyclic ring;
Q₃-ring is an aliphatic ring, an aromatic ring, or a heterocyclic ring;
R₀₁, R₀₂, and R₀₃ are each one or more independent substituents on the Q₁-ring, Q₂-ring, and Q₃-ring, they are each selected from: H, D, =O, halogen, cyano, nitro, azido, -OR₀₄, -C(O)R₀₄, -C(O)OR₀₄, -NR₀₅(O)OR₀₄, -OC(O)R₀₄, -NR₀₅SO₂R₀₄, -SO₂NR₀₄R₀₅, -NR₀₅C(O)R₀₄, -C(O)NR₀₄R₀₅, -NR₀₄R₀₅, -SR₀₄, -S(O)R₀₄, -S(O)₂R₀₄, -SO₃H, C₁-C₁₀ alkyl, C₂-C₁₀ alkenyl, C₂-C₁₀ alkynyl, -(CH₂)ₜ₁(C₆-C₁₀ aryl), -SO₂(CH₂)ₜ₁(C₆-C₁₀ aryl), -S(CH₂)ₜ₁(C₆-C₁₀ aryl), -O(CH₂)ₜ₁(C₆-C₁₀ aryl), -(CH₂)ₜ₁(4-10 membered heterocyclyl), -SO₂(CH₂)ₜ₁(4-10 membered heterocyclyl), -S(CH₂)ₜ₁(4-10 membered heterocyclyl), -O(CH₂)ₜ₁(4-10 membered heterocyclyl), -(CH₂)ₜ₁(C₃-C₁₀ cycloalkyl), -SO₂(CH₂)ₜ₁(C₃-C₁₀ cycloalkyl), -S(CH₂)ₜ₁(C₃-C₁₀ cycloalkyl), and -O(CH₂)ₜ₁(C₃-C₁₀ cycloalkyl), and t1 is an integer selected from 0 to 10 (such as 0, 1, 2, 3, 4, 5, 6, 7, 8, 9, and 10); wherein 0-6 methylene units in the C₁-C₁₀ alkyl are optionally substituted with groups selected from: -Cy-, -O-, -S-, -S-S-, -Si-, -C(O)-, -C(S)-, -C(O)O-, -OC(O)-, -OC(O)O-, -C(O)N(R₀₄)-, -N(R₀₄)C(O)-, -N(R₀₄C(O)O-, -N(R₀₄)C(O)N(R₀₅)-, -N(R₀₄)-, -S(O)₂-, -S(O)₂N(R₀₄)-, -N(R₀₄)S(O)₂-, -S(O)-, -S(O)N(R₀₄)-, -N(R₀₄)S(O)-, -OP(O)(OR₀ₐ)O-, -P(O)(OR₀₄)O-, -P(O)-, -OP(O)N(R₀₄)-, -P(O)N(R₀₄)-, -P(O)(N(R₀₄R₀₅))-, -OP(O)(OR₀₄)₂N(R₀₅)-, -P(O)(OR₀₄)₂N(R₀₅)-, -N(R₀₄)P(O)(OR₀₅)O-, -N(R₀₄)P(O)-, 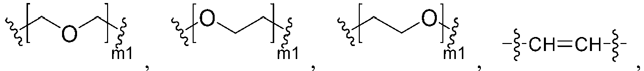 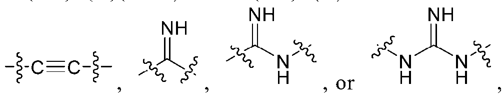 and m1 is an integer selected from 1 to 10 (such as 1, 2, 3, 4, 5, 6, 7, 8, 9, and 10); wherein H on the C₁-C₂₀ alkyl, C₂-C₁₀ alkenyl, C₂-C₁₀ alkynyl, C₃-C₁₀ cycloalkyl, C₆-C₁₀ aryl, and 4-10 membered heterocyclyl is optionally substituted with one or more R₀₆ groups;
each R₀₄ and R₀₅ are independently selected from: H, D, C₁-C₁₀ alkyl, C₂-C₁₀ alkenyl, C₂-C₁₀ alkynyl, -(CH₂)ₜ₁(C₆-C₁₀ aryl), -(CH₂)ₜ₁(4-10 membered heterocyclyl), and -(CH₂)ₜ₁(C₃-C₁₀ cycloalkyl), and t1 is an integer selected from 1 to 10 (such as 1, 2, 3, 4, 5, 6, 7, 8, 9, and 10); wherein H on the C₁-C₁₀ alkyl, C₂-C₁₀ alkenyl, C₂-C₁₀ alkynyl, C₃-C₁₀ cycloalkyl, C₆-C₁₀ aryl, and 4-10 membered heterocyclyl is optionally substituted with one or more R₀₆ groups;
each R₀₆ is selected from: D, halogen, cyano, nitro, azido, C₁-C₁₀ alkyl, C₂-C₁₀ alkenyl, C₂-C₁₀ alkynyl, C₁-C₁₀ haloalkyl, -OR₀₇, -C(O)R₀₇, -C(O)OR₀₇, -NR₀₈C(O)OR₀₇, -OC(O)R₀₇, -NR₀₈SO₂R₀₇, -SO₂NR₀₇R₀₈, -NR₀₇C(O)R₀₈, -C(O)NR₀₇R₀₈, -NR₀₇R₀₈, -SR₀₇, -S(O)R₀₇, -S(O)₂R₀₇, -SO₃H, -(CH₂)ₜ₂(C₆-C₁₀ aryl), -SO₂(CH₂)ₜ₂(C₆-C₁₀ aryl), -S(CH₂)ₜ₂(C₆-C₁₀ aryl), -O(CH₂)ₜ₂(C₆-C₁₀ aryl), -(CH₂)ₜ₂(4-10 membered heterocyclyl), -SO₂(CH₂)ₜ₂(4-10 membered heterocyclyl), -S(CH₂)ₜ₂(4-10 membered heterocyclyl), -O(CH₂)ₜ₂(4-10 membered heterocyclyl), -(CH₂)ₜ₂(C₃-C₁₀ cycloalkyl), -SO₂(CH₂)ₜ₂(C₃-C₁₀ cycloalkyl), -S(CH₂)ₜ₂(C₃-C₁₀ cycloalkyl), and -O(CH₂)ₜ₂(C₃-C₁₀ cycloalkyl), and t2 is an integer selected from 1 to 10 (such as 0, 1, 2, 3, 4, 5, 6, 7, 8, 9, and 10); each R₀₇ and R₀₈ are independently selected from: H, C₁-C₁₀ alkyl, C₂-C₁₀ alkenyl, C₂-C₁₀ alkynyl, -(CH₂)ₜ(C₆-C₁₀ aryl), -(CH₂)ₜ(4-10 membered heterocyclyl), and -(CH₂)ₜ(C₃-C₁₀ cycloalkyl);
each R₀₇ and R₀₈ are independently selected from: H, D, C₁-C₁₀ alkyl, C₂-C₁₀ alkenyl, C₂-C₁₀ alkynyl, -(CH₂)ₜ₃(C₆-C₁₀ aryl), -(CH₂)ₜ₃(4-10 membered heterocyclyl), and -(CH₂)ₜ₃(C₃-C₁₀ cycloalkyl), and t3 is an integer selected from 1 to 10 (such as 0, 1, 2, 3, 4, 5, 6, 7, 8, 9, and 10); wherein H on the C₁-C₁₀ alkyl, C₂-C₁₀ alkenyl, C₂-C₁₀ alkynyl, C₃-C₁₀ cycloalkyl, C₆-C₁₀ aryl, and 4-10 membered heterocyclyl is optionally substituted with D, halogen, cyano, nitro, azido, C₁-C₁₀ alkyl, -N(C₀-C₁₀ alkyl)(C₀-C₁₀ alkyl), -O(C₀-C₁₀ alkyl), -C(O)(C₀-C₁₀ alkyl), -C(O)O(C₀-C₁₀ alkyl), -OC(O)(C₀-C₁₀ alkyl), -C(O)N(C₀-C₁₀ alkyl)(C₀-C₁₀ alkyl), -N(C₀-C₁₀ alkyl)C(O)(C₀-C₁₀ alkyl), -SO₂N(C₀-C₁₀ alkyl)(C₀-C₁₀ alkyl), -N(C₀-C₁₀ alkyl)SO₂(C₀-C₁₀ alkyl), -OCH₂F, -OCHF₂, -OCF₃, C₃-C₁₀ cycloalkyl, C₆-C₁₀ aryl, or 4-10 membered heterocyclyl;
L₀₁ and L₀₂ are linking groups each independently selected from a single bond and C₁-C₁₀ alkylidene, wherein 0-6 methylene units in the C₁-C₁₀ alkyl are optionally substituted with groups selected from: -Cy-, -O-, -S-, -S-S-, -Si-, -C(O)-, -C(S)-, -C(O)O-, -OC(O)-, -OC(O)O-, -C(O)N(R_{L1})-, -N(R_{L1})C(O)-, -N(R_{L1})C(O)O-, -N(R_{L1})C(O)N(R_{L2})-, -N(R_{L1})-, -S(O)₂-, -S(O)₂N(R_{L1})-, -N(R_{L1})S(O)₂-, -S(O)-, -S(O)N(R_{L1})-, -N(R_{L1})S(O)-, , -S(O)-, -S(O)N(R_{L1})-, -N(R_{L1})S(O)-, -OP(O)(OR_{L1})O-, -P(O)(OR_{L1})O-, -P(O)-, -OP(O)N(R_{L1})-, -P(O)N(R_{L1})-, -P(O)(N(R_{L1}R₁₂))-, -OP(O)(OR_{L1})₂N(R_{L1})-, -P(O)(OR_{L1})₂N(R_{L2})-, -N(R_{L1})P(O)(OR_{L2})O-, -N(R_{L1})P(O)-, 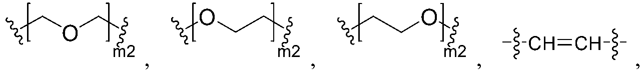 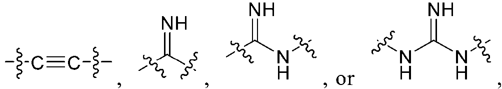 and m2 is an integer selected from 1 to 10 (such as 1, 2, 3, 4, 5, 6, 7, 8, 9, and 10); wherein H on the C₁-C₁₀ alkyl is optionally substituted with one or more R_{L3} groups;
each R_{L1} and R_{L2} are independently selected from: H, D, C₁-C₁₀ alkyl, C₂-C₁₀ alkenyl, C₂-C₁₀ alkynyl, -(CH₂)ₜ₄(C₆-C₁₀ aryl), -(CH₂)ₜ₄(4-10 membered heterocyclyl), and -(CH₂)ₜ₄(C₃-C₁₀ cycloalkyl), and t4 is an integer selected from 1 to 10 (such as 0, 1, 2, 3, 4, 5, 6, 7, 8, 9, and 10); wherein H on the C₁-C₁₀ alkyl, C₂-C₁₀ alkenyl, C₂-C₁₀ alkynyl, C₃-C₁₀ cycloalkyl, C₆-C₁₀ aryl, and 4-10 membered heterocyclyl is optionally substituted with groups selected from: D, halogen, cyano, nitro, azido, C₁-C₁₀ alkyl, -N(C₀-C₁₀ alkyl)(C₀-C₁₀ alkyl), -O(C₀-C₁₀ alkyl), -C(O)(C₀-C₁₀ alkyl), -C(O)O(C₀-C₁₀ alkyl), -OC(O)(C₀-C₁₀ alkyl), -C(O)N(C₀-C₁₀ alkyl)(C₀-C₁₀ alkyl), -N(C₀-C₁₀ alkyl)C(O)(C₀-C₁₀ alkyl), -SO₂N(C₀-C₁₀ alkyl)(C₀-C₁₀ alkyl), -N(C₀-C₁₀ alkyl)SO₂(C₀-C₁₀ alkyl), -OCH₂F, -OCHF₂, -OCF₃, C₃-C₁₀ cycloalkyl, C₆-C₁₀ aryl, or 4-10 membered heterocyclyl;
R_{L3} is selected from: D, halogen, cyano, nitro, azido, C₁-C₁₀ alkyl, C₂-C₁₀ alkenyl, C₂-C₁₀ alkynyl, -(CH₂)ₜ₅(C₆-C₁₀ aryl), -(CH₂)ₜ₅(4-10 membered heterocyclyl), and -(CH₂)ₜ₅(C₃-C₁₀ cycloalkyl), and t5 is an integer selected from 1 to 10 (such as 0, 1, 2, 3, 4, 5, 6, 7, 8, 9, and 10); wherein H on the C₁-C₁₀ alkyl, C₂-C₁₀ alkenyl, C₂-C₁₀ alkynyl, C₃-C₁₀ cycloalkyl, C₆-C₁₀ aryl, and 4-10 membered heterocyclyl is optionally substituted with groups selected from: D, halogen, cyano, nitro, azido, C₁-C₁₀ alkyl, -N(C₀-C₁₀ alkyl)(C₀-C₁₀ alkyl), -O(C₀-C₁₀ alkyl), -C(O)(C₀-C₁₀ alkyl), -C(O)O(C₀-C₁₀ alkyl), -OC(O)(C₀-C₁₀ alkyl), -C(O)N(C₀-C₁₀ alkyl)(C₀-C₁₀ alkyl), -N(C₀-C₁₀ alkyl)C(O)(C₀-C₁₀ alkyl), -SO₂N(C₀-C₁₀ alkyl)(C₀-C₁₀ alkyl), -N(C₀-C₁₀ alkyl)SO₂(C₀-C₁₀ alkyl), -OCH₂F, -OCHF₂, -OCF₃, C₃-C₁₀ cycloalkyl, C₆-C₁₀ aryl, or 4-10 membered heterocyclyl; and
each -Cy- is independently an optionally substituted divalent ring selected from: arylene, cycloalkylene, or heterocyclylene.

2. The compound according to claim 1, wherein the Q₁-ring, containing a lactam structure, is a monocyclic or bicyclic heterocycle, preferably selected from: 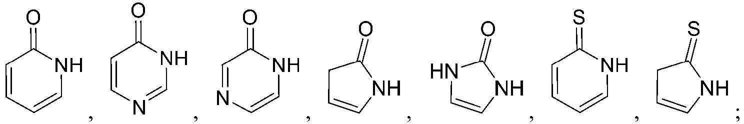 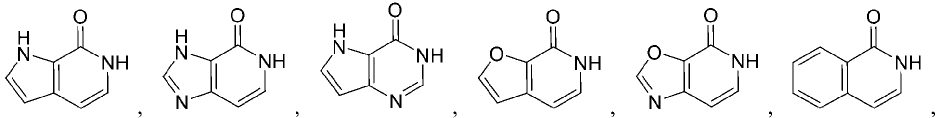 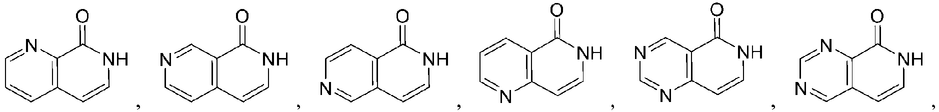 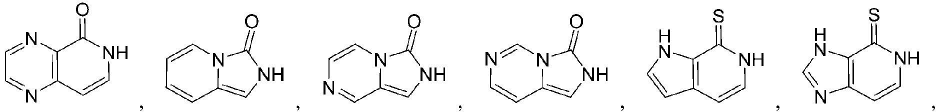 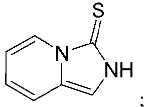
preferably, 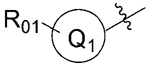 moiety is 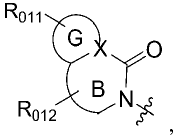 more preferably 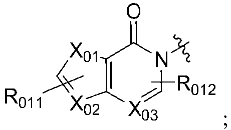 wherein, the definition of R₀₁₁ and R₀₁₂ is identical to that of R₀₁, X₀₁ is selected from a group consisting of CH₂, NH, O, C(O), 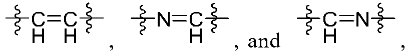 and X₀₂ and X₀₃ are independently selected from CH and N;
more preferably, R₀₁₁ contains the following group: 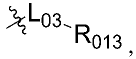 wherein, L₀₃ is selected from a group consisting of a single bond, -O-, -S-, -C(O)-, -CH(R_{L1})-, and -OCH(R_{L1})-, and R₀₁₃ is -NR₀₁₄R₀₁₅ or substituted or unsubstituted nitrogen-containing heterocyclyl;
R₀₁₄ and R₀₁₅ are independently selected from H, D, and C₁-C₁₀ alkyl; wherein 0-6 methylene units in the C₁-C₁₀ alkyl are optionally substituted with groups selected from: -O-, -S-, -S-S-, -Si-, -C(O)-, -C(S)-, -C(O)O-, -OC(O)-, -OC(O)O-, -C(O)N(C₀-C₁₀ alkyl)-, -N(C₀-C₁₀ alkyl)C(O)-, -N(C₀-C₁₀ alkyl)C(O)O-, -N(C₀-C₁₀ alkyl)C(O)N(C₀-C₁₀ alkyl)-, -N(C₀-C₁₀ alkyl)-, -S(O)₂-, -S(O)₂N(C₀-C₁₀ alkyl)-, -N(C₀-C₁₀ alkyl)S(O)₂-, C₃-C₁₀ cycloalkylene (such as 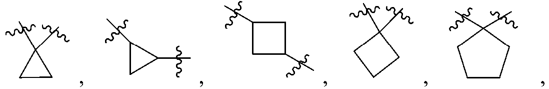 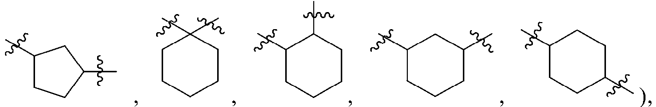 C₆-C₁₀ arylene (e.g., phenylene), or 4-10 membered heterocyclylene; wherein H on the C₁-C₁₀ alkyl is optionally substituted with groups selected from: D, halogen, cyano, nitro, azido, -N(C₀-C₁₀ alkyl)(C₀-C₁₀ alkyl), -O(C₀-C₁₀ alkyl), -C(O)(C₀-C₁₀ alkyl), -C(O)O(C₀-C₁₀ alkyl), -OC(O)(C₀-C₁₀ alkyl), -C(O)N(C₀-C₁₀ alkyl)(C₀-C₁₀ alkyl), -N(C₀-C₁₀ alkyl)C(O)(C₀-C₁₀ alkyl), -SO₂N(C₀-C₁₀ alkyl)(C₀-C₁₀ alkyl), -N(C₀-C₁₀ alkyl)SO₂(C₀-C₁₀ alkyl), -OCH₂F, -OCHF₂, -OCF₃, C₃-C₁₀ cycloalkyl, C₆-C₁₀ aryl, or 4-10 membered heterocyclyl; and H on the C₃-C₁₀ cycloalkylene, C₆-C₁₀ arylene, and 4-10 membered heterocyclylene is optionally substituted with groups selected from: D, halogen, cyano, nitro, azido, -N(C₀-C₁₀ alkyl)(C₀-C₁₀ alkyl), -O(C₀-C₁₀ alkyl), -C(O)(C₀-C₁₀ alkyl), -C(O)O(C₀-C₁₀ alkyl), -OC(O)(C₀-C₁₀ alkyl), -C(O)N(C₀-C₁₀ alkyl)(C₀-C₁₀ alkyl), -N(C₀-C₁₀ alkyl)C(O)(C₀-C₁₀ alkyl), -SO₂N(C₀-C₁₀ alkyl)(C₀-C₁₀ alkyl), -N(C₀-C₁₀ alkyl)SO₂(C₀-C₁₀ alkyl), -OCH₂F, -OCHF₂, -OCF₃, C₃-C₁₀ cycloalkyl, C₆-C₁₀ aryl, or 4-10 membered heterocyclyl;
H on the nitrogen-containing heterocyclyl is optionally substituted with groups selected from: D, halogen, cyano, nitro, azido, C₁-C₁₀ alkyl, -N(C₀-C₁₀ alkyl)(C₀-C₁₀ alkyl), -O(C₀-C₁₀ alkyl), -C(O)(C₀-C₁₀ alkyl), -C(O)O(C₀-C₁₀ alkyl), -OC(O)(C₀-C₁₀ alkyl), -C(O)N(C₀-C₁₀ alkyl)(C₀-C₁₀ alkyl), -N(C₀-C₁₀ alkyl)C(O)(C₀-C₁₀ alkyl), -SO₂N(C₀-C₁₀ alkyl)(C₀-C₁₀ alkyl), -N(C₀-C₁₀ alkyl)SO₂(C₀-C₁₀ alkyl), -OCH₂F, -OCHF₂, -OCF₃, C₃-C₁₀ cycloalkyl, C₆-C₁₀ aryl, or 4-10 membered heterocyclyl; wherein H on the C₁-C₁₀ alkyl, C₃-C₁₀ cycloalkyl, C₆-C₁₀ aryl, and 4-10 membered heterocyclyl is optionally substituted with groups selected from: D, halogen, cyano, nitro, azido, -N(C₀-C₁₀ alkyl)(C₀-C₁₀ alkyl), -O(C₀-C₁₀ alkyl), -C(O)(C₀-C₁₀ alkyl), -C(O)O(C₀-C₁₀ alkyl), -OC(O)(C₀-C₁₀ alkyl), -C(O)N(C₀-C₁₀ alkyl)(C₀-C₁₀ alkyl), -N(C₀-C₁₀ alkyl)C(O)(C₀-C₁₀ alkyl), -SO₂N(C₀-C₁₀ alkyl)(C₀-C₁₀ alkyl), -N(C₀-C₁₀ alkyl)SO₂(C₀-C₁₀ alkyl), -OCH₂F, -OCHF₂, -OCF₃, C₃-C₁₀ cycloalkyl, C₆-C₁₀ aryl, or 4-10 membered heterocyclyl; and
R₀₁₂ is selected from: H, D, halogen, cyano, nitro, azido, C₁-C₁₀ alkyl, -N(C₀-C₁₀ alkyl)(C₀-C₁₀ alkyl), -O(C₀-C₁₀ alkyl), -C(O)(C₀-C₁₀ alkyl), -C(O)O(C₀-C₁₀ alkyl), -OC(O)(C₀-C₁₀ alkyl), -C(O)N(C₀-C₁₀ alkyl)(C₀-C₁₀ alkyl), -N(C₀-C₁₀ alkyl)C(O)(C₀-C₁₀ alkyl), -SO₂N(C₀-C₁₀ alkyl)(C₀-C₁₀ alkyl), -N(C₀-C₁₀ alkyl)SO₂(C₀-C₁₀ alkyl), -OCH₂F, -OCHF₂, -OCF₃, C₃-C₁₀ cycloalkyl, C₆-C₁₀ aryl, and 4-10 membered heterocyclyl; wherein H on the C₁-C₁₀ alkyl, C₃-C₁₀ cycloalkyl, C₆-C₁₀ aryl, and 4-10 membered heterocyclyl is optionally substituted with groups selected from: D, halogen, cyano, nitro, azido, -N(C₀-C₁₀ alkyl)(C₀-C₁₀ alkyl), -O(C₀-C₁₀ alkyl), -C(O)(C₀-C₁₀ alkyl), -C(O)O(C₀-C₁₀ alkyl), -OC(O)(C₀-C₁₀ alkyl), -C(O)N(C₀-C₁₀ alkyl)(C₀-C₁₀ alkyl), -N(C₀-C₁₀ alkyl)C(O)(C₀-C₁₀ alkyl), -SO₂N(C₀-C₁₀ alkyl)(C₀-C₁₀ alkyl), -N(C₀-C₁₀ alkyl)SO₂(C₀-C₁₀ alkyl), -OCH₂F, -OCHF₂, -OCF₃, C₃-C₁₀ cycloalkyl, C₆-C₁₀ aryl, or 4-10 membered heterocyclyl.

3. The compound according to claim 2, wherein a nitrogen-containing heterocycle is a saturated 4-10 membered nitrogen-containing heterocycle, such as 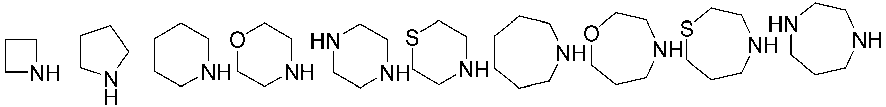 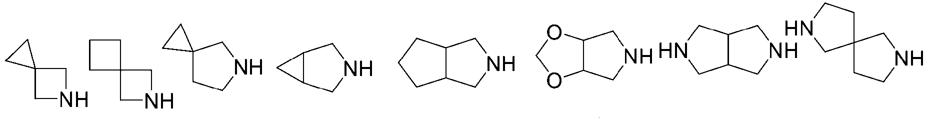 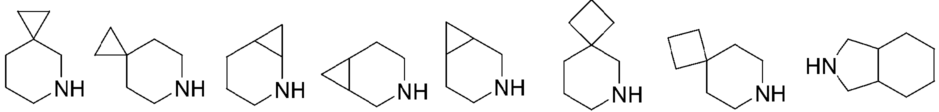 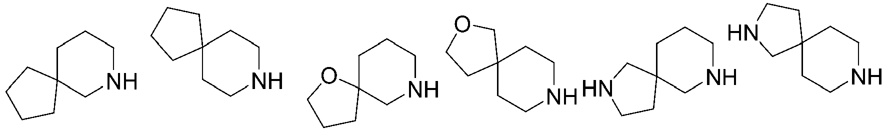 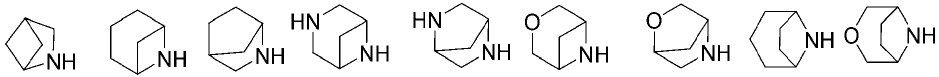 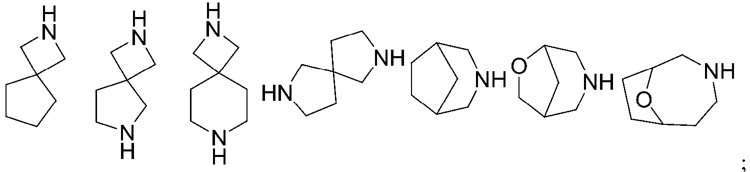
preferably, L₀₃ is -C(R_{L1}R_{L1})-, R_{L1} and R_{L2} are independently selected from: H, D, halogen, C₁-C₆ alkyl, and C₁-C₆ haloalkyl; preferably -CH₂- or -CD₂-;
preferably, R₀₁₃ is selected from: 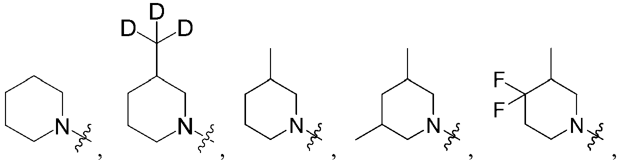 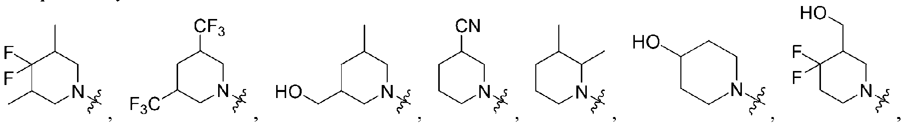 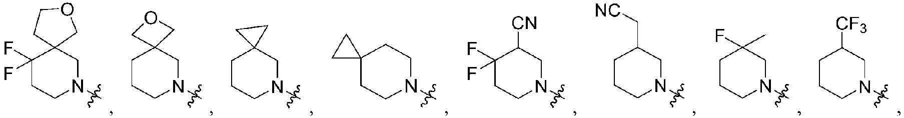 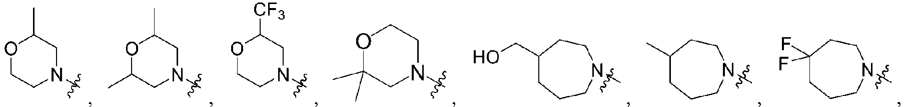 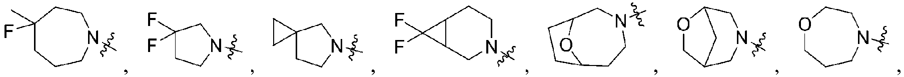 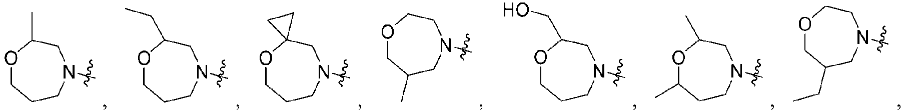 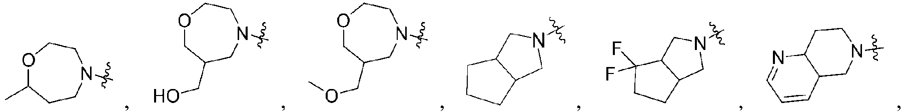 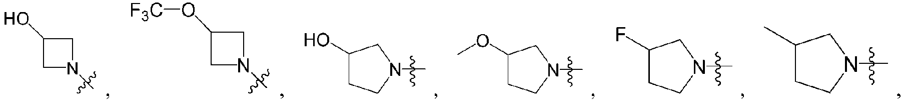 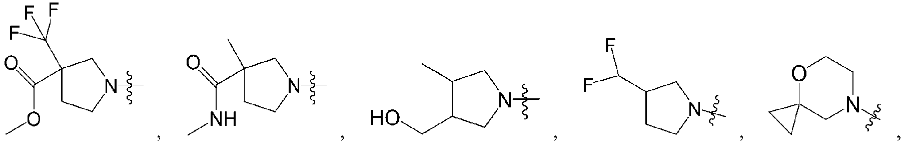 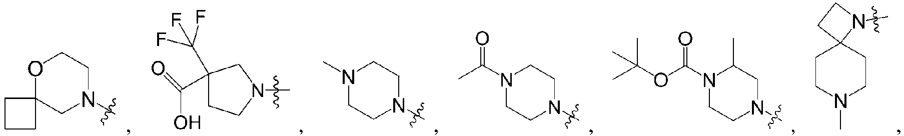 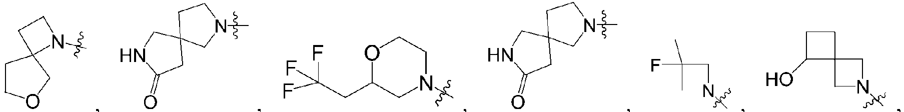 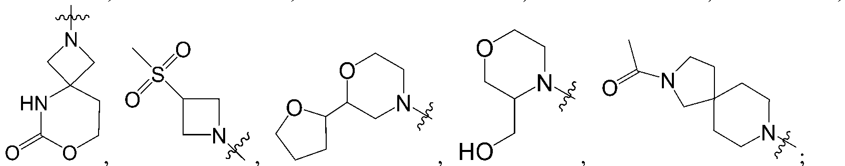 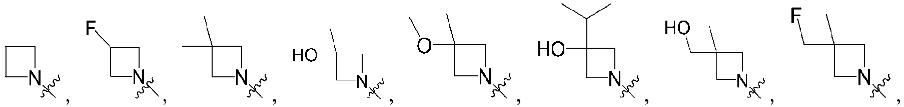 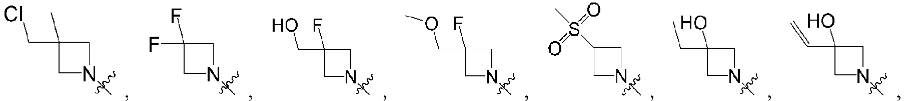 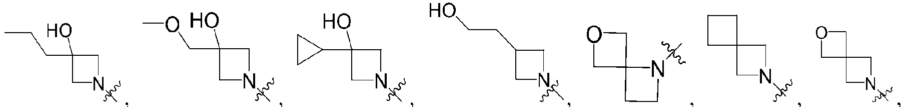 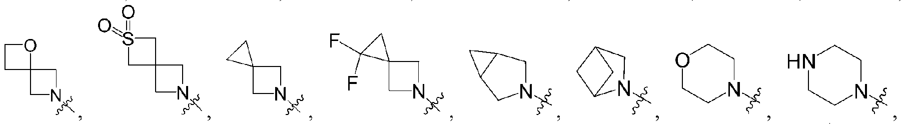 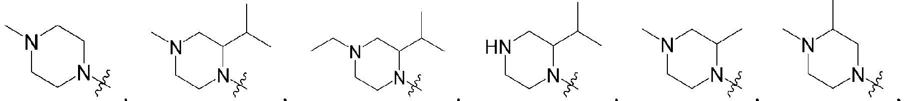 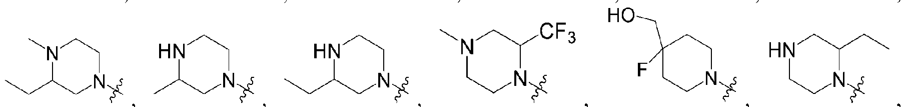 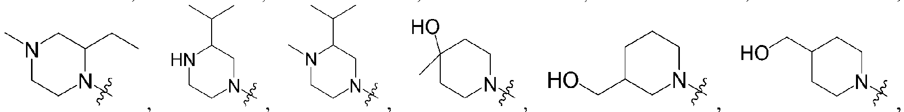 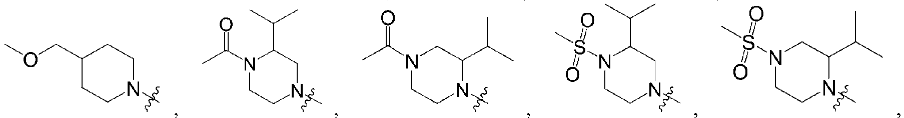 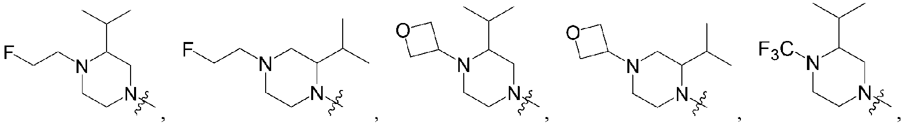 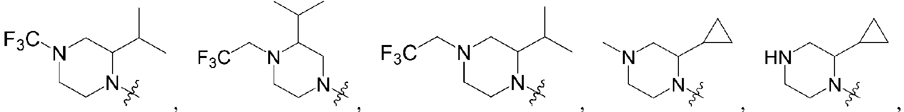 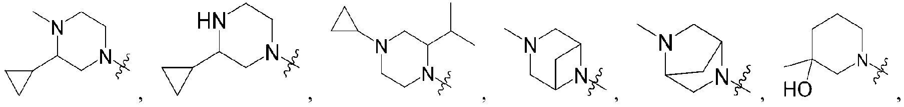 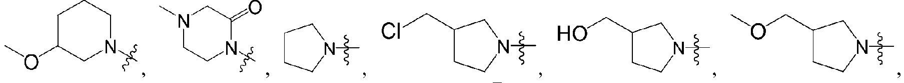 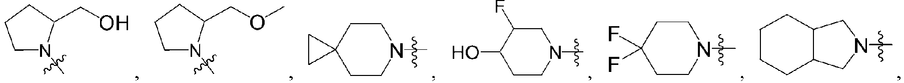 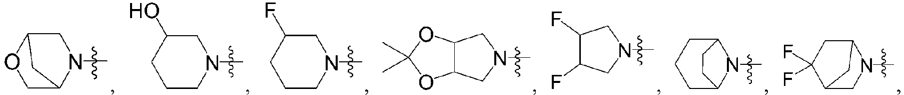 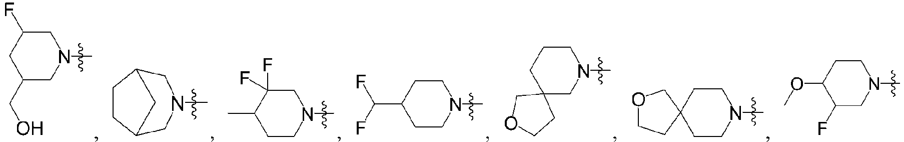 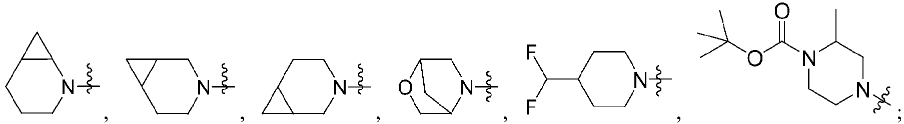 or,
R₀₁₁ is selected from:

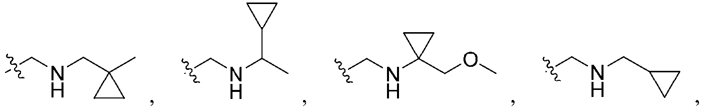

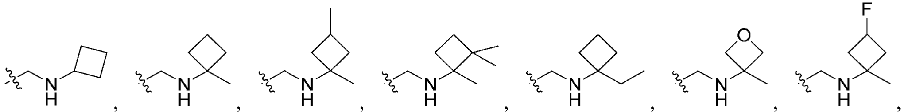

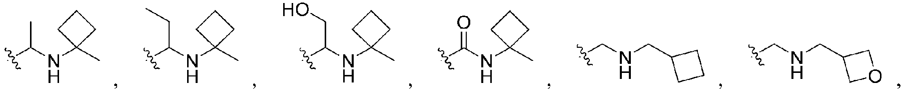

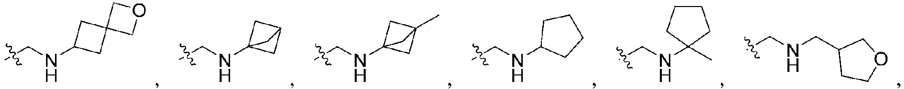

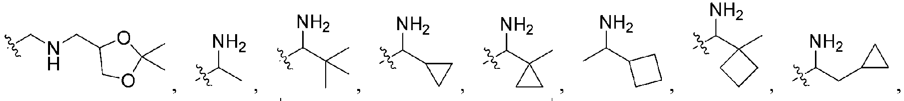

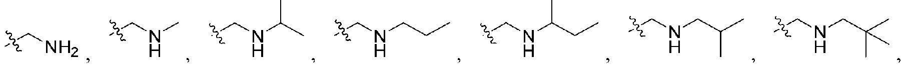

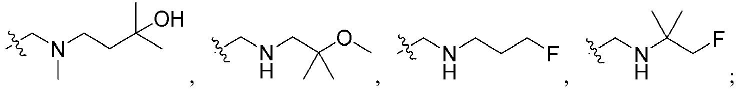

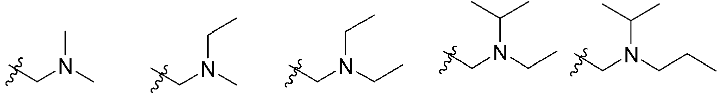

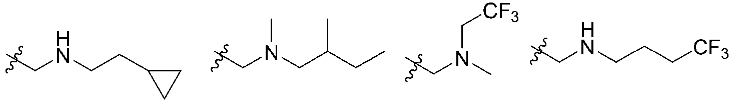

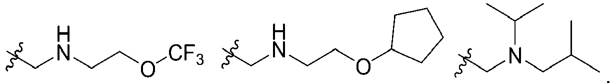

4. The compound according to any one of claims 1 to 3, wherein the Q₂-ring is an aromatic ring or a heterocyclic aromatic ring, and is preferably selected from: 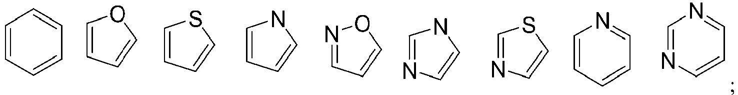
preferably, 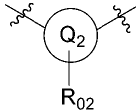 is partially selected from the following structure: 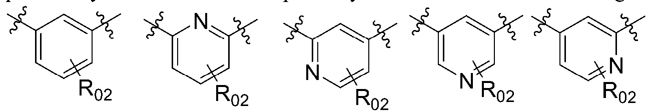 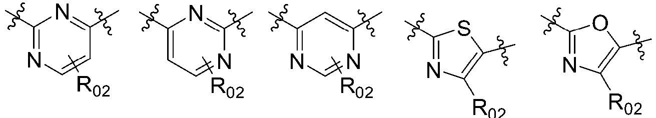 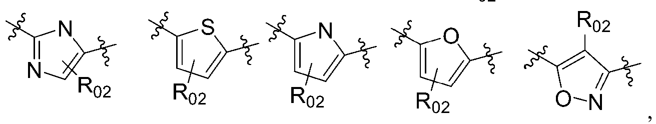 and
more preferably, R₀₂ is selected from: H, D, halogen, cyano, nitro, azido, C₁-C₁₀ alkyl, -N(C₀-C₁₀ alkyl)(C₀-C₁₀ alkyl), -O(C₀-C₁₀ alkyl), -C(O)(C₀-C₁₀ alkyl), -C(O)O(C₀-C₁₀ alkyl), -OC(O)(C₀-C₁₀ alkyl), -C(O)N(C₀-C₁₀ alkyl)(C₀-C₁₀ alkyl), -N(C₀-C₁₀ alkyl)C(O)(C₀-C₁₀ alkyl), -SO₂N(C₀-C₁₀ alkyl)(C₀-C₁₀ alkyl), -N(C₀-C₁₀ alkyl)SO₂(C₀-C₁₀ alkyl), -OCH₂F, -OCHF₂, -OCF₃, C₃-C₁₀ cycloalkyl, C₆-C₁₀ aryl, and 4-10 membered heterocyclyl; wherein H on the C₁-C₁₀ alkyl, C₃-C₁₀ cycloalkyl, C₆-C₁₀ aryl, and 4-10 membered heterocyclyl is optionally substituted with groups selected from: D, halogen, cyano, nitro, azido, -N(C₀-C₁₀ alkyl)(C₀-C₁₀ alkyl), -O(C₀-C₁₀ alkyl), -C(O)(C₀-C₁₀ alkyl), -C(O)O(C₀-C₁₀ alkyl), -OC(O)(C₀-C₁₀ alkyl), -C(O)N(C₀-C₁₀ alkyl)(C₀-C₁₀ alkyl), -N(C₀-C₁₀ alkyl)C(O)(C₀-C₁₀ alkyl), -SO₂N(C₀-C₁₀ alkyl)(C₀-C₁₀ alkyl), -N(C₀-C₁₀ alkyl)SO₂(C₀-C₁₀ alkyl), -OCH₂F, -OCHF₂, -OCF₃, C₃-C₁₀ cycloalkyl, C₆-C₁₀ aryl, or 4-10 membered heterocyclyl.

5. The compound according to any one of claims 1 to 4, wherein the Q₃-ring is an aromatic ring or a heterocyclic aromatic ring, preferably a 5 membered heteroaromatic ring; and
preferably, 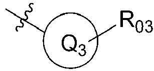 moiety is 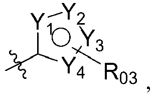 and Y₁,Y₂, Y₃ and Y₄ are independently selected from: C, N, O, and S, and any two of Y₁,Y₂, Y₃ and Y₄ are two O atoms, two S atoms, or O atom and S atom are not directly bound.

6. The compound according to any one of claims 1 to 5, wherein L₀₁ is selected from: a single bond, -C(O)O-, -OC(O)-, -C(O)N(R_{L1})-, -N(R_{L1})C(O)-, -N(R_{L1})-, -S(O)₂-, -S(O)₂N(R_{L1})-, and -N(R_{L1})S(O)₂-;
preferably, R_{L1} is selected from : H, D, and C₁-C₆ alkyl; and
more preferably, L₀₁ is a single bond.

7. The compound according to any one of claims 1 to 6, wherein L₀₂ has the following structure: 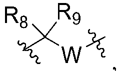 wherein
R₈ and R₉ are independently selected from: H, D, halogen, cyano, nitro, C₁-C₁₀ alkyl, C₂-C₁₀ alkenyl, C₂-C₁₀ alkynyl, -(C₀-C₆ alkylidene)-(C₃-C₁₀ cycloalkyl), -(C₀-C₆ alkylidene)-(C₆-C₁₀ aryl), -(C₀-C₆ alkylidene)-(4-10 membered heterocyclyl), -O(C₀-C₁₀ alkyl), -S(C₀-C₁₀ alkyl), -C(O)(C₀-C₁₀ alkyl), -C(O)N(C₀-C₁₀ alkyl)(C₀-C₁₀ alkyl), -N(C₀-C₁₀ alkyl)(C₀-C₁₀ alkyl), -C(O)O(C₀-C₁₀ alkyl), -S(O)₂(C₀-C₁₀ alkyl), -S(O)₂-(C₀-C₆ alkylidene)-(C₃-C₁₀ cycloalkyl), -S(O)₂-(C₀-C₆ alkylidene)-(4-10 membered heterocyclyl), -S(O)₂N(C₀-C₁₀ alkyl)(C₀-C₁₀ alkyl), -S(O)₂N(C₀-C₁₀ alkyl)((C₀-C₆ alkylidene)-(C₃-C₁₀ cycloalkyl)), -S(O)₂N(C₀-C₁₀ alkyl)((C₀-C₆ alkylidene)-(4-10 membered heterocyclyl)), C₁-C₁₀ haloalkyl, C₁-C₁₀ cyanoalkyl, C₁-C₁₀ hydroxyalkyl, C₁-C₁₀ aminoalkyl, C₁-C₁₀ carboxyalkyl, C₁-C₁₀ alkoxyalkyl, and C₁-C₁₀ alkylaminoalkyl; wherein H on the alkylene, alkyl, alkenyl, alkynyl, cycloalkyl, aryl, and heterocyclyl is each optionally substituted with one or more of independent R groups;
or, R₈ and R₉, together with carbon atom to which they are attached, form a cycloalkyl or a heterocyclyl, wherein the cycloalkyl or the heterocyclyl is optionally substituted with one or more independent substituents selected from: oxo(=O), (=alkylidene), halogen, cyano, nitro, C₁-C₁₀ alkyl, C₂-C₁₀ alkenyl, C₂-C₁₀ alkynyl, -(C₀-C₆ alkylidene)-(C₃-C₁₀ cycloalkyl), -(C₀-C₆ alkylidene)-(C₆-C₁₀ aryl), -(C₀-C₆ alkylidene)-(4-10 membered heterocyclyl), -O(C₀-C₁₀ alkyl), -S(C₀-C₁₀ alkyl), -C(O)(C₀-C₁₀ alkyl), -C(O)N(C₀-C₁₀ alkyl)(C₀-C₁₀ alkyl), -N(C₀-C₁₀ alkyl)(C₀-C₁₀ alkyl), -C(O)O(C₀-C₁₀ alkyl), -S(O)₂(C₀-C₁₀ alkyl), -S(O)₂-(C₀-C₆ alkylidene)-(C₃-C₁₀ cycloalkyl), -S(O)₂-(C₀-C₆ alkylidene)-(4-10 membered heterocyclyl), -S(O)₂N(C₀-C₁₀ alkyl)(C₀-C₁₀ alkyl), -S(O)₂N(C₀-C₁₀ alkyl)((C₀-C₆ alkylidene)-(C₃-C₁₀ cycloalkyl)), -S(O)₂N(C₀-C₁₀ alkyl)((C₀-C₆ alkylidene)-(4-10 membered heterocyclyl)), C₁-C₁₀ haloalkyl, C₁-C₁₀ cyanoalkyl, C₁-C₁₀ hydroxyalkyl, C₁-C₁₀ aminoalkyl, C₁-C₁₀ carboxyalkyl, C₁-C₁₀ alkoxyalkyl, and C₁-C₁₀ alkylaminoalkyl; wherein H on the alkylene, alkyl, alkenyl, alkynyl, cycloalkyl, aryl, and heterocyclyl is each optionally substituted with one or more of independent R groups;
W is a single bond, 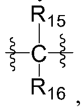
or S, wherein R₁₅ and R₁₆ are independently selected from: H, D, halogen, cyano, nitro, C₁-C₁₀ alkyl, C₂-C₁₀ alkenyl, C₂-C₁₀ alkynyl, -(C₀-C₆ alkylidene)-(C₃-C₁₀ cycloalkyl), -(C₀-C₆ alkylidene)-(C₆-C₁₀ aryl), -(C₀-C₆ alkylidene)-(4-10 membered heterocyclyl), -O(C₀-C₁₀ alkyl), -S(C₀-C₁₀ alkyl), -C(O)(C₀-C₁₀ alkyl), -C(O)N(C₀-C₁₀ alkyl)(C₀-C₁₀ alkyl), -N(C₀-C₁₀ alkyl)(C₀-C₁₀ alkyl), -C(O)O(C₀-C₁₀ alkyl), -S(O)₂(C₀-C₁₀ alkyl), -S(O)₂-(C₀-C₆ alkylidene)-(C₃-C₁₀ cycloalkyl), -S(O)₂-(C₀-C₆ alkylidene)-(4-10 membered heterocyclyl), -S(O)₂N(C₀-C₁₀ alkyl)(C₀-C₁₀ alkyl), -S(O)₂N(C₀-C₁₀ alkyl)((C₀-C₆ alkylidene)-(C₃-C₁₀ cycloalkyl)), -S(O)₂N(C₀-C₁₀ alkyl)((C₀-C₆ alkylidene)-(4-10 membered heterocyclyl)), C₁-C₁₀ haloalkyl, C₁-C₁₀ cyanoalkyl, C₁-C₁₀ hydroxyalkyl, C₁-C₁₀ aminoalkyl, C₁-C₁₀ carboxyalkyl, C₁-C₁₀ alkoxyalkyl, and C₁-C₁₀ alkylaminoalkyl; wherein H on the alkylene, alkyl, alkenyl, alkynyl, cycloalkyl, aryl, and heterocyclyl is each optionally substituted with one or more of independent R groups;
or, R₁₅ and R₁₆, together with carbon atom to which they are attached, form a cycloalkyl or a heterocyclyl, wherein H on the cycloalkyl or the heterocyclyl is optionally substituted with one or more independent substituents selected from: oxo(=O), (=alkylidene), halogen, cyano, nitro, C₁-C₁₀ alkyl, C₂-C₁₀ alkenyl, C₂-C₁₀ alkynyl, -(C₀-C₆ alkylidene)-(C₃-C₁₀ cycloalkyl), -(C₀-C₆ alkylidene)-(C₆-C₁₀ aryl), -(C₀-C₆ alkylidene)-(4-10 membered heterocyclyl), -O(C₀-C₁₀ alkyl), -S(C₀-C₁₀ alkyl), -C(O)(C₀-C₁₀ alkyl), -C(O)N(C₀-C₁₀ alkyl)(C₀-C₁₀ alkyl), -N(C₀-C₁₀ alkyl)(C₀-C₁₀ alkyl), -C(O)O(C₀-C₁₀ alkyl), -S(O)₂(C₀-C₁₀ alkyl), -S(O)₂-(C₀-C₆ alkylidene)-(C₃-C₁₀ cycloalkyl), -S(O)₂-(C₀-C₆ alkylidene)-(4-10 membered heterocyclyl), -S(O)₂N(C₀-C₁₀ alkyl)(C₀-C₁₀ alkyl), -S(O)₂N(C₀-C₁₀ alkyl)((C₀-C₆ alkylidene)-(C₃-C₁₀ cycloalkyl)), -S(O)₂N(C₀-C₁₀ alkyl)((C₀-C₆ alkylidene)-(4-10 membered heterocyclyl)), C₁-C₁₀ haloalkyl, C₁-C₁₀ cyanoalkyl, C₁-C₁₀ hydroxyalkyl, C₁-C₁₀ aminoalkyl, C₁-C₁₀ carboxyalkyl, C₁-C₁₀ alkoxyalkyl, and C₁-C₁₀ alkylaminoalkyl; wherein H on the alkylene, alkyl, alkenyl, alkynyl, cycloalkyl, aryl, and heterocyclyl is each optionally substituted with one or more of independent R groups;
or, R₁₅ and R₉, together with the intervening carbon atom, form a cycloalkyl, an aryl, or a heterocyclyl, wherein H on the cycloalkyl, the aryl, and the heterocyclyl is optionally substituted with one or more independent substituents selected from: oxo(=O), (=alkylidene), halogen, cyano, nitro, C₁-C₁₀ alkyl, C₂-C₁₀ alkenyl, C₂-C₁₀ alkynyl, -(C₀-C₆ alkylidene)-(C₃-C₁₀ cycloalkyl), -(C₀-C₆ alkylidene)-(C₆-C₁₀ aryl), -(C₀-C₆ alkylidene)-(4-10 membered heterocyclyl), -O(C₀-C₁₀ alkyl), -S(C₀-C₁₀ alkyl), -C(O)(C₀-C₁₀ alkyl), -C(O)N(C₀-C₁₀ alkyl)(C₀-C₁₀ alkyl), -N(C₀-C₁₀ alkyl)(C₀-C₁₀ alkyl), -C(O)O(C₀-C₁₀ alkyl), -S(O)₂(C₀-C₁₀ alkyl), -S(O)₂-(C₀-C₆ alkylidene)-(C₃-C₁₀ cycloalkyl), -S(O)₂-(C₀-C₆ alkylidene)-(4-10 membered heterocyclyl), -S(O)₂N(C₀-C₁₀ alkyl)(C₀-C₁₀ alkyl), -S(O)₂N(C₀-C₁₀ alkyl)((C₀-C₆ alkylidene)-(C₃-C₁₀ cycloalkyl)), -S(O)₂N(C₀-C₁₀ alkyl)((C₀-C₆ alkylidene)-(4-10 membered heterocyclyl)), C₁-C₁₀ haloalkyl, C₁-C₁₀ cyanoalkyl, C₁-C₁₀ hydroxyalkyl, C₁-C₁₀ aminoalkyl, C₁-C₁₀ carboxyalkyl, C₁-C₁₀ alkoxyalkyl, and C₁-C₁₀ alkylaminoalkyl; wherein H on the alkylene, alkyl, alkenyl, alkynyl, cycloalkyl, aryl, and heterocyclyl is optionally substituted with one or more of independent R groups.

8. The compound according to claim 1, wherein the compound has the following structure: 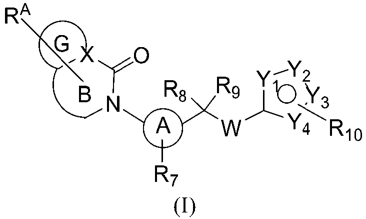 wherein,
A-ring is an aromatic ring or a heterocyclic aromatic ring;
B-ring is a 5-7 membered heterocyclic ring;
G-ring is a 5-7 membered heterocyclic ring;
X is CH or N;
R^{A} is one or more independent substituents on the ring 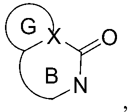 each independently selected from: H, D, 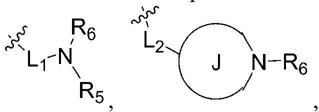 and 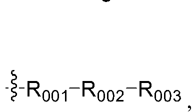 wherein L₁ is selected from: a single bond, C(O), and C(R₃R₄); L₂ is selected from: a single bond, O, S, C(R₃R₄), N(R₅), and 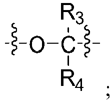 R₀₀₁ is selected from: a single bond and C₁-C₁₀ alkylidene; R₀₀₂ is selected from: a single bond, C₁-C₁₀ alkylidene, O, S, N(R₂), S(O)₂, S(O)₂N(R₂), S(O), S(O)N(R₂), C(O), C(O)O, C(O)N(R₂), OC(O), OC(O)N(R₂), N(R₂)C(O)O, N(R₂)C(O), and N(R₂)S(O)₂; R₀₀₃ is selected from: H, D, halogen, cyano, nitro, C₁-C₁₀ alkyl, C₁-C₁₀ haloalkyl, C₂-C₁₀ alkenyl, C₂-C₁₀ alkynyl, -(C₀-C₆ alkylidene)-(C₃-C₁₀ cycloalkyl), -(C₀-C₆ alkylidene)-(C₆-C₁₀ aryl), and -(C₀-C₆ alkylidene)-(4-10 membered heterocyclyl); wherein H on the alkylene, alkyl, alkenyl, alkynyl, cycloalkyl, aryl, and heterocyclyl is each optionally substituted with one or more of independent R groups;
R₂ is selected from: H, D, C₁-C₁₀ alkyl, and -(C₀-C₆ alkylidene)-(C₃-C₁₀ cycloalkyl);
R₃ and R₄ are independently selected from: H, D, halogen, cyano, nitro, C₁-C₁₀ alkyl, C₁-C₁₀ haloalkyl, C₂-C₁₀ alkenyl, C₂-C₁₀ alkynyl, -(C₀-C₆ alkylidene)-(C₃-C₁₀ cycloalkyl), -(C₀-C₆ alkylidene)-(C₆-C₁₀ aryl), and -(C₀-C₆ alkylidene)-(4-10 membered heterocyclyl); wherein H on the alkylene, alkyl, alkenyl, alkynyl, cycloalkyl, aryl, and heterocyclyl is each optionally substituted with one or more of independent R groups;
or, R₃ and R₄, together with carbon atom to which they are attached, form a cycloalkyl or a heterocyclyl, wherein H on the cycloalkyl or the heterocyclyl is optionally substituted with one or more of independent R groups;
J-ring is a 3-10 membered nitrogen-containing heterocyclic ring (J-ring is bonded to L₂ via a carbon atom), and the J-ring is optionally substituted by groups selected from: oxo(=O), halogen, cyano, nitro, C₁-C₁₀ alkyl, C₂-C₁₀ alkenyl, C₂-C₁₀ alkynyl, -(C₀-C₆ alkylidene)-(C₃-C₁₀ cycloalkyl), -(C₀-C₆ alkylidene)-(C₆-C₁₀ aryl), -(C₀-C₆ alkylidene)-(4-10 membered heterocyclyl), -O(C₀-C₁₀ alkyl), -S(C₀-C₁₀ alkyl), -C(O)(C₀-C₁₀ alkyl), -C(O)N(C₀-C₁₀ alkyl)(C₀-C₁₀ alkyl), -N(C₀-C₁₀ alkyl)(C₀-C₁₀ alkyl), -C(O)O(C₀-C₁₀ alkyl), -S(O)₂(C₀-C₁₀ alkyl), -S(O)₂-(C₀-C₆ alkylidene)-(C₃-C₁₀ cycloalkyl), -S(O)₂-(C₀-C₆ alkylidene)-(4-10 membered heterocyclyl), -S(O)₂N(C₀-C₁₀ alkyl)(C₀-C₁₀ alkyl), -S(O)₂N(C₀-C₁₀ alkyl)((C₀-C₆ alkylidene)-(C₃-C₁₀ cycloalkyl)), -S(O)₂N(C₀-C₁₀ alkyl)((C₀-C₆ alkylidene)-(4-10 membered heterocyclyl)), C₁-C₁₀ haloalkyl, C₁-C₁₀ cyanoalkyl, C₁-C₁₀ hydroxyalkyl, C₁-C₁₀ aminoalkyl, C₁-C₁₀ carboxyalkyl, C₁-C₁₀ alkoxyalkyl, and C₁-C₁₀ alkylaminoalkyl; wherein H on the alkylene, alkyl, alkenyl, alkynyl, cycloalkyl, aryl, and heterocyclyl is each optionally substituted with one or more of independent R groups;
R₅ and R₆ are independently selected from: H, D, C₁₋₁₀ alkyl, -(C₀-C₆ alkylidene)-(C₃-C₁₀ cycloalkyl), -(C₀-C₆ alkylidene)-(C₆-C₁₀ aryl), and -(C₀-C₆ alkylidene)-(4-10 membered heterocyclyl); wherein H on the alkylidene, alkyl, cycloalkyl, aryl, and heterocyclyl is each optionally substituted with one or more independent substituents selected from: halogen, cyano, nitro, C₁-C₁₀ alkyl, C₂-C₁₀ alkenyl, C₂-C₁₀ alkynyl, -(C₀-C₆ alkylidene)-(C₃-C₁₀ cycloalkyl), -(C₀-C₆ alkylidene)-(C₆-C₁₀ aryl), -(C₀-C₆ alkylidene)-(4-10 membered heterocyclyl), -O(C₀-C₁₀ alkyl), -S(C₀-C₁₀ alkyl), -C(O)(C₀-C₁₀ alkyl), -C(O)N(C₀-C₁₀ alkyl)(C₀-C₁₀ alkyl), -N(C₀-C₁₀ alkyl)(C₀-C₁₀ alkyl), -C(O)O(C₀-C₁₀ alkyl), -S(O)₂(C₀-C₁₀ alkyl), -S(O)₂-(C₀-C₆ alkylidene)-(C₃-C₁₀ cycloalkyl), -S(O)₂-(C₀-C₆ alkylidene)-(4-10 membered heterocyclyl), -S(O)₂N(C₀-C₁₀ alkyl)(C₀-C₁₀ alkyl), -S(O)₂N(C₀-C₁₀ alkyl)((C₀-C₆ alkylidene)-(C₃-C₁₀ cycloalkyl)), -S(O)₂N(C₀-C₁₀ alkyl)((C₀-C₆ alkylidene)-(4-10 membered heterocyclyl)), C₁-C₁₀ haloalkyl, C₁-C₁₀ cyanoalkyl, C₁-C₁₀ hydroxyalkyl, C₁-C₁₀ aminoalkyl, C₁-C₁₀ carboxyalkyl, C₁-C₁₀ alkoxyalkyl, C₁-C₁₀ alkylaminoalkyl; wherein H on the alkylene, alkyl, alkenyl, alkynyl, cycloalkyl, aryl, and heterocyclyl is each optionally substituted with one or more of independent R groups;
or, R₅ and R₆, together with the nitrogen atom to which they are attached, form a heterocyclyl, wherein H on the heterocyclyl is optionally substituted with one or more independent substituents selected from: oxo(=O), halogen, cyano, nitro, C₁-C₁₀ alkyl, C₂-C₁₀ alkenyl, C₂-C₁₀ alkynyl, -(C₀-C₆ alkylidene)-(C₃-C₁₀ cycloalkyl), -(C₀-C₆ alkylidene)-(C₆-C₁₀ aryl), -(C₀-C₆ alkylidene)-(4-10 membered heterocyclyl), -O(C₀-C₁₀ alkyl), -S(C₀-C₁₀ alkyl), -C(O)(C₀-C₁₀ alkyl), -C(O)N(C₀-C₁₀ alkyl)(C₀-C₁₀ alkyl), -N(C₀-C₁₀ alkyl)(C₀-C₁₀ alkyl), -C(O)O(C₀-C₁₀ alkyl), -S(O)₂(C₀-C₁₀ alkyl), -S(O)₂-(C₀-C₆ alkylidene)-(C₃-C₁₀ cycloalkyl), -S(O)₂-(C₀-C₆ alkylidene)-(4-10 membered heterocyclyl), -S(O)₂N(C₀-C₁₀ alkyl)(C₀-C₁₀ alkyl), -S(O)₂N(C₀-C₁₀ alkyl)((C₀-C₆ alkylidene)-(C₃-C₁₀ cycloalkyl)), -S(O)₂N(C₀-C₁₀ alkyl)((C₀-C₆ alkylidene)-(4-10 membered heterocyclyl)), C₁-C₁₀ haloalkyl, C₁-C₁₀ cyanoalkyl, C₁-C₁₀ hydroxyalkyl, C₁-C₁₀ aminoalkyl, C₁-C₁₀ carboxyalkyl, C₁-C₁₀ alkoxyalkyl, and C₁-C₁₀ alkylaminoalkyl; wherein H on the alkylene, alkyl, alkenyl, alkynyl, cycloalkyl, aryl, and heterocyclyl is optionally substituted with one or more of independent R groups;
R₇ is one or more independent substituents on the A ring, each independently selected from: H, D, halogen, cyano, nitro, and 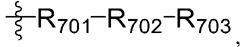 wherein, R₇₀₁ is selected from: a single bond and C₁₋₁₀ alkylidene; R₇₀₂ is selected from: a single bond, C₁₋₁₀ alkylidene, O, S, N(R₇₀₄), S(O)₂, S(O)₂N(R₇₀₄), S(O), S(O)N(R₇₀₄), C(O), C(O)O, C(O)N(R₇₀₄), OC(O), OC(O)N(R₇₀₄), N(R₇₀₄)C(O)O, N(R₇₀₄)C(O),and N(R₇₀₄)S(O)₂; R₇₀₃ is selected from: H, D, halogen, cyano, nitro, C₁₋₁₀ alkyl, C₁₋₁₀ haloalkyl, C₂₋₁₀ alkenyl, C₂₋₁₀ alkynyl, -(C₀-C₆ alkylidene)-(C₃-C₁₀ cycloalkyl), -(C₀-C₆ alkylidene)-(C₆-C₁₀ aryl), and -(C₀-C₆ alkylidene)-(4-10 membered heterocyclyl); R₇₀₄ is selected from: H, D, and C₁₋₁₀ alkyl; wherein H on the alkylene, alkyl, alkenyl, alkynyl, cycloalkyl, aryl, and heterocyclyl is each optionally substituted with one or more of independent R groups;
R₈ and R₉ are independently selected from: H, D, halogen, cyano, nitro, C₁-C₁₀ alkyl, C₂-C₁₀ alkenyl, C₂-C₁₀ alkynyl, -(C₀-C₆ alkylidene)-(C₃-C₁₀ cycloalkyl), -(C₀-C₆ alkylidene)-(C₆-C₁₀ aryl), -(C₀-C₆ alkylidene)-(4-10 membered heterocyclyl), -O(C₀-C₁₀ alkyl), -S(C₀-C₁₀ alkyl), -C(O)(C₀-C₁₀ alkyl), -C(O)N(C₀-C₁₀ alkyl)(C₀-C₁₀ alkyl), -N(C₀-C₁₀ alkyl)(C₀-C₁₀ alkyl), -C(O)O(C₀-C₁₀ alkyl), -S(O)₂(C₀-C₁₀ alkyl), -S(O)₂-(C₀-C₆ alkylidene)-(C₃-C₁₀ cycloalkyl), -S(O)₂-(C₀-C₆ alkylidene)-(4-10 membered heterocyclyl), -S(O)₂N(C₀-C₁₀ alkyl)(C₀-C₁₀ alkyl), -S(O)₂N(C₀-C₁₀ alkyl)((C₀-C₆ alkylidene)-(C₃-C₁₀ cycloalkyl)), -S(O)₂N(C₀-C₁₀ alkyl)((C₀-C₆ alkylidene)-(4-10 membered heterocyclyl)), C₁-C₁₀ haloalkyl, C₁-C₁₀ cyanoalkyl, C₁-C₁₀ hydroxyalkyl, C₁-C₁₀ aminoalkyl, C₁-C₁₀ carboxyalkyl, C₁-C₁₀ alkoxyalkyl, and C₁-C₁₀ alkylaminoalkyl; wherein H on the alkylene, alkyl, alkenyl, alkynyl, cycloalkyl, aryl, and heterocyclyl is each optionally substituted with one or more of independent R groups;
or, R₈ and R₉, together with carbon atom to which they are attached, form a cycloalkyl or a heterocyclyl, wherein H on the cycloalkyl or the heterocyclyl is optionally substituted with one or more independent substituents selected from: oxo(=O), (=alkylidene), halogen, cyano, nitro, C₁-C₁₀ alkyl, C₂-C₁₀ alkenyl, C₂-C₁₀ alkynyl, -(C₀-C₆ alkylidene)-(C₃-C₁₀ cycloalkyl), -(C₀-C₆ alkylidene)-(C₆-C₁₀ aryl), -(C₀-C₆ alkylidene)-(4-10 membered heterocyclyl), -O(C₀-C₁₀ alkyl), -S(C₀-C₁₀ alkyl), -C(O)(C₀-C₁₀ alkyl), -C(O)N(C₀-C₁₀ alkyl)(C₀-C₁₀ alkyl), -N(C₀-C₁₀ alkyl)(C₀-C₁₀ alkyl), -C(O)O(C₀-C₁₀ alkyl), -S(O)₂(C₀-C₁₀ alkyl), -S(O)₂-(C₀-C₆ alkylidene)-(C₃-C₁₀ cycloalkyl), -S(O)₂-(C₀-C₆ alkylidene)-(4-10 membered heterocyclyl), -S(O)₂N(C₀-C₁₀ alkyl)(C₀-C₁₀ alkyl), -S(O)₂N(C₀-C₁₀ alkyl)((C₀-C₆ alkylidene)-(C₃-C₁₀ cycloalkyl)), -S(O)₂N(C₀-C₁₀ alkyl)((C₀-C₆ alkylidene)-(4-10 membered heterocyclyl)), C₁-C₁₀ haloalkyl, C₁-C₁₀ cyanoalkyl, C₁-C₁₀ hydroxyalkyl, C₁-C₁₀ aminoalkyl, C₁-C₁₀ carboxyalkyl, C₁-C₁₀ alkoxyalkyl, and C₁-C₁₀ alkylaminoalkyl; wherein H on the alkylene, alkyl, alkenyl, alkynyl, cycloalkyl, aryl, and heterocyclyl is each optionally substituted with one or more of independent R groups;
W is a single bond, 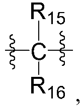 or S; wherein, R₁₅ and R₁₆ are independently selected from: H, D, halogen, cyano, nitro, C₁-C₁₀ alkyl, C₂-C₁₀ alkenyl, C₂-C₁₀ alkynyl, -(C₀-C₆ alkylidene)-(C₃-C₁₀ cycloalkyl), -(C₀-C₆ alkylidene)-(C₆-C₁₀ aryl), -(C₀-C₆ alkylidene)-(4-10 membered heterocyclyl), -O(C₀-C₁₀ alkyl), -S(C₀-C₁₀ alkyl), -C(O)(C₀-C₁₀ alkyl), -C(O)N(C₀-C₁₀ alkyl)(C₀-C₁₀ alkyl), -N(C₀-C₁₀ alkyl)(C₀-C₁₀ alkyl), -C(O)O(C₀-C₁₀ alkyl), -S(O)₂(C₀-C₁₀ alkyl), -S(O)₂-(C₀-C₆ alkylidene)-(C₃-C₁₀ cycloalkyl), -S(O)₂-(C₀-C₆ alkylidene)-(4-10 membered heterocyclyl), -S(O)₂N(C₀-C₁₀ alkyl)(C₀-C₁₀ alkyl), -S(O)₂N(C₀-C₁₀ alkyl)((C₀-C₆ alkylidene)-(C₃-C₁₀ cycloalkyl)), -S(O)₂N(C₀-C₁₀ alkyl)((C₀-C₆ alkylidene)-(4-10 membered heterocyclyl)), C₁-C₁₀ haloalkyl, C₁-C₁₀ cyanoalkyl, C₁-C₁₀ hydroxyalkyl, C₁-C₁₀ aminoalkyl, C₁-C₁₀ carboxyalkyl, C₁-C₁₀ alkoxyalkyl, and C₁-C₁₀ alkylaminoalkyl; wherein H on the alkylene, alkyl, alkenyl, alkynyl, cycloalkyl, aryl, and heterocyclyl is each optionally substituted with one or more of independent R groups;
or, R₁₅ and R₁₆, together with carbon atom to which they are attached, form a cycloalkyl or a heterocyclyl, wherein H on the cycloalkyl or the heterocyclyl is optionally substituted with one or more independent substituents selected from: oxo(=O), (=alkylidene), halogen, cyano, nitro, C₁-C₁₀ alkyl, C₂-C₁₀ alkenyl, C₂-C₁₀ alkynyl, -(C₀-C₆ alkylidene)-(C₃-C₁₀ cycloalkyl), -(C₀-C₆ alkylidene)-(C₆-C₁₀ aryl), -(C₀-C₆ alkylidene)-(4-10 membered heterocyclyl), -O(C₀-C₁₀ alkyl), -S(C₀-C₁₀ alkyl), -C(O)(C₀-C₁₀ alkyl), -C(O)N(C₀-C₁₀ alkyl)(C₀-C₁₀ alkyl), -N(C₀-C₁₀ alkyl)(C₀-C₁₀ alkyl), -C(O)O(C₀-C₁₀ alkyl), -S(O)₂(C₀-C₁₀ alkyl), -S(O)₂-(C₀-C₆ alkylidene)-(C₃-C₁₀ cycloalkyl), -S(O)₂-(C₀-C₆ alkylidene)-(4-10 membered heterocyclyl), -S(O)₂N(C₀-C₁₀ alkyl)(C₀-C₁₀ alkyl), -S(O)₂N(C₀-C₁₀ alkyl)((C₀-C₆ alkylidene)-(C₃-C₁₀ cycloalkyl)), -S(O)₂N(C₀-C₁₀ alkyl)((C₀-C₆ alkylidene)-(4-10 membered heterocyclyl)), C₁-C₁₀ haloalkyl, C₁-C₁₀ cyanoalkyl, C₁-C₁₀ hydroxyalkyl, C₁-C₁₀ aminoalkyl, C₁-C₁₀ carboxyalkyl, C₁-C₁₀ alkoxyalkyl, and C₁-C₁₀ alkylaminoalkyl; wherein H on the alkylene, alkyl, alkenyl, alkynyl, cycloalkyl, aryl, and heterocyclyl is each optionally substituted with one or more of independent R groups;
or, R₁₅ and R₉, together with the intervening carbon atom, form a cycloalkyl, an aryl, or a heterocyclyl, wherein H on the cycloalkyl, the aryl, or the heterocyclyl is optionally substituted with one or more independent substituents selected from: oxo(=O), (=alkylidene), halogen, cyano, nitro, C₁-C₁₀ alkyl, C₂-C₁₀ alkenyl, C₂-C₁₀ alkynyl, -(C₀-C₆ alkylidene)-(C₃-C₁₀ cycloalkyl), -(C₀-C₆ alkylidene)-(C₆-C₁₀ aryl), -(C₀-C₆ alkylidene)-(4-10 membered heterocyclyl), -O(C₀-C₁₀ alkyl), -S(C₀-C₁₀ alkyl), -C(O)(C₀-C₁₀ alkyl), -C(O)N(C₀-C₁₀ alkyl)(C₀-C₁₀ alkyl), -N(C₀-C₁₀ alkyl)(C₀-C₁₀ alkyl), -C(O)O(C₀-C₁₀ alkyl), -S(O)₂(C₀-C₁₀ alkyl), -S(O)₂-(C₀-C₆ alkylidene)-(C₃-C₁₀ cycloalkyl), -S(O)₂-(C₀-C₆ alkylidene)-(4-10 membered heterocyclyl), -S(O)₂N(C₀-C₁₀ alkyl)(C₀-C₁₀ alkyl), -S(O)₂N(C₀-C₁₀ alkyl)((C₀-C₆ alkylidene)-(C₃-C₁₀ cycloalkyl)), -S(O)₂N(C₀-C₁₀ alkyl)((C₀-C₆ alkylidene)-(4-10 membered heterocyclyl)), C₁-C₁₀ haloalkyl, C₁-C₁₀ cyanoalkyl, C₁-C₁₀ hydroxyalkyl, C₁-C₁₀ aminoalkyl, C₁-C₁₀ carboxyalkyl, C₁-C₁₀ alkoxyalkyl, and C₁-C₁₀ alkylaminoalkyl; wherein H on the alkylene, alkyl, alkenyl, alkynyl, cycloalkyl, aryl, and heterocyclyl is each optionally substituted with one or more of independent R groups;
Y₁, Y₂, Y₃ and Y₄ are independently selected from: C, N, O, and S, and any two of Y₁,Y₂, Y₃ and Y₄ are two O atoms, two S atoms, or an O atom and an S atom are not directly bonded;
R₁₀ is one or more independent substituents on the ring, each R₁₀ independently selected from: H, D, oxo(=O), halogen, cyano, nitro, C₁-C₁₀ alkyl, C₂-C₁₀ alkenyl, C₂-C₁₀ alkynyl, -(C₀-C₆ alkylidene)-(C₃-C₁₀ cycloalkyl), -(C₀-C₆ alkylidene)-(C₆-C₁₀ aryl), -(C₀-C₆ alkylidene)-(4-10 membered heterocyclyl), -O(C₀-C₁₀ alkyl), -S(C₀-C₁₀ alkyl), -C(O)(C₀-C₁₀ alkyl), -C(O)N(C₀-C₁₀ alkyl)(C₀-C₁₀ alkyl), -N(C₀-C₁₀ alkyl)(C₀-C₁₀ alkyl), -C(O)O(C₀-C₁₀ alkyl), -S(O)₂(C₀-C₁₀ alkyl), -S(O)₂-(C₀-C₆ alkylidene)-(C₃-C₁₀ cycloalkyl), -S(O)₂-(C₀-C₆ alkylidene)-(4-10 membered heterocyclyl), -S(O)₂N(C₀-C₁₀ alkyl)(C₀-C₁₀ alkyl), -S(O)₂N(C₀-C₁₀ alkyl)((C₀-C₆ alkylidene)-(C₃-C₁₀ cycloalkyl)), -S(O)₂N(C₀-C₁₀ alkyl)((C₀-C₆ alkylidene)-(4-10 membered heterocyclyl)), C₁-C₁₀ haloalkyl, C₁-C₁₀ cyanoalkyl, C₁-C₁₀ hydroxyalkyl, C₁-C₁₀ aminoalkyl, C₁-C₁₀ carboxyalkyl, C₁-C₁₀ alkoxyalkyl, and C₁-C₁₀ alkylaminoalkyl; wherein H on the alkylene, alkyl, alkenyl, alkynyl, cycloalkyl, aryl, and heterocyclyl is each optionally substituted with one or more of independent R groups; 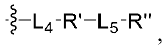
each R group is independently selected from: D, halogen, cyano, nitro, and , wherein L₄ and L₅ are independently selected from: a single bond, O, S, N(R‴), S(O)₂, S(O)₂N(R‴), S(O), S(O)N(R‴), C(O), C(O)O, C(O)N(R‴), OC(O), OC(O)N(R‴), N(R‴)C(O)O, N(R‴)C(O), and N(R‴)S(O)₂;
each R' group is independently selected from: a single bond, C₁-C₁₀ alkylidene, C₂-C₁₀ alkenylene, phenylene, C₃-C₁₀ cycloalkylene, and 4-10 membered;
each R" group is independently selected from: H, D, -CD₃, halogen, cyano, nitro, C₁-C₁₀ alkyl, C₁-C₁₀ haloalkyl, C₂-C₁₀ alkenyl, C₂-C₁₀ alkynyl, C₃-C₁₀ cycloalkyl, and 4-10 membered heterocyclyl; and
each R‴ group is independently selected from: H, D, C₁-C₁₀alkyl, C₁-C₁₀ haloalkyl, C₂-C₁₀ alkenyl, C₂-C₁₀ alkynyl, C₃-C₁₀ cycloalkyl, and 4-10 membered heterocyclyl;
preferably, the compound has the following structure: 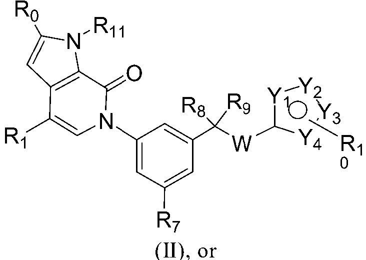 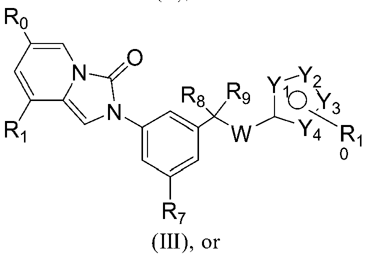 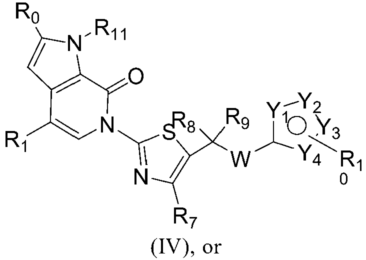 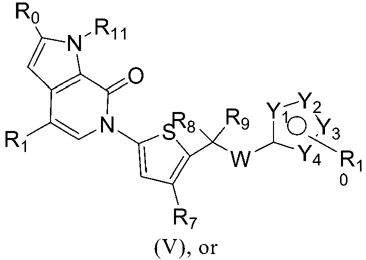 wherein, 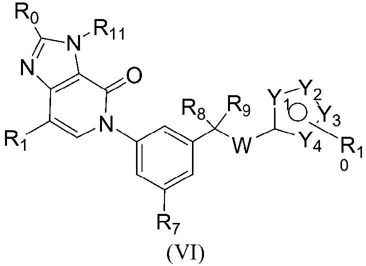
R₀ and R₁ are independently selected from: H, D, 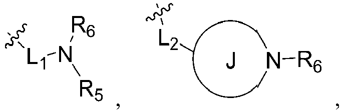 and 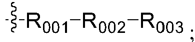 and
R₁₁ is selected from: H, D, C₁-C₁₀ alkyl, C₁-C₁₀ haloalkyl, C₂-C₁₀ alkenyl, C₂-C₁₀ alkynyl, -(C₀-C₆ alkylidene)-(C₃-C₁₀ cycloalkyl), -(C₀-C₆ alkylidene)-(C₆-C₁₀ aryl), and -(C₀-C₆ alkylidene)-(4-10 membered heterocyclyl); wherein the alkylidene, alkyl, alkenyl, alkynyl, cycloalkyl, aryl, and heterocyclyl are each optionally substituted with one or more of independent R groups.

9. The compound according to claim 8, wherein R₀ is selected from: H, D, C₁-C₆ alkyl, C₁-C₆ haloalkyl, C₃-C₆ cycloalkyl, C₁-C₆ hydroxyalkyl, C₁-C₆ alkoxyalkyl, and C₁₋₆ amidoalkyl; or
R₀ is 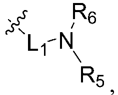 preferably 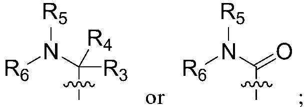 or
R₀ is

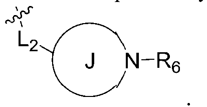

10. The compound according to any one of claims 8 to 9, wherein R₃ and R₄ are independently selected from: H, D, halogen, C₁-C₆ alkyl, C₁-C₆ haloalkyl, C₁-C₆ cyanoalkyl, C₁-C₆ hydroxyalkyl, C₁-C₆ alkoxyalkyl, C₁-C₆ aminoalkyl, C₁-C₆ alkylaminoalkyl, C₃-C₆ cycloalkyl, and C₄-C₁₀ cycloalkylalkyl;
preferably, R₃ is H and R₄ is selected from: H, D, C₁-C₆ alkyl, C₁-C₆ haloalkyl, C₁-C₆ hydroxyalkyl, C₃-C₆ cycloalkyl, and C₄-C₁₀ cycloalkylalkyl; and
more preferably, R₃ is H and R₄ is H; or, R₃ is D and R₄ is D; or, R₃ is H and R₄ is -CH₃; or, R₃ is H and R₄ is cyclopropyl; or, R₃ is H and R₄ is cyclobutyl; or, R₃ is H and R₄ is 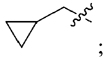 or, R₃ is H and R₄ is 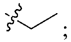 or, R₃ is H and R₄ is 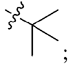 or, R₃ is H and R₄ is

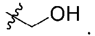

11. The compound according to any one of claims 8 to 10, wherein R₆ is selected from: H, D, C₁-C₆ alkyl, C₃-C₆ cycloalkyl, C₄-C₁₀ cycloalkylalkyl, saturated 4-10 membered heterocyclyl, and heterocyclylalkyl, wherein the alkyl, cycloalkyl, cycloalkylalkyl, heterocyclyl, heterocyclylalkyl is optionally substituted with one or more independent substituents selected from: C₁-C₆ alkyl, C₂-C₆ alkenyl, C₃-C₆ cycloalkyl, 4-6 membered heterocyclyl, halogen, hydroxyl, C₁-C₆ hydroxyalkyl, C₁-C₆ alkoxyl, C₁-C₆ alkoxyalkyl, cyano, C₁-C₆ cyanoalkyl, carboxyl, C₁-C₆ carboxyalkyl, C₁-C₆ haloalkyl, and C₂-C₆ sulfonyl;
preferably, R₆ is selected from: H, D, C₁-C₆ alkyl, C₁-C₆ haloalkyl, C₁-C₆ hydroxyalkyl, C₁-C₆ alkoxyalkyl, 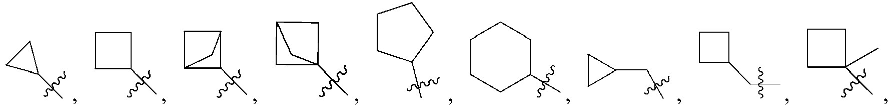 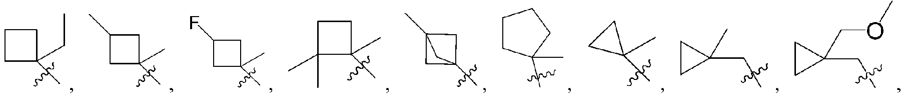 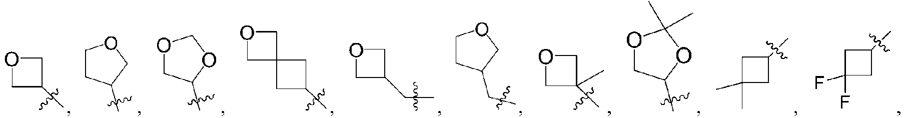 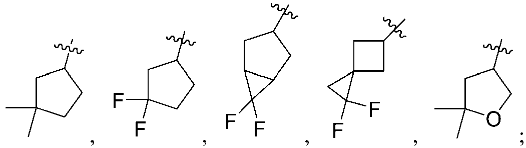 and
more preferably, R₀ is selected from:

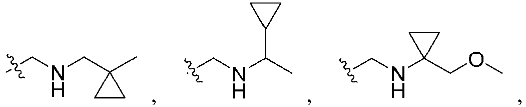

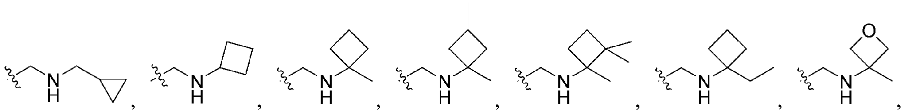

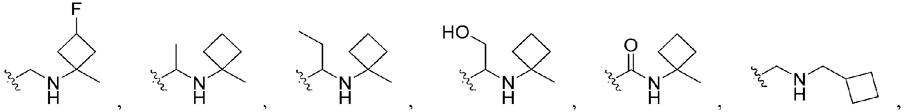

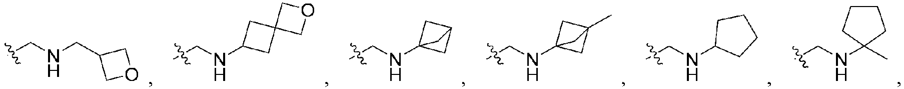

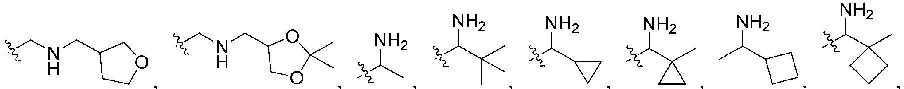

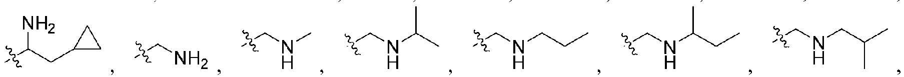

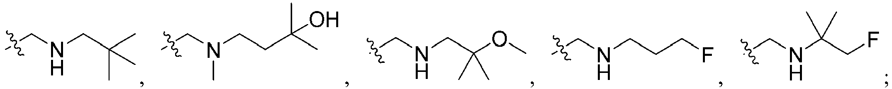

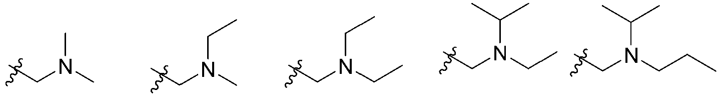

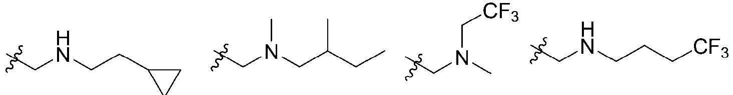

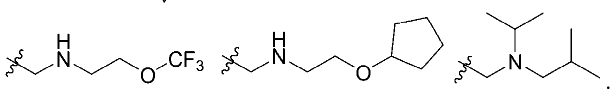

12. The compound according to any one of claims 6 to 10, wherein R₅ and R₆, together with the nitrogen atom to which they are attached, form a heterocyclyl 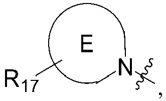 wherein E-ring is 4-14 membered heterocyclic ring;
R₁₇ is one or more independent substituents on the E-ring, each R₁₇ independently selected from: H, D, (=O), halogen, cyano, nitro, C₁-C₁₀ alkyl, C₁-C₁₀ deuterated alkyl, C₂-C₁₀ alkenyl, C₂-C₁₀ alkynyl, -(C₀-C₆ alkylidene)-(C₃-C₁₀ cycloalkyl), -(C₀-C₆ alkylidene)-(C₆-C₁₀ aryl), -(C₀-C₆ alkylidene)-(4-10 membered heterocyclyl), -O(C₀-C₁₀ alkyl), -S(C₀-C₁₀ alkyl), -C(O)(C₀-C₁₀ alkyl), -C(O)N(C₀-C₁₀ alkyl)(C₀-C₁₀ alkyl), -N(C₀-C₁₀ alkyl)(C₀-C₁₀ alkyl), -C(O)O(C₀-C₁₀ alkyl), -S(O)₂(C₀-C₁ oalkyl), -S(O)₂-(C₀-C₆ alkylidene)-(C₃-C₁₀ cycloalkyl), -S(O)₂-(C₀-C₆ alkylidene)-(4-10 membered heterocyclyl), -S(O)₂N(C₀-C₁₀ alkyl)(C₀-C₁₀ alkyl), -S(O)₂N(C₀-C₁₀ alkyl)((C₀-C₆ alkylidene)-(C₃-C₁₀ cycloalkyl)), -S(O)₂N(C₀-C₁₀ alkyl)((C₀-C₆ alkylidene)-(4-10 membered heterocyclyl)), C₁-C₁₀ haloalkyl, C₁-C₁₀ cyanoalkyl, C₁-C₁₀ hydroxyalkyl, C₁-C₁₀ aminoalkyl, C₁-C₁₀ carboxyalkyl, C₁-C₁₀ alkoxyalkyl, and C₁-C₁₀ alkylaminoalkyl; wherein the alkylidene, alkyl, alkenyl, alkynyl, cycloalkyl, aryl, heterocyclyl is each optionally substituted with one or more independent substituents selected from: D, halogen, cyano, nitro, C₁₋₁₀ alkyl, -(C₀-C₆ alkylidene)-(C₃-C₁₀ cycloalkyl), -(C₀-C₆ alkylidene)-(C₆-C₁₀ aryl), -(C₀-C₆ alkylidene)-(4-10 membered heterocyclyl), -O(C₀-C₁₀ alkyl), -S(C₀-C₁₀ alkyl), -C(O)(C₀-C₁₀ alkyl), -C(O)N(C₀-C₁₀ alkyl)(C₀-C₁₀ alkyl), -N(C₀-C₁₀ alkyl)(C₀-C₁₀ alkyl), -C(O)O(C₀-C₁₀ alkyl), -S(O)₂(C₀-C₁₀ alkyl), -S(O)₂-(C₀-C₆ alkylidene)-(C₃-C₁₀ cycloalkyl), -S(O)₂-(C₀-C₆ alkylidene)-(4-10 membered heterocyclyl), -S(O)₂N(C₀-C₁₀ alkyl)(C₀-C₁₀ alkyl), -S(O)₂N(C₀-C₁₀ alkyl)((C₀-C₆ alkylidene)-(C₃-C₁₀ cycloalkyl)), and -S(O)₂N(C₀-C₁₀ alkyl)((C₀-C₆ alkylidene)-(4-10 membered heterocyclyl)); wherein the heterocyclyl is optionally substituted with one or more independent substituents selected from: halogen, cyano, nitro, hydroxyl, amino, C₁-C₁₀ alkyl, substituted or unsubstituted phenyl, or 4-6 membered heterocyclyl;
preferably, E-ring is selected from: 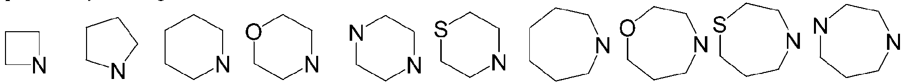 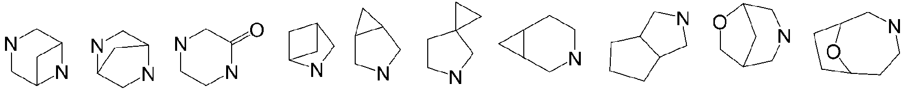 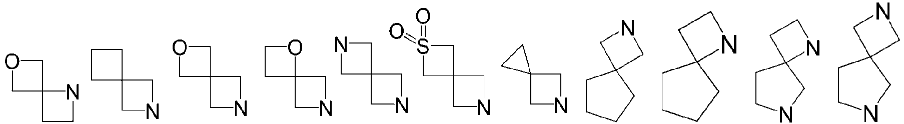 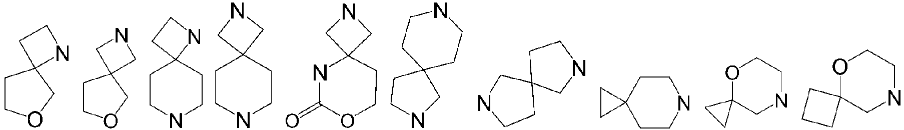 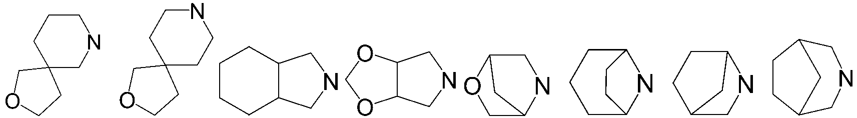 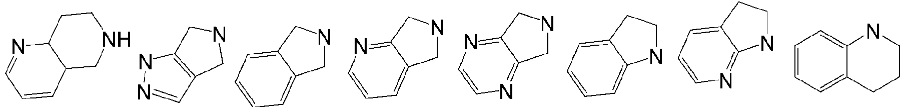 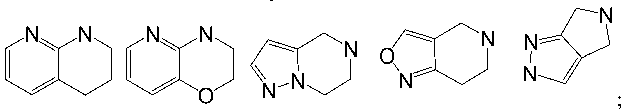
preferably, each R₁₇ is independently selected from: H, D, (=O), halogen, C₁-C₆ alkyl, C₁-C₆ deuterated alkyl, C₂-C₆ alkenyl, C₁-C₆ haloalkyl, cyano, C₁-C₆ cyanoalkyl, -OH, C₁-C₆ alkoxyl, C₁-C₆ deuterated alkoxyl, C₁-C₆ hydroxyalkyl, C₁-C₆ alkoxyalkyl, -NH₂, C₁-C₆ alkylamino, C₁-C₆ alkylaminoalkyl, -(C₀-C₃ alkylidene)-(C₃-C₆ cycloalkyl), -(C₀-C₆ alkylidene)-(saturated 4-10 membered heterocyclyl), -S(O)₂(C₀-C₆ alkyl), -S(O)₂-(C₀-C₆ alkylidene)-(C₃-C₆ cycloalkyl), -S(O)₂-(C₀-C₆ alkylidene)-(4-6 membered heterocyclyl), -S(O)₂N(C₀-C₆ alkyl)(C₀-C₆ alkyl), -S(O)₂N(C₀-C₆ alkyl)((C₀-C₆ alkylidene)-(C₃-C₆ cycloalkyl)), -S(O)₂N(C₀-C₆ alkyl)((C₀-C₆ alkylidene)-(4-6 membered heterocyclyl)), -C(O)(C₀-C₆ alkyl), -C(O)O(C₀-C₁₀ alkyl), -C(O)N(C₀-C₆ alkyl)(C₀-C₆ alkyl), C₁-C₆ haloalkoxyl, and -C(O)O(C₀-C₆ alkyl); and
more preferably, the 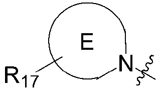 is selected from the following structure:

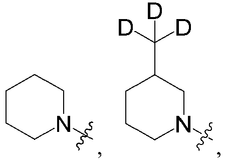

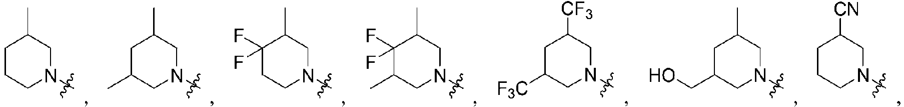

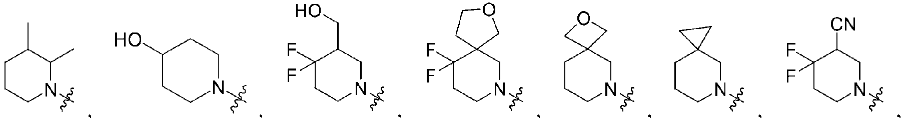

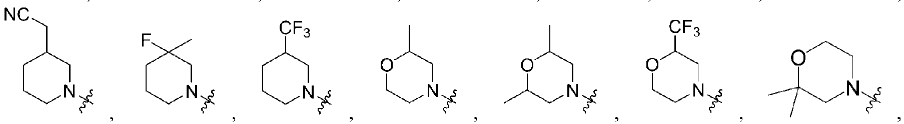

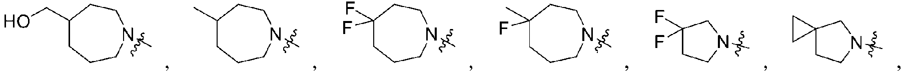

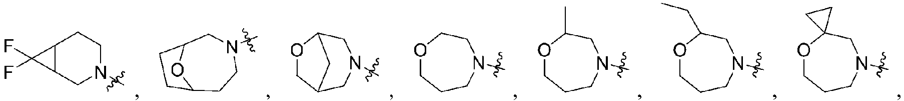

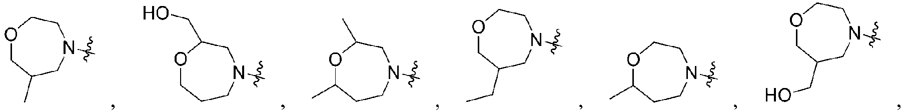

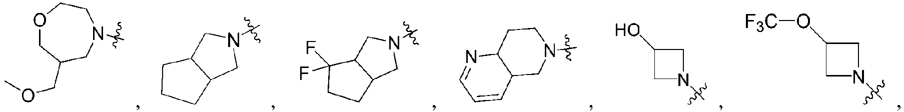

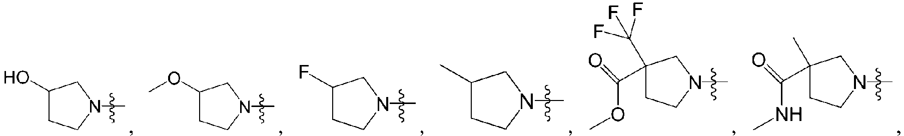

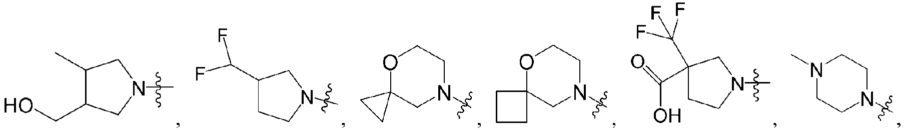

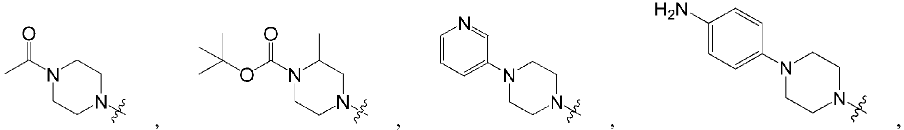

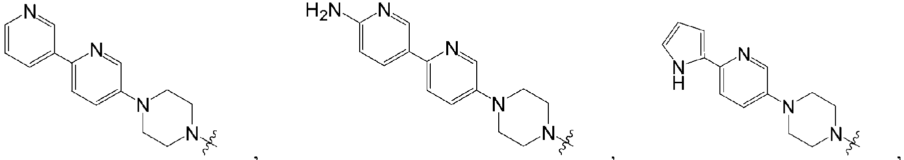

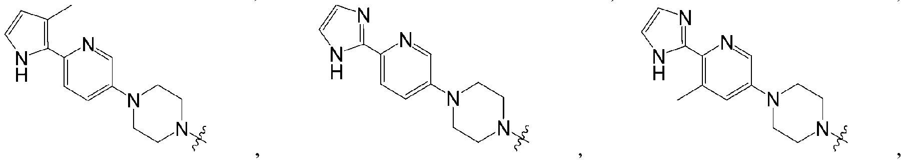

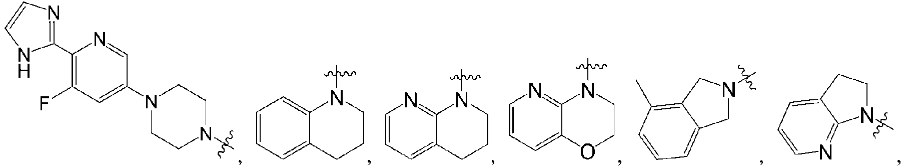

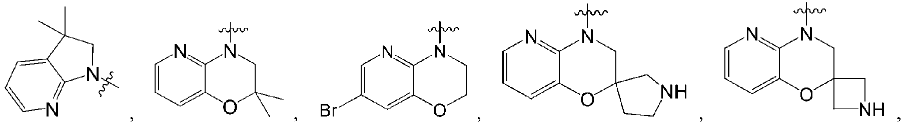

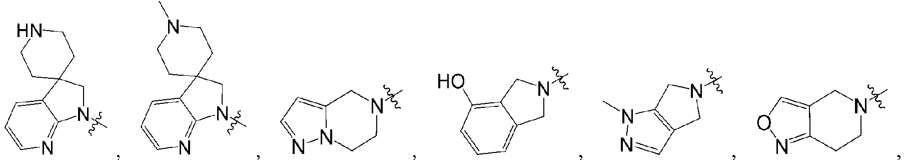

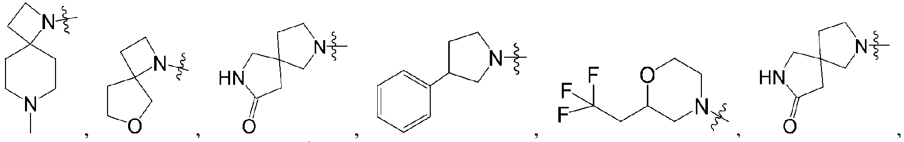

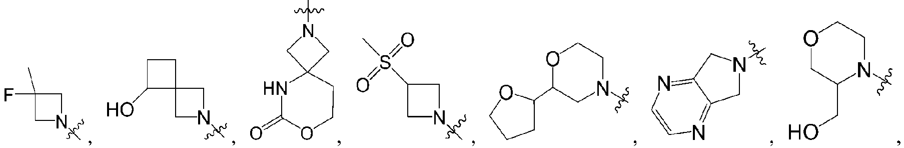

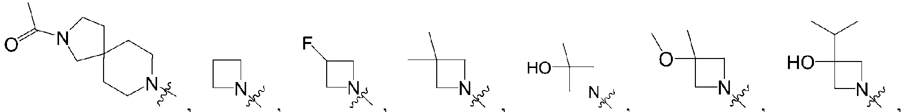

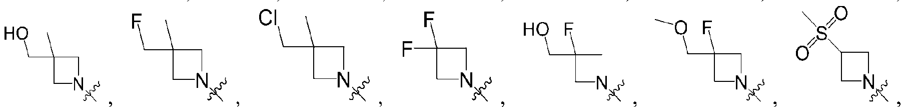

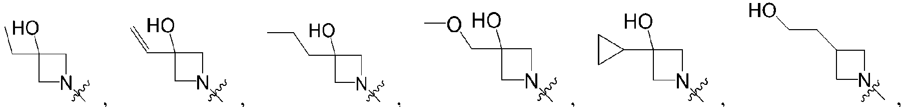

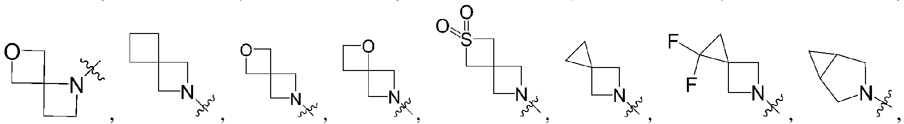

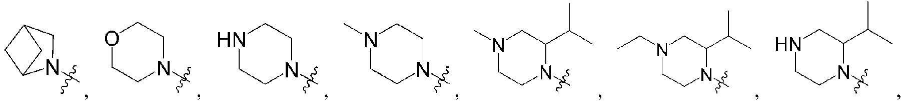

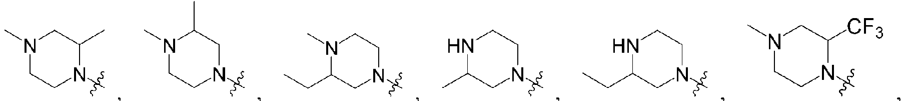

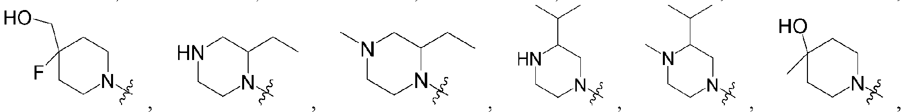

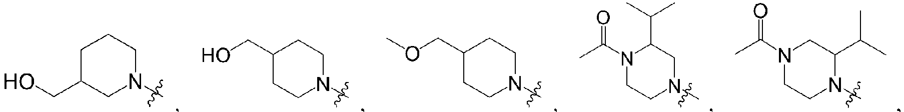

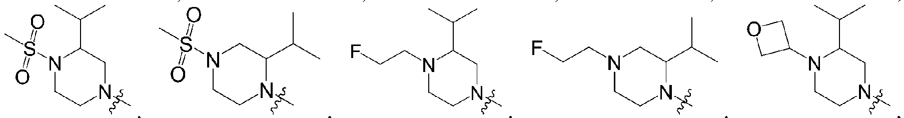

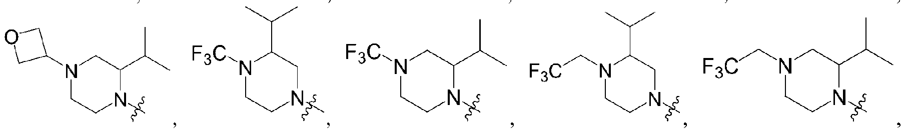

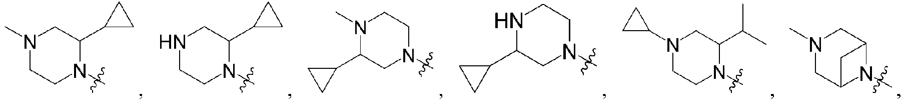

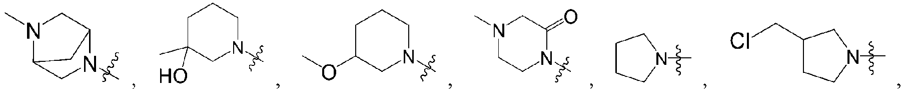

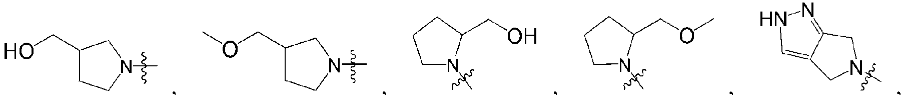

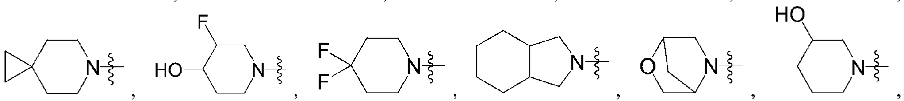

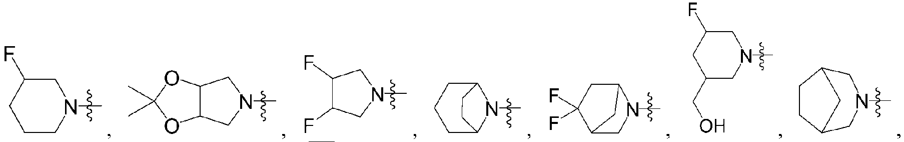

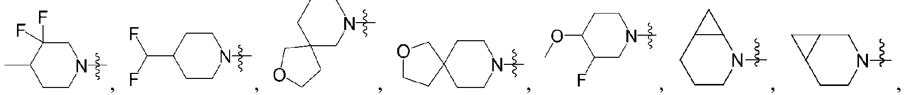

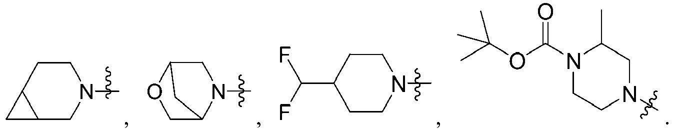

13. The compound according to any one of claims 8 to 11, wherein the J-ring a 5 or 6 membered heterocyclic aromatic ring (such as 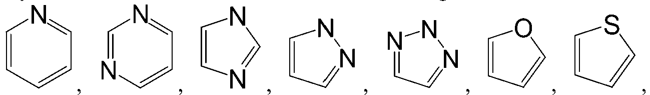 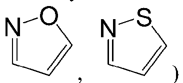 or a saturated 4-8 membered heterocyclic ring 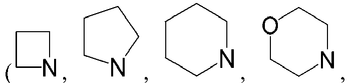 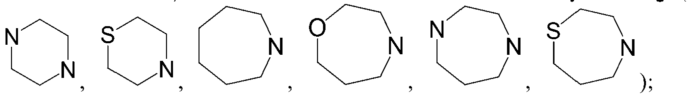 preferably, the 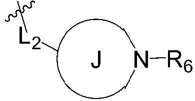 is selected from the following structure:

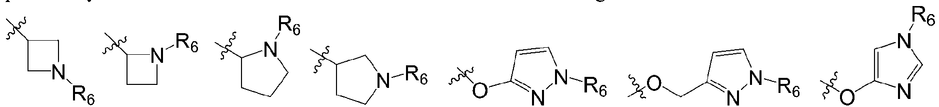

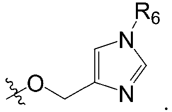

14. The compound according to any one of claims 8 to 13, wherein R₁ is 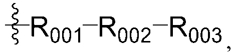 and R₀₀₁ is selected from: a single bond and C₁-C₆ alkylidene; R₀₀₂ is selected from: a single bond, C₁-C₆ alkylidene, O, S, N(R₂), S(O)₂, S(O)₂N(R₂), S(O), S(O)N(R₂), C(O), C(O)O, C(O)N(R₂), OC(O), OC(O)N(R₂), N(R₂)C(O)O, N(R₂)C(O), and N(R₂)S(O)₂; R₀₀₃ is selected from: H, D, halogen, cyano, nitro, hydroxyl, C₁-C₆ alkyl, C₁-C₆ haloalkyl, C₂-C₆ alkenyl, C₂-C₆ alkynyl, C₃-C₆ cycloalkyl, C₃-C₆ halocycloalkyl, C₄-C₁₀ cycloalkylalkyl, saturated 4-10 membered heterocyclyl, and heterocyclylalkyl; R₁ is optionally substituted by one or more independent substituents selected from: halogen, hydroxyl, C₁-C₆ alkoxyl, amino, C₁-C₆ alkylamino, cyano, and carboxyl;
preferably, R₁ is selected from: H, D, halogen, hydroxyl, C₁-C₆ alkyl, C₁-C₆ haloalkyl, C₂-C₆ alkenyl, C₁-C₆ alkoxyl, C₁-C₆ haloalkoxyl, C₁-C₆ alkoxyalkyl, C₁-C₆ haloalkoxyalkyl, C₃-C₆ cycloalkyl, C₃-C₆ halocycloalkyl, C₄-C₁₀ cycloalkylalkyl, saturated 4-6 membered heterocyclyl, -S(O)₂(C₀-C₆ alkyl), -S(O)₂-(C₀-C₆ alkylidene)-(C₃-C₆ cycloalkyl), -S(O)₂-(C₀-C₆ alkylidene)-(4-6 membered heterocyclyl), -S(O)₂N(C₀-C₆ alkyl)(C₀-C₆ alkyl), -S(O)₂N(C₀-C₆ alkyl)((C₀-C₆ alkylidene)-(C₃-C₆ cycloalkyl)), and -S(O)₂N(C₀-C₆ alkyl)((C₀-C₆ alkylidene)-(4-6 membered heterocyclyl)); and
more preferably, R₁ is selected from: H, D, F, Br, -CH₃, 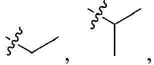 -CHF₂, -CH₂F, -CF₃, -CH₂-CF₃, 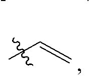 -OH, 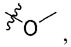 -OCHF₂, -OCH₂F, -OCF₃,

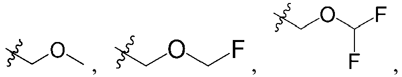

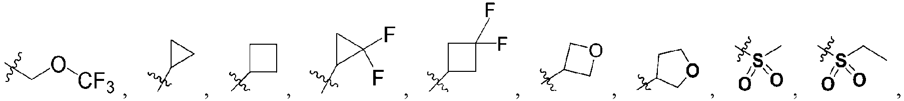

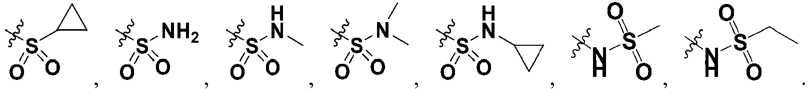

15. The compound according to any one of claims 8 to 14, wherein R₇ is selected from: H, D, halogen, cyano, C₁-C₆ alkyl, C₁-C₆ haloalkyl, -(C₀-C₆ alkylidene)-(C₃-C₆ cycloalkyl), -(C₀-C₆ alkylidene)-(4-6 membered heterocyclyl), -C(O)(C₁-C₆ alkyl), -C(O)N(C₀-C₆ alkyl)(C₀-C₆ alkyl), -N(C₀-C₆ alkyl)(C₀-C₆ alkyl), -N(C₀-C₆ alkyl)((C₀-C₆ alkylidene)-(C₃₋₆ cycloalkyl)), -N(C₀₋₆ alkyl)((C₀₋₆ alkylidene)-(4-6 membered heterocyclyl)), -O-(C₀₋₆ alkylidene)-(C₃-C₆ cycloalkyl), -O-(C₀-C₆ alkylidene)-(4-6 membered heterocyclyl), -S(O)₂(C₀-C₆ alkyl), -S(O)₂-(C₀-C₆ alkylidene)-(C₃-C₆ cycloalkyl), -S(O)₂-(C₀-C₆ alkylidene)-(4-6 membered heterocyclyl), -S(O)₂N(C₀-C₆ alkyl)(C₀-C₆ alkyl), -S(O)₂N(C₀-C₆ alkyl)((C₀-C₆ alkylidene)-(C₃-C₆ cycloalkyl)), and -S(O)₂N(C₀-C₆ alkyl)((C₀-C₆ alkylidene)-(4-6 membered heterocyclyl));
preferably, R₇ is selected from: -H, D, -Cl, -CN, -COOH, -CONH₂, 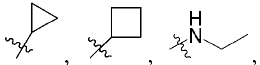 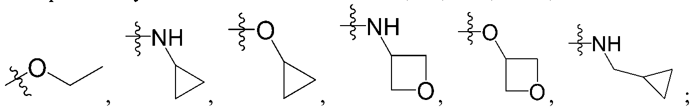 and
more preferably, R₇ is H.

16. The compound according to any one of claims 7 to 15, wherein R₈ and R₉ are independently selected from: H, D, halogen, C₁-C₆ alkyl, C₁-C₆ haloalkyl, and 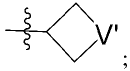 wherein V' is selected from: a single bond, O, 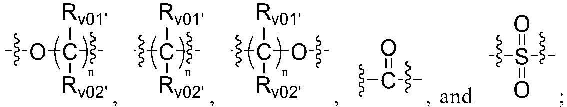 n is 1, 2, or 3; Rᵥ₀₁' and Rᵥ₀₂' are independently selected from: H, D, halogen, cyano, nitro, hydroxyl, amino, C₁-C₆ alkyl, C₁-C₆ deuterated alkyl, C₁-C₆ alkoxyl, C₁-C₆ deuterated alkoxyl, C₃-C₆ cycloalkyl, and 3-8 membered heterocycloalkyl; or, Rᵥ₀₁' and Rᵥ₀₂', together with carbon atom to which they are attached, form a cycloalkyl or a heterocyclyl; wherein the alkyl is optionally substituted by one or more independent substituents selected from: halogen, cyano, nitro, hydroxyl, C₁-C₆ alkoxyl, amino, C₁-C₆ alkylamino, -S(O)₂(C₀-C₆ alkyl), -S(O)₂-(C₀-C₆ alkylidene)-(C₃-C₆ cycloalkyl), -S(O)₂-(C₀-C₆ alkylidene)-(4-6 membered heterocyclyl), -S(O)₂N(C₀-C₆ alkyl)(C₀-C₆ alkyl), -S(O)₂N(C₀-C₆ alkyl)((C₀-C₆ alkylidene)-(C₃-C₆ cycloalkyl)), -S(O)₂N(C₀-C₆ alkyl)((C₀-C₆ alkylidene)-(4-6 membered heterocyclyl)), -C(O)(C₀-C₆ alkyl), and -C(O)N(C₀-C₆ alkyl)(C₀-C₆ alkyl);
preferably, R₈ and R₉ are independently selected from: H, D, F, -CH₃, -CHF₂, -CH₂F, -CF₃, 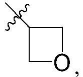 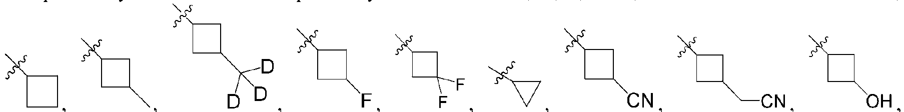 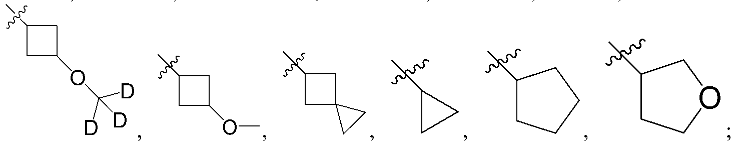 and
more preferably, R₈ and R₉ are both methyl; or, R₈ and R₉ are both H; or, R₈ and R₉ are both halogen (e.g., F); or, R₈ is methyl and R₉ is halogen (e.g., F); or, R₈ is H and R₉ is

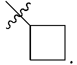

17. The compound according to any one of claims 7 to 15, wherein R₈ and R₉, together with carbon atom to which they are attached, form 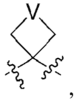 wherein V is selected from: a single bond, O, 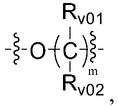 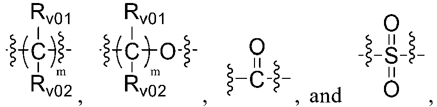 and m is 1, 2 or 3; Rᵥ₀₁ and Rᵥ₀₂ are independently selected from: H, D, halogen, cyano, nitro, hydroxyl, amino, C₁-C₆ alkyl, C₁-C₆ deuterated alkyl, C₁-C₆ alkoxyl, and C₁-C₆ deuterated alkoxyl, or, Rᵥ₀₁ and Rᵥ₀₂, together with carbon atom to which they are attached, form a cycloalkyl or a heterocyclyl, wherein the alkyl is optionally substituted by one or more independent substituents selected from: halogen, cyano, nitro, hydroxyl, C₁-C₆ alkoxyl, amino, C₁-C₆ alkylamino, -S(O)₂(C₀-C₆ alkyl), -S(O)₂-(C₀-C₆ alkylidene)-(C₃-C₆ cycloalkyl), -S(O)₂-(C₀-C₆ alkylidene)-(4-6 membered heterocyclyl), -S(O)₂N(C₀-C₆ alkyl)(C₀-C₆ alkyl), -S(O)₂N(C₀-C₆ alkyl)((C₀-C₆ alkylidene)-(C₃-C₆ cycloalkyl)), -S(O)₂N(C₀-C₆ alkyl)((C₀-C₆ alkylidene)-(4-6 membered heterocyclyl)), -C(O)(C₀-C₆ alkyl), and -C(O)N(C₀-C₆ alkyl)(C₀-C₆ alkyl); or, Rᵥ₀₁ and Rᵥ₀₂ form C₁-C₆ alkylidene;
preferably, Rᵥ₀₁ and Rᵥ₀₂ are independently selected from: H, D, halogen, cyano, nitro, hydroxyl, amino, C₁-C₆ alkyl, C₁-C₆ deuterated alkyl, C₁-C₆ haloalkyl, C₁-C₆ cyanoalkyl, C₁-C₆ hydroxyalkyl, C₁-C₆ alkoxyalkyl, C₁-C₆ deuterated alkoxyl, C₁-C₆ aminoalkyl, and C₁-C₆ alkylaminoalkyl;
more preferably, Rᵥ₀₁ and Rᵥ₀₂ are both H; or, Rᵥ₀₁ and Rᵥ₀₂ are both halogen (e.g., F); or, Rᵥ₀₁ is H, and Rᵥ₀₂ is halogen(e.g., F); or, Rᵥ₀₁ is H, and Rᵥ₀₂ is methyl; or, Rᵥ₀₁ is H, and Rᵥ₀₂ id -CD₃; Rᵥ₀₁ is -CN or -CH₂-CN, and Rᵥ₀₂ is H; or, Rᵥ₀₁ is H, and Rᵥ₀₂ is hydroxyl; or, Rᵥ₀₁ is H, and Rᵥ₀₂ is C₁₋C₃ alkoxyl; or, Rᵥ₀₁ is H, and Rᵥ₀₂ is C₁₋C₃ deuterated alkoxyl; or, Rᵥ₀₁ and Rᵥ₀₂, together with carbon atom to which they are attached, form C₃₋₆ cycloalkyl; or, Rᵥ₀₁ and Rᵥ₀₂ form 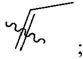 and
preferably, 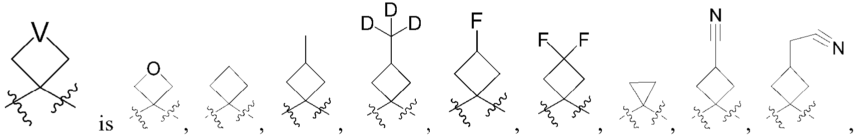 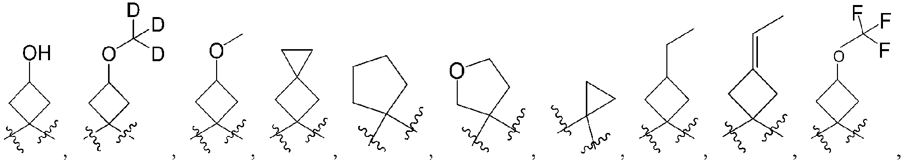 especially

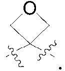

18. The compound according to any one of claims 7 to 17, wherein R₁₅ and R₁₆ are independently selected from: H, D, halogen(e.g., F), -O(C₀-C₆ alkyl), -S(C₀-C₆ alkyl), -N(C₀-C₆ alkyl)(C₀-C₆ alkyl), C₁-C₆ alkyl, C₁-C₆ haloalkyl, C₁-C₆ hydroxyalkyl, and C₁-C₆ aminoalkyl; and
preferably, W is a single bond, -CH₂-, 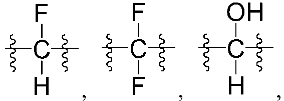 or S.

19. The compound according to any one of claims 7 to 17, wherein R₁₅ and R₉, together with carbon atom to which they are attached, form 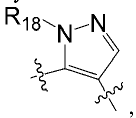 wherein R₁₈ is selected from: H, D, C₁-C₆ alkyl, C₁-C₆ haloalkyl, and C₃-C₆ cycloalkyl; or R₁₅ and R₉, together with intervening carbon atom, form 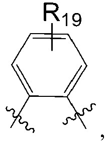 wherein R₁₉ is selected from: H, D, halogen, -CN, -NO₂, -OH, C₁-C₆ alkyl, C₁-C₆ haloalkyl, and C₃-C₆ cycloalkyl.

20. The compound according to any one of claims 8 to 19, wherein 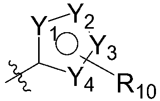 can be selected from a group consisting of 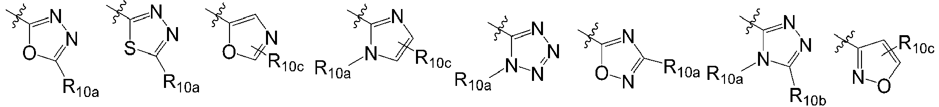
wherein, R_{10c} is one or more independent substituents on the ring and R₁₀ₐ, R_{10b} and R_{10c} can be selected from: H, D, C₁₋₆ alkyl, C₁₋₆ haloalkyl, and C₃₋₆ cycloalkyl;
preferably, R₁₀ₐ, R_{10b} and R_{10c} can be selected from: H, D, -CH₃, -CHF₂, -CH₂F, -CF₃, 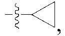 and 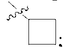 and
preferably, 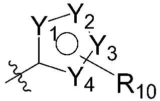 is 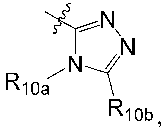 more preferably

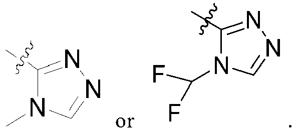

21. The compound according to claim 1, wherein the compound is selected from the following structure:

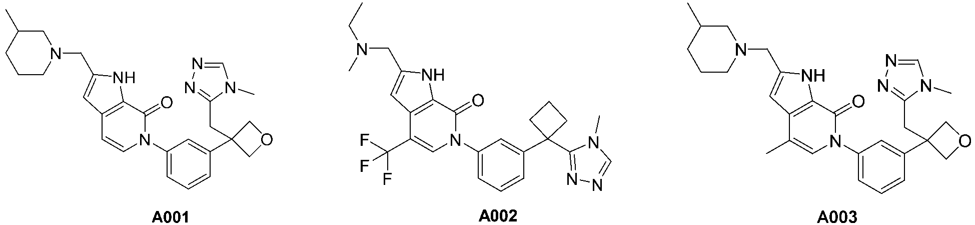

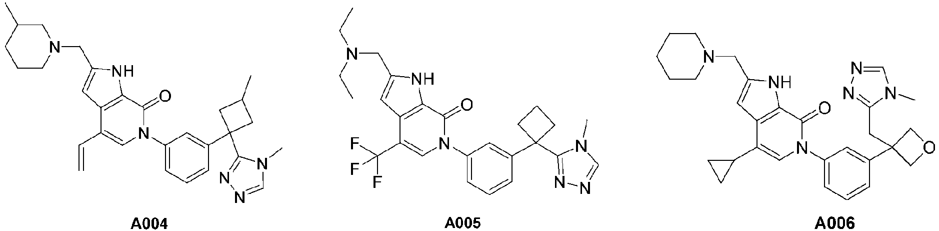

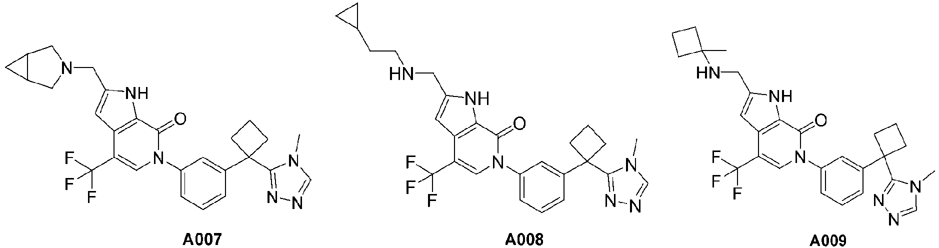

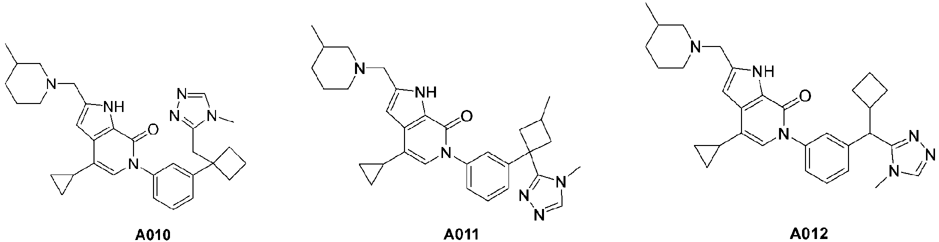

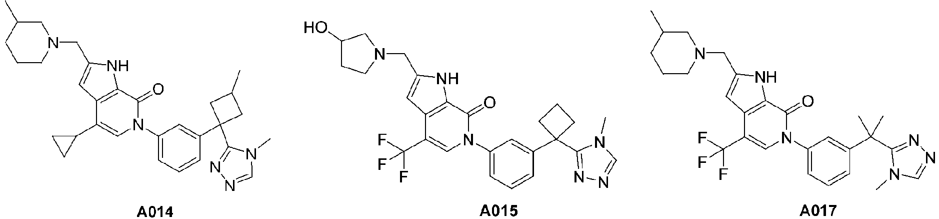

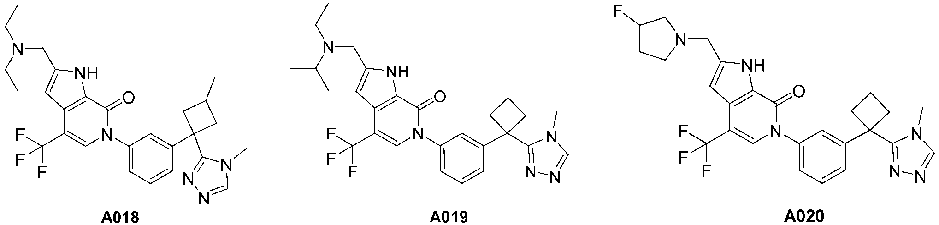

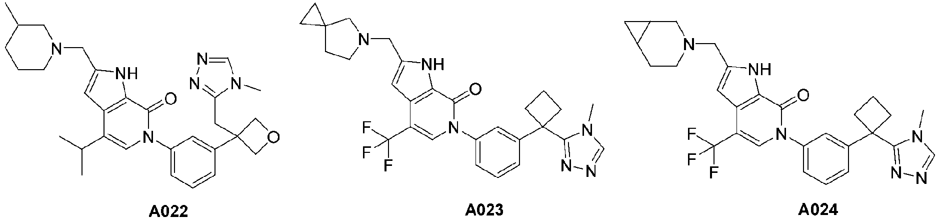

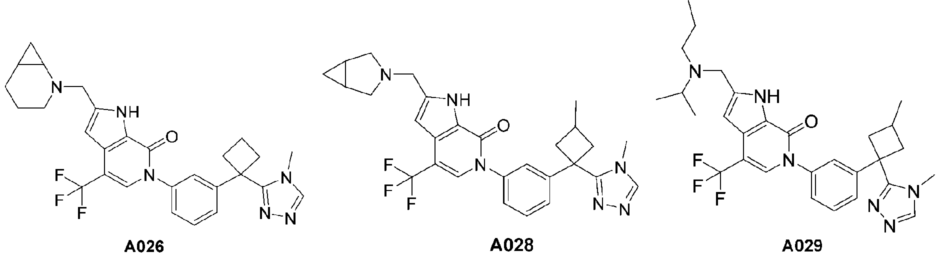

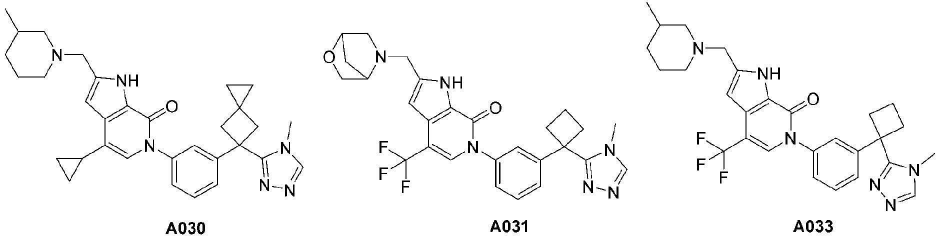

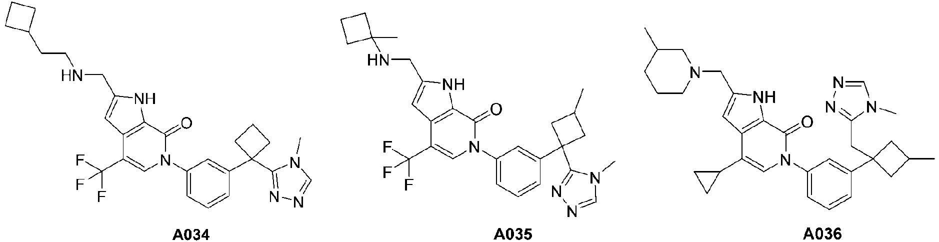

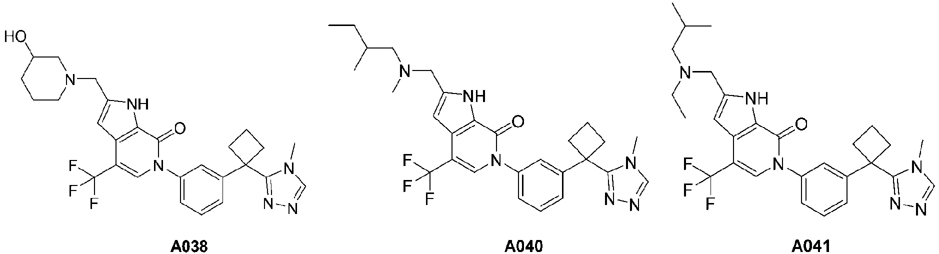

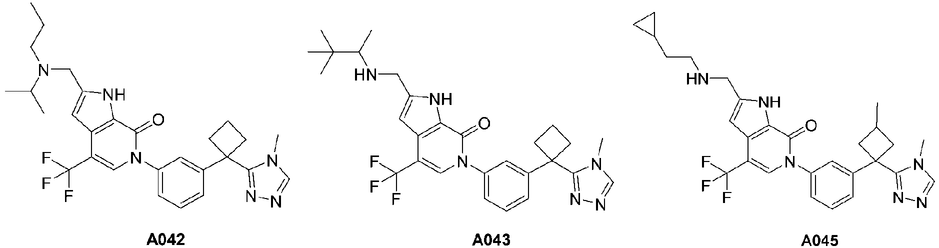

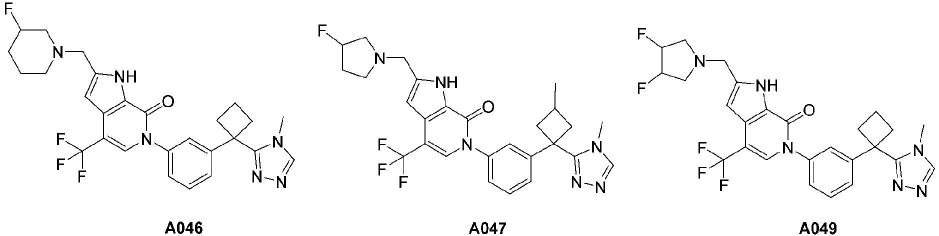

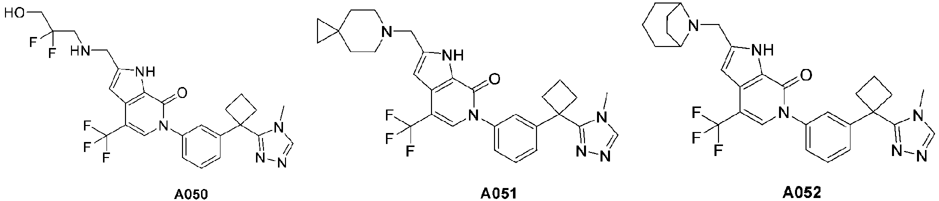

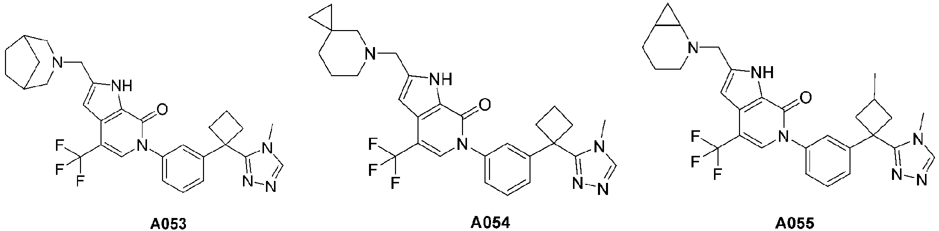

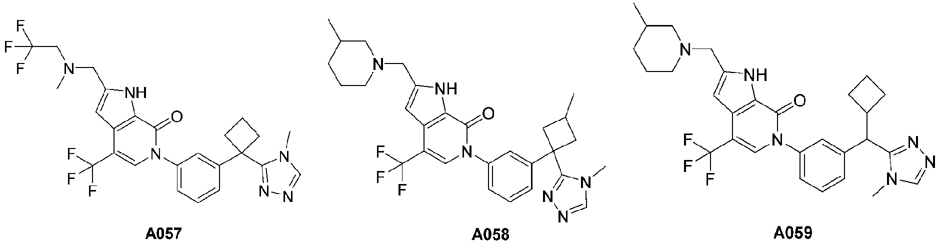

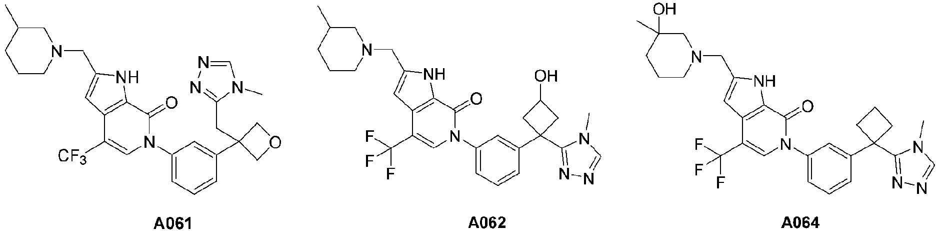

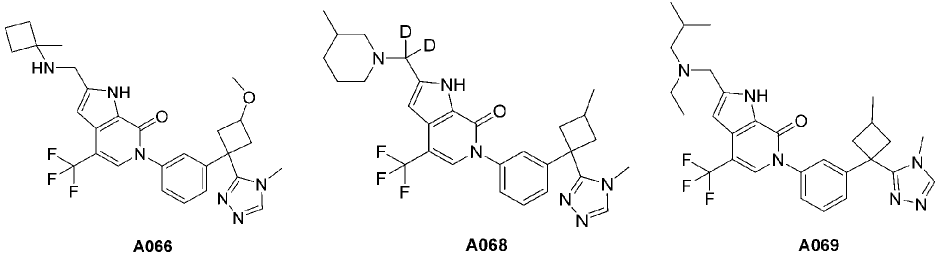

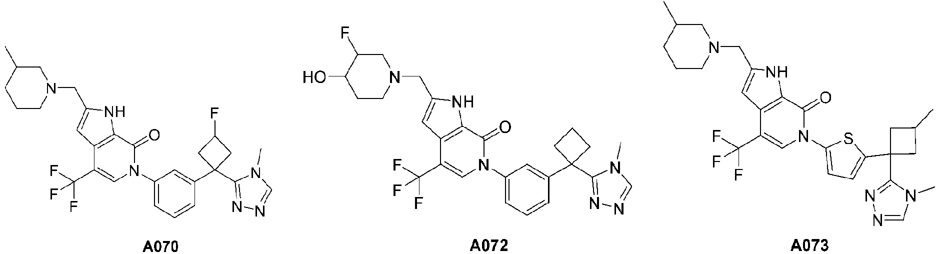

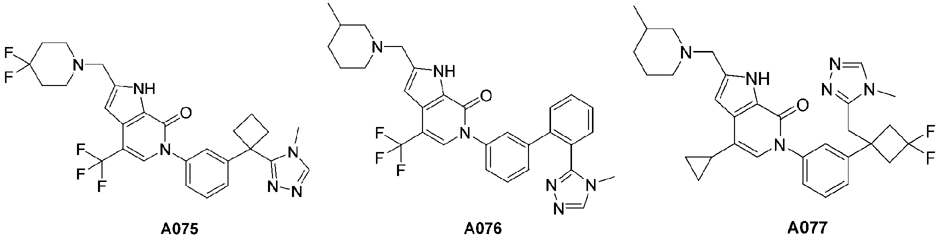

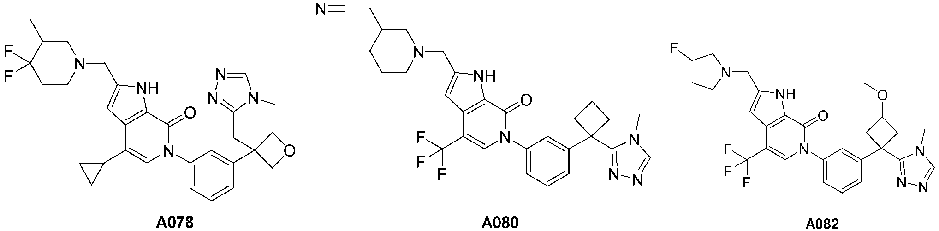

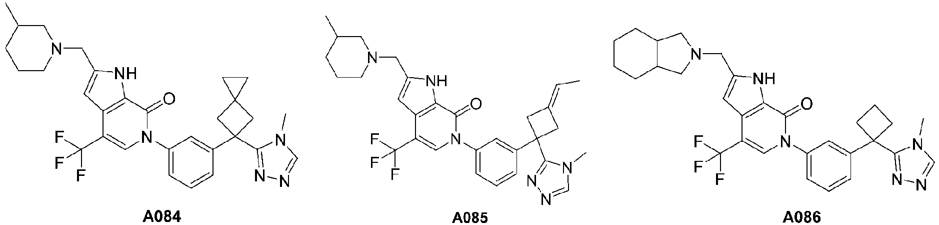

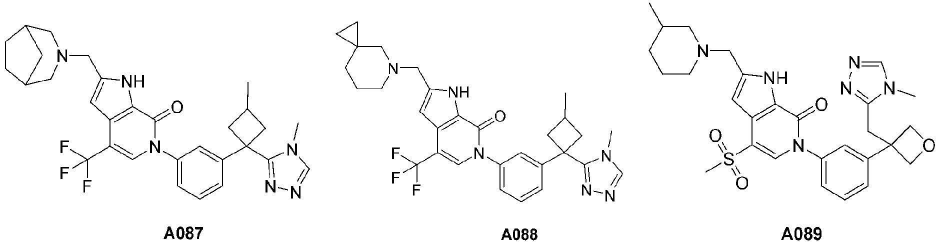

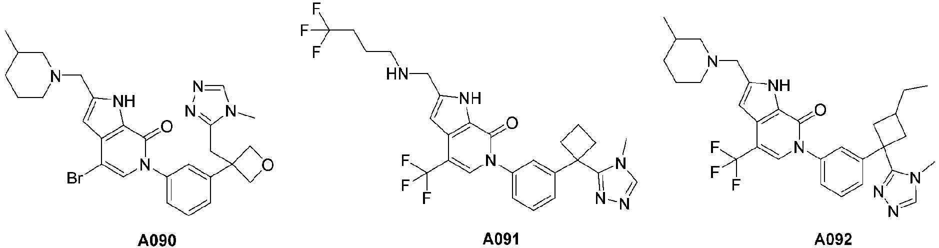

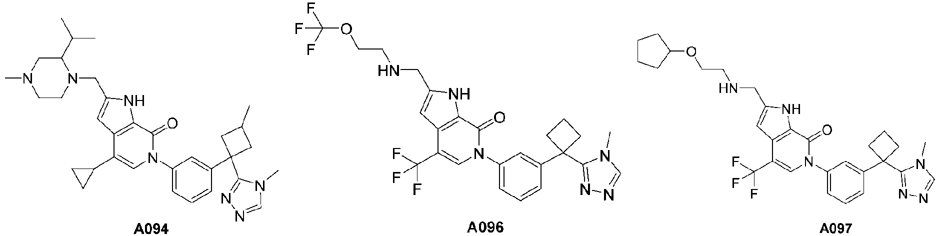

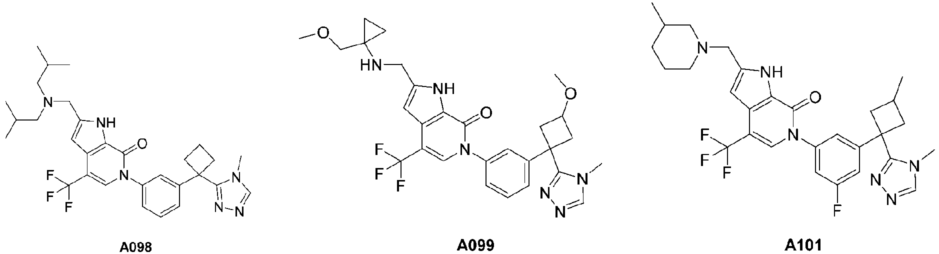

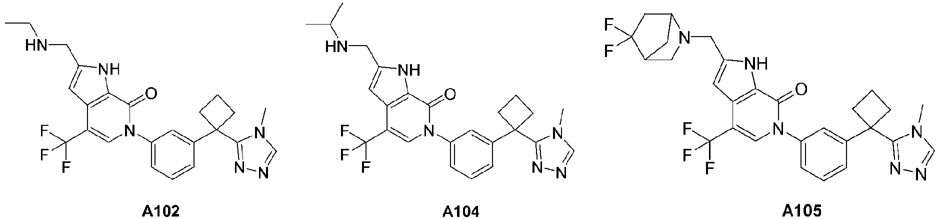

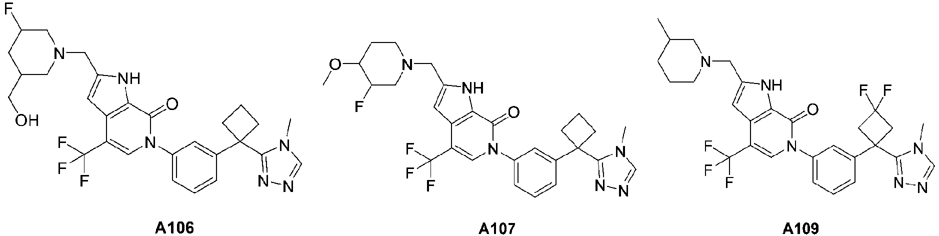

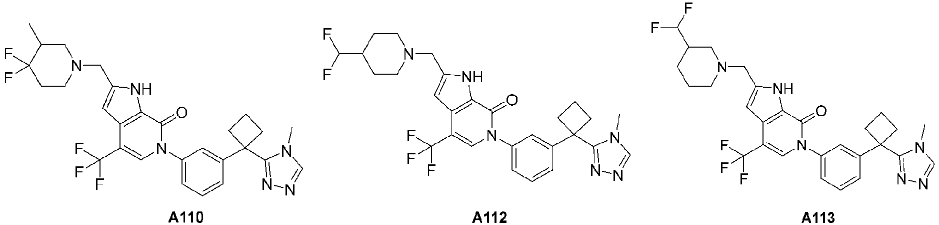

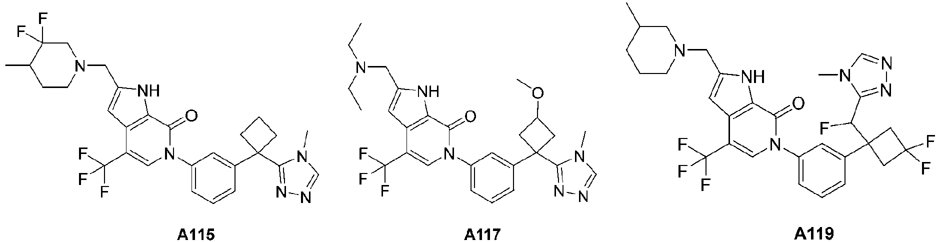

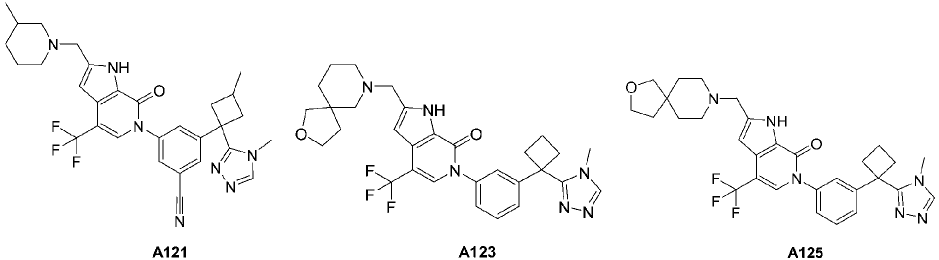

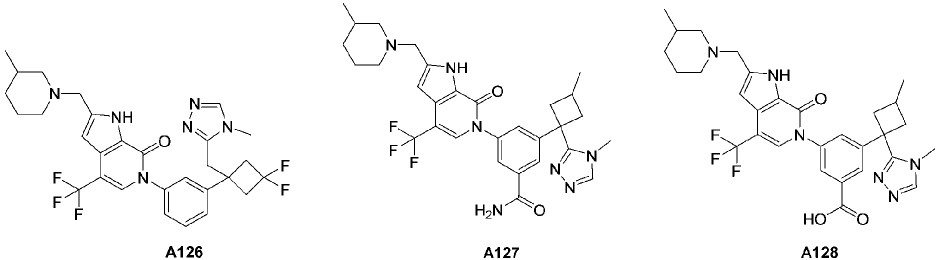

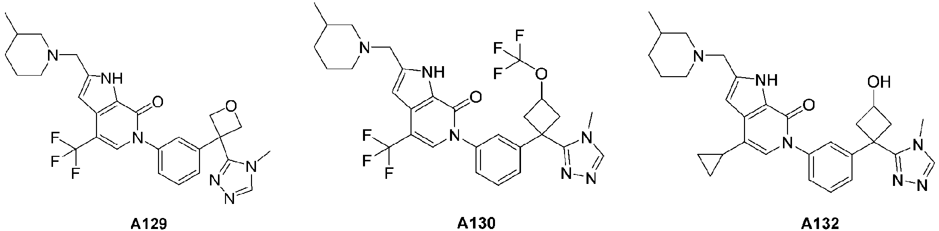

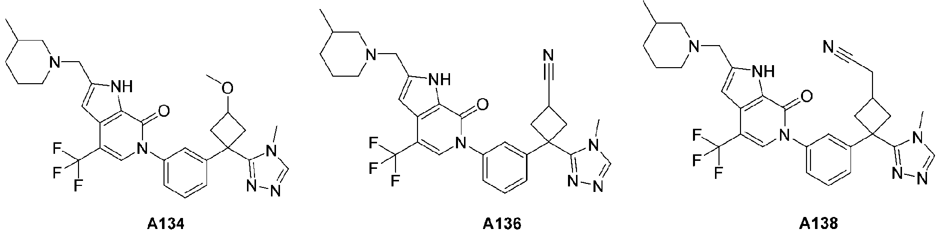

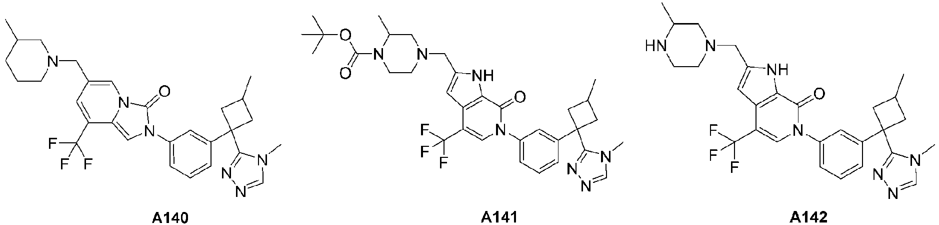

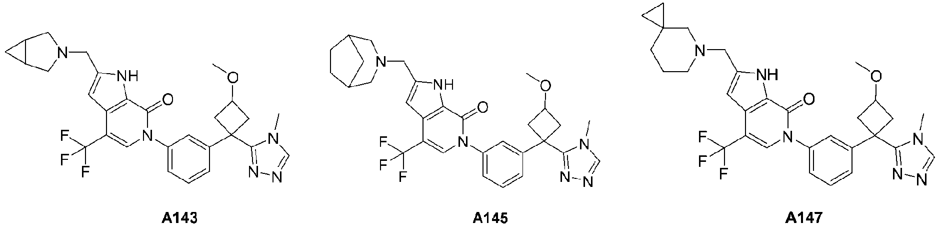

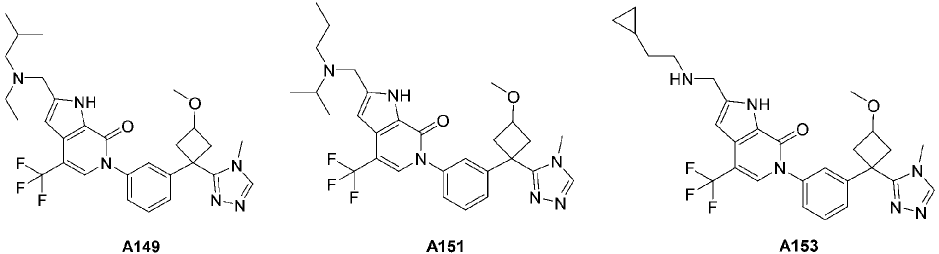

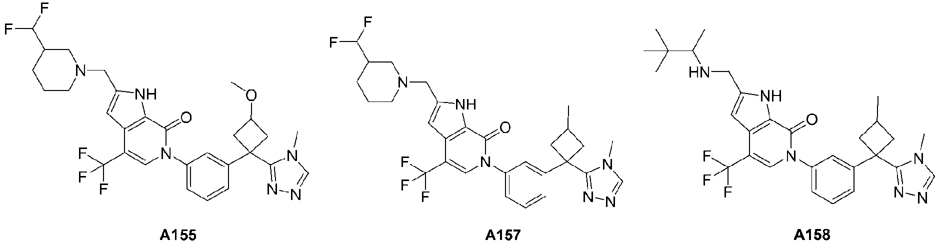

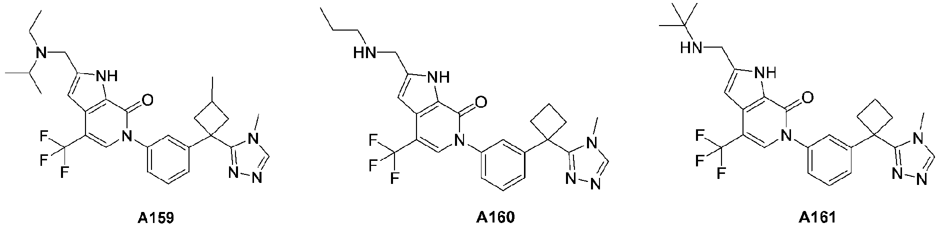

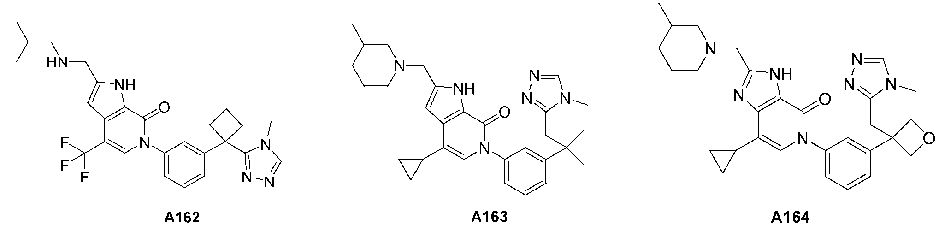

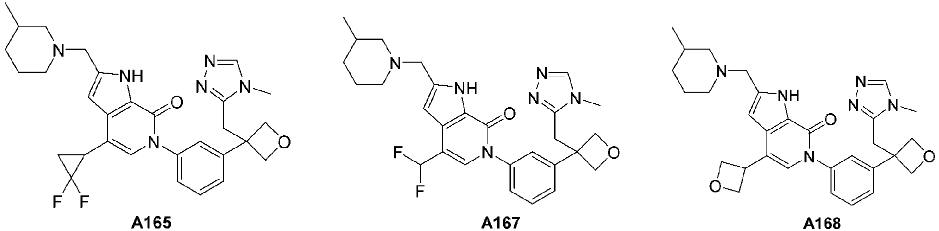

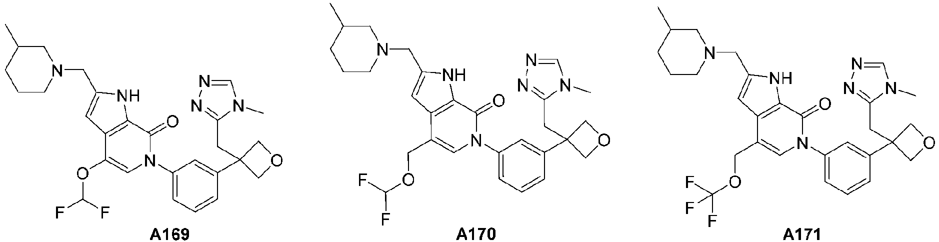

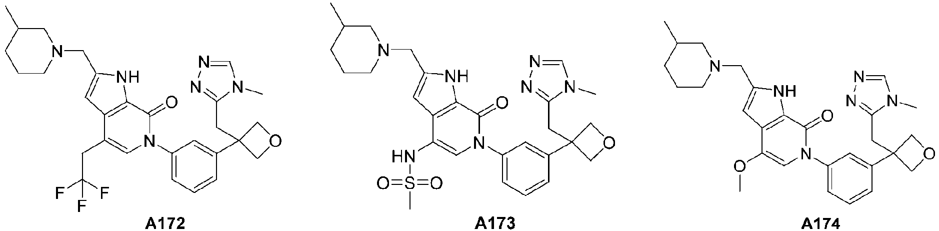

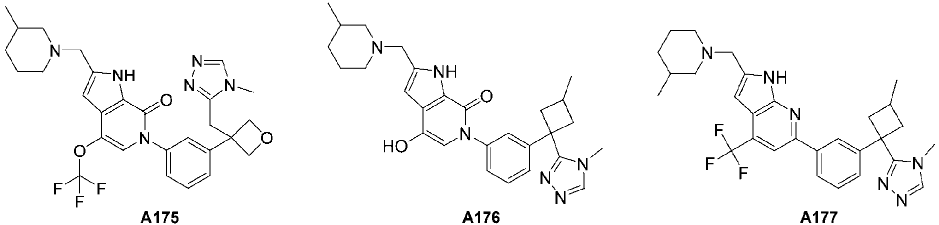

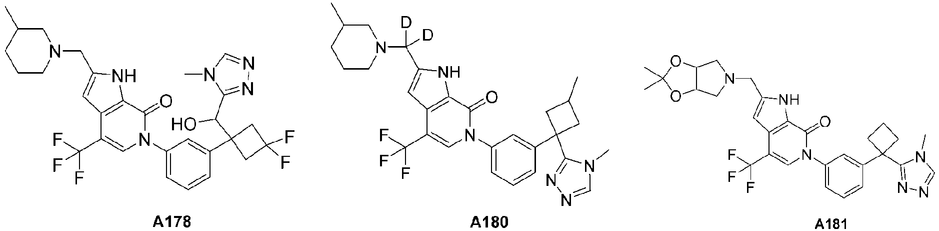

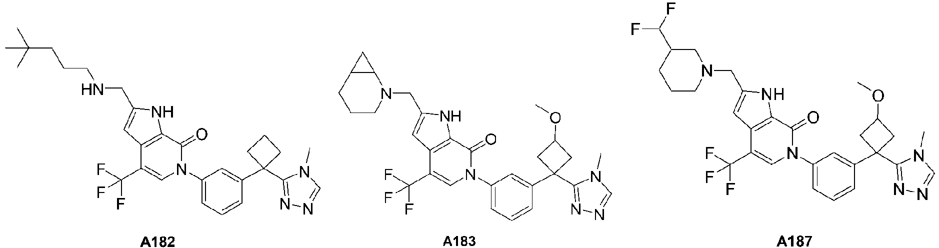

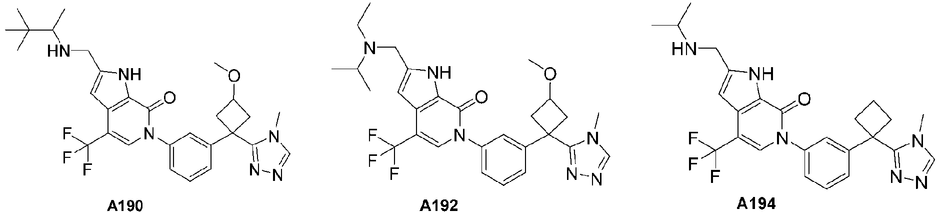

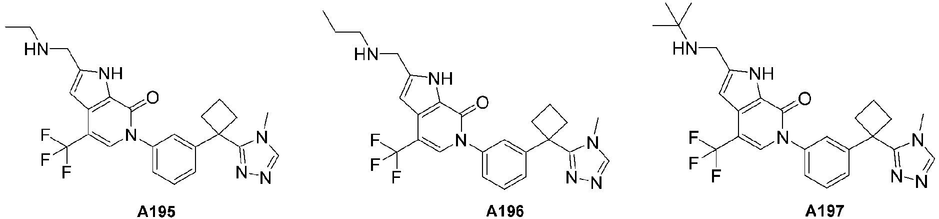

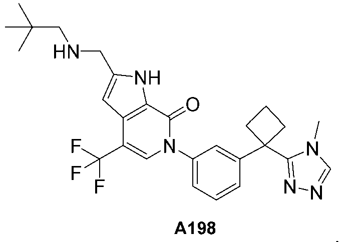

22. The compound according to claim 1, wherein the stereoisomer is selected from the following structure:

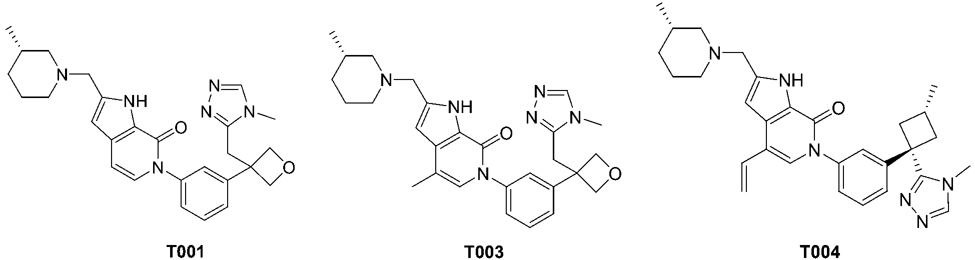

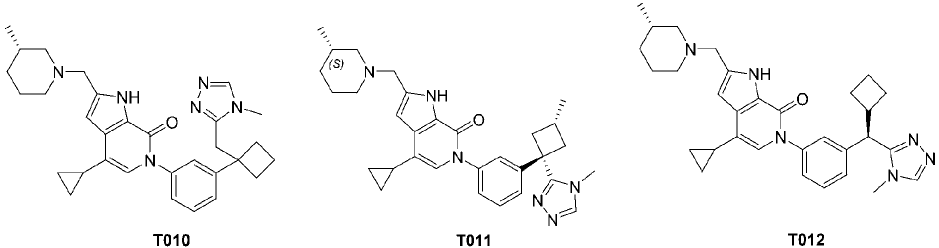

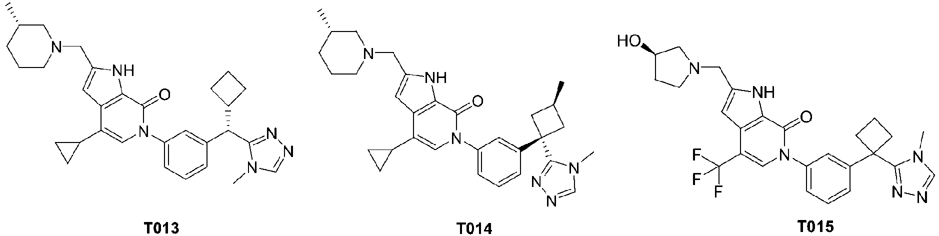

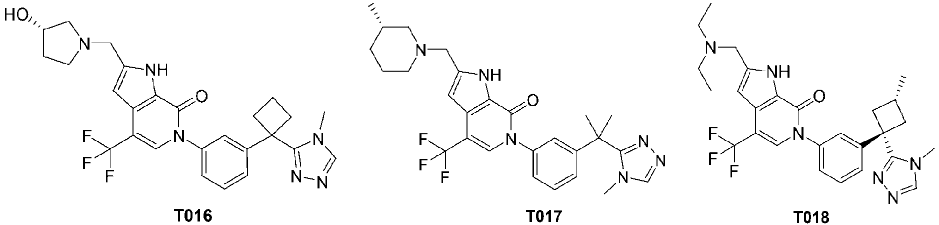

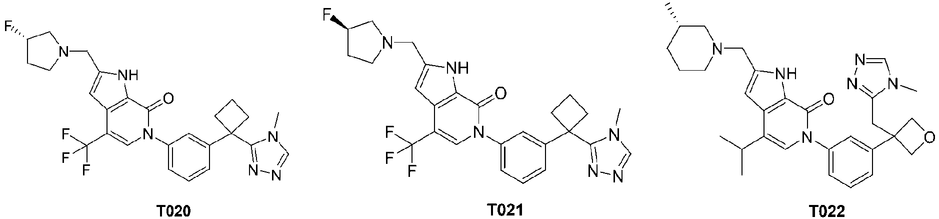

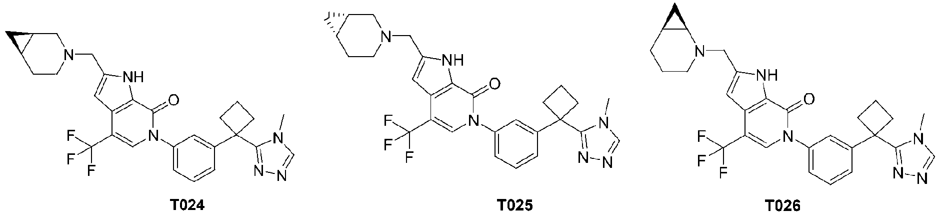

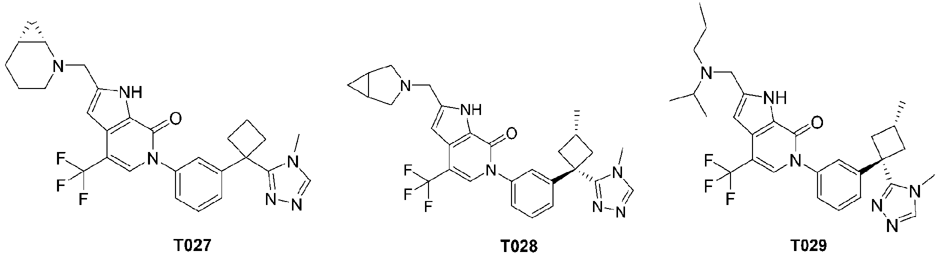

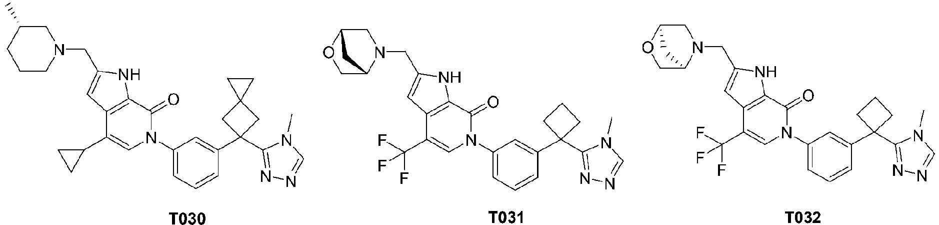

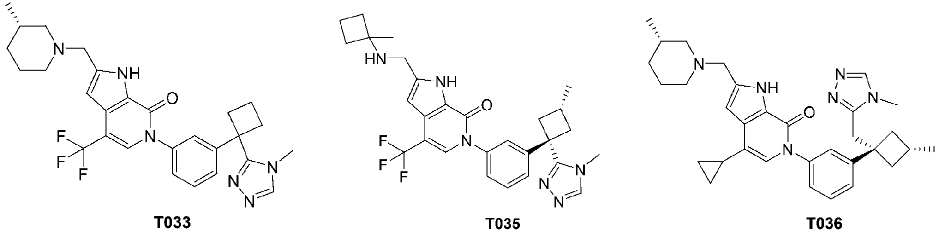

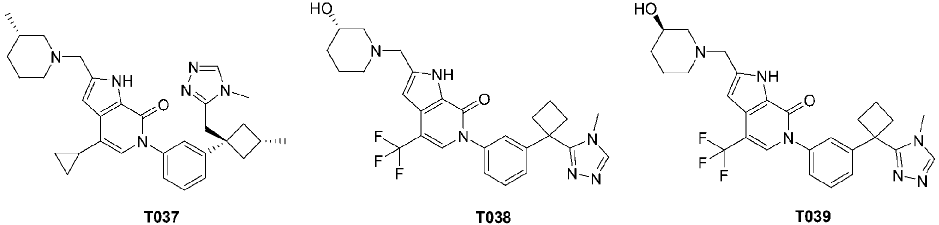

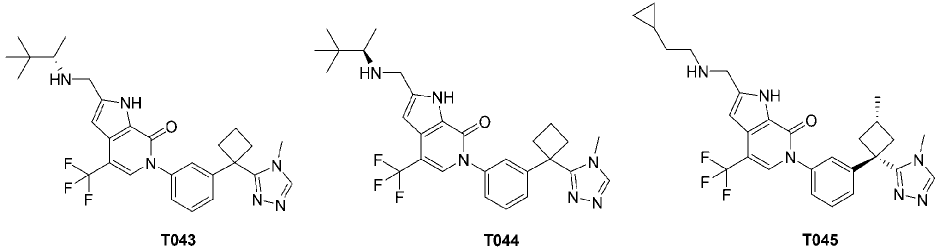

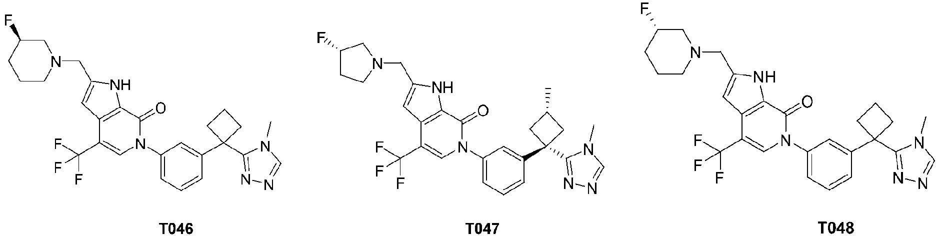

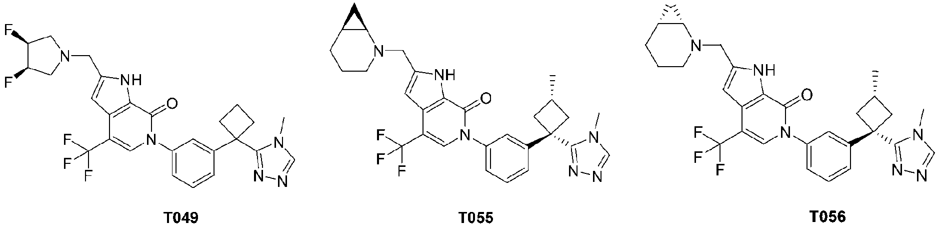

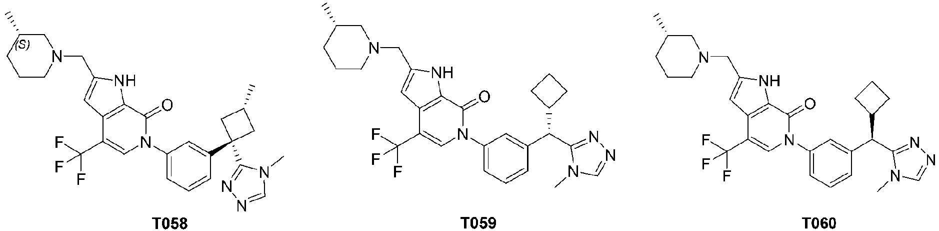

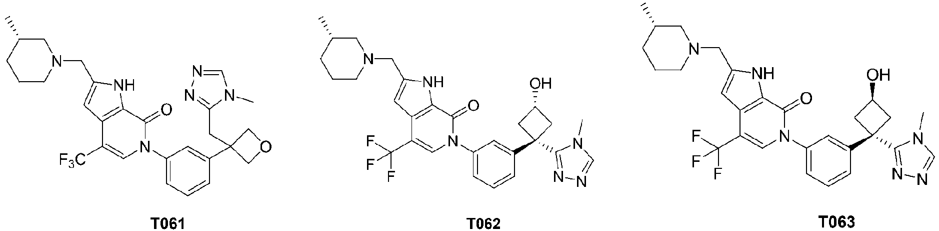

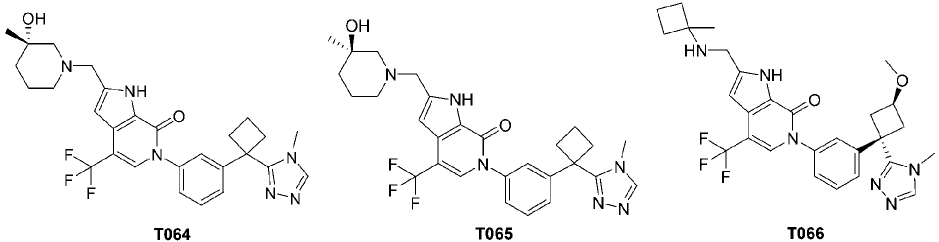

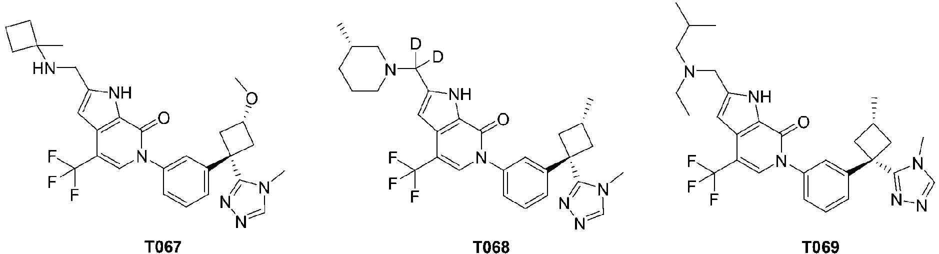

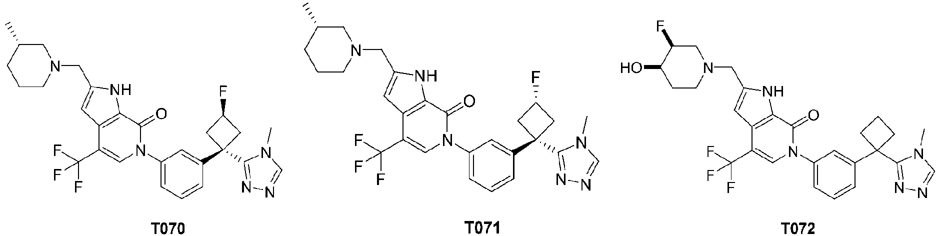

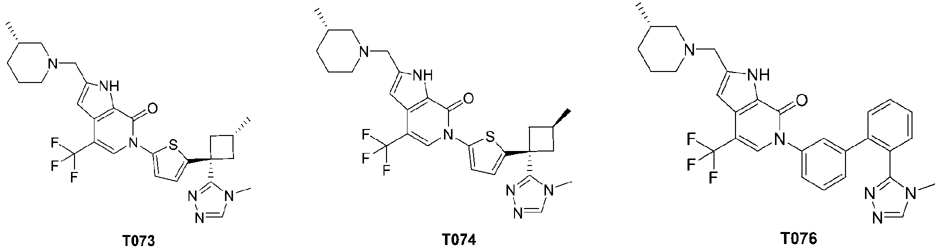

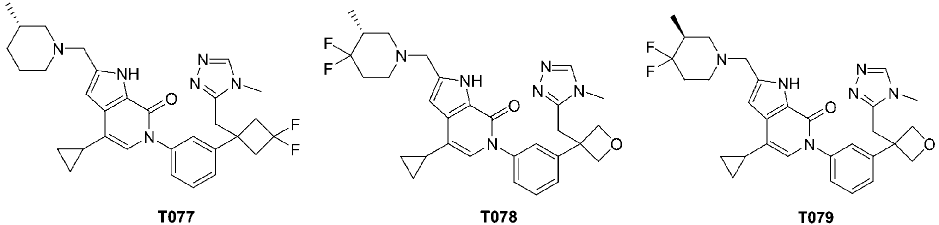

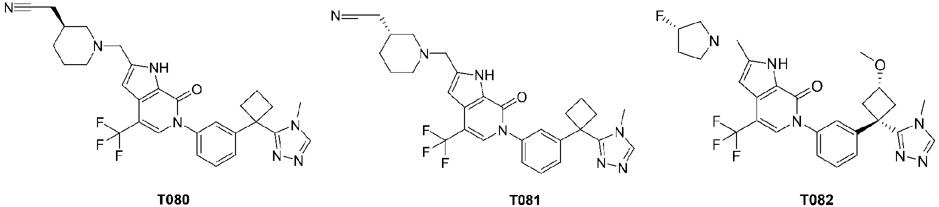

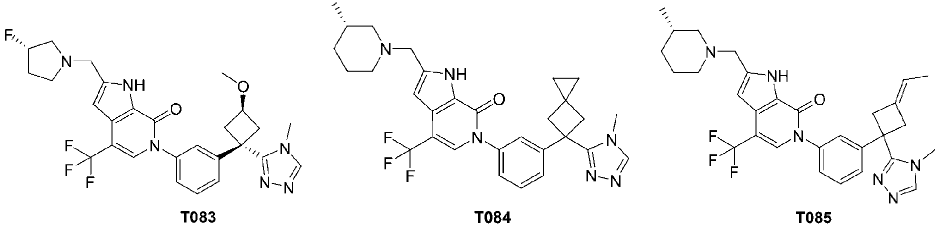

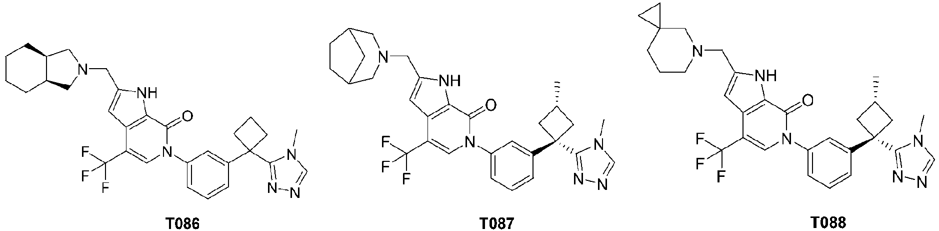

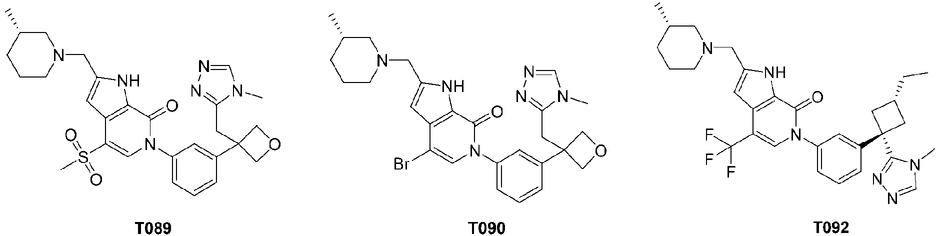

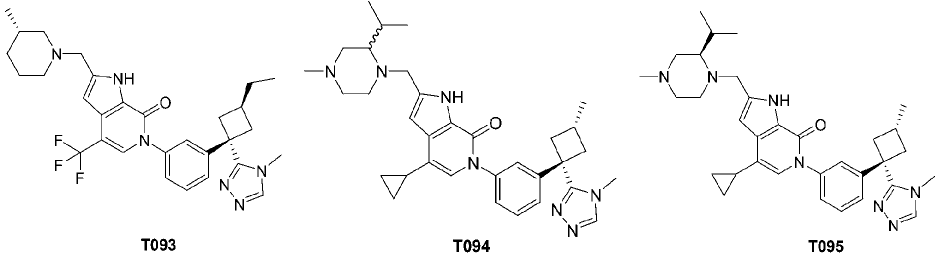

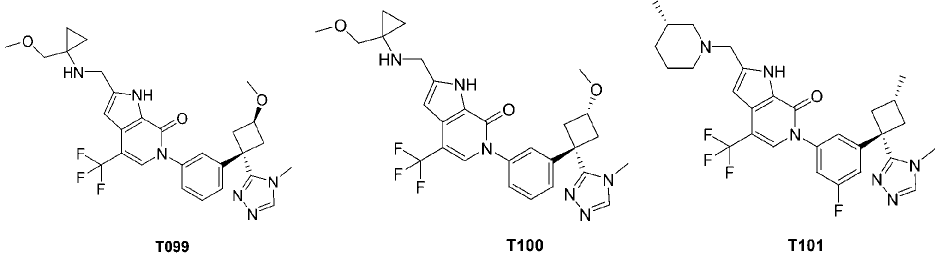

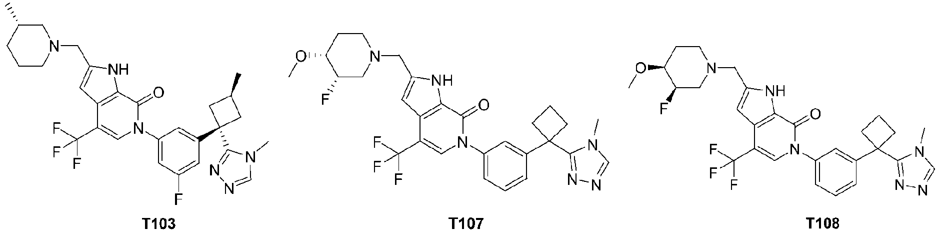

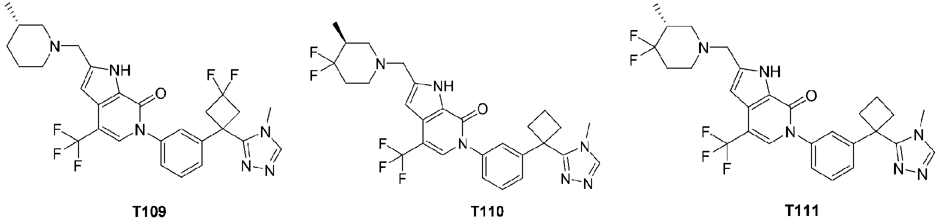

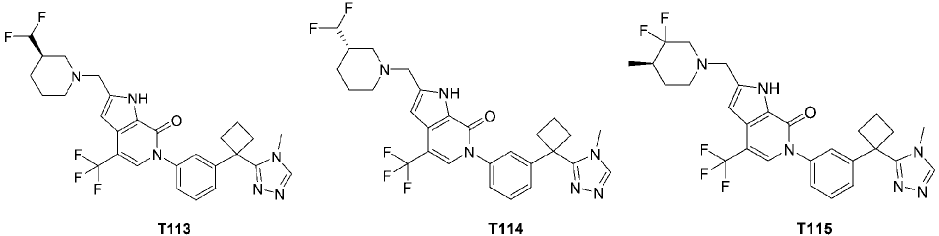

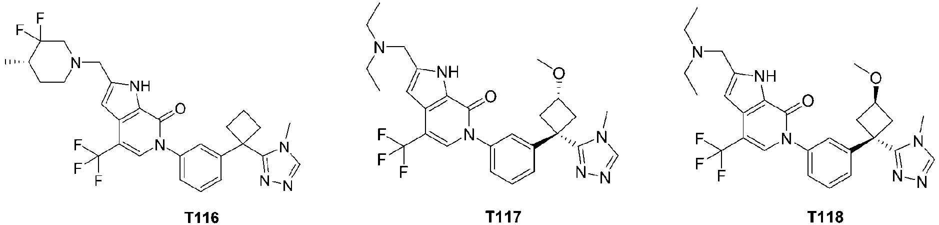

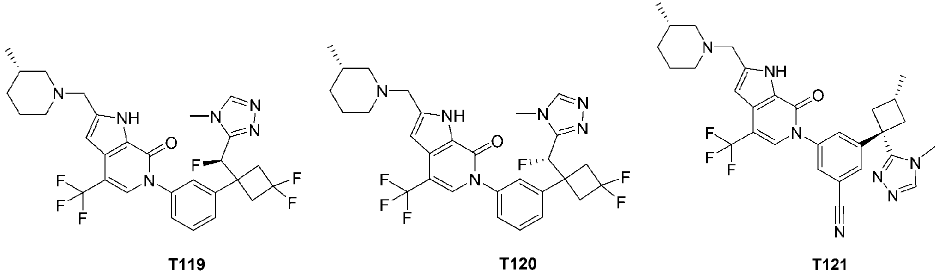

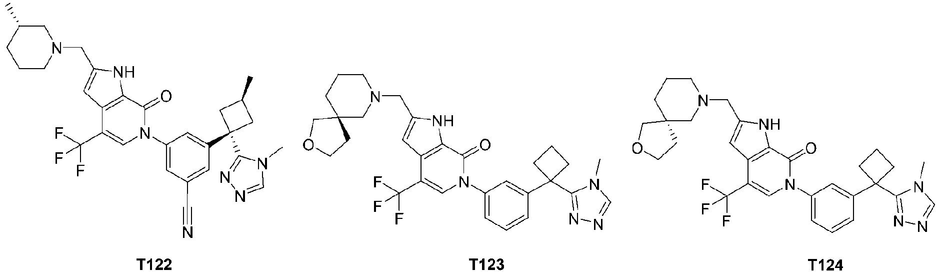

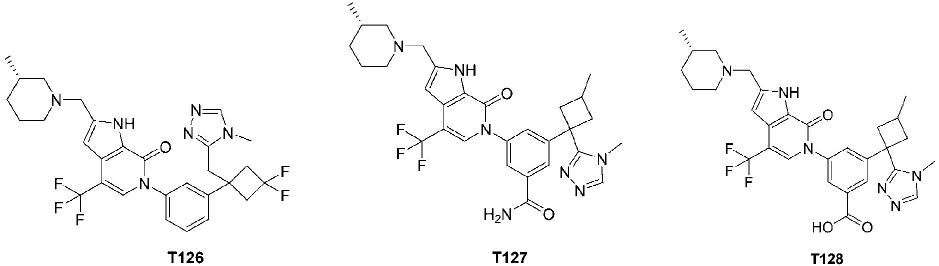

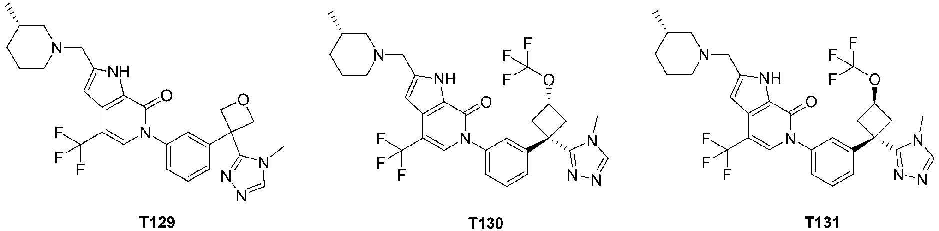

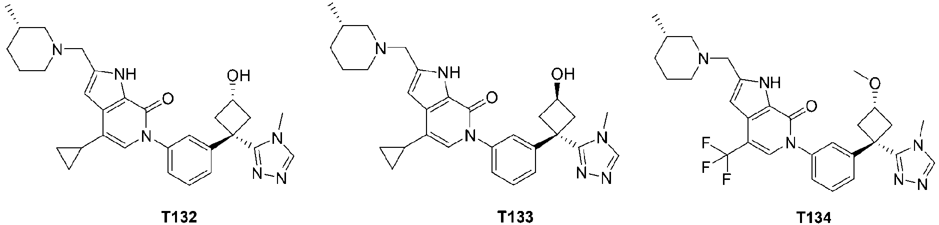

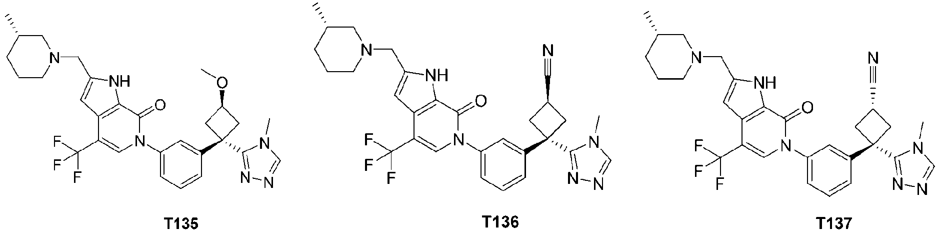

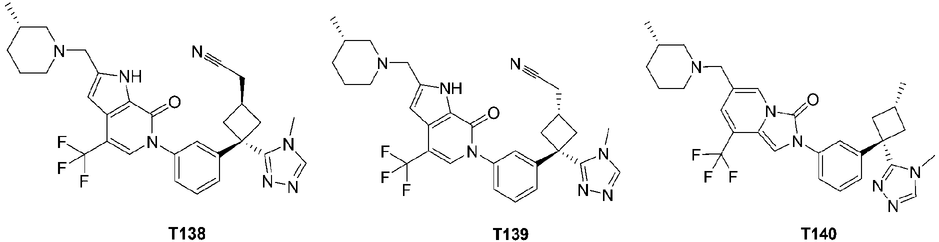

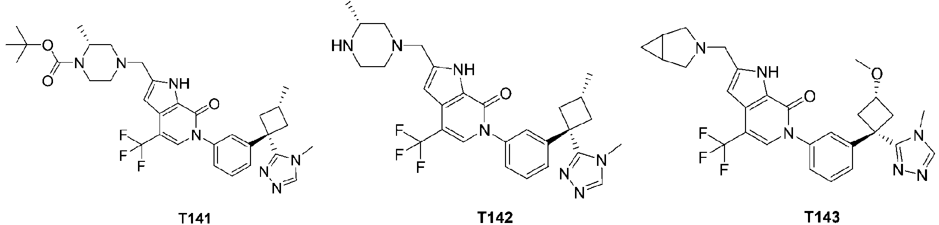

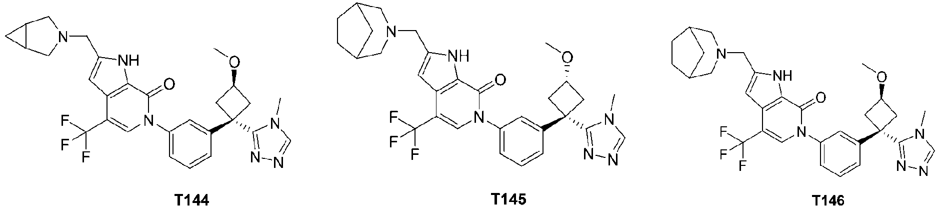

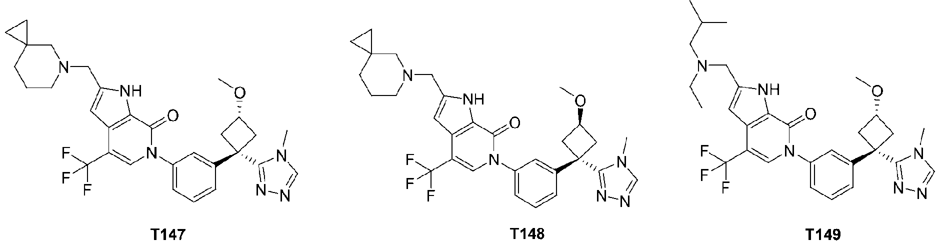

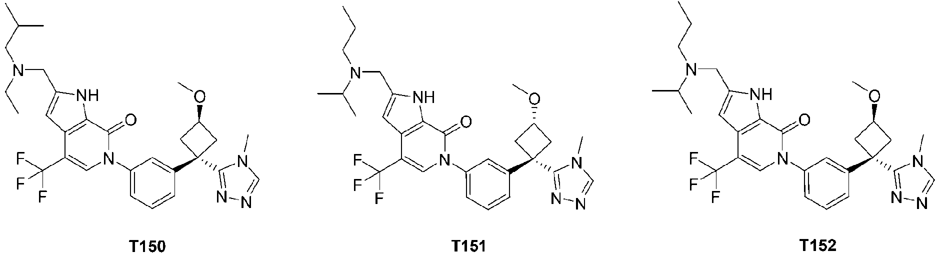

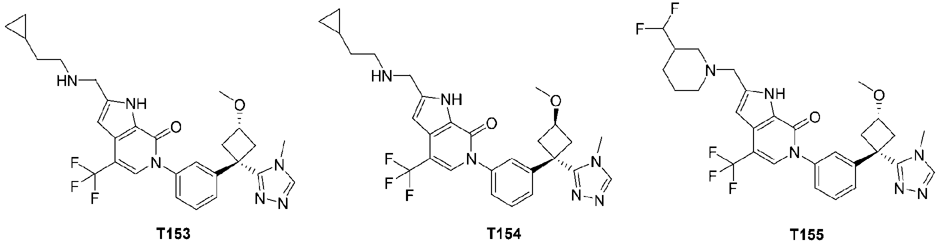

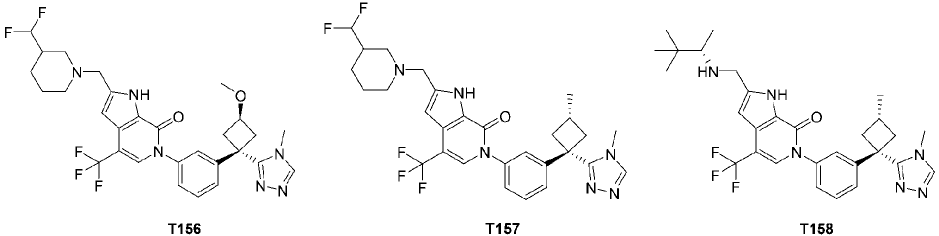

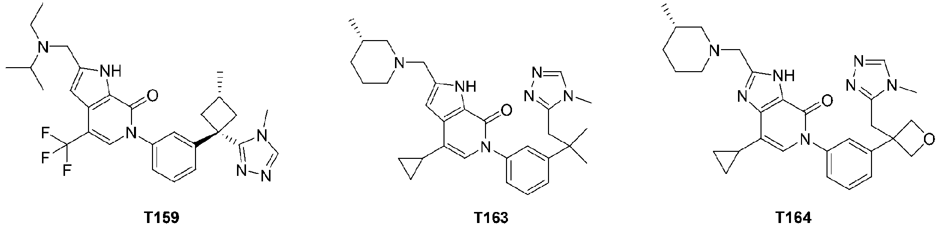

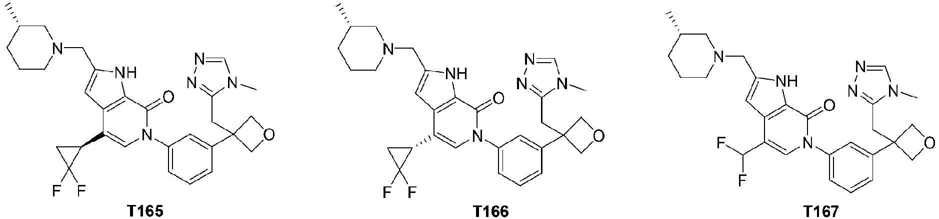

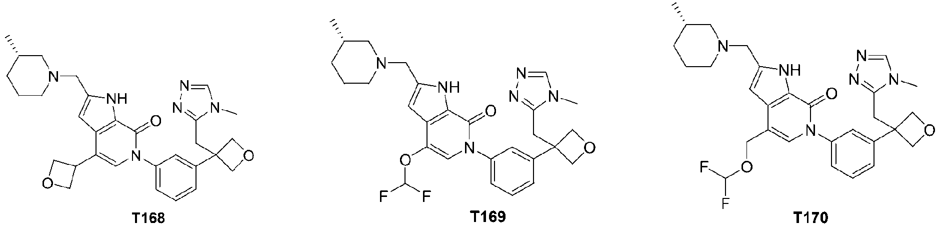

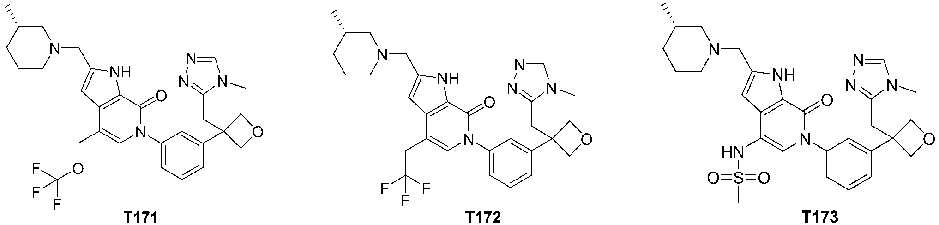

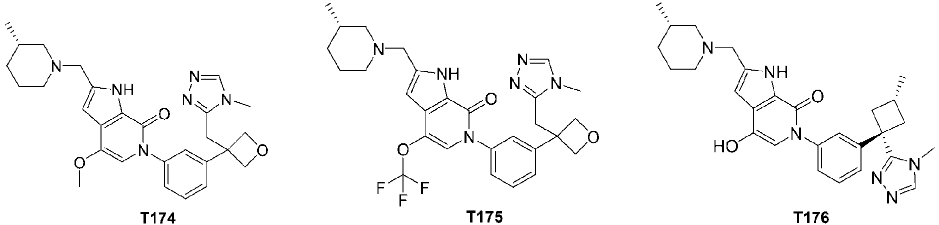

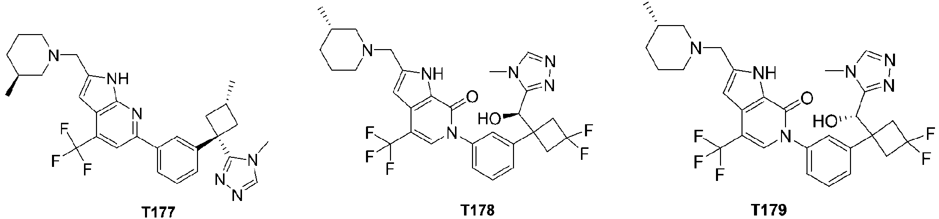

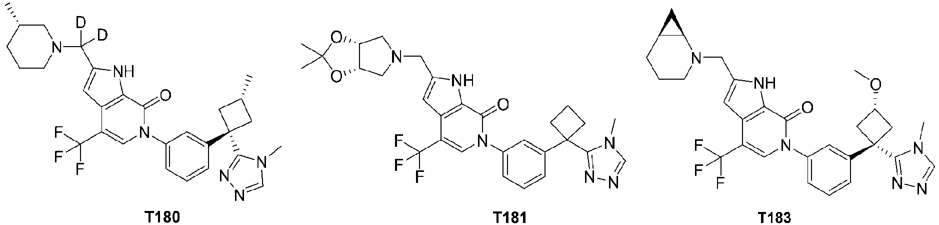

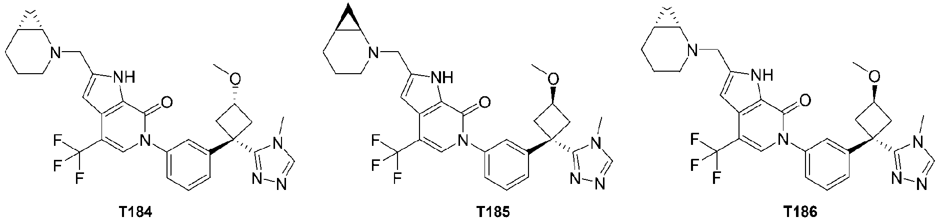

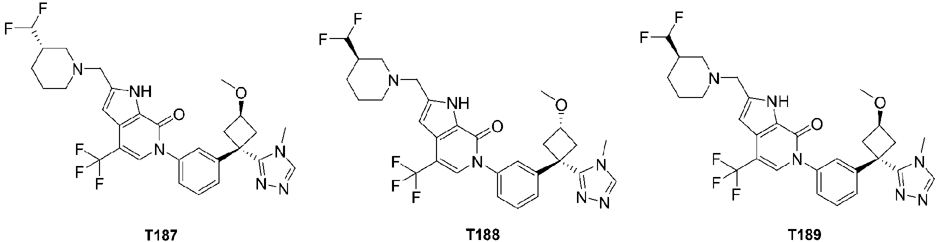

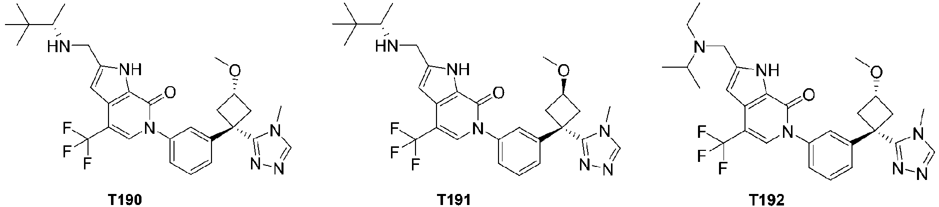

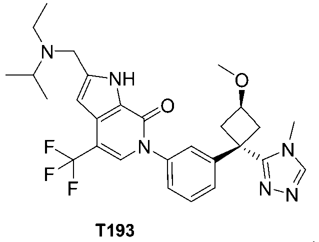

23. A pharmaceutical composition, comprising the compound, or the pharmaceutically acceptable salt, stereoisomer, ester, prodrug, solvate, or deuterated compound thereof according to any one of claims 1 to 22, and one or more pharmaceutically acceptable excipients.

24. The pharmaceutical composition according to claim 23, wherein the compound or the pharmaceutically acceptable salt, stereoisomer, ester, prodrug, solvate, or deuterated compound thereof is used alone, or the compound or the pharmaceutically acceptable salt, stereoisomer, ester, prodrug, solvate, or deuterated compound thereof is used in combination with a second active ingredient; and
preferably, the second active ingredient is a serotonin receptor antagonist, preferably a serotonin inhibitor, and more preferably selected from: ondansetron, granisetron, palonosetron, and dolasetron.

25. A use of the compound or the pharmaceutically acceptable salt, stereoisomer, ester, prodrug, solvate, or deuterated compound thereof according to any one of claims 1 to 22, in preparation of a drug for preventing and/or treating a disease related to Cbl-b activity.

26. The use according to claim 25, wherein the disease is selected from: an autoimmune disease, inflammatory disease, tumor, disease caused by pathogen infection, or disease related to pathogen infection;
preferably, the autoimmune disease is selected from: Achalasia, Addison's disease, adult Still's disease, agammaglobulinemia, alopecia areata, amyloidosis, ankylosing spondylitis, anti-glomerular basement membrane nephritis, antiphospholipid syndrome, autoimmune angioedema, autoimmune autonomic dysfunction, autoimmune encephalomyelitis, autoimmune hepatitis, autoimmune inner ear disease, autoimmune myocarditis, autoimmune oophoritis, autoimmune orchitis, autoimmune pancreatitis, autoimmune retinopathy, autoimmune urticaria, acute motor sensory axonal neuropathy, Barlow's disease, Behcet's disease, benign mucous membrane pemphigoid, bullous pemphigoid, Castleman disease, celiac disease, Chagas disease, chronic inflammatory demyelinating polyneuropathy, chronic recurrent multifocal osteomyelitis, Churg-Strauss syndrome, Cogan syndrome, cold agglutinin disease, congenital heart block, coxsackievirus myocarditis, CREST syndrome, Crohn's disease, dermatitis herpetiformis, dermatomyositis, Devic's disease, discoid lupus erythematosus, Dressler syndrome, endometriosis, eosinophilic esophagitis, eosinophilic fasciitis, erythema nodosum, essential mixed cryoglobulinemia, Evans syndrome, fibromyalgia, fibrosing alveolitis, giant cell arteritis, giant cell myocarditis, glomerulonephritis, Goodpasture syndrome, granulomatosis with polyangiitis, Graves' disease, Guillain-Barre syndrome, Hashimoto's thyroiditis, hemolytic anemia, Henoch-Schonlein purpura, herpes gestationis or pemphigoid gestationis, hidradenitis suppurativa, hypogammaglobulinaemia, IgA nephropathy, IgG4-related sclerosing diseases, immune thrombocytopenic purpura, inclusion body myositis, interstitial cystitis, juvenile idiopathic arthritis, juvenile dermatomyositis, Kawasaki disease, Lambert-Eaton myasthenic syndrome, leukocytoclastic vasculitis, lichen planus, lichen sclerosus et atrophicus, ligneous conjunctivitis, linear IgA disease, chronic Lyme disease, Meniere's disease, microscopic polyangiitis, mixed connective tissue disease, Mooren's ulcer, Mucha-Habermann disease, multifocal motor neuropathy, multiple sclerosis, myasthenia gravis, myositis, narcolepsy, neonatal lupus, neutropenia, ocular cicatricial pemphigoid, optic neuritis, palindromic rheumatism, pediatric autoimmune neuropsychiatric disorders associated with streptococcal infections (PANDAS), paraneoplastic cerebellar degeneration, paroxysmal nocturnal hemoglobinuria, Parry-Romberg syndrome, pars planitis, Parsonage-Turner syndrome, pemphigus, peripheral neuropathy, perivenous encephalomyelitis, pernicious anemia, POEMS syndrome, polyarteritis nodosa, autoimmune polyendocrine syndrome type I, II, and III, polymyalgia rheumatica, polymyositis, post-myocardial infarction syndrome, post-pericardotomy syndrome, primary biliary cholangitis, primary sclerosing cholangitis, progesterone dermatitis, psoriasis, psoriatic arthritis, pure red cell aplasia, pyoderma gangrenosum, Raynaud's phenomenon, reactive arthritis, reflex sympathetic dystrophy, relapsing polychondritis, restless legs syndrome, retroperitoneal fibrosis, rheumatic fever, rheumatoid arthritis, sarcoidosis, Schmidt syndrome, scleritis, scleroderma, Sjogren's syndrome, autoimmune orchitis and spermatogenic autoimmunity, stif person syndrome, subacute bacterial endocarditis, Susac syndrome, sympathetic ophthalmia, systemic lupus erythematosus, Takayasu arteritis, temporal arteritis, thyroid eye disease, Tolosa-Hunt syndrome, type 1 diabetes mellitus, ulcerative colitis, undifferentiated connective tissue disease, uveitis, vasculitis, vitiligo, and Koyanagi Harada disease;
preferably, the inflammatory disease is selected from: gout, chronic obstructive pulmonary disease, interstitial lung disease, inflammatory bowel disease, sepsis, asthma, and allergy;
preferably, the tumor is selected from: blastoma, medulloblastoma, retinoblastoma, sarcoma, liposarcoma, synovial sarcoma, neuroendocrine tumor, carcinoid tumor, gastrinoma, islet cell carcinoma, mesothelioma, schwannoma, acoustic neuroma, meningioma, adenocarcinoma, melanoma, squamous cell carcinoma, epithelial squamous cell carcinoma, lung cancer, peritoneal cancer, hepatocellular carcinoma, gastric cancer, intestinal cancer, pancreatic cancer, glioblastoma, cervical cancer, ovarian cancer, liver cancer, bladder cancer, liver cancer, breast cancer, colon cancer, rectal cancer, colorectal cancer, uterine cancer, salivary gland cancer, kidney cancer, prostate cancer, vulvar cancer, thyroid cancer, liver cancer, anal cancer, penile cancer, Merkel cell carcinoma, esophageal cancer, biliary tumors, head and neck cancer, and hematological malignancies;
more preferably, the tumor is a hematologic malignancy, such as leukemia, lymphoma, or multiple myeloma (MM); more preferably selected from: chronic lymphocytic leukemia, chronic myeloid leukemia, acute lymphoblastic leukemia (ALL), acute myeloid leukemia (AML), acute monocytic leukemia, B-cell lymphoma (e.g., diffuse large B-cell lymphoma (DLBCL), follicular lymphoma, mucosa-associated lymphoid tissue lymphoma (MALT), small lymphocytic lymphoma/chronic lymphocytic leukemia, mantle cell lymphoma (MCL)), and T/NK-cell lymphoma; and
more preferably, the tumor is a solid tumor, such as neuroblastoma, renal cell carcinoma, colon cancer, colorectal cancer, breast cancer, epithelial squamous cell carcinoma, melanoma, stomach cancer, esophageal cancer, gastroesophageal junction (GEJ) cancer, brain cancer, lung cancer, pancreatic cancer, cervical cancer, ovarian cancer, liver cancer, bladder cancer, prostate cancer, testicular cancer, thyroid cancer, uterine cancer, adrenal cancer, head and neck cancer, or urothelial carcinoma; especially, ovarian cancer, stomach cancer, gastroesophageal junction (GEJ) cancer, head and neck squamous cell carcinoma, metastatic or unresectable melanoma, non-small cell lung cancer, metastatic castration-resistant prostate cancer (mCRPC), malignant pleural mesothelioma (MPM), breast cancer, metastatic urothelial carcinoma, cervical cancer, and metastatic colorectal cancer.
